# EUROPEAN PATENT APPLICATION

(11) **EP 4 821 928 A1**
(43) Date of publication of application: **14.10.2026**
(21) Application number: 24900728.7
(22) Date of filing: 09.12.2024
(51) Int. Cl.: C07D 413/04, A01N 43/86, A01N 43/90, A01N 47/12, A01P 3/00, C07D 417/04, C07D 471/04

(54) **BICYCLIC HETEROCYCLIC COMPOUND AND FUNGICIDE FOR AGRICULTURAL AND HORTICULTURAL USE CONTAINING SAME AS ACTIVE INGREDIENT**

(30) Priority: 08.12.2023 JP 2023207933
(71) Applicant: Mitsui Chemicals Crop & Life Solutions, Inc., Tokyo 103-0027 (JP)
(72) Inventor: YANAGI, Masanori, Mobara-shi, Chiba 297-0017 (JP); YASUDOMI, Takaya, Mobara-shi, Chiba 297-0017 (JP); HIRAOKA, Daiki, Mobara-shi, Chiba 297-0017 (JP); SAKURADA, Akane, Mobara-shi, Chiba 297-0017 (JP); UMEZAKI, Yui, Mobara-shi, Chiba 297-0017 (JP); SHINO, Mamiko, Mobara-shi, Chiba 297-0017 (JP); ASANO, Kouta, Mobara-shi, Chiba 297-0017 (JP); UMETANI, Hideki, Tokyo 103-0027 (JP)
(74) Representative: J A Kemp LLP
(86) International application number: PCT/JP2024/043477
(87) International publication number: WO 2025/121436

(57) **Abstract**

The present invention relates to a compound represented by the following formula (1) or a salt thereof: 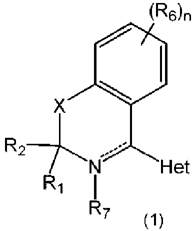 wherein Het represents the formula (2): 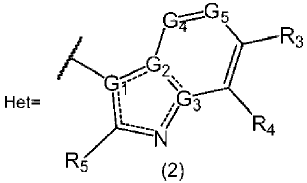 and each group in the formula (1) and the formula (2) is defined as described in the specification.

## Description

### [Technical Field]

The present invention relates to a bicyclic heterocyclic compound and an agricultural chemical containing the same as active ingredient.

### [Background Art]

The control of diseases in agricultural and horticultural crops plays an important role in securing stable agricultural production. Hence, various germicides are used. However, use of such germicides over a long year causes emergence of drug-resistant strains. Therefore, there is a strong demand for novel germicides effective for not only drug-sensitive strains but drug-resistant strains.

By the way, as for 2H-benzo[e][1,3]oxazine compounds, for example, Patent Literatures 1 and 2 each disclose 2,2-disubstituted 4-(quinolin-3-yl)-2H-benzo[e][1,3]oxazine compound. For example, Patent Literature 3 discloses a 2,2-disubstituted 4-(pyridin-3-yl)-2H-benzo[e][1,3]oxazine compound.

As for 2H-benzo[e][1,3]thiazine compounds, for example, Patent Literature 1 discloses a 2,2-disubstituted 4-(quinolin-3-yl)-2H-benzo[e][1,3]thiazine compound. For example, Patent Literature 4 discloses a 2,2-disubstituted 4-(pyridin-3-yl)-2H-benzo[e][1,3]thiazine compound.

As for benzo[d]imidazole compounds and pyrazolo[1,5-a]pyridine compounds having a nitrogen-containing 5-6 condensed heterocycle, for example, Patent Literature 5 discloses a 1-(1H-benzo[d]imidazol-1-yl)-3,3,4,4-tetrasubstituted 3,4-dihydroisoquinoline compound. For example, Patent Literature 6 discloses a 3,3,4,4-tetrasubstituted 1-(pyrazolo[1,5-a]pyridin-3-yl)-3,4-dihydroisoquinoline compound.

### [Citation List]

### [Patent Literature]

[Patent Literature 1] International Publication No. WO 2009/119089
[Patent Literature 2] International Publication No. WO 2022/243109
[Patent Literature 3] International Publication No. WO 2022/243107
[Patent Literature 4] International Publication No. WO 2023/111215
[Patent Literature 5] International Publication No. WO 2016/156085
[Patent Literature 6] International Publication No. WO 2017/025510

### [Summary of Invention]

### [Problems to be Solved by the Invention]

Problems of conventional control agents, however, are insufficient control effects on plant diseases and limited use of the control agents due to the emergence of pathogens having drug resistance. Novel control agents are desired for solving such problems.

An object of the present invention is to provide a novel compound, an agricultural and horticultural pest control agent and an agricultural and horticultural germicide containing the compound or a salt thereof, and a method for controlling a plant disease.

### [Means for Solving the Problems]

The present inventors have found that a compound having a predetermined structure can attain the object, leading to the completion of the present invention.

Specifically, the present invention encompasses the following aspects.
[1] A compound represented by the following formula (1) or a salt thereof: 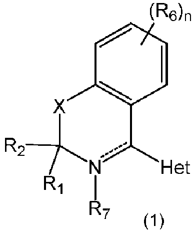 wherein
   R₁ and R₂ each independently represent a group selected from the group consisting of a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C1 to C6 acyl group optionally substituted by substituent A, an aryl group optionally substituted by substituent B, a heteroaryl group optionally substituted by substituent B, -C(=O)NRaRb (wherein Ra and Rb each independently represent a group selected from the group consisting of a hydrogen atom, a C1 to C6 alkyl group optionally substituted by substituent A, a C1 to C6 haloalkyl group, a C3 to C8 cycloalkyl group, a C1 to C6 acyl group, and -C(=O)ORc (wherein Rc represents a group selected from the group consisting of a hydrogen atom, a C1 to C6 alkyl group optionally substituted by substituent A, a C1 to C6 haloalkyl group, and a C3 to C8 cycloalkyl group), or Ra and Rb form, together with the nitrogen atom bonded thereto, an aziridinyl group, an azetidinyl group, a pyrrolidinyl group, a piperidinyl group, a homopiperidinyl group, or an azocanyl group), and -C(=O)ORc (wherein Rc is as defined above),or
   R₁ and R₂ together optionally form a C3 to C8 cycloalkyl ring optionally substituted by substituent A, or a C3 to C8 saturated or unsaturated heterocyclic ring optionally substituted by substituent A;
   Het represents the formula (2): 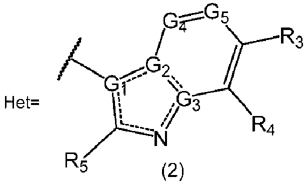
   R₃, R₄, and R₅ in the formula (2) each independently represent a group selected from the group consisting of a hydrogen atom, a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, -NRaRb (wherein Ra and Rb are as defined above), - C(=O)NRaRb (wherein Ra and Rb are as defined above), -C(=O)ORc (wherein Rc is as defined above), - OC(=O)Rc (wherein Rc is as defined above), and -L-Rd (wherein Rd represents a group selected from the group consisting of a C1 to C6 alkyl group and a C1 to C6 haloalkyl group, and L represents a group selected from the group consisting of S, S(=O), and SO₂), or
   R₃ and R₄ together form a C5 to C10 unsaturated ring optionally substituted by substituent A;
   n represents an integer of 0 to 4;
   R₆ represents a group selected from the group consisting of a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, -NRaRb (wherein Ra and Rb are as defined above), -C(=O)ORc (wherein Rc is as defined above), -C(=O)NRaRb (wherein Ra and Rb are as defined above), -OC(=O)Rc (wherein Rc is as defined above), and -L-Rd (wherein L and Rd are as defined above);
   R₇ represents a group selected from the group consisting of a lone pair, a hydrogen atom, a C1 to C6 alkyl group optionally substituted by substituent A, a C1 to C6 haloalkyl group, a C2 to C6 alkenyl group optionally substituted by substituent A, a C2 to C6 haloalkenyl group, a C2 to C6 alkynyl group optionally substituted by substituent A, a C2 to C6 haloalkynyl group, and an acyl group optionally substituted by substituent A;
   G₁, G₂, and G₃ each independently represent a group selected from the group consisting of a carbon atom and a nitrogen atom (on the proviso that at least one of G₁, G₂, and G₃ is a carbon atom);
   G₄ and G₅ each independently represent a group selected from the group consisting of a nitrogen atom and CRx;
   Rx represents a group selected from the group consisting of a hydrogen atom, a halogen atom, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, -NRaRb (wherein Ra and Rb are as defined above), - C(=O)NRaRb (wherein Ra and Rb are as defined above), -C(=O)ORc (wherein Rc is as defined above), and -L-Rd (wherein L and Rd are as defined above);
   the binding containing the broken line represents a single bond or a double bond;
   X represents a group selected from the group consisting of O, S, S(=O), SO₂, and NRe;
   Re represents a group selected from the group consisting of a hydrogen atom, a C1 to C6 alkyl group optionally substituted by substituent A, a C1 to C6 haloalkyl group, a C2 to C6 alkenyl group optionally substituted by substituent A, a C2 to C6 haloalkenyl group, a C2 to C6 alkynyl group optionally substituted by substituent A, and a C2 to C6 haloalkynyl group;
   the substituent A is at least one member selected from the group consisting of a hydroxy group, a cyano group, a C3 to C8 cycloalkyl group, a C1 to C6 alkoxy group, a C1 to C6 haloalkoxy group, and a C1 to C6 cycloalkoxy group, a C1 to C6 alkenyloxy group, a C1 to C6 haloalkenyloxy group, a C1 to C6 alkynyloxy group, and a C1 to C6 haloalkynyloxy group, a C1 to C6 acyl group, an aryl group optionally substituted by substituent B, a heteroaryl group optionally substituted by substituent B, a phenoxy group optionally substituted by substituent B, -NRaRb (wherein Ra and Rb are as defined above), -C(=O)NRaRb (wherein Ra and Rb are as defined above), -C(=O)ORc (wherein Rc is as defined above), -OC(=O)Rc (wherein Rc is as defined above), and -L-Rd (wherein L and Rd are as defined above); and
   the substituent B is at least one member selected from the group consisting of a halogen atom, a hydroxy group, a cyano group, a nitro group, a C1 to C6 alkyl group optionally substituted by substituent A, a C1 to C6 haloalkyl group, a C3 to C8 cycloalkyl group, a C1 to C6 alkoxy group, a C1 to C6 haloalkoxy group, a C3 to C8 cycloalkoxy group, and Rd-L- (wherein Rd is as defined above).
[2] The compound or the salt thereof according to [1], wherein at least one of G₄ and G₅ is a nitrogen atom.
[3] The compound or the salt thereof according to [1] or [2], wherein
   R₃, R₄, and R₅ each independently represent a group selected from the group consisting of a hydrogen atom, a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, and a C3 to C6 haloalkynyloxy group, or
   R₃ and R₄ together form a C5 to C10 unsaturated ring optionally substituted by substituent A;
   n represents an integer of 0 to 2;
      R₆ represents a group selected from the group consisting of a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, and -NRaRb (wherein Ra and Rb are as defined above);
   R₇ represents a group selected from the group consisting of a lone pair, a hydrogen atom, a C1 to C6 alkyl group optionally substituted by substituent A, a C1 to C6 haloalkyl group, and an acyl group optionally substituted by substituent A;
   G₄ and G₅ each independently represent a group selected from the group consisting of a nitrogen atom and CRx; and
   Rx represents a group selected from the group consisting of a hydrogen atom, a halogen atom, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, and a C2 to C6 haloalkynyl group.
   In [1] to [3],
   R₁ and R₂ each independently represent a group selected from the group consisting of a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C1 to C6 acyl group optionally substituted by substituent A, an aryl group optionally substituted by substituent B, and a heteroaryl group optionally substituted by substituent B, or
   R₁ and R₂ together form a C3 to C8 cycloalkyl ring optionally substituted by substituent A or a C3 to C8 saturated or unsaturated heterocyclic ring optionally substituted by substituent A, or
   R₁ and R₂ each independently represent a group selected from the group consisting of a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C1 to C6 acyl group optionally substituted by substituent A, an aryl group optionally substituted by substituent B, and a heteroaryl group optionally substituted by substituent B, or
   R₁ and R₂ together form a C3 to C8 cycloalkyl ring optionally substituted by substituent A.
[4] The compound or the salt thereof according to any of [1] to [3], wherein at least one of G₄ and G₅ is a nitrogen atom.
[5] The compound or the salt thereof according to any of [1] to [4], wherein
   R₁ and R₂ each independently represent a group selected from the group consisting of a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, a C1 to C6 acyl group optionally substituted by substituent A, an aryl group optionally substituted by substituent B, a heteroaryl group optionally substituted by substituent B, and -C(=O)ORc (wherein Rc is as defined above), or
   R₁ and R₂ together form a C3 to C8 cycloalkyl ring optionally substituted by substituent A or a C3 to C8 saturated or unsaturated heterocyclic ring optionally substituted by substituent A;
   R₃, R₄, and R₅ each independently represent a group selected from the group consisting of a hydrogen atom, a halogen atom, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, and a C2 to C6 alkenyl group optionally substituted by substituent A, or R₃ and R₄ together form a C5 to C10 unsaturated ring optionally substituted by substituent A;
   n represents an integer of 0 to 2;
   R₆ represents a group selected from the group consisting of a halogen atom, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C3 to C8 cycloalkoxy group optionally substituted by substituent A, a C2 to C6 alkenyl group optionally substituted by substituent A, and -NRaRb (wherein Ra and Rb are as defined above);
   R₇ represents a lone pair;
   at least one of G₁, G₂, and G₃ represents a carbon atom and at least one represents a nitrogen atom;
   G₄ and G₅ each independently represent a group selected from the group consisting of a nitrogen atom and CRx;
   Rx represents a group selected from the group consisting of a hydrogen atom and a C1 to C6 alkyl group optionally substituted by substituent A;
   the binding containing the broken line represents a single bond or a double bond; and
   X represents a group selected from the group consisting of O, S, S(=O), and SO₂.
[5A] The compound or the salt thereof according to any of [1] to [5], wherein
   R₁ and R₂ each independently represent a methyl group, an ethyl group, a propyl group, an isopropyl group, a butyl group, an isobutyl group, a s-butyl group, a t-butyl group, a pentyl group, a isopentyl group, a 1-methylbutyl group, a 2-methylbutyl group, a neopentyl group, a 1-ethylpropyl group, a 1,2-dimethylpropyl group, a hexyl group, a chloromethyl group, a cyclopropyl group, a cyclobutyl group, a 1-butenyl group, a 4-methyl-3-pentenyl group, an acetyl group, a phenyl group, a 2-fluorophenyl group, a 3-fluorophenyl group, a 4-fluorophenyl group, a 2- chlorophenyl group, a 3- chlorophenyl group, a 4- chlorophenyl group, a 2-methylphenyl group, a 3- methylphenyl group, a 4- methylphenyl group, a 2-methoxyphenyl group, a 3-methoxyphenyl group, a 4-methoxyphenyl group, a 5-chlorothiophene-2-yl group, an ethoxycarbonyl group, a methoxymethyl group, a methoxyethyl group, an ethoxyethyl group, a methylthiomethyl group, a methylsulfonylmethyl group, a 2-amino-oxoethyl group, a 2-(methylamino)-2-oxoethyl group, a 2-(dimethylamino)-2-oxoethyl group, a 2- carboxymethyl group, a 2-methoxy-2-oxoethyl group, a 2-ethoxy-2-oxoethyl group, a 3-methoxy-3-oxopropyl group, a 3-cyanopropyl group, or a 2-phenethyl group; or
   R₁ and R₂ together form a cyclobutyl group, a cyclopentyl group, a cyclohexyl group, or a 3-oxetanyl group.
[5B] The compound or the salt thereof according to any of [1] to [5], wherein
   R₃ and R₄ each independently represent a hydrogen atom, a fluoro group, a chloro group, a bromo group, a methyl group, an ethyl group, difluoromethyl group, a trifluoromethyl group, a cyclopropyl group, a methoxy group, a vinyl group, or a 1-propen-2-yl group; or
   R₃ and R₄ together form a cyclopentenyl group.
[5C] The compound or the salt thereof according to any of [1] to [5], wherein R₅ is a hydrogen atom or a methyl group.
[5D] The compound or the salt thereof according to any of [1] to [5], wherein R₆ is a fluoro group, a chloro group, a bromo group, a methyl group, an ethyl group, a propyl group, a cyclopropyl group, a methoxy group, a vinyl group, a 1-propen-2-yl group, a methylamino group, an ethylamino group, a dimethylamino group, ethylmethylamino group, or a t-butoxycarbonylamino group.
[5E] The compound or the salt thereof according to any of [1] to [5], wherein n represents an integer of 0 to 2.
[5F] The compound or the salt thereof according to any of [1] to [5], wherein R₇ represents a lone pair and the binding containing the broken line which a nitrogen atom bonded to R₇ forms represents a double bond.
[5G] The compound or the salt thereof according to any of [1] to [5], wherein X represents a group selected from the group consisting of O, S, S(=O), and SO₂.
   In [5A] to [5G], [5] in the description "The compound or the salt thereof according to any of [1] to [5]" is used in the meaning to include the preceding [5A] to [5G]. Also, in "any of [1] to [integer being 5 or more]" of [6] to [12] below, [5] is used in the meaning to include [5A] to [5G].
[6] The compound or the salt thereof according to any of [1] to [5], wherein the formula (2) is selected from the group consisting of following (2-1), (2-2), (2-3), (2-4), (2-5), (2-6), (2-7), (2-8), (2-9), (2-10), and (2-11), 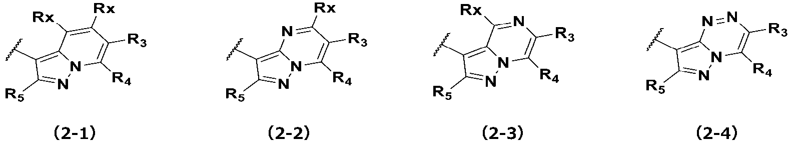 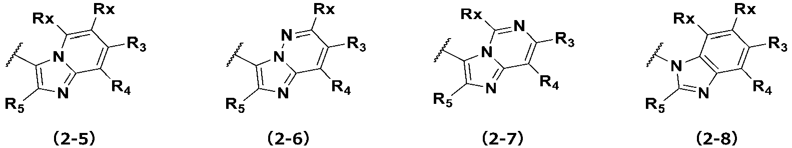 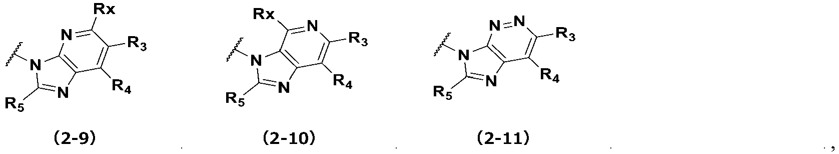 and R₃, R₄, and Rx are as defined above and preferably Rx represents a hydrogen atom or a methyl group.
[7] The compound or the salt thereof according to any of [1] to [5], wherein the formula (2) is selected from the group consisting of following (2-1), (2-2), (2-3), (2-5), (2-6), (2-8), and (2-9), 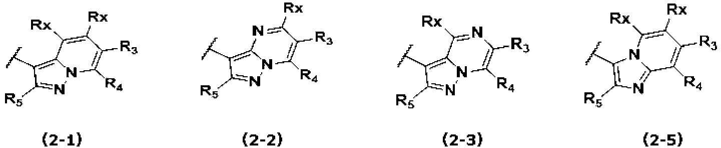 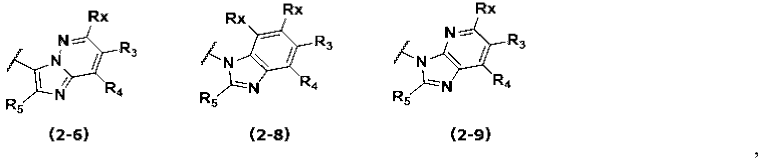 and
   R₃, R₄, and Rx are as defined above and preferably Rx represents a hydrogen atom or a methyl group.
[8] The compound or the salt thereof according to any of [1] to [5], wherein the formula (2) is selected from the group consisting of following (2-2), (2-3), (2-6), and (2-9), 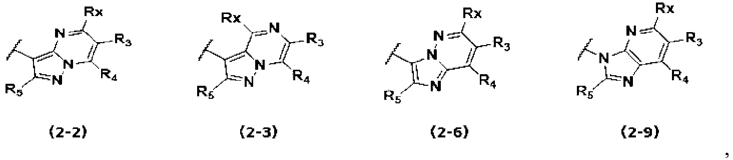 and
   R₃, R₄, and Rx are as defined above and preferably Rx represents a hydrogen atom or a methyl group.
[9] An agricultural and horticultural pest control agent comprising a compound or a salt thereof according to any of [1] to [8].
[10] An agricultural and horticultural germicide comprising a compound or a salt thereof according to any of [1] to [8].
[11] A method for controlling a plant disease, comprising applying a compound or a salt thereof according to any of [1] to [8] or an agricultural and horticultural pest control agent according to [9] to a plant, a plant seed, or soil for cultivating a plant.
[12] A method for controlling a plant disease, comprising applying a compound or a salt thereof according to any of [1] to [8] or an agricultural and horticultural germicide according to [10] to a plant, a plant seed, or soil for cultivating a plant.

### [Advantageous Effects of Invention]

The present invention can provide a novel compound, an agricultural and horticultural pest control agent and an agricultural and horticultural germicide containing the compound or a salt thereof, and a method for controlling a plant disease.

### [Mode for Carrying Out the Invention]

Hereinafter, the mode for carrying out the present invention (hereinafter, referred to as the "present embodiment") will be described in detail. The present embodiment is an exemplary embodiment for illustrating the present invention and does not intend to limit the present invention to the contents given below. The present invention can be carried out through appropriate changes or modifications made without departing from the spirit of the present invention.

In the present specification, the abbreviations used will be described below.

DMF: N,N-dimethylformamide, THF: tetrahydrofuran, Me: a methyl group, Et: an ethyl group, Pr: a propyl group, Bu: a butyl group, Ac: an acetyl group, Ph: a phenyl group, i: iso, sec: secondary, t: tertiary, c: cyclo, o: ortho, m: meta, p: para, =: a double bond, and ≡: a triple bond. In the columns of tables, Pr and Bu without a prefix mean normal groups.

Hereinafter, the definitions of terms used in the present specification will be described.

In the present specification, the term "optionally substituted" means that a group may be substituted by a substituent or may not be substituted. The term "optionally substituted" is used interchangeably with the term "substituted or unsubstituted". When the number of substituents is not specified, the number of substituents is not particularly limited.

In the present specification, the term "Cx to Cy" means having x to y carbon atoms. In this context, x and y each represent an integer, and it should be understood that all integers present between x and y are individually disclosed. For example, C1 to C6 means having 1, 2, 3, 4, 5, or 6 carbon atoms, C2 to C6 means having 2, 3, 4, 5, or 6 carbon atoms, C3 to C8 means having 3, 4, 5, 6, 7, or 8 carbon atoms, C3 to C6 means having 3, 4, 5, or 6 carbon atoms, and C1 to C3 means having 1, 2, or 3 carbon atoms.

In the present specification, the "C1 to C6 alkyl group" is a linear or branched hydrocarbon group having 1 to 6 carbon atoms. Examples of the "C1 to C6 alkyl group" include a methyl group, an ethyl group, a n-propyl group, an isopropyl group, a n-butyl group, an isobutyl group, a sec-butyl group, a tert-butyl group, a n-pentyl group, an isopentyl group, a 1-methylbutyl group, a 2-methylbutyl group, a neopentyl group, a 1-ethylpropyl group, a 1,2-dimethylpropyl group, a n-hexyl group, a 1-methylpentyl group, a 2-methylpentyl group, a 3-methylpentyl group, a 4-methylpentyl group, a 1,1-dimethylbutyl group, a 2,2-dimethylbutyl group, a 3,3-dimethylbutyl group, a 1,2-dimethylbutyl group, a 1,3-dimethylbutyl group, a 2,3-dimethylbutyl group, a 2-ethylbutyl group, a 1-isopropylpropyl group, a 1,1,2-trimethylpropyl group, and a 1,2,2-trimethylpropyl group.

In the present specification, examples of the "halogen atom" include a fluorine atom, a chlorine atom, a bromine atom, and an iodine atom.

In the present specification, the "C1 to C6 haloalkyl group" is a group in which at least one hydrogen atom in the C1 to C6 alkyl group is replaced with a halogen atom. When two or more hydrogen atoms are replaced with halogen atoms, these halogen atoms may be the same or different. The number of halogen atoms as substituents is not particularly limited as long as their presence as substituents is attained. Examples of the "C1 to C6 haloalkyl group" include a monofluoromethyl group, a difluoromethyl group, a trifluoromethyl group, a monochloromethyl group, a dichloromethyl group, a monobromomethyl group, a dibromomethyl group, a monoiodomethyl group, a diiodomethyl group, a chlorodifluoromethyl group, a bromodifluoromethyl group, a trichloromethyl group, a 1-fluoroethyl group, a 2-fluoroethyl group, a 1,1-difluoroethyl group, a 2,2-difluoroethyl group, a 2,2,2-trifluoroethyl group, a 1,1,2,2-tetrafluoroethyl group, a pentafluoroethyl group, a 2,2,2-trichloroethyl group, a 3,3-difluoropropyl group, a 3,3,3-trifluoropropyl group, a heptafluoropropyl group, a heptafluoroisopropyl group, a 2,2,2-trifluoro-1-(trifluoromethyl)ethyl group, a nonafluorobutyl group, a nonafluoro-sec-butyl group, a 3,3,4,4,5,5,5-heptafluoropentyl group, a undecafluoropentyl group, and a tridecafluorohexyl group.

In the present specification, the "C3 to C8 cycloalkyl group" is a cyclic hydrocarbon group having 3 to 8 carbon atoms. Examples of the "C3 to C8 cycloalkyl group" include a cyclopropyl group, a cyclobutyl group, a cyclopentyl group, a cyclohexyl group, a cycloheptyl group, and a cyclooctyl group.

In the present specification, the "C2 to C6 alkenyl group" is a group having at least one double bond in a C2 to C6 alkyl group among the C1 to C6 alkyl group. A geometric isomer, if any, can be an E form, a Z form, or a combination. Examples of the "C2 to C6 alkenyl group" include a vinyl group, an allyl group, a 1-propenyl group, an isopropenyl group, a 1-butenyl group, a 2-butenyl group, a 3-butenyl group, a 1,3-butanedienyl group, a 1-pentenyl group, a 2-pentenyl group, a 3-pentenyl group, a 4-pentenyl group, a 3-methyl-2-butenyl group, a 1-hexenyl group, a 2-hexenyl group, a 3-hexenyl group, a 4-hexenyl group, a 5-hexenyl group, a 4-methyl-3-pentenyl group, and a 3-methyl-2-pentenyl group.

In the present specification, the "C2 to C6 haloalkenyl group" is a group in which at least one hydrogen atom in the C2 to C6 alkenyl group is replaced with a halogen atom. When two or more hydrogen atoms are replaced with halogen atoms, these halogen atoms may be the same or different. The number of halogen atoms as substituents is not particularly limited as long as their presence as substituents is attained. Examples of the "C2 to C6 haloalkenyl group" include a 2-fluorovinyl group, a 2,2-difluorovinyl group, a 2,2-dichlorovinyl group, a 3-fluoroallyl group, a 3,3-difluoroallyl group, a 3,3-dichloroallyl group, a 4,4-difluoro-3-butenyl group, a 5,5-difluoro-4-pentenyl group, and a 6,6-difluoro-5-hexenyl group.

In the present specification, the "C3 to C10 cycloalkenyl group" is a cyclic hydrocarbon group having 3 to 10 carbon atoms and having at least one double bond. Examples of the "C3 to C10 cycloalkenyl group" include a cyclopropenyl group, a cyclobutenyl group, a cyclopentenyl group, a cyclopentadienyl group, and a cyclohexenyl group.

In the present specification, the "C2 to C6 alkenyloxy group" is a group in which one of the two bonds of an oxygen atom is attached to the C2 to C6 alkenyl group. In the "C2 to C6 alkenyloxy group", the C2 to C6 alkenyl group is bonded via an oxygen atom. Examples of the "C2 to C6 alkenyloxy group" include a vinyloxy group, a 1-propenyloxy group, an allyloxy group, a 1-butenyloxy group, a 2-butenyloxy group, a 3-butenyloxy group, a 1-pentenyloxy group, a 2-pentenyloxy group, a 3-pentenyloxy group, a 4-pentenyloxy group, a 3-methyl-2-butenyloxy group, a 1-hexenyloxy group, a 2-hexenyloxy group, a 3-hexenyloxy group, a 4-hexenyloxy group, a 5-hexenyloxy group, a 3-methyl-2-pentenyloxy group, and a 4-methyl-3-pentenyloxy group.

In the present specification, the "C2 to C6 haloalkenyloxy group" is a group in which one of the two bonds of an oxygen atom is attached to the C2 to C6 haloalkenyl group. In the "C2 to C6 haloalkenyloxy group", the C2 to C6 haloalkenyl group is bonded via an oxygen atom. Examples of the "C2 to C6 haloalkenyloxy group" include a 2-fluorovinyloxy group, a 2,2-difluorovinyloxy group, a 2,2-dichlorovinyloxy group, a 3-fluoroallyloxy group, a 3,3-difluoroallyloxy group, a 3,3-dichloroallyloxy group, a 4,4-difluoro-3-butenyloxy group, a 5,5-difluoro-4-pentenyloxy group, and a 6,6-difluoro-5-hexenyloxy group.

In the present specification, the "C2 to C6 alkynyl group" is a group having at least one triple bond in a C2 to C6 alkyl group among the C1 to C6 alkyl group. Examples of the "C2 to C6 alkynyl group" include an ethynyl group, a 1-propynyl group, a propargyl group, a 1-butynyl group, a 2-butynyl group, a 3-butynyl group, a 1-pentynyl group, a 2-pentynyl group, a 3-pentynyl group, a 4-pentynyl group, a 1,1-dimethyl-2-propynyl group, a 1-hexynyl group, a 2-hexynyl group, a 3-hexynyl group, a 4-hexynyl group, and a 5-hexynyl group.

In the present specification, the "C2 to C6 haloalkynyl group" is a group in which at least one hydrogen atom in the C2 to C6 alkynyl group is replaced with a halogen atom. When two or more hydrogen atoms are replaced with halogen atoms, these halogen atoms may be the same or different. The number of halogen atoms as substituents is not particularly limited as long as their presence as substituents is attained. Examples of the "C2 to C6 haloalkynyl group" include a 2-fluoroethynyl group, a 2-chloroethynyl group, a 2-bromoethynyl group, a 2-iodoethynyl group, a 3,3-difluoro-1-propynyl group, a 3-chloro-3,3-difluoro-1-propynyl group, a 3-bromo-3,3-difluoro-1-propynyl group, a 3,3,3-trifluoro-1-propynyl group, a 4,4-difluoro-1-butynyl group, a 4,4-difluoro-2-butynyl group, a 4-chloro-4,4-difluoro-1-butynyl group, a 4-chloro-4,4-difluoro-2-butynyl group, a 4-bromo-4,4-difluoro-1-butynyl group, a 4-bromo-4,4-difluoro-2-butynyl group, a 4,4,4-trifluoro-1-butynyl group, a 4,4,4-trifluoro-2-butynyl group, a 5,5-difluoro-3-pentynyl group, a 5-chloro-5,5-difluoro-3-pentynyl group, a 5-bromo-5,5-difluoro-3-pentynyl group, a 5,5,5-trifluoro-3-pentynyl group, a 6,6-difluoro-4-hexynyl group, a 6-chloro-6,6-difluoro-4-hexynyl group, a 6-bromo-6,6-difluoro-4-hexynyl group, and a 6,6,6-trifluoro-4-hexynyl group.

In the present specification, the "C3 to C6 alkynyloxy group" is a group in which one of the two bonds of an oxygen atom is attached to the C2 to C6 alkynyl group. In the "C3 to C6 alkynyloxy group", the C2 to C6 alkynyl group is bonded via an oxygen atom. Examples of the "C3 to C6 alkynyloxy group" include a propargyloxy group, a 2-butynyloxy group, a 3-butynyloxy group, a 2-pentynyloxy group, a 3-pentynyloxy group, a 4-pentynyloxy group, a 1,1-dimethyl-2-propynyloxy group, a 2-hexynyloxy group, a 3-hexynyloxy group, a 4-hexynyloxy group, and a 5-hexynyloxy group.

In the present specification, the "C3 to C6 haloalkynyloxy group" is a group in which one of the two bonds of an oxygen atom is attached to the C2 to C6 haloalkynyl group. In the "C3 to C6 haloalkynyloxy group", the C3 to C6 haloalkynyl group is bonded via an oxygen atom. Examples of the "C3 to C6 haloalkynyloxy group" include a 1,1-difluoro-2-propynyloxy group, a 4,4-difluoro-2-butynyloxy group, a 4-chloro-4,4-difluoro-2-butynyloxy group, a 4-bromo-4,4-difluoro-2-butynyloxy group, a 4,4,4-trifluoro-2-butynyloxy group, a 5,5-difluoro-3-pentynyloxy group, a 5-chloro-5,5-difluoro-3-pentynyloxy group, a 5-bromo-5,5-difluoro-3-pentynyloxy group, a 5,5,5-trifluoro-3-pentynyloxy group, a 6,6-difluoro-4-hexynyloxy group, a 6-chloro-6,6-difluoro-4-hexynyloxy group, a 6-bromo-6,6-difluoro-4-hexynyloxy group, and a 6,6,6-trifluoro-4-hexynyloxy group.

In the present specification, the "C1 to C6 acyl group" is a formyl group or a carbonyl group bonded to a C1 to C5 alkyl group. Examples of the "C1 to C6 acyl group" include a formyl group, an acetyl group, a propionyl group, a butyryl group, an isobutyryl group, a pentanoyl group, a 2-methylbutanoyl group, a 3-methylbutanoyl group, a pivaloyl group, a hexanoyl group, a 2-methylpentanoyl group, a 3-methylpentanoyl group, a 4-methylpentanoyl group, a 2,2-dimethylbutanoyl group, a 2,3-dimethylbutanoyl group, a 2-ethylbutanoyl group, and a 3-ethylbutanoyl group.

In the present specification, the "aryl group" may be any monocyclic aryl group or condensed polycyclic aryl group. Examples of the "aryl group" include a phenyl group, a naphthyl group, a phenanthrenyl group, and an anthracenyl group.

In the present specification, the "heteroaryl group" may be any monocyclic heteroaryl group or condensed polycyclic heteroaryl group and is an aromatic ring group containing one or more heteroatoms (e.g., an oxygen atom, a nitrogen atom, or a sulfur atom) as ring-constituting atoms. Examples of the "heteroaryl group" include a pyrrolyl group, a furyl group, a thienyl group, a pyrazolyl group, an imidazolyl group, an oxazolyl group, an isoxazolyl group, an oxadiazolyl group, a thiazolyl group, an isothiazolyl group, a thiadiazolyl group, a tetrazolyl group, a pyridyl group, a pyridazinyl group, a pyrimidinyl group, a pyrazinyl group, a quinolyl group, an isoquinolyl group, a naphthyridinyl group, a quinoxalinyl group, a cinnolinyl group, a quinazolinyl group, a phthalazinyl group, an imidazopyridyl group, an imidazothiazolyl group, an imidazoxazolyl group, a benzothiazolyl group, a benzoxazolyl group, a benzimidazolyl group, an indolyl group, an isoindolyl group, an indazolyl group, a pyrrolopyridyl group, a thienopyridyl group, a furopyridyl group, a benzothiadiazolyl group, a benzoxadiazolyl group, a pyridopyrimidinyl group, a benzofuryl group, a benzothienyl group, a benzo[1,3]dioxole group, a thienofuryl group, a chromenyl group, a chromanyl group, a coumarinyl group, and a quinolonyl group.

In the present specification, the "C3 to C8 saturated or unsaturated heterocyclic ring" is a saturated or unsaturated ring constituted by 3 to 8 carbon atoms and at least one heteroatom (e.g., an oxygen atom, a nitrogen atom, or a sulfur atom). Examples of the "C3 to C8 saturated or unsaturated heterocyclic ring" include azetidine, oxetane, thietane, pyrrolidine, imidazolidine, triazolidine, tetrazolidine, pyrazolidine, tetrahydrofuran, tetrahydrothiophene, tetrahydrooxazole (oxazolidine), tetrahydroisoxazole (isoxazolidine), tetrahydrothiazole (thiazolidine), tetrahydroisothiazole (isothiazolidine), tetrahydrofurazane, tetrahydrooxadiazole (oxadiazolidine), tetrahydrothiadiazole (thiadiazolidine), dioxolane, dithiolane, piperidine, piperazine, perhydropyrimidine, perhydropyridazine, tetrahydropyran, tetrahydrothiopyran, tetrahydrooxazine, tetrahydrooxadiazine, tetrahydrothiazine, tetrahydrothiadiazine, morpholine, thiomorpholine, oxathiane, dioxane, dithiane, thiophene, furan, pyrrole, selenophene, thiazole, oxazole, imidazole, selenazole, thiadiazole, an oxadiazole ring, a triazole ring, a pyridine ring, a pyran ring, a pyrazine ring, a pyrimidine ring, a thienothiophene ring, and a benzothiophene ring.

In the present specification, the "C1 to C6 alkoxy group" is a group in which one of the two bonds of an oxygen atom is attached to the C1 to C6 alkyl group. In the "C1 to C6 alkoxy group", the C1 to C6 alkyl group is bonded via an oxygen atom. Examples of the "C1 to C6 alkoxy group" include a methoxy group, an ethoxy group, a propyloxy group, an isopropyloxy group, a butoxy group, an isobutoxy group, a sec-butoxy group, a tert-butoxy group, a pentyloxy group, an isopentyloxy group, a 1-methylbutoxy group, a 2-methylbutoxy group, a neopentyloxy group, a 1-ethylpropyloxy group, a 1,2-dimethylpropyloxy group, a hexyloxy group, a 1-methylpentyloxy group, a 2-methylpentyloxy group, a 3-methylpentyloxy group, a 4-methylpentyloxy group, a 1,1-dimethylbutoxy group, a 2,2-dimethylbutoxy group, a 3,3-dimethylbutoxy group, a 1,2-dimethylbutoxy group, a 1,3-dimethylbutoxy group, a 2,3-dimethylbutoxy group, a 2-ethylbutoxy group, a 1-isopropylpropyloxy group, a 1,1,2-trimethylpropyloxy group, and a 1,2,2-trimethylpropyloxy group.

In the present specification, the "C1 to C6 haloalkoxy group" represents a group in which a hydrogen atom in the C1 to C6 alkoxy group is optionally replaced with one or more halogen atom. When two or more hydrogen atoms are replaced with halogen atoms, these halogen atoms may be the same or different. The number of halogen atoms as substituents is not particularly limited as long as their presence as substituents is attained. Examples of the C1 to C6 haloalkoxy group include a difluoromethoxy group, a trifluoromethoxy group, a chlorodifluoromethoxy group, a bromodifluoromethoxy group, a 2-fluoroethoxy group, a 2,2-difluoroethoxy group, a 2,2,2-trifluoroethoxy group, a 1,1,2,2-tetrafluoroethoxy group, a pentafluoroethoxy group, a 2,2,2-trichloroethoxy group, a 3,3-difluoropropyloxy group, a 3,3,3-trifluoropropyloxy group, a heptafluoropropyloxy group, a heptafluoroisopropyloxy group, a 2,2,2-trifluoro-1-(trifluoromethyl)-ethoxy group, a nonafluorobutoxy group, a nonafluoro-sec-butoxy group, a 3,3,4,4,5,5,5-heptafluoropentyloxy group, a undecafluoropentyloxy group, and a tridecafluorohexyloxy group.

In the present specification, the "C2 to C6 alkenyloxy group" represents a group in which the C2 to C6 alkenyl group is bonded via an oxygen atom. A geometric isomer, if any, can be an E form, a Z form, or a mixture of E and Z forms at an arbitrary ratio, and it is not limited as long as it is within the specified carbon number range. Examples of the C2 to C6 alkenyloxy group include a vinyloxy group, a 1-propenyloxy group, an allyloxy group, a 1-butenyloxy group, a 2-butenyloxy group, a 3-butenyloxy group, a 1-pentenyloxy group, a 2-pentenyloxy group, a 3-pentenyloxy group, a 4-pentenyloxy group, a 3-methyl-2-butenyloxy group, a 1-hexenyloxy group, a 2-hexenyloxy group, a 3-hexenyloxy group, a 4-hexenyloxy group, a 5-hexenyloxy group, a 4-methyl-3-pentenyloxy group, and a 3-methyl-2-pentenyloxy group.

In the present specification, the "C2 to C6 haloalkenyloxy group" represents a group in which a hydrogen atom in the C2 to C6 alkenyloxy group is optionally substituted by one or more halogen atoms. When hydrogen atoms are replaced with two or more halogen atoms, these halogen atoms may be the same or different. The number of halogen atoms as substituents is not particularly limited as long as their presence as substituents is attained. Examples of the C2 to C6 haloalkenyloxy group include a 2-fluorovinyloxy group, a 2,2-difluorovinyloxy group, a 2,2-dichlorovinyloxy group, a 3-fluoroallyloxy group, a 3,3-difluoroallyloxy group, a 3,3-dichloroallyloxy group, a 4,4-difluoro-3-butenyloxy group, a 5,5-difluoro-4-pentenyloxy group, and a 6,6-difluoro-5-hexenyloxy group.

In the present specification, the "C3 to C6 alkynyloxy group" represents a group in which in the C2 to C6 alkenyl group, the C3 to C6 alkenyl group is bonded via an oxygen atom. Examples of the C3 to C6 alkynyloxy group include a propargyloxy group, a 2-butynyloxy group, a 3-butynyloxy group, a 2-pentynyloxy group, a 3-pentynyloxy group, a 4-pentynyloxy group, a 1,1-dimethyl-2-propynyloxy group, a 2-hexynyloxy group, a 3-hexynyloxy group, a 4-hexynyloxy group, and a 5-hexynyloxy group.

In the present specification, the "C3 to C6 haloalkynyloxy group" represents a group in which a hydrogen atom in the C3 to C6 alkynyloxy group is optionally substituted by one or more halogen atoms. When hydrogen atoms are replaced with two or more halogen atoms, these halogen atoms may be the same or different. The number of halogen atoms as substituents is not particularly limited as long as their presence as substituents is attained. Examples of the C3 to C6 haloalkynyloxy group include a 1,1-difluoro-2-propynyloxy group, a 4,4-difluoro-2-butynyloxy group, a 4-chloro-4,4-difluoro-2-butynyloxy group, a 4-bromo-4,4-difluoro-2-butynyloxy group, a 4,4,4-trifluoro-2-butynyloxy group, a 5,5-difluoro-3-pentynyloxy group, a 5-chloro-5,5-difluoro-3-pentynyloxy group, a 5-bromo-5,5-difluoro-3-pentynyloxy group, a 5,5,5-trifluoro-3-pentynyloxy group, a 6,6-difluoro-4-hexynyloxy group, a 6-chloro-6,6-difluoro-4-hexynyloxy group, a 6-bromo-6,6-difluoro-4-hexynyloxy group, and a 6,6,6-trifluoro-4-hexynyloxy group.

In the present specification, the "C3 to C8 cycloalkoxy group" is a group in which one of the two bonds of an oxygen atom is attached to the C3 to C8 cycloalkyl group. In the "C3 to C8 cycloalkoxy group", the C3 to C8 cycloalkyl group is bonded via an oxygen atom. Examples of the "C3 to C8 cycloalkoxy group" include a cyclopropyloxy group, a cyclobutoxy group, a cyclopentyloxy group, a cyclohexyloxy group, a cycloheptyloxy group, and a cyclooctyloxy group.

In the present specification, the "C5 to C10 unsaturated ring" is an unsaturated ring constituted by 5 to 10 carbon atoms that may contain a heteroatom (e.g., an oxygen atom, a nitrogen atom, or a sulfur atom). The "C5 to C10 unsaturated ring" may or may not have aromaticity. Examples of the "C5 to C10 unsaturated ring" include the aryl group, the heteroaryl group, the unsaturated heterocyclic ring, and the cycloalkenyl group.

### [Compound]

One embodiment of the present invention is a compound represented by the following formula (1) or a salt thereof.

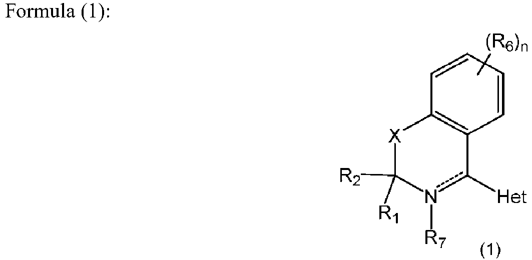

Hereinafter, the formula (1) will be described.

When R₁ and R₂ in the formula (1) are different, the compound represented by the formula (1) is either a R form or a S form, or is a mixture of R and S forms at an arbitrary ratio.

R₁ and R₂ in the formula (1) each independently represent a group selected from the group consisting of a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C1 to C6 acyl group optionally substituted by substituent A, an aryl group optionally substituted by substituent B, a heteroaryl group optionally substituted by substituent B, -C(=O)NRaRb (wherein Ra and Rb each independently represent a group selected from the group consisting of a hydrogen atom, a C1 to C6 alkyl group optionally substituted by substituent A, a C1 to C6 haloalkyl group, a C3 to C8 cycloalkyl group, a C1 to C6 acyl group, and -C(=O)ORc (wherein Rc represents a group selected from the group consisting of a hydrogen atom, a C1 to C6 alkyl group optionally substituted by substituent A, a C1 to C6 haloalkyl group, and a C3 to C8 cycloalkyl group), or Ra and Rb form, together with the nitrogen atom bonded thereto, an aziridinyl group, an azetidinyl group, a pyrrolidinyl group, a piperidinyl group, a homopiperidinyl group, or an azocanyl group), and -C(=O)ORc (wherein Rc is as defined above), or R₁ and R₂ together represent a C3 to C8 cycloalkyl group optionally substituted by substituent A or a C3 to C8 saturated or unsaturated heterocyclic ring optionally substituted by substituent A.

In particular, preferably, R₁ and R₂ each independently represent a group selected from the group consisting of a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, a C1 to C6 acyl group optionally substituted by substituent A, an aryl group optionally substituted by substituent B, -C(=O)ORc (wherein Rc represents a group selected from the group consisting of a hydrogen atom, a C1 to C6 alkyl group optionally substituted by substituent A, a C1 to C6 haloalkyl group, and a C3 to C8 cycloalkyl group), or Ra and Rb form, together with the nitrogen atom bonded thereto, an aziridinyl group, an azetidinyl group, a pyrrolidinyl group, a piperidinyl group, a homopiperidinyl group, or an azocanyl group , or R₁ and R₂ together represent a C3 to C8 cycloalkyl ring optionally substituted by substituent A.

The C1 to C6 alkyl group in the "C1 to C6 alkyl group optionally substituted by substituent A" represented by R₁ and R₂ in the formula (1) is as defined above and is preferably a methyl group, an ethyl group, a propyl group, an isopropyl group, a butyl group, an isobutyl group, a s-butyl group, a pentyl group, a isopentyl group, a 1-methylbutyl group, a 2-methylbutyl group, a neopentyl group, a 1-ethylpropyl group, a 1,2-dimethylpropyl group, or a hexyl group, more preferably a methyl group, an ethyl group, a propyl group, an isopropyl group, a butyl group, an isobutyl group, a pentyl group, or a isopentyl group. In the case of having substituent A, a hydrogen atom in the C1 to C6 alkyl group is arbitrarily replaced with the substituent A.

The "C1 to C6 haloalkyl group" represented by R₁ and R₂ in the formula (1) is as defined above and is preferably a difluoromethyl group, a trifluoromethyl group, a monochloromethyl group, a dichloromethyl group, a diiodomethyl group, a chlorodifluoromethyl group, a 1,1-difluoroethyl group, a 2,2-difluoroethyl group, a 2,2,2-trifluoroethyl group, a 2,2,2-trichloroethyl group, a 3,3-difluoropropyl group, or a 3,3,3-trifluoropropyl group.

The C3 to C8 cycloalkyl group in the "C3 to C8 cycloalkyl group optionally substituted by substituent A" represented by R₁ and R₂ in the formula (1) is as defined above and is preferably a cyclopropyl group, a cyclobutyl group, a cyclopentyl group, or a cyclohexyl group, more preferably a cyclopropyl group or a cyclobutyl group. In the case of having substituent A, a hydrogen atom in the C3 to C8 cycloalkyl group is arbitrarily replaced with the substituent A.

The C2 to C6 alkenyl group in the "C2 to C6 alkenyl group optionally substituted by substituent A" represented by R₁ and R₂ in the formula (1) is as defined above and is preferably a vinyl group, a 1-propenyl group, an allyl group, a 1-butenyl group, a 2-butenyl group, a 3-butenyl group, a 1-pentenyl group, a 2-pentenyl group, a 3-pentenyl group, a 4-pentenyl group, a 3-methyl-2-butenyl group, or a 4-methyl-3-pentenyl group. In the case of having substituent A, a hydrogen atom in the C2 to C6 alkenyl group is arbitrarily replaced with the substituent A.

The "C2 to C6 haloalkenyl group" represented by R₁ and R₂ in the formula (1) is as defined above and is preferably a 2-fluorovinyl group, a 2,2-difluorovinyl group, a 3-fluoroallyl group, a 3,3-difluoroallyl group, or a 4,4-difluoro-3-butenyl group.

The C2 to C6 alkynyl group in the "C2 to C6 alkynyl group optionally substituted by substituent A" represented by R₁ and R₂ in the formula (1) is as defined above and is preferably an ethynyl group, a 1-propynyl group, a propargyl group, a 1-butynyl group, a 2-butynyl group, or a 3-butynyl group. In the case of having substituent A, a hydrogen atom in the C2 to C6 alkynyl group is arbitrarily replaced with the substituent A.

The "C2 to C6 haloalkynyl group" represented by R₁ and R₂ in the formula (1) is as defined above and is preferably a 2-fluoroethynyl group, a 2-chloroethynyl group, a 2-bromoethynyl group, a 2-iodoethynyl group, a 3,3-difluoro-1-propynyl group, a 3-chloro-3,3-difluoro-1-propynyl group, a 3-bromo-3,3-difluoro-1-propynyl group, a 3,3,3-trifluoro-1-propynyl group, a 4,4-difluoro-1-butynyl group, a 4,4-difluoro-2-butynyl group, a 4-chloro-4,4-difluoro-1-butynyl group, a 4-chloro-4,4-difluoro-2-butynyl group, a 4-bromo-4,4-difluoro-1-butynyl group, a 4-bromo-4,4-difluoro-2-butynyl group, a 4,4,4-trifluoro-1-butynyl group, or a 4,4,4-trifluoro-2-butynyl group, more preferably a 2-fluoroethynyl group, a 3,3-difluoro-1-propynyl group, a 3,3,3-trifluoro-1-propynyl group, a 4,4-difluoro-1-butynyl group, a 4,4-difluoro-2-butynyl group, a 4,4,4-trifluoro-1-butynyl group, or a 4,4,4-trifluoro-2-butynyl group.

The C1 to C6 acyl group in the "C1 to C6 acyl group optionally substituted by substituent A" represented by R₁ and R₂ in the formula (1) is as defined above and is preferably a formyl group, an acetyl group, a propionyl group, a butyryl group, or an isobutyryl group, more preferably an acetyl group or a propionyl group. In the case of having substituent A, a hydrogen atom in the C1 to C6 acyl group is arbitrarily replaced with the substituent A.

The aryl group in the "aryl group optionally substituted by substituent B" represented by R₁ and R₂ in the formula (1) is as defined above and is preferably a phenyl group. In the case of having substituent B, a hydrogen atom in the aryl group is arbitrarily replaced with the substituent B.

The heteroaryl group in the "heteroaryl group optionally substituted by substituent B" represented by R₁ and R₂ in the formula (1) is as defined above and is preferably a pyridyl group, a pyrazinyl group, a thienyl group, a thiazolyl group, an isothiazolyl group, a furyl group, an imidazolyl group, a pyrazolyl group, an oxazolyl group, an isoxazolyl group, a triazolyl group, an oxadiazolyl group, or a tetrazolyl group, and is more preferably a thienyl group. In the case of having substituent B, a hydrogen atom in the heteroaryl group is arbitrarily replaced with the substituent B.

Ra and Rb of "-C(=O)NRaRb" in R₁ and R₂ of the formula (1) is as defined above.

"-C(=O)NRaRb" is preferably an aminocarbonyl group, a methylaminocarbonyl group, an ethylaminocarbonyl group, a (methoxymethyl)aminocarbonyl group, a (2-methoxyethyl)aminocarbonyl group, a (cyanomethyl)aminocarbonyl group, a (2-cyanoethyl)aminocarbonyl group, a dimethylaminocarbonyl group, an ethylmethylaminocarbonyl group, a diethylaminocarbonyl group, a (methoxymethyl)methylaminocarbonyl group, a (2-methoxyethyl)methylaminocarbonyl group, a (cyanomethyl)methylaminocarbonyl group, a (2-cyanoethyl)methylaminocarbonyl group, a 2,2-difluoroethylaminocarbonyl group, a 2,2,2-trifluoroethylaminocarbonyl group, a cyclopropylaminocarbonyl group, a (cyclopropyl)methylaminocarbonyl group, a pyrrolidinyl group, or a piperidinyl group, more preferably an aminocarbonyl group, a methylaminocarbonyl group, a dimethylaminocarbonyl group, an ethylmethylaminocarbonyl group, or a diethylaminocarbonyl group, more preferably an aminocarbonyl group, a methylaminocarbonyl group, or a dimethylaminocarbonyl group.

Rc in "-C(=O)ORc" in R₁ and R₂ of the formula (1) is as defined above.

"-C(=O)ORc" is preferably a carboxy group, a methoxycarbonyl group, an ethoxycarbonyl group, a propyloxycarbonyl group, an isopropyloxycarbonyl group, a butoxycarbonyl group, or isobutoxycarbonyl group, a difluoromethoxycarbonyl group, a trifluoromethoxycarbonyl group, a 2,2-difluoroethoxycarbonyl group, a 2,2,2-trifluoroethoxycarbonyl group, a 3,3-difluoropropyloxycarbonyl group, a 3,3,3-trifluoropropyloxy group, a cyclopropyloxycarbonyl group, a cyclobutoxycarbonyl group, a cyclopentyloxycarbonyl group, or a cyclohexyloxycarbonyl group, more preferably a methoxycarbonyl group, an ethoxycarbonyl group, a difluoromethoxycarbonyl group, a trifluoromethoxycarbonyl group, a 2,2-difluoroethoxycarbonyl group, a 2,2,2-trifluoroethoxycarbonyl group, or a cyclopropyloxycarbonyl group, further preferably a methoxycarbonyl group or a ethoxycarbonyl group. In the case of having substituent A, a hydrogen atom in the C1 to C6 alkyl group is arbitrarily replaced with the substituent A.

The C3 to C8 cycloalkyl group in the "C3 to C8 cycloalkyl group optionally substituted by substituent A" formed by R₁ and R₂ together in the formula (1) is as defined above and is preferably a cyclopropyl group, a cyclobutyl group, a cyclopentyl group, or a cyclohexyl group, more preferably a cyclopropyl group or a cyclobutyl group. In the case of having substituent A, a hydrogen atom in the C3 to C8 cycloalkyl group is arbitrarily replaced with the substituent A.

The C3 to C8 saturated or unsaturated heterocyclic ring in the "C3 to C8 saturated or unsaturated heterocyclic ring optionally substituted by substituent A" formed by R₁ and R₂ together in the formula (1) is as defined above and is preferably a 3-oxetanyl group, a 3-thietanyl group, a 3-tetrahydrofuranyl group, a 3-tetrahydrothiophenyl group, a 3-tetrahydropyranyl group, a 4-tetrahydropyranyl group, a 3-tetrahydrothiopyranyl group, or a 4-tetrahydrothiopyranyl group. In the case of having substituent A, a hydrogen atom in the C3 to C8 saturated or unsaturated heterocyclic ring is arbitrarily replaced with the substituent A.

Hereinafter, Het in the formula (1) will be described in detail.

Het in the formula (1) represents a partial structure represented by the formula (2). The wavy line in the formula (2) represents a bond site in the formula (1).

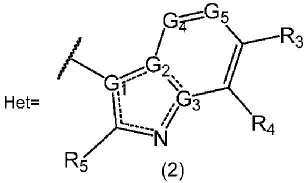

R₃ and R₄ in the formula (2) each independently represent a hydrogen atom, a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, -NRaRb (wherein Ra and Rb each independently represent a hydrogen atom, a C1 to C6 alkyl group optionally substituted by substituent A, a C1 to C6 haloalkyl group, a C3 to C8 cycloalkyl group, a C1 to C6 acyl group, or -C(=O)ORc (wherein Rc represents a hydrogen atom, a C1 to C6 alkyl group optionally substituted by substituent B, a C1 to C6 haloalkyl group, or a C3 to C8 cycloalkyl group), or Ra and Rb represent to form, together with the nitrogen atom bonded thereto, an aziridinyl group, an azetidinyl group, a pyrrolidinyl group, a piperidinyl group, a homopiperidinyl group, or an azocanyl group), -C(=O)NRaRb (wherein Ra and Rb are as defined above), -C(=O)ORc (wherein Rc is as defined above), -OC(=O)Rc (wherein Rc is as defined above), or - L-Rd (wherein Rd represents a C1 to C6 alkyl group or a C1 to C6 haloalkyl group, and L represents S, S(=O), or SO₂), or R₃ and R₄ together represent a C5 to C10 unsaturated ring optionally substituted by substituent A.

In particular, preferably, R₃ and R₄ each independently represent a group selected from the group consisting of a hydrogen atom, a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, and a C3 to C6 haloalkynyloxy group, or R₃ and R₄ together represent a C5 to C10 unsaturated ring optionally substituted by substituent A. Particularly preferably, R₃ and R₄ each independently represent a group selected from the group consisting of a hydrogen atom, a halogen atom, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, and a C2 to C6 alkenyl group optionally substituted by substituent A, or R₃ and R₄ together represent a C5 to C10 unsaturated ring optionally substituted by substituent A.

R₃ and R₄ in the formula (2) include a hydrogen atom, a hydroxy group, a cyano group, or a nitro group.

The halogen atom represented by R₃ and R₄ in the formula (2) is as defined above and is preferably a fluorine atom, a chlorine atom, a bromine atom, or an iodine atom, more preferably a fluorine atom a chlorine atom, or a bromine atom.

The C1 to C6 alkyl group in the "C1 to C6 alkyl group optionally substituted by substituent A" represented by R₃ and R₄ in the formula (2) is as defined above and is preferably a methyl group, an ethyl group, a propyl group, more preferably a methyl group or an ethyl group, more preferably a methyl group. In the case of having substituent A, a hydrogen atom in the C1 to C6 alkyl group is arbitrarily replaced with the substituent A.

The "C1 to C6 haloalkyl group" represented by R₃ and R₄ in the formula (2) is as defined above and is preferably a difluoromethyl group, a trifluoromethyl group, a 1,1-difluoroethyl group, a 2,2-difluoroethyl group, a 2,2,2-trifluoroethyl group, a 2,2,2-trichloroethyl group, a 3,3-difluoropropyl group, or a 3,3,3-trifluoropropyl group, more preferably a difluoromethyl group, a trifluoromethyl group, a 1,1-difluoroethyl group, a 2,2-difluoroethyl group, or a 2,2,2-trifluoroethyl group, further preferably a difluoromethyl group or a trifluoromethyl group.

The C3 to C8 cycloalkyl group in the "C3 to C8 cycloalkyl group optionally substituted by substituent A" represented by R₃ and R₄ in the formula (2) is as defined above and is preferably a cyclopropyl group, a cyclobutyl group, a cyclopentyl group, or a cyclohexyl group, more preferably a cyclopropyl group or a cyclobutyl group, further preferably a cyclopropyl group. In the case of having substituent A, a hydrogen atom in the C3 to C8 cycloalkyl group is arbitrarily replaced with the substituent A.

The C1 to C6 alkoxy group in the "C1 to C6 alkoxy group optionally substituted by substituent A" represented by R₃ and R₄ in the formula (2) is as defined above and is preferably a methoxy group, an ethoxy group, a propyloxy group, an isopropyloxy group, a butoxy group, or an isobutoxy group, more preferably a methoxy group or an ethoxy group, further preferably a methoxy group. In the case of having substituent A, a hydrogen atom in the C1 to C6 alkoxy group is arbitrarily replaced with the substituent A.

The "C1 to C6 haloalkoxy group" represented by R₃ and R₄ in the formula (2) is as defined above and is preferably a difluoromethoxy group, a trifluoromethoxy group, a 2,2-difluoroethoxy group, a 2,2,2-trifluoroethoxy group, a 3,3-difluoropropyloxy group, or a 3,3,3-trifluoropropyloxy group, more preferably a difluoromethoxy group, a trifluoromethoxy group, a 2,2-difluoroethoxy group, or a 2,2,2-trifluoroethoxy group, further preferably a difluoromethoxy group or a trifluoromethoxy group.

The C3 to C8 cycloalkoxy group in the "C3 to C8 cycloalkoxy group optionally substituted by substituent A" represented by R₃ and R₄ in the formula (2) is as defined above and is preferably a cyclopropyloxy group, a cyclobutoxy group, a cyclopentyloxy group, or a cyclohexyloxy group, more preferably a cyclopropyloxy group or a cyclobutoxy group. In the case of having substituent A, a hydrogen atom in the C3 to C8 cycloalkoxy group is arbitrarily replaced with the substituent A.

The C2 to C6 alkenyl group in the "C2 to C6 alkenyl group optionally substituted by substituent A" represented by R₃ and R₄ in the formula (2) is as defined above and is preferably a vinyl group, a 1-propenyl group, an allyl group, a 1-butenyl group, a 2-butenyl group, or a 3-butenyl group, more preferably a vinyl group, a 1-propenyl group, or an allyl group. In the case of having substituent A, a hydrogen atom in the C2 to C6 alkenyl group is arbitrarily replaced with the substituent A.

The "C2 to C6 haloalkenyl group" represented by R₃ and R₄ in the formula (2) is as defined above and is preferably a 2-fluorovinyl group, a 2,2-difluorovinyl group, a 3-fluoroallyl group, a 3,3-difluoroallyl group, or a 4,4-difluoro-3-butenyl group.

The C2 to C6 alkynyl group in the "C2 to C6 alkynyl group optionally substituted by substituent A" represented by R₃ and R₄ in the formula (2) is as defined above and is preferably an ethynyl group, a 1-propynyl group, a propargyl group, a 1-butynyl group, a 2-butynyl group, or a 3-butynyl group, more preferably an ethynyl group. In the case of having substituent A, a hydrogen atom in the C2 to C6 alkynyl group is arbitrarily replaced with the substituent A.

The "C2 to C6 haloalkynyl group" represented by R₃ and R₄ in the formula (2) is as defined above and is preferably a 2-fluoroethynyl group, a 2-chloroethynyl group, a 2-bromoethynyl group, or a 2-iodoethynyl group, more preferably a 2-fluoroethynyl group.

The C2 to C6 alkenyloxy group in the "C2 to C6 alkenyloxy group optionally substituted by substituent A" represented by R₃ and R₄ in the formula (2) is as defined above and is preferably a vinyloxy group, a 1-propenyloxy group, or an allyloxy group, more preferably an allyloxy group. In the case of having substituent A, a hydrogen atom in the C2 to C6 alkenyloxy group is arbitrarily replaced with the substituent A.

The "C2 to C6 haloalkenyloxy group" represented by R₃ and R₄ in the formula (2) is as defined above and is preferably a 2-fluorovinyloxy group, a 2,2-difluorovinyloxy group, a 3-fluoroallyloxy group, or a 3,3-difluoroallyloxy group, more preferably a 2-fluorovinyloxy group or a 2,2-difluorovinyloxy group.

The C3 to C6 alkynyloxy group in the "C3 to C6 alkynyloxy group optionally substituted by substituent A" represented by R₃ and R₄ in the formula (2) is as defined above and is preferably a propargyloxy group, a 2-butynyloxy group, or a 3-butynyloxy group, more preferably a propargyloxy group. In the case of having substituent A, a hydrogen atom in the C3 to C6 alkynyloxy group is arbitrarily replaced with the substituent A.

The "C3 to C6 haloalkynyloxy group" represented by R₃ and R₄ in the formula (2) is as defined above and is preferably a 4,4-difluoro-2-butynyloxy group, a 4-chloro-4,4-difluoro-2-butynyloxy group, a 4-bromo-4,4-difluoro-2-butynyloxy group, or a 4,4,4-trifluoro-2-butynyloxy group, more preferably a 4,4-difluoro-2-butynyloxy group or a 4,4,4-trifluoro-2-butynyloxy group.

Ra and Rb in "RaRbN-" represented by R₃ and R₄ in the formula (2) are as defined above.

"RaRbN-" is preferably an amino group, a methylamino group, an ethylamino group, a (methoxymethyl)amino group, a (2-methoxyethyl)amino group, a (cyanomethyl)amino group, a (2-cyanoethyl)amino group, a dimethylamino group, an ethylmethylamino group, a diethylamino group, a (methoxymethyl)methylamino group, a (2-methoxyethyl)methylamino group, a (cyanomethyl)methylamino group, a (2-cyanoethyl)methylamino group, a 2,2-difluoroethylamino group, a 2,2,2-trifluoroethylamino group, a cyclopropylamino group, a (cyclopropyl)methylamino group, a pyrrolidinyl group, or a piperidinyl group, more preferably an amino group, a methylamino group, a dimethylamino group, an ethylmethylamino group, or a diethylamino group, further preferably an amino group, a methylamino group, or a dimethylamino group.

Ra and Rb in "-C(=O)NRaRb" represented by R₃ and R₄ in the formula (2) are as defined above.

"-C(=O)NRaRb" is preferably an aminocarbonyl group, a methylaminocarbonyl group, an ethylaminocarbonyl group, a (methoxymethyl)aminocarbonyl group, a (2-methoxyethyl)aminocarbonyl group, a (cyanomethyl)aminocarbonyl group, a (2-cyanoethyl)aminocarbonyl group, a dimethylaminocarbonyl group, an ethylmethylaminocarbonyl group, a diethylaminocarbonyl group, a (methoxymethyl)methylaminocarbonyl group, a (2-methoxyethyl)methylaminocarbonyl group, a (cyanomethyl)methylaminocarbonyl group, a (2-cyanoethyl)methylaminocarbonyl group, a 2,2-difluoroethylaminocarbonyl group, a 2,2,2-trifluoroethylaminocarbonyl group, a cyclopropylaminocarbonyl group, a (cyclopropyl)methylaminocarbonyl group, a pyrrolidinyl group, or a piperidinyl group, more preferably an aminocarbonyl group, a methylaminocarbonyl group, a dimethylaminocarbonyl group, an ethylmethylaminocarbonyl group, or a diethylaminocarbonyl group, more preferably an aminocarbonyl group, a methylaminocarbonyl group, or a dimethylaminocarbonyl group.

Rc in "-C(=O)ORc" represented by R₃ and R₄ in the formula (2) is as defined above.

"-C(=O)ORc" is preferably a carboxy group, a methoxycarbonyl group, an ethoxycarbonyl group, a propyloxycarbonyl group, an isopropyloxycarbonyl group, a butoxycarbonyl group, or isobutoxycarbonyl group, a difluoromethoxycarbonyl group, a trifluoromethoxycarbonyl group, a 2,2-difluoroethoxycarbonyl group, a 2,2,2-trifluoroethoxycarbonyl group, a 3,3-difluoropropyloxycarbonyl group, a 3,3,3-trifluoropropyloxy group, a cyclopropyloxycarbonyl group, a cyclobutoxycarbonyl group, a cyclopentyloxycarbonyl group, or a cyclohexyloxycarbonyl group, more preferably a methoxycarbonyl group, an ethoxycarbonyl group, a difluoromethoxycarbonyl group, a trifluoromethoxycarbonyl group, a 2,2-difluoroethoxycarbonyl group, a 2,2,2-trifluoroethoxycarbonyl group, or a cyclopropyloxycarbonyl group, further preferably a methoxycarbonyl group. In the case of having substituent A, a hydrogen atom in the C1 to C6 alkyl group is arbitrarily replaced with the substituent A.

Rc in "-OC(=O)Rc" represented by R₃ and R₄ in the formula (2) is as defined above.

"-OC(=O)Rc" is preferably a formyloxy group, an acetyloxy group, a methoxyacetyloxy group, a cyanoacetyloxy group, a propionyloxy group, a difluoroacetyloxy group, a trifluoroacetyloxy group, a cyclopropanecarbonyloxy group, a methoxycarbonyloxy group, an ethoxycarbonyloxy group, a 2,2-difluoroethoxycarbonyloxy group, a 2,2,2-trifluoroethoxycarbonyloxy group, a 3,3,3-trifluoropropyloxycarbonyloxy group, a cyclopropyloxycarbonyloxy group, an aminocarbonyloxy group, a methylaminocarbonyloxy group, an ethylaminocarbonyloxy group, a (methoxymethyl)aminocarbonyloxy group, a (2-methoxyethyl)aminocarbonyloxy group, a (cyanomethyl)aminocarbonyloxy group, a (2-cyanoethyl)aminocarbonyloxy group, a dimethylaminocarbonyloxy group, an ethylmethylaminocarbonyloxy group, a diethylaminocarbonyloxy group, a (methoxymethyl)methylaminocarbonyloxy group, a (2-methoxyethyl)methylaminocarbonyloxy group, a (cyanomethyl)methylaminocarbonyloxy group, a (2-cyanoethyl)methylaminocarbonyloxy group, a 2,2-difluoroethylaminocarbonyloxy group, a 2,2,2-trifluoroethylaminocarbonyloxy group, a cyclopropylaminocarbonyloxy group, a (cyclopropyl)methylaminocarbonyloxy group, a pyrrolidinylcarbonyloxy group, or a piperidinylcarbonyloxy group, more preferably a formyloxy group, an acetyloxy group, or a trifluoroacetyloxy group.

Rd and L in "Rd-L-" represented by R₃ and R₄ in the formula (2) are as defined above. "Rd-L-" is preferably a methylthio group, a methanesulfinyl group, a methanesulfonyl group, a trifluoromethylthio group, a trifluoromethanesulfinyl group, or a trifluoromethanesulfonyl group, more preferably a methylthio group, a methanesulfinyl group, or a methanesulfonyl group.

The C5 to C10 unsaturated ring in the "C5 to C10 unsaturated ring optionally substituted by substituent A" formed by R₃ and R₄ together in the formula (2) is as defined above and is preferably a cyclopentenyl group, a cyclohexenyl group, a cycloheptenyl group, or a phenyl group.

R₅ in the formula (2) represents a hydrogen atom, a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, -NRaRb (wherein Ra and Rb are as defined above), -C(=O)NRaRb (wherein Ra and Rb are as defined above), -C(=O)ORc (wherein Rc is as defined above), -OC(=O)Rc (wherein Rc is as defined above), or -L-Rd (wherein Rd and L are as defined above).

In particular, preferably, R₅ is a hydrogen atom, a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, or a C3 to C6 haloalkynyloxy group. Particularly preferably, R₅ is a hydrogen atom, a halogen atom, or a C1 to C6 alkyl group optionally substituted by substituent A.

R₅ in the formula (2) includes a hydrogen atom, a hydroxy group, a cyano group, or a nitro group.

The "halogen atom" represented by R₅ in the formula (2) is as defined above and is preferably a fluorine atom, a chlorine atom, a bromine atom, or an iodine atom, more preferably a fluorine atom a chlorine atom, or a bromine atom.

The C1 to C6 alkyl group in the "C1 to C6 alkyl group optionally substituted by substituent A" represented by R₅ in the formula (2) is as defined above and is preferably a methyl group, an ethyl group, a propyl group, more preferably a methyl group or an ethyl group, further preferably a methyl group. In the case of having substituent A, a hydrogen atom in the C1 to C6 alkyl group is arbitrarily replaced with the substituent A.

The "C1 to C6 haloalkyl group" represented by R₅ in the formula (2) is as defined above and is preferably a difluoromethyl group, a trifluoromethyl group, a 1,1-difluoroethyl group, a 2,2-difluoroethyl group, a 2,2,2-trifluoroethyl group, a 2,2,2-trichloroethyl group, a 3,3-difluoropropyl group, or a 3,3,3-trifluoropropyl group, more preferably a difluoromethyl group, a trifluoromethyl group, a 1,1-difluoroethyl group, a 2,2-difluoroethyl group, or a 2,2,2-trifluoroethyl group, further preferably a difluoromethyl group or a trifluoromethyl group.

The C3 to C8 cycloalkyl group in the "C3 to C8 cycloalkyl group optionally substituted by substituent A" represented by R₅ in the formula (2) is as defined above and is preferably a cyclopropyl group, a cyclobutyl group, a cyclopentyl group, or a cyclohexyl group, more preferably a cyclopropyl group or a cyclobutyl group, further preferably a cyclopropyl group. In the case of having substituent A, a hydrogen atom in the C3 to C8 cycloalkyl group is arbitrarily replaced with the substituent A.

The C1 to C6 alkoxy group in the "C1 to C6 alkoxy group optionally substituted by substituent A" represented by R₅ in the formula (2) is as defined above and is preferably a methoxy group, an ethoxy group, a propyloxy group, an isopropyloxy group, a butoxy group, or an isobutoxy group, more preferably a methoxy group or an ethoxy group, further preferably a methoxy group. In the case of having substituent A, a hydrogen atom in the C1 to C6 alkoxy group is arbitrarily replaced with the substituent A.

The "C1 to C6 haloalkoxy group" represented by R₅ in the formula (2) is as defined above and is preferably a difluoromethoxy group, a trifluoromethoxy group, a 2,2-difluoroethoxy group, a 2,2,2-trifluoroethoxy group, a 3,3-difluoropropyloxy group, or a 3,3,3-trifluoropropyloxy group, more preferably a difluoromethoxy group, a trifluoromethoxy group, a 2,2-difluoroethoxy group, or a 2,2,2-trifluoroethoxy group, further preferably a difluoromethoxy group or a trifluoromethoxy group.

The C3 to C8 cycloalkoxy group in the "C3 to C8 cycloalkoxy group optionally substituted by substituent A" represented by R₅ in the formula (2) is as defined above and is preferably a cyclopropyloxy group, a cyclobutoxy group, a cyclopentyloxy group, or a cyclohexyloxy group, more preferably a cyclopropyloxy group or a cyclobutoxy group. In the case of having substituent A, a hydrogen atom in the C3 to C8 cycloalkoxy group is arbitrarily replaced with the substituent A.

The C2 to C6 alkenyl group in the "C2 to C6 alkenyl group optionally substituted by substituent A" represented by R₅ in the formula (2) is as defined above and is preferably a vinyl group, a 1-propenyl group, an allyl group, a 1-butenyl group, a 2-butenyl group, or a 3-butenyl group, more preferably a vinyl group, a 1-propenyl group, or an allyl group. In the case of having substituent A, a hydrogen atom in the C2 to C6 alkenyl group is arbitrarily replaced with the substituent A.

The "C2 to C6 haloalkenyl group" represented by R₅ in the formula (2) is as defined above and is preferably a 2-fluorovinyl group, a 2,2-difluorovinyl group, a 3-fluoroallyl group, a 3,3-difluoroallyl group, or a 4,4-difluoro-3-butenyl group.

The C2 to C6 alkynyl group in the "C2 to C6 alkynyl group optionally substituted by substituent A" represented by R₅ in the formula (2) is as defined above and is preferably an ethynyl group, a 1-propynyl group, a propargyl group, a 1-butynyl group, a 2-butynyl group, or a 3-butynyl group, more preferably an ethynyl group. In the case of having substituent A, a hydrogen atom in the C2 to C6 alkynyl group is arbitrarily replaced with the substituent A.

The "C2 to C6 haloalkynyl group" represented by R₅ in the formula (2) is as defined above and is preferably a 2-fluoroethynyl group, a 2-chloroethynyl group, a 2-bromoethynyl group, or a 2-iodoethynyl group, more preferably a 2-fluoroethynyl group.

The C2 to C6 alkenyloxy group in the "C2 to C6 alkenyloxy group optionally substituted by substituent A" represented by R₅ in the formula (2) is as defined above and is preferably a vinyloxy group, a 1-propenyloxy group, or an allyloxy group, more preferably an allyloxy group. In the case of having substituent A, a hydrogen atom in the C2 to C6 alkenyloxy group is arbitrarily replaced with the substituent A.

The "C2 to C6 haloalkenyloxy group" represented by R₅ in the formula (2) is as defined above and is preferably a 2-fluorovinyloxy group, a 2,2-difluorovinyloxy group, a 3-fluoroallyloxy group, or a 3,3-difluoroallyloxy group, more preferably a 2-fluorovinyloxy group or a 2,2-difluorovinyloxy group.

The C3 to C6 alkynyloxy group in the "C3 to C6 alkynyloxy group optionally substituted by substituent A" represented by R₅ in the formula (2) is as defined above and is preferably a propargyloxy group, a 2-butynyloxy group, or a 3-butynyloxy group, more preferably a propargyloxy group. In the case of having substituent A, a hydrogen atom in the C3 to C6 alkynyloxy group is arbitrarily replaced with the substituent A.

The "C3 to C6 haloalkynyloxy group" represented by R₅ in the formula (2) is as defined above and is preferably a 4,4-difluoro-2-butynyloxy group, a 4-chloro-4,4-difluoro-2-butynyloxy group, a 4-bromo-4,4-difluoro-2-butynyloxy group, or a 4,4,4-trifluoro-2-butynyloxy group, more preferably a 4,4-difluoro-2-butynyloxy group or a 4,4,4-trifluoro-2-butynyloxy group.

Ra and Rb in "RaRbN-" represented by R₅ in the formula (2) are as defined above.

"RaRbN-" is preferably an amino group, a methylamino group, an ethylamino group, a (methoxymethyl)amino group, a (2-methoxyethyl)amino group, a (cyanomethyl)amino group, a (2-cyanoethyl)amino group, a dimethylamino group, an ethylmethylamino group, a diethylamino group, a (methoxymethyl)methylamino group, a (2-methoxyethyl)methylamino group, a (cyanomethyl)methylamino group, a (2-cyanoethyl)methylamino group, a 2,2-difluoroethylamino group, a 2,2,2-trifluoroethylamino group, a cyclopropylamino group, a (cyclopropyl)methylamino group, a pyrrolidinyl group, or a piperidinyl group, more preferably an amino group, a methylamino group, a dimethylamino group, an ethylmethylamino group, or a diethylamino group, further preferably an amino group, a methylamino group, or a dimethylamino group.

Ra and Rb in "-C(=O)NRaRb" represented by R₅ in the formula (2) are as defined above.

"-C(=O)NRaRb" is preferably an aminocarbonyl group, a methylaminocarbonyl group, an ethylaminocarbonyl group, a (methoxymethyl)aminocarbonyl group, a (2-methoxyethyl)aminocarbonyl group, a (cyanomethyl)aminocarbonyl group, a (2-cyanoethyl)aminocarbonyl group, a dimethylaminocarbonyl group, an ethylmethylaminocarbonyl group, a diethylaminocarbonyl group, a (methoxymethyl)methylaminocarbonyl group, a (2-methoxyethyl)methylaminocarbonyl group, a (cyanomethyl)methylaminocarbonyl group, a (2-cyanoethyl)methylaminocarbonyl group, a 2,2-difluoroethylaminocarbonyl group, a 2,2,2-trifluoroethylaminocarbonyl group, a cyclopropylaminocarbonyl group, a (cyclopropyl)methylaminocarbonyl group, a pyrrolidinyl group, or a piperidinyl group, more preferably an aminocarbonyl group, a methylaminocarbonyl group, a dimethylaminocarbonyl group, an ethylmethylaminocarbonyl group, or a diethylaminocarbonyl group, further preferably an aminocarbonyl group, a methylaminocarbonyl group, or a dimethylaminocarbonyl group.

Rc in "-C(=O)ORc" represented by R₅ in the formula (2) is as defined above.

"-C(=O)ORc" is preferably a carboxy group, a methoxycarbonyl group, an ethoxycarbonyl group, a propyloxycarbonyl group, an isopropyloxycarbonyl group, a butoxycarbonyl group, or isobutoxycarbonyl group, a difluoromethoxycarbonyl group, a trifluoromethoxycarbonyl group, a 2,2-difluoroethoxycarbonyl group, a 2,2,2-trifluoroethoxycarbonyl group, a 3,3-difluoropropyloxycarbonyl group, a 3,3,3-trifluoropropyloxy group, a cyclopropyloxycarbonyl group, a cyclobutoxycarbonyl group, a cyclopentyloxycarbonyl group, or a cyclohexyloxycarbonyl group, more preferably a methoxycarbonyl group, an ethoxycarbonyl group, a difluoromethoxycarbonyl group, a trifluoromethoxycarbonyl group, a 2,2-difluoroethoxycarbonyl group, a 2,2,2-trifluoroethoxycarbonyl group, or a cyclopropyloxycarbonyl group, further preferably a methoxycarbonyl group. In the case of having substituent A, a hydrogen atom in the C1 to C6 alkyl group is arbitrarily replaced with the substituent A.

Rc in "-OC(=O)Rc" represented by R₅ in the formula (2) is as defined above.

"-OC(=O)Rc" is preferably a formyloxy group, an acetyloxy group, a methoxyacetyloxy group, a cyanoacetyloxy group, a propionyloxy group, a difluoroacetyloxy group, a trifluoroacetyloxy group, a cyclopropanecarbonyloxy group, a methoxycarbonyloxy group, an ethoxycarbonyloxy group, a 2,2-difluoroethoxycarbonyloxy group, a 2,2,2-trifluoroethoxycarbonyloxy group, a 3,3,3-trifluoropropyloxycarbonyloxy group, a cyclopropyloxycarbonyloxy group, an aminocarbonyloxy group, a methylaminocarbonyloxy group, an ethylaminocarbonyloxy group, a (methoxymethyl)aminocarbonyloxy group, a (2-methoxyethyl)aminocarbonyloxy group, a (cyanomethyl)aminocarbonyloxy group, a (2-cyanoethyl)aminocarbonyloxy group, a dimethylaminocarbonyloxy group, an ethylmethylaminocarbonyloxy group, a diethylaminocarbonyloxy group, a (methoxymethyl)methylaminocarbonyloxy group, a (2-methoxyethyl)methylaminocarbonyloxy group, a (cyanomethyl)methylaminocarbonyloxy group, a (2-cyanoethyl)methylaminocarbonyloxy group, a 2,2-difluoroethylaminocarbonyloxy group, a 2,2,2-trifluoroethylaminocarbonyloxy group, a cyclopropylaminocarbonyloxy group, a (cyclopropyl)methylaminocarbonyloxy group, a pyrrolidinylcarbonyloxy group, or a piperidinylcarbonyloxy group, more preferably a formyloxy group, an acetyloxy group, or a trifluoroacetyloxy group.

Rd and L in "Rd-L-" represented by R₅ in the formula (2) are as defined above. "Rd-L-" is preferably a methylthio group, a methanesulfinyl group, a methanesulfonyl group, a trifluoromethylthio group, a trifluoromethanesulfinyl group, or a trifluoromethanesulfonyl group, more preferably a methylthio group, a methanesulfinyl group, or a methanesulfonyl group.

In the formula (2), G₁, G₂, and G₃ each independently represent a carbon atom or a nitrogen atom (on the proviso that at least one of G₁, G₂, and G₃ is a carbon atom).

For G₁, G₂, and G₃ in the formula (2), preferably, any one of G₁, G₂, and G₃ is a nitrogen atom.

In the formula (2), G₄ and G₅ each independently represent a nitrogen atom or CRx. When G₄ and G₅ each represent CRx, the Rx moieties may be the same or different.

For G₄ and G₅ in the formula (2), preferably, any one of G₄ and G₅ is a nitrogen atom.

Rx in the formula (2) represents a hydrogen atom, a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, -NRaRb (wherein Ra and Rb are as defined above), -C(=O)NRaRb (wherein Ra and Rb are as defined above), -C(=O)ORc (wherein Rc is as defined above), -OC(=O)Rc (wherein Rc is as defined above), or -L-Rd (wherein Rd and L are as defined above).

In particular, preferably, Rx is a hydrogen atom, a halogen atom, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, or a C2 to C6 haloalkynyl group. Particularly preferably, Rx is a hydrogen atom or a C1 to C6 alkyl group optionally substituted by substituent A.

The C1 to C6 alkyl group in the "C1 to C6 alkyl group optionally substituted by substituent A" represented by Rx in the formula (2) is as defined above and is preferably a methyl group, an ethyl group, or a propyl group, more preferably a methyl group or an ethyl group, further preferably a methyl group. In the case of having substituent A, a hydrogen atom in the C1 to C6 alkyl group is arbitrarily replaced with the substituent A.

The "C1 to C6 haloalkyl group" represented by Rx in the formula (2) is as defined above and is preferably a difluoromethyl group, a trifluoromethyl group, a 1,1-difluoroethyl group, a 2,2-difluoroethyl group, a 2,2,2-trifluoroethyl group, a 2,2,2-trichloroethyl group, a 3,3-difluoropropyl group, or a 3,3,3-trifluoropropyl group, more preferably a difluoromethyl group, a trifluoromethyl group, a 1,1-difluoroethyl group, a 2,2-difluoroethyl group, or a 2,2,2-trifluoroethyl group, further preferably a difluoromethyl group or a trifluoromethyl group.

The C3 to C8 cycloalkyl group in the "C3 to C8 cycloalkyl group optionally substituted by substituent A" represented by Rx in the formula (2) is as defined above and is preferably a cyclopropyl group, a cyclobutyl group, a cyclopentyl group, or a cyclohexyl group, more preferably a cyclopropyl group or a cyclobutyl group, further preferably a cyclopropyl group. In the case of having substituent A, a hydrogen atom in the C3 to C8 cycloalkyl group is arbitrarily replaced with the substituent A.

The C1 to C6 alkoxy group in the "C1 to C6 alkoxy group optionally substituted by substituent A" represented by Rx in the formula (2) is as defined above and is preferably a methoxy group, an ethoxy group, a propyloxy group, an isopropyloxy group, a butoxy group, or an isobutoxy group, more preferably a methoxy group or an ethoxy group, further preferably a methoxy group. In the case of having substituent A, a hydrogen atom in the C1 to C6 alkoxy group is arbitrarily replaced with the substituent A.

The "C1 to C6 haloalkoxy group" represented by Rx in the formula (2) is as defined above and is preferably a difluoromethoxy group, a trifluoromethoxy group, a 2,2-difluoroethoxy group, a 2,2,2-trifluoroethoxy group, a 3,3-difluoropropyloxy group, or a 3,3,3-trifluoropropyloxy group, more preferably a difluoromethoxy group, a trifluoromethoxy group, a 2,2-difluoroethoxy group, or a 2,2,2-trifluoroethoxy group, further preferably a difluoromethoxy group or a trifluoromethoxy group.

The C3 to C8 cycloalkoxy group in the "C3 to C8 cycloalkoxy group optionally substituted by substituent A" represented by Rx in the formula (2) is as defined above and is preferably a cyclopropyloxy group, a cyclobutoxy group, a cyclopentyloxy group, or a cyclohexyloxy group, more preferably a cyclopropyloxy group or a cyclobutoxy group. In the case of having substituent A, a hydrogen atom in the C3 to C8 cycloalkoxy group is arbitrarily replaced with the substituent A.

The C2 to C6 alkenyl group in the "C2 to C6 alkenyl group optionally substituted by substituent A" represented by Rx in the formula (2) is as defined above and is preferably a vinyl group, a 1-propenyl group, an allyl group, a 1-butenyl group, a 2-butenyl group, or a 3-butenyl group, more preferably a vinyl group, a 1-propenyl group, or an allyl group. In the case of having substituent A, a hydrogen atom in the C2 to C6 alkenyl group is arbitrarily replaced with the substituent A.

The "C2 to C6 haloalkenyl group" represented by Rx in the formula (2) is as defined above and is preferably a 2-fluorovinyl group, a 2,2-difluorovinyl group, a 3-fluoroallyl group, a 3,3-difluoroallyl group, or a 4,4-difluoro-3-butenyl group.

The C2 to C6 alkynyl group in the "C2 to C6 alkynyl group optionally substituted by substituent A" represented by Rx in the formula (2) is as defined above and is preferably an ethynyl group, a 1-propynyl group, a propargyl group, a 1-butynyl group, a 2-butynyl group, or a 3-butynyl group, more preferably an ethynyl group. In the case of having substituent A, a hydrogen atom in the C2 to C6 alkynyl group is arbitrarily replaced with the substituent A.

The "C2 to C6 haloalkynyl group" represented by Rx in the formula (2) is as defined above and is preferably a 2-fluoroethynyl group, a 2-chloroethynyl group, a 2-bromoethynyl group, or a 2-iodoethynyl group, more preferably a 2-fluoroethynyl group.

The C2 to C6 alkenyloxy group in the "C2 to C6 alkenyloxy group optionally substituted by substituent A" represented by Rx in the formula (2) is as defined above and is preferably a vinyloxy group, a 1-propenyloxy group, or an allyloxy group, more preferably an allyloxy group. In the case of having substituent A, a hydrogen atom in the C2 to C6 alkenyloxy group is arbitrarily replaced with the substituent A.

The "C2 to C6 haloalkenyloxy group" represented by Rx in the formula (2) is as defined above and is preferably a 2-fluorovinyloxy group, a 2,2-difluorovinyloxy group, a 3-fluoroallyloxy group, or a 3,3-difluoroallyloxy group, more preferably a 2-fluorovinyloxy group or a 2,2-difluorovinyloxy group.

The C3 to C6 alkynyloxy group in the "C3 to C6 alkynyloxy group optionally substituted by substituent A" represented by Rx in the formula (2) is as defined above and is preferably a propargyloxy group, a 2-butynyloxy group, or a 3-butynyloxy group, more preferably a propargyloxy group. In the case of having substituent A, a hydrogen atom in the C3 to C6 alkynyloxy group is arbitrarily replaced with the substituent A.

The "C3 to C6 haloalkynyloxy group" represented by Rx in the formula (2) is as defined above and is preferably a 4,4-difluoro-2-butynyloxy group, a 4-chloro-4,4-difluoro-2-butynyloxy group, a 4-bromo-4,4-difluoro-2-butynyloxy group, or a 4,4,4-trifluoro-2-butynyloxy group, more preferably a 4,4-difluoro-2-butynyloxy group or a 4,4,4-trifluoro-2-butynyloxy group.

Ra and Rb in "RaRbN-" represented by Rx in the formula (2) are as defined above. "RaRbN-" is preferably an amino group, a methylamino group, an ethylamino group, a (methoxymethyl)amino group, a (2-methoxyethyl)amino group, a (cyanomethyl)amino group, a (2-cyanoethyl)amino group, a dimethylamino group, an ethylmethylamino group, a diethylamino group, a (methoxymethyl)methylamino group, a (2-methoxyethyl)methylamino group, a (cyanomethyl)methylamino group, a (2-cyanoethyl)methylamino group, a 2,2-difluoroethylamino group, a 2,2,2-trifluoroethylamino group, a cyclopropylamino group, a (cyclopropyl)methylamino group, a pyrrolidinyl group, or a piperidinyl group, more preferably an amino group, a methylamino group, a dimethylamino group, an ethylmethylamino group, or a diethylamino group, further preferably an amino group, a methylamino group, or a dimethylamino group.

Ra and Rb in "-C(=O)NRaRb" represented by Rx in the formula (2) are as defined above. "-C(=O)NRaRb" is preferably an aminocarbonyl group, a methylaminocarbonyl group, an ethylaminocarbonyl group, a (methoxymethyl)aminocarbonyl group, a (2-methoxyethyl)aminocarbonyl group, a (cyanomethyl)aminocarbonyl group, a (2-cyanoethyl)aminocarbonyl group, a dimethylaminocarbonyl group, an ethylmethylaminocarbonyl group, a diethylaminocarbonyl group, a (methoxymethyl)methylaminocarbonyl group, a (2-methoxyethyl)methylaminocarbonyl group, a (cyanomethyl)methylaminocarbonyl group, a (2-cyanoethyl)methylaminocarbonyl group, a 2,2-difluoroethylaminocarbonyl group, a 2,2,2-trifluoroethylaminocarbonyl group, a cyclopropylaminocarbonyl group, a (cyclopropyl)methylaminocarbonyl group, a pyrrolidinyl group, or a piperidinyl group, more preferably an aminocarbonyl group, a methylaminocarbonyl group, a dimethylaminocarbonyl group, an ethylmethylaminocarbonyl group, or a diethylaminocarbonyl group, further preferably an aminocarbonyl group, a methylaminocarbonyl group, or a dimethylaminocarbonyl group.

Rc in "-C(=O)ORc" represented by Rx in the formula (2) is as defined above.

"-C(=O)ORc" is preferably a carboxy group, a methoxycarbonyl group, an ethoxycarbonyl group, a propyloxycarbonyl group, an isopropyloxycarbonyl group, a butoxycarbonyl group, isobutoxycarbonyl group, a difluoromethoxycarbonyl group, a trifluoromethoxycarbonyl group, a 2,2-difluoroethoxycarbonyl group, a 2,2,2-trifluoroethoxycarbonyl group, a 3,3-difluoropropyloxycarbonyl group, a 3,3,3-trifluoropropyloxy group, a cyclopropyloxycarbonyl group, a cyclobutoxycarbonyl group, a cyclopentyloxycarbonyl group, or a cyclohexyloxycarbonyl group, more preferably a methoxycarbonyl group, an ethoxycarbonyl group, a difluoromethoxycarbonyl group, a trifluoromethoxycarbonyl group, a 2,2-difluoroethoxycarbonyl group, a 2,2,2-trifluoroethoxycarbonyl group, or a cyclopropyloxycarbonyl group, further preferably a methoxycarbonyl group. In the case of having substituent A, a hydrogen atom in the C1 to C6 alkyl group is arbitrarily replaced with the substituent A.

Rc in "-OC(=O)Rc" represented by Rx in the formula (2) is as defined above.

"-OC(=O)Rc" is preferably a formyloxy group, an acetyloxy group, a methoxyacetyloxy group, a cyanoacetyloxy group, a propionyloxy group, a difluoroacetyloxy group, a trifluoroacetyloxy group, a cyclopropanecarbonyloxy group, a methoxycarbonyloxy group, an ethoxycarbonyloxy group, a 2,2-difluoroethoxycarbonyloxy group, a 2,2,2-trifluoroethoxycarbonyloxy group, a 3,3,3-trifluoropropyloxycarbonyloxy group, a cyclopropyloxycarbonyloxy group, an aminocarbonyloxy group, a methylaminocarbonyloxy group, an ethylaminocarbonyloxy group, a (methoxymethyl)aminocarbonyloxy group, a (2-methoxyethyl)aminocarbonyloxy group, a (cyanomethyl)aminocarbonyloxy group, a (2-cyanoethyl)aminocarbonyloxy group, a dimethylaminocarbonyloxy group, an ethylmethylaminocarbonyloxy group, a diethylaminocarbonyloxy group, a (methoxymethyl)methylaminocarbonyloxy group, a (2-methoxyethyl)methylaminocarbonyloxy group, a (cyanomethyl)methylaminocarbonyloxy group, a (2-cyanoethyl)methylaminocarbonyloxy group, a 2,2-difluoroethylaminocarbonyloxy group, a 2,2,2-trifluoroethylaminocarbonyloxy group, a cyclopropylaminocarbonyloxy group, a (cyclopropyl)methylaminocarbonyloxy group, a pyrrolidinylcarbonyloxy group, or a piperidinylcarbonyloxy group, more preferably a formyloxy group, an acetyloxy group, or a trifluoroacetyloxy group.

Rd and L in "Rd-L-" represented by Rx in the formula (2) are as defined above. "Rd-L-" is preferably a methylthio group, a methanesulfinyl group, a methanesulfonyl group, a trifluoromethylthio group, a trifluoromethanesulfinyl group, or a trifluoromethanesulfonyl group, more preferably a methylthio group, a methanesulfinyl group, or a methanesulfonyl group.

In the formula (2), the bond including the broken line represents a site represented by
-̅ -̅ -̅ -̅

The bond including the broken line in the formula (2) represents a double bond or a single bond.

Preferred specific examples of the partial structure having the combination of G₁, G₂, G₃, G₄, G₅, and the broken line in the formula (2) are shown below (in the following specific examples, R₃, R₄, R₅, and Rₓ are as defined above).

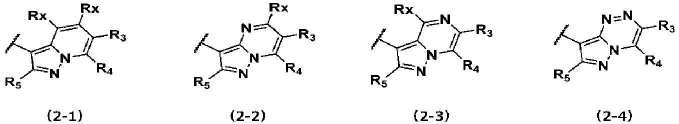

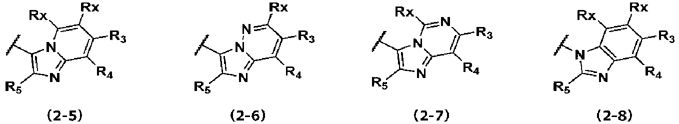

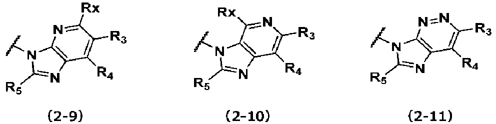

More preferred specific examples of the partial structure having the combination of G₁, G₂, G₃, G₄, G₅, and the binding containing the broken line in the formula (2) are shown below.

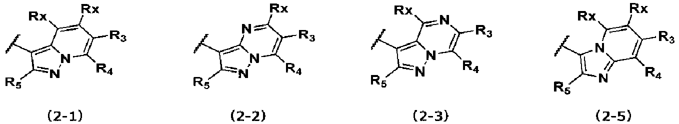

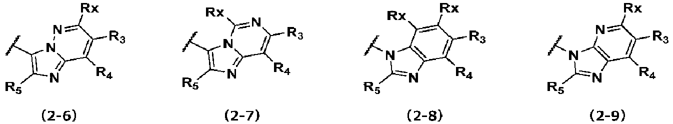

Further preferred specific examples of the partial structure having the combination of G₁, G₂, G₃, G₄, G₅, and the binding containing the broken line in the formula (2) are shown below.

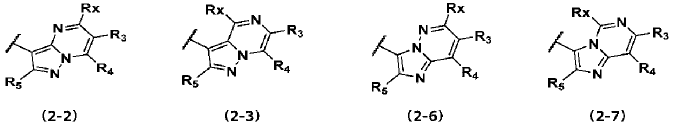

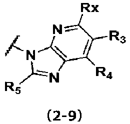

R₆ in the formula (1) represents a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, -NRaRb (wherein Ra and Rb are as defined above), -C(=O)ORc (wherein Rc is as defined above), -C(=O)NRaRb (wherein Ra and Rb are as defined above), -OC(=O)Rc (wherein Rc is as defined above), or -L-Rd (wherein L and Rd are as defined above).

In particular, preferably R₆ is a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, -NRaRb (wherein Ra and Rb are as defined above), -C(=O)ORc (wherein Rc is as defined above), -C(=O)NRaRb (wherein Ra and Rb are as defined above), or -L-Rd (wherein L and Rd are as defined above). Particularly preferably, R₆ is a halogen atom, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C3 to C8 cycloalkoxy group optionally substituted by substituent A, a C2 to C6 alkenyl group optionally substituted by substituent A, - NRaRb (wherein Ra and Rb are as defined above), -C(=O)NRaRb (wherein Ra and Rb are as defined above), or -L-Rd (wherein L and Rd are as defined above).

R₆ in the formula (1) includes a hydrogen atom, a hydroxy group, a cyano group, or a nitro group.

The halogen atom represented by R₆ in the formula (1) is as defined above and is preferably a fluorine atom, a chlorine atom, a bromine atom, or an iodine atom, more preferably a fluorine atom, a chlorine atom, or a bromine atom.

The C1 to C6 alkyl group in the "C1 to C6 alkyl group optionally substituted by substituent A" represented by R₆ in the formula (1) is as defined above and is preferably a methyl group, an ethyl group, or a propyl group, more preferably a methyl group or an ethyl group, further preferably a methyl group. In the case of having substituent A, a hydrogen atom in the C1 to C6 alkyl group is arbitrarily replaced with the substituent A.

The "C1 to C6 haloalkyl group" represented by R₆ in the formula (1) is as defined above and is preferably a difluoromethyl group, a trifluoromethyl group, a 1,1-difluoroethyl group, a 2,2-difluoroethyl group, a 2,2,2-trifluoroethyl group, a 2,2,2-trichloroethyl group, a 3,3-difluoropropyl group, or a 3,3,3-trifluoropropyl group, more preferably a difluoromethyl group, a trifluoromethyl group, a 1,1-difluoroethyl group, a 2,2-difluoroethyl group, or a 2,2,2-trifluoroethyl group, further preferably a difluoromethyl group or a trifluoromethyl group.

The C3 to C8 cycloalkyl group in the "C3 to C8 cycloalkyl group optionally substituted by substituent A" represented by R₆ in the formula (1) is as defined above and is preferably a cyclopropyl group, a cyclobutyl group, a cyclopentyl group, or a cyclohexyl group, more preferably a cyclopropyl group or a cyclobutyl group, further preferably a cyclopropyl group. In the case of having substituent A, a hydrogen atom in the C3 to C8 cycloalkyl group is arbitrarily replaced with the substituent A.

The C1 to C6 alkoxy group in the "C1 to C6 alkoxy group optionally substituted by substituent A" represented by R₆ in the formula (1) is as defined above and is preferably a methoxy group, an ethoxy group, a propyloxy group, an isopropyloxy group, a butoxy group, or an isobutoxy group, more preferably a methoxy group or an ethoxy group, further preferably a methoxy group. In the case of having substituent A, a hydrogen atom in the C1 to C6 alkoxy group is arbitrarily replaced with the substituent A.

The "C1 to C6 haloalkoxy group" represented by R₆ in the formula (1) is as defined above and is preferably a difluoromethoxy group, a trifluoromethoxy group, a 2,2-difluoroethoxy group, a 2,2,2-trifluoroethoxy group, a 3,3-difluoropropyloxy group, or a 3,3,3-trifluoropropyloxy group, more preferably a difluoromethoxy group, a trifluoromethoxy group, a 2,2-difluoroethoxy group, or a 2,2,2-trifluoroethoxy group, further preferably a difluoromethoxy group or a trifluoromethoxy group.

The C3 to C8 cycloalkoxy group in the "C3 to C8 cycloalkoxy group optionally substituted by substituent A" represented by R₆ in the formula (1) is as defined above and is preferably a cyclopropyloxy group, a cyclobutoxy group, a cyclopentyloxy group, or a cyclohexyloxy group, more preferably a cyclopropyloxy group or a cyclobutoxy group. In the case of having substituent A, a hydrogen atom in the C3 to C8 cycloalkoxy group is arbitrarily replaced with the substituent A.

The C2 to C6 alkenyl group in the "C2 to C6 alkenyl group optionally substituted by substituent A" represented by R₆ in the formula (1) is as defined above and is preferably a vinyl group, a 1-propenyl group, an allyl group, a 1-butenyl group, a 2-butenyl group, or a 3-butenyl group, more preferably a vinyl group, a 1-propenyl group, or an allyl group. In the case of having substituent A, a hydrogen atom in the C2 to C6 alkenyl group is arbitrarily replaced with the substituent A.

The "C2 to C6 haloalkenyl group" represented by R₆ in the formula (1) is as defined above and is preferably a 2-fluorovinyl group, a 2,2-difluorovinyl group, a 3-fluoroallyl group, a 3,3-difluoroallyl group, or a 4,4-difluoro-3-butenyl group.

The C2 to C6 alkynyl group in the "C2 to C6 alkynyl group optionally substituted by substituent A" represented by R₆ in the formula (1) is as defined above and is preferably an ethynyl group, a 1-propynyl group, a propargyl group, a 1-butynyl group, a 2-butynyl group, or a 3-butynyl group, more preferably an ethynyl group. In the case of having substituent A, a hydrogen atom in the C2 to C6 alkynyl group is arbitrarily replaced with the substituent A.

The "C2 to C6 haloalkynyl group" represented by R₆ in the formula (1) is as defined above and is preferably a 2-fluoroethynyl group, a 2-chloroethynyl group, a 2-bromoethynyl group, or a 2-iodoethynyl group, more preferably a 2-fluoroethynyl group.

The C2 to C6 alkenyloxy group in the "C2 to C6 alkenyloxy group optionally substituted by substituent A" represented by R₆ in the formula (1) is as defined above and is preferably a vinyloxy group, a 1-propenyloxy group, or an allyloxy group, more preferably an allyloxy group. In the case of having substituent A, a hydrogen atom in the C2 to C6 alkenyloxy group is arbitrarily replaced with the substituent A.

The "C2 to C6 haloalkenyloxy group" represented by R₆ in the formula (1) is as defined above and is preferably a 2-fluorovinyloxy group, a 2,2-difluorovinyloxy group, a 3-fluoroallyloxy group, or a 3,3-difluoroallyloxy group, more preferably a 2-fluorovinyloxy group or a 2,2-difluorovinyloxy group.

The C3 to C6 alkynyloxy group in the "C3 to C6 alkynyloxy group optionally substituted by substituent A" represented by R₆ in the formula (1) is as defined above and is preferably a propargyloxy group, a 2-butynyloxy group, or a 3-butynyloxy group, more preferably a propargyloxy group. In the case of having substituent A, a hydrogen atom in the C3 to C6 alkynyloxy group is arbitrarily replaced with the substituent A.

The "C3 to C6 haloalkynyloxy group" represented by R₆ in the formula (1) is as defined above and is preferably a 4,4-difluoro-2-butynyloxy group, a 4-chloro-4,4-difluoro-2-butynyloxy group, a 4-bromo-4,4-difluoro-2-butynyloxy group, or a 4,4,4-trifluoro-2-butynyloxy group, more preferably a 4,4-difluoro-2-butynyloxy group or a 4,4,4-trifluoro-2-butynyloxy group.

Ra and Rb in "RaRbN-" represented by R₆ in the formula (1) are as defined above.

"RaRbN-" is preferably an amino group, a methylamino group, an ethylamino group, a (methoxymethyl)amino group, a (2-methoxyethyl)amino group, a (cyanomethyl)amino group, a (2-cyanoethyl)amino group, a dimethylamino group, an ethylmethylamino group, a diethylamino group, a (methoxymethyl)methylamino group, a (2-methoxyethyl)methylamino group, a (cyanomethyl)methylamino group, a (2-cyanoethyl)methylamino group, a 2,2-difluoroethylamino group, a 2,2,2-trifluoroethylamino group, a cyclopropylamino group, a (cyclopropyl)methylamino group, a pyrrolidinyl group, or a piperidinyl group, more preferably an amino group, a methylamino group, a dimethylamino group, an ethylmethylamino group, or a diethylamino group, further preferably an amino group, a methylamino group, or a dimethylamino group.

Ra and Rb in "-C(=O)NRaRb" represented by R₆ in the formula (1) are as defined above. "-C(=O)NRaRb" is preferably an aminocarbonyl group, a methylaminocarbonyl group, an ethylaminocarbonyl group, a (methoxymethyl)aminocarbonyl group, a (2-methoxyethyl)aminocarbonyl group, a (cyanomethyl)aminocarbonyl group, a (2-cyanoethyl)aminocarbonyl group, a dimethylaminocarbonyl group, an ethylmethylaminocarbonyl group, a diethylaminocarbonyl group, a (methoxymethyl)methylaminocarbonyl group, a (2-methoxyethyl)methylaminocarbonyl group, a (cyanomethyl)methylaminocarbonyl group, a (2-cyanoethyl)methylaminocarbonyl group, a 2,2-difluoroethylaminocarbonyl group, a 2,2,2-trifluoroethylaminocarbonyl group, a cyclopropylaminocarbonyl group, a (cyclopropyl)methylaminocarbonyl group, a pyrrolidinyl group, or a piperidinyl group, more preferably an aminocarbonyl group, a methylaminocarbonyl group, a dimethylaminocarbonyl group, an ethylmethylaminocarbonyl group, or a diethylaminocarbonyl group, further preferably an aminocarbonyl group, a methylaminocarbonyl group, or a dimethylaminocarbonyl group.

Rc in "-C(=O)ORc" represented by R₆ in the formula (1) is as defined above.

"-C(=O)ORc" is preferably a carboxy group, a methoxycarbonyl group, an ethoxycarbonyl group, a propyloxycarbonyl group, an isopropyloxycarbonyl group, a butoxycarbonyl group, or isobutoxycarbonyl group, a difluoromethoxycarbonyl group, a trifluoromethoxycarbonyl group, a 2,2-difluoroethoxycarbonyl group, a 2,2,2-trifluoroethoxycarbonyl group, a 3,3-difluoropropyloxycarbonyl group, a 3,3,3-trifluoropropyloxy group, a cyclopropyloxycarbonyl group, a cyclobutoxycarbonyl group, a cyclopentyloxycarbonyl group, or a cyclohexyloxycarbonyl group, more preferably a methoxycarbonyl group, an ethoxycarbonyl group, a difluoromethoxycarbonyl group, a trifluoromethoxycarbonyl group, a 2,2-difluoroethoxycarbonyl group, a 2,2,2-trifluoroethoxycarbonyl group, or a cyclopropyloxycarbonyl group, further preferably a methoxycarbonyl group. In the case of having substituent A, a hydrogen atom in the C1 to C6 alkyl group is arbitrarily replaced with the substituent A.

Rc in "-OC(=O)Rc" represented by R₆ in the formula (1) is as defined above.

"-OC(=O)Rc" is preferably a formyloxy group, an acetyloxy group, a methoxyacetyloxy group, a cyanoacetyloxy group, a propionyloxy group, a difluoroacetyloxy group, a trifluoroacetyloxy group, a cyclopropanecarbonyloxy group, a methoxycarbonyloxy group, an ethoxycarbonyloxy group, a 2,2-difluoroethoxycarbonyloxy group, a 2,2,2-trifluoroethoxycarbonyloxy group, a 3,3,3-trifluoropropyloxycarbonyloxy group, a cyclopropyloxycarbonyloxy group, an aminocarbonyloxy group, a methylaminocarbonyloxy group, an ethylaminocarbonyloxy group, a (methoxymethyl)aminocarbonyloxy group, a (2-methoxyethyl)aminocarbonyloxy group, a (cyanomethyl)aminocarbonyloxy group, a (2-cyanoethyl)aminocarbonyloxy group, a dimethylaminocarbonyloxy group, an ethylmethylaminocarbonyloxy group, a diethylaminocarbonyloxy group, a (methoxymethyl)methylaminocarbonyloxy group, a (2-methoxyethyl)methylaminocarbonyloxy group, a (cyanomethyl)methylaminocarbonyloxy group, a (2-cyanoethyl)methylaminocarbonyloxy group, a 2,2-difluoroethylaminocarbonyloxy group, a 2,2,2-trifluoroethylaminocarbonyloxy group, a cyclopropylaminocarbonyloxy group, a (cyclopropyl)methylaminocarbonyloxy group, a pyrrolidinylcarbonyloxy group, or a piperidinylcarbonyloxy group, more preferably a formyloxy group, an acetyloxy group, or a trifluoroacetyloxy group.

Rd and L in "Rd-L-" represented by R₆ in the formula (1) are as defined above. "Rd-L-" is preferably a methylthio group, a methanesulfinyl group, a methanesulfonyl group, a trifluoromethylthio group, a trifluoromethanesulfinyl group, or a trifluoromethanesulfonyl group, more preferably a methylthio group, a methanesulfinyl group, or a methanesulfonyl group.

R₇ in the formula (1) represents a lone pair, a hydrogen atom, a C1 to C6 alkyl group optionally substituted by substituent A, a C1 to C6 haloalkyl group, a C2 to C6 alkenyl group optionally substituted by substituent A, a C2 to C6 haloalkenyl group, a C2 to C6 alkynyl group optionally substituted by substituent A, a C2 to C6 haloalkynyl group, or an acyl group optionally substituted by substituent A.

In particular, preferably, R₇ is a lone pair, a hydrogen atom, a C1 to C6 alkyl group optionally substituted by substituent A, a C1 to C6 haloalkyl group, or an acyl group optionally substituted by substituent A. Particularly preferably, R₇ is a lone pair.

When R₇ is a lone pair, the binding containing the broken line which the nitrogen atom bonded to R₇ forms represents a double bond. The resulting compound represented by the formula (1) has a structure represented by the following formula:

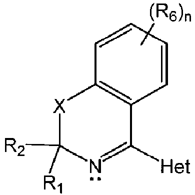

R₇ in the formula (1) includes a lone pair or a hydrogen atom.

The C1 to C6 alkyl group in the "C1 to C6 alkyl group optionally substituted by substituent A" represented by R₇ in the formula (1) is as defined above and is preferably a methyl group, an ethyl group, a propyl group, an isopropyl group, a butyl group, an isobutyl group, a s-butyl group, a pentyl group, or a hexyl group, more preferably a methyl group, an ethyl group, a propyl group, an isopropyl group, a butyl group, or an isobutyl group. In the case of having substituent A, a hydrogen atom in the C1 to C6 alkyl group is arbitrarily replaced with the substituent A.

The "C1 to C6 haloalkyl group" represented by R₇ in the formula (1) is as defined above and is preferably a difluoromethyl group, a trifluoromethyl group, a 1,1-difluoroethyl group, a 2,2-difluoroethyl group, a 2,2,2-trifluoroethyl group, a 2,2,2-trichloroethyl group, a 3,3-difluoropropyl group, or a 3,3,3-trifluoropropyl group.

The C2 to C6 alkenyl group in the "C2 to C6 alkenyl group optionally substituted by substituent A" represented by R₇ in the formula (1) is as defined above and is preferably a vinyl group, a 1-propenyl group, an allyl group, a 1-butenyl group, a 2-butenyl group, or a 3-butenyl group. In the case of having substituent A, a hydrogen atom in the C2 to C6 alkenyl group is arbitrarily replaced with the substituent A.

The "C2 to C6 haloalkenyl group" represented by R₇ in the formula (1) is as defined above and is preferably a 2-fluorovinyl group, a 2,2-difluorovinyl group, a 3-fluoroallyl group, a 3,3-difluoroallyl group, or a 4,4-difluoro-3-butenyl group.

The C2 to C6 alkynyl group in the "C2 to C6 alkynyl group optionally substituted by substituent A" represented by R₇ in the formula (1) is as defined above and is preferably an ethynyl group, a 1-propynyl group, a propargyl group, a 1-butynyl group, a 2-butynyl group, or a 3-butynyl group. In the case of having substituent A, a hydrogen atom in the C2 to C6 alkynyl group is arbitrarily replaced with the substituent A.

The "C2 to C6 haloalkynyl group" represented by R₇ in the formula (1) is as defined above and is preferably a 2-fluoroethynyl group, a 2-chloroethynyl group, a 2-bromoethynyl group, a 2-iodoethynyl group, a 3,3-difluoro-1-propynyl group, a 3-chloro-3,3-difluoro-1-propynyl group, a 3-bromo-3,3-difluoro-1-propynyl group, a 3,3,3-trifluoro-1-propynyl group, a 4,4-difluoro-1-butynyl group, a 4,4-difluoro-2-butynyl group, a 4-chloro-4,4-difluoro-1-butynyl group, a 4-chloro-4,4-difluoro-2-butynyl group, a 4-bromo-4,4-difluoro-1-butynyl group, a 4-bromo-4,4-difluoro-2-butynyl group, a 4,4,4-trifluoro-1-butynyl group, or a 4,4,4-trifluoro-2-butynyl group, more preferably a 2-fluoroethynyl group, a 3,3-difluoro-1-propynyl group, a 3,3,3-trifluoro-1-propynyl group, a 4,4-difluoro-1-butynyl group, a 4,4-difluoro-2-butynyl group, a 4,4,4-trifluoro-1-butynyl group, or a 4,4,4-trifluoro-2-butynyl group.

The C1 to C6 acyl group in the "C1 to C6 acyl group optionally substituted by substituent A" represented by R₇ in the formula (1) is as defined above and is preferably a formyl group, an acetyl group, a propionyl group, a butyryl group, or an isobutyryl group, more preferably an acetyl group or a propionyl group. In the case of having substituent A, a hydrogen atom in the C1 to C6 acyl group is arbitrarily replaced with the substituent A.

n represents an integer of 0 to 4. The integer of n is preferably 0 to 2, more preferably 0 or 1.

The binding containing the broken line which the nitrogen atom bonded to R₇ forms in the formula (1) will be described in detail.

In the formula (1), the binding containing the broken line which the nitrogen atom bonded to R₇ forms represents a site represented by
-̅ -̅ -̅ -̅

When the binding containing the broken line which the nitrogen atom bonded to R₇ forms in the formula (1) is a double bond, the resulting compound represents a compound represented by the formula (1a) or a salt thereof:

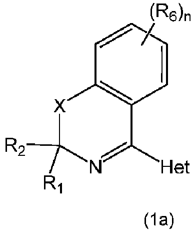

When the binding containing the broken line which the nitrogen atom bonded to R₇ forms in the formula (1) is a single bond, the resulting compound represents a compound represented by the formula (1b) or a salt thereof:

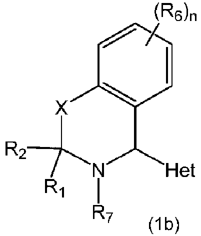

The compound of the formula (1b) wherein R₁ and R₂ are the same is either a R form or a S form, or is a mixture of R and S forms at an arbitrary ratio. Alternatively, the compound of the formula (1b) wherein R₁ and R₂ are different is only any one of (R,R), (R,S), (S,R), and (S,S) forms of diastereomer mixtures, or an arbitrary mixture of (R,R), (R,S), (S,R), and (S,S) forms.

X in the formula (1) represents O, S, S(=O), SO₂, or NRe. In particular, preferably, X is O, S, S(=O), or SO₂.

Re in the formula (1) represents a hydrogen atom, a C1 to C6 alkyl group optionally substituted by substituent A, a C1 to C6 haloalkyl group, a C2 to C6 alkenyl group optionally substituted by substituent A, a C2 to C6 haloalkenyl group, a C2 to C6 alkynyl group optionally substituted by substituent A, a C2 to C6 haloalkynyl group, or a C1 to C6 acyl group.

Re in the formula (1) includes a hydrogen atom.

The C1 to C6 alkyl group in the "C1 to C6 alkyl group optionally substituted by substituent A" represented by Re in the formula (1) is as defined above and is preferably a methyl group, an ethyl group, a propyl group, an isopropyl group, a butyl group, an isobutyl group, a s-butyl group, a pentyl group, or a hexyl group, more preferably a methyl group, an ethyl group, a propyl group, an isopropyl group, a butyl group or an isobutyl group. In the case of having substituent A, a hydrogen atom in the C1 to C6 alkyl group is arbitrarily replaced with the substituent A.

The "C1 to C6 haloalkyl group" represented by Re in the formula (1) is as defined above and is preferably a difluoromethyl group, a trifluoromethyl group, a 1,1-difluoroethyl group, a 2,2-difluoroethyl group, a 2,2,2-trifluoroethyl group, a 2,2,2-trichloroethyl group, a 3,3-difluoropropyl group, or a 3,3,3-trifluoropropyl group.

The C2 to C6 alkenyl group in the "C2 to C6 alkenyl group optionally substituted by substituent A" represented by Re in the formula (1) is as defined above and is preferably a vinyl group, a 1-propenyl group, an allyl group, a 1-butenyl group, a 2-butenyl group, or a 3-butenyl group. In the case of having substituent A, a hydrogen atom in the C2 to C6 alkenyl group is arbitrarily replaced with the substituent A.

The "C2 to C6 haloalkenyl group" represented by Re in the formula (1) is as defined above and is preferably a 2-fluorovinyl group, a 2,2-difluorovinyl group, a 3-fluoroallyl group, a 3,3-difluoroallyl group, or a 4,4-difluoro-3-butenyl group.

The C2 to C6 alkynyl group in the "C2 to C6 alkynyl group optionally substituted by substituent A" represented by Re in the formula (1) is as defined above and is preferably an ethynyl group, a 1-propynyl group, a propargyl group, a 1-butynyl group, a 2-butynyl group, or a 3-butynyl group. In the case of having substituent A, a hydrogen atom in the C2 to C6 alkynyl group is arbitrarily replaced with the substituent A.

The "C2 to C6 haloalkynyl group" represented by Re in the formula (1) is as defined above and is preferably a 2-fluoroethynyl group, a 2-chloroethynyl group, a 2-bromoethynyl group, a 2-iodoethynyl group, a 3,3-difluoro-1-propynyl group, a 3-chloro-3,3-difluoro-1-propynyl group, a 3-bromo-3,3-difluoro-1-propynyl group, a 3,3,3-trifluoro-1-propynyl group, a 4,4-difluoro-1-butynyl group, a 4,4-difluoro-2-butynyl group, a 4-chloro-4,4-difluoro-1-butynyl group, a 4-chloro-4,4-difluoro-2-butynyl group, a 4-bromo-4,4-difluoro-1-butynyl group, a 4-bromo-4,4-difluoro-2-butynyl group, a 4,4,4-trifluoro-1-butynyl group, or a 4,4,4-trifluoro-2-butynyl group, more preferably a 2-fluoroethynyl group, a 3,3-difluoro-1-propynyl group, a 3,3,3-trifluoro-1-propynyl group, a 4,4-difluoro-1-butynyl group, a 4,4-difluoro-2-butynyl group, a 4,4,4-trifluoro-1-butynyl group, or a 4,4,4-trifluoro-2-butynyl group.

The C1 to C6 acyl group in the "C1 to C6 acyl group optionally substituted by substituent A" represented by Re in the formula (1) is as defined above and is preferably a formyl group, an acetyl group, a propionyl group, a butyryl group, or an isobutyryl group, more preferably an acetyl group, a propionyl group. In the case of having substituent A, a hydrogen atom in the C1 to C6 acyl group is arbitrarily replaced with the substituent A.

In the present specification, the "substituent A" represents at least one member selected from the group consisting of a hydroxy group, a cyano group, a C3 to C8 cycloalkyl group, a C1 to C6 alkoxy group, a C1 to C6 haloalkoxy group, a C1 to C6 cycloalkoxy group, a C1 to C6 alkenyloxy group, a C1 to C6 haloalkenyloxy group, a C1 to C6 alkynyloxy group, a C1 to C6 haloalkynyloxy group, a C1 to C6 acyl group, an aryl group optionally substituted by substituent B, a heteroaryl group optionally substituted by substituent B, a phenoxy group optionally substituted by substituent B, -NRaRb (wherein Ra and Rb are as defined above), - C(=O)NRaRb (wherein Ra and Rb are as defined above), -C(=O)ORc (wherein Rc is as defined above), - OC(=O)Rc (wherein Rc is as defined above), and -L-Rd (wherein L and Rd are as defined above).

In particular, preferably, the "substituent A" is a hydroxy group, a cyano group, a C3 to C8 cycloalkyl group, a C1 to C6 alkoxy group, a C1 to C6 acyl group, an aryl group optionally substituted by substituent B, a phenoxy group optionally substituted by substituent B, -C(=O)NRaRb (wherein Ra and Rb are as defined above), - C(=O)ORc (wherein Rc is as defined above), -OC(=O)Rc (wherein Rc is as defined above), and -L-Rd (wherein L and Rd are as defined above). Particularly preferably, the "substituent A" is a cyano group, a C1 to C6 alkoxy group, an aryl group optionally substituted by substituent B, -C(=O)NRaRb (wherein Ra and Rb are as defined above), -C(=O)ORc (wherein Rc is as defined above), and -L-Rd (wherein L and Rd are as defined above).

Preferred specific examples of the substituent A are: a hydroxy group; a cyano group;
as a C3 to C8 cycloalkyl group, a cyclopropyl group, a cyclobutyl group, a cyclopentyl group, and a cyclohexyl group;
as a C1 to C6 alkoxy group, a methoxy group, an ethoxy group, a propyloxy group, and an isopropyloxy group; as a C1 to C6 acyl group, an acetyl group, a propionyl group, a butyryl group, an isobutyryl group; as an aryl group optionally substituted by substituent B, a phenyl group (in the case of having substituent B, a hydrogen atom in the aryl group is arbitrarily replaced with the substituent B);
as a heteroaryl group optionally substituted by substituent B, a pyridyl group, a pyrazinyl group, a thienyl group, a thiazolyl group, an isothiazolyl group, a furyl group, an imidazolyl group, a pyrazolyl group, an oxazolyl group, an isoxazolyl group, a triazolyl group, an oxadiazolyl group, and a tetrazolyl group (in the case of having substituent B, a hydrogen atom in the heteroaryl group is arbitrarily replaced with the substituent B); as a phenoxy group optionally substituted by substituent B, a phenoxy group (in the case of having substituent B, a hydrogen atom in the phenoxy group is arbitrarily replaced with the substituent B)
as "-NRaRb" (wherein Ra and Rb are as defined above), an amino group, a methylamino group, an ethylamino group, a (methoxymethyl)amino group, a (2-methoxyethyl)amino group, a (cyanomethyl)amino group, a (2-cyanoethyl)amino group, a dimethylamino group, an ethylmethylamino group, a diethylamino group, a (methoxymethyl)methylamino group, a (2-methoxyethyl)methylamino group, a (cyanomethyl)methylamino group, a (2-cyanoethyl)methylamino group, a 2,2-difluoroethylamino group, a 2,2,2-trifluoroethylamino group, a cyclopropylamino group, a (cyclopropyl)methylamino group, a pyrrolidinyl group, and a piperidinyl group; as "-C(=O)NRaRb" (wherein Ra and Rb are as defined above), an aminocarbonyl group, a methylaminocarbonyl group, an ethylaminocarbonyl group, a (methoxymethyl)aminocarbonyl group, a (2-methoxyethyl)aminocarbonyl group, a (cyanomethyl)aminocarbonyl group, a (2-cyanoethyl)aminocarbonyl group, a dimethylaminocarbonyl group, an ethylmethylaminocarbonyl group, a diethylaminocarbonyl group, a (methoxymethyl)methylaminocarbonyl group, a (2-methoxyethyl)methylaminocarbonyl group, a (cyanomethyl)methylaminocarbonyl group, a (2-cyanoethyl)methylaminocarbonyl group, a 2,2-difluoroethylaminocarbonyl group, a 2,2,2-trifluoroethylaminocarbonyl group, a cyclopropylaminocarbonyl group, a (cyclopropyl)methylaminocarbonyl group, a pyrrolidinyl group, and a piperidinyl group; as "-C(=O)ORc" (wherein Rc is as defined above), a carboxy group, an ethoxycarbonyl group, a propyloxycarbonyl group, an isopropyloxycarbonyl group, a butoxycarbonyl group, or an isobutoxycarbonyl group, a difluoromethoxycarbonyl group, a trifluoromethoxycarbonyl group, a 2,2-difluoroethoxycarbonyl group, a 2,2,2-trifluoroethoxycarbonyl group, a 3,3-difluoropropyloxycarbonyl group, or a 3,3,3-trifluoropropyloxy group, a cyclopropyloxycarbonyl group, a cyclobutoxycarbonyl group, a cyclopentyloxycarbonyl group, and a cyclohexyloxycarbonyl group;
as "-OC(=O)Rc" (wherein Rc is as defined above), a formyloxy group, an acetyloxy group, a methoxyacetyloxy group, a cyanoacetyloxy group, a propionyloxy group, a difluoroacetyloxy group, a trifluoroacetyloxy group, a cyclopropanecarbonyloxy group, a methoxycarbonyloxy group, an ethoxycarbonyloxy group, a 2,2-difluoroethoxycarbonyloxy group, a 2,2,2-trifluoroethoxycarbonyloxy group, a 3,3,3-trifluoropropyloxycarbonyloxy group, a cyclopropyloxycarbonyloxy group, an aminocarbonyloxy group, a methylaminocarbonyloxy group, an ethylaminocarbonyloxy group, a (methoxymethyl)aminocarbonyloxy group, a (2-methoxyethyl)aminocarbonyloxy group, a (cyanomethyl)aminocarbonyloxy group, a (2-cyanoethyl)aminocarbonyloxy group, a dimethylaminocarbonyloxy group, an ethylmethylaminocarbonyloxy group, a diethylaminocarbonyloxy group, a (methoxymethyl)methylaminocarbonyloxy group, a (2-methoxyethyl)methylaminocarbonyloxy group, a (cyanomethyl)methylaminocarbonyloxy group, a (2-cyanoethyl)methylaminocarbonyloxy group, a 2,2-difluoroethylaminocarbonyloxy group, a 2,2,2-trifluoroethylaminocarbonyloxy group, a cyclopropylaminocarbonyloxy group, a (cyclopropyl)methylaminocarbonyloxy group, a pyrrolidinylcarbonyloxy group, and a piperidinylcarbonyloxy group; and
as "-L-Rd" (wherein L and Rd are as defined above), a methylthio group, a methanesulfinyl group, a methanesulfonyl group, a trifluoromethylthio group, a trifluoromethanesulfinyl group, and a trifluoromethanesulfonyl group.

More preferred specific examples of the substituent A are: a cyano group;
as a C3 to C8 cycloalkyl group, a cyclopropyl group and a cyclobutyl group;
as a C1 to C6 alkoxy group, a methoxy group and an ethoxy group;
as an aryl group optionally substituted by substituent B, a phenyl group (in the case of having substituent B, a hydrogen atom in the aryl group is arbitrarily replaced with the substituent B);
as a heteroaryl group optionally substituted by substituent B, a pyridyl group (in the case of having substituent B, a hydrogen atom in the heteroaryl group is arbitrarily replaced with the substituent B);
as "-NRaRb" (wherein Ra and Rb are as defined above), a methylamino group, an ethylamino group, a dimethylamino group, an ethylmethylamino group, and a diethylamino group;
as "-C(=O)NRaRb" (wherein Ra and Rb are as defined above), an aminocarbonyl group, a methylaminocarbonyl group, an ethylaminocarbonyl group, and a dimethylaminocarbonyl group;
as "-C(=O)ORc" (wherein Rc is as defined above), a carboxy group, a methoxycarbonyl group, an ethoxycarbonyl group, a propyloxycarbonyl group, and an isopropyloxycarbonyl group;
as "-OC(=O)Rc" (wherein Rc is as defined above), an acetyloxy group, a methoxyacetyloxy group, a cyanoacetyloxy group, and a propionyloxy group; and
as "-L-Rd" (wherein L and Rd are as defined above), a methylthio group, a methanesulfinyl group, and a methanesulfonyl group.

Further preferred specific examples of the substituent A are a cyano group;
as a C3 to C8 cycloalkyl group, a cyclopropyl group and a cyclobutyl group;
as a C1 to C6 alkoxy group, a methoxy group and an ethoxy group;
as an aryl group optionally substituted by substituent B, a phenyl group (in the case of having substituent B, a hydrogen atom in the aryl group is arbitrarily replaced with the substituent B);
as "-NRaRb" (wherein Ra and Rb are as defined above), a methylamino group and a dimethylamino group;
as "-C(=O)NRaRb" (wherein Ra and Rb are as defined above), a methylaminocarbonyl group and a dimethylaminocarbonyl group;
as "-C(=O)ORc" (wherein Rc is as defined above), a carboxy group, a methoxycarbonyl group, a ethoxycarbonyl group, and a propyloxycarbonyl group;
as "-OC(=O)Rc" (wherein Rc is as defined above), an acetyloxy group and a propionyloxy group; and as "-L-Rd" (wherein L and Rd are as defined above), a methylthio group and a methanesulfonyl group.

In the present specification, the "substituent B" represents at least one member selected from the group consisting of a halogen atom, a hydroxy group, a cyano group, a nitro group, a C1 to C6 alkyl group, a C1 to C6 haloalkyl group, a C3 to C8 cycloalkyl group, a C1 to C6 alkoxy group, a C1 to C6 haloalkoxy group, a C3 to C8 cycloalkoxy group, and -L-Rd (wherein L and Rd are as defined above).

In particular, preferably, the substituent B is a halogen atom, a cyano group, a C1 to C6 alkyl group, a C1 to C6 haloalkyl group, a C3 to C8 cycloalkyl group, a C1 to C6 alkoxy group, and a C1 to C6 haloalkoxy group.

Preferred specific examples of the substituent B in the formula (1) are: a cyano group; as a halogen atom, a fluorine atom, a chlorine atom, a bromine atom, and an iodine atom;
as a C1 to C6 alkyl group, a methyl group, an ethyl group, a propyl group, and an isopropyl group;
as a C1 to C6 haloalkyl group, a difluoromethyl group, a trifluoromethyl group, a 1,1-difluoroethyl group, a 2,2-difluoroethyl group, a 2,2,2-trifluoroethyl group, a 2,2,2-trichloroethyl group, a 3,3-difluoropropyl group, and a 3,3,3-trifluoropropyl group;
as a C3 to C8 cycloalkyl group, a cyclopropyl group, a cyclobutyl group, a cyclopentyl group, and a cyclohexyl group;
as a C1 to C6 alkoxy group, a methoxy group, an ethoxy group, a propyloxy group, and an isopropyloxy group;
as a C1 to C6 haloalkoxy group, a fluoromethoxy group, a trifluoromethoxy group, a 2,2-difluoroethoxy group, a 2,2,2-trifluoroethoxy group, a 3,3-difluoropropyloxy group, and a 3,3,3-trifluoropropyloxy group, more preferably a difluoromethoxy group, a trifluoromethoxy group, a 2,2-difluoroethoxy group, and a 2,2,2-trifluoroethoxy group; and
as a C3 to C8 cycloalkoxy group, a cyclopropyloxy group, a cyclobutoxy group, a cyclopentyloxy group, and a cyclohexyloxy group, more preferably a cyclopropyloxy group.

More preferred specific examples of the substituent B in the formula (1) are: a cyano group;
as a halogen atom, a fluorine atom and a chlorine atom;
as a C1 to C6 alkyl group, a methyl group and an ethyl group;
as a C1 to C6 haloalkyl group, a difluoromethyl group and a trifluoromethyl group; as a C3 to C8 cycloalkyl group, a cyclopropyl group and a cyclobutyl group;
as a C1 to C6 alkoxy group, a methoxy group and an ethoxy group;
as a C1 to C6 haloalkoxy group, a difluoromethoxy group and a trifluoromethoxy group; and as a C3 to C8 cycloalkoxy group, a cyclopropyloxy group and a cyclobutoxy group.

The compound represented by the formula (1) may contain an asymmetric atom. In this respect, the isomer ratio is a single proportion or an arbitrary mixing ratio and is not particularly limited.

The compound represented by the formula (1) may include a geometric isomer. In this respect, the isomer ratio is a single proportion or an arbitrary mixing ratio and is not particularly limited.

The compound represented by the formula (1) can form a salt. Examples of such a salt include salts of acids such as hydrochloric acid, sulfuric acid, acetic acid, fumaric acid, and maleic acid, and salts of metals such as sodium, potassium, and calcium. The salt is not particularly limited as long as the salt can be used as an agricultural and horticultural germicide.

The entire scope of compounds obtained by arbitrarily combining preferred ranges of R₁, R₂, R₃, R₄, R₅, R₆, R₇, X, G₁, G₂, G₃, G₄, G₅, Het, the bond including the broken line, the substituent A, and the substituent B described above should also be interpreted as the scope of the compound of the formula (1) of the present invention or an intermediate compound for production thereof.

The compound represented by the formula (1) or a salt thereof is preferably a following compound or a salt thereof, in the formula (1),
R₁ and R₂ each independently represent a group selected from the group consisting of a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C1 to C6 acyl group optionally substituted by substituent A, an aryl group optionally substituted by substituent B, a heteroaryl group optionally substituted by substituent B, -C(=O)NRaRb (wherein Ra and Rb each independently represent a group selected from the group consisting of a hydrogen atom, a C1 to C6 alkyl group optionally substituted by substituent A, a C1 to C6 haloalkyl group, a C3 to C8 cycloalkyl group, a C1 to C6 acyl group, and -C(=O)ORc (wherein Rc represents a group selected from the group consisting of a hydrogen atom, a C1 to C6 alkyl group optionally substituted by substituent A, a C1 to C6 haloalkyl group, and a C3 to C8 cycloalkyl group), or Ra and Rb form, together with the nitrogen atom bonded thereto, an aziridinyl group, an azetidinyl group, a pyrrolidinyl group, a piperidinyl group, a homopiperidinyl group, or an azocanyl group), and -C(=O)ORc (wherein Rc is as defined above),or
R₁ and R₂ together optionally form a C3 to C8 cycloalkyl ring optionally substituted by substituent A, or a C3 to C8 saturated or unsaturated heterocyclic ring optionally substituted by substituent A;
Het represents the formula (2): 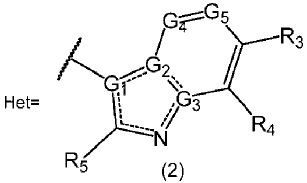
R₃, R₄, and R₅ in the formula (2) each independently represent a group selected from the group consisting of a hydrogen atom, a halogen atom, a C1 to C6 alkyl group optionally substituted by substituent A, a C3 to C8 cycloalkyl group optionally substituted by substituent A, a C1 to C6 alkoxy group optionally substituted by substituent A, and a C2 to C6 alkenyl group optionally substituted by substituent A, or
R₃ and R₄ together form a C5 to C10 unsaturated ring optionally substituted by substituent A;
R₅ represents a hydrogen atom;
n represents an integer of 0 to 4;
R₆ represents a group selected from the group consisting of a halogen atom, a C1 to C6 alkyl group optionally substituted by substituent A, a C3 to C8 cycloalkyl group optionally substituted by substituent A, a C1 to C6 alkoxy group optionally substituted by substituent A, a C2 to C6 alkenyl group optionally substituted by substituent A, and -NRaRb (wherein Ra and Rb are as defined above);
R₇ represents a lone pair;
G₁, G₂, and G₃ each independently represent a group selected from the group consisting of a carbon atom and a nitrogen atom (on the proviso that at least one of G₁, G₂, and G₃ is a carbon atom);
G₄ and G₅ each independently represent a group selected from the group consisting of a nitrogen atom and CRx;
Rx represents a hydrogen atom or a C1 to C6 alkyl group optionally substituted by substituent A, among them, preferably represents a hydrogen atom or a methyl group;
the binding containing the broken line represents a single bond or a double bond;
X represents a group selected from the group consisting of O, S, and SO₂;
the substituent A is at least one member selected from the group consisting of a hydroxy group, a cyano group, a C3 to C8 cycloalkyl group, a C1 to C6 alkoxy group, a C1 to C6 haloalkoxy group, and a C1 to C6 cycloalkoxy group, a C1 to C6 alkenyloxy group, a C1 to C6 haloalkenyloxy group, a C1 to C6 alkynyloxy group, and a C1 to C6 haloalkynyloxy group, a C1 to C6 acyl group, an aryl group optionally substituted by substituent B, a heteroaryl group optionally substituted by substituent B, a phenoxy group optionally substituted by substituent B, -NRaRb (wherein Ra and Rb are as defined above), -C(=O)NRaRb (wherein Ra and Rb are as defined above), -C(=O)ORc (wherein Rc is as defined above), -OC(=O)Rc (wherein Rc is as defined above), and -L-Rd (wherein L and Rd are as defined above); and
the substituent B is at least one member selected from the group consisting of a halogen atom, a hydroxy group, a cyano group, a nitro group, a C1 to C6 alkyl group optionally substituted by substituent A, a C1 to C6 haloalkyl group, a C3 to C8 cycloalkyl group, a C1 to C6 alkoxy group, a C1 to C6 haloalkoxy group, a C3 to C8 cycloalkoxy group, and Rd-L- (wherein Rd is as defined above).

The formula (2) may represent the structures (A) to (C) described in Examples.

Next, the compound of the present invention is specifically represented by the formula (1) (wherein X is an oxygen atom or a sulfur atom, n represents an integer of 0 to 4, and the bond including the broken line represents a double bond or a single bond) and a combination of substituents R₁-1 to R₁-543 and R₂-1 to R₂-543 shown in Table 1, which represent R₁ and R₂ (wherein R₁ and R₂ are as defined above), substituents R₃-1 to R₃-75 and R₄-1 to R₄-75 shown in Table 2, which represent R₃ and R₄ (wherein R₃ and R₄ are as defined above), substituents R₅-1 to R₅-81 shown in Table 3, which represent R₅ (wherein R₅ is as defined above), substituents R₆-1 to R₆-80 shown in Table 4, which represent R₆ (wherein R₆ is as defined above), substituents R₇-1 to R₇-111 shown in Table 5, which represent R₇ (wherein R₇ is as defined above), and structural formulas Het-A1 to Het-A16, Het-B1 to Het-B15, and Het-C1 to Het-C16 of Het (wherein Het is as defined above) shown in Table 6 (wherein the bond position cut by the wavy line in Table 6 represents the position at which Het in the formula (1) is bonded). These compounds are exemplary compounds given for illustration, and the present invention is not limited thereby.

In the tables given below, for example, "-2-F-Ph" means that a fluorine atom is bonded to the 2-position of a phenyl group. In the present specification, the same holds true for the description below.

**Table 1:**

| No. | Substituent examples of R₁ | No. | Substituent examples of R₂ |
|---|---|---|---|
| R₁-1 | -Me | R₂-1 | -Me |
| R₁-2 | -Et | R₂-2 | -Et |
| R₁-3 | -Pr | R₂-3 | -Pr |
| R₁-4 | -i-Pr | R₂-4 | -i-Pr |
| R₁-5 | -Bu | R₂-5 | -Bu |
| R₁-6 | -i-Bu | R₂-6 | -i-Bu |
| R₁-7 | -sec-Bu | R₂-7 | -sec-Bu |
| R₁-8 | -t-Bu | R₂-8 | -t-Bu |
| R₁-9 | -Pent | R₂-9 | -Pent |
| R₁-10 | -Hex | R₂-10 | -Hex |
| R₁-11 | -CH(CH₃)CH₂CH₂CH₃ | R₂-11 | -CH(CH₃)CH₂CH₂CH₃ |
| R₁-12 | -CH(CH₃)CH(CH₃)₂ | R₂-12 | -CH(CH₃)CH(CH₃)₂ |
| R₁-13 | -C(CH₃)₂CH₂CH₃ | R₂-13 | -C(CH₃)₂CH₂CH₃ |
| R₁-14 | -CH(CH₂CH₃)2 | R₂-14 | -CH(CH₂CH₃)2 |
| R₁-15 | -CH₂CH₂CH(CH₃)₂ | R₂-15 | -CH₂CH₂CH(CH₃)₂ |
| R₁-16 | -CH₂CH₂CH₂CH(CH₃)₂ | R₂-16 | -CH₂CH₂CH₂CH(CH₃)₂ |
| R₁-17 | -CH₂CH₂CH(CH₃)CH₂CH₃ | R₂-17 | -CH₂CH₂CH(CH₃)CH₂CH₃ |
| R₁-18 | -CH₂CH(CH₃)CH₂CH₂CH₃ | R₂-18 | -CH₂CH(CH₃)CH₂CH₂CH₃ |
| R₁-19 | -CH(CH₃)CH₂CH₂CH₂CH₃ | R₂-19 | -CH(CH₃)CH₂CH₂CH₂CH₃ |
| R₁-20 | -CH(CH₃)CH₂CH(CH₃)₂ | R₂-20 | -CH(CH₃)CH₂CH(CH₃)₂ |
| R₁-21 | -CH(CH₃)CH(CH₃)CH₂CH₃ | R₂-21 | -CH(CH₃)CH(CH₃)CH₂CH₃ |
| R₁-22 | -C(CH₃)₂CH₂CH₂CH₃ | R₂-22 | -C(CH₃)₂CH₂CH₂CH₃ |
| R₁-23 | -CH(CH₃)C(CH₃)₃ | R₂-23 | -CH(CH₃)C(CH₃)₃ |
| R₁-24 | -C(CH₃)₂CH(CH₃)₂ | R₂-24 | -C(CH₃)₂CH(CH₃)₂ |
| R₁-25 | -CH₂CH₂C(CH₃)₃ | R₂-25 | -CH₂CH₂C(CH₃)₃ |
| R₁-26 | -CH(CH₃)CH₂CH₂CH₃ | R₂-26 | -CH(CH₃)CH₂CH₂CH₃ |
| R₁-27 | -CH₂CH(CH₃)₂CH₂CH₃ | R₂-27 | -CH₂CH(CH₃)₂CH₂CH₃ |
| R₁-28 | -CFH₂ | R₂-28 | -CFH₂ |
| R₁-29 | -CF₂H | R₂-29 | -CF₂H |
| R₁-30 | -CF₃ | R₂-30 | -CF₃ |
| R₁-31 | -CClH₂ | R₂-31 | -CClH₂ |
| R₁-32 | -CCl₂H | R₂-32 | -CCl₂H |
| R₁-33 | -CCl₃ | R₂-33 | -CCl₃ |
| R₁-34 | -CBrH₂ | R₂-34 | -CBrH₂ |
| R₁-35 | -CBr₂H | R₂-35 | -CBr₂H |
| R₁-36 | -CBr₃ | R₂-36 | -CBr₃ |
| R₁-37 | -CH₂I | R₂-37 | -CH₂I |
| R₁-38 | -CHI2 | R₂-38 | -CHI2 |
| R₁-39 | -CI₃ | R₂-39 | -CI₃ |
| R₁-40 | -CH₂CF₂H | R₂-40 | -CH₂CF₂H |
| R₁-41 | -CH₂CF₃ | R₂-41 | -CH₂CF₃ |
| R₁-42 | -CH₂CH₂CF₂H | R₂-42 | -CH₂CH₂CF₂H |
| R₁-43 | -CH₂CH₂CF₃ | R₂-43 | -CH₂CH₂CF₃ |
| R₁-44 | -CH₂CH₂CH₂CF₂H | R₂-44 | -CH₂CH₂CH₂CF₂H |
| R₁-45 | -CH₂CH₂CH₂CF₃ | R₂-45 | -CH₂CH₂CH₂CF₃ |
| R₁-46 | -CF₂CF₂H | R₂-46 | -CF₂CF₂H |
| R₁-47 | -CF₂CF₃ | R₂-47 | -CF₂CF₃ |
| R₁-48 | -CHFCF₃ | R₂-48 | -CHFCF₃ |
| R₁-49 | -CH₂CF₂CF₂H | R₂-49 | -CH₂CF₂CF₂H |
| R₁-50 | -CH₂CF₂CF₃ | R₂-50 | -CH₂CF₂CF₃ |
| R₁-51 | -CF₂CF₂CF₃ | R₂-51 | -CF₂CF₂CF₃ |
| R₁-52 | -CH₂CF₂CF₂CF₃ | R₂-52 | -CH₂CF₂CF₂CF₃ |
| R₁-53 | -CF₂CF₂CF₂CF₃ | R₂-53 | -CF₂CF₂CF₂CF₃ |
| R₁-54 | -CH₂CF₂CF₂CF₂CF₃ | R₂-54 | -CH₂CF₂CF₂CF₂CF₃ |
| R₁-55 | -c-Pr | R₂-55 | -c-Pr |
| R₁-56 | -c-Bu | R₂-56 | -c-Bu |
| R₁-57 | -c-pent | R₂-57 | -c-pent |
| R₁-58 | -c-Hex | R₂-58 | -c-Hex |
| R₁-59 | -c-Hept | R₂-59 | -c-Hept |
| R₁-60 | -c-Oct | R₂-60 | -c-Oct |
| R₁-61 | -CH=CH₂ | R₂-61 | -CH=CH₂ |
| R₁-62 | -CH₂CH=CH₂ | R₂-62 | -CH₂CH=CH₂ |
| R₁-63 | -C(CH₃)=CH₂ | R₂-63 | -C(CH₃)=CH₂ |
| R₁-64 | -CH=CHCH₃ | R₂-64 | -CH=CHCH₃ |
| R₁-65 | -CH₂C(CH₃)=CH₂ | R₂-65 | -CH₂C(CH₃)=CH₂ |
| R₁-66 | -CH₂CH₂CH=CH₂ | R₂-66 | -CH₂CH₂CH=CH₂ |
| R₁-67 | -CH₂CH=CHCH₃ | R₂-67 | -CH₂CH=CHCH₃ |
| R₁-68 | -CH=CHCH₂CH₃ | R₂-68 | -CH=CHCH₂CH₃ |
| R₁-69 | -CH₂CH=C(CH₃)₂ | R₂-69 | -CH₂CH=C(CH₃)₂ |
| R₁-70 | -CH₂CH₂CH=C(CH₃)₂ | R₂-70 | -CH₂CH₂CH=C(CH₃)₂ |
| R₁-71 | -CH=CFH | R₂-71 | -CH=CFH |
| R₁-72 | -CH=CF₂ | R₂-72 | -CH=CF₂ |
| R₁-73 | -CH=CCl₂ | R₂-73 | -CH=CCl₂ |
| R₁-74 | -CH₂CH=CFH | R₂-74 | -CH₂CH=CFH |
| R₁-75 | -CH₂CH=CF₂ | R₂-75 | -CH₂CH=CF₂ |
| R₁-76 | -CH₂CH=CCl₂ | R₂-76 | -CH₂CH=CCl₂ |
| R₁-77 | -CH₂CH₂CH=CF₂ | R₂-77 | -CH₂CH₂CH=CF₂ |
| R₁-78 | -CH₂CH₂CH₂CH=CF₂ | R₂-78 | -CH₂CH₂CH₂CH=CF₂ |
| R₁-79 | -CH₂CH₂CH₂CH₂CH=CF₂ | R₂-79 | -CH₂CH₂CH₂CH₂CH=CF₂ |
| R₁-80 | -C≡CH | R₂-80 | -C≡CH |
| R₁-81 | -C≡CCH₃ | R₂-81 | -C≡CCH₃ |
| R₁-82 | -CH₂C≡CH | R₂-82 | -CH₂C≡CH |
| R₁-83 | -C≡CCH₂CH₃ | R₂-83 | -C≡CCH₂CH₃ |
| R₁-84 | -CH₂C≡CCH₃ | R₂-84 | -CH₂C≡CCH₃ |
| R₁-85 | -CH₂CH₂C≡CH | R₂-85 | -CH₂CH₂C≡CH |
| R₁-86 | -C≡CCH₂CH₂CH₃ | R₂-86 | -C≡CCH₂CH₂CH₃ |
| R₁-87 | -CH₂C≡CCH₂CH₃ | R₂-87 | -CH₂C≡CCH₂CH₃ |
| R₁-88 | -C(CH₃)₂C≡CH | R₂-88 | -C(CH₃)₂C≡CH |
| R₁-89 | -C≡CF | R₂-89 | -C≡CF |
| R₁-90 | -C≡CCF₂H | R₂-90 | -C≡CCF₂H |
| R₁-91 | -C≡CCF₃ | R₂-91 | -C≡CCF₃ |
| R₁-92 | -C≡CCH₂CF₂H | R₂-92 | -C≡CCH₂CF₂H |
| R₁-93 | -C≡CCH₂CF₃ | R₂-93 | -C≡CCH₂CF₃ |
| R₁-94 | -CH₂C≡CCF₂H | R₂-94 | -CH₂C≡CCF₂H |
| R₁-95 | -CH₂C≡CCF₃ | R₂-95 | -CH₂C≡CCF₃ |
| R₁-96 | -Ph | R₂-96 | -Ph |
| R₁-97 | -2-F-Ph | R₂-97 | -2-F-Ph |
| R₁-98 | -3-F-Ph | R₂-98 | -3-F-Ph |
| R₁-99 | -4-F-Ph | R₂-99 | -4-F-Ph |
| R₁-100 | -2-Cl-Ph | R₂-100 | -2-Cl-Ph |
| R₁-101 | -3-Cl-Ph | R₂-101 | -3-Cl-Ph |
| R₁-102 | -4-Cl-Ph | R₂-102 | -4-Cl-Ph |
| R₁-103 | -2-Br-Ph | R₂-103 | -2-Br-Ph |
| R₁-104 | -3-Br-Ph | R₂-104 | -3-Br-Ph |
| R₁-105 | -4-Br-Ph | R₂-105 | -4-Br-Ph |
| R₁-106 | -2-I-Ph | R₂-106 | -2-I-Ph |
| R₁-107 | -3-I-Ph | R₂-107 | -3-I-Ph |
| R₁-108 | -4-I-Ph | R₂-108 | -4-I-Ph |
| R₁-109 | -2-N≡C-Ph | R₂-109 | -2-N≡C-Ph |
| R₁-110 | -3-N≡C-Ph | R₂-110 | -3-N≡C-Ph |
| R₁-111 | -4-N≡C-Ph | R₂-111 | -4-N≡C-Ph |
| R₁-112 | -2-Me-Ph | R₂-112 | -2-Me-Ph |
| R₁-113 | -3-Me-Ph | R₂-113 | -3-Me-Ph |
| R₁-114 | -4-Me-Ph | R₂-114 | -4-Me-Ph |
| R₁-115 | -2-Et-Ph | R₂-115 | -2-Et-Ph |
| R₁-116 | -3-Et-Ph | R₂-116 | -3-Et-Ph |
| R₁-117 | -4-Et-Ph | R₂-117 | -4-Et-Ph |
| R₁-118 | -2-Pr-Ph | R₂-118 | -2-Pr-Ph |
| R₁-119 | -3-Pr-Ph | R₂-119 | -3-Pr-Ph |
| R₁-120 | -4-Pr-Ph | R₂-120 | -4-Pr-Ph |
| R₁-121 | -2-i-Pr-Ph | R₂-121 | -2-i-Pr-Ph |
| R₁-122 | -3-i-Pr-Ph | R₂-122 | -3-i-Pr-Ph |
| R₁-123 | -4-i-Pr-Ph | R₂-123 | -4-i-Pr-Ph |
| R₁-124 | -2-CF₂H-Ph | R₂-124 | -2-CF₂H-Ph |
| R₁-125 | -3-CF₂H-Ph | R₂-125 | -3-CF₂H-Ph |
| R₁-126 | -4-CF₂H-Ph | R₂-126 | -4-CF₂H-Ph |
| R₁-127 | -2-CF₃-Ph | R₂-127 | -2-CF₃-Ph |
| R₁-128 | -3-CF₃-Ph | R₂-128 | -3-CF₃-Ph |
| R₁-129 | -4-CF₃-Ph | R₂-129 | -4-CF₃-Ph |
| R₁-130 | -2-c-Pr-Ph | R₂-130 | -2-c-Pr-Ph |
| R₁-131 | -3-c-Pr-Ph | R₂-131 | -3-c-Pr-Ph |
| R₁-132 | -4-c-Pr-Ph | R₂-132 | -4-c-Pr-Ph |
| R₁-133 | -2-OMe-Ph | R₂-133 | -2-OMe-Ph |
| R₁-134 | -3-OMe-Ph | R₂-134 | -3-OMe-Ph |
| R₁-135 | -4-OMe-Ph | R₂-135 | -4-OMe-Ph |
| R₁-136 | -2-OEt-Ph | R₂-136 | -2-OEt-Ph |
| R₁-137 | -3-OEt-Ph | R₂-137 | -3-OEt-Ph |
| R₁-138 | -4-OEt-Ph | R₂-138 | -4-OEt-Ph |
| R₁-139 | -2-OCF₂H-Ph | R₂-139 | -2-OCF₂H-Ph |
| R₁-140 | -3-OCF₂H-Ph | R₂-140 | -3-OCF₂H-Ph |
| R₁-141 | -4-OCF₂H-Ph | R₂-141 | -4-OCF₂H-Ph |
| R₁-142 | -2-OCF₃-Ph | R₂-142 | -2-OCF₃-Ph |
| R₁-143 | -3-OCF₃-Ph | R₂-143 | -3-OCF₃-Ph |
| R₁-144 | -4-OCF₃-Ph | R₂-144 | -4-OCF₃-Ph |
| R₁-145 | -2-SMe-Ph | R₂-145 | -2-SMe-Ph |
| R₁-146 | -3-SMe-Ph | R₂-146 | -3-SMe-Ph |
| R₁-147 | -4-SMe-Ph | R₂-147 | -4-SMe-Ph |
| R₁-148 | -2-S(=O)Me-Ph | R₂-148 | -2-S(=O)Me-Ph |
| R₁-149 | -3-S(=O)Me-Ph | R₂-149 | -3-S(=O)Me-Ph |
| R₁-150 | -4-S(=O)Me-Ph | R₂-150 | -4-S(=O)Me-Ph |
| R₁-151 | -2-SO₂Me-Ph | R₂-151 | -2-SO₂Me-Ph |
| R₁-152 | -3-SO₂Me-Ph | R₂-152 | -3-SO₂Me-Ph |
| R₁-153 | -4-SO₂Me-Ph | R₂-153 | -4-SO₂Me-Ph |
| R₁-154 | -2-SCF₃-Ph | R₂-154 | -2-SCF₃-Ph |
| R₁-155 | -3-SCF₃-Ph | R₂-155 | -3-SCF₃-Ph |
| R₁-156 | -4-SCF₃-Ph | R₂-156 | -4-SCF₃-Ph |
| R₁-157 | -2-S(=O)CF₃-Ph | R₂-157 | -2-S(=O)CF₃-Ph |
| R₁-158 | -3-S(=O)CF₃-Ph | R₂-158 | -3-S(=O)CF₃-Ph |
| R₁-159 | -4-S(=O)CF₃-Ph | R₂-159 | -4-S(=O)CF₃-Ph |
| R₁-160 | -2-SO₂CF₃-Ph | R₂-160 | -2-SO₂CF₃-Ph |
| R₁-161 | -3-SO₂CF₃-Ph | R₂-161 | -3-SO₂CF₃-Ph |
| R₁-162 | -4-SO₂CF₃-Ph | R₂-162 | -4-SO₂CF₃-Ph |
| R₁-163 | -CH₂C(=O)Me | R₂-163 | -CH₂C(=O)Me |
| R₁-164 | -CH₂C(=O)Et | R₂-164 | -CH₂C(=O)Et |
| R₁-165 | -CH₂C(=O)Pr | R₂-165 | -CH₂C(=O)Pr |
| R₁-166 | -CH₂C(=O)i-Pr | R₂-166 | -CH₂C(=O)i-Pr |
| R₁-167 | -CH₂C(=O)Bu | R₂-167 | -CH₂C(=O)Bu |
| R₁-168 | -CH₂C(=O)i-Bu | R₂-168 | -CH₂C(=O)i-Bu |
| R₁-169 | -CH₂C(=O)sec-Bu | R₂-169 | -CH₂C(=O)sec-Bu |
| R₁-170 | -CH₂C(=O)t-Bu | R₂-170 | -CH₂C(=O)t-Bu |
| R₁-171 | -CH₂CH₂C(=O)Me | R₂-171 | -CH₂CH₂C(=O)Me |
| R₁-172 | -CH₂CH₂CH₂C(=O)Me | R₂-172 | -CH₂CH₂CH₂C(=O)Me |
| R₁-173 | -CH₂CH₂CH₂CH₂C(=O)Me | R₂-173 | -CH₂CH₂CH₂CH₂C(=O)Me |
| R₁-174 | -CH₂C(=O)Et | R₂-174 | -CH₂C(=O)Et |
| R₁-175 | -CH₂CH₂C(=O)Et | R₂-175 | -CH₂CH₂C(=O)Et |
| R₁-176 | -CH₂CH₂CH₂C(=O)Et | R₂-176 | -CH₂CH₂CH₂C(=O)Et |
| R₁-177 | -CH₂C(=O)Pr | R₂-177 | -CH₂C(=O)Pr |
| R₁-178 | -CH₂CH₂C(=O)Pr | R₂-178 | -CH₂CH₂C(=O)Pr |
| R₁-179 | -CH₂C(=O)i-Pr | R₂-179 | -CH₂C(=O)i-Pr |
| R₁-180 | -CH₂CH₂C(=O)i-Pr | R₂-180 | -CH₂CH₂C(=O)i-Pr |
| R₁-181 | -CH₂C(=O)Bu | R₂-181 | -CH₂C(=O)Bu |
| R₁-182 | -CH₂C(=O)i-Bu | R₂-182 | -CH₂C(=O)i-Bu |
| R₁-183 | -CH₂C(=O)sec-Bu | R₂-183 | -CH₂C(=O)sec-Bu |
| R₁-184 | -CH₂C(=O)t-Bu | R₂-184 | -CH₂C(=O)t-Bu |
| R₁-185 | -C(=O)H | R₂-185 | -C(=O)H |
| R₁-186 | -C(=O)Me | R₂-186 | -C(=O)Me |
| R₁-187 | -C(=O)Et | R₂-187 | -C(=O)Et |
| R₁-188 | -C(=O)Pr | R₂-188 | -C(=O)Pr |
| R₁-189 | -C(=O)i-Pr | R₂-189 | -C(=O)i-Pr |
| R₁-190 | -C(=O)Bu | R₂-190 | -C(=O)Bu |
| R₁-191 | -C(=O)i-Bu | R₂-191 | -C(=O)i-Bu |
| R₁-192 | -C(=O)sec-Bu | R₂-192 | -C(=O)sec-Bu |
| R₁-193 | -C(=O)t-Bu | R₂-193 | -C(=O)t-Bu |
| R₁-194 | -C(=O)c-Pr | R₂-194 | -C(=O)c-Pr |
| R₁-195 | -C(=O)c-Bu | R₂-195 | -C(=O)c-Bu |
| R₁-196 | -C(=O)CHF₂ | R₂-196 | -C(=O)CHF₂ |
| R₁-197 | -C(=O)CF₃ | R₂-197 | -C(=O)CF₃ |
| R₁-198 | -C(=O)CH₂CHF₂ | R₂-198 | -C(=O)CH₂CHF₂ |
| R₁-199 | -C(=O)CH₂CF₂H | R₂-199 | -C(=O)CH₂CF₂H |
| R₁-200 | -C(=O)CH₂CF₃ | R₂-200 | -C(=O)CH₂CF₃ |
| R₁-201 | -C(=O)CH₂CH₂CF₂H | R₂-201 | -C(=O)CH₂CH₂CF₂H |
| R₁-202 | -C(=O)CH₂CH₂CF₃ | R₂-202 | -C(=O)CH₂CH₂CF₃ |
| R₁-203 | -C(=O)CH₂CH₂CH₂CF₂H | R₂-203 | -C(=O)CH₂CH₂CH₂CF₂H |
| R₁-204 | -C(=O)CH₂CH₂CH₂CF₃ | R₂-204 | -C(=O)CH₂CH₂CH₂CF₃ |
| R₁-205 | -C(=O)CF₂CF₂H | R₂-205 | -C(=O)CF₂CF₂H |
| R₁-206 | -C(=O)CF₂CF₃ | R₂-206 | -C(=O)CF₂CF₃ |
| R₁-207 | -C(=O)CHFCF₃ | R₂-207 | -C(=O)CHFCF₃ |
| R₁-208 | -C(=O)CH₂CF₂CF₂H | R₂-208 | -C(=O)CH₂CF₂CF₂H |
| R₁-209 | -C(=O)CH₂CF₂CF₃ | R₂-209 | -C(=O)CH₂CF₂CF₃ |
| R₁-210 | -C(=O)CF₂CF₂CF₃ | R₂-210 | -C(=O)CF₂CF₂CF₃ |
| R₁-211 | -C(=O)CH₂CF₂CF₂CF₃ | R₂-211 | -C(=O)CH₂CF₂CF₂CF₃ |
| R₁-212 | -C(=O)CF₂CF₂CF₂CF₃ | R₂-212 | -C(=O)CF₂CF₂CF₂CF₃ |
| R₁-213 | -C(=O)CH₂OMe | R₂-213 | -C(=O)CH₂OMe |
| R₁-214 | -C(=O)CH₂OCH₂CH₃ | R₂-214 | -C(=O)CH₂OCH₂CH₃ |
| R₁-215 | -C(=O)CH₂C≡N | R₂-215 | -C(=O)CH₂C≡N |
| R₁-216 | -C(=O)CH₂CH₂C≡N | R₂-216 | -C(=O)CH₂CH₂C≡N |
| R₁-217 | -C(=O)Ph | R₂-217 | -C(=O)Ph |
| R₁-218 | -CH₂C(=O)OH | R₂-218 | -CH₂C(=O)OH |
| R₁-219 | -CH₂C(=O)OMe | R₂-219 | -CH₂C(=O)OMe |
| R₁-220 | -CH₂C(=O)OEt | R₂-220 | -CH₂C(=O)OEt |
| R₁-221 | -CH₂C(=O)OPr | R₂-221 | -CH₂C(=O)OPr |
| R₁-222 | -CH₂C(=O)Oi-Pr | R₂-222 | -CH₂C(=O)Oi-Pr |
| R₁-223 | -CH₂C(=O)OBu | R₂-223 | -CH₂C(=O)OBu |
| R₁-224 | -CH₂C(=O)Oi-Bu | R₂-224 | -CH₂C(=O)Oi-Bu |
| R₁-225 | -CH₂C(=O)Osec-Bu | R₂-225 | -CH₂C(=O)Osec-Bu |
| R₁-226 | -CH₂C(=O)Ot-Bu | R₂-226 | -CH₂C(=O)Ot-Bu |
| R₁-227 | -CH₂C(=O)Oc-Pr | R₂-227 | -CH₂C(=O)Oc-Pr |
| R₁-228 | -CH₂C(=O)Oc-Bu | R₂-228 | -CH₂C(=O)Oc-Bu |
| R₁-229 | -CH₂C(=O)OCHF₂ | R₂-229 | -CH₂C(=O)OCHF₂ |
| R₁-230 | -CH₂C(=O)OCF₃ | R₂-230 | -CH₂C(=O)OCF₃ |
| R₁-231 | -CH₂C(=O)OCH₂CHF₂ | R₂-231 | -CH₂C(=O)OCH₂CHF₂ |
| R₁-232 | -CH₂C(=O)OCH₂CF₂H | R₂-232 | -CH₂C(=O)OCH₂CF₂H |
| R₁-233 | -CH₂C(=O)OCH₂CF₃ | R₂-233 | -CH₂C(=O)OCH₂CF₃ |
| R₁-234 | -CH₂C(=O)OCH₂CH₂CF₂H | R₂-234 | -CH₂C(=O)OCH₂CH₂CF₂H |
| R₁-235 | -CH₂C(=O)OCH₂CH₂CF₃ | R₂-235 | -CH₂C(=O)OCH₂CH₂CF₃ |
| R₁-236 | -CH₂C(= O)OCH₂CH₂CH₂CF₂H | R₂-236 | -CH₂C(=O)OCH₂CH₂CH₂CF₂H |
| R₁-237 | -CH₂C(=O)OCH₂CH₂CH₂CF₃ | R₂-237 | -CH₂C(=O)OCH₂CH₂CH₂CF₃ |
| R₁-238 | -CH₂C(=O)OCF₂CF₂H | R₂-238 | -CH₂C(=O)OCF₂CF₂H |
| R₁-239 | -CH₂C(=O)OCF₂CF₃ | R₂-239 | -CH₂C(=O)OCF₂CF₃ |
| R₁-240 | -CH₂C(=O)OCHFCF₃ | R₂-240 | -CH₂C(=O)OCHFCF₃ |
| R₁-241 | -CH₂C(=O)OCH₂CF₂CF₂H | R₂-241 | -CH₂C(=O)OCH₂CF₂CF₂H |
| R₁-242 | -CH₂C(=O)OCH₂CF₂CF₃ | R₂-242 | -CH₂C(=O)OCH₂CF₂CF₃ |
| R₁-243 | -CH₂C(=O)OCF₂CF₂CF₃ | R₂-243 | -CH₂C(=O)OCF₂CF₂CF₃ |
| R₁-244 | -CH₂C(=O)OCH₂CF₂CF₂CF₃ | R₂-244 | -CH₂C(=O)OCH₂CF₂CF₂CF₃ |
| R₁-245 | -CH₂C(=O)OCF₂CF₂CF₂CF₃ | R₂-245 | -CH₂C(=O)OCF₂CF₂CF₂CF₃ |
| R₁-246 | -CH₂C(=O)OCH₂OMe | R₂-246 | -CH₂C(=O)OCH₂OMe |
| R₁-247 | -CH₂C(=O)OCH₂OCH₂CH₃ | R₂-247 | -CH₂C(=O)OCH₂OCH₂CH₃ |
| R₁-248 | -CH₂C(=O)OCH₂C≡N | R₂-248 | -CH₂C(=O)OCH₂C≡N |
| R₁-249 | -CH₂C(=O)OCH₂CH₂C≡N | R₂-249 | -CH₂C(=O)OCH₂CH₂C≡N |
| R₁-250 | -CH₂CH₂C(=O)OH | R₂-250 | -CH₂CH₂C(=O)OH |
| R₁-251 | -CH₂CH₂C(=O)OMe | R₂-251 | -CH₂CH₂C(=O)OMe |
| R₁-252 | -CH₂CH₂C(=O)OEt | R₂-252 | -CH₂CH₂C(=O)OEt |
| R₁-253 | -CH₂CH₂C(=O)OPr | R₂-253 | -CH₂CH₂C(=O)OPr |
| R₁-254 | -CH₂CH₂C(=O)Oi-Pr | R₂-254 | -CH₂CH₂C(=O)Oi-Pr |
| R₁-255 | -CH₂CH₂C(=O)OBu | R₂-255 | -CH₂CH₂C(=O)OBu |
| R₁-256 | -CH₂CH₂C(=O)Oi-Bu | R₂-256 | -CH₂CH₂C(=O)Oi-Bu |
| R₁-257 | -CH₂CH₂C(=O)Osec-Bu | R₂-257 | -CH₂CH₂C(=O)Osec-Bu |
| R₁-258 | -CH₂CH₂C(=O)Ot-Bu | R₂-258 | -CH₂CH₂C(=O)Ot-Bu |
| R₁-259 | -CH₂CH₂C(=O)Oc-Pr | R₂-259 | -CH₂CH₂C(=O)Oc-Pr |
| R₁-260 | -CH₂CH₂C(=O)Oc-Bu | R₂-260 | -CH₂CH₂C(=O)Oc-Bu |
| R₁-261 | -CH₂CH₂C(=O)OCHF₂ | R₂-261 | -CH₂CH₂C(=O)OCHF₂ |
| R₁-262 | -CH₂CH₂C(=O)OCF₃ | R₂-262 | -CH₂CH₂C(=O)OCF₃ |
| R₁-263 | -CH₂CH₂C(=O)OCH₂CHF₂ | R₂-263 | -CH₂CH₂C(=O)OCH₂CHF₂ |
| R₁-264 | -CH₂CH₂C(=O)OCH₂CF₂H | R₂-264 | -CH₂CH₂C(=O)OCH₂CF₂H |
| R₁-265 | -CH₂CH₂C(=O)OCH₂CF₃ | R₂-265 | -CH₂CH₂C(=O)OCH₂CF₃ |
| R₁-266 | -CH₂CH₂C(= O)OCH₂CH₂CF₂H | R₂-266 | -CH₂CH₂C(=O)OCH₂CH₂CF₂H |
| R₁-267 | -CH₂CH₂C(=O)OCH₂CH₂CF₃ | R₂-267 | -CH₂CH₂C(=O)OCH₂CH₂CF₃ |
| R₁-268 | -CH₂CH₂C(= O)OCH₂CH₂CH₂CF₂H | R₂-268 | -CH₂CH₂C(= O)OCH₂CH₂CH₂CF₂H |
| R₁-269 | -CH₂CH₂C(= O)OCH₂CH₂CH₂CF₃ | R₂-269 | -CH₂CH₂C(= O)OCH₂CH₂CH₂CF₃ |
| R₁-270 | -CH₂CH₂C(=O)OCF₂CF₂H | R₂-270 | -CH₂CH₂C(=O)OCF₂CF₂H |
| R₁-271 | -CH₂CH₂C(=O)OCF₂CF₃ | R₂-271 | -CH₂CH₂C(=O)OCF₂CF₃ |
| R₁-272 | -CH₂CH₂C(=O)OCHFCF₃ | R₂-272 | -CH₂CH₂C(=O)OCHFCF₃ |
| R₁-273 | -CH₂CH₂C(=O)OCH₂CF₂CF₂H | R₂-273 | -CH₂CH₂C(=O)OCH₂CF₂CF₂H |
| R₁-274 | -CH₂CH₂C(=O)OCH₂CF₂CF₃ | R₂-274 | -CH₂CH₂C(=O)OCH₂CF₂CF₃ |
| R₁-275 | -CH₂CH₂C(=O)OCF₂CF₂CF₃ | R₂-275 | -CH₂CH₂C(=O)OCF₂CF₂CF₃ |
| R₁-276 | -CH₂CH₂C(= O)OCH₂CF₂CF₂CF₃ | R₂-276 | -CH₂CH₂C(= O)OCH₂CF₂CF₂CF₃ |
| R₁-277 | -CH₂CH₂C(= O)OCF₂CF₂CF₂CF₃ | R₂-277 | -CH₂CH₂C(=O)OCF₂CF₂CF₂CF₃ |
| R₁-278 | -CH₂CH₂C(=O)OCH₂OMe | R₂-278 | -CH₂CH₂C(=O)OCH₂OMe |
| R₁-279 | -CH₂CH₂C(= O)OCH₂OCH₂CH₃ | R₂-279 | -CH₂CH₂C(=O)OCH₂OCH₂CH₃ |
| R₁-280 | -CH₂CH₂C(=O)OCH₂C≡N | R₂-280 | -CH₂CH₂C(=O)OCH₂C≡N |
| R₁-281 | -CH₂CH₂C(=O)OCH₂CH₂C≡N | R₂-281 | -CH₂CH₂C(=O)OCH₂CH₂C≡N |
| R₁-282 | -C(=O)OH | R₂-282 | -C(=O)OH |
| R₁-283 | -C(=O)OMe | R₂-283 | -C(=O)OMe |
| R₁-284 | -C(=O)OEt | R₂-284 | -C(=O)OEt |
| R₁-285 | -C(=O)OPr | R₂-285 | -C(=O)OPr |
| R₁-286 | -C(=O)Oi-Pr | R₂-286 | -C(=O)Oi-Pr |
| R₁-287 | -C(=O)OBu | R₂-287 | -C(=O)OBu |
| R₁-288 | -C(=O)Oi-Bu | R₂-288 | -C(=O)Oi-Bu |
| R₁-289 | -C(=O)Osec-Bu | R₂-289 | -C(=O)Osec-Bu |
| R₁-290 | -C(=O)Ot-Bu | R₂-290 | -C(=O)Ot-Bu |
| R₁-291 | -C(=O)Oc-Pr | R₂-291 | -C(=O)Oc-Pr |
| R₁-292 | -C(=O)Oc-Bu | R₂-292 | -C(=O)Oc-Bu |
| R₁-293 | -C(=O)OCHF₂ | R₂-293 | -C(=O)OCHF₂ |
| R₁-294 | -C(=O)OCF₃ | R₂-294 | -C(=O)OCF₃ |
| R₁-295 | -C(=O)OCH₂CHF₂ | R₂-295 | -C(=O)OCH₂CHF₂ |
| R₁-296 | -C(=O)OCH₂CF₂H | R₂-296 | -C(=O)OCH₂CF₂H |
| R₁-297 | -C(=O)OCH₂CF₃ | R₂-297 | -C(=O)OCH₂CF₃ |
| R₁-298 | -C(=O)OCH₂CH₂CF₂H | R₂-298 | -C(=O)OCH₂CH₂CF₂H |
| R₁-299 | -C(=O)OCH₂CH₂CF₃ | R₂-299 | -C(=O)OCH₂CH₂CF₃ |
| R₁-300 | -C(=O)OCH₂CH₂CH₂CF₂H | R₂-300 | -C(=O)OCH₂CH₂CH₂CF₂H |
| R₁-301 | -C(=O)OCH₂CH₂CH₂CF₃ | R₂-301 | -C(=O)OCH₂CH₂CH₂CF₃ |
| R₁-302 | -C(=O)OCF₂CF₂H | R₂-302 | -C(=O)OCF₂CF₂H |
| R₁-303 | -C(=O)OCF₂CF₃ | R₂-303 | -C(=O)OCF₂CF₃ |
| R₁-304 | -C(=O)OCHFCF₃ | R₂-304 | -C(=O)OCHFCF₃ |
| R₁-305 | -C(=O)OCH₂CF₂CF₂H | R₂-305 | -C(=O)OCH₂CF₂CF₂H |
| R₁-306 | -C(=O)OCH₂CF₂CF₃ | R₂-306 | -C(=O)OCH₂CF₂CF₃ |
| R₁-307 | -C(=O)OCF₂CF₂CF₃ | R₂-307 | -C(=O)OCF₂CF₂CF₃ |
| R₁-308 | -C(=O)OCH₂CF₂CF₂CF₃ | R₂-308 | -C(=O)OCH₂CF₂CF₂CF₃ |
| R₁-309 | -C(=O)OCF₂CF₂CF₂CF₃ | R₂-309 | -C(=O)OCF₂CF₂CF₂CF₃ |
| R₁-310 | -C(=O)OCH₂OMe | R₂-310 | -C(=O)OCH₂OMe |
| R₁-311 | -C(=O)OCH₂OCH₂CH₃ | R₂-311 | -C(=O)OCH₂OCH₂CH₃ |
| R₁-312 | -C(=O)OCH₂C≡N | R₂-312 | -C(=O)OCH₂C≡N |
| R₁-313 | -C(=O)OCH₂CH₂C≡N | R₂-313 | -C(=O)OCH₂CH₂C≡N |
| R₁-314 | -CH₂C≡N | R₂-314 | -CH₂C≡N |
| R₁-315 | -CH(CH₃)C≡N | R₂-315 | -CH(CH₃)C≡N |
| R₁-316 | -CH₂CH₂C≡N | R₂-316 | -CH₂CH₂C≡N |
| R₁-317 | -CH₂CH₂CH₂C≡N | R₂-317 | -CH₂CH₂CH₂C≡N |
| R₁-318 | -CH₂-c-Pr | R₂-318 | -CH₂-c-Pr |
| R₁-319 | -CH₂-c-Bu | R₂-319 | -CH₂-c-Bu |
| R₁-320 | -CH₂OH | R₂-320 | -CH₂OH |
| R₁-321 | -CH₂OCH₃ | R₂-321 | -CH₂OCH₃ |
| R₁-322 | -CH₂OCH₂CH₃ | R₂-322 | -CH₂OCH₂CH₃ |
| R₁-323 | -CH₂OCH₂CH₂CH₃ | R₂-323 | -CH₂OCH₂CH₂CH₃ |
| R₁-324 | -CH₂OCH(CH₃)CH₃ | R₂-324 | -CH₂OCH(CH₃)CH₃ |
| R₁-325 | -CH₂CH₂OCH₃ | R₂-325 | -CH₂CH₂OCH₃ |
| R₁-326 | -CH₂CH₂OCH₂CH₃ | R₂-326 | -CH₂CH₂OCH₂CH₃ |
| R₁-327 | -CH₂SH | R₂-327 | -CH₂SH |
| R₁-328 | -CH₂SCH₃ | R₂-328 | -CH₂SCH₃ |
| R₁-329 | -CH₂SCH₂CH₃ | R₂-329 | -CH₂SCH₂CH₃ |
| R₁-330 | -CH₂SCH₂CH₂CH₃ | R₂-330 | -CH₂SCH₂CH₂CH₃ |
| R₁-331 | -CH₂SCH(CH₃)CH₃ | R₂-331 | -CH₂SCH(CH₃)CH₃ |
| R₁-332 | -CH₂CH₂SCH₃ | R₂-332 | -CH₂CH₂SCH₃ |
| R₁-333 | -CH₂CH₂SCH₂CH₃ | R₂-333 | -CH₂CH₂SCH₂CH₃ |
| R₁-334 | -CH₂S(=O)H | R₂-334 | -CH₂S(=O)H |
| R₁-335 | -CH₂S(=O)CH₃ | R₂-335 | -CH₂S(=O)CH₃ |
| R₁-336 | -CH₂S(=O)CH₂CH₃ | R₂-336 | -CH₂S(=O)CH₂CH₃ |
| R₁-337 | -CH₂S(=O)CH₂CH₂CH₃ | R₂-337 | -CH₂S(=O)CH₂CH₂CH₃ |
| R₁-338 | -CH₂S(-O)CH(CH₃)CH₃ | R₂-338 | -CH₂S(-O)CH(CH₃)CH₃ |
| R₁-339 | -CH₂CH₂S(-O)CH₃ | R₂-339 | -CH₂CH₂S(-O)CH₃ |
| R₁-340 | -CH₂CH₂S(-O)CH₂CH₃ | R₂-340 | -CH₂CH₂S(-O)CH₂CH₃ |
| R₁-341 | -CH₂SO₂H | R₂-341 | -CH₂SO₂H |
| R₁-342 | -CH₂SO₂CH₃ | R₂-342 | -CH₂SO₂CH₃ |
| R₁-343 | -CH₂SO₂CH₂CH₃ | R₂-343 | -CH₂SO₂CH₂CH₃ |
| R₁-344 | -CH₂SO₂CH₂CH₂CH₃ | R₂-344 | -CH₂SO₂CH₂CH₂CH₃ |
| R₁-345 | -CH₂SO₂CH(CH₃)CH₃ | R₂-345 | -CH₂SO₂CH(CH₃)CH₃ |
| R₁-346 | -CH₂CH₂SO₂CH₃ | R₂-346 | -CH₂CH₂SO₂CH₃ |
| R₁-347 | -CH₂CH₂SO₂CH₂CH₃ | R₂-347 | -CH₂CH₂SO₂CH₂CH₃ |
| R₁-348 | -CH₂NH₂ | R₂-348 | -CH₂NH₂ |
| R₁-349 | -CH₂NHCH₃ | R₂-349 | -CH₂NHCH₃ |
| R₁-350 | -CH₂NHCH₂CH₃ | R₂-350 | -CH₂NHCH₂CH₃ |
| R₁-351 | -CH₂NHCH₂CH₂CH₃ | R₂-351 | -CH₂NHCH₂CH₂CH₃ |
| R₁-352 | -CH₂NHCH(CH₃)CH₃ | R₂-352 | -CH₂NHCH(CH₃)CH₃ |
| R₁-353 | -CH₂N(CH₃)₂ | R₂-353 | -CH₂N(CH₃)₂ |
| R₁-354 | -CH₂N(CH₃)CH₂CH₃ | R₂-354 | -CH₂N(CH₃)CH₂CH₃ |
| R₁-355 | -CH₂N(CH₃)CH₂CH₂CH₃ | R₂-355 | -CH₂N(CH₃)CH₂CH₂CH₃ |
| R₁-356 | -CH₂N(CH₃)CH(CH₃)CH₃ | R₂-356 | -CH₂N(CH₃)CH(CH₃)CH₃ |
| R₁-357 | -CH₂N(CH₂CH₃)2 | R₂-357 | -CH₂N(CH₂CH₃)2 |
| R₁-358 | -CH₂C(=O)NH₂ | R₂-358 | -CH₂C(=O)NH₂ |
| R₁-359 | -CH₂C(=O)NHMe | R₂-359 | -CH₂C(=O)NHMe |
| R₁-360 | -CH₂C(=O)NHEt | R₂-360 | -CH₂C(=O)NHEt |
| R₁-361 | -CH₂C(=O)NHPr | R₂-361 | -CH₂C(=O)NHPr |
| R₁-362 | -CH₂C(=O)NHi-Pr | R₂-362 | -CH₂C(=O)NHi-Pr |
| R₁-363 | -CH₂C(=O)NHBu | R₂-363 | -CH₂C(=O)NHBu |
| R₁-364 | -CH₂C(=O)NHi-Bu | R₂-364 | -CH₂C(=O)NHi-Bu |
| R₁-365 | -CH₂C(=O)NHsec-Bu | R₂-365 | -CH₂C(=O)NHsec-Bu |
| R₁-366 | -CH₂C(=O)NHt-Bu | R₂-366 | -CH₂C(=O)NHt-Bu |
| R₁-367 | -CH₂C(=O)NHc-Pr | R₂-367 | -CH₂C(=O)NHc-Pr |
| R₁-368 | -CH₂C(=O)NHc-Bu | R₂-368 | -CH₂C(=O)NHc-Bu |
| R₁-369 | -CH₂C(=O)NHCHF₂ | R₂-369 | -CH₂C(=O)NHCHF₂ |
| R₁-370 | -CH₂C(=O)NHCF₃ | R₂-370 | -CH₂C(=O)NHCF₃ |
| R₁-371 | -CH₂C(=O)NHCH₂CHF₂ | R₂-371 | -CH₂C(=O)NHCH₂CHF₂ |
| R₁-372 | -CH₂C(=O)NHCH₂CF₂H | R₂-372 | -CH₂C(=O)NHCH₂CF₂H |
| R₁-373 | -CH₂C(=O)NHCH₂CF₃ | R₂-373 | -CH₂C(=O)NHCH₂CF₃ |
| R₁-374 | -CH₂C(=O)NHCH₂CH₂CF₂H | R₂-374 | -CH₂C(=O)NHCH₂CH₂CF₂H |
| R₁-375 | -CH₂C(=O)NHCH₂CH₂CF₃ | R₂-375 | -CH₂C(=O)NHCH₂CH₂CF₃ |
| R₁-376 | -CH₂C(= O)NHCH₂CH₂CH₂CF₂H | R₂-376 | -CH₂C(= O)NHCH₂CH₂CH₂CF₂H |
| R₁-377 | -CH₂C(= O)NHCH₂CH₂CH₂CF₃ | R₂-377 | -CH₂C(=O)NHCH₂CH₂CH₂CF₃ |
| R₁-378 | -CH₂C(=O)NHCF₂CF₂H | R₂-378 | -CH₂C(=O)NHCF₂CF₂H |
| R₁-379 | -CH₂C(=O)NHCF₂CF₃ | R₂-379 | -CH₂C(=O)NHCF₂CF₃ |
| R₁-380 | -CH₂C(=O)NHCHFCF₃ | R₂-380 | -CH₂C(=O)NHCHFCF₃ |
| R₁-381 | -CH₂C(=O)NHCH₂CF₂CF₂H | R₂-381 | -CH₂C(=O)NHCH₂CF₂CF₂H |
| R₁-382 | -CH₂C(=O)NHCH₂CF₂CF₃ | R₂-382 | -CH₂C(=O)NHCH₂CF₂CF₃ |
| R₁-383 | -CH₂C(=O)NHCF₂CF₂CF₃ | R₂-383 | -CH₂C(=O)NHCF₂CF₂CF₃ |
| R₁-384 | -CH₂C(=O)NHCH₂CF₂CF₂CF₃ | R₂-384 | -CH₂C(=O)NHCH₂CF₂CF₂CF₃ |
| R₁-385 | -CH₂C(=O)NHCF₂CF₂CF₂CF₃ | R₂-385 | -CH₂C(=O)NHCF₂CF₂CF₂CF₃ |
| R₁-386 | -CH₂C(=O)NHCH₂OMe | R₂-386 | -CH₂C(=O)NHCH₂OMe |
| R₁-387 | -CH₂C(=O)NHCH₂OCH₂CH₃ | R₂-387 | -CH₂C(=O)NHCH₂OCH₂CH₃ |
| R₁-388 | -CH₂C(=O)NHCH₂C≡N | R₂-388 | -CH₂C(=O)NHCH₂C≡N |
| R₁-389 | -CH₂C(=O)NHCH₂CH₂C≡N | R₂-389 | -CH₂C(=O)NHCH₂CH₂C≡N |
| R₁-390 | -CH₂C(=O)NHPh | R₂-390 | -CH₂C(=O)NHPh |
| R₁-391 | -C(=O)NH₂ | R₂-391 | -C(=O)NH₂ |
| R₁-392 | -C(=O)NHMe | R₂-392 | -C(=O)NHMe |
| R₁-393 | -C(=O)NHEt | R₂-393 | -C(=O)NHEt |
| R₁-394 | -C(=O)NHPr | R₂-394 | -C(=O)NHPr |
| R₁-395 | -C(=O)NHi-Pr | R₂-395 | -C(=O)NHi-Pr |
| R₁-396 | -C(=O)NHBu | R₂-396 | -C(=O)NHBu |
| R₁-397 | -C(=O)NHi-Bu | R₂-397 | -C(=O)NHi-Bu |
| R₁-398 | -C(=O)NHsec-Bu | R₂-398 | -C(=O)NHsec-Bu |
| R₁-399 | -C(=O)NHt-Bu | R₂-399 | -C(=O)NHt-Bu |
| R₁-400 | -C(=O)NHc-Pr | R₂-400 | -C(=O)NHc-Pr |
| R₁-401 | -C(=O)NHc-Bu | R₂-401 | -C(=O)NHc-Bu |
| R₁-402 | -C(=O)NHCHF₂ | R₂-402 | -C(=O)NHCHF₂ |
| R₁-403 | -C(=O)NHCF₃ | R₂-403 | -C(=O)NHCF₃ |
| R₁-404 | -C(=O)NHCH₂CHF₂ | R₂-404 | -C(=O)NHCH₂CHF₂ |
| R₁-405 | -C(=O)NHCH₂CF₂H | R₂-405 | -C(=O)NHCH₂CF₂H |
| R₁-406 | -C(=O)NHCH₂CF₃ | R₂-406 | -C(=O)NHCH₂CF₃ |
| R₁-407 | -C(=O)NHCH₂CH₂CF₂H | R₂-407 | -C(=O)NHCH₂CH₂CF₂H |
| R₁-408 | -C(=O)NHCH₂CH₂CF₃ | R₂-408 | -C(=O)NHCH₂CH₂CF₃ |
| R₁-409 | -C(=O)NHCH₂CH₂CH₂CF₂H | R₂-409 | -C(=O)NHCH₂CH₂CH₂CF₂H |
| R₁-410 | -C(=O)NHCH₂CH₂CH₂CF₃ | R₂-410 | -C(=O)NHCH₂CH₂CH₂CF₃ |
| R₁-411 | -C(=O)NHCF₂CF₂H | R₂-411 | -C(=O)NHCF₂CF₂H |
| R₁-412 | -C(=O)NHCF₂CF₃ | R₂-412 | -C(=O)NHCF₂CF₃ |
| R₁-413 | -C(=O)NHCHFCF₃ | R₂-413 | -C(=O)NHCHFCF₃ |
| R₁-414 | -C(=O)NHCH₂CF₂CF₂H | R₂-414 | -C(=O)NHCH₂CF₂CF₂H |
| R₁-415 | -C(=O)NHCH₂CF₂CF₃ | R₂-415 | -C(=O)NHCH₂CF₂CF₃ |
| R₁-416 | -C(=O)NHCF₂CF₂CF₃ | R₂-416 | -C(=O)NHCF₂CF₂CF₃ |
| R₁-417 | -C(=O)NHCH₂CF₂CF₂CF₃ | R₂-417 | -C(=O)NHCH₂CF₂CF₂CF₃ |
| R₁-418 | -C(=O)NHCF₂CF₂CF₂CF₃ | R₂-418 | -C(=O)NHCF₂CF₂CF₂CF₃ |
| R₁-419 | -C(=O)NHCH₂OMe | R₂-419 | -C(=O)NHCH₂OMe |
| R₁-420 | -C(=O)NHCH₂OCH₂CH₃ | R₂-420 | -C(=O)NHCH₂OCH₂CH₃ |
| R₁-421 | -C(=O)NHCH₂C≡N | R₂-421 | -C(=O)NHCH₂C≡N |
| R₁-422 | -C(=O)NHCH₂CH₂C≡N | R₂-422 | -C(=O)NHCH₂CH₂C≡N |
| R₁-423 | -C(=O)NHPh | R₂-423 | -C(=O)NHPh |
| R₁-424 | -CH₂-Ph | R₂-424 | -CH₂-Ph |
| R₁-425 | -CH₂-2-F-Ph | R₂-425 | -CH₂-2-F-Ph |
| R₁-426 | -CH₂-3-F-Ph | R₂-426 | -CH₂-3-F-Ph |
| R₁-427 | -CH₂-4-F-Ph | R₂-427 | -CH₂-4-F-Ph |
| R₁-428 | -CH₂-2-Cl-Ph | R₂-428 | -CH₂-2-Cl-Ph |
| R₁-429 | -CH₂-3-Cl-Ph | R₂-429 | -CH₂-3-Cl-Ph |
| R₁-430 | -CH₂-4-Cl-Ph | R₂-430 | -CH₂-4-Cl-Ph |
| R₁-431 | -CH₂-2-Br-Ph | R₂-431 | -CH₂-2-Br-Ph |
| R₁-432 | -CH₂-3-Br-Ph | R₂-432 | -CH₂-3-Br-Ph |
| R₁-433 | -CH₂-4-Br-Ph | R₂-433 | -CH₂-4-Br-Ph |
| R₁-434 | -CH₂-2-I-Ph | R₂-434 | -CH₂-2-I-Ph |
| R₁-435 | -CH₂-3-I-Ph | R₂-435 | -CH₂-3-I-Ph |
| R₁-436 | -CH₂-4-I-Ph | R₂-436 | -CH₂-4-I-Ph |
| R₁-437 | -CH₂-2-N≡C-Ph | R₂-437 | -CH₂-2-N≡C-Ph |
| R₁-438 | -CH₂-3-N≡C-Ph | R₂-438 | -CH₂-3-N≡C-Ph |
| R₁-439 | -CH₂-4-N≡C-Ph | R₂-439 | -CH₂-4-N≡C-Ph |
| R₁-440 | -CH₂-2-Me-Ph | R₂-440 | -CH₂-2-Me-Ph |
| R₁-441 | -CH₂-3-Me-Ph | R₂-441 | -CH₂-3-Me-Ph |
| R₁-442 | -CH₂-4-Me-Ph | R₂-442 | -CH₂-4-Me-Ph |
| R₁-443 | -CH₂-2-Et-Ph | R₂-443 | -CH₂-2-Et-Ph |
| R₁-444 | -CH₂-3-Et-Ph | R₂-444 | -CH₂-3-Et-Ph |
| R₁-445 | -CH₂-4-Et-Ph | R₂-445 | -CH₂-4-Et-Ph |
| R₁-446 | -CH₂-2-Pr-Ph | R₂-446 | -CH₂-2-Pr-Ph |
| R₁-447 | -CH₂-3-Pr-Ph | R₂-447 | -CH₂-3-Pr-Ph |
| R₁-448 | -CH₂-4-Pr-Ph | R₂-448 | -CH₂-4-Pr-Ph |
| R₁-449 | -CH₂-2-i-Pr-Ph | R₂-449 | -CH₂-2-i-Pr-Ph |
| R₁-450 | -CH₂-3-i-Pr-Ph | R₂-450 | -CH₂-3-i-Pr-Ph |
| R₁-451 | -CH₂-4-i-Pr-Ph | R₂-451 | -CH₂-4-i-Pr-Ph |
| R₁-452 | -CH₂-2-CF₂H-Ph | R₂-452 | -CH₂-2-CF₂H-Ph |
| R₁-453 | -CH₂-3-CF₂H-Ph | R₂-453 | -CH₂-3-CF₂H-Ph |
| R₁-454 | -CH₂-4-CF₂H-Ph | R₂-454 | -CH₂-4-CF₂H-Ph |
| R₁-455 | -CH₂-2-CF₃-Ph | R₂-455 | -CH₂-2-CF₃-Ph |
| R₁-456 | -CH₂-3-CF₃-Ph | R₂-456 | -CH₂-3-CF₃-Ph |
| R₁-457 | -CH₂-4-CF₃-Ph | R₂-457 | -CH₂-4-CF₃-Ph |
| R₁-458 | -CH₂-2-c-Pr-Ph | R₂-458 | -CH₂-2-c-Pr-Ph |
| R₁-459 | -CH₂-3-c-Pr-Ph | R₂-459 | -CH₂-3-c-Pr-Ph |
| R₁-460 | -CH₂-4-c-Pr-Ph | R₂-460 | -CH₂-4-c-Pr-Ph |
| R₁-461 | -CH₂-2-OMe-Ph | R₂-461 | -CH₂-2-OMe-Ph |
| R₁-462 | -CH₂-3-OMe-Ph | R₂-462 | -CH₂-3-OMe-Ph |
| R₁-463 | -CH₂-4-OMe-Ph | R₂-463 | -CH₂-4-OMe-Ph |
| R₁-464 | -CH₂-2-OEt-Ph | R₂-464 | -CH₂-2-OEt-Ph |
| R₁-465 | -CH₂-3-OEt-Ph | R₂-465 | -CH₂-3-OEt-Ph |
| R₁-466 | -CH₂-4-OEt-Ph | R₂-466 | -CH₂-4-OEt-Ph |
| R₁-467 | -CH₂-2-OCF₂H-Ph | R₂-467 | -CH₂-2-OCF₂H-Ph |
| R₁-468 | -CH₂-3-OCF₂H-Ph | R₂-468 | -CH₂-3-OCF₂H-Ph |
| R₁-469 | -CH₂-4-OCF₂H-Ph | R₂-469 | -CH₂-4-OCF₂H-Ph |
| R₁-470 | -CH₂-2-OCF₃-Ph | R₂-470 | -CH₂-2-OCF₃-Ph |
| R₁-471 | -CH₂-3-OCF₃-Ph | R₂-471 | -CH₂-3-OCF₃-Ph |
| R₁-472 | -CH₂-4-OCF₃-Ph | R₂-472 | -CH₂-4-OCF₃-Ph |
| R₁-473 | -CH₂-2-SMe-Ph | R₂-473 | -CH₂-2-SMe-Ph |
| R₁-474 | -CH₂-3-SMe-Ph | R₂-474 | -CH₂-3-SMe-Ph |
| R₁-475 | -CH₂-4-SMe-Ph | R₂-475 | -CH₂-4-SMe-Ph |
| R₁-476 | -CH₂-2-S(=O)Me-Ph | R₂-476 | -CH₂-2-S(=O)Me-Ph |
| R₁-477 | -CH₂-3-S(=O)Me-Ph | R₂-477 | -CH₂-3-S(=O)Me-Ph |
| R₁-478 | -CH₂-4-S(=O)Me-Ph | R₂-478 | -CH₂-4-S(=O)Me-Ph |
| R₁-479 | -CH₂-2-SO₂Me-Ph | R₂-479 | -CH₂-2-SO₂Me-Ph |
| R₁-480 | -CH₂-3-SO₂Me-Ph | R₂-480 | -CH₂-3-SO₂Me-Ph |
| R₁-481 | -CH₂-4-SO₂Me-Ph | R₂-481 | -CH₂-4-SO₂Me-Ph |
| R₁-482 | -CH₂-2-SCF₃-Ph | R₂-482 | -CH₂-2-SCF₃-Ph |
| R₁-483 | -CH₂-3-SCF₃-Ph | R₂-483 | -CH₂-3-SCF₃-Ph |
| R₁-484 | -CH₂-4-SCF₃-Ph | R₂-484 | -CH₂-4-SCF₃-Ph |
| R₁-485 | -CH₂-2-S(=O)CF₃-Ph | R₂-485 | -CH₂-2-S(=O)CF₃-Ph |
| R₁-486 | -CH₂-3-S(=O)CF₃-Ph | R₂-486 | -CH₂-3-S(=O)CF₃-Ph |
| R₁-487 | -CH₂-4-S(=O)CF₃-Ph | R₂-487 | -CH₂-4-S(=O)CF₃-Ph |
| R₁-488 | -CH₂-2-SO₂CF₃-Ph | R₂-488 | -CH₂-2-SO₂CF₃-Ph |
| R₁-489 | -CH₂-3-SO₂CF₃-Ph | R₂-489 | -CH₂-3-SO₂CF₃-Ph |
| R₁-490 | -CH₂-4-SO₂CF₃-Ph | R₂-490 | -CH₂-4-SO₂CF₃-Ph |
| R₁-491 | -CH₂CH₂-Ph | R₂-491 | -CH₂CH₂-Ph |
| R₁-492 | -CH₂CH₂-2-F-Ph | R₂-492 | -CH₂CH₂-2-F-Ph |
| R₁-493 | -CH₂CH₂-3-F-Ph | R₂-493 | -CH₂CH₂-3-F-Ph |
| R₁-494 | -CH₂CH₂-4-F-Ph | R₂-494 | -CH₂CH₂-4-F-Ph |
| R₁-495 | -CH₂CH₂-2-Cl-Ph | R₂-495 | -CH₂CH₂-2-Cl-Ph |
| R₁-496 | -CH₂CH₂-3-Cl-Ph | R₂-496 | -CH₂CH₂-3-Cl-Ph |
| R₁-497 | -CH₂CH₂-4-Cl-Ph | R₂-497 | -CH₂CH₂-4-Cl-Ph |
| R₁-498 | -CH₂CH₂-2-Br-Ph | R₂-498 | -CH₂CH₂-2-Br-Ph |
| R₁-499 | -CH₂CH₂-3-Br-Ph | R₂-499 | -CH₂CH₂-3-Br-Ph |
| R₁-500 | -CH₂CH₂-4-Br-Ph | R₂-500 | -CH₂CH₂-4-Br-Ph |
| R₁-501 | -CH₂CH₂-2-I-Ph | R₂-501 | -CH₂CH₂-2-I-Ph |
| R₁-502 | -CH₂CH₂-3-I-Ph | R₂-502 | -CH₂CH₂-3-I-Ph |
| R₁-503 | -CH₂CH₂-4-I-Ph | R₂-503 | -CH₂CH₂-4-I-Ph |
| R₁-504 | -CH₂CH₂-2-N≡C-Ph | R₂-504 | -CH₂CH₂-2-N≡C-Ph |
| R₁-505 | -CH₂CH₂-3-N≡C-Ph | R₂-505 | -CH₂CH₂-3-N≡C-Ph |
| R₁-506 | -CH₂CH₂-4-N≡C-Ph | R₂-506 | -CH₂CH₂-4-N≡C-Ph |
| R₁-507 | -CH₂CH₂-2-Me-Ph | R₂-507 | -CH₂CH₂-2-Me-Ph |
| R₁-508 | -CH₂CH₂-3-Me-Ph | R₂-508 | -CH₂CH₂-3-Me-Ph |
| R₁-509 | -CH₂CH₂-4-Me-Ph | R₂-509 | -CH₂CH₂-4-Me-Ph |
| R₁-510 | -CH₂CH₂-2-Et-Ph | R₂-510 | -CH₂CH₂-2-Et-Ph |
| R₁-511 | -CH₂CH₂-3-Et-Ph | R₂-511 | -CH₂CH₂-3-Et-Ph |
| R₁-512 | -CH₂CH₂-4-Et-Ph | R₂-512 | -CH₂CH₂-4-Et-Ph |
| R₁-513 | -CH₂CH₂-2-Pr-Ph | R₂-513 | -CH₂CH₂-2-Pr-Ph |
| R₁-514 | -CH₂CH₂-3-Pr-Ph | R₂-514 | -CH₂CH₂-3-Pr-Ph |
| R₁-515 | -CH₂CH₂-4-Pr-Ph | R₂-515 | -CH₂CH₂-4-Pr-Ph |
| R₁-516 | -CH₂CH₂-2-i-Pr-Ph | R₂-516 | -CH₂CH₂-2-i-Pr-Ph |
| R₁-517 | -CH₂CH₂-3-i-Pr-Ph | R₂-517 | -CH₂CH₂-3-i-Pr-Ph |
| R₁-518 | -CH₂CH₂-4-i-Pr-Ph | R₂-518 | -CH₂CH₂-4-i-Pr-Ph |
| R₁-519 | -CH₂CH₂-2-CF₂H-Ph | R₂-519 | -CH₂CH₂-2-CF₂H-Ph |
| R₁-520 | -CH₂CH₂-3-CF₂H-Ph | R₂-520 | -CH₂CH₂-3-CF₂H-Ph |
| R₁-521 | -CH₂CH₂-4-CF₂H-Ph | R₂-521 | -CH₂CH₂-4-CF₂H-Ph |
| R₁-522 | -CH₂CH₂-2-CF₃-Ph | R₂-522 | -CH₂CH₂-2-CF₃-Ph |
| R₁-523 | -CH₂CH₂-3-CF₃-Ph | R₂-523 | -CH₂CH₂-3-CF₃-Ph |
| R₁-524 | -CH₂CH₂-4-CF₃-Ph | R₂-524 | -CH₂CH₂-4-CF₃-Ph |
| R₁-525 | -CH₂CH₂-2-c-Pr-Ph | R₂-525 | -CH₂CH₂-2-c-Pr-Ph |
| R₁-526 | -CH₂CH₂-3-c-Pr-Ph | R₂-526 | -CH₂CH₂-3-c-Pr-Ph |
| R₁-527 | -CH₂CH₂-4-c-Pr-Ph | R₂-527 | -CH₂CH₂-4-c-Pr-Ph |
| R₁-528 | -CH₂CH₂-2-OMe-Ph | R₂-528 | -CH₂CH₂-2-OMe-Ph |
| R₁-529 | -CH₂CH₂-3-OMe-Ph | R₂-529 | -CH₂CH₂-3-OMe-Ph |
| R₁-530 | -CH₂CH₂-4-OMe-Ph | R₂-530 | -CH₂CH₂-4-OMe-Ph |
| R₁-531 | -CH₂CH₂-2-OEt-Ph | R₂-531 | -CH₂CH₂-2-OEt-Ph |
| R₁-532 | -CH₂CH₂-3-OEt-Ph | R₂-532 | -CH₂CH₂-3-OEt-Ph |
| R₁-533 | -CH₂CH₂-4-OEt-Ph | R₂-533 | -CH₂CH₂-4-OEt-Ph |
| R₁-534 | -CH₂CH₂-2-OCF₂H-Ph | R₂-534 | -CH₂CH₂-2-OCF₂H-Ph |
| R₁-535 | -CH₂CH₂-3-OCF₂H-Ph | R₂-535 | -CH₂CH₂-3-OCF₂H-Ph |
| R₁-536 | -CH₂CH₂-4-OCF₂H-Ph | R₂-536 | -CH₂CH₂-4-OCF₂H-Ph |
| R₁-537 | -CH₂CH₂-2-OCF₃-Ph | R₂-537 | -CH₂CH₂-2-OCF₃-Ph |
| R₁-538 | -CH₂CH₂-3-OCF₃-Ph | R₂-538 | -CH₂CH₂-3-OCF₃-Ph |
| R₁-539 | -CH₂CH₂-4-OCF₃-Ph | R₂-539 | -CH₂CH₂-4-OCF₃-Ph |
| R₁-540 | -CH₂CH₂-2-SMe-Ph | R₂-540 | -CH₂CH₂-2-SMe-Ph |
| R₁-541 | -CH₂CH₂-3-SMe-Ph | R₂-541 | -CH₂CH₂-3-SMe-Ph |
| R₁-542 | -CH₂CH₂-4-SMe-Ph | R₂-542 | -CH₂CH₂-4-SMe-Ph |
| R₁-543 | -CH₂CH₂-2-S(=O)Me-Ph | R₂-543 | -CH₂CH₂-2-S(=O)Me-Ph |
| R₁-544 | -CH₂CH₂-3-S(=O)Me-Ph | R₂-544 | -CH₂CH₂-3-S(=O)Me-Ph |
| R₁-545 | -CH₂CH₂-4-S(=O)Me-Ph | R₂-545 | -CH₂CH₂-4-S(=O)Me-Ph |
| R₁-546 | -CH₂CH₂-2-SO₂Me-Ph | R₂-546 | -CH₂CH₂-2-SO₂Me-Ph |
| R₁-547 | -CH₂CH₂-3-SO₂Me-Ph | R₂-547 | -CH₂CH₂-3-SO₂Me-Ph |
| R₁-548 | -CH₂CH₂-4-SO₂Me-Ph | R₂-548 | -CH₂CH₂-4-SO₂Me-Ph |
| R₁-549 | -CH₂CH₂-2-SCF₃-Ph | R₂-549 | -CH₂CH₂-2-SCF₃-Ph |
| R₁-550 | -CH₂CH₂-3-SCF₃-Ph | R₂-550 | -CH₂CH₂-3-SCF₃-Ph |
| R₁-551 | -CH₂CH₂-4-SCF₃-Ph | R₂-551 | -CH₂CH₂-4-SCF₃-Ph |
| R₁-552 | -CH₂CH₂-2-S(=O)CF₃-Ph | R₂-552 | -CH₂CH₂-2-S(=O)CF₃-Ph |
| R₁-553 | -CH₂CH₂-3-S(=O)CF₃-Ph | R₂-553 | -CH₂CH₂-3-S(=O)CF₃-Ph |
| R₁-554 | -CH₂CH₂-4-S(=O)CF₃-Ph | R₂-554 | -CH₂CH₂-4-S(=O)CF₃-Ph |
| R₁-555 | -CH₂CH₂-2-SO₂CF₃-Ph | R₂-555 | -CH₂CH₂-2-SO₂CF₃-Ph |
| R₁-556 | -CH₂CH₂-3-SO₂CF₃-Ph | R₂-556 | -CH₂CH₂-3-SO₂CF₃-Ph |
| R₁-557 | -CH₂CH₂-4-SO₂CF₃-Ph | R₂-557 | -CH₂CH₂-4-SO₂CF₃-Ph |
| R₁,R₂-558 | -CH₂CH₂- | | |
| R₁,R₂-559 | -CH₂CH₂CH₂- | | |
| R₁,R₂-560 | -CH₂CH₂CH₂CH₂- | | |
| R₁,R₂-561 | -CH₂CH₂CH₂CH₂CH₂- | | |

**Table 2:**

| No. | Substituent examples of R₃ | No. | Substituent examples of R₄ |
|---|---|---|---|
| R₃-1 | -H | R₄-1 | -H |
| R₃-2 | -CN | R₄-2 | -CN |
| R₃-3 | -NO2 | R₄-3 | -NO2 |
| R₃-4 | -F | R₄-4 | -F |
| R₃-5 | -Cl | R₄-5 | -Cl |
| R₃-6 | -Br | R₄-6 | -Br |
| R₃-7 | -I | R₄-7 | -I |
| R₃-8 | -Me | R₄-8 | -Me |
| R₃-9 | -Et | R₄-9 | -Et |
| R₃-10 | -Pr | R₄-10 | -Pr |
| R₃-11 | -i-Pr | R₄-11 | -i-Pr |
| R₃-12 | -CFH₂ | R₄-12 | -CFH₂ |
| R₃-13 | -CF₂H | R₄-13 | -CF₂H |
| R₃-14 | -CF₃ | R₄-14 | -CF₃ |
| R₃-15 | -CClH₂ | R₄-15 | -CClH₂ |
| R₃-16 | -CCl₂H | R₄-16 | -CCl₂H |
| R₃-17 | -CCl₃ | R₄-17 | -CCl₃ |
| R₃-18 | -CBrH₂ | R₄-18 | -CBrH₂ |
| R₃-19 | -CBr₂H | R₄-19 | -CBr₂H |
| R₃-20 | -CBr₃ | R₄-20 | -CBr₃ |
| R₃-21 | -CH₂I | R₄-21 | -CH₂I |
| R₃-22 | -CHI₂ | R₄-22 | -CHI₂ |
| R₃-23 | -CI₃ | R₄-23 | -CI₃ |
| R₃-24 | -CH₂CF₂H | R₄-24 | -CH₂CF₂H |
| R₃-25 | -CH₂CF₃ | R₄-25 | -CH₂CF₃ |
| R₃-26 | -CF₂CF₂H | R₄-26 | -CF₂CF₂H |
| R₃-27 | -CF₂CF₃ | R₄-27 | -CF₂CF₃ |
| R₃-28 | -CHFCF₃ | R₄-28 | -CHFCF₃ |
| R₃-29 | -c-Pr | R₄-29 | -c-Pr |
| R₃-30 | -c-Bu | R₄-30 | -c-Bu |
| R₃-31 | -CH=CH₂ | R₄-31 | -CH=CH₂ |
| R₃-32 | -CH₂CH=CH₂ | R₄-32 | -CH₂CH=CH₂ |
| R₃-33 | -C(CH₃)=CH₂ | R₄-33 | -C(CH₃)=CH₂ |
| R₃-34 | -CH=CHCH₃ | R₄-34 | -CH=CHCH₃ |
| R₃-35 | -CH=CFH | R₄-35 | -CH=CFH |
| R₃-36 | -CH=CF₂ | R₄-36 | -CH=CF₂ |
| R₃-37 | -CH=CCl₂ | R₄-37 | -CH=CCl₂ |
| R₃-38 | -C≡CH | R₄-38 | -C=CH |
| R₃-39 | -C≡CCH₃ | R₄-39 | -C=CCH₃ |
| R₃-40 | -CH₂C≡CH | R₄-40 | -CH₂C≡CH |
| R₃-41 | -OH | R₄-41 | -OH |
| R₃-42 | -OMe | R₄-42 | -OMe |
| R₃-43 | -OEt | R₄-43 | -OEt |
| R₃-44 | -OPr | R₄-44 | -OPr |
| R₃-45 | -Oi-Pr | R₄-45 | -Oi-Pr |
| R₃-46 | -OCFH₂ | R₄-46 | -OCFH₂ |
| R₃-47 | -OCF₂H | R₄-47 | -OCF₂H |
| R₃-48 | -OCF₃ | R₄-48 | -OCF₃ |
| R₃-49 | -OCClH₂ | R₄-49 | -OCClH₂ |
| R₃-50 | -OCCl₂H | R₄-50 | -OCCl₂H |
| R₃-51 | -OCCl₃ | R₄-51 | -OCCl₃ |
| R₃-52 | -OCBrH₂ | R₄-52 | -OCBrH₂ |
| R₃-53 | -OCBr₂H | R₄-53 | -OCBr₂H |
| R₃-54 | -OCBr₃ | R₄-54 | -OCBr₃ |
| R₃-55 | -OCH₂I | R₄-55 | -OCH₂I |
| R₃-56 | -OCHI₂ | R₄-56 | -OCHI₂ |
| R₃-57 | -OCI₃ | R₄-57 | -OCI₃ |
| R₃-58 | -OCH₂CF₂H | R₄-58 | -OCH₂CF₂H |
| R₃-59 | -OCH₂CF₃ | R₄-59 | -OCH₂CF₃ |
| R₃-60 | -OCF₂CF₂H | R₄-60 | -OCF₂CF₂H |
| R₃-61 | -OCF₂CF₃ | R₄-61 | -OCF₂CF₃ |
| R₃-62 | -OCHFCF₃ | R₄-62 | -OCHFCF₃ |
| R₃-63 | -Oc-Pr | R₄-63 | -Oc-Pr |
| R₃-64 | -Oc-Bu | R₄-64 | -Oc-Bu |
| R₃-65 | -OCH=CH₂ | R₄-65 | -OCH=CH₂ |
| R₃-66 | -OCH₂CH=CH₂ | R₄-66 | -OCH₂CH=CH₂ |
| R₃-67 | -OCH=CHCH₃ | R₄-67 | -OCH=CHCH₃ |
| R₃-68 | -OCH=CFH | R₄-68 | -OCH=CFH |
| R₃-69 | -OCH=CF₂ | R₄-69 | -OCH=CF₂ |
| R₃-70 | -OCH=CCl₂ | R₄-70 | -OCH=CCl₂ |
| R₃-71 | -OC≡CH | R₄-71 | -OC≡CH |
| R₃-72 | -OC≡CCH₃ | R₄-72 | -OC≡CCH₃ |
| R₃-73 | -OCH₂C≡CH | R₄-73 | -OCH₂C≡CH |
| R₃-74, R₄-74 | -CH₂CH₂CH₂- | | |
| R₃-75, R₄-75 | -CH₂CH₂CH₂CH₂- | | |

**Table 3:**

| No. | Substituent examples of R₅ |
|---|---|
| R₅-1 | -H |
| R₅-2 | -F |
| R₅-3 | -Cl |
| R₅-4 | -Br |
| R₅-5 | -I |
| R₅-6 | -Me |
| R₅-7 | -Et |
| R₅-8 | -Pr |
| R₅-9 | -i-Pr |
| R₅-10 | -CFH₂ |
| R₅-11 | -CF₂H |
| R₅-12 | -CF₃ |
| R₅-13 | -CClH₂ |
| R₅-14 | -CCl₂H |
| R₅-15 | -CCl₃ |
| R₅-16 | -CBrH₂ |
| R₅-17 | -CBr₂H |
| R₅-18 | -CBr₃ |
| R₅-19 | -CH₂I |
| R₅-20 | -CHI₂ |
| R₅-21 | -CI₃ |
| R₅-22 | -CH₂CF₂H |
| R₅-23 | -CH₂CF₃ |
| R₅-24 | -CF₂CF₂H |
| R₅-25 | -CF₂CF₃ |
| R₅-26 | -CHFCF₃ |
| R₅-27 | -c-Pr |
| R₅-28 | -c-Bu |
| R₅-29 | -CH=CH₂ |
| R₅-30 | -CH₂CH=CH₂ |
| R₅-31 | -C(CH₃)=CH₂ |
| R₅-32 | -CH=CHCH₃ |
| R₅-33 | -CH=CFH |
| R₅-34 | -CH=CF₂ |
| R₅-35 | -CH=CCl₂ |
| R₅-36 | -C≡CH |
| R₅-37 | -C≡CCH₃ |
| R₅-38 | -CH₂C≡CH |
| R₅-39 | -OH |
| R₅-40 | -OMe |
| R₅-41 | -OEt |
| R₅-42 | -OPr |
| R₅-43 | -Oi-Pr |
| R₅-44 | -OCFH₂ |
| R₅-45 | -OCF₂H |
| R₅-46 | -OCF₃ |
| R₅-47 | -OCClH₂ |
| R₅-48 | -OCCl₂H |
| R₅-49 | -OCCl₃ |
| R₅-50 | -OCBrH₂ |
| R₅-51 | -OCBr₂H |
| R₅-52 | -OCBr₃ |
| R₅-53 | -OCH₂I |
| R₅-54 | -OCHI₂ |
| R₅-55 | -OCI₃ |
| R₅-56 | -OCH₂CF₂H |
| R₅-57 | -OCH₂CF₃ |
| R₅-58 | -OCF₂CF₂H |
| R₅-59 | -OCF₂CF₃ |
| R₅-60 | -OCHFCF₃ |
| R₅-61 | -Oc-Pr |
| R₅-62 | -Oc-Bu |
| R₅-63 | -OCH=CH₂ |
| R₅-64 | -OCH₂CH=CH₂ |
| R₅-65 | -OCH=CHCH₃ |
| R₅-66 | -OCH=CFH |
| R₅-67 | -OCH=CF₂ |
| R₅-68 | -OCH=CCl₂ |
| R₅-69 | -OC≡CH |
| R₅-70 | -OC≡CCH₃ |
| R₅-71 | -OCH₂C≡CH |
| R₅-72 | -NH₂ |
| R₅-73 | -NHCH₃ |
| R₅-74 | -NHCH₂CH₃ |
| R₅-75 | -NHCH₂CH₂CH₃ |
| R₅-76 | -NHCH(CH₃)CH₃ |
| R₅-77 | -N(CH₃)₂ |
| R₅-78 | -N(CH₃)CH₂CH₃ |
| R₅-79 | -N(CH₃)CH₂CH₂CH₃ |
| R₅-80 | -N(CH₃)CH(CH₃)CH₃ |
| R₅-81 | -N(CH₂CH₃)₂ |

**Table 4:**

| No. | Substituent examples of R₆ |
|---|---|
| R₆-1 | -F |
| R₆-2 | -Cl |
| R₆-3 | -Br |
| R₆-4 | -I |
| R₆-5 | -Me |
| R₆-6 | -Et |
| R₆-7 | -Pr |
| R₆-8 | -i-Pr |
| R₆-9 | -CFH₂ |
| R₆-10 | -CF₂H |
| R₆-11 | -CF₃ |
| R₆-12 | -CClH₂ |
| R₆-13 | -CCl₂H |
| R₆-14 | -CCl₃ |
| R₆-15 | -CBrH₂ |
| R₆-16 | -CBr₂H |
| R₆-17 | -CBr₃ |
| R₆-18 | -CH₂I |
| R₆-19 | -CHI₂ |
| R₆-20 | -CI₃ |
| R₆-21 | -CH₂CF₂H |
| R₆-22 | -CH₂CF₃ |
| R₆-23 | -CF₂CF₂H |
| R₆-24 | -CF₂CF₃ |
| R₆-25 | -CHFCF₃ |
| R₆-26 | -c-Pr |
| R₆-27 | -c-Bu |
| R₆-28 | -CH=CH₂ |
| R₆-29 | -CH₂CH=CH₂ |
| R₆-30 | -C(CH₃)=CH₂ |
| R₆-31 | -CH=CHCH₃ |
| R₆-32 | -CH=CFH |
| R₆-33 | -CH=CF₂ |
| R₆-34 | -CH=CCl₂ |
| R₆-35 | -C≡CH |
| R₆-36 | -C≡CCH₃ |
| R₆-37 | -CH₂C≡CH |
| R₆-38 | -OH |
| R₆-39 | -OMe |
| R₆-40 | -OEt |
| R₆-41 | -OPr |
| R₆-42 | -Oi-Pr |
| R₆-43 | -OCFH₂ |
| R₆-44 | -OCF₂H |
| R₆-45 | -OCF₃ |
| R₆-46 | -OCClH₂ |
| R₆-47 | -OCCl₂H |
| R₆-48 | -OCCl₃ |
| R₆-49 | -OCBrH₂ |
| R₆-50 | -OCBr₂H |
| R₆-51 | -OCBr₃ |
| R₆-52 | -OCH₂I |
| R₆-53 | -OCHI₂ |
| R₆-54 | -OCI₃ |
| R₆-55 | -OCH₂CF₂H |
| R₆-56 | -OCH₂CF₃ |
| R₆-57 | -OCF₂CF₂H |
| R₆-58 | -OCF₂CF₃ |
| R₆-59 | -OCHFCF₃ |
| R₆-60 | -Oc-Pr |
| R₆-61 | -Oc-Bu |
| R₆-62 | -OCH=CH₂ |
| R₆-63 | -OCH₂CH=CH₂ |
| R₆-64 | -OCH=CHCH₃ |
| R₆-65 | -OCH=CFH |
| R₆-66 | -OCH=CF₂ |
| R₆-67 | -OCH=CCl₂ |
| R₆-68 | -OC≡CH |
| R₆-69 | -OC≡CCH₃ |
| R₆-70 | -OCH₂C≡CH |
| R₆-71 | -NH₂ |
| R₆-72 | -NHCH₃ |
| R₆-73 | -NHCH₂CH₃ |
| R₆-74 | -NHCH₂CH₂CH₃ |
| R₆-75 | -NHCH(CH₃)CH₃ |
| R₆-76 | -N(CH₃)₂ |
| R₆-77 | -N(CH₃)CH₂CH₃ |
| R₆-78 | -N(CH₃)CH₂CH₂CH₃ |
| R₆-79 | -N(CH₃)CH(CH₃)CH₃ |
| R₆-80 | -N(CH₂CH₃)₂ |

**Table 5:**

| No. | Substituent examples of R₇ |
|---|---|
| R₇-1 | lone pair |
| R₇-2 | -H |
| R₇-3 | -Me |
| R₇-4 | -Et |
| R₇-5 | -Pr |
| R₇-6 | -CH₂CF₂H |
| R₇-7 | -CH₂CF₃ |
| R₇-8 | -CF₂CF₂H |
| R₇-9 | -CF₂CF₃ |
| R₇-10 | -CHFCF₃ |
| R₇-11 | -c-Pr |
| R₇-12 | -c-Bu |
| R₇-13 | -CH₂CH=CH₂ |
| R₇-14 | -C(CH₃)=CH₂ |
| R₇-15 | -CH₂C≡CH |
| R₇-16 | -CH₂-c-Pr |
| R₇-17 | -CH₂-c-Bu |
| R₇-18 | -CH₂OH |
| R₇-19 | -CH₂OCH₃ |
| R₇-20 | -CH₂SH |
| R₇-21 | -CH₂SCH₃ |
| R₇-22 | -CH₂S(=O)CH₃ |
| R₇-23 | -CH₂SO₂CH₃ |
| R₇-24 | -C(=O)Me |
| R₇-25 | -C(=O)Et |
| R₇-26 | -C(=O)Pr |
| R₇-27 | -C(=O)i-Pr |
| R₇-28 | -C(=O)c-Pr |
| R₇-29 | -C(=O)c-Bu |
| R₇-30 | -C(=O)CHF₂ |
| R₇-31 | -C(=O)CF₃ |
| R₇-32 | -C(=O)CH₂CHF₂ |
| R₇-33 | -C(=O)CH₂CF₂H |
| R₇-34 | -C(=O)CH₂CF₃ |
| R₇-35 | -C(=O)Ph |
| R₇-36 | -C(=O)OMe |
| R₇-37 | -C(=O)OEt |
| R₇-38 | -C(=O)OPr |
| R₇-39 | -C(=O)Oi-Pr |
| R₇-40 | -C(=O)OBu |
| R₇-41 | -C(=O)Oi-Bu |
| R₇-42 | -C(=O)Osec-Bu |
| R₇-43 | -C(=O)Ot-Bu |
| R₇-44 | -C(=O)Oc-Pr |
| R₇-45 | -C(=O)Oc-Bu |
| R₇-46 | -CH₂-2-F-Ph |
| R₇-47 | -CH₂-3-F-Ph |
| R₇-48 | -CH₂-4-F-Ph |
| R₇-49 | -CH₂-2-Cl-Ph |
| R₇-50 | -CH₂-3-Cl-Ph |
| R₇-51 | -CH₂-4-Cl-Ph |
| R₇-52 | -CH₂-2-Br-Ph |
| R₇-53 | -CH₂-3-Br-Ph |
| R₇-54 | -CH₂-4-Br-Ph |
| R₇-55 | -CH₂-2-I-Ph |
| R₇-56 | -CH₂-3-I-Ph |
| R₇-57 | -CH₂-4-I-Ph |
| R₇-58 | -CH₂-2-N≡C-Ph |
| R₇-59 | -CH₂-3-N≡C-Ph |
| R₇-60 | -CH₂-4-N≡C-Ph |
| R₇-61 | -CH₂-2-Me-Ph |
| R₇-62 | -CH₂-3-Me-Ph |
| R₇-63 | -CH₂-4-Me-Ph |
| R₇-64 | -CH₂-2-Et-Ph |
| R₇-65 | -CH₂-3-Et-Ph |
| R₇-66 | -CH₂-4-Et-Ph |
| R₇-67 | -CH₂-2-Pr-Ph |
| R₇-68 | -CH₂-3-Pr-Ph |
| R₇-69 | -CH₂-4-Pr-Ph |
| R₇-70 | -CH₂-2-i-Pr-Ph |
| R₇-71 | -CH₂-3-i-Pr-Ph |
| R₇-72 | -CH₂-4-i-Pr-Ph |
| R₇-73 | -CH₂-2-CF₂H-Ph |
| R₇-74 | -CH₂-3-CF₂H-Ph |
| R₇-75 | -CH₂-4-CF₂H-Ph |
| R₇-76 | -CH₂-2-CF₃-Ph |
| R₇-77 | -CH₂-3-CF₃-Ph |
| R₇-78 | -CH₂-4-CF₃-Ph |
| R₇-79 | -CH₂-2-c-Pr-Ph |
| R₇-80 | -CH₂-3-c-Pr-Ph |
| R₇-81 | -CH₂-4-c-Pr-Ph |
| R₇-82 | -CH₂-2-OMe-Ph |
| R₇-83 | -CH₂-3-OMe-Ph |
| R₇-84 | -CH₂-4-OMe-Ph |
| R₇-85 | -CH₂-2-OEt-Ph |
| R₇-86 | -CH₂-3-OEt-Ph |
| R₇-87 | -CH₂-4-OEt-Ph |
| R₇-88 | -CH₂-2-OCF₂H-Ph |
| R₇-89 | -CH₂-3-OCF₂H-Ph |
| R₇-90 | -CH₂-4-OCF₂H-Ph |
| R₇-91 | -CH₂-2-OCF₃-Ph |
| R₇-92 | -CH₂-3-OCF₃-Ph |
| R₇-93 | -CH₂-4-OCF₃-Ph |
| R₇-94 | -CH₂-2-SMe-Ph |
| R₇-95 | -CH₂-3-SMe-Ph |
| R₇-96 | -CH₂-4-SMe-Ph |
| R₇-97 | -CH₂-2-S(=O)Me-Ph |
| R₇-98 | -CH₂-3-S(=O)Me-Ph |
| R₇-99 | -CH₂-4-S(-O)Me-Ph |
| R₇-100 | -CH₂-2-SO₂Me-Ph |
| R₇-101 | -CH₂-3-SO₂Me-Ph |
| R₇-102 | -CH₂-4-SO₂Me-Ph |
| R₇-103 | -CH₂-2-SCF₃-Ph |
| R₇-104 | -CH₂-3-SCF₃-Ph |
| R₇-105 | -CH₂-4-SCF₃-Ph |
| R₇-106 | -CH₂-2-S(-O)CF₃-Ph |
| R₇-107 | -CH₂-3-S(-O)CF₃-Ph |
| R₇-108 | -CH₂-4-S(=O)CF₃-Ph |
| R₇-109 | -CH₂-2-SO₂CF₃-Ph |
| R₇-110 | -CH₂-3-SO₂CF₃-Ph |
| R₇-111 | -CH₂-4-SO₂CF₃-Ph |

**Table 6:**

| [Table 6-1] | | | |
|---|---|---|---|
| **Het-A1** | **Het-A2** | **Het-A3** | **Het-A4** |
| 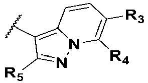 | 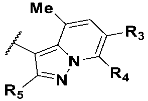 | 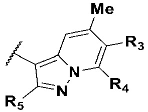 | 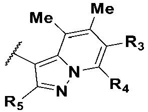 |
| **Het-A5** | **Het-A6** | **Het-A7** | **Het-A8** |
| 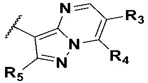 | 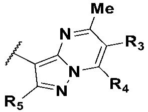 | 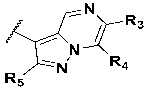 | 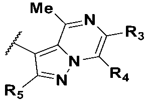 |
| **Het-A9** | **Het-A10** | **Het-A11** | **Het-A12** |
| 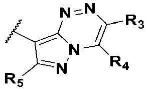 | 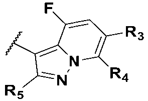 | 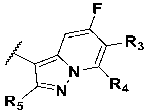 | 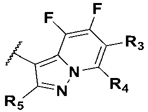 |
| **Het-A13** | **Het-A14** | **Het-A15** | **Het-A16** |
| 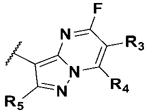 | 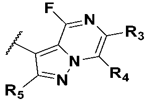 | 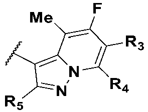 | 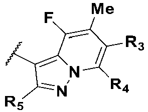 |

**[Table 6-2]**

| | | | |
|---|---|---|---|
| **Het-B1** | **Het-B2** | **Het-B3** | **Het-B4** |
| 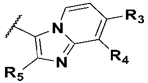 | 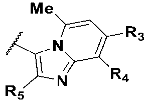 | 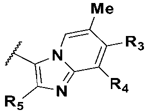 | 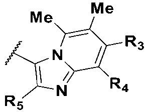 |
| **Het-B5** | **Het-B6** | **Het-B7** | **Het-B8** |
| 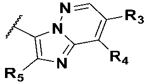 | 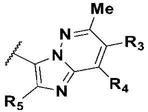 | 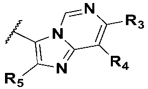 | 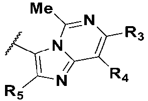 |
| **Het-B9** | **Het-B10** | **Het-B11** | **Het-B12** |
| 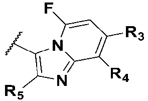 | 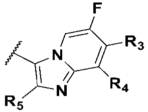 | 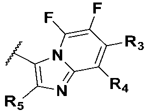 | 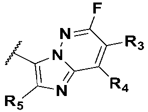 |
| **Het-B13** | **Het-B14** | **Het-B15** | |
| 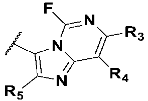 | 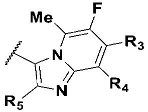 | 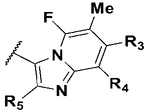 | |

**[Table 6-3]**

| | | | |
|---|---|---|---|
| **Het-C1** | **Het-C2** | **Het-C3** | **Het-C4** |
| 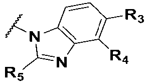 | 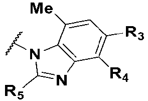 | 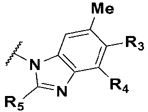 | 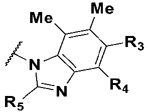 |
| **Het-C5** | **Het-C6** | **Het-C7** | **Het-C8** |
| 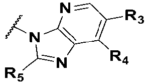 | 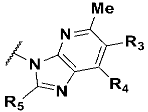 | 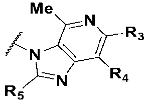 | 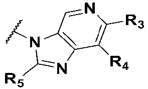 |
| **Het-C9** | **Het-C10** | **Het-C11** | **Het-C12** |
| 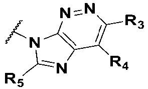 | 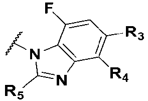 | 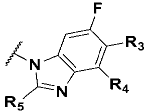 | 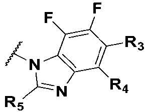 |
| **Het-C13** | **Het-C14** | **Het-C15** | **Het-C16** |
| 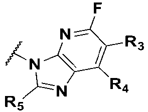 | 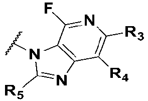 | 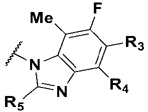 | 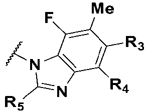 |

Hereinafter, exemplary methods for producing the compound represented by the formula (1) will be illustrated. The methods for producing the compound of the present invention are not limited to production method A to production method AH.

### [Production method A]

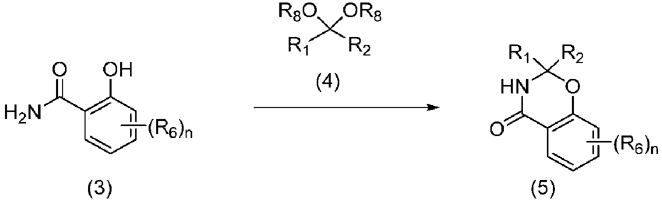

In the formulas, R₈ represents a C1 to C6 alkyl group optionally substituted by substituent A, and R₁, R₂, R₆, and n are as defined above.

The production method A is a method for obtaining a compound represented by the formula (5) and is a production method comprising reacting a compound represented by the formula (3) with a compound represented by the formula (4) in the presence of an acid.

The compound represented by the formula (3) for use in this reaction can be obtained as a commercially available product or produced by a method known in the art.

The compound represented by the formula (4) for use in this reaction can be obtained as a commercially available product or produced by a method known in the art.

The compound represented by the formula (4) for use in this reaction can be of 1 equivalent or more based on the compound represented by the formula (3). The amount is not particularly limited as long as the reaction of interest proceeds. The amount is preferably 1 equivalent or more and 200 equivalents or less. The compound of the formula (4) in a liquid state may be used as a solvent.

Examples of the acid for use in this reaction include inorganic acids such as hydrochloric acid and sulfuric acid, and organic acids such as acetic acid, methanesulfonic acid, trifluoromethanesulfonic acid, p-toluenesulfonic acid, and pyridinium p-toluenesulfonate. The acid is not particularly limited as long as the reaction of interest proceeds. The acid is preferably p-toluenesulfonic acid.

A solvent can be used, albeit not always essential, in this reaction.

The solvent for use in this reaction is not particularly limited as long as the reaction of interest proceeds. Examples thereof include acidic solvents such as acetic acid and methanesulfonic acid, ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents and benzene solvents, more preferably acetic acid and toluene.

The amount of the solvent for use in this reaction is not particularly limited as long as the reaction of interest proceeds. The amount is usually 3 or more times and 200 or less times the weight of the compound represented by the formula (3).

The temperature in performing this reaction is not particularly limited as long as the reaction of interest proceeds. The temperature is 50°C or higher and 180°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound represented by the formula (5), water can be removed, albeit not essential, with a desiccant such as sodium sulfate or magnesium sulfate.

From the thus-obtained reaction mixture containing the compound represented by the formula (5), the solvent may be distilled off under reduced pressure without degrading the compound.

The resulting reaction mixture containing the compound represented by the formula (5) from which the solvent has thus been distilled off can be purified by washing, reprecipitation, recrystallization, column chromatography, or the like using an appropriate solvent. The purification approach can be appropriately set depending on the purity of interest.

### [Production method B]

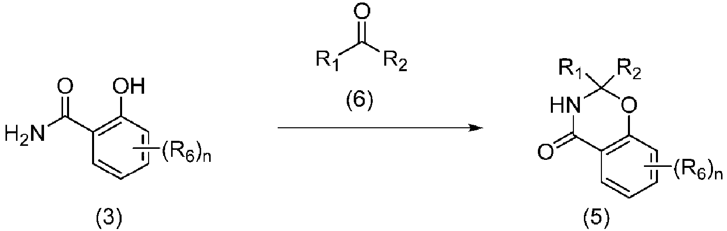

In the formulas, R₁, R₂, R₆, and n are as defined above.

The production method B is a method for obtaining a compound represented by the formula (5) and is a production method comprising reacting a compound represented by the formula (3) with a compound represented by the formula (6) in the presence of an acid.

The compound represented by the formula (6) for use in this reaction can be obtained as a commercially available product or produced by a method known in the art.

The production method B can be carried out in accordance with the production method A by using the compound represented by the formula (6) instead of the compound represented by the formula (4) in the production method A.

### [Production method C]

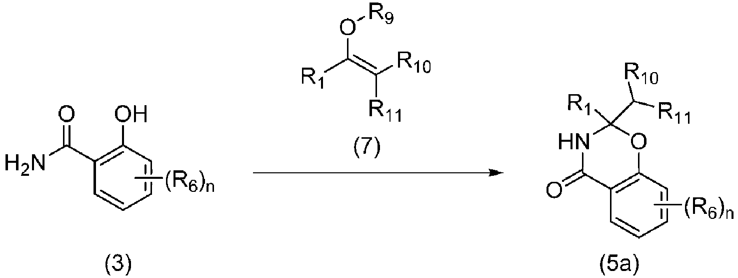

In the formulas, R₉ represents a C1 to C6 alkyl group, R₁₀ and R₁₁ each independently represent a hydrogen atom, a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C1 to C6 acyl group optionally substituted by substituent A, an aryl group optionally substituted by substituent B, or a heteroaryl group optionally substituted by substituent B, and R₁, R₆, and n are as defined above.

The production method C is a method for obtaining a compound represented by the formula (5a) and is a production method comprising reacting a compound represented by the formula (3) with a compound represented by the formula (7) in the presence of an acid.

The compound represented by the formula (7) for use in this reaction can be obtained as a commercially available product or produced by a method known in the art.

The production method C can be carried out in accordance with the production method A by using the compound represented by the formula (7) instead of the compound represented by the formula (4) in the production method A.

### [Production method D]

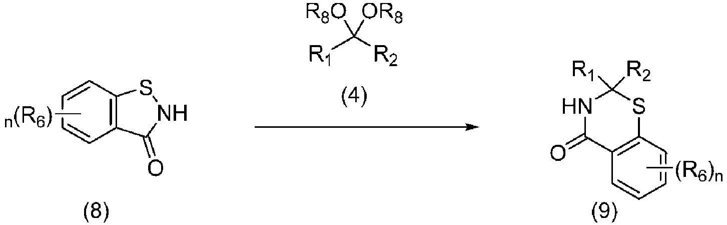

In the formulas, R₁, R₂, R₆, R₈, and n are as defined above.

The production method D is a production method for obtaining a benzothiazine compound represented by the formula (9) and is a production method comprising subjecting a compound represented by the formula (8) to N-S bond cleavage reaction using a reducing agent, followed by N,S-acetalization reaction with a compound represented by the formula (4).

First, the reaction to synthesize 2-mercaptobenzamide from the compound represented by the formula (8) will be described.

Examples of the reducing agent for synthesizing 2-mercaptobenzamide include Raney nickel, catalytic reduction using a palladium catalyst and hydrogen, molybdenum complexes such as molybdenum-hexacarbonyl, iron powders, zinc powders, and samarium iodide.

The amount of the reducing agent for use in this reaction can be 1 equivalent or more based on the compound represented by the formula (8) and is not particularly limited as long as the reaction of interest proceeds. The amount is preferably 1 equivalent or more and 10 equivalents or less.

A solvent can be used, albeit not always essential, in this reaction.

The solvent for use in this reaction is not particularly limited as long as the reaction of interest proceeds. Examples thereof include acidic solvents such as acetic acid and methanesulfonic acid, ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents, more preferably acetic acid.

The amount of the solvent for use in this reaction is not particularly limited as long as the reaction of interest proceeds. The amount is usually 3 or more times and 200 or less times the weight of the compound represented by the formula (8).

The temperature in performing this reaction is not particularly limited as long as the reaction of interest proceeds. The temperature is -10°C or higher and 150°C or lower or a temperature equal to or lower than the boiling point of the solvent.

The thus-obtained reaction mixture containing the 2-mercaptobenzamide derivative may be used directly in the next reaction, or the solvent may be distilled off under reduced pressure without degrading the compound.

The resulting reaction mixture containing the 2-mercaptobenzamide derivative from which the solvent has thus been distilled off may be used directly in the next reaction or can be purified by washing, reprecipitation, recrystallization, column chromatography, or the like using an appropriate solvent. The purification approach can be appropriately set depending on the purity of interest.

Next, the N,S-acetalization reaction of the thus-obtained 2-mercaptobenzamide derivative with the compound represented by the formula (4) will be described.

The compound represented by the formula (4) for use in this reaction can be obtained as a commercially available product or produced by a method known in the art.

The compound represented by the formula (4) for use in this reaction can be of 1 equivalent or more based on the compound represented by the formula (8). The amount is not particularly limited as long as the reaction of interest proceeds. The amount is preferably 1 equivalent or more and 200 equivalents or less.

Examples of the acid for use in this reaction include inorganic acids such as hydrochloric acid and sulfuric acid, and organic acids such as acetic acid, methanesulfonic acid, trifluoromethanesulfonic acid, p-toluenesulfonic acid, and pyridinium p-toluenesulfonate. The acid is not particularly limited as long as the reaction of interest proceeds. The acid is preferably p-toluenesulfonic acid.

A solvent can be used, albeit not always essential, in this reaction.

The solvent for use in this reaction is not particularly limited as long as the reaction of interest proceeds. Examples thereof include acidic solvents such as acetic acid and methanesulfonic acid, ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents and benzene solvents, more preferably acetic acid and toluene.

The amount of the solvent for use in this reaction is not particularly limited as long as the reaction of interest proceeds. The amount is usually 3 or more times and 200 or less times the weight of the compound represented by the formula (8).

The temperature in performing this reaction is not particularly limited as long as the reaction of interest proceeds. The temperature is 50°C or higher and 180°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound represented by the formula (9), water can be removed, albeit not essential, with a desiccant such as sodium sulfate or magnesium sulfate.

From the thus-obtained reaction mixture containing the compound represented by the formula (9), the solvent may be distilled off under reduced pressure without degrading the compound.

The resulting reaction mixture containing the compound represented by the formula 5 from which the solvent has thus been distilled off can be purified by washing, reprecipitation, recrystallization, column chromatography, or the like using an appropriate solvent. The purification approach can be appropriately set depending on the purity of interest.

### [Production method E]

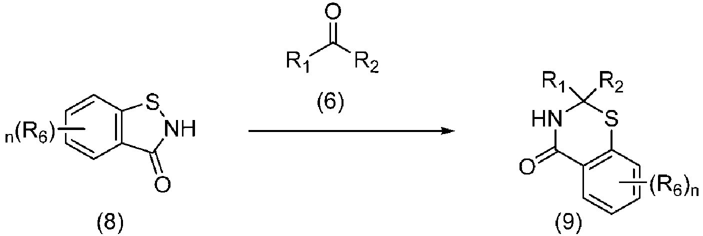

In the formulas, R₁, R₂, R₆, and n are as defined above.

The production method E is a production method for obtaining a benzothiazine compound represented by the formula (9) and is a production method comprising subjecting a compound represented by the formula (8) to N-S bond cleavage reaction using a reducing agent, followed by N,S-acetalization reaction with a compound represented by the formula (6).

The compound represented by the formula (6) for use in this reaction can be obtained as a commercially available product or produced by a method known in the art.

The production method E can be carried out in accordance with the production method D by using the compound represented by the formula (6) instead of the compound represented by the formula (4) in the production method D.

### [Production method F]

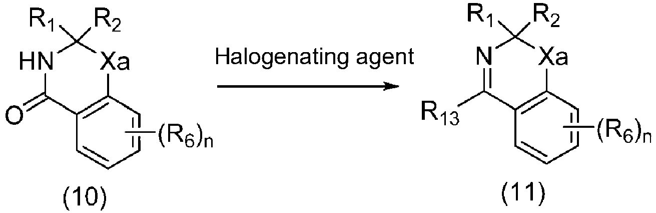

In the formulas, R₁₃ represents a halogen atom, Xa represents an oxygen atom or a sulfur atom, and R₁, R₂, R₆, and n are as defined above.

The production method F is a method for obtaining a compound represented by the formula (11) and is a production method comprising subjecting a compound represented by the formula (10) to halogenation reaction in a solvent.

Examples of the halogenating agent for use in this reaction include thionyl chloride, oxalyl chloride, phosphoryl chloride, sulfuryl chloride, phosphorus trichloride, phosphorus pentachloride, phosphorus tribromide, and Vilsmeier reagents.

The amount of the halogenating agent for use in this reaction can be 1 equivalent or more based on the compound represented by the formula (10) and is not particularly limited as long as the reaction of interest proceeds. The amount is preferably 1 equivalent or more and 10 equivalents or less.

In the case of carrying out this reaction, DMF may coexist as a catalyst. The amount of the catalyst for use in this reaction is not particularly limited as long as the reaction of interest proceeds. The amount is preferably 0.1 equivalents or more and 10 equivalents or less based on the compound represented by the formula (10).

A solvent can be used, albeit not always essential, in this reaction.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio.

The amount of the solvent for use in this reaction is not particularly limited as long as the reaction of interest proceeds. The amount is usually 1 or more times and 200 or less times the weight of the compound represented by the formula (10).

The temperature in performing this reaction is not particularly limited as long as the reaction of interest proceeds. The temperature is usually -10°C or higher and 150°C or lower or a temperature equal to or lower than the boiling point of the solvent.

From the thus-obtained reaction mixture containing the compound represented by the formula (11), the solvent may be distilled off under reduced pressure without degrading the compound.

The resulting reaction mixture containing the compound represented by the formula (11) from which the solvent has thus been distilled off may be used directly in the next reaction or can be purified by washing, reprecipitation, recrystallization, column chromatography, or the like using an appropriate solvent. The purification approach can be appropriately set depending on the purity of interest.

### [Production method G]

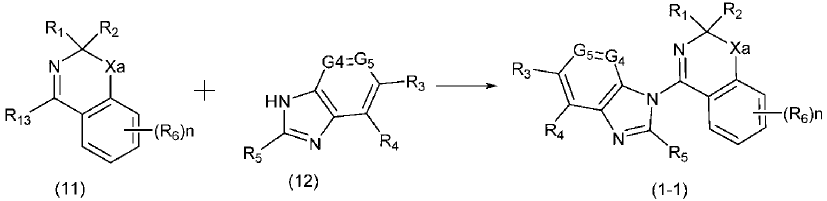

R₁, R₂, R₃, R₄, R₅, R₆, R₁₃, G₄, G₅, Xa, and n are as defined above.

The production method G is a method for obtaining the compound of the present invention represented by the formula (1-1) and is a production method comprising subjecting a compound represented by the formula (11) and a compound represented by the formula (12) to elimination-addition reaction in a solvent under basic conditions.

The compound represented by the formula (12) for use in this reaction can be obtained as a commercially available product or produced by a method known in the art.

Examples of the base for use in this reaction include organic bases such as triethylamine, tributylamine, diisopropylethylamine, pyridine, collidine, and 2,6-lutidine, inorganic bases such as sodium carbonate, potassium carbonate, cesium carbonate, and tripotassium phosphate, and metal hydrides such as sodium hydride.

The amount of the base for use in this reaction is not particularly limited as long as the reaction of interest proceeds. The amount is usually 1 or more times and 10 or less times the weight of the compound represented by the formula (11).

A solvent can be used, albeit not always essential, in this reaction.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio.

The amount of the solvent for use in this reaction is not particularly limited as long as the reaction of interest proceeds. The amount is usually 1 or more times and 200 or less times the weight of the compound represented by the formula (11).

The temperature in performing this reaction is not particularly limited as long as the reaction of interest proceeds. The temperature is usually 50°C or higher and 180°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-1), water can be removed, albeit not essential, with a desiccant such as sodium sulfate or magnesium sulfate.

From the thus-obtained reaction mixture containing the compound of the present invention represented by the formula (1-1), the solvent may be distilled off under reduced pressure without degrading the compound.

The resulting reaction mixture containing the compound of the present invention represented by the formula (1-1) from which the solvent has thus been distilled off may be used directly in the next reaction or can be purified by washing, reprecipitation, recrystallization, column chromatography, or the like using an appropriate solvent. The purification approach can be appropriately set depending on the purity of interest.

### [Production method H]

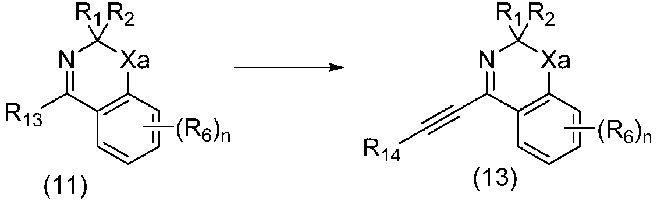

In the formulas, R₁₄ is a hydrogen atom or a C1-C6 alkyl group, and R₁, R₂, R₆, R₁₃, Xa, and n are as defined above.

The production method H is a method for obtaining a compound represented by the formula (13) and is a production method comprising reacting a compound represented by the formula (11) with a terminal alkyne compound by Sonogashira coupling in a solvent in the presence of transition metals and a base.

The terminal alkyne compound for use in this reaction can be obtained as a commercially available product or produced by a method known in the art. Trimethylsilylacetylene may be used as the terminal alkyne compound. In this case, it is necessary to introduce a trimethylsilylethynyl group to the compound represented by the formula (11), followed by desilylation. The desilylation can be performed with reference to non patent literatures such as Journal of the American Chemical Society, Vol. 131, No. 2, p. 634-643 (2009) and Journal of Organometallic Chemistry, Vol. 696, No. 25, p. 4039-4045 (2011).

The amount of the terminal alkyne compound for use in this reaction can be 1 equivalent or more based on the compound represented by the formula (11) and is not particularly limited as long as the reaction of interest proceeds. The amount is preferably 1 equivalent or more and 10 equivalents or less.

The transition metals for use in this reaction may have a ligand and are, for example, palladiums such as palladium acetate, [1,1'-bis(diphenylphosphino)ferrocene]palladium dichloride, tris(dibenzylideneacetone)dipalladium, tetrakis(triphenylphosphine)palladium, and bis(triphenylphosphine)palladium dichloride. Coppers such as copper chloride, copper bromide, and copper iodide are also used at the same time therewith.

The amounts of the transition metals for use in this reaction can be 0.001 equivalents or more each of the palladiums, etc. and the coppers based on the compound represented by the formula (11) and are not particularly limited as long as the reaction of interest proceeds. Both the amounts are preferably 0.001 equivalents or more and 1 equivalent or less.

Examples of the base for use in this reaction include organic amines such as triethylamine, tributylamine, isopropylamine, diethylamine, diisopropylamine, and diisopropylethylamine, and inorganic bases such as sodium carbonate, potassium carbonate, and cesium carbonate.

The amount of the base for use in this reaction can be 1 equivalent or more based on the compound represented by the formula (11) and is not particularly limited as long as the reaction of interest proceeds. The amount is preferably 1 equivalent or more and 50 equivalents or less. The organic base in a liquid state can be used as a solvent.

In order to allow this reaction to proceed efficiently, a phosphine ligand such as tri-t-butylphosphine or 2-dicyclohexylphosphino-2'4'6'-triisopropylbiphenyl can be added, albeit not essential.

The amount of the phosphine ligand for use in this reaction is 0.001 equivalents or more and 1 equivalent or less based on the compound represented by the formula (11). The amount is not particularly limited as long as the reaction of interest proceeds.

Examples of the base for use in this reaction include inorganic bases such as sodium carbonate, potassium carbonate, cesium carbonate, and tripotassium phosphate, and metal alkoxides such as sodium methoxide, sodium ethoxide, and potassium t-butoxide.

The amount of the base for use in this reaction can be 1 equivalent or more based on the compound represented by the formula (11) and is not particularly limited as long as the reaction of interest proceeds. The amount is preferably 1 equivalent or more and 50 equivalents or less.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, halogen solvents such as dichloromethane, dichloroethane, chloroform, and carbon tetrachloride, and organic amine solvents such as triethylamine, tributylamine, isopropylamine, diethylamine, diisopropylamine, and diisopropylethylamine. These solvents can be used each singly or as a mixture of two or more types at an arbitrary ratio.

The amount of the solvent for use in this reaction is not particularly limited as long as the reaction of interest proceeds. The amount is usually 3 or more times and 200 or less times the weight of the compound represented by the formula (11).

The temperature in performing this reaction is not particularly limited as long as the reaction of interest proceeds. The temperature is usually 0°C or higher and 150°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, an aqueous solution containing a sulfur atom-containing salt such as sodium thiosulfate or sodium sulfite dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, and chloroform, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound represented by the formula (13), water can be removed, albeit not essential, with a desiccant such as sodium sulfate or magnesium sulfate.

From the thus-obtained reaction mixture containing the compound represented by the formula (13), the solvent may be distilled off under reduced pressure without degrading the compound.

The resulting reaction mixture containing the compound represented by the formula (13) from which the solvent has thus been distilled off may be used directly in the next reaction or can be purified by washing, reprecipitation, recrystallization, column chromatography, or the like using an appropriate solvent. The purification approach can be appropriately set depending on the purity of interest.

### [Production method I]

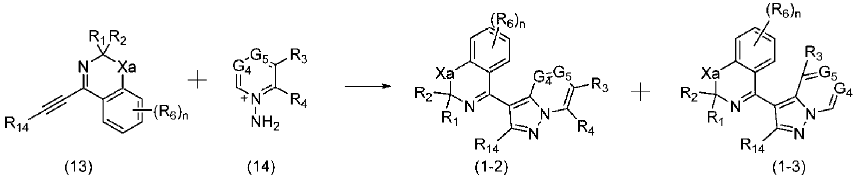

In the formulas, R₁,R₂, R₃, R₄, R₆, R₁₄, G₄, G₅, Xa, and n are as defined above.

The production method I is a production method for obtaining the compounds of the present invention represented by the formula (1-2) and the formula (1-3) and is a production method comprising reacting a compound represented by the formula (13) with a compound represented by the formula (14) in a solvent under basic conditions.

The ratio between the compound represented by the formula (1-2) and the compound represented by the formula (1-3) obtained through this reaction can be a single proportion of one of these compounds or an arbitrary ratio as a mixture and is not particularly limited.

The compound represented by the formula (14) for use in this reaction can be obtained as a commercially available product or produced by a method known in the art.

The compound represented by the formula (14) for use in this reaction has a counter anion.

The counter anion for the formula (14) is not particularly limited as long as the reaction of interest proceeds. Examples thereof include fluoride ions, chloride ions, bromide ions, iodide ions, trifluoroacetate ions, 2,4-dinitrophenolate ions, and mesitylenesulfonate ions.

The compound of the formula (14) for use in this reaction can be of 1 equivalent or more based on the compound represented by the formula (13). The amount is not particularly limited as long as the reaction of interest proceeds. The amount is preferably 1 equivalent or more and 10 equivalents or less.

Examples of the base for use in this reaction include organic bases such as triethylamine, tributylamine, diisopropylethylamine, pyridine, collidine, and 2,6-lutidine, and inorganic bases such as sodium carbonate, potassium carbonate, cesium carbonate, and tripotassium phosphate.

A solvent can be used, albeit not always essential, in this reaction.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents and benzene solvents.

The amount of the solvent for use in this reaction is not particularly limited as long as the reaction of interest proceeds. The amount is usually 3 or more times and 200 or less times the weight of the compound represented by the formula (13).

The temperature in performing this reaction is not particularly limited as long as the reaction of interest proceeds. The temperature is usually -10°C or higher and 150°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-2) and the compound of the present invention represented by the formula (1-3), water can be removed, albeit not essential, with a desiccant such as sodium sulfate or magnesium sulfate.

From the thus-obtained reaction mixture containing the compound of the present invention represented by the formula (1-2) and the compound of the present invention represented by the formula (1-3), the solvent may be distilled off under reduced pressure without degrading the compound.

The resulting reaction mixture containing the compound of the present invention represented by the formula (1-2) and the compound of the present invention represented by the formula (1-3) from which the solvent has thus been distilled off can be purified by washing, reprecipitation, recrystallization, column chromatography, or the like using an appropriate solvent. The purification approach can be appropriately set depending on the purity of interest.

### [Production method J]

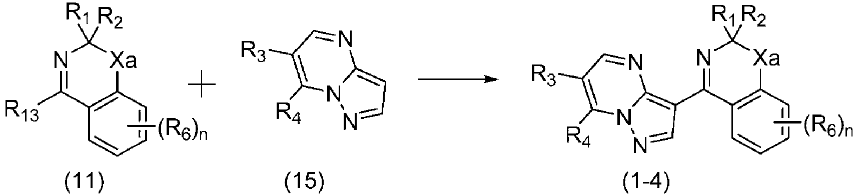

In the formulas, R₁,R₂, R₃, R₄, R₆, R₁₃, Xa, and n are as defined above.

The production method J is a method for obtaining the compound of the present invention represented by the formula (1-4) and is a production method comprising reacting a compound represented by the formula (11) with a pyrazolopyrimidine derivative (15) by cross coupling via C-H activation in a solvent in the presence of transition metals and a base.

The compound represented by the formula (15) for use in this reaction can be obtained as a commercially available product or produced by a method known in the art.

The amount of the compound represented by the formula (15) for use in this reaction can 1 equivalent or more based on the compound represented by the formula (11) and is not particularly limited as long as the reaction of interest proceeds. The amount is preferably 1 equivalent or more and 10 equivalents or less.

The transition metals for use in this reaction are, for example, palladium, nickel, or ruthenium and may have a ligand. Examples thereof preferably include palladiums such as palladium acetate, [1,1'-bis(diphenylphosphino), ferrocene]palladium dichloride, tris(dibenzylideneacetone)dipalladium, tetrakis(triphenylphosphine)palladium, bis(triphenylphosphine), and palladium dichloride.

The amount of the transition metals for use in this reaction is 0.001 equivalents or more and 1 equivalent or less based on the compound represented by the formula (11). The amount is not particularly limited as long as the reaction of interest proceeds.

In order to allow this reaction to proceed efficiently, a phosphine ligand such as triphenylphosphine, tricyclohexylphosphine, or tri-tert-butylphosphonium tetrafluoroborate can be added.

The amount of the phosphine ligand for use in this reaction is 0.001 equivalents or more and 1 equivalent or less based on the compound represented by the formula (11). The amount is not particularly limited as long as the reaction of interest proceeds.

Examples of the base for use in this reaction include inorganic bases such as sodium carbonate, potassium carbonate, cesium carbonate, and tripotassium phosphate, and metal alkoxides such as sodium methoxide, sodium ethoxide, and potassium t-butoxide.

The amount of the base for use in this reaction can be 1 equivalent or more based on the compound represented by the formula (11) and is not particularly limited as long as the reaction of interest proceeds. The amount is preferably 1 equivalent or more and 50 equivalents or less.

The solvent for use in this reaction is not particularly limited as long as the reaction of interest proceeds. Examples thereof include water solvents, ether solvents such as diethyl ether, diisopropyl ether, methyl-t-butyl ether, dimethoxyethane, tetrahydrofuran, and dioxane, and benzene solvents such as benzene, toluene, xylene, mesitylene, chlorobenzene, and dichlorobenzene. These solvents can be used each singly or as a mixture of two or more types at an arbitrary ratio.

The amount of the solvent for use in this reaction is not particularly limited as long as the reaction of interest proceeds. The amount is usually 3 or more times and 200 or less times the weight of the compound represented by the formula (11).

The temperature in performing this reaction is not particularly limited as long as the reaction of interest proceeds. The temperature is usually 30°C or higher and 100°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, an aqueous solution containing a sulfur atom-containing salt such as sodium thiosulfate or sodium sulfite dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, and chloroform, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-4), water can be removed, albeit not essential, with a desiccant such as sodium sulfate or magnesium sulfate.

From the thus-obtained reaction mixture containing the compound of the present invention represented by the formula (1-4), the solvent may be distilled off under reduced pressure without degrading the compound.

The resulting reaction mixture containing the compound of the present invention represented by the formula (1-4) from which the solvent has thus been distilled off may be used directly in the next reaction or can be purified by washing, reprecipitation, recrystallization, column chromatography, or the like using an appropriate solvent. The purification approach can be appropriately set depending on the purity of interest.

### [Production method K]

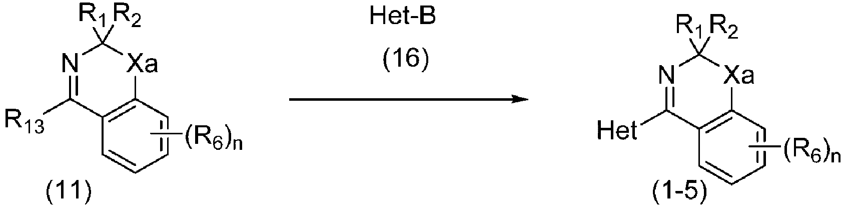

Het-B represents organic boronic acids, and R₁, R₂, R₆, R₁₃, Xa, Het, and n are as defined above.

The production method K is a method for obtaining the compound of the present invention represented by the formula (1-5) and is a production method comprising reacting a compound represented by the formula (11) with an organic boronic acid compound represented by the formula (16) by Suzuki-Miyaura coupling in a solvent.

The compound represented by the formula (16) for use in this reaction refers to organic boronic acids such as organic boronic acid or organic boronic acid ester and can be obtained as a commercially available product or produced by a method known in the art.

The amount of the compound represented by the formula (16) for use in this reaction can be 1 equivalent or more based on the compound represented by the formula (11) and is not particularly limited as long as the reaction of interest proceeds. The amount is preferably 1 equivalent or more and 10 equivalents or less.

The transition metals for use in this reaction are, for example, palladium, nickel, or ruthenium and may have a ligand. Examples thereof preferably include palladiums such as palladium acetate, [1,1'-bis(diphenylphosphino), ferrocene]palladium dichloride, tris(dibenzylideneacetone)dipalladium, tetrakis(triphenylphosphine)palladium, bis(triphenylphosphine), and palladium dichloride.

The amount of the transition metals for use in this reaction is 0.001 equivalents or more and 1 equivalent or less based on the compound represented by the formula (11). The amount is not particularly limited as long as the reaction of interest proceeds.

In order to allow this reaction to proceed efficiently, a phosphine ligand such as triphenylphosphine or tricyclohexylphosphine can be added.

The amount of the phosphine ligand for use in this reaction is 0.001 equivalents or more and 1 equivalent or less based on the compound represented by the formula (11). The amount is not particularly limited as long as the reaction of interest proceeds.

Examples of the base for use in this reaction include inorganic bases such as sodium carbonate, potassium carbonate, cesium carbonate, and tripotassium phosphate, and metal alkoxides such as sodium methoxide, sodium ethoxide, and potassium t-butoxide.

The amount of the base for use in this reaction can be 1 equivalent or more based on the compound represented by the formula (11) and is not particularly limited as long as the reaction of interest proceeds. The amount is preferably 1 equivalent or more and 50 equivalents or less.

The solvent for use in this reaction is not particularly limited as long as the reaction of interest proceeds. Examples thereof include water solvents, ether solvents such as diethyl ether, diisopropyl ether, methyl-t-butyl ether, dimethoxyethane, tetrahydrofuran, and dioxane, and benzene solvents such as benzene, toluene, xylene, mesitylene, chlorobenzene, and dichlorobenzene. These solvents can be used each singly or as a mixture of two or more types at an arbitrary ratio.

The amount of the solvent for use in this reaction is not particularly limited as long as the reaction of interest proceeds. The amount is usually 3 or more times and 200 or less times the weight of the compound represented by the formula (11).

The temperature in performing this reaction is not particularly limited as long as the reaction of interest proceeds. The temperature is usually 30°C or higher and 100°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, an aqueous solution containing a sulfur atom-containing salt such as sodium thiosulfate or sodium sulfite dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, and chloroform, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-5), water can be removed, albeit not essential, with a desiccant such as sodium sulfate or magnesium sulfate.

From the thus-obtained reaction mixture containing the compound of the present invention represented by the formula (1-5), the solvent may be distilled off under reduced pressure without degrading the compound.

The resulting reaction mixture containing the compound of the present invention represented by the formula (1-5) from which the solvent has thus been distilled off may be used directly in the next reaction or can be purified by washing, reprecipitation, recrystallization, column chromatography, or the like using an appropriate solvent. The purification approach can be appropriately set depending on the purity of interest.

### [Production method L]

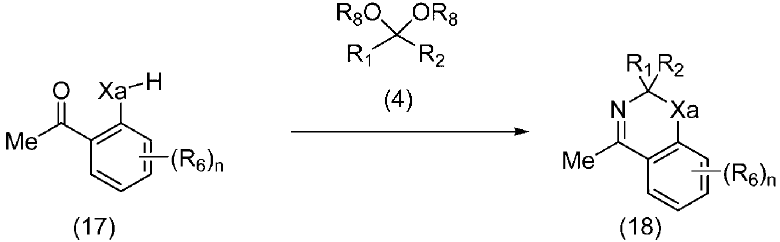

In the formulas, R₁, R₂, R₆, R₈, Xa, and n are as defined above.

The production method L is a method for obtaining a compound represented by the formula (18) and is a production method comprising reacting a compound represented by the formula (17) with a compound represented by the formula (4) in the presence of an ammonium salt.

The compound represented by the formula (17) for use in this reaction can be obtained as a commercially available product or produced by a method known in the art.

The compound represented by the formula (4) for use in this reaction can be obtained as a commercially available product or produced by a method known in the art.

The compound of the formula (4) for use in this reaction can be of 1 equivalent or more based on the compound represented by the formula (17). The amount is not particularly limited as long as the reaction of interest proceeds. The amount is preferably 1 equivalent or more and 200 equivalents or less. The compound of the formula (4) in a liquid state may be used as a solvent.

Examples of the ammonium salt for use in this reaction include ammonium chloride, ammonium acetate, ammonium sulfate, and ammonium phosphate. The ammonium salt is not particularly limited as long as the reaction of interest proceeds. The ammonium salt is preferably ammonium acetate.

A solvent can be used, albeit not always essential, in this reaction.

The solvent for use in this reaction is not particularly limited as long as the reaction of interest proceeds. Examples thereof include acidic solvents such as acetic acid and methanesulfonic acid, ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents and benzene solvents, more preferably acetic acid and toluene.

The amount of the solvent for use in this reaction is not particularly limited as long as the reaction of interest proceeds. The amount is usually 3 or more times and 200 or less times the weight of the compound represented by the formula (17).

The temperature in performing this reaction is not particularly limited as long as the reaction of interest proceeds. The temperature is usually 50°C or higher and 180°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound represented by the formula (18), water can be removed, albeit not essential, with a desiccant such as sodium sulfate or magnesium sulfate.

From the thus-obtained reaction mixture containing the compound represented by the formula (18), the solvent may be distilled off under reduced pressure without degrading the compound.

The resulting reaction mixture containing the compound represented by the formula (18) from which the solvent has thus been distilled off can be purified by washing, reprecipitation, recrystallization, column chromatography, or the like using an appropriate solvent. The purification approach can be appropriately set depending on the purity of interest.

### [Production method M]

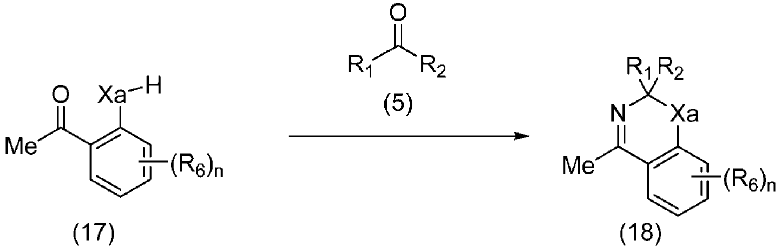

In the formulas, R₁, R₂, R₆, Xa, and n are as defined above.

The production method M is a method for obtaining a compound represented by the formula (18) and is a production method comprising reacting a compound represented by the formula (17) with a compound represented by the formula (5) in the presence of an ammonium salt.

The production method M can be carried out in accordance with the production method L by using the compound represented by the formula (5) instead of the compound represented by the formula (4) in the production method L.

### [Production method N]

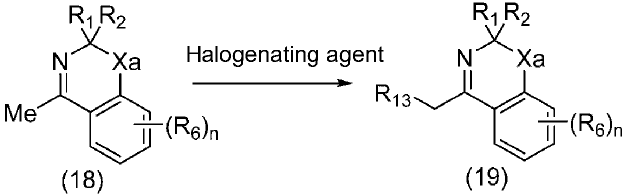

In the formulas, R₁, R₂, R₆, R₁₃, Xa, and n are as defined above.

The production method N is a method for obtaining a compound represented by the formula (19) and is a production method comprising subjecting a compound represented by the formula (18) to halogenation reaction in a solvent using a halogenating agent.

In order to allow this reaction to proceed efficiently, azobisisobutyronitrile, 2,2'-azobis(4-methoxy-2,4-dimethylvaleronitrile), benzoyl peroxide, or the like can be added. The amount of the radical initiator for use in this reaction is not particularly limited as long as the reaction of interest proceeds. The amount is usually 0.01 equivalents or more and 1.0 equivalent or less based on the compound represented by the formula (18).

Examples of the halogenating agent for use in this reaction include N-chlorosuccinimide, N-bromosuccinimide, N-iodosuccinimide, 1,3-dichloro-5,5-dimethylhydantoin, 1,3-dibromo-5,5-dimethylhydantoin, 1,3-diiodo-5,5-dimethylhydantoin, bromine, and copper(II) bromide.

The amount of the halogenating agent for use in this reaction can be 2 equivalents or more based on the compound represented by the formula (18) and is not particularly limited as long as the reaction of interest proceeds. The amount is usually 2 equivalents or more and 2.8 equivalents or less.

The solvent for use in this reaction is not particularly limited as long as the reaction of interest proceeds. Examples thereof include halogenated benzene solvents such as chlorobenzene and dichlorobenzene, ester solvents such as ethyl acetate, isopropyl acetate, and butyl acetate, halogen solvents such as dichloromethane, dichloroethane, chloroform, and carbon tetrachloride, and hydrocarbon solvents such as hexane, heptane, cyclohexane, and methylcyclohexane. These solvents can be used each singly or as a mixture of two or more types at an arbitrary ratio.

The amount of the solvent for use in this reaction is not particularly limited as long as the reaction of interest proceeds. The amount is usually 3 or more times and 200 or less times the weight of the compound represented by the formula (18).

The temperature in performing this reaction is not particularly limited as long as the reaction of interest proceeds. The temperature is usually 20°C or higher and 150°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, an aqueous solution containing a sulfur atom-containing salt such as sodium thiosulfate or sodium sulfite dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, and chloroform, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound represented by the formula (19), water can be removed, albeit not essential, with a desiccant such as sodium sulfate or magnesium sulfate.

From the thus-obtained reaction mixture containing the compound represented by the formula (19), the solvent may be distilled off under reduced pressure without degrading the compound.

The resulting reaction mixture containing the compound represented by the formula (19) from which the solvent has thus been distilled off can be purified by washing, reprecipitation, recrystallization, column chromatography, or the like using an appropriate solvent. The purification approach can be appropriately set depending on the purity of interest.

### [Production method O]

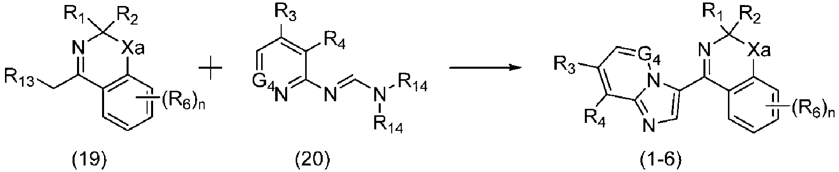

In the formulas, R₁₄ represents a C1 to C6 alkyl group, and R₁, R₂, R₃, R₄, R₆, R₁₃, G₄, Xa and n are as defined above.

The production method O is a production method for obtaining the compound of the present invention represented by the formula (1-6) and is a production method comprising reacting a compound represented by the formula (19) with a compound represented by the formula (20) in a solvent under basic conditions.

The compound represented by the formula (20) for use in this reaction can be obtained as a commercially available product or produced by a method known in the art.

The compound represented by the formula (20) for use in this reaction can be of 1 equivalent or more based on the compound represented by the formula (19). The amount is not particularly limited as long as the reaction of interest proceeds. The amount is preferably 1 equivalent or more and 10 equivalents or less.

Examples of the base for use in this reaction include organic bases such as triethylamine, tributylamine, diisopropylethylamine, pyridine, collidine, and 2,6-lutidine, and inorganic bases such as sodium carbonate, potassium carbonate, cesium carbonate, and tripotassium phosphate.

A solvent can be used, albeit not always essential, in this reaction.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents and benzene solvents.

The amount of the solvent for use in this reaction is not particularly limited as long as the reaction of interest proceeds. The amount is usually 3 or more times and 200 or less times the weight of the compound represented by the formula (19).

The temperature in performing this reaction is not particularly limited as long as the reaction of interest proceeds. The temperature is usually -10°C or higher and 150°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-6), water can be removed, albeit not essential, with a desiccant such as sodium sulfate or magnesium sulfate.

From the thus-obtained reaction mixture containing the compound of the present invention represented by the formula (1-6), the solvent may be distilled off under reduced pressure without degrading the compound.

The resulting reaction mixture containing the compound of the present invention represented by the formula (1-6) from which the solvent has thus been distilled off can be purified by washing, reprecipitation, recrystallization, column chromatography, or the like using an appropriate solvent. The purification approach can be appropriately set depending on the purity of interest.

### [Production method P]

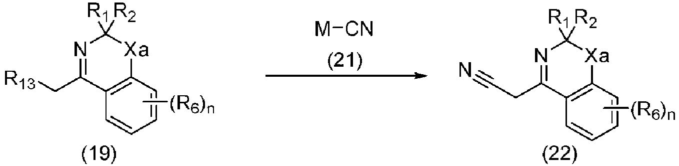

In the formulas, M represents a metal cation such as sodium, potassium, or copper, and R₁, R₂, R₆, R₁₃, Xa, and n are as defined above.

The production method P is a production method for obtaining a compound represented by the formula (22) and is a production method comprising reacting a compound represented by the formula (19) with a cyanating agent represented by the formula (21) in a solvent under basic conditions.

The cyanating agent represented by the formula (21) for use in this reaction can be obtained as a commercially available product or produced by a method known in the art.

The cyanating agent represented by the formula (21) for use in this reaction can be of 1 equivalent or more based on the compound represented by the formula (19). The amount is not particularly limited as long as the reaction of interest proceeds. The amount is preferably 1 equivalent or more and 10 equivalents or less.

Examples of the base for use in this reaction include organic bases such as triethylamine, tributylamine, diisopropylethylamine, pyridine, collidine, and 2,6-lutidine, and inorganic bases such as sodium carbonate, potassium carbonate, cesium carbonate, and tripotassium phosphate.

A solvent can be used, albeit not always essential, in this reaction.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents and benzene solvents.

The amount of the solvent for use in this reaction is not particularly limited as long as the reaction of interest proceeds. The amount is usually 3 or more times and 200 or less times the weight of the compound represented by the formula (19).

The temperature in performing this reaction is not particularly limited as long as the reaction of interest proceeds. The temperature is usually -10°C or higher and 150°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound represented by the formula (22), water can be removed, albeit not essential, with a desiccant such as sodium sulfate or magnesium sulfate.

From the thus-obtained reaction mixture containing the compound represented by the formula (22), the solvent may be distilled off under reduced pressure without degrading the compound.

The resulting reaction mixture containing the compound represented by the formula (22) from which the solvent has thus been distilled off can be purified by washing, reprecipitation, recrystallization, column chromatography, or the like using an appropriate solvent. The purification approach can be appropriately set depending on the purity of interest.

### [Production method Q]

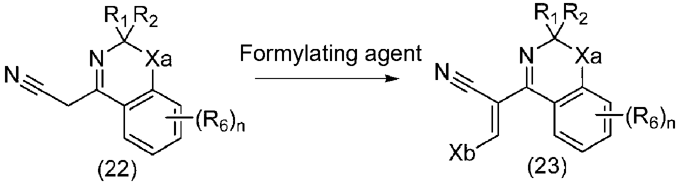

In the formulas, Xb represents a hydroxy group or NRaRb (wherein Ra and Rb are as defined above), and R₁, R₂, R₆, Xa, and n are as defined above.

The production method Q is a production method for obtaining a compound represented by the formula (23) and is a production method comprising subjecting a compound represented by the formula (22) to formylation reaction in a solvent.

Examples of the formylating agent for use in this reaction include DMF, N,N-dimethylformamide dimethyl acetal, 4-formylmorpholine, hexamethylene tetramine, N-methylformanilide, N,N-diethyl-N'-formyl-N'-methoxy urea, cyanomethyl formate, ethyl bromodifluoroacetate, ethyl formate, methyl formate, 1-formylpiperidine, N-formylsaccharin, triethyl orthoformate, trimethyl orthoformate, Vilsmeier reagents, paraformaldehyde, and Bredereck reagents.

The formylating agent for use in this reaction can be of 1 equivalent or more based on the compound represented by the formula (22). The amount is not particularly limited as long as the reaction of interest proceeds. The amount is preferably 1 equivalent or more and 10 equivalents or less.

Examples of the base for use in this reaction include metal hydrides such as sodium hydride, metal alkoxides such as sodium methoxide, sodium ethoxide, and sodium t-butoxide, organic lithiums such as methyllithium, butyllithium, sec-butyllithium, t-butyllithium, and hexyllithium, and metal amides such as lithium diisopropylamide, lithium hexamethyldisilazane, sodium hexamethyldisilazane, and potassium hexamethyldisilazane.

A solvent can be used, albeit not always essential, in this reaction.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents and benzene solvents.

The amount of the solvent for use in this reaction is not particularly limited as long as the reaction of interest proceeds. The amount is usually 3 or more times and 200 or less times the weight of the compound represented by the formula (22).

The temperature in performing this reaction is not particularly limited as long as the reaction of interest proceeds. The temperature is usually -10°C or higher and 150°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound represented by the formula (23), water can be removed, albeit not essential, with a desiccant such as sodium sulfate or magnesium sulfate.

From the thus-obtained reaction mixture containing the compound represented by the formula (23), the solvent may be distilled off under reduced pressure without degrading the compound.

The resulting reaction mixture containing the compound represented by the formula (23) from which the solvent has thus been distilled off can be purified by washing, reprecipitation, recrystallization, column chromatography, or the like using an appropriate solvent. The purification approach can be appropriately set depending on the purity of interest.

### [Production method R]

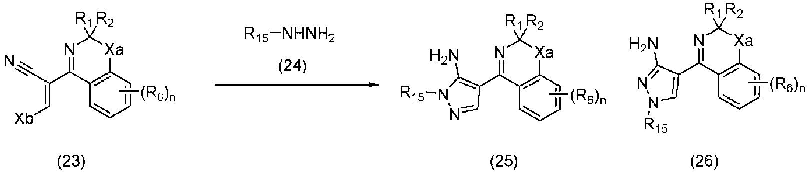

In the formulas, R₁₅ is a hydrogen atom or a protective group deprotectable under acidic conditions, such as Boc, a MOM group, or a PMB group, and R₁, R₂, R₆, Xa, Xb, and n are as defined above.

The production method R is a production method for obtaining a compound represented by the formula (25) and a compound represented by the formula (26) and is a production method comprising reacting a compound represented by the formula (23) with a compound represented by the formula (24) in a solvent.

The ratio between the compound represented by the formula (25) and the compound represented by the formula (26) obtained through this reaction can be a single proportion of one of these compounds or an arbitrary ratio as a mixture and is not particularly limited.

The compound represented by the formula (24) for use in this reaction can be of 1 equivalent or more based on the compound represented by the formula (23). The amount is not particularly limited as long as the reaction of interest proceeds. The amount is preferably 1 equivalent or more and 10 equivalents or less.

In order to allow this reaction to proceed efficiently, an acid catalyst can be used, albeit not essential.

In the case of using an acid in this reaction, examples thereof include inorganic acids such as hydrochloric acid and sulfuric acid, and organic acids such as acetic acid, methanesulfonic acid, trifluoromethanesulfonic acid, p-toluenesulfonic acid, and pyridinium p-toluenesulfonate. The acid is not particularly limited as long as the reaction of interest proceeds. The acid is preferably p-toluenesulfonic acid.

In the case of using an acid in this reaction, the amount of the acid used is 0.001 equivalents or more and 1 equivalent or less based on the compound represented by the formula (23). The amount is not particularly limited as long as the reaction of interest proceeds.

A solvent can be used, albeit not always essential, in this reaction.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents and benzene solvents.

The amount of the solvent for use in this reaction is not particularly limited as long as the reaction of interest proceeds. The amount is usually 3 or more times and 200 or less times the weight of the compound represented by the formula (23).

The temperature in performing this reaction is not particularly limited as long as the reaction of interest proceeds. The temperature is usually -10°C or higher and 150°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound represented by the formula (25) and the compound represented by the formula (26), water can be removed, albeit not essential, with a desiccant such as sodium sulfate or magnesium sulfate.

From the thus-obtained reaction mixture containing the compound represented by the formula (25) and the compound represented by the formula (26), the solvent may be distilled off under reduced pressure without degrading the compound.

The resulting reaction mixture containing the compound represented by the formula (25) and the compound represented by the formula (26) from which the solvent has thus been distilled off can be purified by washing, reprecipitation, recrystallization, column chromatography, or the like using an appropriate solvent. The purification approach can be appropriately set depending on the purity of interest.

### [Production method S]

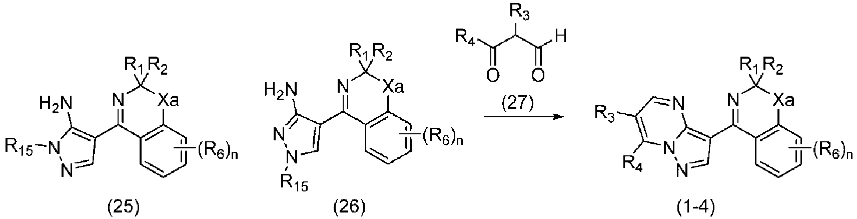

In the formulas, R₁, R₂, R₃, R₄, R₆, R₁₅, Xa, and n are as defined above.

The production method S is a production method for obtaining the compound of the present invention represented by the formula (1-4) and is a production method comprising reacting a compound represented by the formula (25) and a compound represented by the formula (26) with a compound represented by the formula (27) in a solvent under acidic conditions.

The ratio between the compound represented by the formula (25) and the compound represented by the formula (26) for use in this reaction can be a single proportion of one of these compounds or an arbitrary ratio as a mixture and is not particularly limited.

The compound represented by the formula (27) for use in this reaction can be of 1 equivalent or more based on the compound represented by the formula (25) and the compound represented by the formula (26). The amount is not particularly limited as long as the reaction of interest proceeds. The amount is preferably 1 equivalent or more and 10 equivalents or less.

Examples of the acid for use in this reaction include inorganic acids such as hydrochloric acid and sulfuric acid, and organic acids such as acetic acid, methanesulfonic acid, trifluoromethanesulfonic acid, p-toluenesulfonic acid, and pyridinium p-toluenesulfonate. The acid is not particularly limited as long as the reaction of interest proceeds. The acid is preferably hydrochloric acid.

A solvent can be used, albeit not always essential, in this reaction.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents and benzene solvents.

The amount of the solvent for use in this reaction is not particularly limited as long as the reaction of interest proceeds. The amount is usually 3 or more times and 200 or less times the weight of the compound represented by the formula (25) and the compound represented by the formula (26).

The temperature in performing this reaction is not particularly limited as long as the reaction of interest proceeds. The temperature is -10°C or higher and 150°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-4), water can be removed, albeit not essential, with a desiccant such as sodium sulfate or magnesium sulfate.

From the thus-obtained reaction mixture containing the compound represented by the formula (1-4), the solvent may be distilled off under reduced pressure without degrading the compound.

The resulting reaction mixture containing the compound of the present invention represented by the formula (1-4) from which the solvent has thus been distilled off can be purified by washing, reprecipitation, recrystallization, column chromatography, or the like using an appropriate solvent. The purification approach can be appropriately set depending on the purity of interest.

### [Production method T]

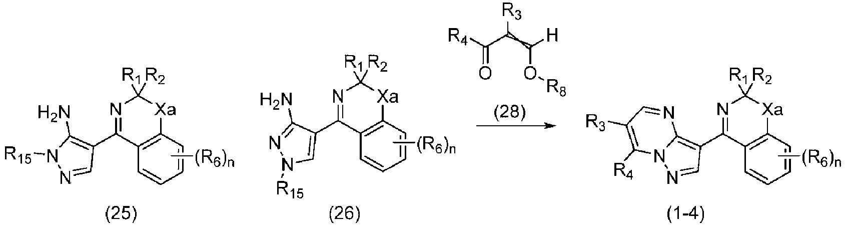

In the formulas, R₁, R₂, R₃, R₄, R₆, R₈, R₁₅, Xa, and n are as defined above.

The production method T is a production method for obtaining the compound of the present invention represented by the formula (1-4) and is a production method comprising reacting a compound represented by the formula (25) and a compound represented by the formula (26) with a compound represented by the formula (28) in a solvent under acidic conditions.

The production method T can be carried out in accordance with the production method S by using the compound represented by the formula (28) instead of the compound represented by the formula (27) in the production method S.

### [Production method U]

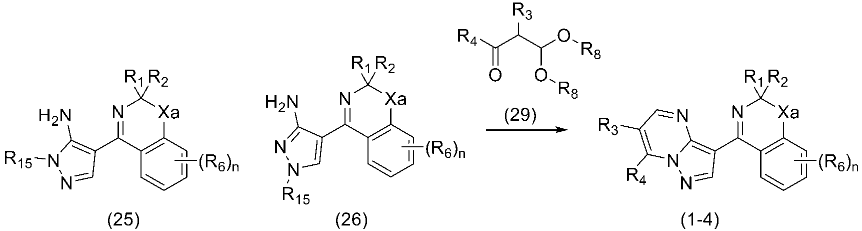

In the formulas, R₁, R₂, R₃, R₄, R₆, R₈, R₁₅, Xa, and n are as defined above.

The production method U is a production method for obtaining the compound of the present invention represented by the formula (1-4) and is a production method comprising reacting a compound represented by the formula (25) and a compound represented by the formula (26) with a compound represented by the formula (29) in a solvent under acidic conditions.

The production method U can be carried out in accordance with the production method S by using the compound represented by the formula (29) instead of the compound represented by the formula (27) in the production method S.

### [Production method V]

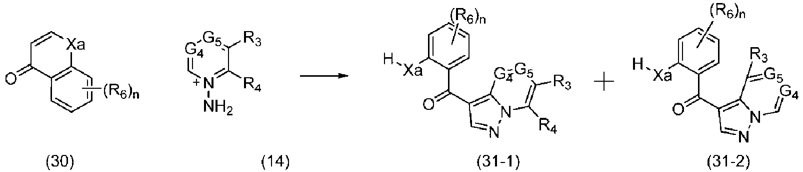

In the formulas, R₃, R₄, R₆, G₄, G₅, Xa, and n are as defined above.

The production method V is a production method for obtaining compounds represented by the formula (31-1) and the formula (31-2) and is a production method comprising reacting a compound represented by the formula (30) with a compound represented by the formula (14) in a solvent under basic conditions.

The ratio between the compound represented by the formula (31-1) and the compound represented by the formula (31-2) obtained through this reaction can be a single proportion of one of these compounds or an arbitrary ratio as a mixture and is not particularly limited.

The compound represented by the formula (14) for use in this reaction can be obtained as a commercially available product or produced by a method known in the art.

The compound represented by the formula (30) for use in this reaction can be obtained as a commercially available product or produced by a method known in the art.

The compound represented by the formula (14) for use in this reaction has a counter anion.

The counter anion for the formula (14) is not particularly limited as long as the reaction of interest proceeds. Examples thereof include fluoride ions, chloride ions, bromide ions, iodide ions, trifluoroacetate ions, 2,4-dinitrophenolate ions, and mesitylenesulfonate ions.

The compound of the formula (14) for use in this reaction can be of 1 equivalent or more based on the compound represented by the formula (30). The amount is not particularly limited as long as the reaction of interest proceeds. The amount is preferably 1 equivalent or more and 10 equivalents or less.

Examples of the base for use in this reaction include organic bases such as triethylamine, tributylamine, diisopropylethylamine, pyridine, collidine, and 2,6-lutidine, and inorganic bases such as sodium carbonate, potassium carbonate, cesium carbonate, and tripotassium phosphate.

A solvent can be used, albeit not always essential, in this reaction.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents and benzene solvents.

The amount of the solvent for use in this reaction is not particularly limited as long as the reaction of interest proceeds. The amount is usually 3 or more times and 200 or less times the weight of the compound represented by the formula (30).

The temperature in performing this reaction is not particularly limited as long as the reaction of interest proceeds. The temperature is usually -10°C or higher and 150°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound represented by the formula (31-1) and the compound represented by the formula (31-2), water can be removed, albeit not essential, with a desiccant such as sodium sulfate or magnesium sulfate.

From the thus-obtained reaction mixture containing the compound represented by the formula (31-1) and the compound represented by the formula (31-2), the solvent may be distilled off under reduced pressure without degrading the compound.

The resulting reaction mixture containing the compound represented by the formula (31-1) and the compound represented by the formula (31-2) from which the solvent has thus been distilled off can be purified by washing, reprecipitation, recrystallization, column chromatography, or the like using an appropriate solvent. The purification approach can be appropriately set depending on the purity of interest.

### [Production method W]

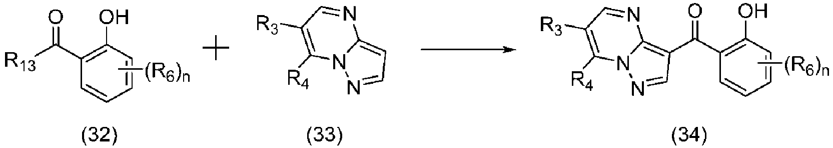

In the formulas, R₃, R₄, R₆, R₁₃, and n are as defined above.

The production method W is a production method for obtaining a compound represented by the formula (34) and is a production method comprising reacting a compound represented by the formula (32) with a compound represented by the formula (33) in a solvent under Lewis acid conditions.

The compound of the formula (33) for use in this reaction can be of 1 equivalent or more based on the compound represented by the formula (32). The amount is not particularly limited as long as the reaction of interest proceeds. The amount is preferably 1 equivalent or more and 10 equivalents or less.

In the case of using a Lewis acid in this reaction, examples thereof include borons such as boron trifluoride-diethyl ether complexes, boron trifluoride-phenol complexes, and boron tribromide, irons such as iron(III) chloride, aluminums such as aluminum chloride and aluminum isopropoxide, titaniums such as titanium chloride and titanium tetraisopropoxide, galliums such as gallium chloride and gallium isopropoxide, indiums such as indium chloride, scandiums such as scandium chloride, and ytterbiums such as ytterbium chloride. The Lewis acid is preferably aluminum chloride or iron chloride.

A solvent can be used, albeit not always essential, in this reaction.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents and benzene solvents.

The amount of the solvent for use in this reaction is not particularly limited as long as the reaction of interest proceeds. The amount is usually 3 or more times and 200 or less times the weight of the compound represented by the formula (32).

The temperature in performing this reaction is not particularly limited as long as the reaction of interest proceeds. The temperature is usually -10°C or higher and 150°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound represented by the formula (34), water can be removed, albeit not essential, with a desiccant such as sodium sulfate or magnesium sulfate.

From the thus-obtained reaction mixture containing the compound represented by the formula (34), the solvent may be distilled off under reduced pressure without degrading the compound.

The resulting reaction mixture containing the compound represented by the formula (34) from which the solvent has thus been distilled off can be purified by washing, reprecipitation, recrystallization, column chromatography, or the like using an appropriate solvent. The purification approach can be appropriately set depending on the purity of interest.

### [Production method X]

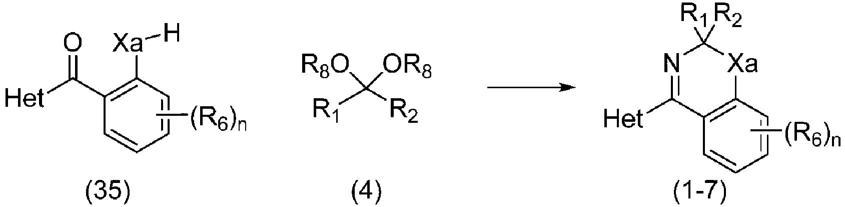

In the formulas, R₁, R₂, R₆, R₈, Xa, Het, and n are as defined above.

The production method X is a method for obtaining the compound of the present invention represented by the formula (1-7) and is a production method comprising reacting a compound represented by the formula (35) with a compound represented by the formula (4) in the presence of an ammonium salt.

The compound represented by the formula (35) can be produced on the basis of production methods or Reference Examples.

The compound represented by the formula (35) for use in this reaction can be produced on the basis of the production method V, the production method W, or Reference Examples.

The compound represented by the formula (4) for use in this reaction can be obtained as a commercially available product or produced by a method known in the art.

The compound represented by the formula (4) for use in this reaction can be of 1 equivalent or more based on the compound represented by the formula (35). The amount is not particularly limited as long as the reaction of interest proceeds. The amount is preferably 1 equivalent or more and 200 equivalents or less. The compound of the formula (4) in a liquid state may be used as a solvent.

Examples of the ammonium salt for use in this reaction include ammonium chloride, ammonium acetate, ammonium sulfate, and ammonium phosphate. The ammonium salt is not particularly limited as long as the reaction of interest proceeds. The ammonium salt is preferably ammonium acetate.

A solvent can be used, albeit not always essential, in this reaction.

The solvent for use in this reaction is not particularly limited as long as the reaction of interest proceeds. Examples thereof include acidic solvents such as acetic acid and methanesulfonic acid, ether solvents such as diethyl ether, diisopropyl ether, methyl-t-butyl ether, dimethoxyethane, tetrahydrofuran, and dioxane, alcohol solvents such as methanol, ethanol, and isopropanol, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, and halogen solvents such as dichloromethane, dichloroethane, chloroform, and carbon tetrachloride. These solvents can be used each singly or as a mixture of two or more types at an arbitrary ratio. Examples of the solvent preferably include acidic solvents and benzene solvents, more preferably acetic acid and toluene.

The amount of the solvent for use in this reaction is not particularly limited as long as the reaction of interest proceeds. The amount is usually 3 or more times and 200 or less times the weight of the compound represented by the formula (35).

The temperature in performing this reaction is not particularly limited as long as the reaction of interest proceeds. The temperature is usually 50°C or higher and 180°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-7), water can be removed, albeit not essential, with a desiccant such as sodium sulfate or magnesium sulfate.

From the thus-obtained reaction mixture containing the compound of the present invention represented by the formula (1-7), the solvent may be distilled off under reduced pressure without degrading the compound.

The resulting reaction mixture containing the compound of the present invention represented by the formula (1-7) from which the solvent has thus been distilled off can be purified by washing, reprecipitation, recrystallization, column chromatography, or the like using an appropriate solvent. The purification approach can be appropriately set depending on the purity of interest.

### [Production method Y]

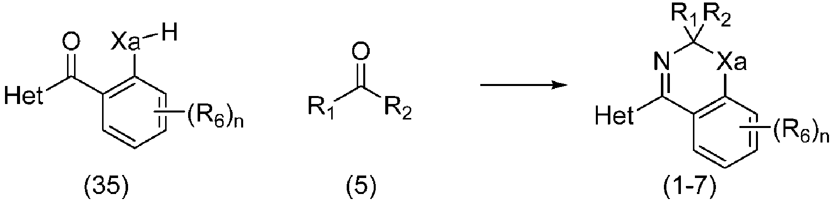

In the formulas, R₁, R₂, R₆, R₈, Xa, Het and n are as defined above.

The production method Y is a method for obtaining the compound of the present invention represented by the formula (1-7) and is a production method comprising reacting a compound represented by the formula (35) with a compound represented by the formula (5) in the presence of an ammonium salt.

The production method Y can be carried out in accordance with the production method X by using the compound represented by the formula (5) instead of the compound represented by the formula (4) in the production method X.

### [Production method Z]

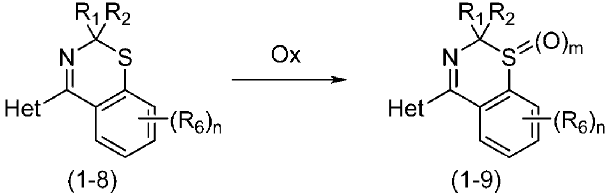

In the formulas, Ox represents an oxidizing agent, m represents an integer of 1 or 2, and R₁, R₂, R₆, and Het are as defined above.

The production method Z is a method for producing the compound of the present invention represented by the formula (1-9) and is a production method comprising reacting the compound of the present invention represented by the formula (1-8) with an oxidizing agent (Ox) in a solvent.

Examples of the oxidizing agent for use in this reaction include peroxides such as hydrogen peroxide water and m-chloroperbenzoic acid. Transition metals such as sodium tungstate may be added.

The amount of the oxidizing agent for use in this reaction is usually 1.0 equivalent or more and 1.2 equivalents or less based on the compound of the present invention represented by the formula (1-8) in producing S(=O), and is usually 2 equivalents or more and 10 equivalents or less in producing SO₂. The transition metals are usually added at 0.001 equivalents or more and 1 equivalent or less.

The solvent for use in this reaction is not particularly limited as long as the reaction of interest proceeds. Examples thereof include water solvents, acidic solvents such as acetic acid, benzene solvents such as benzene, toluene, xylene, mesitylene, chlorobenzene, and dichlorobenzene, nitrile solvents such as acetonitrile, and halogen solvents such as dichloromethane, dichloroethane, chloroform, and carbon tetrachloride. These solvents can be used each singly or as a mixture of two or more types at an arbitrary ratio.

The amount of the solvent for use in this reaction is not particularly limited as long as the reaction of interest proceeds. The amount is usually 3 or more times and 200 or less times the weight of the compound of the present invention represented by the formula (1-8).

The temperature in performing this reaction is not particularly limited as long as the reaction of interest proceeds. The temperature is usually -10°C or higher and 120°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, an aqueous solution containing a sulfur atom-containing salt such as sodium thiosulfate or sodium sulfite dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, chloroform, and carbon tetrachloride, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-9), water can be removed, albeit not essential, with a desiccant such as sodium sulfate or magnesium sulfate.

From the thus-obtained reaction mixture containing the compound of the present invention represented by the formula (1-9), the solvent may be distilled off under reduced pressure without degrading the compound.

The resulting reaction mixture containing the compound of the present invention represented by the formula (1-9) from which the solvent has thus been distilled off can be purified by washing, reprecipitation, recrystallization, column chromatography, or the like using an appropriate solvent. The purification approach can be appropriately set depending on the purity of interest.

### [Production method AA]

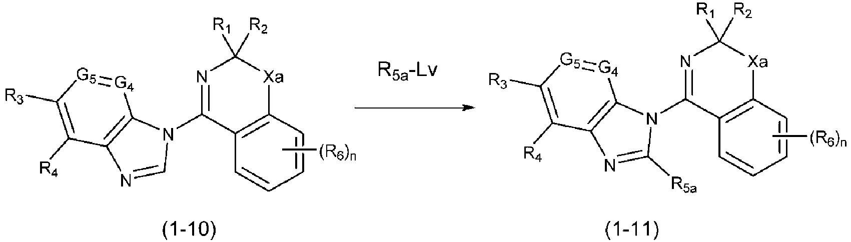

In the formulas, Lv represents a leaving group such as a methanesulfonyl group, a trifluoromethanesulfonyl group, a p-toluenesulfonyl group, or a halogen atom, R₅ₐ represents a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, a C2 to C6 haloalkenyl group, a C2 to C6 alkynyl group optionally substituted by substituent A, or a C2 to C6 haloalkynyl group, and R₁, R₂, R₃, R₄, R₆, G₄, G₅, Xa, and n are as defined above.

The production method AA is a method for synthesizing the compound of the present invention represented by the formula (1-11) and is a production method comprising reacting the compound of the present invention represented by the formula (1-10) with R₅ₐ-Lv in a solvent in the presence of a base.

The compound of the present invention represented by the formula (1-10) for use in this reaction can be produced by the production method E or a method of Reference Examples.

R₅ₐ-Lv for use in this reaction can be obtained as a commercially available product or produced by a method known in the art.

Examples of the base for use in this reaction include metal hydrides such as sodium hydride, organic lithiums such as methyllithium, butyllithium, sec-butyllithium, t-butyllithium, and hexyllithium, and metal amides such as lithium diisopropylamide, lithium hexamethyldisilazane, sodium hexamethyldisilazane, and potassium hexamethyldisilazane.

The amount of the base for use in this reaction can be 1 equivalent or more based on the compound of the present invention represented by the formula (1-10) and is not particularly limited as long as the reaction of interest proceeds. The amount is preferably 1 equivalent or more and 10 equivalents or less.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, and benzene solvents such as benzene, toluene, xylene, mesitylene, chlorobenzene, and dichlorobenzene. These solvents can be used each singly or as a mixture of two or more types at an arbitrary ratio.

The amount of the solvent for use in this reaction is not particularly limited as long as the reaction of interest proceeds. The amount is usually 3 or more times and 200 or less times the weight of the compound of the present invention represented by the formula (1-10).

The temperature in performing this reaction is not particularly limited as long as the reaction of interest proceeds. The temperature is usually -80°C or higher and 100°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, an aqueous solution containing a sulfur atom-containing salt such as sodium thiosulfate or sodium sulfite dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, and chloroform, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-11), water can be removed, albeit not essential, with a desiccant such as sodium sulfate or magnesium sulfate.

From the thus-obtained reaction mixture containing the compound of the present invention represented by the formula (1-11), the solvent may be distilled off under reduced pressure without degrading the compound.

The resulting reaction mixture containing the compound of the present invention represented by the formula (1-11) from which the solvent has thus been distilled off may be used directly in the next reaction or can be purified by washing, reprecipitation, recrystallization, column chromatography, or the like using an appropriate solvent. The purification approach can be appropriately set depending on the purity of interest.

### [Production method AB]

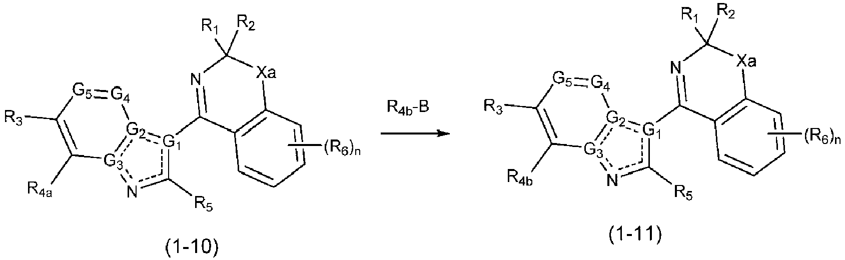

In the formulas, R₄ₐ represents a halogen atom, R_{4b} represents a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, or a C2 to C6 haloalkenyl group, R_{4b}-B represents organic boronic acids, the bond including the broken line is a single bond or a double bond and forms an aromatic ring, and R₁, R₂, R₃, R₅, R₆, G₁, G₂, G₃, G₄, G₅, Xa, and n are as defined above.

The production method AB is a method for synthesizing the compound of the present invention represented by the formula (1-11) and is a production method comprising reacting the compound of the present invention represented by the formula (1-10) with organic boronic acids (R_{4b}-B) by Suzuki-Miyaura coupling in a solvent in the presence of transition metals and a base.

The compound of the present invention represented by the formula (1-10) for use in this reaction can be produced with reference to the production method G, the production method I, the production method J, the production method K, the production method O, the production method S, the production method T, the production method U, the production method X, the production method Y, or Reference Examples.

R_{4b}-B for use in this reaction represents organic boronic acids such as organic boronic acid or organic boronic acid ester and can be obtained as a commercially available product or produced by a method known in the art.

The amount of R_{4b}-B for use in this reaction can be 1 equivalent or more based on the compound of the present invention represented by the formula (1-10) and is not particularly limited as long as the reaction of interest proceeds. The amount is preferably 1 equivalent or more and 10 equivalents or less.

The transition metals for use in this reaction are, for example, palladium, nickel, or ruthenium and may have a ligand. Examples thereof preferably include palladiums such as palladium acetate, [1,1'-bis(diphenylphosphino), ferrocene]palladium dichloride, tris(dibenzylideneacetone)dipalladium, tetrakis(triphenylphosphine)palladium, bis(triphenylphosphine), and palladium dichloride.

The amount of the transition metals for use in this reaction is 0.001 equivalents or more and 1 equivalent or less based on the compound of the present invention represented by the formula (1-10). The amount is not particularly limited as long as the reaction of interest proceeds.

In order to allow this reaction to proceed efficiently, a phosphine ligand such as triphenylphosphine or tricyclohexylphosphine can be added.

The amount of the phosphine ligand for use in this reaction is 0.001 equivalents or more and 1 equivalent or less based on the compound of the present invention represented by the formula (1-10). The amount is not particularly limited as long as the reaction of interest proceeds.

Examples of the base for use in this reaction include inorganic bases such as sodium carbonate, potassium carbonate, cesium carbonate, and tripotassium phosphate, and metal alkoxides such as sodium methoxide, sodium ethoxide, and potassium t-butoxide.

The amount of the base for use in this reaction can be 1 equivalent or more based on the compound of the present invention represented by the formula (1-10) and is not particularly limited as long as the reaction of interest proceeds. The amount is preferably 1 equivalent or more and 50 equivalents or less.

The solvent for use in this reaction is not particularly limited as long as the reaction of interest proceeds. Examples thereof include water solvents, ether solvents such as diethyl ether, diisopropyl ether, methyl-t-butyl ether, dimethoxyethane, tetrahydrofuran, and dioxane, and benzene solvents such as benzene, toluene, xylene, mesitylene, chlorobenzene, and dichlorobenzene. These solvents can be used each singly or as a mixture of two or more types at an arbitrary ratio.

The amount of the solvent for use in this reaction is not particularly limited as long as the reaction of interest proceeds. The amount is usually 3 or more times and 200 or less times the weight of the compound of the present invention represented by the formula (1-10).

The temperature in performing this reaction is not particularly limited as long as the reaction of interest proceeds. The temperature is usually 30°C or higher and 100°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, an aqueous solution containing a sulfur atom-containing salt such as sodium thiosulfate or sodium sulfite dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, and chloroform, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-11), water can be removed, albeit not essential, with a desiccant such as sodium sulfate or magnesium sulfate.

From the thus-obtained reaction mixture containing the compound represented by the formula (1-11), the solvent may be distilled off under reduced pressure without degrading the compound.

The resulting reaction mixture containing the compound of the present invention represented by the formula (1-11) from which the solvent has thus been distilled off may be used directly in the next reaction or can be purified by washing, reprecipitation, recrystallization, column chromatography, or the like using an appropriate solvent. The purification approach can be appropriately set depending on the purity of interest.

### [Production method AC]

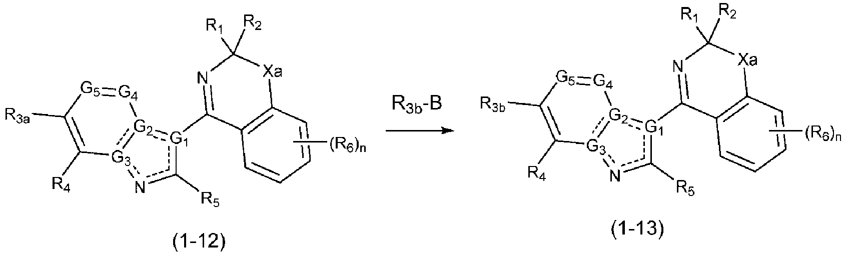

In the formulas, R₃ₐ represents a halogen atom, R_{3b} represents a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, or a C2 to C6 haloalkenyl group, R_{3b}-B represents organic boronic acids, and R₁, R₂, R₄, R₅, R₆, G₁, G₂, G₃, G₄, G₅, Xa, the binding containing the broken line, and n are as defined above.

The production method AC is a method for synthesizing the compound of the present invention represented by the formula (1-13) and is a production method comprising reacting the compound of the present invention represented by the formula (1-12) with organic boronic acids (R_{3b}-B) by Suzuki-Miyaura coupling in a solvent in the presence of transition metals and a base.

The compound of the present invention represented by the formula (1-13) for use in this reaction can be produced with reference to the production method G, the production method I, the production method J, the production method K, the production method O, the production method S, the production method T, the production method U, the production method X, the production method Y, or Reference Examples.

The production method AC can be carried out in accordance with the production method AB by using the compound of the present invention represented by the formula (1-12) instead of the compound of the present invention represented by the formula (1-10) in the production method AB.

### [Production method AD]

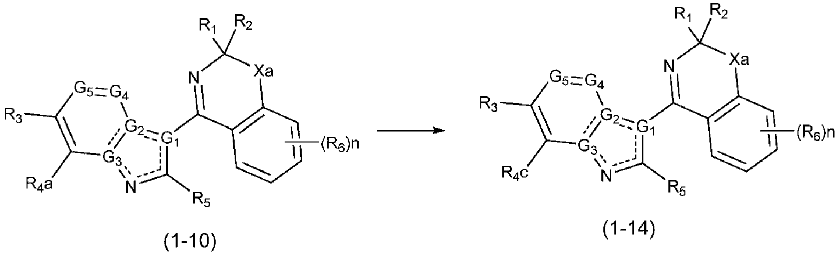

In the formulas, R_{4c} represents a C2 to C6 alkynyl group optionally substituted by substituent A, a C2 to C6 haloalkynyl group, or a C2 to C6 haloalkenyl group, and R₁, R₂, R₃, R₄ₐ, R₅, R₆, G₁, G₂, G₃, G₄, G₅, Xa, the binding containing the broken line, and n are as defined above.

The production method AD is a method for synthesizing the compound of the present invention represented by the formula (1-14) and is a production method comprising reacting the compound of the present invention represented by the formula (1-10) with a terminal alkyne compound by Sonogashira coupling in a solvent in the presence of transition metals and a base.

The terminal alkyne compound for use in this reaction can be obtained as a commercially available product or produced by a method known in the art. Trimethylsilylacetylene may be used as the terminal alkyne compound. In this case, it is necessary to introduce a trimethylsilylethynyl group to the compound of the present invention represented by the formula (1-10), followed by desilylation. The desilylation can be performed with reference to non patent literatures such as Journal of the American Chemical Society, Vol. 131, No. 2, p. 634-643 (2009) and Journal of Organometallic Chemistry, Vol. 696, No. 25, p. 4039-4045 (2011).

The amount of the terminal alkyne compound for use in this reaction can be 1 equivalent or more based on the compound of the present invention represented by the formula (1-10) and is not particularly limited as long as the reaction of interest proceeds. The amount is preferably 1 equivalent or more and 10 equivalents or less.

The transition metals for use in this reaction may have a ligand and are, for example, palladiums such as palladium acetate, [1,1'-bis(diphenylphosphino)ferrocene]palladium dichloride, tris(dibenzylideneacetone)dipalladium, tetrakis(triphenylphosphine)palladium, and bis(triphenylphosphine)palladium dichloride. Coppers such as copper chloride, copper bromide, and copper iodide are also used at the same time therewith.

The amounts of the transition metals for use in this reaction can be 0.001 equivalents or more each of the palladiums, etc. and the coppers based on the compound of the present invention represented by the formula (1-10) and are not particularly limited as long as the reaction of interest proceeds. Both the amounts are preferably 0.001 equivalents or more and 1 equivalent or less.

Examples of the base for use in this reaction include organic amines such as triethylamine, tributylamine, isopropylamine, diethylamine, diisopropylamine, and diisopropylethylamine, and inorganic bases such as sodium carbonate, potassium carbonate, and cesium carbonate.

The amount of the base for use in this reaction can be 1 equivalent or more based on the compound of the present invention represented by the formula (1-10) and is not particularly limited as long as the reaction of interest proceeds. The amount is preferably 1 equivalent or more and 50 equivalents or less. The organic base in a liquid state can be used as a solvent.

In order to allow this reaction to proceed efficiently, a phosphine ligand such as tri-t-butylphosphine or 2-dicyclohexylphosphino-2'4'6'-triisopropylbiphenyl can be added, albeit not essential.

The amount of the phosphine ligand for use in this reaction is 0.001 equivalents or more and 1 equivalent or less based on the compound of the present invention represented by the formula (1-10). The amount is not particularly limited as long as the reaction of interest proceeds.

The solvent for use in this reaction is not particularly limited as long as the reaction of interest proceeds. Examples thereof include ether solvents such as diethyl ether, diisopropyl ether, methyl-t-butyl ether, dimethoxyethane, tetrahydrofuran, and dioxane, benzene solvents such as benzene, toluene, xylene, mesitylene, chlorobenzene, and dichlorobenzene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, urea solvents such as 1,3-dimethyl-2-imidazolidinone, halogen solvents such as dichloromethane, dichloroethane, chloroform, and carbon tetrachloride, and organic amine solvents such as triethylamine, tributylamine, isopropylamine, diethylamine, diisopropylamine, and diisopropylethylamine. These solvents can be used each singly or as a mixture of two or more types at an arbitrary ratio.

The amount of the solvent for use in this reaction is not particularly limited as long as the reaction of interest proceeds. The amount is usually 3 or more times and 200 or less times the weight of the compound of the present invention represented by the formula (1-10).

The temperature in performing this reaction is not particularly limited as long as the reaction of interest proceeds. The temperature is usually 0°C or higher and 150°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, an aqueous solution containing a sulfur atom-containing salt such as sodium thiosulfate or sodium sulfite dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, and chloroform, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-14), water can be removed, albeit not essential, with a desiccant such as sodium sulfate or magnesium sulfate.

From the thus-obtained reaction mixture containing the compound represented by the formula (1-14), the solvent may be distilled off under reduced pressure without degrading the compound.

The resulting reaction mixture containing the compound of the present invention represented by the formula (1-14) from which the solvent has thus been distilled off may be used directly in the next reaction or can be purified by washing, reprecipitation, recrystallization, column chromatography, or the like using an appropriate solvent. The purification approach can be appropriately set depending on the purity of interest.

### [Production method AE]

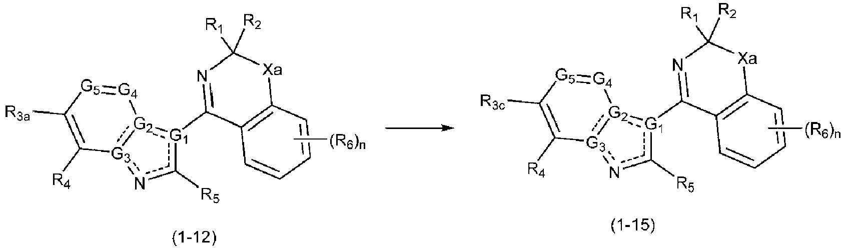

In the formulas, R_{3c} represents a C2 to C6 alkynyl group optionally substituted by substituent A, a C2 to C6 haloalkynyl group, or a C2 to C6 haloalkenyl group, and R₁, R₂, R₃ₐ, R₄, R₅, R₆, G₁, G₂, G₃, G₄, G₅, Xa, the binding containing the broken line, and n are as defined above.

The production method I is a method for synthesizing the compound of the present invention represented by the formula (1-15) and is a production method comprising reacting the compound of the present invention represented by the formula (1-12) with a terminal alkyne compound by Sonogashira coupling in a solvent in the presence of transition metals and a base.

The production method AE can be carried out in accordance with the production method AD by using the compound of the present invention represented by the formula (1-12) instead of the compound of the present invention represented by the formula (1-10) in the production method AD.

### [Production method AF]

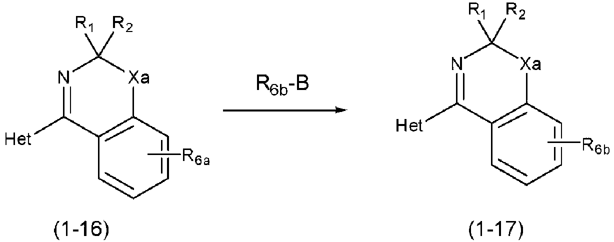

In the formulas, R₆ₐ represents a halogen atom, R_{6b} represents a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, or a C2 to C6 haloalkenyl group, R_{6b}-B represents organic boronic acids, and R₁, R₂, Xa, and Het are as defined above.

The production method AF is a method for synthesizing the compound of the present invention represented by the formula (1-17) and is a production method comprising reacting the compound of the present invention represented by the formula (1-16) with organic boronic acids (R_{6b}-B) by Suzuki-Miyaura coupling in a solvent in the presence of transition metals and a base.

The compound of the present invention represented by the formula (1-16) for use in this reaction can be produced with reference to the production method G, the production method I, the production method J, the production method K, the production method O, the production method S, the production method T, the production method U, the production method X, the production method Y, or Reference Examples.

The production method AF can be carried out in accordance with the production method AB by using the compound of the present invention represented by the formula (1-16) instead of the compound of the present invention represented by the formula (1-10) in the production method AB.

### [Production method AG]

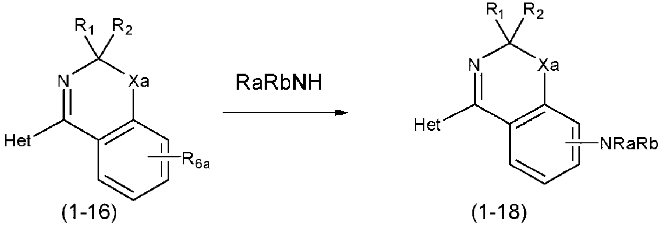

In the formulas, R₁, R₂, R₆ₐ, Xa, Het, Ra, and Rb are as defined above.

The production method AG is a production method for obtaining the compound of the present invention represented by the formula (1-18) and is a production method comprising reacting the compound of the present invention represented by the formula (1-16) with a compound represented by RaRbNH by coupling in a solvent in the presence of transition metals and a base.

RaRb-NH for use in this reaction can be obtained as a commercially available product or produced by a method known in the art.

RaRb-NH for use in this reaction can be of 1 equivalent or more based on the compound of the present invention represented by the formula (1-16). The amount is not particularly limited as long as the reaction of interest proceeds. The amount is preferably 1 equivalent or more and 100 equivalents or less. RaRbNH in a liquid state can be used as a solvent.

The transition metals for use in this reaction may have a ligand and are, for example, coppers such as copper powders, copper chloride, copper bromide, copper iodide, and copper acetate, and palladiums such as palladium acetate, [1,1'-bis(diphenylphosphino)ferrocene]palladium dichloride, tris(dibenzylideneacetone)dipalladium, tetrakis(triphenylphosphine)palladium, and bis(triphenylphosphine)palladium dichloride.

The amounts of the transition metals for use in this reaction can be 0.001 equivalents or more each of the transition metals based on the compound of the present invention represented by the formula (1-16) and are not particularly limited as long as the reaction of interest proceeds. Both the amounts are preferably 0.001 equivalents or more and 1 equivalent or less.

In order to allow this reaction to proceed efficiently, a phosphine ligand such as triphenylphosphine, 1,1'-bis(diphenylphosphino)ferrocene, tri-t-butylphosphine, 2-dicyclohexylphosphino-2'4'6'-triisopropylbiphenyl, or 4,5-bis(diphenylphosphino)-9,9-dimethylxanthene can be added for the palladium transition metal used. Amine ligands such as N,N'-dimethylcyclohexane-1,2-diamine or N,N'-dimethylethylenediamine, or oxime ligands such as 1-(pyridin-2-yl)ethan-1-one oxime can be added, albeit not essential, for the copper transition metal used.

The amount of the ligand for use in this reaction is 0.001 equivalents or more and 1 equivalent or less based on the compound of the present invention represented by the formula (1-16). The amount is not particularly limited as long as the reaction of interest proceeds.

The base for use in this reaction are, for example, inorganic bases such as sodium carbonate, potassium carbonate, cesium carbonate, and tripotassium phosphate, metal alkoxides such as sodium methoxide, sodium ethoxide, and potassium t-butoxide, and organic bases such as triethylamine, tributylamine, and diisopropylethylamine.

The amount of the base for use in this reaction can be 1 equivalent or more based on the compound represented by the formula (1-17) and is not particularly limited as long as the reaction of interest proceeds. The amount is preferably 1 equivalent or more and 50 equivalents or less.

The solvent for use in this reaction is not particularly limited as long as the reaction of interest proceeds. Examples thereof include organic amine solvents represented by RaRbNH, alcohol solvents such as methanol, ethanol, isopropyl alcohol, and t-butyl alcohol, ether solvents such as diethyl ether, diisopropyl ether, methyl-t-butyl ether, dimethoxyethane, tetrahydrofuran, and dioxane, benzene solvents such as benzene, toluene, xylene, and mesitylene, ester solvents such as ethyl acetate, isopropyl acetate, and butyl acetate, nitrile solvents such as acetonitrile, amide solvents such as N-methylpyrrolidone, N,N-dimethylformamide, and N,N-dimethylacetamide, and urea solvents such as 1,3-dimethyl-2-imidazolidinone. These solvents can be used each singly or as a mixture of two or more types at an arbitrary ratio.

The amount of the solvent for use in this reaction is not particularly limited as long as the reaction of interest proceeds. The amount is usually 3 or more times and 200 or less times the weight of the compound of the present invention represented by the formula (1-16).

The temperature in performing this reaction is not particularly limited as long as the reaction of interest proceeds. The temperature is usually 0°C or higher and 180°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, an aqueous solution containing a sulfur atom-containing salt such as sodium thiosulfate or sodium sulfite dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, and chloroform, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-18), water can be removed, albeit not essential, with a desiccant such as sodium sulfate or magnesium sulfate.

From the thus-obtained reaction mixture containing the compound of the present invention represented by the formula (1-18), the solvent may be distilled off under reduced pressure without degrading the compound.

The resulting reaction mixture containing the compound of the present invention represented by the formula (1-18) from which the solvent has thus been distilled off may be used directly in the next reaction or can be purified by washing, reprecipitation, recrystallization, column chromatography, or the like using an appropriate solvent. The purification approach can be appropriately set depending on the purity of interest.

### [Production method AH]

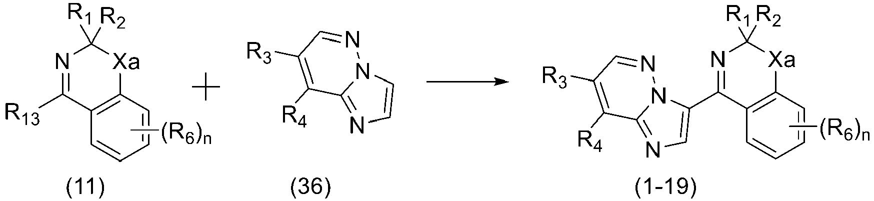

In the formulas, R₁, R₂, R₃, R₄, R₆, R₁₃, Xa, and n are as defined above.

The production method AG is a production method for obtaining the compound of the present invention represented by the formula (1-19) and is a production method comprising reacting a compound represented by the formula (11) with a imidazopyridazine derivative (36) by cross coupling via C-H activation in a solvent in the presence of transition metals and a base.

The compound represented by the formula (36) for use in this reaction can be obtained as a commercially available product or produced by a method known in the art.

The amount of the compound represented by the formula (36) for use in this reaction can be 1 equivalent or more based on the compound represented by the formula (11) and is not particularly limited as long as the reaction of interest proceeds. The amount is preferably 1 equivalent or more and 10 equivalents or less.

The transition metals for use in this reaction are, for example, palladium, nickel, or ruthenium and may have a ligand. Examples thereof preferably include palladiums such as palladium acetate, [1,1'-bis(diphenylphosphino), ferrocene]palladium dichloride, tris(dibenzylideneacetone)dipalladium, tetrakis(triphenylphosphine)palladium, bis(triphenylphosphine), and palladium dichloride.

The amount of the transition metals for use in this reaction is 0.001 equivalents or more and 1 equivalent or less based on the compound represented by the formula (11). The amount is not particularly limited as long as the reaction of interest proceeds.

In order to allow this reaction to proceed efficiently, a phosphine ligand such as triphenylphosphine, tricyclohexylphosphine, or tri-tert-butylphosphonium tetrafluoroborate can be added.

The amount of the phosphine ligand for use in this reaction is 0.001 equivalents or more and 1 equivalent or less based on the compound represented by the formula (11). The amount is not particularly limited as long as the reaction of interest proceeds.

Examples of the base for use in this reaction include inorganic bases such as sodium carbonate, potassium carbonate, cesium carbonate, and tripotassium phosphate, and metal alkoxides such as sodium methoxide, sodium ethoxide, and potassium t-butoxide.

The amount of the base for use in this reaction can be 1 equivalent or more based on the compound represented by the formula (11) and is not particularly limited as long as the reaction of interest proceeds. The amount is preferably 1 equivalent or more and 50 equivalents or less.

The solvent for use in this reaction is not particularly limited as long as the reaction of interest proceeds. Examples thereof include water solvents, ether solvents such as diethyl ether, diisopropyl ether, methyl-t-butyl ether, dimethoxyethane, tetrahydrofuran, and dioxane, and benzene solvents such as benzene, toluene, xylene, mesitylene, chlorobenzene, and dichlorobenzene. These solvents can be used each singly or as a mixture of two or more types at an arbitrary ratio.

The amount of the solvent for use in this reaction is not particularly limited as long as the reaction of interest proceeds. The amount is usually 3 or more times and 200 or less times the weight of the compound represented by the formula (11).

The temperature in performing this reaction is not particularly limited as long as the reaction of interest proceeds. The temperature is usually 30°C or higher and 100°C or lower or a temperature equal to or lower than the boiling point of the solvent.

In the aftertreatment of the reaction, the reaction mixture may be subjected to phase separation operation by the addition of water or an appropriate aqueous solution. In the case of using an aqueous solution, for example, an acidic aqueous solution containing hydrochloric acid, sulfuric acid, or ammonium chloride dissolved therein, an alkaline aqueous solution containing potassium hydroxide, sodium hydroxide, sodium carbonate, potassium carbonate, sodium bicarbonate, or potassium bicarbonate dissolved therein, an aqueous solution containing a sulfur atom-containing salt such as sodium thiosulfate or sodium sulfite dissolved therein, or saline can be arbitrarily used. For the phase separation operation, if necessary, a solvent incompatible with water may be further added, examples of which include benzene solvents such as toluene, xylene, benzene, chlorobenzene, and dichlorobenzene, ester solvents such as ethyl acetate, isopropyl acetate, and butyl acetate, ether solvents such as diethyl ether, diisopropyl ether, and methyl-t-butyl ether, halogen solvents such as dichloromethane, dichloroethane, and chloroform, and hydrocarbon solvents such as hexane, heptane, cyclohexane, and methylcyclohexane. These solvents may be used each singly or as a mixture of two or more types at an arbitrary ratio. The number of times of phase separation is not particularly limited, and the phase separation can be carried out depending on the purity or yield of interest.

From the thus-obtained reaction mixture containing the compound of the present invention represented by the formula (1-19), water can be removed, albeit not essential, with a desiccant such as sodium sulfate or magnesium sulfate.

From the thus-obtained reaction mixture containing the compound of the present invention represented by the formula (1-19), the solvent may be distilled off under reduced pressure without degrading the compound.

The resulting reaction mixture containing the compound of the present invention represented by the formula (1-19) from which the solvent has thus been distilled off may be used directly in the next reaction or can be purified by washing, reprecipitation, recrystallization, column chromatography, or the like using an appropriate solvent. The purification approach can be appropriately set depending on the purity of interest.

The compound represented by the formula (1) can be produced by arbitrarily combining the production method A to the production method AH described above. Alternatively, the compound represented by the formula (1) can also be produced by arbitrarily combining a method known in the art and the production method A to the production method AH.

### [Agricultural and horticultural pest control agent]

One embodiment of the present invention is an agricultural and horticultural pest control agent comprising a compound represented by the formula (1) or a salt thereof (hereinafter, also simply referred to as a "compound (1)"). The compound (1) can expel or prevent a harmful organism against, particularly, a plant or a portion of a plant (fruits, flowers, leaves, stems, rhizome, roots, seeds, etc.) and can also protect the plant or a portion of the plant from a harmful organism. The compound (1) is therefore preferably used as an agricultural and horticultural pest control agent, for example, as a germicide, an insecticide, an herbicide, or a plant growth regulator, and more preferably used as an agricultural and horticultural germicide. Specifically, one embodiment of the present invention may be an agricultural and horticultural germicide comprising a compound (1). Hereinafter, the agricultural and horticultural pest control agent and the agricultural and horticultural germicide are also referred to as an "agricultural and horticultural pest control agent, etc."

Examples of the harmful organism for plants include fungi, bacteria, spiroplasmas, phytoplasmas, viruses, viroids, parasitic higher plants, and nematodes. The compound (1) is effective, particularly, for the control of fungi. These harmful organisms might cause plant diseases and might cause, for example, systemic abnormal pathological symptoms such as wilting, damping-off, yellowing, dwarfism, or spindly growth, or partial pathological symptoms such as spotting, leaf blight, mosaic pattern, leaf rolling, die back, root rot, club root, or knotting in plants such as crops, ornamental plants, ornamental trees and shrubs, or trees.

Examples of the harmful fungi include fungi that cause fungal diseases (i.e., pathogens), for example, *Plasmodiophora*, *Oomycetes*, *Zygomycetes*, *Ascomycetes*, *Basidiomycetes*, and *Deuteromycetes*. Examples of the *Plasmodiophora* include club root fungus, potato powdery scab fungus, and beet necrotic yellow vein virus. Examples of the *Oomycetes* include blight fungus, downy mildew fungus, *Pythium* species, and *Aphanomyces* species. Examples of the *Zygomycetes* include *Rhizopus* species. Examples of the *Ascomycetes* include peach leaf curl fungus, southern corn leaf blight fungus, rice blast fungus, powdery mildew virus, anthracnose fungus, fusarium head blight fungus, bakanae fungus, and stem rot fungus. Examples of the *Basidiomycetes* include rust fungus, smut fungus, violet root rot fungus, rice cake disease fungus, and rice sheath blight fungus. Examples of the *Deuteromycetes* include, gray mold fungus, *Alternaria* species, *Fusarium* species, *Penicillium* species, *Rhizoctonia* species, and Southern blight fungus.

The agricultural and horticultural pest control agent of the present embodiment is effective against following plant diseases. Examples of the names of plant diseases and their pathogens are shown but are not limited to the following:
rice blast disease (*Magnaporthe grisea*), sheath blight disease (*Thanatephorus cucumeris*), brown sclerotium disease (*Ceratobasidium setariae*), brown small sclerotium disease (*Waitea circinata*), brown sheath blight disease (*Thanatephorus cucumeris*), globular sclerotium disease (*Sclerotium hydrophilum*), red sclerotium disease (*Wairea circinata*), leaf smut (*Entyloma dactylidis*), stem rot (*Magnaporthe salvinii*), gray sclerotium disease (*Ceratobasidium cornigerum*), brown spot (*Cochliobolus miyabeanus*), Cercospora leaf spot (*Sphaerulina oryzina*), Bakanae disease (*Gibberella fujikuroi*), seeding blight (*Pythium* spp., *Fusarium* spp., *Trichoderma* spp., Rhizopus spp., *Rhizoctonia solani*, *Mucor* sp., and *Phoma* sp.), seed and seedling rot (*Pythium* spp., *Achlya* spp., and *Dictyuchus* spp.), false smut (*Claviceps virens*), kernel smut (*Tilletia barclayana*), brown spot (*Curvularia* spp. and *Alternaria* spp.), downy mildew (*Sclerophthora macrospora*), bacterial leaf blight *(Xanthomonas oryzae* pv. *oryzae),* bacterial brown stripe *(Acidovorax avenae* subsp. *avenae),* bacterial palea browning *(Erwinia ananas),* bacterial seedling blight *(Burkholderia plantarii),* bacterial grain rot *(Burkholderia glumae),* sheath brown rot *(Pseudomonas fuscovaginae),* halo blight *(Pseudomonas syringae* pv. *oryzae),* bacterial foot rot *(Erwinia chrysanthemi),* yellow dwarf *(Phytoplasma oryzae),* stripe *(Ricestripe tenuivirus),* and dwarf (Rice dwarf reovirus);
barley, wheat, and similar grains powdery mildew *(Blumeriagra minis* f*.* sp. *hordei;* f. sp. *tritici),* rust disease *(Puccinia striiformis, Puccinia graminis, Puccinia recondita,* and *Puccinia hordei),* mottle leaf *(Pyrenophora graminea),* net blotch *(Pyrenophora teres),* scab *(Gibberella zeae, Fusarium culmorum, Fusarium avenaceum,* and *Monographella nivalis),* snow rot *(Typhula incarnata, Typhula ishikariensis,* and *Monographella nivalis),* loose smut *(Ustilago nuda),* bunt *(Tilletia caries* and *Tilletia controversa),* eyespot *(Pseudocercosporella herpotrichoides),* bacterial foot rot *(Ceratobasidium gramineum),* scald *(Rhynchosporium secalis),* leaf blotch (Septoria tritici), glume blotch *(Phaeosphaeria nodorum),* leaf spot (Ramularia collo-cygni), rice blast (Pyricularia oryzae), seeding blight *(Fusarium* spp., *Pythium* spp., *Rhizoctonia* spp., *Septoria* spp., and *Pyrenophora* spp.), take-all *(Gaeumannomyces graminis),* anthracnose *(Colletotrichum graminicola),* ergot *(Claviceps purpurea),* spot blotch *(Cochliobolus sativus),* and bacterial black node *(Pseudomonas syringae* pv. *syringae);*
corn scab (*Gibberella zeae,* etc.), seeding blight *(Fusarium avenaceum, Penicillium* spp, *Pythium* spp., and *Rhizoctonia* spp.), rust disease *(Puccinia sorghi),* brown spot *(Cochliobolus heterostrophus),* smut *(Ustilago maydis),* anthracnose *(Colletotrichum graminicola),* Northern leaf spot *(Cochliobolus carbonum),* Gray Leaf Spot (Cercospora zeae-maydis), bacterial brown stripe *(Acidovorax avenae* subsp. *avenae),* bacterial stripe *(Burkholderia andropogonis),* bacterial stalk rot (Erwinia chrysanthemi pv. *zeae),* and bacterial wilt *(Erwinia stewartii);*
grape downy mildew *(Plasmopara viticola),* rust disease *(Physopella ampelopsidis),* powdery mildew *(Uncinula necator),* bird's eye rot *(Elsinoe ampelina),* ripe rot *(Glomerella cingulata* and *Colletotrichum acutatum),* black rot *(Guignardia bidwellii),* dead arm *(Phomopsis viticola),* fly speck *(Zygophiala jamaicensis),* gray mold *(Botrytis cinerea),* bud blight *(Diaporthe medusaea),* violet root rot *(Helicobasidium mompa),* white root rot *(Rosellinia necatrix),* and crown gall *(Agrobacterium vitis);*
apple powdery mildew (*Podosphaera leucotricha),* scab *(Venturia inaequalis),* Alternaria blotch *(Alternaria mali),* rust *(Gymnosporangium yamadae),* Monilia leaf blight *(Monilinia mali),* Valsa canker *(Valsa ceratosperma),* ring rot *(Botryosphaeria berengeriana),* anthracnose *(Colletotrichum acutatum* and *Glomerella cingulata),* fly speck *(Zygophiala jamaicensis),* sooty blotch *(Gloeodes pomigena),* fruit spot *(Mycosphaerella pomi),* violet root rot *(Helicobasidium mompa),* white root rot *(Rosellinia necatrix),* Phomopsis canker *(Phomopsis mali* and *Diaporthe tanakae),* blotch *(Diplocarpon mali),* fire blight *(Erwinia amylovora),* crown gall *(Agrobacterium tumefaciens),* and hairy root (Agrobacterium rhizogenes);
pear black spot *(Alternaria kikuchiana),* scab *(Venturia nashicola),* rust *(Gymnosporangium asiaticum),* ring rot *(Botryosphaeria berengeriana f.* sp. *piricola),* Phomopsis canker *(Phomopsis fukushii),* bacterial shoot blight *(Erwinia* sp.), crown gall *(Agrobacterium tumefaciens),* Erwinia rusty canker *(Erwinia chrysanthemi* pv. *chrysanthemi),* and bacterial blossom blight and canker *(Pseudomonas syringae* pv. *syringae);*
common pear Phytophthora blight *(Phytophthora cactorum* and *Phytophthora syringae)* and bacterial shoot blight *(Erwinia* sp.);
peach scab (*Cladosporium carpophilum*), Phomopsis rot (*Phomopsis* sp.), Phytophthora blight (*Phytophthora* sp.), anthracnose (*Colletotrichum gloeosporioides*), leaf curl (*Taphrina deformans)*, bacterial shot hole (*Xhanthomonas campestris* pv. *pruni*), and crown gall (*Agrobacterium tumefaciens*);
yellow peach anthracnose (*Glomerella cingulata*), young fruit rot (*Monilinia kusanoi*), brown rot (*Monilinia fructicola*), crown gall (*Agrobacterium tumefaciens*), and resinosis (*Pseudomonas syringae* pv. *syringae*);
Japanese persimmon anthracnose (*Glomerella cingulata*), angular leaf spot (*Cercospora kaki*; *Mycosphaerella nawae*), powdery mildew (*Phyllactinia kakikora*), *and* crown gall (*Agrobacterium tumefaciens*);
citrus fruit spot (*Diaporthe citri*), common green mold (*Penicillium digitatum*), blue mold (*Penicillium italicum*), spot anthracnose (*Elsinoe fawcettii*), brown rot (*Phytophthora citrophthora*), canker (*Xhanthomonas campestris* pv. *citri*), bacterial brow spot (*Pseudomonas syringae* pv. *syringae*), citrus huanglongbing HLB (*Liberibactor asiaticus*), and crown gall (Agrobacterium tumefaciens);
gray mold of tomato, cucumber, beans, strawberry, potato, cabbage, eggplant, lettuce, etc. (*Botrytis cinerea*);
sclerotium disease of tomato, cucumber, beans, strawberry, potato, rapeseed, cabbage, eggplant, lettuce, etc. (*Sclerotinia sclerotiorum*);
seeding blight of various vegetables such as tomato, cucumber, beans, daikon radish, watermelon, eggplant, rapeseed, bell pepper, spinach, and sugar beet (*Rhizoctonia* spp., *Pythium* spp., *Fusarium* spp., *Phythophthora* spp., *Sclerotinia sclerotiorum*, etc.);
solanaceous plant bacterial wilt disease (*Ralstonia solanacearum*);
gourd downy mildew (*Pseudoperonospora cubensis*), powdery mildew (*Sphaerotheca fuliginea*), anthracnose (*Colletotrichum orbiculare*), gummy stem blight (*Didymella bryoniae*), dead arm (*Fusarium oxysporum*), and Phytophthora blight (*Phytophthora parasitica*, *Phytophthora melonis*, *Phytophthora nicotianae*, *Phytophthora drechsleri*, *Phytophthora capsici*, etc.), bacterial brow spot (*Xhanthomonas campestris* pv. *cucurbitae*), soft rot (*Erwinia carotovora* subsp. *carotovora*), bacterial spot (*Pseudomonas syringae* pv. *lachrymans*), marginal blight (*Pseudomonas marginalis* pv. *marginalis*), root tumor (*Streptomyces* sp.), hairy root (*Agrobacterium rhizogenes*), and mosaic (cucumber mosaic virus);
tomato ring rot (*Alternaria solani*), leaf mold (*Fulvia fulva*), Phytophthora blight (*Phytophthora infestans*), Fusarium wilt (*Fusarium oxysporum*), root rot (*Pythium myriotylum* and *Pythium dissotocum*), anthracnose (Colletotrichum gloeosporioides), canker (*Clavibacter michiganensis*), pith necrosis (*Pseudomonas corrugata*), bacterial black spot (*Pseudomonas viridiflava*), soft rot (*Erwinia carotovora* subsp. *carotovora*), bacterial leaf gall (*Crynebacterium* sp.), yellows (*Phytoplasma asteris*), and downy mildew (Tobacco leaf curl subgroup III geminivirus);
eggplant powdery mildew (*Sphaerotheca fuliginea*, etc.), leaf mold (*Mycovellosiella nattrassii*), Phytophthora blight (*Phytophthora infestans*), brown rot (*Phytophthora capsici*), bacterial brow spot (*Pseudomonas cichorii*), pith necrosis (*Pseudomonas corrugata*), bacterial stem rot (*Erwinia chrysanthemi*), soft rot (*Erwinia carotovora* subsp. *carotovora*), and bacterial spot (*Pseudomonas* sp.);
rapeseed black spot (*Alternaria brassicae*), black rot (*Xhanthomonas campestris* pv. *campestris*), bacterial black spot (*Pseudomonas syringae* pv. *maculicola*), anthracnose (*Colletotrichum higgincianum*), Black leaf spot (*Alternaria brassicicola*), White rust (*Albugo macrospora*), and soft rot (*Erwinia carotovora*);
cruciferous vegetable black spot (*Alternaria brassicae*, etc.), leaf spot (*Cercosporella brassicae*), root rot (*Phoma lingam*), clubroot (*Plasmodiophora brassicae*), downy mildew (P*eronospora parasitica*), black rot (*Xhanthomonas campestris* pv. *campestris*), bacterial black spot (*Pseudomonas syringae* pv. *maculicola*), and soft rot (*Erwinia carotovora* subsp. *carotovora*);
cabbage bacterial foot rot (*Thanatephorus cucumeris*), yellows (*Fusarium oxysporum*), and Alternaria sooty spot (*Alternaria brassisicola*);
napa bottom rot (*Rhizoctonia solani*) and yellows (*Verticilliumd ahliae*);
green onion rust disease (*Puccinia allii*), black spot (*Alternaria porri*), Southern blight (*Sclerotium rolfsii*), white blight (*Phytophthora porri*), white rot (*Sclerotium cepivorum*), fusarium wilt (*Fusarium oxysporum*), downy mildew (*Peronospora destructor*), small sclerotial (*Botrytis squamosa*), and leaf blight (*Stemphylium sp.*);
onion canker (*Curtobacterium flaccumfaciens*), soft rot (*Erwinia carotovora* subsp. *carotovora*), bacterial spot (*Pseudomonas syringae* pv. *syringae*), rot disease (*Erwinia rhapontici*), center rot (*Burkholderia gladioli*), yellows (*Phytoplasma asteris*);
garlic soft rot (*Erwinia carotovora* subsp. *carotovora*) and spring rot (*Pseudomonas marginalis* pv. *marginalis*);
soybean purple stein (*Cercospora kikuchii*), bird's eye rot (*Elsinoe glycines*), fruit spot (*Diaporthe phaseolorum*), Rhizoctonia root rot (*Rhizoctonia solani*), Phytophthora stem blight (*Phytophthora sojae*), downy mildew (*Peronospora manshurica*), rust disease (*Phakopsora pachyrhizi*), anthracnose (*Colletotrichum truncatum*, etc.), target spot (*Corynespora cassicola*), leaf spot (*Cercospora sojina*), septoria brown spot (*Septoria glycines*), powdery mildew (*Erysiphe glycines*, etc.), charcoal rot (*Macrophomina phaseolina*), bacterial pustule (*Xhanthomonas campestris* pv. glycines), and bacterial spot (*Pseudomonas syringae* pv. *glycinea*);
kidney bean anthracnose (*Colletotrichum lindemuthianum*), bacterial wilt disease (*Ralstonia solanacearum*), halo blight (*Pseudomonas syringae* pv. *phaseolicola*), bacterial brow spot (*Pseudomonas viridiflava*), and bacterial pustule (*Xhanthomonas campestris* pv. *phaseoli*);
peanut leaf spot (*Mycosphaerella berkeleyi*), blotch (*Mycosphaerella arachidis*), and bacterial wilt disease (*Ralstonia solanacearum*);
pea powdery mildew (*Erysiphe pisi*), downy mildew (*Peronospora pisi*), bacterial blight (*Pseudomonas syringae* pv. *pisi*), and bacterial stem rot (*Xhanthomonas campestris* pv. *pisi*);
broad bean downy mildew (*Peronospora viciae*) and Phytophthora blight (*Phytophthora nicotianae*);
potato early blight (*Alternaria solani*), black scurf (*Thanatephorus cucumeris*), Phytophthora blight (*Phytophthora infestans*), silver scurf (*Helminthosporium solani*), dry rot (*Fusarium oxysporum* and *Fusarium solani*), powdery scab (*Spongospora subterranea*), bacterial wilt disease (*Ralstonia solanacearum*), black leg (*Erwinia carotovora* subsp. *atroseptica*), spot anthracnose (*Streptomyces scabies* and *Streptomyces acidiscabies*), soft rot (*Erwinia carotovora* subsp. *carotovora*), slimy rot (*Crostridium* spp.), and ring rot (*Clavibacter michiganensis* subsp. *sepedonicus*);
sweet potato foot rot (*Diaporthe destruens*) and take-all (*Streptomyces ipomoeae*);
sugar beet blotch (*Cercospora beticola*), downy mildew (*Peronospora schachtii*), Aphanomyces root rot (*Aphanomyces cochioides*), Phoma root rot (*Phoma betae*), crown gall (*Agrobacterium tumefaciens*), powdery mildew (*Erysiphe glycines*, etc.), leaf spot (*Ramularia beticola*), spot anthracnose (*Streptomyces scabies*), and bacterial spot (*Pseudomonas syringae* pv. *aptata*);
carrot leaf blight (*Alternaria dauci*), bacterial gall (*Rhizobacter dauci*), crown gall (*Agrobacterium tumefaciens*), Streptomyces scab (*Streptomyces* spp.), and soft rot (*Erwinia carotovora* subsp. *carotovora*);
strawberry powdery mildew (*Sphaerotheca aphanis* var. *aphanis*), Phytophthora blight (*Phytophthora nicotianae*, etc.), anthracnose (*Glomerella cingulate*, etc.), fruit rot (*Pythium ultimum*), bacterial wilt disease (*Ralstonia solanacearum*), angular leafspot disease (*Xhanthomonas campestris*), and bacterial blight (*Pseudomonas marginalis* pv. *marginalis*);
tea net blister blight (*Exobasidium reticulatum*), white scab (*Elsinoe leucospila*), anthracnose (*Colletotrichum theae-sinensis*), gray blight (*Pestalotiopsis longiseta*), bacterial shoot blight (*Pseudomonas syringae* pv. *theae*), canker (*Xhanthomonas campestris* pv. *theicola*), and witches' broom (*Pseudomonas* sp.);
tobacco rust (*Alternaria alternata*), powdery mildew (*Erysiphe cichoracearum*), anthracnose (*Colletotrichum gloeosporioides*), Phytophthora blight (*Phytophthora nicotianae*), wild fire (*Pseudomonas syringae* pv. *tabaci*), Wisconsin bacterial leaf spot (*Pseudomonas syringae* pv. *mellea*), hollow stalk rot (*Erwinia carotovora* subsp. *carotovora*), take-all (*Ralstonia solanacearum*), and mosaic (tobacco mosaic virus);
coffee rust disease (*Hemileia vastatrix*);
banana black Sigatoka disease (*Mycosphaerella minima, Mycosphaerella musae,Mycosphaerella musicola, Cercospora musae, Metasphaeria musae*), yellow Sigatoka disease (*Pseudocercospora musae*), Panama disease (*Fusarium oxysporum*), Bunchy top (*Banana bunchy top virus*), mosaic disease (*Cucumber mosaic virus*), peduncle rot (*Ceratocystis paradoxa, Colletotrichum musae, Lasiodiplodia theobromae,Botryodiplodia theobromae*), fruit drop disease (*Colletotrichum musae*), black rot (*Botryodiplodia theobromae*), scab (*Macrophoma musae*), black mold (*Rhizopus stolonifer*), fine line disease (*Cercospora musaecola*), southern sclerotium blight (*Sclerotium rolfsii*), anthracnose (*Colletotrichum musae*), round spot (*Scolecotrichum musae*), and stem rot (*Marasmius semiustus*);
sunflower sclerotium disease (*Sclerotinia sclerotiorum*), angular leaf spot (*Xhanthomonas campestris* pv. *malvacearum*), hollow root (*Erwinia carotovora* subsp. *carotovora*), and bacterial spot (Pseudomonas syringae pv. *helianthi*);
rose scab (*Diplocarpon rosae*), powdery mildew (*Sphaerotheca pannosa*, etc.), Phytophthora blight (*Phytophthora megasperma*), downy mildew (*Peronospora sparsa*), and crown gall (*Agrobacterium tumefaciens*);
chrysanthemums blotch (*Septoria obesa*), white rust (*Puccinia horiana*), Phytophthora blight (*Phytophthora cactorum*), bacterial spot (*Pseudomonas cichorii*), soft rot (Erwinia carotovora subsp. *carotovora*), crown gall (*Agrobacterium tumefaciens*), hairy root (*Agrobacterium rhizogenes*), and chrysanthemum virescence (*Phytoplasma aurantifolia*); and
grass brown patch disease (*Rhizoctonia solani*), dollar spot disease (*Sclerotinia homoeocarpa*), Curvularia leaf blight (*Curvularia* sp.), rust disease (*Puccinia zoysiae*), helminthosporium leaf blight (*Cochliobolus* sp.), scald (*Rhynchosporium secalis*), take-all (*Gaeumannomyces graminis*), anthracnose (*Colletotrichum* sp.), Typhula brown snow blight (*Typhula incarnata*), Typhula black snow blight (*Typhula ishikariensis*), sclerotinia (*Myriosclerotinia borealis*), fairy ring disease (*Marasmius oreades*, etc.), Pythium blight (*Pythium aphanidermatum*, etc.), and blast disease(*Pyricularia grisea*), yellow patch (Rhizoctonia binucleate), halo blight (Pseudomonas syringae pv. atropurprea), bacterial brown stripe (Acidovorax avenae), Drechslera leaf blight (Drechslera sp.), Bipolaris leaf blight (Bipolaris sorokiniana), Melting-out (Drechslera poae), Summer patch (Magnaporthe poae), and Necrotrophic ringspot disease (Ophiosphaerella korrae,Ophiosphaerella herpotricha, Ophiosphaerella spp.).

The agricultural and horticultural pest control agent, etc. of the present embodiment may comprise one or two or more compounds (1) and preferably comprises one compound (1).

In the present embodiment, the content of the compound (1) in the agricultural and horticultural pest control agent, etc. can be an amount that permits formulation and is not particularly limited. The content is, for example, preferably 0.1 to 50% by mass, more preferably 1 to 40% by mass, further preferably 5 to 30% by mass, per 100% by mass of the agricultural and horticultural pest control agent, etc. The content of the compound (1) in the agricultural and horticultural pest control agent, etc. may be determined from the mixing ratio of components in the production of the agricultural and horticultural pest control agent, etc. and may be a content described in a product package.

The agricultural and horticultural pest control agent, etc. of the present embodiment may comprise only the compound (1) or may comprise the compound (1) and an arbitrary component. Examples of the arbitrary component include solid carriers, liquid carriers, gas carriers, colorants, solvents or oils, saccharides, watersoluble polymers, antiseptics or antifungal agents, inorganic salts, ultraviolent blockers, latexes or emulsions, fragrances, mineral powders, dispersants, stabilizers, and surfactants.

Examples of the dosage form of the agricultural and horticultural pest control agent, etc. of the present embodiment include, but are not particularly limited to, dustable powders, granules, macrogranues, tracking powders, dispersible concentrates, bait concentrates, emulsifiable concentrates, liquids, oils, flowables, aerosol dispensers, pastes, smokes, gases, embrocations, microcapsules, and packs.

### [Method for producing agricultural and horticultural pest control agent, etc.]

The method for producing the agricultural and horticultural pest control agent, etc. of the present embodiment is not particularly limited as long as a composition containing the compound (1) can be obtained by the method. A production method known in the art can be used depending on a dosage form. Examples of the method for producing the agricultural and horticultural pest control agent, etc. include a method of mixing the compound (1) with an arbitrary component.

### [Method for controlling plant disease]

One embodiment of the present invention is a method for controlling a plant disease, comprising applying an agricultural and horticultural pest control agent, etc. to a plant, a plant seed, or soil for cultivating a plant.

In the present embodiment, the plant is an organism that belongs to a biological category contrasted with animals, and generally refers to an organism that lives in a fixed location. Examples of the plant include plants that belong to fruit trees, vegetables, cereals, ornamental plants or foliage plants, trees, and other plants. Examples of the fruit trees include citruses such as mandarin orange and lemon, pomaceous fruits such as pear, apple, and Japanese persimmon, stone fleshy fruits such as peach and yellow peach, and small fruits such as grape and blueberry. Examples of the vegetables include tuberous and corm vegetables such as konjac, potato, sweet potato, and taro potato, root vegetables such as sugar beet and carrot, bulb vegetables such as onion and garlic, beans such as soybean, kidney bean, and podded pea, gourds such as cucumber, watermelon, and pumpkin, solanaceous crops such as tomato, eggplant, and bell pepper, cruciferous vegetables such as broccoli, leaf vegetables such as cabbage, lettuce, and spinach, stalk and stem vegetables such as asparagus, and edible flowers. Examples of the cereals include rice, oat, wheat, barley, rye, and corn. Examples of the ornamental plants or the foliage plants include sunflower, rose, tulip, chrysanthemums, and grass. Examples of the trees include pine, ash tree, gingko, maple, oak, poplar, and Japanese zelkova. Examples of other plants include coffee, cotton, tobacco, and sugarcane.

The plant may include genetically modified crops that are created by artificial manipulation of genes or the like and are originally absent in the natural world. Examples thereof include agricultural and horticultural crops such as soybean, corn, and cotton provided with herbicide resistance, rice and tobacco adapted to cold regions, and corn and cotton provided with the ability to produce insecticidal substances.

In the present embodiment, the seed refers to a unit that is used for agricultural production by storing nutrients for the germination of seedlings. Examples of the seed include seeds of corn, soybeans, cotton, rice, sugar beet, wheat, barley, sunflower, tomato, cucumber, eggplant, spinach, podded pea, pumpkin, sugarcane, tobacco, bell pepper, and rape, seed tubers of taro potato, potato, sweet potato, and konjak, bulbs of edible lily and tulip, seed bulbs of scallion, and seeds and tubers of genetically modified crops.

Examples of the method for applying the agricultural and horticultural pest control agent, etc. include a method of contacting the agricultural and horticultural pest control agent, etc. with a plant or a plant seed, and a method of allowing the agricultural and horticultural pest control agent, etc. to be contained in soil for cultivating a plant, and contacting the soil with a plant root or underground stem. Examples of the application method include spraying of the agricultural and horticultural pest control agent, etc. to plant stems and leaves, injection, treatment in seedling nursery boxes, treatment in cell trays, spraying to plant seeds, smearing of plant seeds, immersion of plant seeds, powder coating of plant seeds, spraying to soil surface, mixing into soil after spraying to soil surface, injection into soil, mixing into soil after injection into the soil, soil irrigation, and mixing into soil after soil irrigation. The agricultural and horticultural pest control agent, etc. according to the present embodiment can exert sufficient efficacy by any application method usually used by those skilled in the art.

The amount and concentration of the agricultural and horticultural pest control agent, etc. according to the present embodiment for application may vary depending on the target crop, the target disease, the degree of occurrence of the disease, the dosage form of the compound, the application method, and various environmental conditions, etc. In the case of spraying or irrigation, the amount of the compound (1) as an active ingredient is, for example, preferably 0.1 to 10,000 g per hectare, more preferably 10 to 1,000 g per hectare, for application. In the case of treating seeds, the amount of the compound (1) as an active ingredient is, for example, preferably 0.0001 to 1000 g per kg of the seeds, more preferably 0.001 to 100 g per kg of the seeds, for application. In the case of using the agricultural and horticultural pest control agent, etc. according to the present embodiment in spraying to plant stems and leaves, spraying to soil surface, injection into soil, or soil irrigation, the agricultural and horticultural pest control agent, etc. may be diluted into an appropriate concentration with a carrier and then applied. In the case of contacting the agricultural and horticultural pest control agent, etc. with plant seeds, the agricultural and horticultural pest control agent, etc. may be diluted into an appropriate concentration and then applied to the plant seeds by immersion, powder coating, spraying, or smearing. For the immersion, powder coating, spraying, or smearing treatment, the amount of the compound (1) as an active ingredient is, for example, preferably on the order of 0.05 to 50%, more preferably 0.1 to 30%, based on the dry weight of the plant seeds for application. However, the amount can be set depending on the form of the agricultural and horticultural pest control agent, etc. or the type of the plant seed to be treated and is not limited to these ranges.

The compound (1) or the agricultural and horticultural pest control agent, etc. of the present embodiment can be used as a mixture with, for example, other agricultural chemicals such as germicides, insecticides (including miticides and nematicides), and herbicides, agricultural chemicals such as microbial materials and plant growth regulators, agricultural chemicals such as disease control agents comprising nucleic acids as active ingredients (International Publication No. WO 2014/062775), soil improvers, or fertilizing substances. Examples of the method for controlling a plant disease by mixing the compound (1) or the agricultural and horticultural pest control agent, etc. of the present embodiment with an additional agricultural chemical include a method of using the compound (1) or the agricultural and horticultural pest control agent, etc. of the present embodiment and the additional agricultural chemical formulated in one dosage form, a method of mixing respective formulations in different dosage forms before use and using the mixture, a method of using respective formulations in different dosage forms at the same time, and a method of using any one of respective formulations in different dosage forms and then using the other formulations.

Specific components contained in the insecticide that can be used as a mixture with the compound (1) are as shown in the group b given below and include salts, isomers, and N-oxide forms thereof. However, the insecticide known in the art is not limited thereto.

Group b:

### <b-1: Carbamate-based acetylcholinesterase (AChE) inhibitor>

b-1: Examples of the carbamate-based acetylcholinesterase (AChE) inhibitor include [b-1.1] phosphocarb, [b-1.2] alanycarb, [b-1.3] butocarboxim, [b-1.4] butoxycarboxim, [b-1.5] thiodicarb, [b-1.6] thiofanox, [b-1.7] aldicarb, [b-1.8] bendiocarb, [b-1.9] benfuracarb, [b-1.10] carbaryl, [b-1.11] carbofuran, [b-1.12] carbosulfan, [b-1.13] ethiofencarb, [b-1.14] fenobucarb, [b-1.15] formetanate, [b-1.16] furathiocarb, [b-1.17] isoprocarb, [b-1.18] methiocarb, [b-1.19] methomyl, [b-1.20] oxamyl, [b-1.21] pirimicarb, [b-1.22] propoxur, [b-1.23] trimethacarb, [b-1.24] XMC (3,5-xylyl methylcarbamate), [b-1.25] allyxycarb, [b-1.26] aldoxycarb, [b-1.27] bufencarb, [b-1.28] butacarb, [b-1.29] carbanolate, [b-1.30] metolcarb, [b-1.31] xylylcarb, and [b-1.32] fenothiocarb.

### <b-2: Organophosphorus-based acetylcholinesterase (AChE) inhibitor>

b-2: Examples of the organophosphorus-based acetylcholinesterase (AChE) inhibitor include [b-2.1] acephate, [b-2.2] azamethiphos, [b-2.3] azinphos-methyl, [b-2.4] azinphos-ethyl, [b-2.5] ethephon, [b-2.6] cadusafos, [b-2.7] chlorethoxyfos, [b-2.8] chlorfenvinphos, [b-2.9] chlormephos, [b-2.10] chlorpyrifos, [b-2.11] chlorpyrifos-methyl, [b-2.12] coumaphos, [b-2.13] cyanophos, [b-2.14] demeton-S-methyl, [b-2.15] diazinon, [b-2.16] dichlofenthion, [b-2.17] dichlorvos, [b-2.18] dicrotophos, [b-2.19] dimethoate, [b-2.20] dimethylvinphos, [b-2.21] disulfoton, [b-2.22] O-ethyl O-4-nitrophenyl phenylphosphonothioate, [b-2.23] ethion, [b-2.24] ethoprophos, [b-2.25] famphur, [b-2.26] fenamiphos, [b-2.27] fenitrothion, [b-2.28] fenthion, [b-2.29] fosthiazate, [b-2.30] heptenophos, [b-2.31] isofenphos-methyl, [b-2.32] Isocarbophos, [b-2.33] isoxathion, [b-2.34] malathion, [b-2.35] mecarbam, [b-2.36] methamidophos, [b-2.37] methidathion, [b-2.38] mevinphos, [b-2.39] monocrotophos, [b-2.40] naled, [b-2.41] omethoate, [b-2.42] oxydemeton-methyl, [b-2.43] parathions, [b-2.44] parathion-methyl, [b-2.45] phenthoate, [b-2.46] phorate, [b-2.47] phosalone, [b-2.48] phosmet, [b-2.49] phosphamidon, [b-2.50] phoxim, [b-2.51] pirimiphos-methyl, [b-2.52] profenofos, [b-2.53] propetamphos, [b-2.54] prothiofos, [b-2.55] pyraclofos, [b-2.56] pyridaphenthion, [b-2.57] quinalphos, [b-2.58] sulfotep, [b-2.59] tebupirimfos, [b-2.60] temephos, [b-2.61] terbufos, [b-2.62] thiometon, [b-2.63] triazophos, [b-2.64] trichlorfon, [b-2.65] vamidothion, [b-2.66] chlorothion, [b-2.67] bromfenvinfos, [b-2.68] bromophos, [b-2.69] bromophos-ethyl, [b-2.70] butathiofos, [b-2.71] carbophenothion, [b-2.72] chlorphoxim, [b-2.73] sulprofos, [b-2.74] diamidafos, [b-2.75] tetrachlorvinphos, [b-2.76] propaphos, [b-2.77] mesulfenfos, [b-2.78] dioxabenzofos, [b-2.79] etrimfos, [b-2.80] oxydeprofos, [b-2.81] formothion, [b-2.82] fensulfothion, [b-2.83] isazofos, [b-2.84] imicyafos, [b-2.85] isamidofos, [b-2.86] thionazin, and [b-2.87] fosthietan.

### <b-3: Cyclic diene organochlorine-based GABA-gated chloride ion channel blocker>

b-3: Examples of the cyclic diene organochlorine-based GABA-gated chloride ion channel blocker include [b-3.1] chlordane, [b-3.2] endosulfan, [b-3.3] lindane, and [b-3.4] dienochlor.

### <b-4: Phenylpyrazole-based GABA-gated chloride ion channel blocker>

b-4: Examples of the phenylpyrazole-based GABA-gated chloride ion channel blocker include [b-4.1] ethiprole, [b-4.2] fipronil, and [b-4.3] acetoprole.

### <b-5: Sodium channel modulator>

b-5: Examples of the sodium channel modulator include [b-5.1] acrinathrin, [b-5.2] allethrin, [b-5.3] bifenthrin, [b-5.4] bioallethrin, [b-5.5] bioallethrin S-cyclopentenyl isomer, [b-5.6] bioresmethrin, [b-5.7] cycloprothrin, [b-5.8] cyfluthrin, [b-5.9] beta-cyfluthrin, [b-5.10] cyhalothrin, [b-5.11] gamma-cyhalothrin, [b-5.12] lambda-cyhalothrin, [b-5.13] cypermethrin, [b-5.14] alpha-cypermethrin, [b-5.15] beta-cypermethrin, [b-5.16] theta-cypermethrin, [b-5.17] zeta-cypermethrin, [b-5.18] cyphenothrin [(1R)-trans-isomer], [b-5.19] deltamethrin, [b-5.20] empenthrin [(EZ)-(1R)-isomer], [b-5.21] esfenvalerate, [b-5.22] ethofenprox, [b-5.23] fenpropathrin, [b-5.24] fenvalerate, [b-5.25] flucythrinate, [b-5.26] flumethrin, [b-5.27] tau-fluvalinate, [b-5.28] halfenprox, [b-5.29] imiprothrin, [b-5.30] methothrin, [b-5.31] metofluthrin, [b-5.32] epsilon-metofluthrin, [b-5.33] momfluorothrin, [b-5.34] epsilon-momfluorothrin, [b-5.35] permethrin, [b-5.36] phenothrin [(1R)-transisomer], [b-5.37] prallethrin, [b-5.38] Pyrethrin, [b-5.39] resmethrin, [b-5.40] kadethrin, [b-5.41] silafluofen, [b-5.42] tefluthrin, [b-5.43] tetramethrin, [b-5.44] tetramethrin [(1R)-isomer], [b-5.45] tralomethrin, [b-5.46] transfluthrin, [b-5.47] ZXI8901 (3-(4-bromophenoxy)phenyl]-cyanomethyl 4-(difluoromethoxy)-a-(1-methylethyl)benzeneacetate), [b-5.48] biopermethrin, [b-5.49] furamethrin, [b-5.50] profluthrin, [b-5.51] flubrocythrinate, [b-5.52] dimefluthrin, [b-5.53] phenothrin, [b-5.54] fluvalinate, [b-5.55] DDT (dichlorodiphenyl-trichloroethane), and [b-5.56] methoxychlor.

### <b-6: Nicotinic acetylcholine receptor (nAChR) competitive modulator>

b-6: Examples of the nicotinic acetylcholine receptor (nAChR) competitive modulator include [b-6.1] acetamiprid, [b-6.2] clothianidin, [b-6.3] dinotefuran, [b-6.4] imidacloprid, [b-6.5] nitenpyram, [b-6.6] thiacloprid, [b-6.7] thiamethoxam, [b-6.8] nicotine, [b-6.9] nicotine sulfate, [b-6.10] sulfoxaflor, [b-6.11] flupyradifurone, [b-6.12] triflumezopyrim, and [b-6.13] flupyrimin.

### <b-7: Nicotinic acetylcholine receptor (nAChR) allosteric modulator site I>

b-7: Examples of the nicotinic acetylcholine receptor (nAChR) allosteric modulator site I include [b-7.1] spinosad and [b-7.2] spinetoram.

### <b-8: Glutamate-gated chloride ion channel (GluCl) allosteric modulator>

b-8: Examples of the glutamate-gated chloride ion channel (GluCl) allosteric modulator include [b-8.1] abamectin, [b-8.2] emamectin benzoate, [b-8.3] lepimectin, and [b-8.4] milbemectin.

### <b-9: Juvenile hormone mimic>

b-9: Examples of the juvenile hormone mimic include [b-9.1] hydroprene, [b-9.2] kinoprene, [b-9.3] methoprene, [b-9.4] fenoxycarb, and [b-9.5] pyriproxyfen.

### <b-10: Other nonspecific (multisite) inhibitors>

b-10: Examples of other nonspecific (multisite) inhibitors include [b-10.1] methyl bromide, [b-10.2] chloropicrin, [b-10.3] cryolite, [b-10.4] sulfuryl fluoride, [b-10.5] borax, [b-10.6] boric acid, [b-10.7] disodium octaborate, [b-10.8] sodium metaborate, [b-10.9] tartar emetic, [b-10.10] dazomet, [b-10.11] metam, and [b-10.12] metham sodium.

### <b-11: Chordotonal organ TRPV channel modulator>

b-11: Examples of the chordotonal organ TRPV channel modulator include [b-11.1] pymetrozine, [b-11.2] pyrifluquinazon, and [b-11.3] Afidopyropen.

### <b-12: Mite growth inhibitor acting on CHS1>

b-12: Examples of the mite growth inhibitor acting on CHS1 include [b-12.1] clofentezine, [b-12.2] diflovidazin, [b-12.3] hexythiazox, and [b-12.4] etoxazole.

### <b-13: Microbial disruptor of insect midgut membrane>

b-13: Examples of the microbial disruptor of insect midgut membranes include [b-13.1] *Bacillus thuringiensis*, [b-13.2] *B.t.* subsp. *Israelensis*, [b-13.3] *B.t.* subsp. *Aizawai*, [b-13.4] *B.t.* subsp. *kurstaki*, [b-13.5] *B.t.* subsp. *tenebrionis*, [b-13.6] B.t. crop proteins: Cry1Ab, Cry1Ac, Cry1Fa, Cry1A.105, Cry2Ab, Vip3A,mCry3A, Cry3Ab, Cry 3Bb, and Cry34Ab1/Cry35Ab1, and [b-13.7] *Bacillus sphaericus*.

### <b-14: Mitochondrial ATP synthase inhibitor>

b-14: Examples of the mitochondrial ATP synthase inhibitor include [b-14.1] diafenthiuron, [b-14.2] azocyclotin, [b-14.3] cyhexatin, [b-14.4] fenbutatin oxide, [b-14.5] propargite, and [b-14.6] tetradifon.

### <b-15: Uncoupler of oxidative phosphorylation via disruption of proton gradient>

b-15: Examples of the uncoupler of oxidative phosphorylation via disruption of the proton gradient include [b-15.1] (chlorfenapyl), [b-15.2] DNOC (dinitro-ortho-cresol), [b-15.3] binapacryl, and [b-15.4] sulfluramid.

### <b-16: Nicotinic acetylcholine receptor (nAChR) channel blocker>

b-16: Examples of the nicotinic acetylcholine receptor (nAChR) channel blocker include [b-16.1] bensultap, [b-16.2] cartap hydrochloride, [b-16.3] thiocyclam, [b-16.4] thiosultap, [b-16.5] Thiosultap-sodium, and [b-16.6] monosultap.

### <b-17: Chitin biosynthesis inhibitor acting on CHS1>

b-17: Examples of the chitin biosynthesis inhibitor acting on CHS1 include [b-17.1] bistrifluron, [b-17.2] chlorfluazuron, [b-17.3] diflubenzuron, [b-17.4] flucycloxuron, [b-17.5] flufenoxuron, [b-17.6] hexaflumuron, [b-17.7] lufenuron, [b-17.8] novaluron, [b-17.9] noviflumuron, [b-17.10] teflubenzuron, and [b-17.11] triflumuron.

### <b-18: Chitin biosynthesis inhibitor type 1>

b-18: Examples of the chitin biosynthesis inhibitor type 1 include [b-18.1] buprofezin.

### <b-19: Dipteran molting inhibitor>

b-19: Examples of the dipteran molting inhibitor include [b-19.1] cyromazine.

### <b-20: Molting hormone ecdysone receptor agonist>

b-20: Examples of the molting hormone ecdysone receptor agonist include [b-20.1] chromafenozide, [b-20.2] halofenozide, [b-20.3] methoxyfenozide, and [b-20.4] tebufenozide.

### <b-21: Octopamine receptor agonist>

b-21: Examples of the octopamine receptor agonist include [b-21.1] amitraz.

### <b-22: Mitochondrial electron transport complex III inhibitor>

b-22: Examples of the mitochondrial electron transport complex III inhibitor include [b-22.1] hydramethylnon, [b-22.2] acequinocyl, [b-22.3] fluacrypyrim, and [b-22.4] bifenazate.

### <b-23: Mitochondrial electron transport complex I inhibitor (METI)>

b-23: Examples of the mitochondrial electron transport complex I inhibitor (METI) include [b-23.1] fenazaquin, [b-23.2] fenpyroximate, [b-23.3] pyridaben, [b-23.4] pylimidifen, [b-23.5] tebufenpyrad, [b-23.6] tolfenpyrad, and [b-23.7] rotenone.

### <b-24: Potential-dependent sodium channel blocker>

b-24: Examples of the potential-dependent sodium channel blocker include [b-24.1] indoxacarb and [b-24.2] metaflumizone.

### <b-25: Acetyl CoA carboxylase inhibitor>

b-25: Examples of the acetyl CoA carboxylase inhibitor include [b-25.1] spirodiclofen, [b-25.2] spiromesifen, [b-25.3] spiropidion, and [b-25.4] spirotetramat.

### <b-26: Mitochondrial electron transport complex IV inhibitor>

b-26: Examples of the mitochondrial electron transport complex IV inhibitor include [b-26.1] aluminum phosphide, [b-26.2] calcium phosphide, [b-26.3] phosphine, [b-26.4] zinc phosphide, [b-26.5] calcium cyanide, [b-26.6] potassium cyanide, and [b-26.7] sodium cyanide.

### <b-27: Mitochondrial electron transport complex II inhibitor>

b-27: Examples of the mitochondrial electron transport complex II inhibitor include [b-27.1] cyenopyrafen, [b-27.2] cyflumetofen, and [b-27.3] pyflubumide.

### <b-28: Ryanodine receptor modulator>

b-28: Examples of the ryanodine receptor modulator include [b-28.1] chlorantraniliprole, [b-28.2] cyantraniliprole, [b-28.3] flubendiamide, [b-28.4] cyclaniliprole, and [b-28.5] tetraniliprole.

### <b-29: Chordotonal organ modulator with its target side unidentified>

b-29: Examples of the chordotonal organ modulator insecticide with its target site unidentified include [b-29.1] flonicamid.

### <b-30: GABA-gated chloride ion channel allosteric modulator>

b-30: Examples of the GABA-gated chloride ion channel allosteric modulator include [b-30.1] broflanilide and [b-30.2] fluxametamide.

### <b-31: Baculovirus>

b-31: Examples of the baculovirus include [b-31.1] *Cydia pomonella* GV, [b-31.2] *Thaumatotibia leucotreta* GV, [b-31.3] *Anticarsia gemmatalis* MNPV, and [b-31.4] *Helicoverpa armigera* NPV.

### <b-32: Nicotinic acetylcholine receptor (nAChR) allosteric modulator site II>

b-32: Examples of the nicotinic acetylcholine receptor (nAChR) allosteric modulator site II include [b-32.1] GS-omega/kappa HXTX-Hv1a peptide.

### <b-33: Other insecticides>

b-33: Examples of other insecticides include [b-33.1] azadirachtin, [b-33.2] benzoximate, [b-33.3] phenisobromolate, [b-33.4] chinomethionat, [b-33.5] dicofol, [b-33.6] CaSx, [b-33.7] manzeb, [b-33.8] pyridalyl, [b-33.9] sulfur, [b-33.10] bromopropylate, [b-33.11] *Burkholderia* spp, *Wolbachia pipientis* (Zap), [b-33.12] *Chenopodium ambrosioides* near *ambrosioides* extract, [b-33.13] Fatty acid monoesters with glycerol or propanediol, [b-33.14] Neem oil, [b-33.15] *Beauveria bassiana* strains, [b-33.16] Metarhizium anisopliae strain F52, [b-33.17] *Paecilomyces fumosoroseus* Apopka strain 97, [b-33.18] diatomite, [b-33.19] triazamate, [b-33.20] dicyclanil, [b-33.21] dinobuton, [b-33.22] dinocap, [b-33.23] hydrogen cyanide, [b-33.24] methyl iodide, [b-33.25] karanjin, [b-33.26] mercury chloride, [b-33.27] methyl isothiocyanate, [b-33.28] pentachlorophenol, [b-33.29] phosphine, [b-33.30] piperonyl butoxide, [b-33.31] polynactins, [b-33.32] sabadilla, [b-33.33] sulcofuron-sodium, [b-33.34] tribufos, [b-33.35] aldrin, [b-33.36] amidithion, [b-33.37] amidothioate, [b-33.38] aminocarb, [b-33.39] amiton, [b-33.40] aramite, [b-33.41] athidathion, [b-33.42] azothoate, [b-33.43] barium polysulphide, [b-33.44] benclothiaz, [b-33.45] 5-(1,3-benzodioxole-5-yl)-3-hexylcyclohexa-2-enone, [b-33.46] 1,1-bis(4-chlorophenyl)-2-ethoxyethanol, [b-33.47] butonate, [b-33.48] butopyronoxyl, [b-33.49] 2-(2-butoxyethoxy)ethyl thiocyanate, [b-33.50] camphechlor, [b-33.51] chlorbenside, [b-33.52] chlordecone, [b-33.53] chlordimeform, [b-33.54] chlorfenethol, [b-33.55] chlorfenson, [b-33.56] fluazuron, [b-33.57] metaldehyde, [b-33.58] bialaphos, [b-33.59] levamisol, [b-33.60] amidoflumet, [b-33.61] pyrafluprole, [b-33.62] pyriprole, [b-33.63] tralopyril, [b-33.64] flupyrazofos, [b-33.65] diofenolan, [b-33.66] chlorobenzilate, [b-33.67] flufenzine, [b-33.68] benzomate, [b-33.69] flufenerim, [b-33.70] albendazole, [b-33.71] oxibendazole, [b-33.72] fenbendazole, [b-33.73] metam-sodium, [b-33.74] 1,3-dichloropropene, [b-33.75] ethylene dibromide, [b-33.76] acrylonitrile, [b-33.77] bis(2-chloroethyl)ether, [b-33.78] 1-bromo-2-chloroethane, [b-33.79] 3-bromo-1-chloroprop-1-ene, [b-33.80] bromocyclen, [b-33.81] carbon disulfide, [b-33.82] tetrachloromethane, [b-33.83] nemadectin, [b-33.84] cymiazole, [b-33.85] cytokinin, [b-33.86] 2-(octylthio)ethanol, [b-33.87] potassium oleate, [b-33.88] sodium oleate, [b-33.89] machine oil, [b-33.90] tar oil, [b-33.91] anabasine, [b-33.92] morantel tartrate, [b-33.93] Pyrethrum, [b-33.94] rape seed oil, [b-33.95] soybean lecithin, [b-33.96] starch, [b-33.97] hydroxypropylstarch, [b-33.98] decanoyloctanoylglycerol, [b-33.99] diatomite, [b-33.100] TPIC (tripropyl isocyanurate), [b-33.101] D-D (1,3-Dichloropropene), [b-33.102] peroxocarbonate, [b-33.103] MB-599 (verbutin), [b-33.104] bis(2,3,3,3-tetrachloropropyl) ether, [b-33.105] DCIP (bis(2-chloro-1-methylethyl)ether), [b-33.106] ENT-8184 (N-(2-Ethylhexyl)bicyclohept-5-ene-2,3-dicarboximide), [b-33.107] Bayer 22408 (O,O-diethyl O-naphthalimido phosphorothioate), [b-33.108] Bayer 32394 (tris(1-dodecyl-3-methyl-2-phenylbenzimidazolium)hexacyanoferrate), [b-33.109] flometoquin, [b-33.110] dicloromezotiaz, [b-33.111] fluazaindolizine, [b-33.112] cyhalodiamide, [b-33.113] tioxazafen, [b-33.114] fluhexafon, [b-33.115] fluralaner, [b-33.116] tetrachlorantraniliprole, [b-33.117] sarolaner, [b-33.118] lotilaner, [b-33.119] tigolaner, [b-33.120] cycloxaprid, [b-33.121] fluensulfone, [b-33.122] benzpyrimoxan, [b-33.123] acynonapyr, [b-33.124] fenmezoditiaz, [b-33.125] tyclopyrazoflor, [b-33.126] oxazosulfyl, [b-33.127] isocycloseram, [b-33.128] dimpropyridaz, [b-33.129] cyproflanilide, [b-33.130] nicofluprole, [b-33.131] cyclobutrifluram, [b-33.132] cycloxylidin, [b-33.133] paichongding, [b-33.134] guadipyr, [b-33.135] cyetpyrafen, [b-33.136] flupentiofenox, [b-33.137] pyriminostrobin, [b-33.138] chloroprallethrin, [b-33.139] kappa-bifenthrin, [b-33.140] kappa-tefluthrin, [b-33.141] heptafluthrin,
[b-33.142] a compound represented by the formula (s1): 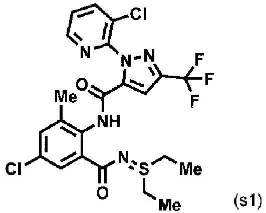 (see International Publication No. WO 2020/018362),
[b-33.143] a compound represented by the formula (s2): 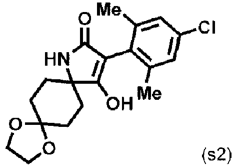 (see International Publication No. WO 2006/089633),
[b-33.144] a compound represented by the formula (s3): 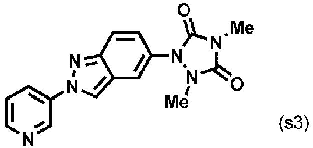 (see International Publication No. WO 2018/011111),
[b-33.145] a compound represented by the formula (s4): 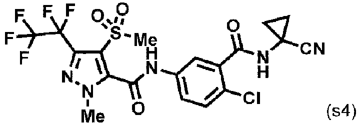 (see International Publication No. WO 2012/126766),
[b-33.146] a compound represented by the formula (s5): 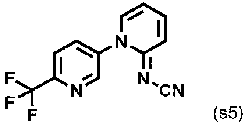 (see International Publication No. WO 2019/215076),
[b-33.147] a compound represented by the formula (s6): 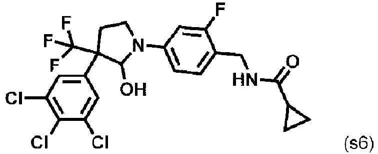 (see International Publication No. WO 2016/180802),
[b-33.148] a compound represented by the formula (s7): 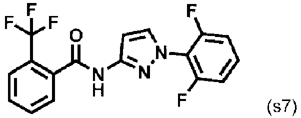 (see International Publication No. WO 2014/053450),
[b-33.149] a compound represented by the formula (s8): 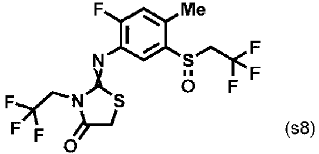 (see International Publication No. WO 2013/092350),
[b-33.150] a compound represented by the formula (s9): 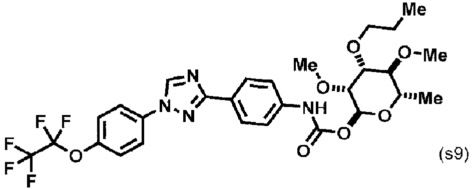 (see International Publication No. WO 2012/027521),
[b-33.151] a compound represented by the formula (s10): 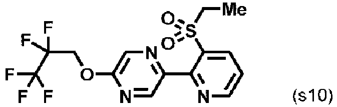 (see International Publication No. WO 2017/065228),
[b-33.152] a compound represented by the formula (s11): 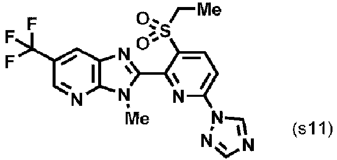 (see International Publication No. WO 2015/133603),
[b-33.153] a compound represented by the formula (s12): 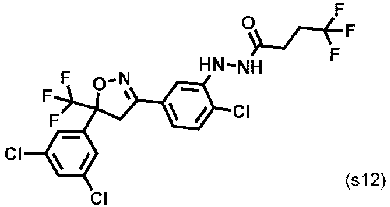 (see International Publication No. WO 2010/090344),
[b-33.154] a compound represented by the formula (s13): 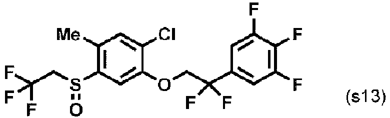 (see International Publication No. WO 2015/025826), and
[b-33.155] a compound represented by the formula (s14): 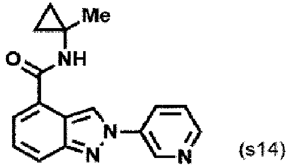 (see International Publication No. WO 2016/144351).

Specific components contained in the germicide that can be used as a mixture with the compound (1) are as shown in the group c given below and include salts, isomers, and N-oxide forms thereof. However, the germicide known in the art is not limited thereto.

Group c:

### <c-1: Phenylamide-based germicide>

c-1: Examples of the phenylamide-based germicide include [c-1.1] benalaxyl, [c-1.2] benalaxyl-M or kiralaxyl, [c-1.3] furalaxyl, [c-1.4] metalaxyl, [c-1.5] metalaxyl-M or mefenoxam, [c-1.6] oxadixyl, and [c-1.7] ofurace.

### <c-2: Hydroxy(2-amino-)pyrimidine-based germicide>

c-2: Examples of the hydroxy(2-amino-)pyrimidine-based germicide include [c-2.1] bupirimate, [c-2.2] dimethirimol, and [c-2.3] ethirimol.

### <c-3: Aromatic heterocyclic germicide>

c-3: Examples of the aromatic heterocyclic germicide include [c-3.1] hymexazole and [c-3.2] octhilinone.

### <c-4: Carboxylic acid-based germicide>

c-4: Examples of the carboxylic acid-based germicide include [c-4.1] oxolinic acid.

### <c-5: DHODHI agent (dihydroorotate dehydrogenase inhibitor)>

c-5: Examples of the DHODHI agent include [c-5.1] ipflufenoquin and [c-5.2]quinofumelin.

### <c-6: β-Tubulin polymerization inhibitor>

c-6: Examples of the β-tubulin polymerization inhibitor include [c-6.1] benomyl, [c-6.2] carbendazim, [c-6.3] fuberidazole, [c-6.4] thiabendazole, [c-6.5] thiophanate, [c-6.6] thiophanate-methyl, [c-6.7] diethofencarb, [c-6.8] zoxamide, and [c-6.9] ethaboxam.

### <c-7: Cell division inhibitor>

c-7: Examples of the cell division inhibitor include [c-7.1] pencycuron.

### <c-8: Delocalization of spectrin-like protein>

Examples of the germicide involved in delocalization of spectrin-like protein include [c-8.1] fluopicolide and [c-8.2] fluopimomide.

### <c-9: Actin/myosin/fimbrin function>

c-9: Examples of the germicide involved in actin/myosin/fimbrin functions include [c-9.1] Phenamacril, [c-9.2] metrafenone, and [c-9.3] pyriofenone.

### <c-10: tubulin dynamics modulator>

c-10: Examples of the germicide involved in tubulin dynamics modulator include [c-10.1] pyridachlometyl.

### <c-11: Complex I (NADH oxidoreductase) inhibitor>

c-11: Examples of the complex I (NADH oxidoreductase) inhibitor include [c-11.1] diflumetorim, [c-11.2] tolfenpyrad, and [c-11.3] fenazaquin.

### <c-12: SDHI (succinate dehydrogenase inhibitor)>

c-12: Examples of the SDHI (succinate dehydrogenase inhibitor) include [c-12.1] benodanil, [c-12.2] benzovindiflupyr, [c-12.3] bixafen, [c-12.4] boscalid, [c-12.5] carboxin, [c-12.6] fenfuram, [c-12.7] fluopyram, [c-12.8] flutolanil, [c-12.9] fluxapyroxad, [c-12.10] furametpyr, [c-12.11] isofetamid, [c-12.12] isopyrazam, [c-12.13] mepronil, [c-12.14] oxycarboxin, [c-12.15] penthiopyrad, [c-12.16] penflufen, [c-12.17] pydiflumetofen, [c-12.18] sedaxane, [c-12.19] thifluzamide, [c-12.20] pyraziflumid, [c-12.21] isoflucypram, [c-12.22] fluindapyr, [c-12.23] inpyrfluxam, and [c-12.24] cyclobutrifluram.

### <c-13: QoI (quinone outside inhibitor)>

c-13: Examples of the QoI (quinone outside inhibitor) include [c-13.1] azoxystrobin, [c-13.2] coumoxystrobin, [c-13.3] dimoxystrobin, [c-13.4] enoxastrobin, [c-13.5] famoxadone, [c-13.6] fenamidone, [c-13.7] fenaminstrobin, [c-13.8] flufenoxystrobin, [c-13.9] fluoxastrobin, [c-13.10] kresoxim-methyl, [c-13.11] mandestrobin, [c-13.12] metominostrobin, [c-13.13] orysastrobin, [c-13.14] picoxystrobin, [c-13.15] pyraclostrobin, [c-13.16] pyrametostrobin, [c-13.17] pyraoxystrobin, [c-13.18] pyribencarb, [c-13.19] triclopyricarb, [c-13.20] trifloxystrobin, and [c-13.21] metyltetraprole.

### <c-14: QiI (quinone inside inhibitor)>

c-14: Examples of the QiI (quinone inside inhibitor) include [c-14.1] amisulbrom, [c-14.2] cyazofamid, [c-14.3] fenpicoxamid, and [c-14.4] florylpicoxamid.

### <c-15: Oxidative phosphorylation uncoupling inhibitor>

c-15: Examples of the oxidative phosphorylation uncoupling inhibitor include [c-15.1] binapacryl, [c-15.2] meptyldinocap, [c-15.3] dinocap, and [c-15.4] fluazinam.

### <c-16: ATP synthase inhibitor>

c-16: Examples of the ATP synthase inhibitor include [c-16.1] fentin acetate, [c-16.2] fentin chloride, and [c-16.3] fentin hydroxide.

### <c-17:ATP transport>

c-17: Examples of the germicide involved in ATP transport include [c-17.1] silthiofam.

### <c-18: QoSI (quinone outside stigmatellin binding subsite inhibitor)>

c-18: Examples of the QoSI (quinone outside stigmatellin binding subsite inhibitor) include [c-18.1] ametoctradin.

### <c-19: Methionine biosynthesis inhibitor>

c-19: Examples of the methionine biosynthesis inhibitor include [c-19.1] cyprodinil, [c-19.2] mepanipyrim, and [c-19.3] pyrimethanil.

### <c-20: Protein biosynthesis inhibitor>

c-20: Examples of the protein biosynthesis inhibitor include [c-20.1] blasticidin-S, [c-20.2] kasugamycin, [c-20.3] streptomycin, and [c-20.4] oxytetracycline.

### <c-21: Leucyl t-RNA synthetase inhibitor>

c-21: Examples of the Leucyl t-RNA synthetase inhibitor include [c-21.4] tavaborole.

### <c-22: Signal transduction inhibitor>

c-22: Examples of the signal transduction inhibitor include [c-22.1] quinoxyfen, [c-22.2] proquinazid, [c-22.3] fenpiclonil, [c-22.4] fludioxonil, [c-22.5] chlozolinate, [c-22.6] dimethachlone, [c-22.7] iprodione, [c-22.8] procymidone, and [c-22.9] vinclozolin.

### <c-23: Phospholipid biosynthesis, methyltransferase>

c-23: Examples of the germicide involved in phospholipid biosynthesis, methyltransferase include [c-23.1] edifenphos, [c-23.2] iprobenfos, [c-23.3] isoprothiolane, and [c-23.4] pyrazophos.

### <c-24: Peroxidation of cellular lipids>

c-24: Examples of the germicide involved in peroxidation of cellular lipids include [c-24.5] biphenyl, [c-24.6] chloroneb, [c-24.7] dicloran, [c-24.8] quintozene, [c-24.9] tecnazene, [c-24.10] tolclofos-methyl, and [c-24.11] echlomezol or etridiazole.

### <c-25: cell membrane permeability, fatty acids>

c-25: Examples of the germicide involved in cell membrane permeability, fatty acids include [c-25.12] iodocarb, [c-25.13] propamocarb, and [c-25.14] prothiocarb.

### <c-26: Ergosterol binding agent>

c-26: Examples of the germicide involved in ergosterol binding include [c-26.1] natamycin.

### <c-27: Oxysterol binding protein inhibitor>

c-27: Examples of the oxysterol binding protein inhibitor include [c-27.1] oxathiapiprolin and [c-27.2] fluoxapiprolin.

### <c-28: DMI (demethylation inhibitor)>

c-28: Examples of the DMI (demethylation inhibitor) include [c-28.1] azaconazole, [c-28.2] bitertanol, [c-28.3] bromuconazole, [c-28.4] cyproconazole, [c-28.5] difenoconazole, [c-28.6] diniconazole, [c-28.7] diniconazole-M, [c-28.8] epoxiconazole, [c-28.9] etaconazole, [c-28.10] fenarimol, [c-28.11] fenbuconazole, [c-28.12] fluquinconazole, [c-28.13] quinconazole, [c-28.14] flusilazole, [c-28.15] flutriafol, [c-28.16] hexaconazole, [c-28.17] imazalil, [c-28.18] imibenconazole, [c-28.19] ipconazole, [c-28.20] metconazole, [c-28.21] myclobutanil, [c-28.22] nuarimol, [c-28.23] oxpoconazole, [c-28.24] oxpoconazole fumarate, [c-28.25] pefurazoate, [c-28.26] penconazole, [c-28.27] prochloraz, [c-28.28] propiconazole, [c-28.29] prothioconazole, [c-28.30] pyrifenox, [c-28.31] pyrisoxazole, [c-28.32] simeconazole, [c-28.33] tebuconazole, [c-28.34] tetraconazole, [c-28.35] triadimefon, [c-28.36] triadimenol, [c-28.37] triflumizole, [c-28.38] triforine, [c-28.39] triticonazole, [c-28.40] mefentrifluconazole, and [c-28.41] ipfentrifluconazole.

### <c-29: Amine-based germicide>

c-29: Examples of the amine-based germicide include [c-29.1] aldimorph, [c-29.2] dodemorph, [c-29.3] fenpropimorph, [c-29.4] tridemorph, [c-29.5] fenpropidin, [c-29.6] piperalin, and [c-29.7] spiroxamine.

### <c-30: 3-Keto reductase inhibitor in C4 demethylation of sterol biosynthesis>

c-30: Examples of the 3-keto reductase inhibitor in C4 demethylation of sterol biosynthesis include [c-30.1] fenhexamid and [c-30.2] fenpyrazamine.

### <c-31: Squalene epoxidase inhibitor of sterol biosynthesis>

c-31: Examples of the squalene epoxidase inhibitor of sterol biosynthesis include [c-31.1] pyributicarb, [c-31.2] naftifine, and [c-31.3] terbinafine.

### <c-32: Cell wall biosynthesis inhibitor>

c-32: Examples of the cell wall biosynthesis inhibitor include [c-32.1] polyoxins, [c-32.2] dimethomorph, [c-32.3] flumorph, [c-32.4] pyrimorph, [c-32.5] benthiavalicarb, [c-32.6] benthivalicarb-isopropyl, [c-32.7] iprovalicarb, [c-32.8] mandipropamid, and [c-32.9] valifenalate.

### <c-33: Melanin biosynthesis inhibitor>

c-33: Examples of the melanin biosynthesis inhibitor include [c-33.1] phthalide or fthalide, [c-33.2] pyroquilone, [c-33.3] tricyclazole, [c-33.4] carpropamid, [c-33.5] diclocymet, [c-33.6] fenoxanil, and [c-33.7] tolprocarb.

### <c-34: Host plant resistance inducer>

c-34: Examples of the host plant resistance inducer include [c-34.1] acibenzolar-S-methyl, [c-34.2] probenazole, [c-34.3] tiadinil, [c-34.4] isotianil, [c-34.5] laminarin, [c-34.6] extract from *Reynoutria sachalinensis* (giant knotweed), [c-34.7] *Bacillus mycoides* isolate J, [c-34.8] cell walls of *Saccharomyces cerevisiae* strain LAS117, [c-34.9] fosetyl, [c-34.10] phosphorous acid, [c-34.11] sodium phosphite, [c-34.12] ammonium phosphite, [c-34.13] potassium phosphite, and [c-34.14] dichlobentiazox.

### <c-35: Dithiocarbamate-based germicide>

c-35: Examples of dithiocarbamate-based germicide include [c-35.1] mancozeb, [c-35.2] manzeb, [c-35.3] maneb, [c-35.4] metiram, [c-35.5] propineb, [c-35.6] thiram, [c-35.7] zinc thiazole, [c-35.8] zineb, [c-35.9] ziram, and [c-35.10] ferbam.

### <c-36: Phthalimide-based germicide>

c-36: Examples of the phthalimide-based germicide include [c-36.1] captan, [c-36.2] captafol, [c-36.3] folpet, and [c-36.4] fluorofolpet.

### <c-37: Guanidine-based germicide>

c-37: Examples of the guanidine-based germicide include [c-37.1] guazatine, [c-37.2] iminoctadine, [c-37.3] iminoctadine albesilate, and [c-37.4] iminoctadine triacetate.

### <c-38: Germicide with multi-site contact activity>

c-38: Examples of the germicide with multi-site contact activity include [c-38.1] chlorothalonil, [c-38.2] dichlofluanid, [c-38.3] tolylfluanid, [c-38.4] copper oxychloride, [c-38.5] copper II hydroxide, [c-38.6] copper hydroxide sulfate, [c-38.7] organocopper compound, [c-38.8] dodecylbenzenesulphonic acid bisethylenediamine copper [II] salt, [c-38.9] sulphur, [c-38.10] fluoroimide, [c-38.11] anilazine, [c-38.12] dithianon, [c-38.13] chinomethionat or quinomethionate, [c-38.14] methasulfocarb, and [c-38.15] amobam.

### <c-39: Biological agricultural chemical having plurality of mechanisms of action>

c-39: Examples of the biological agricultural chemical having a plurality of mechanisms of action include [c-39.1] Polypeptide(lectin), [c-39.2] BLAD, [c-39.3] Phenol, Sesquiterpenes, Triterpenoids, Coumarins, [c-39.4] extract from Swinglea glutinosa, [c-39.5] Terpene Hydrocarbons,Terpene alcohols and Terpene phenols, [c-39.6] extract from Melaleuca alternifolia, [c-39.7] plant oils (mixtures): eugenol, geraniol, thymol, [c-39.8] Fungal Trichoderma spp., [c-39.9] Trichoderma atroviride strain I-1237, [c-39.10]Trichoderma atroviride strain LU132, [c-39.11] Trichoderma atroviride strain SC1, [c-39.12] Trichoderma atroviride strain SKT-1, [c-39.13] Trichoderma atroviride strain 77B, [c-39.14] Trichoderma asperellum strain T34, [c-39.15] Trichoderma asperellum strain Kd, [c-39.16] Trichoderma harzianum strain T-22, [c-39.17] Trichoderma virens strain G-41, [c-39.18] Fungal Clonostachys spp., [c-39.19] Clonostachys rosea strain J1446, [c-39.20] Clonostachys rosea strain CR-7, [c-39.21] Fungal Coniothyrium spp., [c-39.22] Coniothyrium minitans strain CON/M/91-08, [c-39.23] Fungal Hanseniaspora spp., [c-39.24] Hanseniaspora uvarum strain BC18Y, [c-39.25] Fungal Talaromyces spp., [c-39.26] Talaromyces flavus strain SAY-Y-94-01, [c-39.27] Fungal Saccharomyces spp., [c-39.28] Saccharomyces cerevisiae strain LAS02, [c-39.29] Saccharomyces cerevisiae strain DDSF623, [c-39.30] Bacterial Bacillus spp., [c-39.31] Bacillus amyloliquefaciens strain QST713, [c-39.32] Bacillus amyloliquefaciens strain FZB24, [c-39.33]Bacillus amyloliquefaciens strain MBI600, [c-39.34] Bacillus amyloliquefaciens strain D747, [c-39.35] Bacillus amyloliquefaciens strain F727, [c-39.36] Bacillus amyloliquefaciens strain AT-332, [c-39.37] Bacillus subtilis strain AFS032321, [c-39.38] Bacillus subtilis strain Y1336, [c-39.39] Bacillus subtilis strain HAI-0404, [c-39.40] Bacillus subtilis strain RTI477, [c-39.41] Bacillus velezensis strain RTI301, [c-39.42] Bacterial Erwinia spp.(peptide), [c-39.43] PHC25279, [c-39.44] Bacterial Gluconobacter spp., [c-39.45] Gluconobacter cerinus strain BC18B, [c-39.46] Bacterial Pseudomonas spp., [c-39.47] Pseudomonas chlororaphis strain AFS009, [c-39.48] Bacterial Streptomyces spp., [c-39.49] Streptomyces griseovirides strain K61, and [c-39.50] Streptomyces lydicus strain WYEC108.

### <c-40: Other germicides>

c-40: Examples of other germicides include [c-40.1] tecloftalam, [c-40.2] triazoxide, [c-40.3] flusulfamide, [c-40.4] diclomezine, [c-40.5] cyflufenamid, [c-40.6] dodine, [c-40.7] flutianil, [c-40.8] tebufloquin, [c-40.9] validamycins, [c-40.10] cymoxanil, [c-40.11] picarbutrazox, [c-40.12] aminopyrifen, [c-40.13] pyrapropoyne, [c-40.14] dipymetitrone, [c-40.15] D-tagatose, [c-40.16] mineral oils, organic oils, inorganic salts, material of biological origin, [c-40.17] Chloroinconazide, [c-40.18] Flubeneteram, [c-40.19] ferimzone, [c-40.20] seboctylamine, [c-40.21] metarylpicoxamid, [c-40.22] fluoxytioconazole, [c-40.23] flumetylsulforim, [c-40.24] flufenoxadiazam, [c-40.25] fenopyramid, [c-40.26] feneptamidoquin, and [c-40.27] bifemetstrobin.
[c-40.28] a compound represented by the formula (s16): 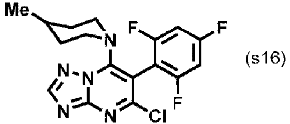 (see International Publication No. WO 98/046607),
[c-40.29] a compound represented by the formula (s17): 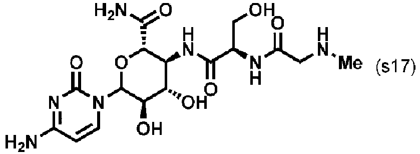 (gougerotin),
[c-40.30] a compound represented by the formula (s18): 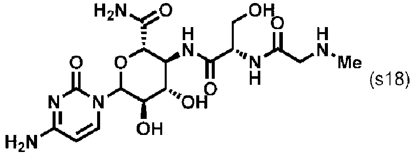 (ningnanmycin),
[c-40.31] a compound represented by the formula (s19): 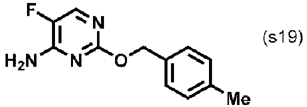 (see International Publication No. WO 2011/085084),
[c-40.32] a compound represented by the formula (s20): 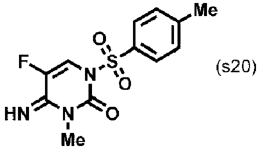 (see International Publication No. WO 2011/137002),
[c-40.33] a compound represented by the formula (s21): 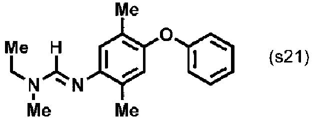 (see International Publication No. WO 2008/110313),
[c-40.34] a compound represented by the formula (s22): 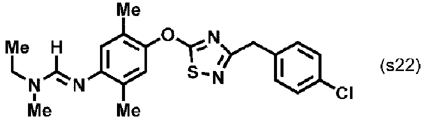 (see International Publication No. WO 2009/156098),
[c-40.35] a compound represented by the formula (s23): 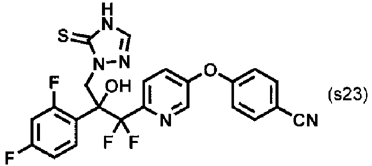 (see International Publication No. WO 2016/187201),
[c-40.36] a compound represented by the formula (s24): 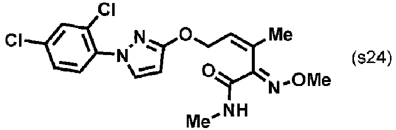 (see International Publication No. WO 2013/092224),
[c-40.37] a compound represented by the formula (s25): 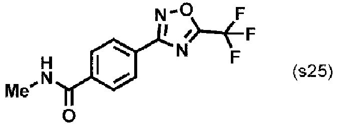 (see International Publication No. WO 2015/185485),
[c-40.38] a compound represented by the formula (s26): 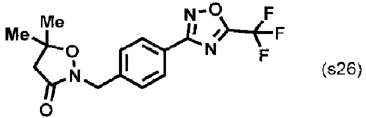 (see International Publication No. WO 2017/093348),
[c-40.39] a compound represented by the formula (s27): 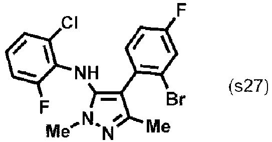 (see International Publication No. WO 2012/031061),
[c-40.40] a compound represented by the formula (s28): 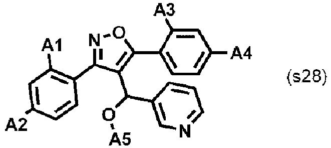 wherein A1, A2, A3, and A4 each independently represent a hydrogen atom or a halogen atom, and A5 represents a hydrogen atom, an acetyl group, or a benzoyl group (see International Publication Nos. WO 2006/031631 and WO 2010/069882),
[c-40.41] a compound represented by the formula (s29): 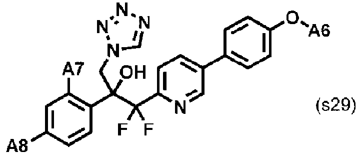 wherein A6 represents a C1 to C6 haloalkyl group, and A7 and A8 each independently represent a hydrogen atom or a halogen atom (see International Publication No. WO 2014/043376),
[c-40.42] a compound represented by the formula (s30): 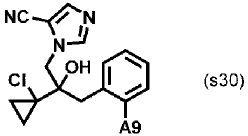 wherein A9 represents a halogen atom (see International Publication No. WO 2016/156290),
[c-40.43] a compound represented by the formula (s31): 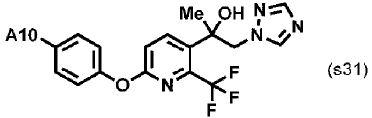 wherein A10 represents a halogen atom (see International Publication No. WO 2017/029179),
[c-40.44] a compound represented by the formula (s32): 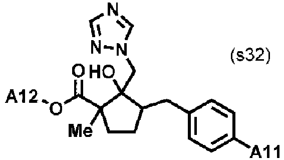 wherein A11 represents a hydrogen atom or a halogen atom, and A12 represents a C1 to C6 alkyl group (see International Publication No. WO 2011/070771),
[c-40.45] a compound represented by the formula (s33): 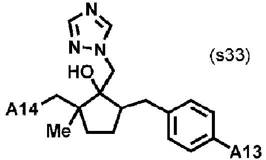 wherein A13 and A14 each represent a halogen atom (see International Publication No. WO 2011/070771),
[c-40.46] a compound represented by the formula (s34): 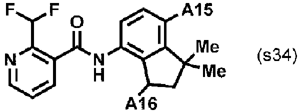 wherein A15 represents a hydrogen atom, or a halogen atom, and A16 represents a methyl group, an ethyl group, or a propyl group (see International Publication No. WO 2014/095675),
[c-40.47] a compound represented by the formula (s35): 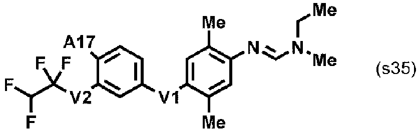 wherein A17 represents a hydrogen atom or a halogen atom, and V1 and V2 each independently represent an oxygen atom or a sulfur atom (see International Publication No. WO 2012/025450),
[c-40.48] a compound represented by the formula (s36): 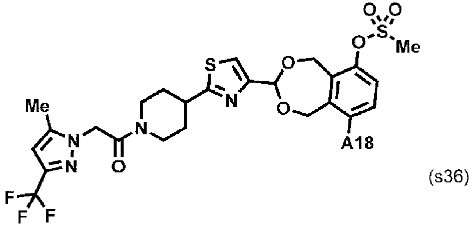 wherein A18 represents a hydrogen atom or a fluorine atom (see International Publication No. WO 2016/024350),
[c-40.49] a compound represented by the formula (s37): 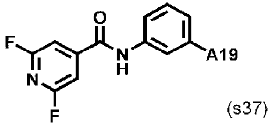 wherein A19 represents a C1 to C6 alkoxy group (see International Publication No. WO 2018/169038),
[c-40.50] a compound represented by the formula (s38): 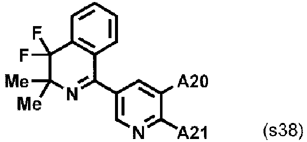 wherein A20 represents a methyl group or a methoxy group, and A21 represents a methyl group or a difluoromethyl group (see International Publication No. WO 2017/016915),
[c-40.51] a compound represented by the formula (s39): 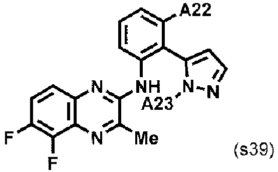 wherein A22 represents a hydrogen atom or a halogen atom, and A23 represents an ethyl group or a propyl group (see International Publication No. WO 2018/197692),
[c-40.52] a compound represented by the formula (s40): 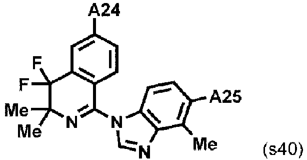 wherein A24 represents a hydrogen atom or a halogen atom, and A25 represents a methyl group, a fluorine atom, or a chlorine atom (see International Publication No. WO 2016/156085),
[c-40.53] a compound represented by the formula (s41): 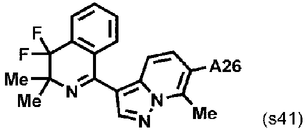 wherein A26 represents a methyl group, a fluorine atom, or a chlorine atom (see International Publication No. WO 2017/025510),
[c-40.54] a compound represented by the formula (s42): 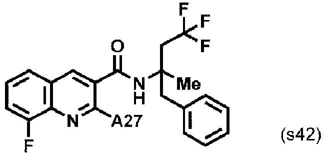 wherein A27 represents a hydrogen atom or methyl group (see International Publication No. WO 2017/153380),
[c-40.55] a compound represented by the formula (s43): 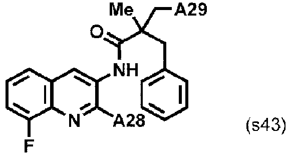 wherein A28 represents a hydrogen atom or a methyl group, and A29 represents an isopropyl group or a trifluoromethyl group (see International Publication No. WO 2018/172133), and
[c-40.56] a compound represented by the formula (s44): 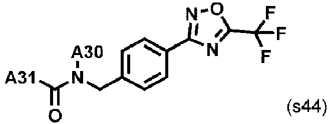 wherein A30 represents a hydrogen atom or a methoxy group, and A31 represents an ethyl group or a cyclopropyl group (see International Publication No. WO 2017/055469).

### [Examples]

Hereinafter, the present embodiment will be described in more detail with reference to Examples. The present embodiment is not limited by these Examples.

### [Synthesis of compound]

### <Reference Example 1>

### Synthesis of 8-chloro-2,2-dimethyl-2,3-dihydro-4H-benzo[e][1,3]oxazin-4-one

A mixed solution containing 5.4 g of 3-chloro-2-hydroxybenzamide and 3.78 g of p-toluenesulfonic acid monohydrate in 27 ml of 2,2-dimethoxypropane and 27 ml of toluene was stirred at 110°C for 3 hours. The reaction mixture was allowed to cool to room temperature and then phase-separated by the addition of a saturated aqueous solution of sodium bicarbonate and ethyl acetate. The obtained organic layer was dried over sodium sulfate, and the solvent was distilled off under reduced pressure. The obtained residue was washed with diisopropyl ether so that the title compound was obtained as 814 mg of a pale yellow solid.

¹H-NMR (CDCl₃) δ: 7.84 (1H, dd, J = 8.0, 1.7 Hz), 7.52 (1H, dd, J = 8.0, 1.7 Hz), 7.02 (1H, t, J = 7.6 Hz), 6.83 (1H, brs), 1.70 (6H, s).

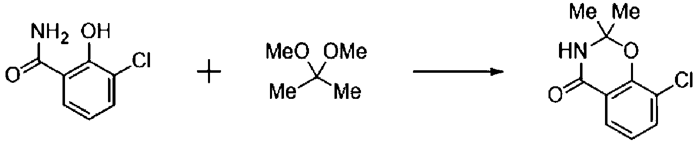

### <Synthesis Example 1 - Step 1>

### Synthesis of 4,8-dichloro2,2-dimethyl-2H-benzo[e][1,3]oxazine

65 µl of oxalyl chloride was added to 1 ml of a dichloromethane solution containing 90 µl of dimethylformamide, and the mixture was stirred at room temperature for 10 minutes. Subsequently, 100 mg of 8-dichloro-2,2-dimethyl-2,3-dihydro-4H-benzo-4H-benzo[e][1,3]oxazin-4-one obtained by the method of Reference Example 1 was added thereto, and the mixture was further stirred at room temperature for 1 hour. The solvent was distilled off from the obtained reaction mixture to obtain 120 mg of a yellow oil containing the title compound. The obtained product was used in the next reaction without being further purified.

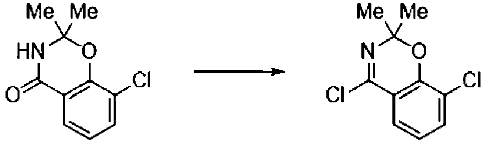

### <Synthesis Example 1 - Step 2>

### Synthesis of 8-dichloro-4-(4,5-dimethyl-1H-benzo[d]imidazole-1-yl)-2,2-dimethyl-2H-benzo[e][1,3]oxazine (compound No. 23)

1 ml of a pyridine solution containing 120 mg of 4,8-dichloro2,2-dimethyl-2H-benzo[e][1,3]oxazine obtained by the method of step 1 of Synthesis Example 1 and 61 mg of 4,5-dimethyl-1H-benzo[d]imidazole was stirred at 100°C for 3 hours. The reaction mixture was allowed to cool to room temperature and then phase-separated with a saturated aqueous solution of sodium bicarbonate and ethyl acetate. The obtained organic layer was dried over sodium sulfate, and the solvent was distilled off under reduced pressure. The obtained residue was purified by silica gel column chromatography. The title compound was obtained as 20.8 mg of a yellow solid.

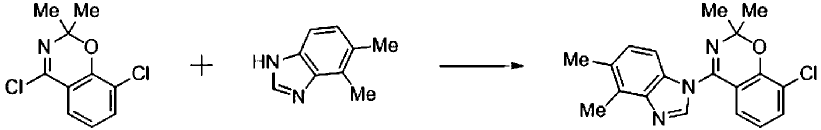

### <Reference Example 2>

### Synthesis of 2,2,8-trimethyl-2,3-dihydro-4H-benzo[e][1,3]oxazin-4-one

20.0 ml of a solution containing 6.43 g of 2-hydroxy-3-methylbenzamide and 9.41 g of p-toluenesulfonic acid monohydrate in 2,2-dimethoxypropane was stirred at 80°C for 3 hours. The reaction mixture was allowed to cool to room temperature and then phase-separated by the addition of a saturated aqueous solution of sodium bicarbonate and ethyl acetate. The obtained organic layer was dried over sodium sulfate, and the solvent was distilled off under reduced pressure. The obtained residue was washed with diisopropyl ether so that the title compound was obtained as 4.16 g of a pale yellow solid.

¹H-NMR (CDCl₃) δ: 7.77 (1H, dd, J = 7.6, 1.5 Hz), 7.32-7.30 (1H, m), 6.96 (1H, t, J = 7.6 Hz), 6.44 (1H, brs), 2.22 (3H, s), 1.65 (6H, s).

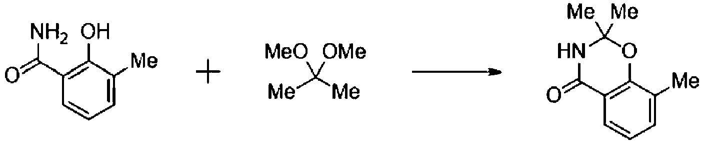

### <Synthesis Example 2 - Step 1>

### Synthesis of 4-chloro-2,2,8-trimethyl-2H-benzo[e][1,3]oxazine

167 µl of oxalyl chloride was added to 7.5 ml of a dichloromethane solution containing 240 µl of dimethylformamide, and the mixture was stirred at room temperature for 10 minutes. Subsequently, 250 mg of 2,2,8-trimethyl-2,3-dihydro-4H-benzo[e][1,3]oxazine-4-one obtained by the method of Reference Example 2 was added thereto, and the mixture was further stirred at room temperature for 30 minutes. The solvent was distilled off from the obtained reaction mixture to obtain 300 mg of a yellow oil containing the title compound. The obtained product was used in the next reaction without being further purified.

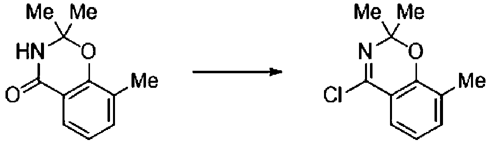

### <Synthesis Example 2 - Step 2>

### Synthesis of 4-(4,5-dimethyl-1H-benzo[d]imidazol-1-yl)-2,2,8-trimethyl-2H-benzo[e][1,3]oxazine (compound No. 9)

2.5 ml of a pyridine solution containing 300 mg of 4-chloro-2,2,8-trimethyl-2H-benzo[e][1,3]oxazine obtained by the method of step 1 of Synthesis Example 2 and 191 mg of 4,5-dimethyl-1H-benzo[d]imidazole was stirred at 100°C for 1.5 hours. The reaction mixture was allowed to cool to room temperature and then phase-separated with a saturated aqueous solution of sodium bicarbonate and ethyl acetate. The obtained organic layer was dried over sodium sulfate, and the solvent was distilled off under reduced pressure. The obtained residue was purified by silica gel column chromatography. The title compound was obtained as 287 mg of a brown solid.

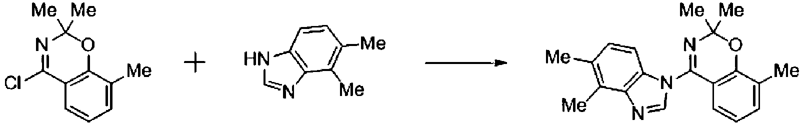

### <Synthesis Example 3>

### Synthesis of 2,2,8-trimethyl-4-(2,4,5-trimethyl-1H-benzo[d]imidazol-1-yl)-2H-benzo[e][1,3]oxazine (compound No. 55)

0.35 ml of a 1.6 mol/l solution of butyllithium in hexane was added dropwise at -78°C to 2.7 ml of a THF solution containing 90 mg of 4-(4,5-dimethyl-1H-benzo[d]imidazole-1-yl)-2,2,8-trimethyl-2H-benzo[e][1,3]oxazine obtained by the method of step 2 of Synthesis Example 2, and the mixture was stirred for 10 minutes. Subsequently, 35 µl of methyl iodide was added dropwise thereto at -78°C, and the mixture was stirred for 20 minutes. The reaction mixture was phase-separated by the addition of a saturated aqueous solution of ammonium chloride and ethyl acetate. The obtained organic layer was dried over sodium sulfate, and the solvent was distilled off under reduced pressure. The obtained residue was purified by silica gel column chromatography. The title compound was obtained as 26.5 mg of a brown solid.

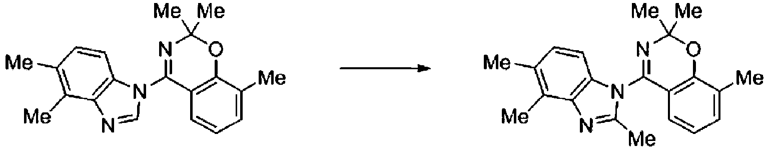

### <Synthesis Example 4>

### Synthesis of 4-(4-bromo-1H-benzo[d]imidazol-1-yl)-2,2,8-trimethyl-2H-benzo[e][1,3]oxazine (compound No. 65)

20 ml of a pyridine solution containing 2.5 g of 4-chloro-2,2,8-trimethyl-2H-benzo[e][1,3]oxazine obtained by the method of step 1 of Synthesis Example 2 and 3.09 g of 4-bromo-1H-benzo[d]imidazole was stirred at 100°C for 1 hour. The reaction mixture was allowed to cool to room temperature and then phase-separated with a saturated aqueous solution of sodium bicarbonate and ethyl acetate. The obtained organic layer was dried over sodium sulfate, and the solvent was distilled off under reduced pressure. The obtained residue was purified by silica gel column chromatography. The title compound was obtained as 1.04 g of a brown amorphous product.

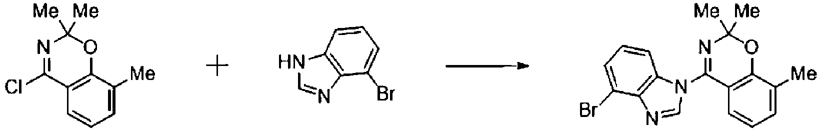

### <Synthesis Example 5>

### Synthesis of 4-(4-ethyl-1H-benzo[d]imidazol-1-yl)-2,2,8-trimethyl-2H-benzo[e][1,3]oxazine (compound No. 69)

1.2 ml of a dioxane solution containing 120 mg of 4-(4-bromo-1H-benzo[d]imidazole-1-yl)-2,2,8-trimethyl-2H-benzo[e][1,3]oxazine obtained by the method of Synthesis Example 4, 71.9 mg of ethylboronic acid, 26.0 mg of [1,1-bis(diphenylphosphino)ferrocene]dichloropalladium(II), and 240 mg of tripotassium phosphate was stirred at 100°C for 2.5 hours. The reaction mixture was allowed to cool to room temperature and then phase-separated with water and ethyl acetate. The obtained organic layer was dried over sodium sulfate, and the solvent was distilled off under reduced pressure. The obtained residue was purified by silica gel column chromatography. The title compound was obtained as 8.9 mg of a colorless gum-like product.

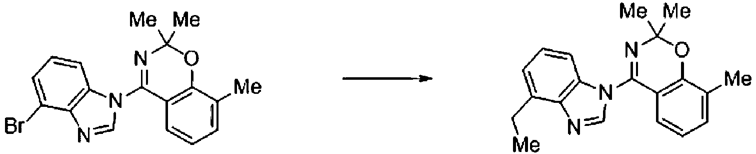

### <Synthesis Example 6>

### Synthesis of 4-(4-cyclopropyl-1H-benzo[d]imidazol-1-yl)-2,2,8-trimethyl-2H-benzo[e][1,3]oxazine (compound No. 70)

1.3 ml of a dioxane solution containing 130 mg of 4-(4-bromo-1H-benzo[d]imidazole-1-yl)-2,2,8-trimethyl-2H-benzo[e][1,3]oxazine obtained by the method of Synthesis Example 4, 45.3 mg of cyclopropylboronic acid, 28.6 mg of [1,1-bis(diphenylphosphino)ferrocene]dichloropalladium(II), and 223 mg of tripotassium phosphate was stirred at 100°C for 1.5 hours. The reaction mixture was allowed to cool to room temperature and then phase-separated with water and ethyl acetate. The obtained organic layer was dried over sodium sulfate, and the solvent was distilled off under reduced pressure. The obtained residue was purified by silica gel column chromatography. The title compound was obtained as 40.3 mg of a light brown amorphous product.

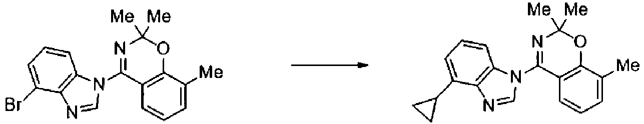

### <Synthesis Example 7>

### Synthesis of 2,2,8-trimethyl-4-(4-vinyl-1H-benzo[d]imidazol-1-yl)-2H-benzo[e][1,3]oxazine (compound No. 72)

2 ml of a dioxane solution containing 100 mg of 4-(4-bromo-1H-benzo[d]imidazole-1-yl)-2,2,8-trimethyl-2H-benzo[e][1,3]oxazine obtained by the method of Synthesis Example 4, 71.9 mg of 4,4,5,5-tetramethyl-2-vinyl-1,3,2-dioxaborolane, 22 mg of [1,1-bis(diphenylphosphino)ferrocene]dichloropalladium(II), and 172 mg of tripotassium phosphate was stirred at 100°C for 3 hours. The reaction mixture was allowed to cool to room temperature and then phase-separated with water and ethyl acetate. The obtained organic layer was dried over sodium sulfate, and the solvent was distilled off under reduced pressure. The obtained residue was purified by silica gel column chromatography. The title compound was obtained as 6.9 mg of a white solid.

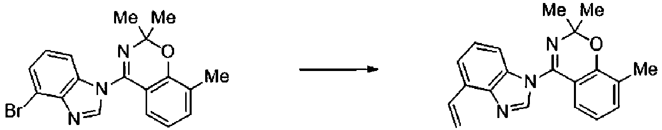

### <Synthesis Example 8>

### Synthesis of 2,2,8-trimethyl-4-(4-(prop-1-en-2-yl)-1H-benzo[d]imidazol-1-yl)-2H-benzo[e][1,3]oxazine (compound No. 73)

4 ml of a dioxane solution containing 400 mg of 4-(4-bromo-1H-benzo[d]imidazole-1-yl)-2,2,8-trimethyl-2H-benzo[e][1,3]oxazine obtained by the method of Synthesis Example 4, 477 mg of potassium isopropenyltrifluoroborate, 88 mg of [1,1-bis(diphenylphosphino)ferrocene]dichloropalladium(II), and 801 mg of tripotassium phosphate was stirred at 100°C for 2 hours. The reaction mixture was allowed to cool to room temperature and then phase-separated with water and ethyl acetate. The obtained organic layer was dried over sodium sulfate, and the solvent was distilled off under reduced pressure. The obtained residue was purified by silica gel column chromatography. The title compound was obtained as 14 mg of a white solid.

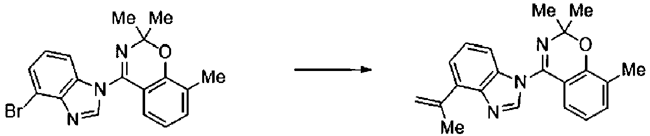

### <Reference Example 3>

### Synthesis of 8-bromo-2,2-dimethyl-2,3-dihydro-4H-benzo[e][1,3]oxazine-4-one

30 ml of a toluene solution containing 6.13 g of 3-bromo-2-hydroxybenzamide, 3.24 g of p-toluenesulfonic acid monohydrate, and 6.95 ml of 2,2-dimethoxypropane was stirred at 110°C for 3.5 hours. The reaction mixture was allowed to cool to room temperature and then phase-separated by the addition of a saturated aqueous solution of sodium bicarbonate and ethyl acetate. The obtained organic layer was dried over sodium sulfate, and the solvent was distilled off under reduced pressure. The obtained residue was purified by silica gel column chromatography. The obtained residue was washed with diisopropyl ether so that the title compound was obtained as 3.20 g of a white solid.

¹H-NMR (CDCl₃) δ: 7.89 (1H, dd, J = 7.6, 1.5 Hz), 7.69 (1H, dd, J = 7.8, 1.7 Hz), 6.96 (1H, t, J = 7.8 Hz), 6.25 (1H, s), 1.70 (6H, s).

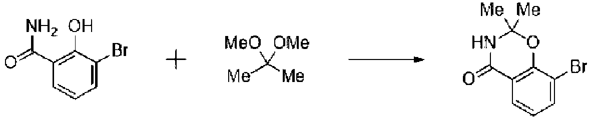

### <Synthesis Example 9 - Step 1>

### Synthesis of 8-bromo-4-chloro-2,2-dimethyl-2H-benzo[e][1,3]oxazine

638 µl of oxalyl chloride was added to 19 ml of a dichloromethane solution containing 936 µl of dimethylformamide, and the mixture was stirred at room temperature for 15 minutes. Subsequently, 1.27 g of 8-bromo-2,2-dimethyl-2,3-dihydro-4H-benzo[e][1,3]oxazin-4-one obtained by the method of Reference Example 3 was added thereto, and the mixture was further stirred at room temperature for 35 minutes. The solvent was distilled off from the obtained reaction mixture to obtain 1.50 g of a yellow oil containing the title compound. The obtained product was used in the next reaction without being further purified.

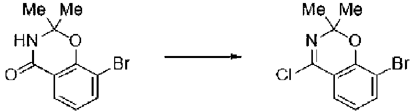

### <Synthesis Example 9 - Step 2>

### Synthesis of 8-bromo-4-(4,5-dimethyl-1H-benzo[d]imidazol-1-yl)-2,2-dimethyl-2H-benzo[e][1,3]oxazine

### (compound No. 94)

13 ml of a pyridine solution containing 1.50 g of 8-bromo-4-chloro-2,2-dimethyl-2H-benzo[e][1,3]oxazine obtained by the method of step 1 of Synthesis Example 9 and 1.10 g of 4,5-dimethyl-1H-benzo[d]imidazole was stirred at 100°C for 3.5 hours. The reaction mixture was allowed to cool to room temperature and then phase-separated with a saturated aqueous solution of sodium bicarbonate and ethyl acetate. The obtained organic layer was dried over sodium sulfate, and the solvent was distilled off under reduced pressure. The obtained residue was purified by silica gel column chromatography. The title compound was obtained as 520 mg of an orange solid.

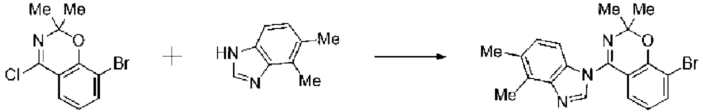

### <Synthesis Example 10>

### Synthesis of 8-cyclopropyl-2,2-dimethyl-4-(2,4,5-trimethyl-1H-benzo[d]imidazol-1-yl)-2H-benzo[e][1,3]oxazine (compound No. 95)

1.00 ml of a dioxane solution containing 100 mg of 8-bromo-4-(4,5-dimethyl-1H-benzo[d]imidazol-1-yl)-2,2-dimethyl-2H-benzo[e][1,3]oxazine obtained by the method of step 2 of Synthesis Example 9, 33.5 mg of cyclopropylboronic acid, 21.2 mg of [1,1-bis(diphenylphosphino)ferrocene]dichloropalladium(II), and 166 mg of tripotassium phosphate was stirred at 100°C for 1.5 hours. The reaction mixture was allowed to cool to room temperature, then 33.5 mg of cyclopropylboronic acid and 21.2 mg of [1,1-bis(diphenylphosphino)ferrocene]dichloropalladium(II) were further added thereto, and the mixture was stirred at 100°C for 1.5 hours. The reaction mixture was phase-separated with water and ethyl acetate. The obtained organic layer was dried over sodium sulfate, and the solvent was distilled off under reduced pressure. The obtained residue was purified by silica gel column chromatography. The title compound was obtained as 71.0 mg of a yellow solid.

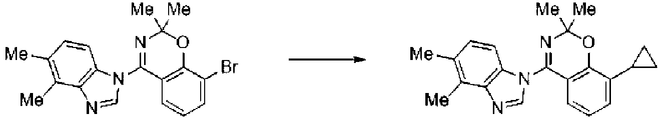

### <Reference Example 4>

### Synthesis of 2-ethyl-2,8-dimethyl-2,3-dihydro-4H-benzo[e][1,3]oxazin-4-one

A mixed solution containing 500 mg of 2-hydroxy-3-methylbenzamide and 581 µl of trifluoromethanesulfonic acid in 2.5 ml of 2-butanone and 2.5 ml of toluene was stirred at 80°C for 30 minutes. The reaction mixture was allowed to cool to room temperature and then phase-separated by the addition of a saturated aqueous solution of sodium bicarbonate and ethyl acetate. The obtained organic layer was dried over sodium sulfate, and the solvent was distilled off under reduced pressure. The obtained residue was purified by silica gel column chromatography. The title compound was obtained as 420 mg of an orange oil.

¹H-NMR (CDCl₃) δ: 7.76 (1H, dd, J = 7.8, 1.7 Hz), 7.32-7.29 (1H, m), 6.95 (1H, t, J = 7.6 Hz), 6.73 (1H, brs), 2.22 (3H, s), 2.00-1.84 (2H, m), 1.59 (3H, s), 1.02 (3H, t, J = 7.4 Hz).

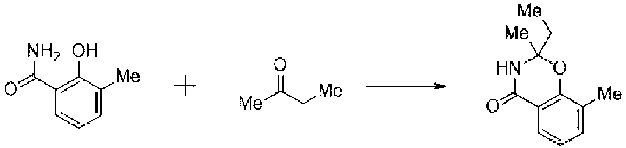

### <Synthesis Example 11 - Step 1>

### Synthesis of 4-chloro-2-ethyl-2,8-dimethyl-2H-benzo[e][1,3]oxazine

125 µl of oxalyl chloride was added to 3.00 ml of a dichloromethane solution containing 199 µl of dimethylformamide, and the mixture was stirred at room temperature for 15 minutes. Subsequently, 200 mg of 2-ethyl-2,8-dimethyl-2,3-dihydro-4H-benzo[e][1,3]oxazin-4-one obtained by the method of Reference Example 4 was added thereto, and the mixture was further stirred at room temperature for 15 minutes. The solvent was distilled off from the obtained reaction mixture to obtain 250 mg of a yellow oil containing the title compound. The obtained product was used in the next reaction without being further purified.

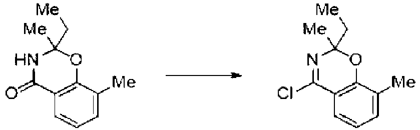

### <Synthesis Example 11 - Step 2>

### Synthesis of 4-(4,5-dimethyl-1H-benzo[d]imidazol-1-yl)-2-ethyl-2,8-dimethyl-2H-benzo[e][1,3]oxazine (compound No. 113)

2.00 ml of a pyridine solution containing 250 mg of 4-chloro-2-ethyl-2,8-dimethyl-2H-benzo[e][1,3]oxazine obtained by the method of step 1 of Synthesis Example 11 and 199 mg of 4,5-dimethyl-1H-benzo[d]imidazole was stirred at 100°C for 2 hours. The reaction mixture was allowed to cool to room temperature and then phase-separated with a saturated aqueous solution of sodium bicarbonate and ethyl acetate. The obtained organic layer was dried over sodium sulfate, and the solvent was distilled off under reduced pressure. The obtained residue was purified by silica gel column chromatography. The title compound was obtained as 24.0 mg of a yellow solid.

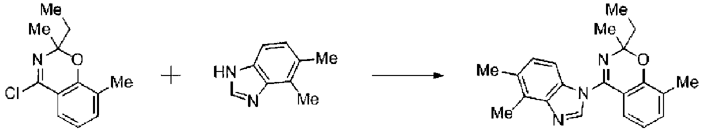

### <Reference Example 5>

### Synthesis of 8-methylspiro[benzo[e][1,3]oxazine-2,1'-cyclobutan]-4(3H)-one

10 ml of a toluene solution containing 2.10 g of 2-hydroxy-3-methylbenzamide, 3.10 ml of cyclobutanone, and 1.30 ml of trifluoromethanesulfonic acid was stirred at 100°C for 3 hours. The reaction mixture was allowed to cool to room temperature and then phase-separated by the addition of a saturated aqueous solution of sodium bicarbonate and ethyl acetate. The obtained organic layer was dried over sodium sulfate, and the solvent was distilled off under reduced pressure. The obtained residue was purified by silica gel column chromatography. The title compound was obtained as 899 mg of an orange solid.

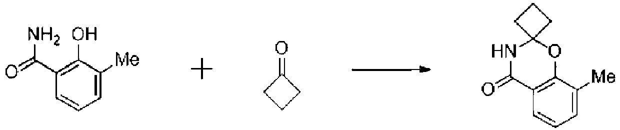

### <Synthesis Example 12 - Step 1>

### Synthesis of 4-chloro-8-methylspiro[benzo[e][1,3]oxazine-2,1'-cyclobutane]

126 µl of oxalyl chloride was added to 3.00 ml of a dichloromethane solution containing 201 µl of dimethylformamide, and the mixture was stirred at room temperature for 15 minutes. Subsequently, 200 mg of 8-methylspiro[benzo[e][1,3]oxazine-2,1'-cyclobutan]-4(3H)-one obtained by the method of Reference Example 5 was added thereto, and the mixture was further stirred at room temperature for 15 minutes. The solvent was distilled off from the obtained reaction mixture to obtain 250 mg of a yellow oil containing the title compound. The obtained product was used in the next reaction without being further purified.

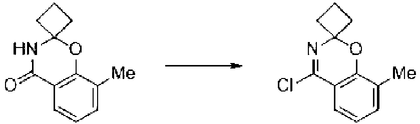

### <Synthesis Example 12 - Step 2>

### Synthesis of 8-methyl-4-(4-methyl-1H-benzo[d]imidazol-1-yl)spiro[benzo[e][1,3]oxazine-2,1'-cyclobutane] (compound No. 120)

2.00 ml of a pyridine solution containing 250 mg of 4-chloro-8-methylspiro[benzo[e][1,3]oxazine-2,1'-cyclobutane] obtained by the method of step 1 of Synthesis Example 2 and 195 mg of 4-methyl-1H-benzo[d]imidazole was stirred at 100°C for 2 hours. The reaction mixture was allowed to cool to room temperature and then phase-separated with a saturated aqueous solution of sodium bicarbonate and ethyl acetate. The obtained organic layer was dried over sodium sulfate, and the solvent was distilled off under reduced pressure. The obtained residue was purified by silica gel column chromatography. The title compound was obtained as 161 mg of an orange oil.

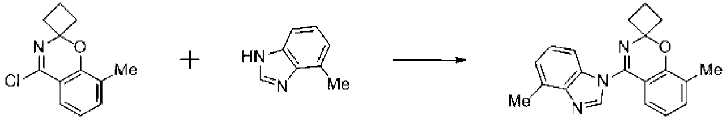

### <Reference Example 6>

### Synthesis of 7-bromo-2,2-dimethyl-2,3-dihydro-4H-benzo[e][1,3]oxazin-4-one

A mixed solution containing 5.00 g of 4-bromo-2-hydroxybenzamide and 4.39 g of p-toluenesulfonic acid monohydrate in 25.0 ml of 2,2-dimethoxypropane and 25 ml of toluene was stirred at 80°C for 3 hours. The reaction mixture was allowed to cool to room temperature and then phase-separated by the addition of a saturated aqueous solution of sodium bicarbonate and ethyl acetate. The obtained organic layer was washed with saturated saline and dried over sodium sulfate. The solvent was distilled off under reduced pressure, and the obtained residue was then washed with diisopropyl ether. The title compound was obtained as 4.50 g of a light brown solid.

¹H-NMR (CDCl₃) δ: 7.78 (1H, d, J = 8.3 Hz,), 7.21 (1H, dd, J = 8.3, 2.0 Hz,), 7.12 (1H, d, J = 2.0 Hz,), 6.34 (1H, brs,), 1.65 (6H, s,).

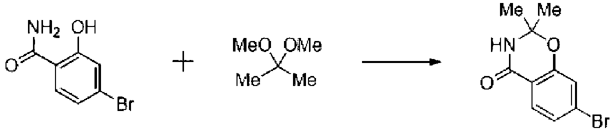

### <Synthesis Example 13 - Step 1>

### Synthesis of 7-bromo-4-chloro-2,2-dimethyl-2H-benzo[e][1,3]oxazine

151 µl of oxalyl chloride was added to 3.00 ml of a dichloromethane solution containing 213 µl of dimethylformamide, and the mixture was stirred at room temperature for 1 hour. Subsequently, 300 mg of 7-bromo-2,2-dimethyl-2,3-dihydro-4H-benzo[e][1,3]oxazin-4-one obtained by the method of Reference Example 6 was added thereto, and the mixture was further stirred at room temperature for 10 minutes. The solvent was distilled off from the obtained reaction mixture to obtain 350 mg of a yellow oil containing the title compound. The obtained product was used in the next reaction without being further purified.

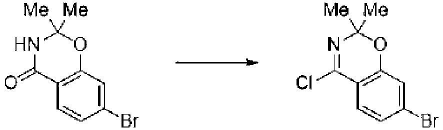

### <Synthesis Example 13 - Step 2>

### Synthesis of 7-bromo-4-(4,5-dimethyl-1H-benzo[d]imidazol-1-yl)-2,2-dimethyl-2H-benzo[e][1,3]oxazine (compound No. 17)

3.00 ml of a pyridine solution containing 350 mg of 7-bromo-4-chloro-2,2-dimethyl-2H-benzo[e][1,3]oxazine obtained by the method of step 1 of Synthesis Example 13 and 257 mg of 4,5-dimethyl-1H-benzo[d]imidazole was stirred at 100°C for 1.5 hours. The reaction mixture was allowed to cool to room temperature and then phase-separated with a saturated aqueous solution of sodium bicarbonate and ethyl acetate. The obtained organic layer was dried over sodium sulfate, and the solvent was distilled off under reduced pressure. The obtained residue was purified by silica gel column chromatography. The title compound was obtained as 15.0 mg of a brown solid.

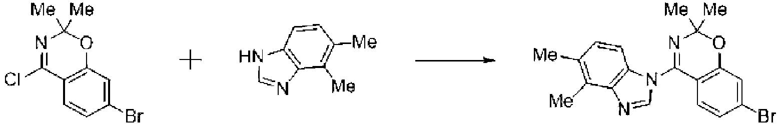

### <Synthesis Example 14>

### Synthesis of 4-(4,5-dimethyl-1H-benzo[d]imidazol-1-yl)-N-ethyl-2,2-dimethyl-2H-benzo[e][1,3]oxazin-7-amine (compound No. 126)

3.00 ml of a toluene solution containing 300 mg of 7-bromo-4-(4,5-dimethyl-1H-benzo[d]imidazol-1-yl)-2,2-dimethyl-2H-benzo[e][1,3]oxazine obtained by the method of step 2 of Synthesis Example 13, 1.95 ml of a 2 mol/l solution of ethylamine in THF, 143 mg of tris(dibenzylideneacetone)dipalladium(0), 180 mg of 4,5-bis(diphenylphosphino)-9,9-dimethylxanthene, and 636 mg of cesium carbonate was stirred at 110°C for 5 hours. The reaction mixture was allowed to cool to room temperature and then phase-separated with water and ethyl acetate. The obtained organic layer was dried over sodium sulfate, and the solvent was distilled off under reduced pressure. The obtained residue was purified by silica gel column chromatography. The title compound was obtained as 140 mg of a yellow amorphous product.

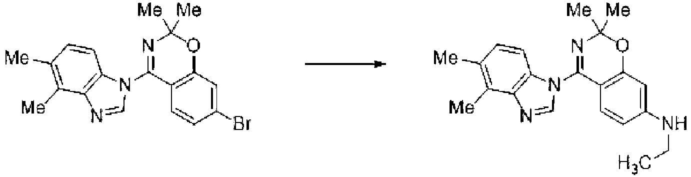

### <Synthesis Example 15>

### Synthesis of 4-(4,5-dimethyl-1H-benzo[d]imidazol-1-yl)-N-ethyl-N,2,2-trimethyl-2H-benzo[e][1,3]oxazin-7-amine (compound No. 127)

A THF solution containing 100 mg of 4-(4,5-dimethyl-1H-benzo[d]imidazol-1-yl)-N-ethyl-2,2-dimethyl-2H-benzo[e][1,3]oxazin-7-amine obtained by the method of Synthesis Example 14 and 17.2 mg of sodium hydride (oil, ca, 60% purity) was stirred at room temperature for 1 hour. Subsequently, 19.7 µl of iodomethane was added to the reaction mixture, and the mixture was stirred at 60°C for 7 hours. The reaction mixture was allowed to cool to room temperature and then phase-separated with water and ethyl acetate. The obtained organic layer was dried over sodium sulfate, and the solvent was distilled off under reduced pressure. The obtained residue was purified by silica gel column chromatography. The title compound was obtained as 40.0 mg of a white amorphous product.

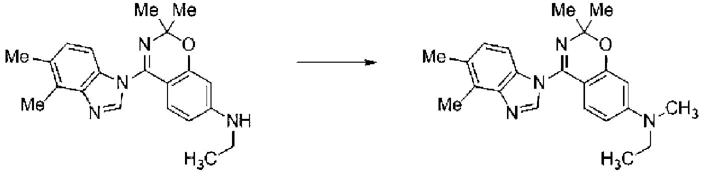

### <Reference Example 7>

### Synthesis of ethyl 2-(2,7-dimethyl-4-oxo-3,4-dihydro-2H-benzo[e][1,3]oxazin-2-yl)acetate

5.00 ml of a solution containing 1.00 g of 2-hydroxy-4-methylbenzamide and 1.70 g of pyridinium p-toluenesulfonate in ethyl acetoacetate was stirred at 120°C for 3 hours. The reaction mixture was allowed to cool to room temperature and then phase-separated with water and ethyl acetate. The obtained organic layer was washed with saturated saline and dried over sodium sulfate. The solvent was distilled off under reduced pressure, and the obtained residue was then purified by silica gel column chromatography. The title compound was obtained as 1.00 g of a white solid.

¹H-NMR (CDCl₃) δ: 7.81 (1H, d, J = 7.8 Hz), 6.90 (1H, d, J = 7.8 Hz), 6.77-6.72 (2H, brm), 4.18 (2H, q, J = 7.2 Hz), 3.04 (1H, d, J = 15.6 Hz), 2.85 (1H, d, J = 15.6 Hz), 2.36 (3H, s), 1.72 (3H, s), 1.27 (3H, t, J = 7.2 Hz).

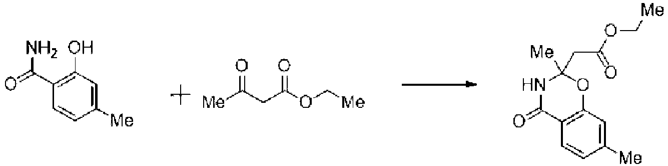

### <Synthesis Example 16 - Step 1>

### Synthesis of ethyl 2-(4-chloro-2,7-dimethyl-2H-benzo[e][1,3]oxazin-2-yl)acetate

980 µl of oxalyl chloride was added to 15.0 ml of a dichloromethane solution containing 1.50 ml of dimethylformamide, and the mixture was stirred at room temperature for 15 minutes. Subsequently, 1.00 g of ethyl 2-(2,7-dimethyl-4-oxo-3,4-dihydro-2H-benzo[e][1,3]oxazin-2-yl)acetate obtained by the method of Reference Example 7 was added thereto, and the mixture was further stirred at room temperature for 30 minutes. The solvent was distilled off from the obtained reaction mixture to obtain 1.20 g of a yellow oil containing the title compound. The obtained product was used in the next reaction without being further purified.

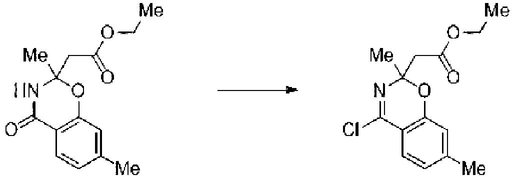

### <Synthesis Example 16 - Step 2>

### Synthesis of ethyl 2-(4-(4,5-dimethyl-1H-benzo[d]imidazol-1-yl)-2,7-dimethyl-2H-benzo[e][1,3]oxazin-2-yl)acetate (compound No. 176)

10.0 ml of a pyridine solution containing 1.20 g of ethyl 2-(4-chloro-2,7-dimethyl-2H-benzo[e][1,3]oxazin-2-yl)acetate obtained by the method of step 1 of Synthesis Example 16 and 877 mg of 4,5-dimethyl-1H-benzo[d]imidazole was stirred at 100°C for 1.5 hours. The reaction mixture was allowed to cool to room temperature and then phase-separated with a saturated aqueous solution of sodium bicarbonate and ethyl acetate. The obtained organic layer was washed with saturated saline and dried over sodium sulfate. The solvent was distilled off under reduced pressure, and the obtained residue was then purified by silica gel column chromatography. The title compound was obtained as 211 mg of an orange solid.

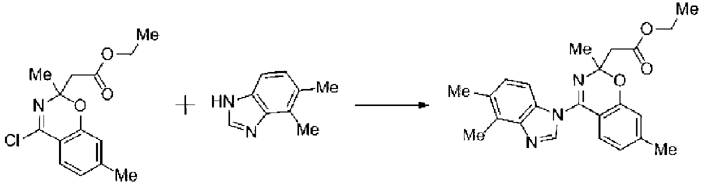

### <Synthesis Example 17>

### Synthesis of 2-(4-(4,5-dimethyl-1H-benzo[d]imidazol-1-yl)-2,7-dimethyl-2H-benzo[e][1,3]oxazin-2-yl)acetic acid (compound No. 177)

A mixed solution containing 199 mg of ethyl 2-(4-(4,5-dimethyl-1H-benzo[d]imidazol-1-yl)-2,7-dimethyl-2H-benzo[e][1,3]oxazin-2-yl)acetate obtained by the method of step 1 of Synthesis Example 16 and 40.6 mg of sodium hydroxide in 1 ml of ethanol and 1 ml of water was stirred at 80°C for 1 hour. The reaction mixture was allowed to cool to room temperature, and 5 ml of a 1 mol/l aqueous hydrochloric acid solution was then added thereto, followed by extraction with ethyl acetate three times. The obtained organic layer was dried over sodium sulfate, and the solvent was distilled off under reduced pressure. The title compound was obtained as 123 mg of a white solid.

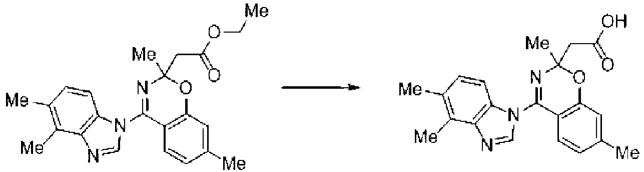

### <Synthesis Example 18>

### Synthesis of 2-(4-(4,5-dimethyl-1H-benzo[d]imidazol-1-yl)-2,7-dimethyl-2H-benzo[e][1,3]oxazin-2-yl)-N-methylacetamide (compound No. 196)

0.3 ml of a DMF solution containing 22.9 mg of 2-(4-(4,5-dimethyl-1H-benzo[d]imidazol-1-yl)-2,7-dimethyl-2H-benzo[e][1,3]oxazin-2-yl)acetic acid obtained by the method of Synthesis Example 17 and 20.4 mg of 1,1'-carbonyldiimidazole was stirred at room temperature for 1 hour, then 0.3 ml of methylamine (40%, methanol solution) was added thereto, and the mixture was further stirred at room temperature for 1 hour. The reaction mixture was phase-separated by the addition of water and ethyl acetate. The obtained organic layer was dried over sodium sulfate, and the solvent was distilled off under reduced pressure. The obtained residue was purified by silica gel column chromatography. The title compound was obtained as 16.8 mg of a white solid.

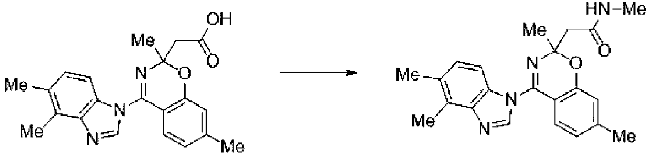

### <Reference Example 8>

### Synthesis of 2,7-dimethyl-2-((methylthio)methyl)-2,3-dihydro-4H-benzo[e][1,3]oxazin-4-one

7.50 ml of a solution containing 1.50 g of 2-hydroxy-4-methylbenzamide and 2.50 g of pyridinium p-toluenesulfonate in 1-methylthio-2-propanone was stirred at 120°C for 5 hours. The reaction mixture was allowed to cool to room temperature and then phase-separated by the addition of a saturated aqueous solution of sodium bicarbonate and ethyl acetate. The obtained organic layer was dried over sodium sulfate, and the solvent was distilled off under reduced pressure. The obtained residue was purified by silica gel column chromatography. The title compound was obtained as 1.27 g of a yellow solid.

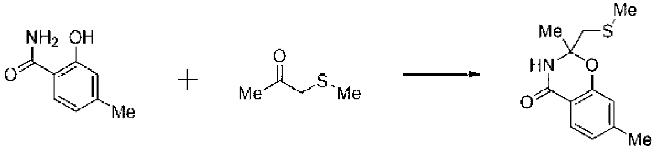

### <Synthesis Example 19 - Step 1>

### Synthesis of 4-chloro-2,7-dimethyl-2-((methylthio)methyl)-2H-benzo[e][1,3]oxazine

1.00 ml of oxalyl chloride was added to 15.0 ml of a dichloromethane solution containing 1.50 ml of dimethylformamide, and the mixture was stirred at room temperature for 15 minutes. Subsequently, 932 mg of 2,7-dimethyl-2-((methylthio)methyl)-2,3-dihydro-4H-benzo[e][1,3]oxazin-4-one obtained by the method of Reference Example 8 was added thereto, and the mixture was further stirred at room temperature for 20 minutes. The solvent was distilled off from the obtained reaction mixture to obtain 1.20 g of a yellow oil containing the title compound. The obtained product was used in the next reaction without being further purified.

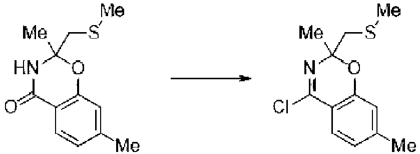

### <Synthesis Example 19 - Step 2>

### Synthesis of 4-(4,5-dimethyl-1H-benzo[d]imidazol-1-yl)-2,7-dimethyl-2-((methylthio)methyl)-2H-benzo[e][1,3]oxazine (compound No. 201)

10.0 ml of a pyridine solution containing 1.20 g of 4-chloro-2,7-dimethyl-2-((methylthio)methyl)-2H-benzo[e][1,3]oxazine obtained by the method of step 1 of Synthesis Example 19 and 899 mg of 4,5-dimethyl-1H-benzo[d]imidazole was stirred at 100°C for 2.5 hours. The reaction mixture was allowed to cool to room temperature and then phase-separated with a saturated aqueous solution of sodium bicarbonate and ethyl acetate. The obtained organic layer was washed with saturated saline and dried over sodium sulfate. The solvent was distilled off under reduced pressure, and the obtained residue was then purified by silica gel column chromatography. The title compound was obtained as 130 mg of a yellow solid.

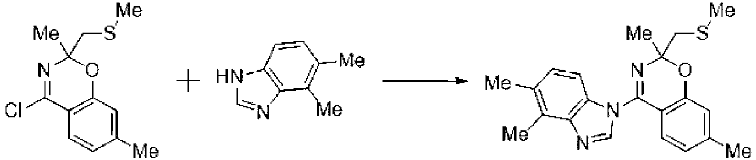

### <Synthesis Example 20>

### Synthesis of 4-(4,5-dimethyl-1H-benzo[d]imidazol-1-yl)-2,7-dimethyl-2-((methylsulfonyl)methyl)-2H-benzo[e][1,3]oxazine (compound No. 211)

1.2 ml of a dichloromethane solution containing 4-(4,5-dimethyl-1H-benzo[d]imidazol-1-yl)-2,7-dimethyl-2-((methylthio)methyl)-2H-benzo[e][1,3]oxazine obtained by the method of step 2 of Synthesis Example 19 and 87.0 mg of 3-chloroperbenzoic acid (ca. 70% purity) was stirred at 0°C for 15 minutes. The reaction mixture was phase-separated by the addition of a saturated aqueous solution of sodium thiosulfate and dichloromethane. The obtained organic layer was washed with a saturated aqueous solution of sodium bicarbonate and dried over sodium sulfate. The solvent was distilled off under reduced pressure, and the obtained residue was then purified by silica gel column chromatography. The title compound was obtained as 36.1 mg of a yellow solid.

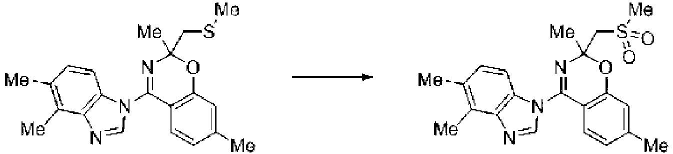

### <Reference Example 9>

### Synthesis of 2,2,8-trimethyl-2,3-dihydro-4H-benzo[e][1,3]thiazin-4-one

3 ml of a solution containing 320 mg of 7-methylbenzo[d]isothiazol-3(2H)-one and 291 mg of zinc in acetic acid was stirred at room temperature for 2 hours. Subsequently, 3 ml of 2,2-dimethoxypropane and 369 mg of p-toluenesulfonic acid monohydrate were added thereto, and the mixture was stirred at 90°C for 4 hours. The reaction mixture was phase-separated by the addition of a saturated aqueous solution of sodium bicarbonate and ethyl acetate, and the organic layer was washed with saturated saline. The obtained organic layer was dried over sodium sulfate, and the solvent was distilled off under reduced pressure. The obtained residue was purified by silica gel column chromatography. The title compound was obtained as 250 mg of a white solid.

¹H-NMR (CDCl₃) δ: 8.00 (1H, d, J = 7.6 Hz,), 7.28 (1H, d, J = 7.6 Hz,), 7.15 (1H, t, J = 7.6 Hz,), 6.63 (1H, brs,), 2.28 (3H, s,), 1.70 (6H, s,).

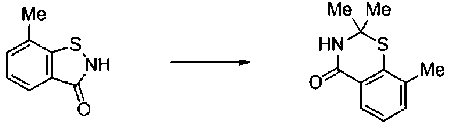

### <Synthesis Example 21 - Step 1>

### Synthesis of 4-chloro-2,2,8-trimethyl-2H-benzo[e][1,3]thiazine

155 µl of oxalyl chloride was added to 3 ml of a dichloromethane solution containing 234 µl of dimethylformamide, and the mixture was stirred at room temperature for 15 minutes. Subsequently, 250 mg of 2,2,8-trimethyl-2,3-dihydro-4H-benzo[e][1,3]thiazin-4-one obtained by the method of Reference Example 9 was added thereto, and the mixture was further stirred at room temperature for 15 minutes. The solvent was distilled off from the obtained reaction mixture to obtain 300 mg of a yellow oil containing the title compound. The obtained product was used in the next reaction without being further purified.

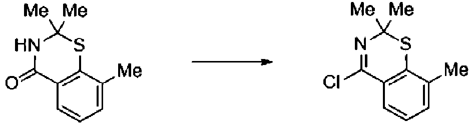

### <Synthesis Example 21 - Step 2>

### Synthesis of 4-(4,5-dimethyl-1H-benzo[d]imidazol-1-yl)-2,2,8-trimethyl-2H-benzo[e][1,3]thiazine (compound No. 75)

3 ml of a pyridine solution containing 300 mg of 4-chloro-2,2,8-trimethyl-2H-benzo[e][1,3]thiazine obtained by the method of step 1 of Synthesis Example 21 and 265 mg of 4,5-dimethyl-1H-benzo[d]imidazole was stirred at 100°C for 2.5 hours. The reaction mixture was allowed to cool to room temperature and then phase-separated with a 1 mol/l aqueous hydrochloric acid solution and ethyl acetate. The obtained organic layer was washed with saturated saline and dried over sodium sulfate. The solvent was distilled off under reduced pressure, and the obtained residue was then purified by silica gel column chromatography. The title compound was obtained as 105 mg of a brown amorphous product.

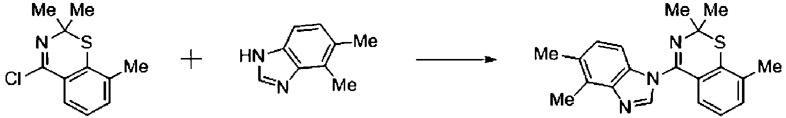

### <Synthesis Example 22>

### Synthesis of 4-(4,5-dimethyl-4-(4,5-dimethyl-1H-benzo[d]imidazol-1-yl)-2,2,8-trimethyl-2H-benzo[e][1,3]thiazine 1,1-dioxide (compound No. 76) and 4-(4,5-dimethyl-1H-benzo[d]imidazol-1-yl)-2,2,8-trimethyl-2H-benzo[e][1,3]thiazine 1-oxide (compound No. 77)

3 ml of a dichloromethane solution containing 30 mg of 4-(4,5-dimethyl-1H-benzo[d]imidazol-1-yl)-2,2,8-trimethyl-2H-benzo[e][1,3]thiazine obtained by the method of step 2 of Synthesis Example 21 and 33.1 mg of m-chloroperbenzoic acid was stirred at room temperature for 2 hours. The reaction mixture was phase-separated with a 1% aqueous sodium thiosulfate solution and ethyl acetate. The obtained organic layer was washed with a saturated aqueous solution of sodium bicarbonate and dried over sodium sulfate. The solvent was distilled off under reduced pressure, and the obtained residue was then purified by silica gel column chromatography. The title compound of No. 76 was obtained as 17.0 mg of a colorless amorphous product, and the title compound of No. 77 was obtained as 13.0 mg of a colorless gum-like product.

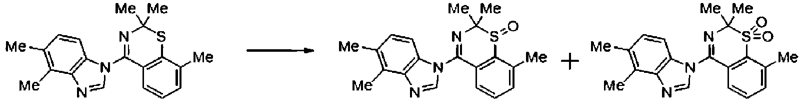

### <Reference Example 10>

### Synthesis of N'-(3,4-dimethylpyridin-2-yl)-N,N-dimethylformimidamide

3 ml of a solution containing 500 mg of 3,4-dimethylpyridin-2-amine and 822 µl of DMF-DMA in toluene was stirred at 100°C for 1 hour. The solvent was distilled off from the obtained reaction mixture to obtain 613 mg of a yellow oil containing the title compound. The obtained product was used in the next reaction without being further purified.

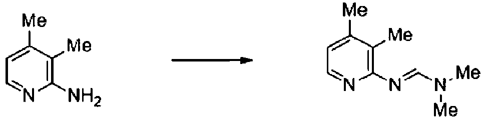

### <Reference Example 11>

### Synthesis of (7,8-dimethylimidazo[1,2-a]pyridin-3-yl)(2-hydroxy-3-methylphenyl)methanone

20 ml of a solution containing 369 mg of N'-(3,4-dimethylpyridin-2-yl)-N,N-dimethylformimidamide obtained by the method of Reference Example 10 and 399 mg of 2-bromo-1-(2-hydroxy-3-methylphenyl)ethan-1-one in DMF was stirred at 80°C for 2 hours. The reaction mixture was allowed to cool to room temperature and then phase-separated with water and ethyl acetate. The obtained organic layer was washed with saturated saline and dried over sodium sulfate. The solvent was distilled off under reduced pressure, and the obtained residue was then purified by silica gel column chromatography. The title compound was obtained as 157 mg of a yellow solid.

¹H-NMR (CDCl₃) δ: 11.73 (1H, d, J = 0.5 Hz,), 9.30 (1H, d, J = 6.8 Hz,), 8.25 (1H, s,), 7.75 (1H, d, J = 8.1 Hz,), 7.38 (1H, d, J = 7.3 Hz,), 6.96 (1H, d, J = 7.1 Hz,), 6.89 (1H, t, J = 7.7 Hz,), 2.63 (3H, s,), 2.45 (3H, s,), 2.33 (3H, s,).

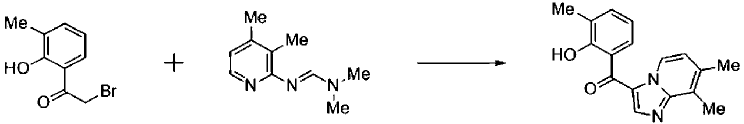

### <Synthesis Example 23 - Step 1>

### Synthesis of 2-((7,8-dimethylimidazo[1,2-a]pyridin-3-yl)(imino)methyl)-6-methylphenol

400 µl of a methanol solution containing 80 mg of (7,8-dimethylimidazo[1,2-a]pyridin-3-yl)(2-hydroxy-3-methylphenyl)methanone obtained by the method of Reference Example 11 and 8 mol/l ammonia was placed in a sealed tube, which was then hermetically closed, followed by stirring at 80°C for 1 hour. The solvent was distilled off from the obtained reaction mixture to obtain 80 mg of a yellow oil containing the title compound. The obtained product was used in the next reaction without being further purified.

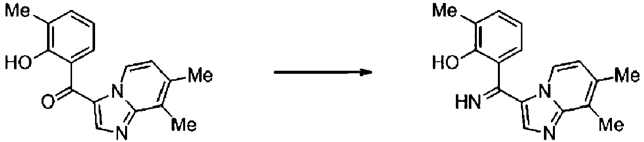

### <Synthesis Example 23 - Step 2>

### Synthesis of 4-(7,8-dimethylimidazo[1,2-a]pyridin-3-yl)-2,2,8-trimethyl-2H-benzo[e][1,3]oxazine (compound No. 129)

A mixed solution containing 80 mg of 2-((7,8-dimethylimidazo[1,2-a]pyridin-3-yl)(imino)methyl)-6-methylpheno obtained by the method of step 1 of Synthesis Example 23 and 54 mg of p-toluenesulfonic acid monohydrate in 400 µl of 2,2-dimethoxypropane and 400 µl of toluene was stirred at 100°C for 2 hours. The reaction mixture was allowed to cool to room temperature and then phase-separated by the addition of a saturated aqueous solution of sodium bicarbonate and ethyl acetate. The obtained organic layer was dried over sodium sulfate, and the solvent was distilled off under reduced pressure. The obtained residue was purified by silica gel column chromatography. The title compound was obtained as 5.1 mg of a brown gum-like product.

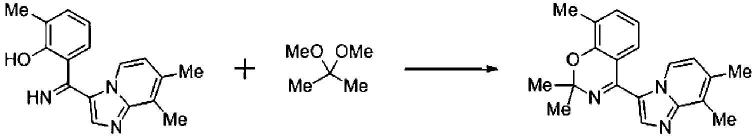

### <Reference Example 12>

### Synthesis of (3-bromo-2-hydroxyphenyl)(6,7-dimethylpyrazolo[1,5-a]pyridin-3-yl)methanone

12 ml of a DMF solution containing 1.20 g of 8-bromo-4H-chromen-4-one, 1.63 g of 1-amino-2,3-dimethyl-pyridinium 2,4-dinitro-phenolate, and 2.21 g of potassium carbonate was stirred at 90°C for 3 hours. The reaction mixture was allowed to cool to room temperature and phase-separated by the addition of water and ethyl acetate. The obtained organic layer was dried over sodium sulfate and concentrated under reduced pressure. The obtained residue was purified by silica gel column chromatography. The title compound was obtained as 760 mg of a brown solid.

¹H-NMR (CDCl₃) δ: 12.84 (1H, s), 8.36 (1H, s), 8.21 (1H, d, J = 9.0 Hz), 7.89 (1H, dd, J = 7.8, 1.5 Hz), 7.75 (1H, dd, J = 7.8, 1.5 Hz), 7.43 (1H, d, J = 9.0 Hz), 6.88 (1H, t, J = 7.8 Hz), 2.82 (3H, s), 2.46 (3H, s).

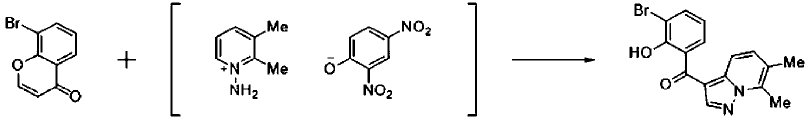

### <Synthesis Example 24 - Step 1>

### Synthesis of 2-bromo-6-((6,7-dimethylpyrazolo[1,5-a]pyridin-3-yl)(imino)methyl)phenol

13.6 ml of a methanol solution containing 680 mg of ((3-bromo-2-hydroxyphenyl)(6,7-dimethylpyrazolo[1,5-a]pyridin-3-yl)methanone obtained by the method of Reference Example 12 and 8 mol/l ammonia was placed in a sealed tube, which was then hermetically closed, followed by stirring at 80°C for 3 hours. The solvent was distilled off from the obtained reaction mixture to obtain 720 mg of a yellow solid containing the title compound. The obtained product was used in the next reaction without being further purified.

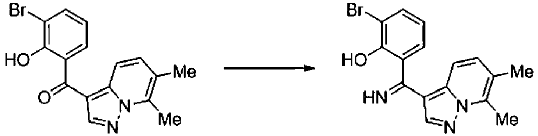

### <Synthesis Example 24 - Step 2>

### Synthesis of 8-bromo-4-(6,7-dimethylpyrazolo[1,5-a]pyridin-3-yl)-2,2-dimethyl-2H-benzo[e][1,3]oxazine (compound No. 174)

6.8 ml of a solution containing 720 mg of 2-((6,7-dimethylpyrazolo[1,5-a]pyridin-3-yl)(imino)methyl)-6-methylphenol obtained by the method of step 1 of Synthesis Example 24, 495 mg of pyridinium p-toluenesulfonate, and 1.4 ml of 2-methoxypropene in DMF was stirred at 90°C for 1.5 hours. The reaction mixture was allowed to cool to room temperature and then phase-separated by the addition of a saturated aqueous solution of sodium bicarbonate and ethyl acetate. The obtained organic layer was dried over sodium sulfate, and the solvent was distilled off under reduced pressure. The obtained residue was purified by silica gel column chromatography. The title compound was obtained as 410 mg of a yellow solid.

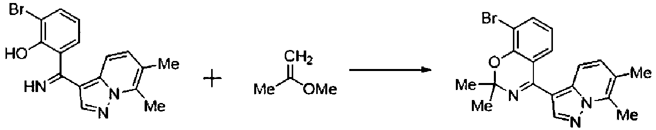

### <Synthesis Example 25>

### Synthesis of 8-cyclopropyl-4-(6,7-dimethylpyrazolo[1,5-a]pyridin-3-yl)-2,2-dimethyl-2H-benzo[e][1,3]oxazine (compound No. 175)

1.3 ml of a dioxane solution containing 100 mg of 8-bromo-4-(6,7-dimethylpyrazolo[1,5-a]pyridin-3-yl)-2,2-dimethyl-2H-benzo[e][1,3]oxazine obtained by the method of step 2 of Synthesis Example 24, 67.0 mg of cyclopropylboronic acid, 21.0 mg of [1,1-bis(diphenylphosphino)ferrocene]dichloropalladium(II), 193 mg of tripotassium phosphate, and 100 µl of H₂O was stirred at 100°C for 2 hours. The reaction mixture was allowed to cool to room temperature and then phase-separated with water and ethyl acetate. The obtained organic layer was dried over sodium sulfate, and the solvent was distilled off under reduced pressure. The obtained residue was purified by silica gel column chromatography. The title compound was obtained as 51.4 mg of a light brown amorphous product.

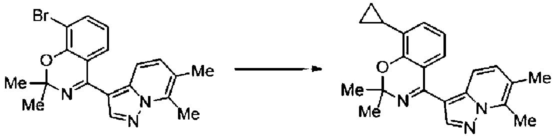

### <Reference Example 13>

### Synthesis of 2-butyl-2-methyl-2,3-dihydro-4H-benzo[e][1,3]thiazin-4-one

10 ml of a solution containing 2.0 g of benzisothiazolinone and 1.73 g of zinc in acetic acid was stirred at room temperature for 1 hour. Subsequently, 10 ml of 2-hexanone and 2.51 g of p-toluenesulfonic acid monohydrate were added thereto, and the mixture was stirred at 110°C for 5 hours. The reaction mixture was phase-separated by the addition of a saturated aqueous solution of sodium bicarbonate and ethyl acetate, and the organic layer was washed with saturated saline. The obtained organic layer was dried over sodium sulfate, and the solvent was distilled off under reduced pressure. The obtained residue was purified by silica gel column chromatography. The title compound was obtained as 2.0 g of a colorless amorphous product.

¹H-NMR (CDCl₃) δ: 8.14-8.11 (1H, m), 7.39 (1H, td, J = 7.6, 1.5 Hz), 7.27-7.23 (2H, m), 6.71 (1H, brs), 1.93-1.88 (2H, m), 1.66 (3H, s), 1.53-1.36 (2H, m), 1.35-1.29 (2H, m), 0.89 (3H, t, J = 7.3 Hz).

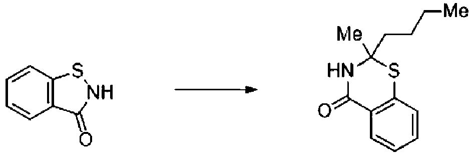

### <Reference Example 14 - Step 1>

### Synthesis of 2-butyl-4-chloro-2-methyl-2H-benzo[e][1,3]thiazine

20 ml of a solution containing 2.0 g of 2-butyl-2-methyl-2,3-dihydro-4H-benzo[e][1,3]thiazin-4-one obtained by the method of Reference Example 13 and 1.31 g of a Vilsmeier reagent in dichloromethane was stirred at room temperature for 1 hour. The solvent was distilled off from the obtained reaction mixture to obtain 2.50 g of a yellow oil containing the title compound. The obtained product was used in the next reaction without being further purified.

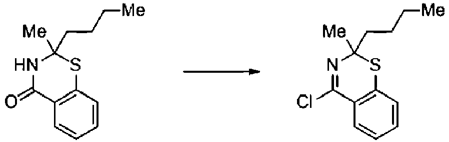

### <Reference Example 14 - Step 2>

### Synthesis of 2-butyl-2-methyl-4-((trimethylsilyl)ethynyl)-2H-benzo[e][1,3]thiazine

20 ml of a solution containing 2.50 g of 2-butyl-4-chloro-2-methyl-2H-benzo[e][1,3]thiazine obtained by the method of step 1 of Reference Example 14, 1.38 ml of trimethylsilylacetylene, 597 mg of bis(triphenylphosphine)palladium(II) dichloride, and 324 mg of copper iodide in triethylamine was stirred at 50°C for 1 hour. The reaction mixture was phase-separated by the addition of water and ethyl acetate, and the organic layer was washed with saturated saline. The obtained organic layer was dried over sodium sulfate, and the solvent was distilled off under reduced pressure. The obtained residue was purified by silica gel column chromatography. The title compound was obtained as 2.0 g of a brown amorphous product.

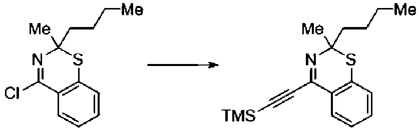

### <Reference Example 14 - Step 3>

### Synthesis of 2-butyl-4-ethynyl-2-methyl-2H-benzo[e][1,3]thiazine

20 ml of a solution containing 2.00 g of 2-butyl-2-methyl-4-((trimethylsilyl)ethynyl)-2H-benzo[e][1,3]thiazine obtained by the method of step 2 of Reference Example 14 and 1.31 g of potassium carbonate in methanol was stirred at room temperature for 1 hour. The reaction mixture was phase-separated by the addition of water and ethyl acetate, and the organic layer was washed with saturated saline. The obtained organic layer was dried over sodium sulfate, and the solvent was distilled off under reduced pressure. The obtained residue was purified by silica gel column chromatography. The title compound was obtained as 1.0 g of a brown amorphous product.

¹H-NMR (CDCl₃) δ: 7.90 (1H, dd, J = 7.6, 1.5 Hz,), 7.32 (1H, td, J = 7.6, 1.5 Hz,), 7.23-7.16 (2H, m,), 3.17 (1H, s,), 1.93-1.82 (2H, m,), 1.54 (3H, t, J = 5.0 Hz,), 1.52-1.38 (2H, m,), 1.35-1.29 (2H, m,), 0.90 (3H, t, J = 7.3 Hz,).

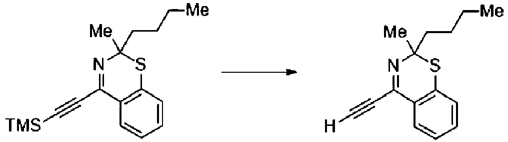

### <Synthesis Example 26>

### Synthesis of 2-butyl-4-(6,7-dimethylpyrazolo[1,5-a]pyridin-3-yl)-2-methyl-2H-benzo[e][1,3]thiazine (compound No. 233) and 2-butyl-2-methyl-4-(4-methylpyrazolo[1,5-a]pyridin-3-yl)-2H-benzo[e][1,3]thiazine (compound No. 234)

10 ml of a DMF solution containing 500 mg of 2-butyl-4-ethynyl-2-methyl-2H-benzo[e][1,3]thiazine obtained by the method of step 3 of Reference Example 14, 692 mg of 1-amino-2,3-dimethyl-pyridinium 2,4-dinitro-phenolate, and 425 mg of potassium carbonate was stirred at room temperature for 3 days. The reaction mixture was phase-separated by the addition of water and ethyl acetate. The obtained organic layer was dried over sodium sulfate and concentrated under reduced pressure. The obtained residue was purified by silica gel column chromatography. The title compound of No. 233 was obtained as 200 mg of a brown solid, and the title compound of No. 234 was obtained as 204 mg of a brown gum-like product.

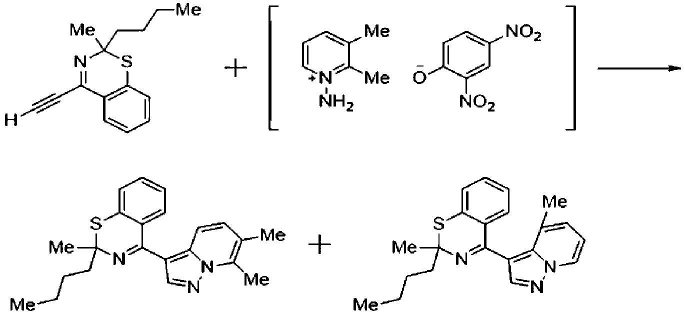

### <Synthesis Example 27>

### Synthesis of 2-butyl-4-(6,7-dimethylpyrazolo[1,5-a]pyridin-3-yl)-2-methyl-2H-benzo[e][1,3]thiazine 1,1-dioxide (compound No. 237)

3 ml of a dichloromethane solution containing 100 mg of 2-butyl-4-(6,7-dimethylpyrazolo[1,5-a]pyridin-3-yl)-2-methyl-2H-benzo[e][1,3]oxazine obtained by the method of Synthesis Example 26 and 142 mg of m-chloroperbenzoic acid was stirred at room temperature for 1 hour. The obtained reaction mixture was phase-separated by the addition of a 1% aqueous sodium thiosulfate solution and ethyl acetate. The obtained organic layer was washed with a saturated aqueous solution of sodium bicarbonate and dried over sodium sulfate. The solvent was distilled off under reduced pressure, and the obtained residue was then purified by silica gel column chromatography. The title compound was obtained as 50.0 mg of a white solid.

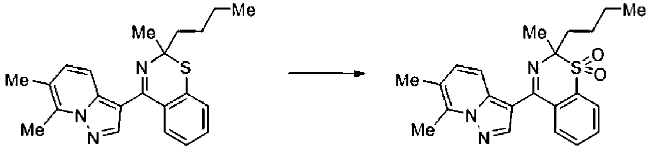

### <Reference Example 15>

### Synthesis of 2-(methoxymethyl)-2,7-dimethyl-2,3-dihydro-4H-benzo[e][1,3]oxazin-4-one

2.50 ml of a solution containing 500 mg of 2-hydroxy-4-methylbenzamide and 83.1 mg of pyridinium p-toluenesulfonate in 1-methoxy-2-propanone was stirred at 120°C for 3.5 hours. The reaction mixture was allowed to cool to room temperature and then phase-separated by the addition of water and ethyl acetate. The obtained organic layer was washed with saturated saline and dried over sodium sulfate. The solvent was distilled off under reduced pressure, and the obtained residue was purified by silica gel column chromatography. The title compound was obtained as 440 mg of a yellow solid.

¹H-NMR (CDCl₃) δ: 7.80 (1H, d, J = 8.0 Hz), 6.90-6.87 (1H, brm), 6.74 (1H, s), 6.32 (1H, brs), 3.55 (2H, dd, J = 24.1, 9.5 Hz), 3.40 (3H, s), 2.36 (3H, s), 1.62 (3H, s).

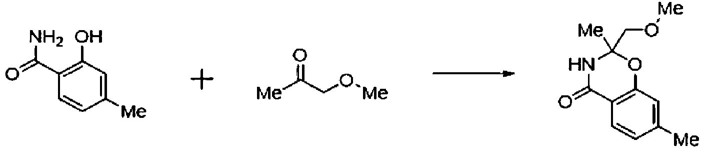

### <Reference Example 16>

### Synthesis of 4-chloro-2-(methoxymethyl)-2,7-dimethyl-2H-benzo[e][1,3]oxazine

276 µl of oxalyl chloride was added to 3.4 ml of a dichloromethane solution containing 408 µl of dimethylformamide, and the mixture was stirred at room temperature for 15 minutes. Subsequently, 224 mg of 2-(methoxymethyl)-2,7-dimethyl-2,3-dihydro-4H-benzo[e][1,3]oxazin-4-one obtained by the method of Reference Example 15 was added thereto, and the mixture was further stirred at room temperature for 1 hour. The solvent was distilled off from the obtained reaction mixture, and the obtained residue was then purified by silica gel column chromatography. The title compound was obtained as 199 mg of a yellow solid.

¹H-NMR (CDCl₃) δ: 7.44 (1H, d, J = 8.1 Hz), 6.80-6.76 (1H, m), 6.69 (1H, s), 3.64 (2H, dd, J = 24.5, 10.4 Hz), 3.45 (3H, s), 2.33 (3H, s), 1.57 (3H, s).

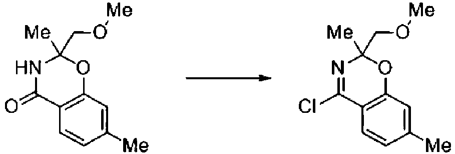

### <Reference Example 17 - Step 1>

### Synthesis of 2-(methoxymethyl)-2,7-dimethyl-4-((trimethylsilyl)ethynyl)-2H-benzo[e][1,3]oxazine

2 ml of a solution containing 199 mg of 4-chloro-2-(methoxymethyl)-2,7-dimethyl-2H-benzo[e][1,3]oxazine obtained by the method of Reference Example 16, 172 µl of trimethylsilylacetylene, 29.1 mg of bis(triphenylphosphine)palladium(II) dichloride, and 15.8 mg of copper iodide in triethylamine was stirred at 50°C for 15 minutes. The reaction mixture was phase-separated by the addition of a saturated aqueous solution of ammonium chloride and ethyl acetate, and the organic layer was washed with saturated saline. The obtained organic layer was dried over sodium sulfate, and the solvent was distilled off under reduced pressure. The obtained residue was purified by silica gel column chromatography. The title compound was obtained as 175 mg of a yellow gum-like product.

¹H-NMR (CDCl₃) δ: 7.42 (1H, d, J = 7.8 Hz), 6.78-6.74 (1H, brm), 6.65 (1H, s), 3.67 (1H, d, J = 10.0 Hz), 3.61 (1H, d, J = 10.0 Hz), 3.44 (3H, s), 2.32 (3H, s), 1.54 (3H, s), 0.28 (9H, s).

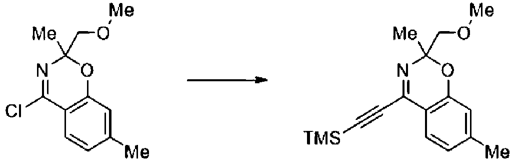

### <Reference Example 17 - Step 2>

### Synthesis of 4-ethynyl-2-(methoxymethyl)-2,7-dimethyl-2H-benzo[e][1,3]oxazine

2 ml of a solution containing 175 mg of 2-(methoxymethyl)-2,7-dimethyl-4-((trimethylsilyl)ethynyl)-2H-benzo[e][1,3]oxazine obtained by the method of step 1 of Reference Example 17 and 88.2 mg of potassium carbonate in methanol was stirred at room temperature for 30 minutes. The reaction mixture was phase-separated by the addition of water and ethyl acetate, and the obtained organic layer was dried over sodium sulfate. The solvent was distilled off under reduced pressure, and the obtained residue was purified by silica gel column chromatography. The title compound was obtained as 117 mg of a yellow gum-like product.

¹H-NMR (CDCl₃) δ: 7.45 (1H, d, J = 7.6 Hz), 6.77-6.75 (1H, m), 6.67 (1H, s), 3.68 (1H, d, J = 10.3 Hz), 3.62 (1H, d, J = 10.3 Hz), 3.45 (3H, s), 3.23 (1H, s), 2.32 (3H, s), 1.55 (3H, s).

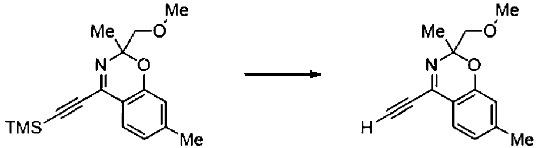

### <Synthesis Example 28>

### Synthesis of 4-(6,7-dimethylpyrazolo[1,5-a]pyridin-3-yl)-2-(methoxymethyl)-2,7-dimethyl-2H-benzo[e][1,3]oxazine (compound No. 212)

1.2 ml of a DMF solution containing 117 mg of 24-ethynyl-2-(methoxymethyl)-2,7-dimethyl-2H-benzo[e][1,3]oxazine obtained by the method of step 2 of Reference Example 17, 156 mg of 1-amino-2,3-dimethyl-pyridinium 2,4-dinitro-phenolate, and 211 mg of potassium carbonate was stirred at room temperature for 15 hours. The reaction mixture was phase-separated by the addition of water and ethyl acetate, and the obtained organic layer was dried over sodium sulfate. The solvent was distilled off under reduced pressure, and the obtained residue was purified by silica gel column chromatography. The title compound was obtained as 50 mg of a yellow gum-like product.

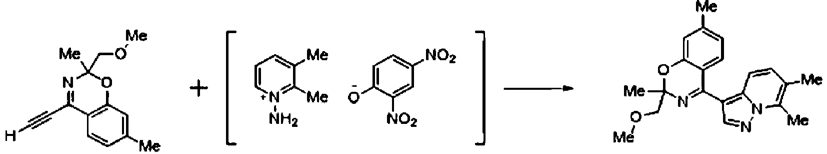

### <Synthesis Example 29>

### Synthesis of 2,2-dimethyl-4-(6-methylpyrazolo[1,5-a]pyrimidin-3-yl)-2H-benzo[e][1,3]oxazine (compound No. 248)

4 ml of a solution containing 170 mg of 4-chloro-2,2-dimethyl-2H-benzo[e][1,3]oxazine, 58 mg of 6-methylpyrazolo[1,5-a]pyrimidine, 20 mg of palladium acetate, 360 mg of potassium carbonate, and 50 mg of tri-t-butylphosphine tetrafluoroborate in toluene was stirred at 110°C for 2 hours. The reaction mixture was phase-separated by the addition of water and ethyl acetate, and the organic layer was dried over sodium sulfate. The solvent was distilled off under reduced pressure, and the obtained residue was purified by silica gel column chromatography. The title compound was obtained as 18 mg of a yellow solid.

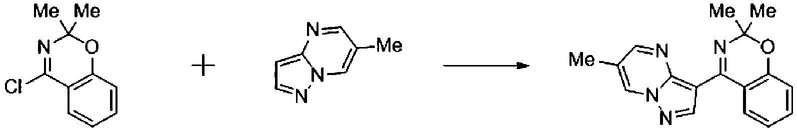

### <Reference Example 18 - Step 1>

### Synthesis of 8-fluoro-2,2-dimethyl-4-((trimethylsilyl)ethynyl)-2H-benzo[e][1,3]thiazine

6 ml of a solution containing 600 mg of 4-chloro-8-fluoro-2,2-dimethyl-2H-benzo[e][1,3]thiazine, 590 µl of trimethylsilylacetylene, 199 mg of bis(triphenylphosphine)palladium(II) dichloride, and 108 mg of copper iodide in triethylamine was stirred at 50°C for 2 hours. The reaction mixture was allowed to cool to room temperature and then phase-separated by the addition of a saturated aqueous solution of ammonium chloride and ethyl acetate, and the organic layer was washed with saturated saline. The obtained organic layer was dried over sodium sulfate, and the solvent was distilled off under reduced pressure. The obtained residue was then purified by silica gel column chromatography, and the title compound was obtained as 665 mg of a brown solid.

¹H-NMR (CDCl₃) δ: 7.76-7.72 (1H,m), 7.21-7.12 (2H,m), 1.63 (6H,s), 0.29 (9H,s).

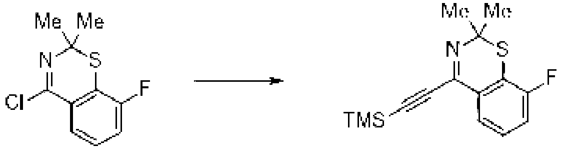

### <Synthesis Example 18 - Step 2>

### Synthesis of 4-ethynyl-8-fluoro-2,2-dimethyl-2H-benzo[e][1,3]thiazine

6.5 ml of a solution containing 656 mg of 8-fluoro-2,2-dimethyl-4-((trimethylsilyl)ethynyl)-2H-benzo[e][1,3]thiazine obtained by the method of step 1 of Synthesis Example 18 and 466 mg of potassium carbonate in methanol was stirred at room temperature for one hour 25 minutes. The reaction mixture was phase-separated by the addition of water and ethyl acetate, and the obtained organic layer was dried over sodium sulfate. The solvent was distilled off under reduced pressure, and the obtained residue was purified by silica gel column chromatography. The title compound was obtained as 344 mg of a brown oil.

¹H-NMR (CDCl₃) δ: 7.75 (1H, dd, J=7.3, 1.5Hz), 7.21-7.12 (2H,m), 3.21 (1H,s), 1.64 (6H,s).

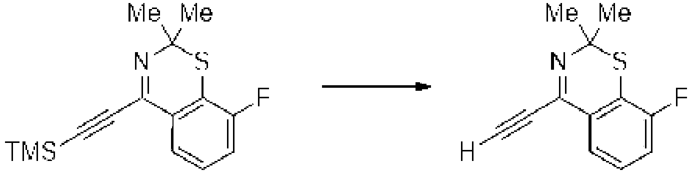

### <Synthesis Example 30>

### Synthesis of 4-(6,7-dimethylpyrazolo[1,5-a]pyridin-3-yl)-8-fluoro-2,2-dimethyl-2H-benzo[e][1,3]thiazine (compound No. 206)

4 ml of a solution containing 318 mg of 4-ethynyl-8-fluoro-2,2-dimethyl-2H-benzo[e][1,3]thiazine obtained by the method of step 2 of Reference Example 18 and 419 mg of 1-amino-2,3-dimethyl-pyridinium 2,4-dinitro-phenolate in methanol was stirred at room temperature for one hour. The reaction mixture was allowed to cool to room temperature and then phase-separated by the addition of water and ethyl acetate. The obtained organic layer was dried over sodium sulfate and the solvent was distilled off under reduced pressure. The obtained residue was purified by silica gel column chromatography. The title compound was obtained as 16.5 mg of a brown oil.

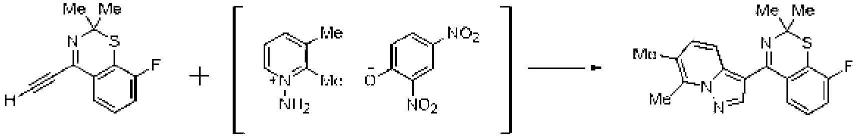

### <Synthesis Example 31>

### Synthesis of 8-fluoro-2,2-dimethyl-4-(7-methylpyrazolo[1,5-a]pyridin-3-yl)-2H-benzo[e][1,3]thiazine (compound No. 325)

0.6 ml of a solution containing 62 mg of 4-ethynyl-8-fluoro-2,2-dimethyl-2H-benzo[e][1,3]thiazine obtained by the method of Reference Example 18, 73 mg of 1-amino-2-methylpyridinium iodide, and 117 mg of potassium carbonate in dimethylformamide was stirred at room temperature for 22 hours. The reaction mixture was phase-separated by the addition of water and ethyl acetate. The obtained organic layer was dried over sodium sulfate and the solvent was distilled off under reduced pressure. The obtained residue was purified by silica gel column chromatography. The title compound was obtained as 23.8 mg of a yellow oil.

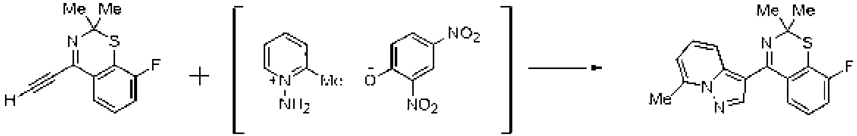

### <Reference Example 19>

### Synthesis of 2,2-dimethyl-4-((trimethylsilyl)ethynyl)-2H-benzo[e][1,3]thiazine

10 ml of a solution containing 1.09 g of 4-chloro-2,2-dimethyl-2H-benzo[e][1,3]thiazine, 837 µl of trimethylsilylacetylene, 363 mg of bis(triphenylphosphine)palladium(II) dichloride, and 197 mg of copper iodide in triethylamine was stirred at room temperature for one hour. The reaction mixture was phase-separated by the addition of water and ethyl acetate. The obtained organic layer was dried over sodium sulfate, and the solvent was distilled off under reduced pressure. The obtained residue was purified by silica gel column chromatography. The title compound was obtained as 800 mg of a brown oil.

1H-NMR (CDCl3) δ: 7.90 (1H, dd, J = 7.8, 1.3 Hz), 7.35-7.31 (1H, m), 7.23-7.19 (2H, m), 1.61 (6H, s), 0.28 (9H, s).

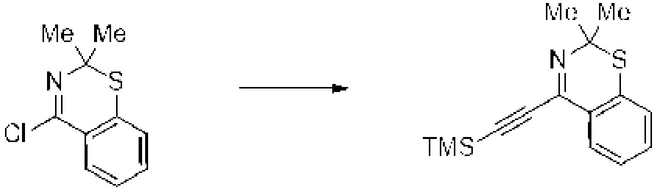

### <Reference Example 20>

### Synthesis of 4-ethynyl-2,2-dimethyl-2H-benzo[e][1,3]thiazine

8 ml of a solution containing 800 mg of 2,2-dimethyl-4-((trimethylsilyl)ethynyl)-2H-benzo[e][1,3]thiazine obtained by the method of Reference Example 19 and 607 mg of potassium carbonate in methanol was stirred at room temperature for one hour. The solvent was distilled off under reduced pressure. The obtained residue was purified by silica gel column chromatography. The title compound was obtained as 200 mg of a brown oil.

1H-NMR (CDCl₃) δ: 7.91 (1H, ddd, J = 7.8, 1.5, 0.3 Hz), 7.36-7.32 (1H, m), 7.24-7.19 (2H, m), 3.18 (1H, s), 1.62 (6H, s).

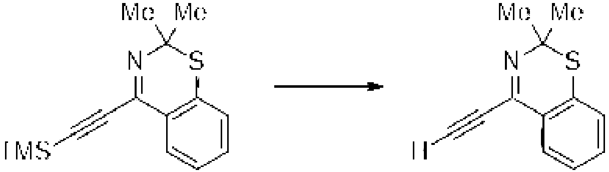

### <Synthesis Example 32>

### Synthesis of 4-(6,7-dimethylpyrazolo[1,5-a]pyridin-3-yl)-2,2-dimethyl-2H-benzo[e][1,3]thiazine (compound No. 207)

3 ml of a solution containing 200 mg of 4-ethynyl-2,2-dimethyl-2H-benzo[e][1,3]thiazine obtained by the method of Reference Example 20, 33 mg of 1-amino-2,3-dimethyl-pyridinium 2,4-dinitro-phenolate and 206 mg of potassium carbonate in dimethylformamide was stirred at room temperature for 17 hours. The reaction mixture was phase-separated by the addition of water and ethyl acetate. The obtained organic layer was dried over sodium sulfate, and the solvent was distilled off under reduced pressure. The obtained residue was purified by silica gel column chromatography. The title compound was obtained as 46.2 mg of a brown oil.

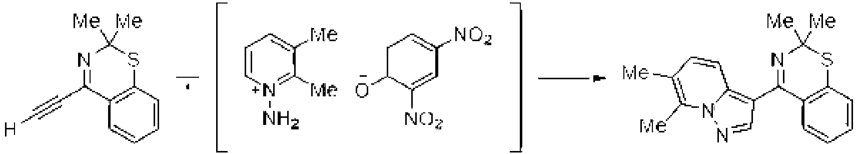

### <Reference Example 21>

### Synthesis of 1-(4-fluoro-2-hydroxy-3-methylphenyl)ethan-1-one

10.6 g of aluminum chloride was added to 21 ml of a solution containing 13.76 g of 3-fluoro-2-methylphenyl acetate in 1,2-dichloroethane while cooling with ice. The reaction mixture was stirred under heat reflux for 2 hours and 40 minutes. The reaction mixture was allowed to cool to room temperature and the solvent was distilled off under reduced pressure. 60 ml of a solution of 2N hydrochloric acid was added to the obtained residue, and the reaction mixture was phase-separated by the addition of dichloromethane. The obtained organic layer was dried over sodium sulfate, and the solvent was distilled off under reduced pressure. The obtained residue was purified by silica gel column chromatography. The title compound was obtained as 9.92 mg of a pale yellow solid.

¹H-NMR (CDCl₃) δ: 12.90 (1H, t, *J* = 2.5 Hz ), 7.59 (1H, dd, *J* = 9.0, 6.3 Hz,), 6.60 (1H, t, *J* = 9.0 Hz,), 2.60 (3H, s ), 2.15 (3H, d, *J* = 1.9 Hz ).

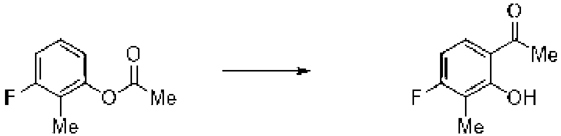

### <Reference Example 22>

### Synthesis of 7-fluoro-8-methyl-4H-chromen-4-one

15 ml of a solution containing 5.00 g of 1-(4-fluoro-2-hydroxy-3-methylphenyl)ethan-1-one obtained by the method of Reference Example 19 in N,N-dimethylformamide dimethylacetal was stirred under heat reflux for one hour. The solvent was distilled off under reduced pressure. 50 ml of dichloromethane and 10 ml of concentrated hydrochloric acid were added to the residue, and the reaction mixture was stirred under heat reflux for one hour. The reaction mixture was allowed to cool to room temperature, added to a saturated aqueous solution of sodium bicarbonate and then phase-separated with ethyl acetate. The obtained organic layer was dried over sodium sulfate, and the solvent was distilled off under reduced pressure. The obtained residue was washed by diisopropyl ether. The title compound was obtained as 3.67 mg of a pale yellow solid.

1H-NMR (CDCl₃) δ: 8.09-8.05 (1H, m, ), 7.90 (1H, d, J = 6.1 Hz, ), 7.12 (1H, t, J = 8.8 Hz, ), 6.34 (1H, d, J = 6.1 Hz, ), 2.38 (3H, d, J = 1.9 Hz, ).

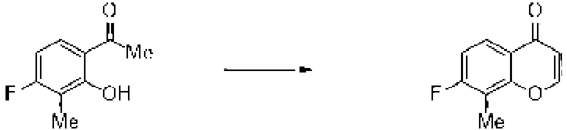

### <Reference Example 23>

### Synthesis of 6,7-dimethylpyrazolo[1,5-a]pyridin-3-yl)(4-fluoro-2-hydroxy-3-methylphenyl)methanone

8 ml of a solution containing 800 g of 7-fluoro-8-methyl-4H-chromen-4-one obtained by the method of Reference Example 21, 1375 mg of 1-amino-2,3-dimethyl-pyridinium 2,4-dinitro-phenolate, , and 1.85 g of potassium carbonate in dimethylformamide was stirred at 90°C for 3 hours. Water was added to the reaction mixture and the precipitated solid was filtered off. The obtained solid was washed in suspension with ethyl acetate. The title compound was obtained as 550.1 mg of a brown solid.

1H-NMR (CDCl₃) δ: 12.84 (1H, d, J = 1.7 Hz, ), 8.33 (1H, s, ), 8.16 (1H, d, J = 8.8 Hz, ), 7.78 (1H, dd, J = 9.3, 6.3 Hz, ), 7.38 (1H, d, J = 9.0 Hz, ), 6.66 (1H, t, J = 8.9 Hz, ), 2.81 (3H, s, ), 2.45 (3H, s, ), 2.22 (3H, d, J = 2.0 Hz, ).

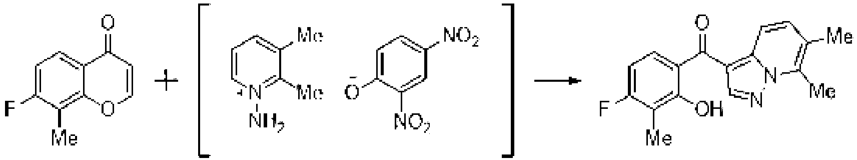

### <Synthesis Example 33>

### Synthesis of 4-(6,7-dimethylpyrazolo[1,5-a]pyridin-3-yl)-7-fluoro-2,2,8-trimethyl-2H-benzo[e][1,3]oxazine (compound No. 245)

7.5 ml of a 7M ammonia methanol solution containing 150 mg of (6,7-dimethylpyrazolo[1,5-a]pyridin-3-yl)(4-fluoro-2-hydroxy-3-methylphenyl)methanone obtained by the method of Reference Example 23 was sealed in a tube and stirred at 80°C for 3 hours. The solvent was distilled off under reduced pressure, and then 1.5 ml of dimethylformamide, 0.75 ml of 2- methoxypropene, and 126.4 mg of pyridinium p-toluenesulfonate were added to the reaction mixture, and the reaction mixture was stirred at 90°C for one hour. The reaction mixture was allowed to cool to room temperature, added to a saturated aqueous solution of sodium bicarbonate and then phase-separated with ethyl acetate. The obtained organic layer was dried over sodium sulfate, and the solvent was distilled off under reduced pressure. The obtained residue was purified by silica gel column chromatography. The title compound was obtained as 27.5 mg of a yellow solid.

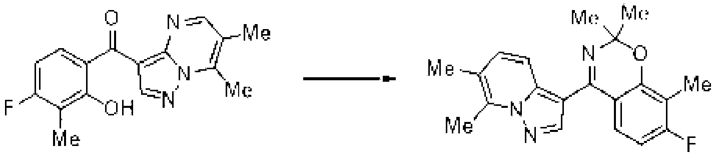

### <Reference Example 24>

### Synthesis of 6,7-dimethylpyrazolo[1,5-a]pyrimidine

100 ml of a solution containing 9.81 g of tert-butyl 5-amino-1H-pyrazole-1-carboxylate and 5.78 g of sodium 2-methyl-3-oxobut-1-en-1-olate in acetic acid was stirred at room temperature for one hour 30 minutes. Then, the solution was heated to 80°C and stirred for one hour. The solvent was distilled off under reduced pressure, and the obtained solid was added into ice water and potassium carbonate was added until it stopped bubbling. The organic layer is extracted with ethyl acetate, and the obtained organic layer was dried over sodium sulfate. After the solvent was distilled off under reduced pressure, the obtained solid was washed in suspension with diisopropyl ether. The title compound was obtained as 3.61 g of a white solid.

1H-NMR (CDCl₃) δ: 8.31 (1H, s), 8.08 (1H, d, J = 2.3 Hz), 6.66 (1H, d, J = 2.3 Hz), 2.78 (3H, s), 2.38 (3H, s).

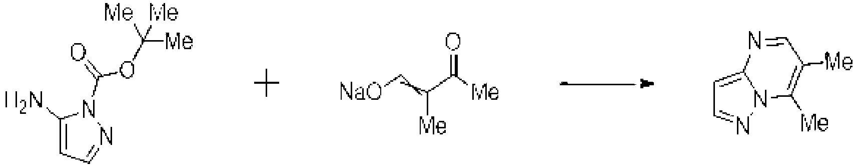

### <Reference Example 25>

### Synthesis of 2-butyl-8-fluoro-2-methyl-2,3-dihydro-4H-benzo[e][1,3]oxazin-4-one

200 ml of a solution containing 20 g of 3-fluoro-2-hydroxybenzamide, 24.5 g of p-toluenesulfonic acid monohydrate, and 60 ml of hexane-2-one in toluene was stirred under heat reflux for one hour. The reaction mixture was allowed to cool to room temperature, added to a saturated aqueous solution of sodium bicarbonate and then extracted with ethyl acetate. The obtained organic layer was washed with saturated salt solution, dried over sodium sulfate, and the solvent was distilled off under reduced pressure. The obtained residue was filtered through diisopropyl ether to remove the solid components. From the obtained filtrate, the solvent was removed under reduced pressure. The title compound was obtained as 3.72 g of a yellow solid.

1H-NMR (CDCl₃) δ: 7.70 (1H, dt, J = 7.8, 1.3 Hz), 7.28-7.24 (3H, m), 7.00 (1H, td, J = 6.7, 4.2 Hz), 6.31 (1H, br s), 1.93-1.90 (2H, m), 1.64 (3H, s), 1.50-1.43 (2H, m), 1.38-1.30 (2H, m), 0.91 (3H, t, J = 7.3 Hz).

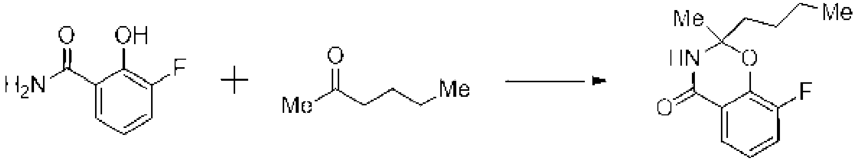

### <Synthesis Example 34 - Step 1>

### Synthesis of 4-bromo-2-butyl-8-fluoro-2-methyl-2H-benzo[e][1,3]oxazine

3 ml of a solution containing 0.31 g of 2-butyl-8-fluoro-2-methyl-2,3-dihydro-4H-benzo[e][1,3]oxazin-4-one obtained by the method of Reference Example 25, 0.54 g of phosphorus oxybromide in 1,2-dichloroethane was stirred at 50°C for 35 minutes. The reaction mixture was allowed to cool to room temperature, and then the reaction mixture was added into ice water. The organic layer is extracted with ethyl acetate. The obtained organic layer was washed once each with water and saturated saline, and dried over sodium sulfate. The solvent was distilled off under reduced pressure. The obtained residue was purified by silica gel column chromatography. The title compound was obtained as 0.21 g of a colorless oil.

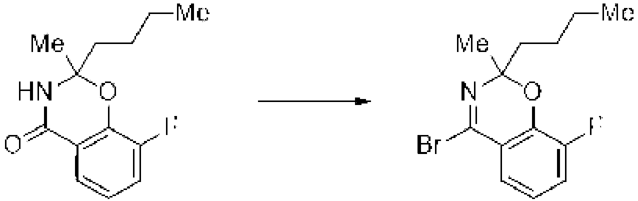

### <Synthesis Example 34 - Step 2>

### Synthesis of 2-butyl-4-(6,7-dimethylpyrazolo[1,5-a]pyrimidin-3-yl)-8-fluoro-2-methyl-2H-benzo[e][1,3]oxazine (compound No. 400)

2.1 ml of a 1,4-dioxane solution containing 0.21 g of 4-bromo-2-butyl-8-fluoro-2-methyl-2H-benzo[e][1,3]oxazine obtained by the method of step 1 of Synthesis Example 34, 0.20 g of 6,7-dimethylpyrazolo[1,5-a]pyrimidine obtained by the method of Reference Example 24, 0.29 g of potassium carbonate and 79.5 mg of palladium acetate was stirred at 90°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 42.2 mg of a yellow solid.

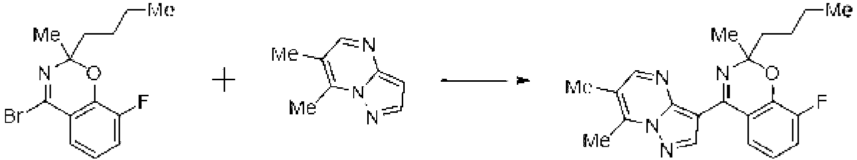

### <Reference Example 26>

### Synthesis of 3,4-difluoro-2-hydroxybenzamide

7.04 g of 1,1'-carbonyldiimidazole was added to 18.9 ml of an N,N-dimethylformamide solution containing 3.78 g of 3,4-difluoro-2-hydroxybenzoic acid at room temperature. The reaction mixture was stirred at room temperature for one hour, and then 7.56 ml of 28% aqueous ammonia solution was dropped down under ice-cold conditions. The reaction mixture was heated to room temperature and stirred for 3 hours, and then water and acetic acid were added. The precipitated solid was filtered and washed with water and hexane. The title compound was obtained as 2.63 g of a white solid.

1H-NMR (DMSO-d₆) δ: 13.71 (1H, br s), 8.67 (1H, s), 8.14 (1H, s), 7.73 (1H, ddd, J = 9.1, 5.9, 2.3 Hz), 6.92 (1H, td, J = 8.8, 7.2 Hz).

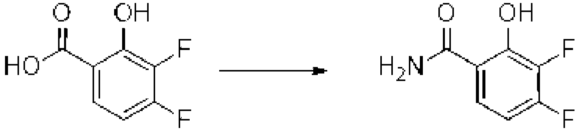

### <Reference Example 27>

### Synthesis of 7,8-difluoro-2-methyl-2-propyl-2,3-dihydro-4H-benzo[e][1,3]oxazin-4-one

19 ml of a toluene solution containing 3.81 g of 3,4-difluoro-2-hydroxybenzamide obtained by the method of Synthesis Example 26, 4.19 g of p-toluenesulfonic acid monohydrate, and 11.4 ml of pentan-2-one was stirred under heat reflux for 4 hours. The reaction mixture was allowed to cool to room temperature, added to a saturated aqueous solution of sodium bicarbonate and then extracted with ethyl acetate. The obtained organic layer was distilled off under reduced pressure. The obtained residue was purified by silica gel column chromatography. The title compound was obtained as 864.1 mg of a yellow oil.

1H-NMR (CDCl₃) δ: 7.72-7.66 (1H, m), 6.87 (1H, td, J = 8.4, 6.5 Hz), 6.61 (1H, s), 1.92-1.89 (2H, m), 1.65 (3H, s), 1.56-1.48 (2H, m), 0.96 (3H, t, J = 7.4 Hz).

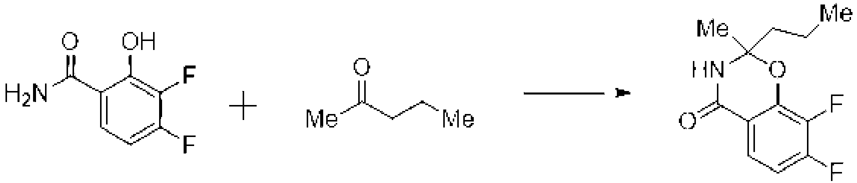

### <Synthesis Example 35 - Step 1>

### Synthesis of 4-bromo-7,8-difluoro-2-methyl-2-propyl-2H-benzo[e][1,3]oxazine

5 ml of a solution containing 0.50 g of 7,8-difluoro-2-methyl-2-propyl-2,3-dihydro-4H-benzo[e][1,3]oxazin-4-one obtained by the method of Reference Example 27 and 0.86 g of phosphorus oxybromide in 1,2-dichloroethane was stirred at 50°C for 25 minutes. The reaction mixture was allowed to cool to room temperature, and then the reaction mixture was added into ice water. The organic layer is extracted with ethyl acetate. The obtained organic layer was washed once each with water and saturated saline, and dried over sodium sulfate. The solvent was distilled off under reduced pressure. The obtained residue was purified by silica gel column chromatography. The title compound was obtained as 121.4 mg of a colorless oil.

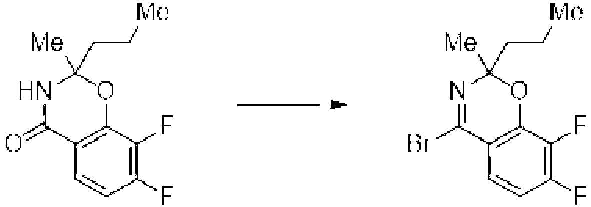

### <Synthesis Example 35 - Step 2>

### Synthesis of 4-(6,7-dimethylpyrazolo[1,5-a]pyrimidin-3-yl)-7,8-difluoro-2-methyl-2-propyl-2H-benzo[e][1,3]oxazine (compound No. 543)

1.2 ml of a 1,4-dioxane solution containing 121.4 mg of 4-bromo-7,8-difluoro-2-methyl-2-propyl-2H-enzo[e][1,3]oxazine obtained by the method of step 1 of Synthesis Example 35, 0.12 g of 6,7-dimethylpyrazolo[1,5-a]pyrimidine obtained by the method of Reference Example 24, 0.16 g of potassium carbonate and 45.1 mg of palladium acetate was stirred at 90°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 11.6 mg of a yellow oil.

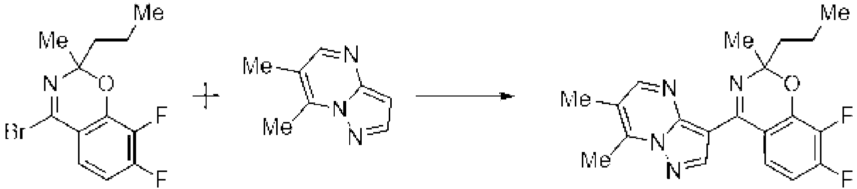

### <Reference Example 28>

### Synthesis of 7-(difluoromethyl)-6-methylpyrazolo[1,5-a]pyrimidine

15 ml of a solution containing 3.13 g of tert-butyl 5-amino-1H-pyrazole-1-carboxylate, 6.16 g of (E)-4-ethoxy-1,1-difluoro-3-methyl-but-3-en-2-one, and 6.8 ml of 20% sodium ethoxide ethanol solution in toluene was stirred at room temperature for 30 minutes, and then stirred under heat reflux for one hour. The reaction mixture was allowed to cool to room temperature. 15 ml of acetic acid was added into the reaction mixture, and then the reaction mixture was stirred at 90°C for one hour. The solvent was distilled off under reduced pressure, and the obtained solid was added into ice water and potassium carbonate was added until it stopped bubbling. The organic layer is extracted with ethyl acetate, and the obtained organic layer was dried over sodium sulfate. After the solvent was distilled off under reduced pressure, the obtained solid was washed in suspension with diisopropyl ether. The title compound was obtained as 0.86 g of a pale yellow solid.

1H-NMR (CDCl₃) δ: 8.41 (1H, s), 8.09 (1H, d, J = 2.4 Hz), 7.80 (1H, t, J = 52.7 Hz), 6.76 (1H, d, J = 2.4 Hz), 2.56 (3H, t, J = 3.2 Hz)

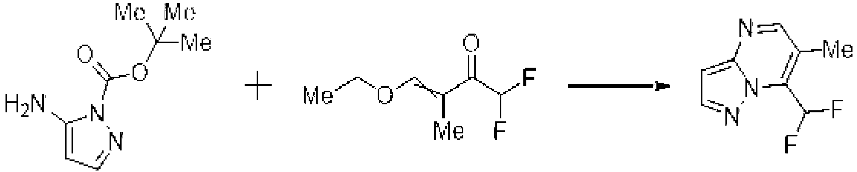

### <Synthesis Example 36 - Step 1>

### Synthesis of 4-bromo-8-fluoro-2-methyl-2-propyl-2H-benzo[e][1,3]oxazine

5 ml of a solution containing 0.50 g of 8-fluoro-2-methyl-2-propyl-2,3-dihydro-4H-benzo[e][1,3]oxazin-4-one and 0.96 g of phosphorus oxybromide in 1,2-dichloroethane was stirred at 50°C for 40 minutes. The reaction mixture was allowed to cool to room temperature, and then the reaction mixture was added into ice water. The organic layer is extracted with ethyl acetate. The obtained organic layer was washed once each with water and saturated saline, and dried over sodium sulfate. The solvent was distilled off under reduced pressure. The obtained residue was purified by silica gel column chromatography. The title compound was obtained as 0.23 g of a colorless oil.

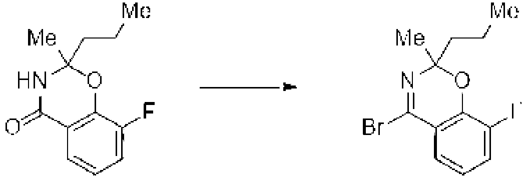

### <Synthesis Example 36 - Step 2>

### Synthesis of 4-(7-(difluoromethyl)-6-methylpyrazolo[1,5-a]pyrimidin-3-yl)-8-fluoro-2-methyl-2-propyl-2H-benzo[e][1,3]oxazine (compound No. 569)

1.3 ml of a 1,4-dioxane solution containing 132.9 mg of 4-bromo-8-fluoro-2-methyl-2-propyl-2H-benzo[e][1,3]oxazine obtained by the method of step 1 of Synthesis Example 36, 0.17 g of 7-(difluoromethyl)-6-methylpyrazolo[1,5-a]pyrimidine obtained by the method of Reference Example 28, 0.19 g of potassium carbonate and 51.1 mg of palladium acetate was stirred at 90°C for 2 hours 15 minutes. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 14.8 mg of a brown oil.

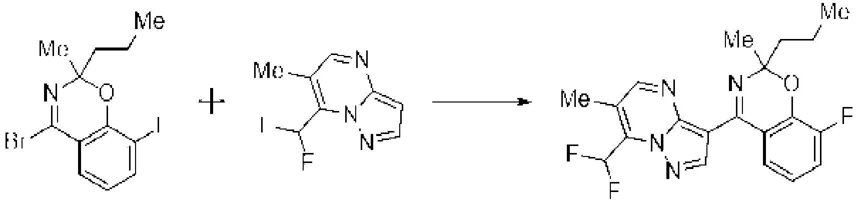

### <Reference Example 29>

### Synthesis of 6-methyl-7-(trifluoromethyl)pyrazolo[1,5-a]pyrimidine

25 ml of a solution containing 4.98 g of tert-butyl 5-amino-1H-pyrazole-1-carboxylate, 6.88 g of 4-ethoxy-1,1,1-trifluoro-3-methylbut-3-ene-2-one, and 10 ml of 20% sodium ethoxide ethanol solution in toluene was stirred at room temperature for 30 minutes, and then stirred under heat reflux for one hour. The reaction mixture was allowed to cool to room temperature. 25 ml of acetic acid was added into the reaction mixture, and then the reaction mixture was stirred at 90°C for 35 minutes. The solvent was distilled off under reduced pressure, and the obtained residue was added into ice water and potassium carbonate was added until it stopped bubbling. The organic layer is extracted with ethyl acetate, and the obtained organic layer was dried over sodium sulfate. After the solvent was distilled off under reduced pressure, the obtained solid was washed in suspension with diisopropyl ether. The title compound was obtained as 2.51 g of a white solid.

1H-NMR (CDCl₃) δ: 8.41 (1H, s), 8.19 (1H, d, J = 2.7 Hz), 6.82 (1H, d, J = 2.7 Hz), 2.58 (3H, q, J = 3.6 Hz).

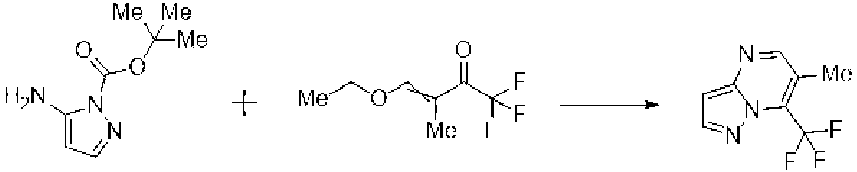

### <Synthesis Example 37>

### Synthesis of 2-butyl-8-fluoro-2-methyl-4-(6-methyl-7-(trifluoromethyl)pyrazolo[1,5-a]pyrimidin-3-yl)-2H-benzo[e][1,3]oxazine (compound No. 570)

1.6 ml of a N,N-dimethylacetamide solution containing 158.7 mg of 4-bromo-2-butyl-8-fluoro-2-methyl-2H-benzo[e][1,3]oxazine obtained by the method of step 1 of Synthesis Example 36, 0.20 g of 6-methyl-7-(trifluoromethyl)pyrazolo[1,5-a]pyrimidine obtained by the method of Reference Example 29, 0.16 g of potassium carbonate and 63.5 mg of palladium acetate was stirred at 90°C for 2 hours 25 minutes. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 39.7 mg of a brown oil.

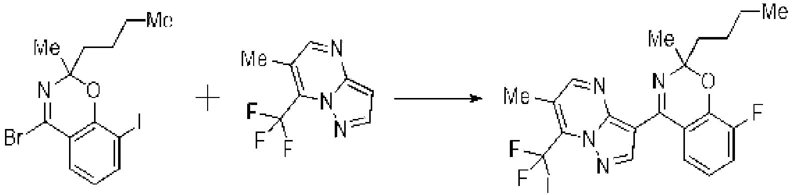

### <Reference Example 30>

### Synthesis of 6-chloro-7-methylpyrazolo[1,5-a]pyrimidine

25 ml of a toluene solution containing 11.65 g of 3-chloro-4,4-diethoxybutan-2-one and 25 ml of 35% hydrochloric acid was stirred at 60°C for 30 minutes. The reaction mixture was allowed to cool to room temperature. 5.00 g of tert-butyl 5-amino-1H-pyrazole-1-carboxylate was added into the reaction mixture, and then the reaction mixture was stirred at room temperature for 30 minutes, and then heated to 90°C and stirred for one hour. The solvent was distilled off under reduced pressure, and potassium carbonate was added to the obtained residue until it stopped bubbling. The organic layer is extracted with ethyl acetate, and the obtained organic layer was dried over sodium sulfate. After the solvent was distilled off under reduced pressure, the obtained solid was washed in suspension with diisopropyl ether. The title compound was obtained as 0.32 g of a yellow solid.

1H-NMR (CDCl₃) δ: 8.42 (1H, s), 8.14 (1H, d, J = 2.2 Hz), 6.74 (1H, d, J = 2.4 Hz), 2.91 (3H, s).

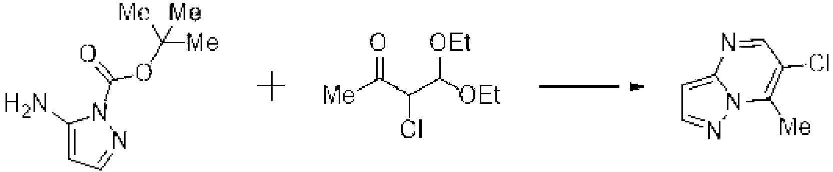

### <Synthesis Example 38>

### Synthesis of 2-butyl-4-(6-chloro-7-methylpyrazolo[1,5-a]pyrimidin-3-yl)-8-fluoro-2-methyl-2H-benzo[e][1,3]oxazine (compound No. 571)

1.6 ml of a 1,4-dioxane solution containing 159.5 mg of 4-bromo-2-butyl-8-fluoro-2-methyl-2H-benzo[e][1,3]oxazine obtained by the method of step 1 of Synthesis Example 36, 0.18 g of 6-chloro-7-methylpyrazolo[1,5-a]pyrimidine obtained by the method of Reference Example 30, 0.22 g of potassium carbonate and 60.7 mg of palladium acetate was stirred at 90°C for one hour 25 minutes. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 7.1 mg of a brown oil.

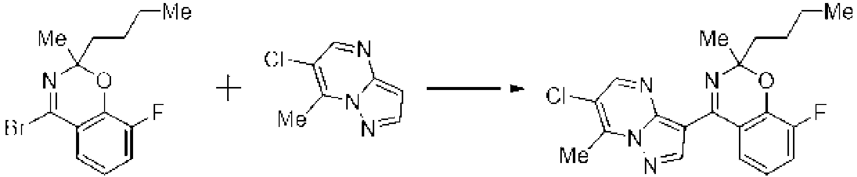

### <Reference Example 31>

### Synthesis of 8-fluoro-2-methyl-2-pentyl-2H-benzo[e][1,3]oxazin-4-ol

200 ml of a toluene solution containing 20 g of 3-fluoro-2-hydroxybenzamide, 25.5 g of p-toluenesulfonic acid monohydrate, and 60 ml of heptan-2-one was stirred under heat reflux for 40 minutes. The reaction mixture was allowed to cool to room temperature, added to a saturated aqueous solution of sodium bicarbonate to quench and then phase-separated with a water and ethyl acetate. The obtained organic layer was washed with saturated saline. The obtained organic layer was dried over sodium sulfate, and the solvent was distilled off under reduced pressure. The obtained residue was purified by silica gel column chromatography. The title compound was obtained as 2.96 g of a yellow oil.

1H-NMR (CDCl₃) δ: 7.71-7.71 (1H, m), 7.28-7.23 (1H, m), 6.99 (1H, td, J = 6.9, 4.5 Hz), 6.46(1H,brs), 1.91-1.89 (2H, m), 1.64(3H ,s), 1.59-1.58 (2H, m), 1.51-1.45 (2H, m), 1.34-1.25 (2H, m), 0.89 (3H, t, J = 6.9 Hz).

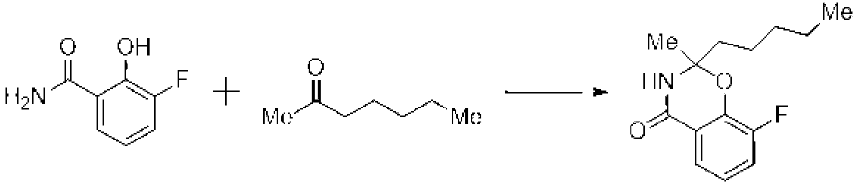

### <Reference Example 39 - Step 1>

### Synthesis of 4-bromo-8-fluoro-2-methyl-2-pentyl-2H-benzo[e][1,3]oxazine

6 ml of a solution containing 0.60 g of 8-fluoro-2-methyl-2-pentyl-2,3-dihydro-4H-benzo[e][1,3]oxazin-4-one and 1.04 g of phosphorus oxybromide in 1,2-dichloroethane was stirred at 50°C for 30 minutes. The reaction mixture was allowed to cool to room temperature, and then the reaction mixture was added into ice water. The organic layer is extracted with ethyl acetate. The obtained organic layer was washed once each with water and saturated saline, and dried over sodium sulfate. The solvent was distilled off under reduced pressure. The obtained residue was purified by silica gel column chromatography. The title compound was obtained as 0.47 g of a colorless oil.

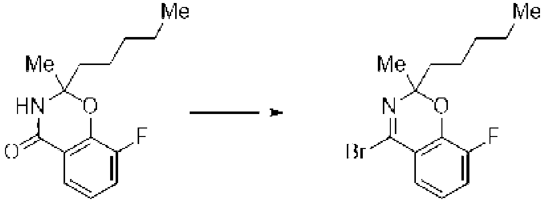

### <Synthesis Example 39 - Step 2>

### Synthesis of 8-fluoro-2-methyl-4-(6-methylpyrazolo[1,5-a]pyrimidin-3-yl)-2-pentyl-2H-benzo[e][1,3]oxazine (compound No. 576)

1.3 ml of a 1,4-dioxane solution containing 129.3 mg of 4-bromo-8-fluoro-2-methyl-2-pentyl-2H-benzo[e][1,3]oxazine obtained by the method of step 1 of Synthesis Example 39, 0.19 g of 6-methylpyrazolo[1,5-a]pyrimidine, 0.12 g of potassium carbonate and 52.5 mg of palladium acetate was stirred at 90°C for 4 hours and 30 minutes. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 28.1 mg of a brown oil.

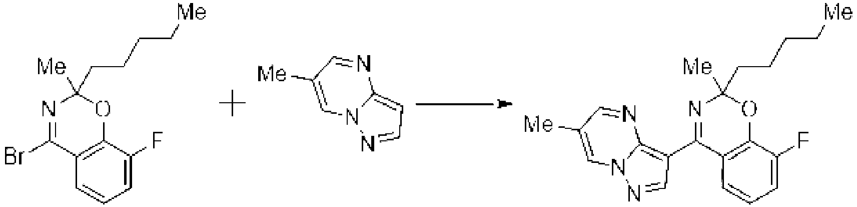

### <Reference Example 32>

### Synthesis of 8-chloro-2-methyl-2-propyl-2,3-dihydro-4H-benzo[e][1,3]oxazin-4-one

19 ml of a toluene solution containing 5.00 g of 3-chloro-2-hydroxybenzamide, 5.54 g of p-toluenesulfonic acid monohydrate, and 25 ml of pentan-2-one was subjected to an azeotrope dehydration reaction at 130°C for one hour with stirring in a reactor installed with a Dean Stark trap. The reaction mixture was allowed to cool to room temperature, added to an aqueous solution of potassium carbonate and then extracted with ethyl acetate. The obtained organic layer was washed with saturated saline and dried over sodium sulfate. The obtained organic layer was distilled off under reduced pressure. The obtained residue was purified by silica gel column chromatography. The title compound was obtained as 4.05 mg of a solid.

1H-NMR (CDCl₃) δ: 7.84 (1H, dd, J = 7.8, 1.7 Hz, ), 7.51 (1H, dd, J = 7.8, 1.7 Hz, ), 7.01 (1H, t, J = 7.8 Hz, ), 6.41 (1H, br s, ), 1.95-1.82 (2H, m, ), 1.64 (3H, s, ), 1.57-1.49 (2H, m, ), 0.95 (3H, t, J = 7.3 Hz, ).

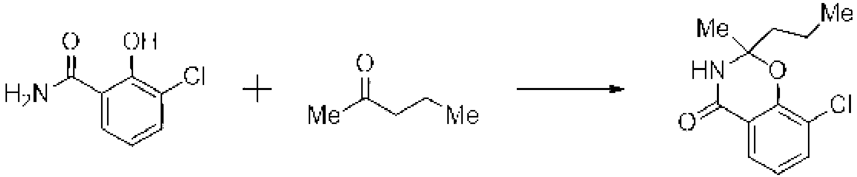

### <Synthesis Example 40 - Step 1>

### Synthesis of 4-bromo-8-chloro-2-methyl-2-propyl-2H-benzo[e][1,3]oxazine

6 ml of a solution containing 0.80 g of 8-chloro-2-methyl-2-propyl-2,3-dihydro-4H-benzo[e][1,3]oxazin-4-one obtained by the method of Reference Example 32 and 1.05 g of phosphorus oxybromide in 1,2-dichloroethane was stirred at 50°C for 15 minutes. The reaction mixture was allowed to cool to room temperature, and then the reaction mixture was added into ice water. The organic layer is extracted with ethyl acetate. The obtained organic layer was washed once each with water and saturated saline, and dried over sodium sulfate. The solvent was distilled off under reduced pressure. The obtained residue was purified by silica gel column chromatography. The title compound was obtained as 0.55 g of a colorless oil.

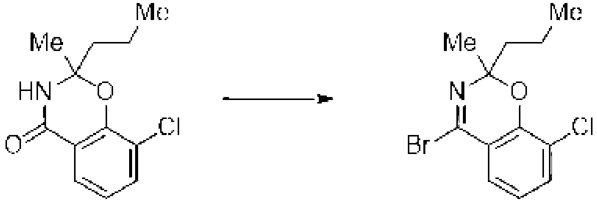

### <Synthesis Example 40 - Step 2>

### Synthesis of 8-chloro-4-(6,7-dimethylpyrazolo[1,5-a]pyrimidin-3-yl)-2-methyl-2-propyl-2H-benzo[e][1,3]oxazine (compound No. 760)

1.4 ml of a 1,4-dioxane solution containing 0.15 g of 4-bromo-8-chloro-2-methyl-2-propyl-2H-benzo[e][1,3]oxazine obtained by the method of step 1 of Synthesis Example 40, 67.9 mg of 6,7-dimethylpyrazolo[1,5-a]pyrimidine obtained by the method of Reference Example 24, 0.21 g of potassium carbonate and 55.9 mg of palladium acetate was stirred at 100°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 34.9 mg of a brown solid.

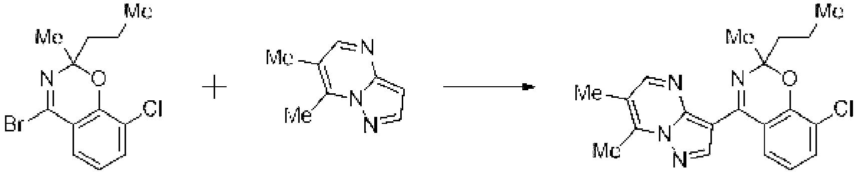

### <Reference Example 33>

### Synthesis of 2-butyl-8-chloro-2-methyl-2,3-dihydro-4H-benzo[e][1,3]oxazin-4-one

Mixed solution of 15 ml of a 2-hexanone and 15 ml of a toluene containing 3.00 g of 3-chloro-2-hydroxybenzamide and 3.3 g of p-toluenesulfonic acid monohydrate was subjected to an azeotrope dehydration reaction at 90°C for one hour with stirring in a reactor installed with a Dean Stark trap. The reaction mixture was allowed to cool to room temperature, phase-separated with potassium carbonate solution and ethyl acetate. The obtained organic layer was dried over sodium sulfate, and the solvent was distilled off under reduced pressure. The obtained residue was filtered through diisopropyl ether. The filtrate was purified by silica gel column chromatography. The title compound was obtained as 2.5 g of a yellow amorphous product.

1H-NMR (CDCl₃) δ: 7.86-7.82 (1H,m), 7.52-7.48 (1H,m), 7.03-6.98 (1H,m), 6.79 (1H,brs), 1.97-1.80 (2H,m), 1.65 (3H,s), 1.52-1.44 (2H,m), 1.38-1.31 (2H,m), 0.92-0.87 (3H,m).

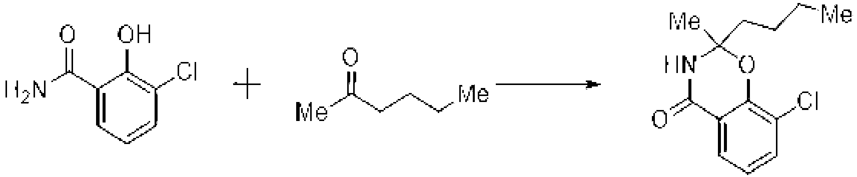

### <Reference Example 41 - Step 1>

### Synthesis of 4-bromo-2-butyl-8-chloro-2-methyl-2H-benzo[e][1,3]oxazine

10 ml of a solution containing 0.80 g of 2-butyl-8-chloro-2-methyl-2,3-dihydro-4H-benzo[e][1,3]oxazin-4-one obtained by the method of Reference Example 33 and 1.36 g of phosphorus oxybromide in 1,2-dichloroethane was stirred at 50°C for 4 hours. The reaction mixture was allowed to cool to room temperature, and then the reaction mixture was added into ice water. The organic layer is extracted with ethyl acetate. The obtained organic layer was washed once each with water and saturated saline, and dried over sodium sulfate. The solvent was distilled off under reduced pressure. The obtained residue was purified by silica gel column chromatography. The title compound was obtained as 0.76 g of a pale yellow oil.

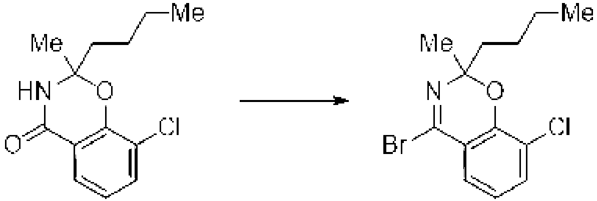

### <Synthesis Example 41 - Step 2>

### Synthesis of 2-butyl-8-chloro-2-methyl-4-(7-methylpyrazolo[1,5-a]pyrimidin-3-yl)-2H-benzo[e][1,3]oxazine (compound No. 870)

1.8 ml of a 1,4-dioxane solution containing 0.18 g of 4-bromo-2-butyl-8-chloro-2-methyl-2H-benzo[e][1,3]oxazine obtained by the method of step 1 of Synthesis Example 41, 76.3 mg of 6-methylpyrazolo[1,5-a]pyrimidine, 0.24 g of potassium carbonate and 65.4 mg of palladium acetate was stirred at 100°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 38.2 mg of a brown oil.

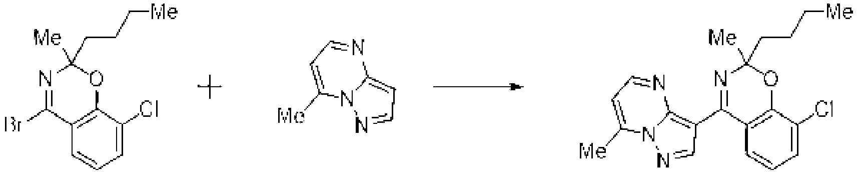

### <Synthesis Example 42>

### Synthesis of 2-butyl-8-chloro-4-(6,7-dimethylpyrazolo[1,5-a]pyrimidin-3-yl)-2-methyl-2H-benzo[e][1,3]oxazine (compound No. 871)

1.8 ml of a 1,4-dioxane solution containing 0.18 g of 4-bromo-2-butyl-8-chloro-2-methyl-2H-benzo[e][1,3]oxazine obtained by the method of step 1 of Synthesis Example 41, 85.3 mg of 6-methylpyrazolo[1,5-a]pyrimidine obtained by the method of Reference Example 17, 0.24 g of potassium carbonate and 67.0 mg of palladium acetate was stirred at 100°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 73.6 mg of a brown oil.

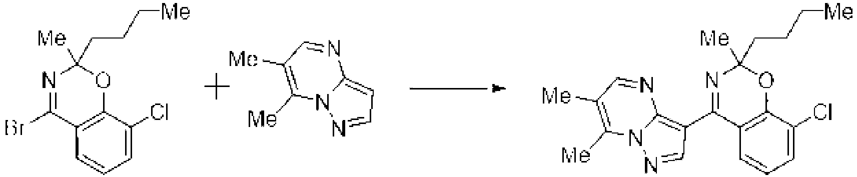

### <Reference Example 34>

### Synthesis of 6,7-dimethylpyrazolo[1,5-a]pyrimidin-3-yl)(2-hydroxyphenyl)methanone

100 ml of a ethanol solution containing 5.00 g of 4-oxo-4H-1-benzopyran-3-carbonitrile, 14.3 g of cesium carbonate, and 3.65 g of ethyl N-aminocarbamate was stirred at 70°C for 30 minutes. 50 ml of acetic acid and 3.44 g of sodium 2-methyl-3-oxobut-1-en-1-olate was added to the residue obtained after removal of the solvent, and the reaction mixture was stirred at 100°C for 2 hours. The reaction mixture was allowed to cool to room temperature, added to a saturated aqueous solution of sodium bicarbonate and then extracted with ethyl acetate. The obtained organic layer was dried over sodium sulfate, and the solvent was distilled off under reduced pressure. The obtained residue was purified by silica gel column chromatography. The title compound was obtained as 1.21 g of a red solid.

1H-NMR (CDCl₃) δ: 12.31 (1H, s), 8.60 (1H, s), 8.52 (1H, s), 7.94 (1H, dd, J = 8.0, 1.5 Hz), 7.52-7.50 (1H, m), 7.07 (1H, d, J = 9.2 Hz), 6.94 (1H, t, J = 6.9 Hz), 2.87 (3H, s), 2.46 (3H, s).

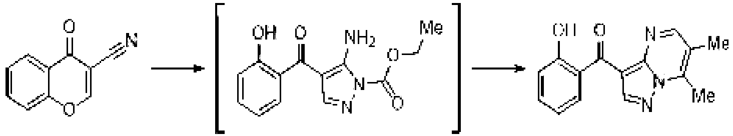

### <Synthesis Example 43>

### Synthesis of Methyl 3-(4-(6,7-dimethylpyrazolo[1,5-a]pyrimidin-3-yl)-2-methyl-2H-benzo[e][1,3]oxazin-2-yl)propanoate (compound No. 956)

4.0 ml of a solution containing 0.10 g of (6,7-dimethylpyrazolo[1,5-a]pyrimidin-3-yl)(2-hydroxyphenyl)methanone obtained by the method of Reference Example 34, 0.5 ml of methyl 4-oxopentanoate, 0.50 g of ammonium acetate, and 1.00 g of sodium sulfate in toluene was stirred under heat reflux for one hour. The reaction mixture was allowed to cool to room temperature, and then phase-separated with a water and ethyl acetate. The obtained organic layer was dried over sodium sulfate, and the solvent was distilled off under reduced pressure. The obtained residue was purified by silica gel column chromatography. The title compound was obtained as 17.4 mg of a pale yellow oil.

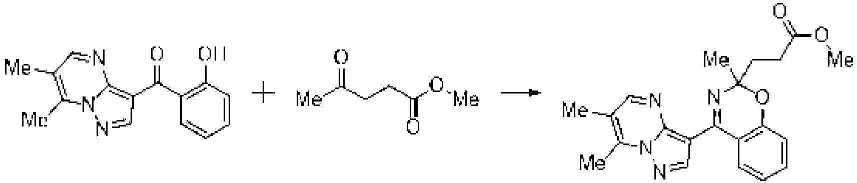

### <Synthesis Example 44>

### Synthesis of 4-(6,7-dimethylpyrazolo[1,5-a]pyrimidin-3-yl)-2-(4-fluorobenzyl)-2-methyl-2H-benzo[e][1,3]oxazine (compound No. 957)

4.0 ml of a solution containing 0.10 g of (6,7-dimethylpyrazolo[1,5-a]pyrimidin-3-yl)(2-hydroxyphenyl)methanone obtained by the method of Reference Example 34, 0.5 ml of 1-(4-fluorophenyl)propan-2-one, and 0.50 g of ammonium acetate in toluene was stirred under heat reflux for one hour. The reaction mixture was allowed to cool to room temperature, and then phase-separated with a water and ethyl acetate. The obtained organic layer was dried over sodium sulfate, and the solvent was distilled off under reduced pressure. The obtained residue was purified by silica gel column chromatography. The title compound was obtained as 45.5 mg of a pale yellow oil.

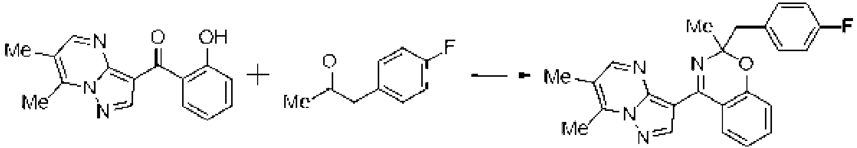

### <Synthesis Example 45>

### Synthesis of 4-(6,7-dimethylpyrazolo[1,5-a]pyrimidin-3-yl)-2-methyl-2-phenethyl-2H-benzo[e][1,3]oxazine (compound No. 959)

4.0 ml of a solution containing 0.10 g of (6,7-dimethylpyrazolo[1,5-a]pyrimidin-3-yl)(2-hydroxyphenyl)methanone obtained by the method of Reference Example 34, 0.5 ml of 4-phenylbutan-2-one, and 0.50 g of ammonium acetate in toluene was stirred under heat reflux for one hour. The reaction mixture was allowed to cool to room temperature, and then phase-separated with a water and ethyl acetate. The obtained organic layer was dried over sodium sulfate, and the solvent was distilled off under reduced pressure. The obtained residue was purified by silica gel column chromatography. The title compound was obtained as 32.7 mg of a brown oil.

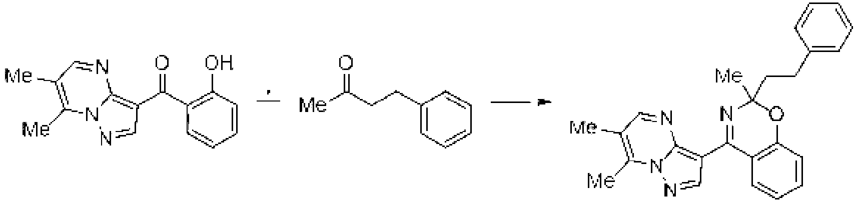

### <Synthesis Example 46>

### Synthesis of 4-(6,7-dimethylpyrazolo[1,5-a]pyrimidin-3-yl)-2-methyl-2-(4-methylpent-3-en-1-yl)-2H-benzo[e][1,3]oxazine (compound No. 961)

1.0 ml of a solution containing 89.6 mg of (6,7-dimethylpyrazolo[1,5-a]pyrimidin-3-yl)(2-hydroxyphenyl)methanone obtained by the method of Reference Example 34, 0.5 ml of 6-methylhept-5-en-2-one, and 0.50 g of ammonium acetate in toluene was stirred under heat reflux for one hour. The reaction mixture was allowed to cool to room temperature, and then phase-separated with a water and ethyl acetate. The obtained organic layer was dried over sodium sulfate, and the solvent was distilled off under reduced pressure. The obtained residue was purified by silica gel column chromatography. The title compound was obtained as 32.2 mg of a yellow oil.

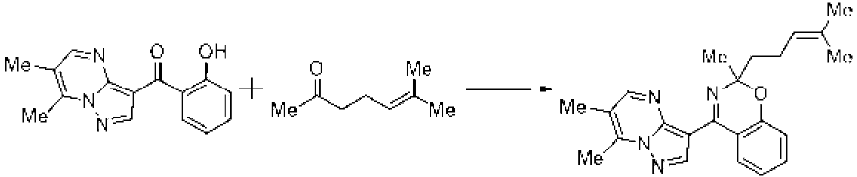

### <Synthesis Example 47>

### Synthesis of 4-(4-(6,7-dimethylpyrazolo[1,5-a]pyrimidin-3-yl)-2-methyl-2H-benzo[e][1,3]oxazin-2-yl)butanenitrile (compound No. 974)

3.9 ml of a solution containing 98.4 mg of (6,7-dimethylpyrazolo[1,5-a]pyrimidin-3-yl)(2-hydroxyphenyl)methanone obtained by the method of Reference Example 34, 0.49 ml of 5-oxohexanenitrile, and 0.98 g of ammonium acetate in toluene was stirred under heat reflux for one hour and 30 minutes. The reaction mixture was allowed to cool to room temperature, and then phase-separated with a water and ethyl acetate. The obtained organic layer was dried over sodium sulfate, and the solvent was distilled off under reduced pressure. The obtained residue was purified by silica gel column chromatography. The title compound was obtained as 17.9 mg of a yellow oil.

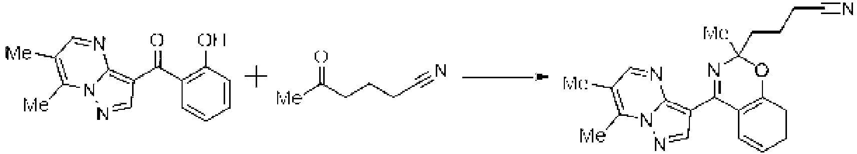

### <Synthesis Example 48>

### Synthesis of 4-(6,7-dimethylpyrazolo[1,5-a]pyrimidin-3-yl)-2-methyl-2-phenyl-2H-benzo[e][1,3]oxazine (compound No. 976)

4.0 ml of a solution containing 0.10 g of (6,7-dimethylpyrazolo[1,5-a]pyrimidin-3-yl)(2-hydroxyphenyl)methanone obtained by the method of Reference Example 34, 0.5 ml of acetophenone, and 0.50 g of ammonium acetate in toluene was stirred under heat reflux for one hour. The reaction mixture was allowed to cool to room temperature, and then phase-separated with a water and ethyl acetate. The obtained organic layer was dried over sodium sulfate, and the solvent was distilled off under reduced pressure. The obtained residue was purified by silica gel column chromatography. The title compound was obtained as 13.3 mg of a yellow oil.

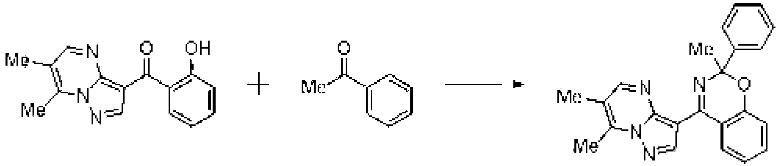

### <Reference Example 35>

### Synthesis of 7,8-dimethylimidazo[1,2-b]pyridazine Hydrochloride

Mixed solution of 150 ml of ethanol containing 11.1 g of 6-chloro-7,8-dimethylimidazo[1,2-b]pyridazine and 4.2 g of Perlman catalyst (20% palladium, approximately 50% water-wetted product) was stirred at room temperature under a hydrogen atmosphere for 3 hours and 30 minutes. After replacing the reaction chamber with a nitrogen atmosphere, the reaction mixture was filtered through celite and washed with methanol. The solvent was distilled off under reduced pressure. The title compound was obtained as 10.5 g of a white solid.

1H-NMR (CDCl₃) δ: 8.72 (1H, s), 8.46 (1H, d, J = 1.8 Hz), 8.15 (1H, d, J = 1.8 Hz), 2.64 (3H, s), 2.43 (3H, s).

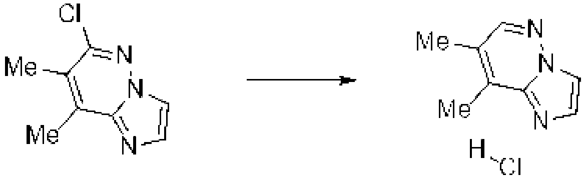

### <Reference Example 36>

### Synthesis of 7-methylimidazo[1,2-b]pyridazine Hydrochloride

Mixed solution of 100 ml of ethanol containing 9.8 g of 6-chloro-7-methylimidazo[1,2-b]pyridazine and 4.2 g of Perlman catalyst (20% palladium, approximately 50% water-wetted product) was stirred at room temperature under a hydrogen atmosphere for 6 hours and 30 minutes. After replacing the reaction chamber with a nitrogen atmosphere, the reaction mixture was filtered through celite and washed with methanol. The solvent was distilled off under reduced pressure. The title compound was obtained as 7.9 g of a white solid.

1H-NMR (CDCl₃) δ: 8.62 (1H, d, J = 2.0 Hz), 8.56-8.53 (1H, m), 8.05 (1H, d, J = 1.0 Hz), 7.94 (1H, d, J = 2.2 Hz), 2.62 (3H, d, J = 1.0 Hz).

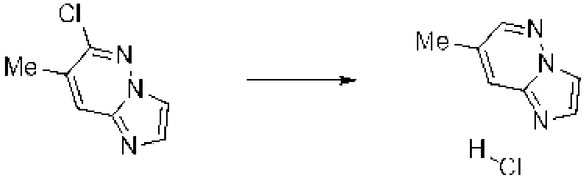

### <Reference Example 37>

### Synthesis of 8-methylimidazo[1,2-b]pyridazine Hydrochloride

Mixed solution of 90 ml of ethanol containing 9.0 g of 6-chloro-8-methylimidazo[1,2-b]pyridazine and 3.8 g of Perlman catalyst (20% palladium, approximately 50% water-wetted product) was stirred at room temperature under a hydrogen atmosphere for 2 hours. After replacing the reaction chamber with a nitrogen atmosphere, the reaction mixture was filtered through celite and washed with methanol. The solvent was distilled off under reduced pressure. The title compound was obtained as 8.0 g of a pale yellow solid.

1H-NMR (DMSO-d₆) δ: 8.83 (1H, d, J = 4.9 Hz, ), 8.68 (1H, d, J = 2.0 Hz, ), 8.35 (1H, d, J = 2.0 Hz, ), 7.64 (1H, dd, J = 4.9, 1.0 Hz, ), 2.70 (3H, d, J = 1.0 Hz, ).

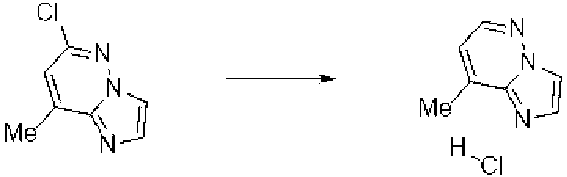

### <Reference Example 38>

### Synthesis of 2-methyl-2-pentyl-2H-benzo[e][1,3]oxazin-4-ol

200 ml of a toluene solution containing 20 g of 2-hydroxybenzamide, 25.5 g of p-toluenesulfonic acid monohydrate, and 60 ml of heptan-2-one was stirred under heat reflux for 3 hours. The reaction mixture was allowed to cool to room temperature, added to a saturated aqueous solution of sodium bicarbonate to quench and then phase-separated with a water and ethyl acetate. Regardning the obtained organic layer, the solvent was distilled off under reduced pressure. The obtained residue was purified by silica gel column chromatography. The title compound was obtained as 4.64 g of a yellow oil.

1H-NMR (CDCl₃) δ: 7.91 (1H, dd, J = 7.8, 1.7 Hz), 7.44 (1H, ddd, J = 8.7, 7.0, 1.3 Hz), 7.28-7.26 (1H, br m), 7.05 (1H, td, J = 5.4, 1.1 Hz), 6.91 (1H, br s), 1.89-1.85 (2H, m), 1.60 (3H, s), 1.51-1.43 (2H, m), 1.31-1.23 (4H, m), 0.86 (3H, t, J = 7.3 Hz).

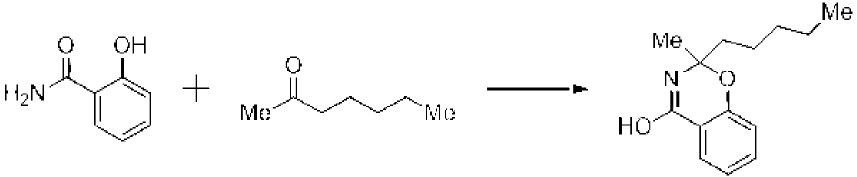

### <Reference Example 49 - Step 1>

### Synthesis of 4-bromo-2-methyl-2-pentyl-2H-benzo[e][1,3]oxazine

7 ml of a solution containing 0.70 g of 2-methyl-2-pentyl-2H-benzo[e][1,3]oxazin-4-ol obtained by the method of Reference Example 38, and 1.32 g of phosphorus oxybromide in 1,2-dichloroethane was stirred at 50°C for 40 minutes. The reaction mixture was allowed to cool to room temperature, and then the reaction mixture was added into ice water. The organic layer is extracted with ethyl acetate. The obtained organic layer was washed once each with water and saturated saline, and dried over sodium sulfate. The solvent was distilled off under reduced pressure. The obtained residue was purified by silica gel column chromatography. The title compound was obtained as 0.56 g of a colorless oil.

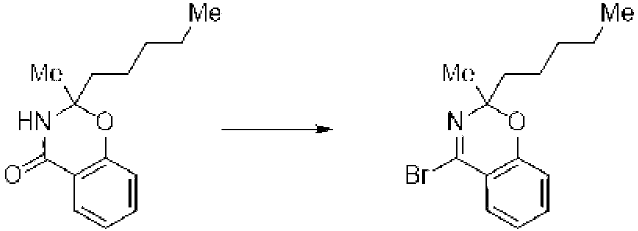

### <Reference Example 49 - Step 2>

### Synthesis of 2-methyl-4-(8-methylimidazo[1,2-b]pyridazin-3-yl)-2-pentyl-2H-benzo[e][1,3]oxazine (compound No. 612)

1.6 ml of a N,N-dimethylacetamide solution containing 163.0 mg of 4-bromo-2-methyl-2-pentyl-2H-benzo[e][1,3]oxazine obtained by the method of step 1 of Synthesis Example 49, 80.4 mg of 8-methylimidazo[1,2-b]pyridazine hydrochloride obtained by the method of Reference Example 37, 0.17 g of potassium carbonate and 63.3 mg of palladium acetate was stirred at 100°C for 5 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 33.8 mg of a brown solid.

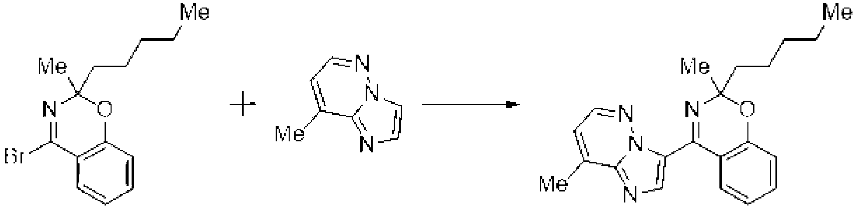

### <Reference Example 39>

### Synthesis of 2-butyl-2,8-dimethyl-1,2-dihydroquinazolin-4-ol

50 ml of a hexane-2-one solution containing 5.00 g of 2-hydroxy-3-methylbenzamide, 6.29 g of p-toluenesulfonic acid monohydrate, and 5 ml of sulfuric acid was stirred at 100°C. The reaction mixture was allowed to cool to room temperature, added to a saturated aqueous solution of sodium bicarbonate to quench and then phase-separated with a water and ethyl acetate. The obtained organic layer was washed with saturated saline, and dried over sodium sulfate. The solvent was distilled off under reduced pressure. The obtained residue was filtered after washing with hexane. The residue obtained by concentrating the filtrate was purified by silica gel column chromatography. The title compound was obtained as 3.11 g of a solid.

1H-NMR (CDCl₃) δ: 7.77-7.75 (1H, m), 7.31-7.29 (1H, m), 6.96 (1H, t, J = 7.4 Hz), 6.03 (1H, br s), 2.22 (3H, s), 1.91-1.82 (2H, m), 1.58 (3H, s), 1.46-1.42 (2H, m), 1.37-1.29 (2H, m), 0.90 (3H, t, J = 7.3 Hz).

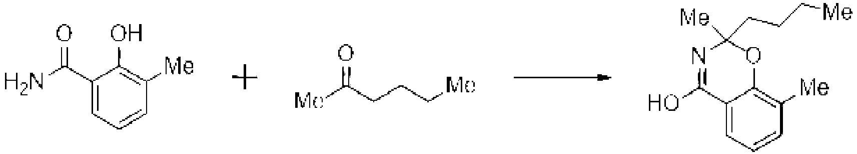

### <Synthesis Example 50 - Step 1>

### Synthesis of 4-bromo-2-butyl-2,8-dimethyl-2H-benzo[e][1,3]oxazine

5 ml of a solution containing 0.25 g of 2-butyl-2,8-dimethyl-2,3-dihydro-4H-benzo[e][1,3]oxazin-4-one obtained by the method of Reference Example 39, 0.50 g of phosphorus oxybromide in 1,2-dichloroethane was stirred at 50°C for 4 hours. The reaction mixture was allowed to cool to room temperature, and then the reaction mixture was added into ice water. The organic layer is extracted with ethyl acetate. The obtained organic layer was washed once each with water and saturated saline, and dried over sodium sulfate. The solvent was distilled off under reduced pressure. The obtained residue was purified by silica gel column chromatography. The title compound was obtained as 0.28 g of a pale yellow oil.

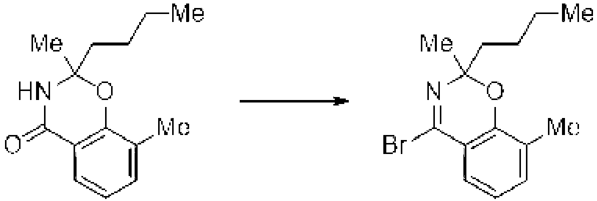

### <Synthesis Example 50 - Step 2>

### Synthesis of 2-butyl-4-(7,8-dimethylimidazo[1,2-b]pyridazin-3-yl)-2,8-dimethyl-2H-benzo[e][1,3]oxazine (compound No. 653)

5 ml of a N,N-dimethylacetamide solution containing 230 mg of 4-bromo-2-butyl-2,8-dimethyl-2H-benzo[e][1,3]oxazine obtained by the method of step 1 of Synthesis Example 50, 126 mg of 7,8-dimethylimidazo[1,2-b]pyridazine hydrochloride obtained by the method of Reference Example 35, 322 mg of potassium carbonate and 87 mg of palladium acetate was stirred at 110°C for 2 hours. The reaction mixture was allowed to cool to room temperature and then water was added to the reaction mixture and the reaction mixture was filtered through celite. The organic layer obtained by separating the filtrate with ethyl acetate was dried over sodium sulfate, and the solvent was distilled off under reduced pressure. The obtained residue was purified by silica gel column chromatography. The title compound was obtained as 85.0 mg of a brown oil.

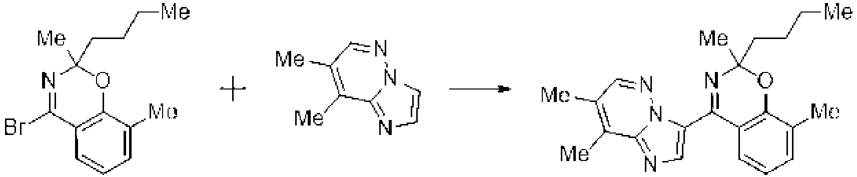

### <Synthesis Example 51>

### Synthesis of 2-butyl-2,8-dimethyl-4-(8-methylimidazo[1,2-b]pyridazin-3-yl)-2H-benzo[e][1,3]oxazine (compound No. 654)

2.5 ml of a N,N-dimethylacetamide solution containing 230 mg of 4-bromo-2-butyl-2,8-dimethyl-2H-benzo[e][1,3]oxazine obtained by the method of step 1 of Synthesis Example 46, 112 mg of 8-methylimidazo[1,2-b]pyridazine hydrochloride obtained by the method of Reference Example 37, 318 mg of potassium carbonate and 17.2 mg of palladium acetate was stirred at 110°C for 2 hours. The reaction mixture was allowed to cool to room temperature and then water was added to the reaction mixture and the reaction mixture was filtered through celite. The organic layer obtained by separating the filtrate with ethyl acetate was dried over sodium sulfate, and the solvent was distilled off under reduced pressure. The obtained residue was purified by silica gel column chromatography. The title compound was obtained as 10.0 mg of a brown oil.

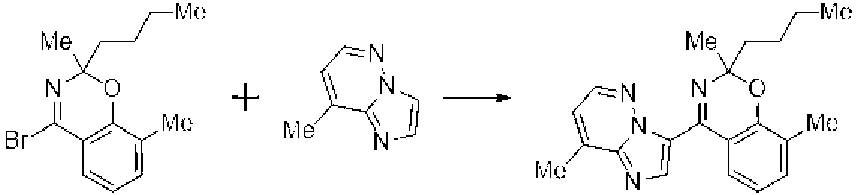

### <Reference Example 40>

### Synthesis of 2-butyl-2,7-dimethyl-2,3-dihydro-4H-benzo[e][1,3]oxazin-4-one

50 ml of a 2-hexanone solution containing 5 g of 2-hydroxy-4-methylbenzamide, 1.6 g of p-toluenesulfonic acid monohydrate, 5 ml of sulfuric acid was stirred at 120°C for 3 hours. The reaction mixture was allowed to cool to room temperature and then phase-separated by the addition of a saturated aqueous solution of sodium bicarbonate and ethyl acetate, and the organic layer was washed with saturated saline. The obtained organic layer was dried over sodium sulfate, and the solvent was distilled off under reduced pressure. The obtained residue was then purified by silica gel column chromatography, and the title compound was obtained as 1.6 g of a solid.

1H-NMR (CDCl₃) δ: 7.78 (1H, d, J = 7.8 Hz), 6.98 (1H, s), 6.86 (1H, dq, J = 7.9, 0.7 Hz), 6.72 (1H, s), 2.36 (3H, s), 1.87-1.85 (2H, m), 1.58 (3H, s), 1.48-1.39 (2H, m), 1.36-1.27 (2H, m), 0.89 (3H, t, J = 7.2 Hz).

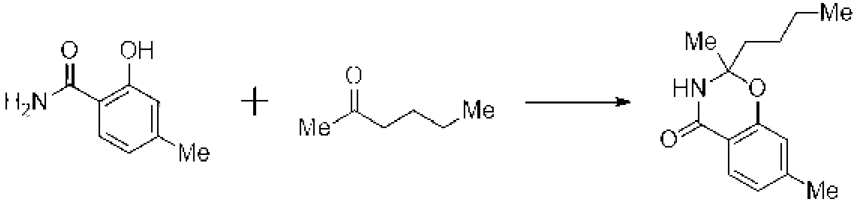

### <Synthesis Example 52 - Step 1>

### Synthesis of 4-bromo-2-butyl-2,7-dimethyl-2H-benzo[e][1,3]oxazine

7 ml of a solution containing 700 mg of 2-butyl-2,7-dimethyl-2,3-dihydro-4H-benzo[e][1,3]oxazin-4-one obtained by the method of Reference Example 40, 1.29 g of phosphorus oxybromide in 1,2-dichloroethane was stirred at 50°C for 3 hours and 30 minutes. The reaction mixture was allowed to cool to room temperature, and then the reaction mixture was added into ice water. The organic layer is extracted with ethyl acetate. The obtained organic layer was washed once each with water and saturated saline, and dried over sodium sulfate. The solvent was distilled off under reduced pressure. The obtained residue was purified by silica gel column chromatography. The title compound was obtained as 320 mg of a colorless oil.

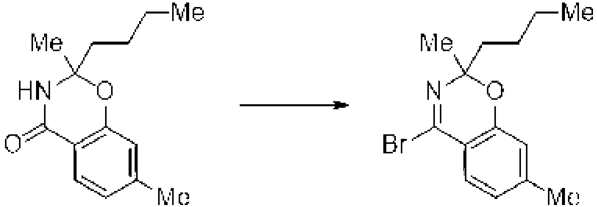

### <Synthesis Example 52 - Step 2>

### Synthesis of 2-butyl-2,7-dimethyl-4-(7-methylimidazo[1,2-b]pyridazin-3-yl)-2H-benzo[e][1,3]oxazine (compound No. 713)

6 ml of a N,N-dimethylacetamide solution containing 300 mg of 4-bromo-2-butyl-2,7-dimethyl-2H-benzo[e][1,3]oxazine obtained by the method of step 1 of Synthesis Example 52, 150 mg of 7-methylimidazo[1,2-b]pyridazine hydrochloride obtained by the method of Reference Example 29, 400 mg of potassium acetate and 100 mg of palladium acetate was stirred at 110°C for 3 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 28.7 mg of a brown oil.

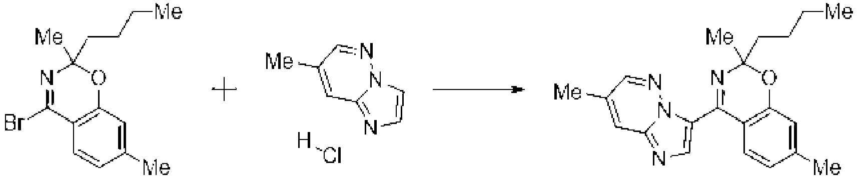

### <Reference Example 41>

### Synthesis of 2-isobutyl-2-methyl-2H-benzo[e][1,3]oxazin-4-ol

50 ml of a toluene solution containing 10 g of 2-hydroxybenzamide, 2.51 g of p-toluenesulfonic acid monohydrate, and 50 ml of 4-methylpentan-2-one was stirred under reflux for 5 hours with a Dean-Stark device. The reaction mixture was allowed to cool to room temperature, added to a saturated aqueous solution of potassium carbonate to quench and then phase-separated with a water and ethyl acetate. Regarding the obtained organic layer, the solvent was distilled off under reduced pressure. The obtained residue was purified by silica gel column chromatography. The title compound was obtained as 8.51 g of a white solid.

1H-NMR (CDCl₃) δ: 7.92 (1H, dd, J = 7.6, 1.9 Hz, ), 7.46-7.42 (1H, m, ), 7.07-7.04 (1H, m, ), 6.91-6.88 (1H, m, ), 6.41 (1H, br s, ), 2.00-1.92 (1H, m, ), 1.83 (1H, dd, J = 15.0, 6.5 Hz, ), 1.77 (1H, dd, J = 15.0, 6.5 Hz, ), 1.61 (3H, s, ), 0.98 (6H, dd, J = 6.5, 4.0 Hz, ).

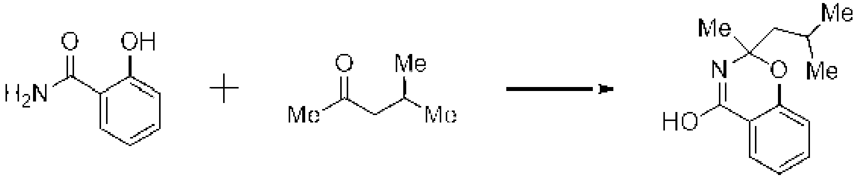

### <Synthesis Example 53 - Step 1>

### Synthesis of 4-bromo-2-isobutyl-2-methyl-2H-benzo[e][1,3]oxazine

7 ml of a solution containing 0.70 g of 2-isobutyl-2-methyl-2H-benzo[e][1,3]oxazin-4-ol obtained by the method of Reference Example 41 and 1.12 g of phosphorus oxybromide in 1,2-dichloroethane was stirred at 50°C for 30 minutes. The reaction mixture was allowed to cool to room temperature, and then the reaction mixture was added into ice water. The organic layer is extracted with ethyl acetate. The obtained organic layer was washed once each with water and saturated saline, and dried over sodium sulfate. The solvent was distilled off under reduced pressure. The obtained residue was purified by silica gel column chromatography. The title compound was obtained as 0.43 g of a pale yellow oil.

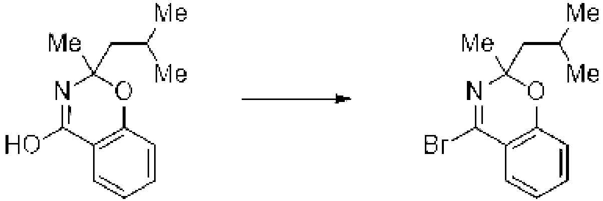

### <Synthesis Example 53 - Step 2>

### Synthesis of 4-(7,8-dimethylimidazo[1,2-b]pyridazin-3-yl)-2-isobutyl-2-methyl-2H-benzo[e][1,3]oxazine (compound No. 775)

4.2 ml of a N,N-dimethylacetamide solution containing 0.14 g of 4-bromo-2-isobutyl-2-methyl-2H-benzo[e][1,3]oxazine obtained by the method of step 1 of Synthesis Example 53, 0.11 g of 7,8-dimethylimidazo[1,2-b]pyridazine hydrochloride, 0.15 g of potassium acetate and 56.5 mg of palladium acetate was stirred at 100°C for 2 hours. The reaction mixture was allowed to cool to room temperature and then water was added to the reaction mixture and the reaction mixture was filtered through celite. The organic layer obtained by separating the filtrate with ethyl acetate was dried over sodium sulfate, and the solvent was distilled off under reduced pressure. The obtained residue was purified by silica gel column chromatography. The title compound was obtained as 57.0 mg of a brown oil.

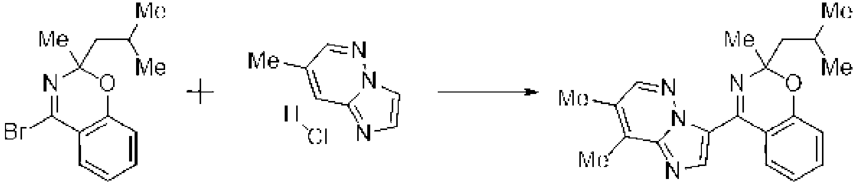

### <Synthesis Example 54>

### Synthesis of 2-butyl-8-chloro-4-(7,8-dimethylimidazo[1,2-b]pyridazin-3-yl)-2-methyl-2H-benzo[e][1,3]oxazine (compound No. 911)

5 ml of a N,N-dimethylacetamide solution containing 240 mg of 4-bromo-2-butyl-8-chloro-2-methyl-2H-benzo[e][1,3]oxazine obtained by the method of step 1 of Synthesis Example 41, 130 mg of 7,8-dimethylimidazo[1,2-b]pyridazine hydrochloride obtained by the method of Reference Example 35, 300 mg of potassium acetate and 90.0 mg of palladium acetate was stirred at 110°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 90.1 mg of a brown oil.

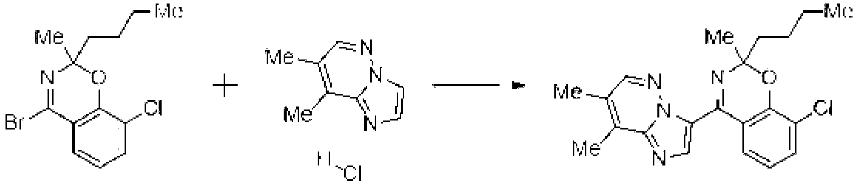

### <Synthesis Example 55>

### Synthesis of 2-butyl-8-chloro-2-methyl-4-(7-methylimidazo[1,2-b]pyridazin-3-yl)-2H-benzo[e][1,3]oxazine (compound No. 912)

5 ml of a N,N-dimethylacetamide solution containing 0.24 g of 4-bromo-2-butyl-8-chloro-2-methyl-2H-benzo[e][1,3]oxazine obtained by the method of step 1 of Synthesis Example 41, 0.12 g of 7-methylimidazo[1,2-b]pyridazine hydrochloride obtained by the method of Reference Example 36, 0.30 g of potassium acetate and 0.090 g of palladium acetate was stirred at 110°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 44.1 mg of a brown solid.

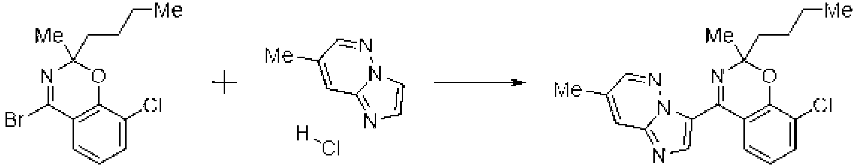

### <Synthesis Example 56>

### Synthesis of 2-butyl-8-chloro-2-methyl-4-(8-methylimidazo[1,2-b]pyridazin-3-yl)-2H-benzo[e][1,3]oxazine (compound No. 913)

5 ml of a N,N-dimethylacetamide solution containing 0.24 g of 4-bromo-2-butyl-8-chloro-2-methyl-2H-benzo[e][1,3]oxazine obtained by the method of step 1 of Synthesis Example 41, 0.12 g of 8-methylimidazo[1,2-b]pyridazine hydrochloride obtained by the method of Reference Example 37, 0.30 g of potassium acetate and 0.090 g of palladium acetate was stirred at 110°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 97.4 mg of a brown oil.

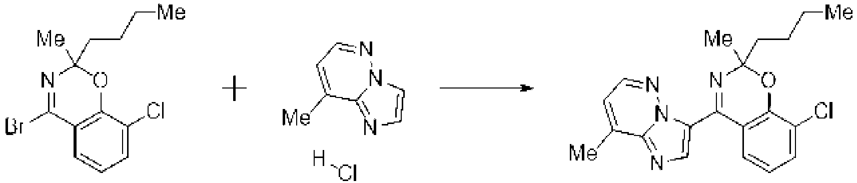

### <Synthesis Example 57>

### Synthesis of 2-butyl-2,8-dimethyl-4-(7-methylimidazo[1,2-b]pyridazin-3-yl)-2H-benzo[e][1,3]oxazine (compound No. 941)

5 ml of a N,N-dimethylacetamide solution containing 0.28 g of 4-bromo-2-butyl-2,8-dimethyl-2H-benzo[e][1,3]oxazine obtained by the method of step 1 of Synthesis Example 46, 0.12 g of 7-methylimidazo[1,2-b]pyridazine hydrochloride obtained by the method of Reference Example 36, 0.30 g of potassium acetate and 0.090 g of palladium acetate was stirred at 110°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 30.7 mg of a pale yellow oil.

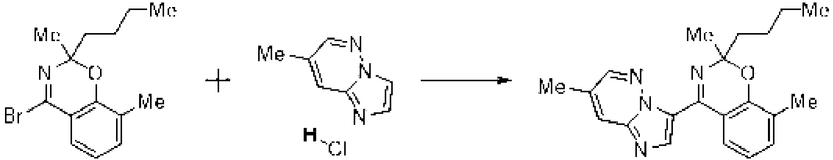

### <Reference Example 42>

### Synthesis of 2-butyl-8-methoxy-2-methyl-2,3-dihydro-4H-benzo[e][1,3]oxazin-4-one

Mixed solution of 25 ml of a 2-hexanone and 25 ml of a toluene containing 5.00 g of 2-hydroxy-3-methoxybenzamide and 1.5 g of pyridinium p-toluenesulfonate was subjected to an azeotrope dehydration reaction at 130°C for 5 hours and 30 minutes with stirring in a reactor installed with a Dean Stark trap. The reaction mixture was allowed to cool to room temperature, phase-separated with a saturated aqueous solution of sodium bicarbonate and ethyl acetate. The obtained organic layer was dried over sodium sulfate, and the solvent was distilled off under reduced pressure. The obtained residue was filtered through diisopropyl ether. The filtrate was purified by silica gel column chromatography. The title compound was obtained as 4.5 g of a pale yellow solid.

1H-NMR (CDCl₃) δ: 7.53 (1H, dd, J = 7.6, 1.7 Hz), 7.07-6.98 (2H, m), 6.27 (1H, br s), 3.89 (3H, s), 1.98-1.86 (2H, m), 1.64 (3H, s), 1.51-1.40 (2H, m), 1.38-1.27 (2H, m), 0.89 (3H, t, J = 7.3 Hz).

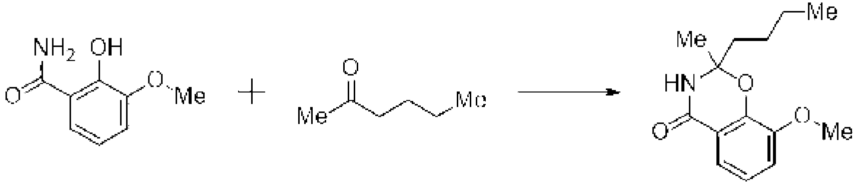

### <Synthesis Example 58 - Step 1>

### Synthesis of 4-bromo-2-butyl-8-methoxy-2-methyl-2H-benzo[e][1,3]oxazine

3 ml of a solution containing 0.80 g of 2-butyl-8-methoxy-2-methyl-2,3-dihydro-4H-benzo[e][1,3]oxazin-4-one obtained by the method of Reference Example 42 and 1.38 g of phosphorus oxybromide in 1,2-dichloroethane was stirred at 50°C for 4 hours. The reaction mixture was allowed to cool to room temperature, and then the reaction mixture was added into ice water. The organic layer is extracted with ethyl acetate. The obtained organic layer was washed once each with water and saturated saline, and dried over sodium sulfate. The solvent was distilled off under reduced pressure. The obtained residue was purified by silica gel column chromatography. The title compound was obtained as 0.72 g of a pale yellow oil.

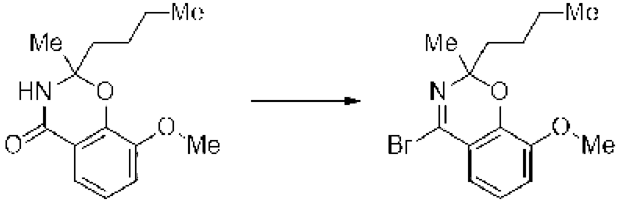

### <Synthesis Example 58 - Step 2>

### Synthesis of 2-butyl-4-(7,8-dimethylimidazo[1,2-b]pyridazin-3-yl)-8-methoxy-2-methyl-2H-benzo[e][1,3]oxazine (compound No. 971)

5 ml of a N,N-dimethylacetamide solution containing 0.24 g of 4-bromo-2-butyl-8-methoxy-2-methyl-2H-benzo[e][1,3]oxazine obtained by the method of step 1 of Synthesis Example 58, 0.13 g of 7,8-dimethylimidazo[1,2-b]pyridazine hydrochloride obtained by the method of Reference Example 35, 0.30 g of potassium acetate and 0.090 g of palladium acetate was stirred at 110°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 0.11 g of a brown oil.

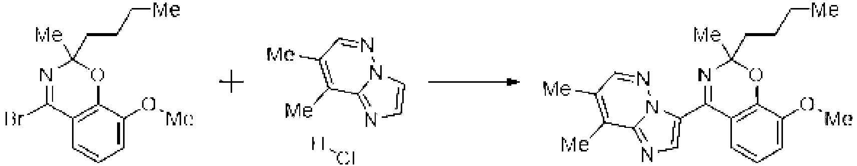

### <Synthesis Example 59>

### Synthesis of 2-butyl-8-methoxy-2-methyl-4-(7-methylimidazo[1,2-b]pyridazin-3-yl)-2H-benzo[e][1,3]oxazine (compound No. 972)

5 ml of a N,N-dimethylacetamide solution containing 0.24 g of 4-bromo-2-butyl-8-methoxy-2-methyl-2H-benzo[e][1,3]oxazine obtained by the method of step 1 of Synthesis Example 58, 0.12 g of 7-methylimidazo[1,2-b]pyridazine hydrochloride obtained by the method of Reference Example 36, 0.30 g of potassium acetate and 0.090 g of palladium acetate was stirred at 110°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 0.11 g of a brown oil.

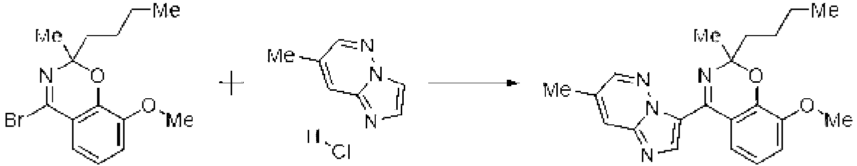

### <Synthesis Example 60>

### Synthesis of 2-butyl-8-methoxy-2-methyl-4-(8-methylimidazo[1,2-b]pyridazin-3-yl)-2H-benzo[e][1,3]oxazine (compound No. 973)

5 ml of a N,N-dimethylacetamide solution containing 0.24 g of 4-bromo-2-butyl-8-methoxy-2-methyl-2H-benzo[e][1,3]oxazine obtained by the method of step 1 of Synthesis Example 58, 0.12 g of 8-methylimidazo[1,2-b]pyridazine hydrochloride obtained by the method of Reference Example 37, 0.30 g of potassium acetate and 0.090 g of palladium acetate was stirred at 110°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 0.13 g of a brown oil.

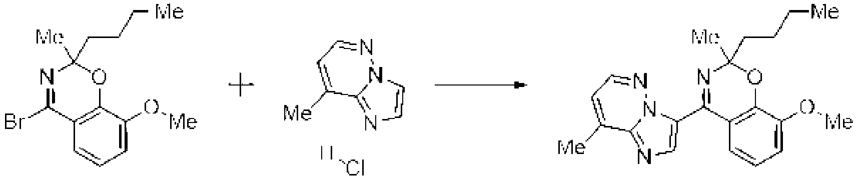

### <Synthesis Example 61 - Step 1>

### Synthesis of 4-chloro-2-methyl,2-propyl-2H-benzo[e][1,3]oxazine

501 µl of oxalyl chloride was added to 6 ml of a dichloromethane solution containing 736 µl of dimethylformamide, and stirred at room temperature for 15 minutes. Then 400 mg of 2-methyl-2-propyl-2,3-dihydro-4H-benzo[e][1,3]oxazine-4-one was added to the reaction mixture and stirred at room temperature for 15 minutes. The solvent of the reaction mixture was distilled off under reduced pressure. The obtained residue was purified by silica gel column chromatography. The title compound was obtained as 346 mg of a colorless oil.

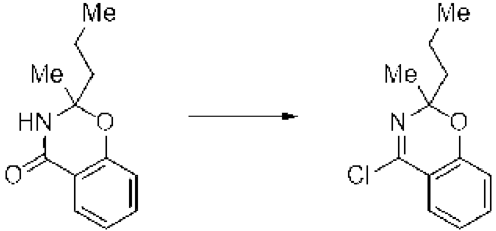

### <Synthesis Example 61 - Step 2>

### Synthesis of 4-(6-bromo-7-methyl-3H-imidazo[4,5-b]pyridine-3-yl)-2-methyl-2-propyl-2H-benzo[e][1,3]oxazine (compound No. 601)

345 mg of potassium carbonate was added to 1.9 ml of a dimethylformamide solution containing 186 mg of 4-chloro-2-methyl,2-propyl-2H-benzo[e][1,3]oxazine obtained by the method of step 1 of Synthesis Example 61 and 212 mg 6-bromo-7-methyl-3H-imidazo[4,5-b]pyridine. The reaction mixture was stirred at 150°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 210 mg of a pale yellow solid.

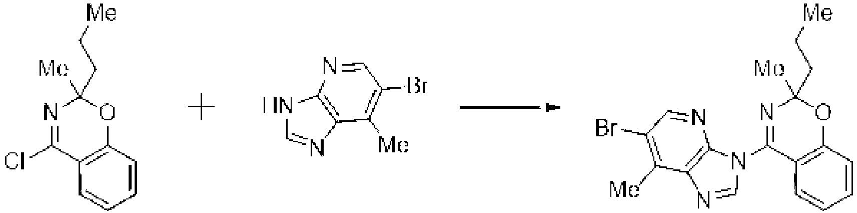

### <Synthesis Example 62>

### Synthesis of 4-(6,7-dimethyl-3H-imidazo[4,5-b]pyridine-3-yl)-2-methyl-2-propyl-2H-benzo[e][1,3]oxazine (compound No. 639)

14 mg of palladium acetate, 36 mg of tricyclohexylphosphine, 64 mg of methylboronic acid, and 68 mg of tripotassium phosphate were added to the mixed solution of 1 ml of toluene solution and 400 µl of water containing 85 mg of 4-(6-bromo-7-methyl-3H-imidazo[4,5-b]pyridine-3-yl)-2-methyl-2-propyl-2H-benzo[e][1,3]oxazine obtained by the method of step 2 of Synthesis Example 61. Then the reaction mixture was stirred at 120°C for 1.5 hours. The reaction mixture was allowed to cool to room temperature. The solvent of the reaction mixture was distilled off. The obtained residue was purified by silica gel column chromatography. The title compound was obtained as 35 mg of a pale yellow solid.

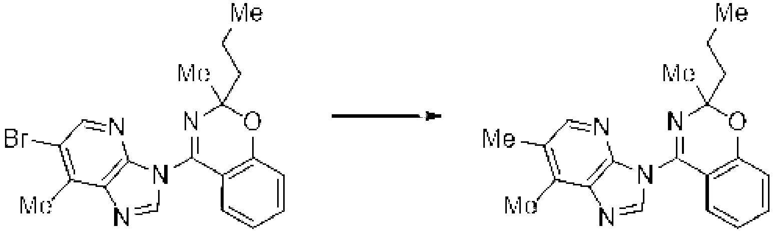

### <Synthesis Example 63 - Step 1>

### Synthesis of 4-chloro-8-fluoro-2-methyl,2-propyl-2H-benzo[e][1,3]oxazine

346 µl of oxalyl chloride was added to 4.5 ml of a dichloromethane solution containing 507 µl of dimethylformamide and stirred at room temperature for 15 minutes. 300 mg of 8-fluoro-2-methyl-2-propyl-2,3-dihydro-4H-benzo[e][1,3]oxazine-4-one was added to the reaction mixture and stirred at room temperature for 15 minutes. The solvent of the reaction mixture was distilled off under reduced pressure. The obtained residue was purified by silica gel column chromatography. The title compound was obtained as 305 mg of a colorless oil.

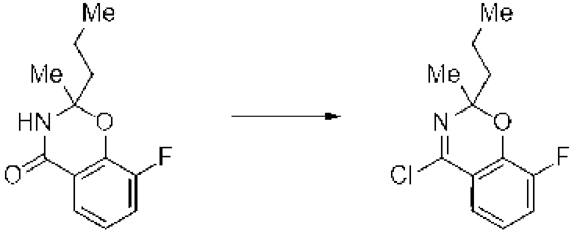

### <Synthesis Example 63 - Step 2>

### Synthesis of 4-(6-bromo-7-methyl-3H-imidazo[4,5-b]pyridine-3-yl)-8-fluoro-2-methyl-2-propyl-2H-benzo[e][1,3]oxazine (compound No. 563)

524 mg of potassium carbonate was added to 3.0 ml of a dimethylformamide solution containing 305 mg of 4-chloro-8-fluoro-2-methyl,2-propyl-2H-benzo[e][1,3]oxazine obtained by the method of step 1 of Synthesis Example 63 and 321 mg 6-bromo-7-methyl-3H-imidazo[4,5-b]pyridine. The reaction mixture was stirred at 150°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 407 mg of a pale yellow solid.

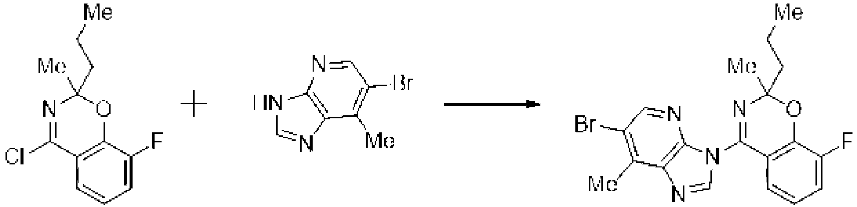

### <Synthesis Example 64>

### Synthesis of 4-(6,7-dimethyl-3H-imidazo[4,5-b]pyridine-3-yl)-8-fluoro-2-methyl-2-propyl-2H-benzo[e][1,3]oxazine (compound No. 640)

61 mg of palladium acetate, 153 mg of tricyclohexylphosphine, 272 mg of methylboronic acid, and 290 mg of tripotassium phosphate were added to the mixed solution of 4 ml of toluene solution and 400 µl of water containing 380 mg of 4-(6-bromo-7-methyl-3H-imidazo[4,5-b]pyridine-3-yl)-8-fluoro-2-methyl-2-propyl-2H-benzo[e][1,3]oxazine obtained by the method of step 2 of Synthesis Example 63. Then the reaction mixture was stirred at 120°C for 2 hours. The reaction mixture was allowed to cool to room temperature. The solvent of the reaction mixture was distilled off. The obtained residue was purified by silica gel column chromatography. The title compound was obtained as 231 mg of a pale yellow solid.

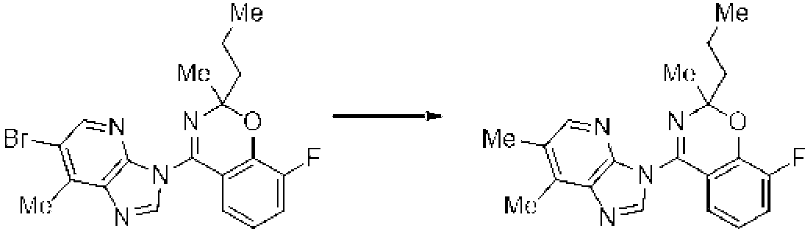

### <Synthesis Example 65>

### Synthesis of 4-(6-bromo-7-methyl-3H-imidazo[4,5-b]pyridine-3-yl)-2,2-dimethyl-2H-benzo[e][1,3]thiazine (compound No. 736)

430 mg of potassium carbonate was added to 2.2 ml of a dimethylformamide solution containing 220 mg of 4-chloro-2,2-dimethyl-2H-benzo[e][1,3]thiazine. The reaction mixture was stirred at 150°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 216 mg of a yellow solid.

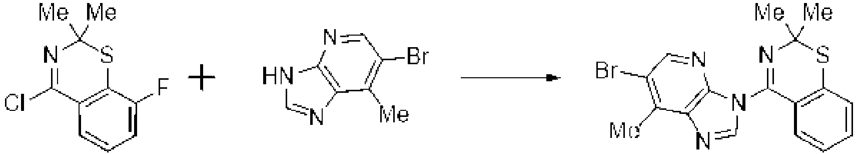

### <Synthesis Example 66>

### Synthesis of 4-(6,7-dimethyl-3H-imidazo[4,5-b]pyridine-3-yl)-2,2-dimethyl-2H-benzo[e][1,3]thiazine (compound No. 746)

35 mg of palladium acetate, 86 mg of tricyclohexylphosphine, 154 mg of methylboronic acid, and 164 mg of tripotassium phosphate were added to the mixed solution of 2 ml of toluene solution and 200 µl of water containing 199 mg of 4-(6-bromo-7-methyl-3H-imidazo[4,5-b]pyridine-3-yl)-2,2-dimethyl-2H-benzo[e][1,3]thiazine obtained by the method of Synthesis Example 65. Then the reaction mixture was stirred at 120°C for 2 hours. The reaction mixture was allowed to cool to room temperature. The solvent of the reaction mixture was distilled off. The obtained residue was purified by silica gel column chromatography. The title compound was obtained as 68 mg of a yellow amorphous product.

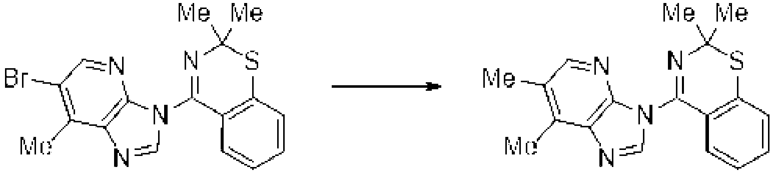

### <Synthesis Example 67 - Step 1>

### Synthesis of 2-butyl-4,8-dichloro-2-methyl-2H-benzo[e][1,3]oxazine

406 µl of oxalyl chloride was added to 6 ml of a dichloromethane solution containing 595 µl of dimethylformamide, and stirred at room temperature for 15 minutes. Then 400 mg of 2-butyl-8-chloro-2-methyl-2,3-dihydro-4H-benzo[e][1,3]oxazin-4-one obtained by the method of Reference Example 26 was added to the reaction mixture and stirred at room temperature for 15 minutes. The solvent of the reaction mixture was distilled off under reduced pressure. The obtained residue was purified by silica gel column chromatography. The title compound was obtained as 412 mg of a colorless oil.

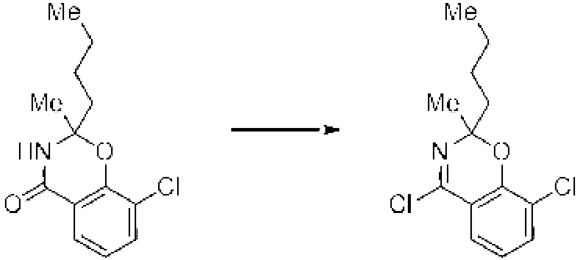

### <Synthesis Example 67 - Step 2>

### Synthesis of 4-(6-bromo-7-methyl-3H-imidazo[4,5-b]pyridine-3-yl)-2-butyl-8-chloro-2-methyl-2H-benzo[e][1,3]oxazine (compound No. 979)

365 mg of potassium carbonate was added to 2.4 ml of a dimethylformamide solution containing 239 mg of 2-butyl-4,8-dichloro-2-methyl-2H-benzo[e][1,3]oxazine obtained by the method of step 1 of Synthesis Example 67 and 224 mg 6-bromo-7-methyl-3H-imidazo[4,5-b]pyridine. The reaction mixture was stirred at 150°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 407 mg of a pale yellow oil.

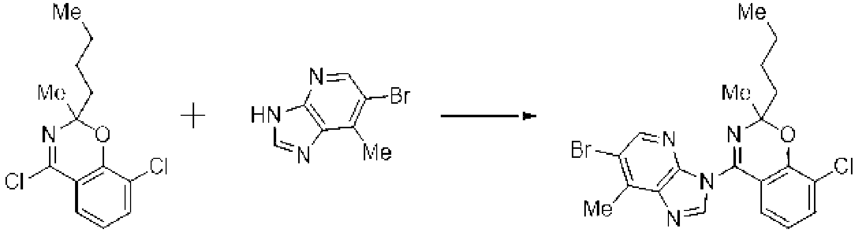

### <Synthesis Example 68>

### Synthesis of 2-butyl-8-chloro-4-(6,7-dimethyl-3H-imidazo[4,5-b]pyridine-3-yl)-2-methyl-2H-benzo[e][1,3]oxazine (compound No. 985)

28 mg of palladium acetate, 70 mg of tricyclohexylphosphine, 124 mg of methylboronic acid, and 132 mg of tripotassium phosphate were added to the mixed solution of 1.9 ml of toluene solution and 190 µl of water containing 186 mg of 4-(6-bromo-7-methyl-3H-imidazo[4,5-b]pyridine-3-yl)-2-butyl-8-chloro-2-methyl-2H-benzo[e][1,3]oxazine obtained by the method of step 2 of Synthesis Example 67. Then the reaction mixture was stirred at 120°C for 1.5 hours. The reaction mixture was allowed to cool to room temperature. The solvent of the reaction mixture was distilled off. The obtained residue was purified by silica gel column chromatography. The title compound was obtained as 42 mg of a pale yellow oil.

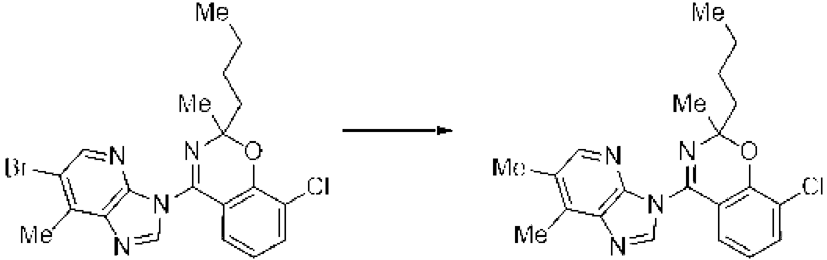

### <Reference Example 43>

### Synthesis of 7-fluoro-2-isobutyl-2,8-dimethyl-2,3-dihydro-4H-benzo[e][1,3]oxazin-4-one

40 ml of a 4-methyl-2-pentanone solution containing 4.0 g of 4-fluoro-3-methyl-2-hydroxybenzamide, 4.5 g of p-toluenesulfonic acid monohydrate was subjected to an azeotrope dehydration reaction at 90°C for one hour with stirring in a reactor installed with a Dean Stark trap. The reaction mixture was allowed to cool to room temperature, phase-separated with potassium carbonate solution and ethyl acetate. The obtained organic layer was dried over sodium sulfate, and the solvent was distilled off under reduced pressure. The obtained residue was purified by silica gel column chromatography. The title compound was obtained as 2.3 g of a white amorphous product.

1H-NMR (CDCl₃) δ: 7.77 (1H,dd, J=8.8, 6.3Hz), 6.75 (1H,t, J=8.8Hz), 6.10 (1H,s), 2.12 (3H, d, J=1.9Hz), 1.99-1.92 (1H,m), 1.86-1.77 (2H,m), 1.60 (3H,s), 1.01 (3H, d, J=2.7Hz), 1.00 (3H, d, J=2.7Hz).

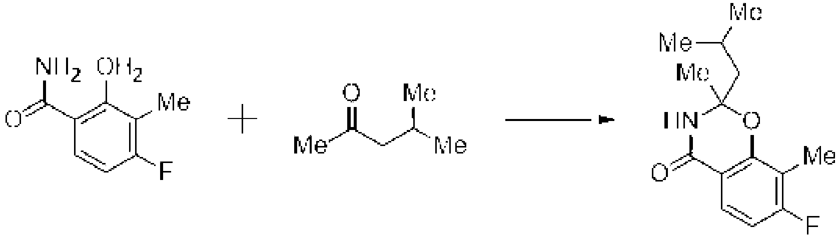

### <Synthesis Example 69 - Step 1>

### Synthesis of 4-chloro-7-fluoro-2-isobutyl-2,8-dimethyl-2H-benzo[e][1,3]oxazine

348 µl of oxalyl chloride was added to 5 ml of a dichloromethane solution containing 511 µl of dimethylformamide, and stirred at room temperature for 15 minutes. Then 340 mg of 7-fluoro-2-isobutyl-2,8-dimethyl-2,3-dihydro-4H-benzo[e][1,3]oxazin-4-one obtained by the method of Reference Example 43 was added to the reaction mixture and stirred at room temperature for 15 minutes. The solvent of the reaction mixture was distilled off under reduced pressure. The obtained residue was purified by silica gel column chromatography. The title compound was obtained as 300 mg of a colorless oil.

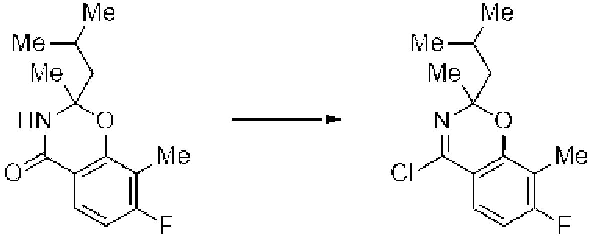

### <Synthesis Example 69 - Step 2>

### Synthesis of 4-(6-bromo-7-methyl-3H-imidazo[4,5-b]pyridine-3-yl)-7-fluoro-2-isobutyl-2,8-dimethyl-2H-benzo[e][1,3]oxazine (compound No. 980)

461 mg of potassium carbonate was added to 3 ml of a dimethylformamide solution containing 300 mg of 4-chloro-7-fluoro-2-isobutyl-2,8-dimethyl-2H-benzo[e][1,3]oxazine obtained by the method of step 1 of Synthesis Example 69 and 259 mg 6-bromo-7-methyl-3H-imidazo[4,5-b]pyridine. The reaction mixture was stirred at 150°C for 2 hours. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 56 mg of a pale yellow oil.

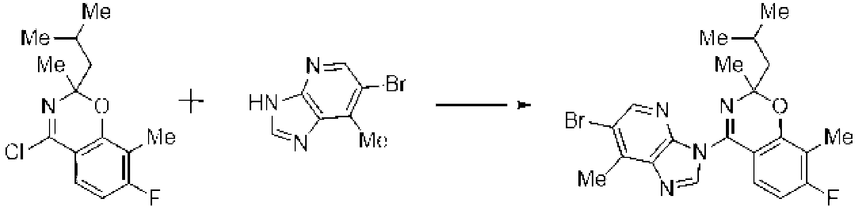

### <Synthesis Example 70>

### Synthesis of 4-(6,7-dimethyl-3H-imidazo[4,5-b]pyridine-3-yl)-7-fluoro-2-isobutyl-2,8-dimethyl-2H-benzo[e][1,3]oxazine (compound No. 986)

8 mg of palladium acetate, 19 mg of tricyclohexylphosphine, 33 mg of methylboronic acid, and 36 mg of tripotassium phosphate were added to the mixed solution of 0.5 ml of toluene solution and 50 µl of water containing 50 mg of 4-(6-bromo-7-methyl-3H-imidazo[4,5-b]pyridine-3-yl)-7-fluoro-2-isobutyl-2,8-dimethyl-2H-benzo[e][1,3]oxazine obtained by the method of step 2 of Synthesis Example 69. Then the reaction mixture was stirred at 120°C for 1.5 hours. The reaction mixture was allowed to cool to room temperature. The solvent of the reaction mixture was distilled off. The obtained residue was purified by silica gel column chromatography. The title compound was obtained as 24 mg of a pale yellow oil.

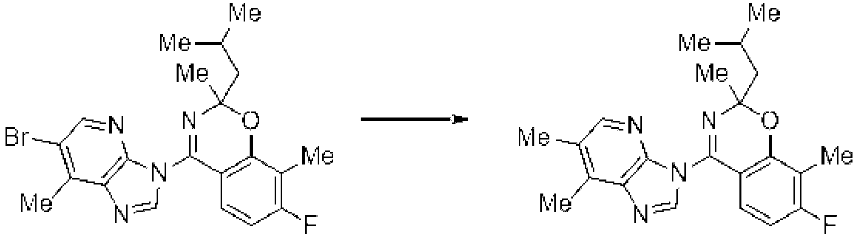

### <Reference Example 44>

### Synthesis of ethyl 2-(2-methyl-4-oxo-3,4-dihydro-2H-benzo[e][1,3]thiazin-2-yl)acetate

50 ml of an acetic acid solution containing 10.0 g of benzoisothiazolinone and 8.65 g of zinc was stirred at room temperature for one hour. Then 50 ml of ethyl acetoacetate and 12.6 g of p-toluenesulfonic acid monohydrate was added to the reaction mixture, and stirred at 110°C for 8 hours. The reaction mixture was phase-separated with potassium carbonate solution and ethyl acetate, and the organic layer was washed with saturated saline. The obtained organic layer was dried over sodium sulfate, and the solvent was distilled off under reduced pressure. The obtained residue was purified by silica gel column chromatography. The title compound was obtained as 8.0 g of a colorless oil.

1H-NMR (CDCl₃) δ: 8.17-8.14 (1H, m, ), 7.44-7.38 (1H, m, ), 7.30-7.24 (2H, m, ), 7.09 (1H, br s, ), 4.15 (2H, q, J = 7.3 Hz, ), 3.10 (1H, d, J = 15.3 Hz, ), 2.81 (1H, d, J = 15.7 Hz, ), 1.85 (3H, s, ), 1.26 (3H, t, J = 7.3 Hz, ).

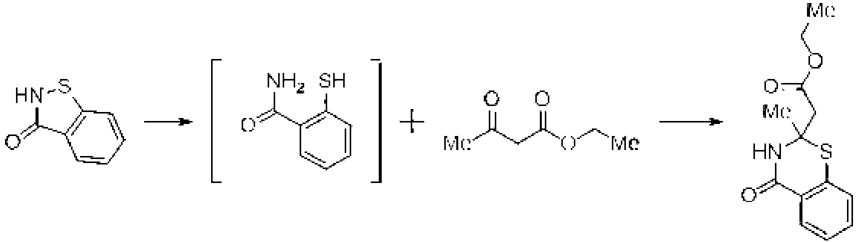

### <Synthesis Example 71 - Step 1>

### Synthesis of ethyl 2-(4-chloro-2-methyl-2H-benzo[e][1,3]thiazin-2-yl)acetate

776 µl of oxalyl chloride was added to 12 ml of a dichloromethane solution containing 1.1 ml of dimethylformamide, and stirred at room temperature for 15 minutes. Then 800 mg of ethyl 2-(2-methyl-4-oxo-3,4-dihydro-2H-benzo[e][1,3]thiazin-2-yl)acetate obtained by the method of Reference Example 44 was added to the reaction mixture and stirred at room temperature for 30 minutes. The solvent of the reaction mixture was distilled off under reduced pressure. The obtained residue was purified by silica gel column chromatography. The title compound was obtained as 706 mg of a colorless oil.

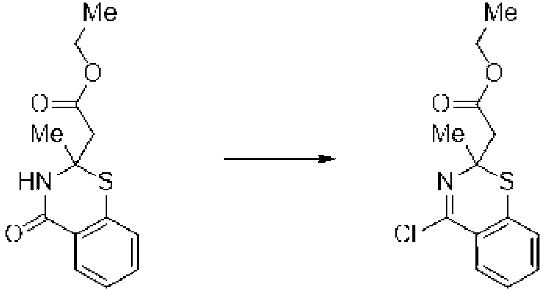

### <Synthesis Example 71 - Step 2>

### Synthesis of ethyl 2-(4-(6-chloro-7-methyl-3H-imidazo[4,5-b]pyridin-3-yl)-2-methyl-2H-benzo[e][1,3]thiazin-2-yl)acetate (compound No. 989)

365 mg of potassium carbonate was added to 1.5 ml of a dimethylformamide solution containing 150 mg of ethyl 2-(4-chloro-2-methyl-2H-benzo[e][1,3]thiazin-2-yl)acetate obtained by the method of step 1 of Synthesis Example 71 and 89 mg 6-chloro-7-methyl-3H-imidazo[4,5-b]pyridine. The reaction mixture was stirred at 100°C for one hour. The reaction mixture was allowed to cool to room temperature and then phase-separated with a water and ethyl acetate. The obtained organic layer was dried over sodium sulfate, and the solvent was distilled off under reduced pressure. The obtained residue was purified by silica gel column chromatography. The title compound was obtained as 40 mg of a pale yellow oil.

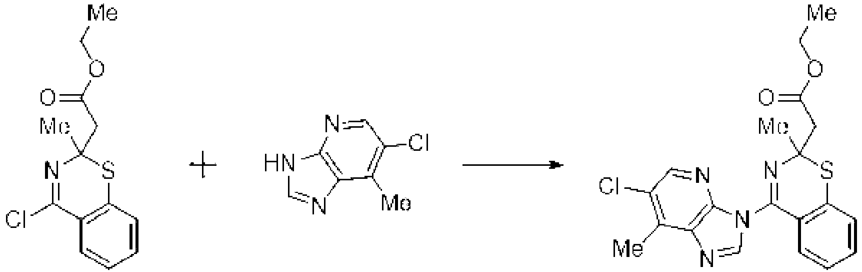

A list of synthesized compounds is shown in Table 7 below. In the table given below, structures A, B, and C are as shown below. In Table 7 below, the substitution position of R₆ represents that substitution occurs at a location of a number described in the structures A, B, and C. For example, "-5-F" means that a fluorine atom is bonded to the position represented by 5 in the structures A, B, and C. The same holds true for the description below in the present specification.

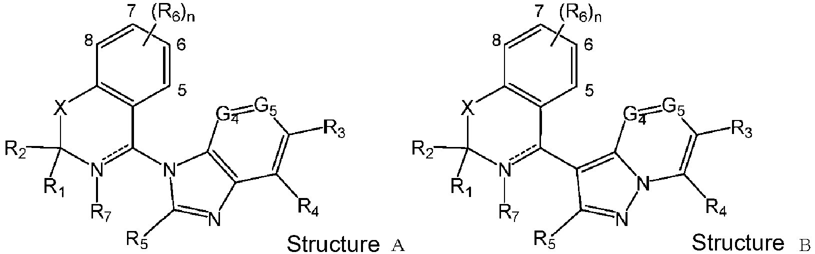

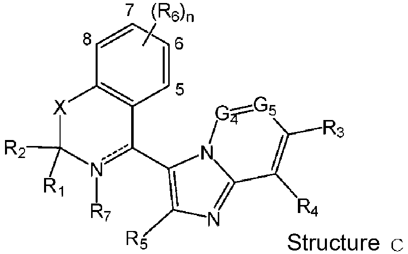

**Table 7:**

| [Table 7-1] | | | | | | | | | | | | | |
|---|---|---|---|---|---|---|---|---|---|---|---|---|---|
| Compound | Structure | R₁ | R₂ | R₃ | R₄ | R₅ | R₆ | R₇ | X | n | Broken line | G₄ | G₅ |
| 1 | A | CH₃ | CH₃ | H | H | H | - | lone pair | O | 0 | double bond | CH | CH |
| 2 | A | CH₃ | CH₃ | CH₃ | H | H | - | lone pair | O | 0 | double bond | CH | CCH₃ |
| 3 | A | -CH₂(CH₂)₃CH₂- | | CH₃ | H | H | - | lone pair | O | 0 | double bond | CH | CCH₃ |
| 4 | A | CH₃ | CH₃ | H | H | H | 8-CH₃ | lone pair | O | 1 | double bond | CH | CH |
| 5 | A | CH₂CH₃ | CH₂CH₃ | H | H | H | - | lone pair | O | 0 | double bond | CH | CH |
| 6 | A | CH₃ | CH₃ | H | CH₃ | H | - | lone pair | O | 0 | double bond | CH | CH |
| 7 | A | CH₃ | CH₃ | CH₃ | CH₃ | H | - | lone pair | O | 0 | double bond | CH | CH |
| 8 | A | CH₃ | CH₃ | CH₃ | CH₃ | H | 8-F | lone pair | O | 1 | double bond | CH | CH |
| 9 | A | CH₃ | CH₃ | CH₃ | CH₃ | H | 8-CH₃ | lone pair | O | 1 | double bond | CH | CH |
| 10 | A | CH₃ | CH₃ | CH₃ | CH₃ | H | 7-CH₃ | lone pair | O | 1 | double bond | CH | CH |
| 11 | A | CH₃ | CH₃ | H | H | H | 7-CH₃ | lone pair | O | 1 | double bond | CH | CH |
| 12 | A | CH₃ | CH₃ | Cl | CH₃ | H | 7-CH₃ | lone pair | O | 1 | double bond | CH | CH |
| 13 | A | CH₃ | CH₃ | Cl | CH₃ | H | 7-OCH₃ | lone pair | O | 1 | double bond | CH | CH |
| 14 | A | CH₃ | CH₃ | CH₃ | CH₃ | H | 7-OCH₃ | lone pair | O | 1 | double bond | CH | CH |
| 15 | A | CH₃ | CH₃ | Cl | CH₃ | H | 8-F | lone pair | O | 1 | double bond | CH | CH |
| 16 | A | CH₃ | CH₃ | H | OCH₃ | H | 8-F | lone pair | O | 1 | double bond | CH | CH |
| 17 | A | CH₃ | CH₃ | CH₃ | CH₃ | H | 7-Br | lone pair | O | 1 | double bond | CH | CH |
| 18 | A | CH₃ | CH₃ | Cl | CH₃ | H | 7-Br | lone pair | O | 1 | double bond | CH | CH |
| 19 | A | CH₃ | CH₃ | F | F | H | 7-OCH₃ | lone pair | O | 1 | double bond | CH | CH |
| 20 | A | CH₃ | CH₃ | Cl | CH₃ | H | 8-CH₃ | lone pair | O | 1 | double bond | CH | CH |
| 21 | A | CH₃ | CH₃ | F | F | H | 8-CH₃ | lone pair | O | 1 | double bond | CH | CH |
| 22 | A | CH₃ | CH₃ | F CH₃ | CH₃ | H | 8-CH₃ | lone pair | O | 1 | double bond | CH | CH |
| 23 | A | CH₃ | CH₃ | | CH₃ | H | 8-Cl | lone pair | O | 1 | double bond | CH | CH |
| 24 | A | CH₃ | CH₃ | Cl | CH₃ | H | 8-Cl | lone pair | O | 1 | double bond | CH | CH |
| 25 | A | CH₃ | CH₃ | F | CH₃ | H | 7-Br | lone pair | O | 1 | double bond | CH | CH |
| 26 | A | CH₃ | CH₃ | H | OCH₃ | H | 7-Br | lone pair | O | 1 | double bond | CH | CH |
| 27 | A | CH₃ | CH₃ | F | CH₃ | H | 8-Cl | lone pair | O | 1 | double bond | CH | CH |
| 28 | A | CH₃ | CH₃ | H | OCH₃ | H | 8-Cl | lone pair | O | 1 | double bond | CH | CH |
| 29 | A | CH₃ | CH₃ | CH₃ | CH₃ | H | 6-Me | lone pair | O | 1 | double bond | CH | CH |
| 30 | A | CH₃ | CH₃ | Cl | CH₃ | H | 6-Me | lone pair | O | 1 | double bond | CH | CH |
| 31 | A | CH₃ | CH₃ | CH₃ | CH₃ | H | 5-F | lone pair | O | 1 | double bond | CH | CH |
| 32 | A | CH₃ | CH₃ | F | CH₃ | H | 5-F | lone pair | O | 1 | double bond | CH | CH |
| 33 | A | CH₃ | CH₃ | F | F | H | 5-F | lone pair | O | 1 | double bond | CH | CH |
| 34 | A | CH₃ | CH₃ | CH₃ | CH₃ | H | 8-OCH₃ | lone pair | O | 1 | double bond | CH | CH |

**[Table 7-2]**

| | | | | | | | | | | | | | |
|---|---|---|---|---|---|---|---|---|---|---|---|---|---|
| 35 | A | CH₃ | CH₃ | H | CH₃ | H | 8-CH₃ | lone pair | O | 1 | double bond | N | CH |
| 36 | A | CH₃ | CH₃ | H | CF3 | H | 8-CH₃ | lone pair | O | 1 | double bond | CH | CH |
| 37 | A | CH₃ | CH₃ | Cl | CH₃ | H | 5-F | lone pair | O | 1 | double bond | CH | CH |
| 38 | A | CH₃ | CH₃ | H | CH₃ | H | 5-F | lone pair | O | 1 | double bond | N | CH |
| 39 | A | CH₃ | CH₃ | CH₃ | CH₃ | H | 7-F | lone pair | O | 1 | double bond | CH | CH |
| 40 | A | CH₃ | CH₃ | Br | CH₃ | H | 7-F | lone pair | O | 1 | double bond | CH | CH |
| 41 | A | CH₃ | CH₃ | Cl | CH₃ | H | 7-F | lone pair | O | 1 | double bond | CH | CH |
| 42 | A | CH₃ | CH₃ | Cl | CH₃ | H | 8-OCH₃ | lone pair | O | 1 | double bond | CH | CH |
| 43 | A | CH₃ | CH₃ | H | CF3 | H | 8-OCH₃ | lone pair | O | 1 | double bond | CH | CH |
| 44 | A | CH₃ | CH₃ | Br | CH₃ | H | 8-CH₃ | lone pair | O | 1 | double bond | CH | CH |
| 45 | A | CH₃ | CH₃ | H | OCH₃ | H | 8-CH₃ | lone pair | O | 1 | double bond | CH | CH |
| 46 | A | CH₃ | CH₃ | H | Cl | H | 8-CH₃ | lone pair | O | 1 | double bond | CH | CH |
| 47 | A | CH₃ | CH₃ | H | F | H | 8-CH₃ | lone pair | O | 1 | double bond | CH | CH |
| 48 | A | CH₃ | CH₃ | H | Cl | H | 8-OCH₃ | lone pair | O | 1 | double bond | CH | CH |
| 49 | A | CH₃ | CH₃ | H | OCH₃ | H | 8-OCH₃ | lone pair | O | 1 | double bond | CH | CH |
| 50 | A | CH₃ | CH₃ | H | F | H | 8-OCH₃ | lone pair | O | 1 | double bond | CH | CH |
| 51 | A | CH₃ | CH₃ | CH₃ | CH₃ | H | - | lone pair | S | 0 | double bond | CH | CH |
| 52 | A | CH₃ | CH₃ | Br | CH₃ | H | - | lone pair | S | 0 | double bond | CH | CH |
| 53 | A | CH₃ | CH₃ | H | CH₃ | H | 8-CH₃ | lone pair | O | 1 | double bond | CH | CH |
| 54 | A | CH₃ | CH₃ | H | CH₃ | H | 8-OCH₃ | lone pair | O | 1 | double bond | CH | CH |
| 55 | A | CH₃ | CH₃ | CH₃ | CH₃ | CH₃ | 8-CH₃ | lone pair | O | 1 | double bond | CH | CH |
| 56 | A | CH₃ | CH₃ | CH₃ | CH₃ | H | 7-CH₃ | lone pair | O | 1 | double bond | CH | CH |
| 57 | A | CH₃ | CH₃ | H | Cl | H | 7-CH₃ | lone pair | O | 1 | double bond | CH | CH |
| 58 | A | CH₃ | CH₃ | H | CH₃ | H | 7-CH₃ | lone pair | O | 1 | double bond | CH | CH |
| 59 | A | CH₃ | CH₃ | H | F | H | 7-CH₃ | lone pair | O | 1 | double bond | CH | CH |
| 60 | A | CH₃ | CH₃ | Br | CH₃ | H | - | lone pair | SO₂ | 0 | double bond | CH | CH |
| 61 | A | CH₃ | CH₃ | H | CH₃ | H | - | lone pair | S | 0 | double bond | CH | CH |
| 62 | A | CH₃ | CH₃ | CH₃ | CH₃ | H | - | lone pair | SO₂ | 0 | double bond | CH | CH |
| 63 | A | CH₃ | CH₃ | CH₃ | CH₃ | H | - | lone pair | SO | 0 | double bond | CH | CH |
| 64 | A | CH₃ | CH₃ | H | Br | H | 7-CH₃ | lone pair | O | 1 | double bond | CH | CH |
| 65 | A | CH₃ | CH₃ | H | Br | H | 8-CH₃ | lone pair | O | 1 | double bond | CH | CH |
| 66 | A | CH₃ | CH₃ | H | CH₃ | H | 7-OCH₃ | lone pair | O | 1 | double bond | CH | CH |
| 67 | A | CH₃ | CH₃ | H | Cl | H | 7-OCH₃ | lone pair | O | 1 | double bond | CH | CH |
| 68 | A | CH₃ | CH₃ | H | cyclopropyl | H | 7-CH₃ | lone pair | O | 1 | double bond | CH | CH |
| 69 | A | CH₃ | CH₃ | H | CH₂CH₃ | H | 8-CH₃ | lone pair | O | 1 | double bond | CH | CH |

**[Table 7-3]**

| | | | | | | | | | | | | | |
|---|---|---|---|---|---|---|---|---|---|---|---|---|---|
| 70 | A | CH₃ | CH₃ | H | cyclopropyl | H | 8-CH₃ | lone pair | O | 1 | double bond | CH | CH |
| 71 | B | CH₃ | CH₃ | CH₃ | H | H | 8-CH₃ | lone pair | O | 1 | double bond | N | CCH₃ |
| 72 | A | CH₃ | CH₃ | H | -CH=CH₂ | H | 8-CH₃ | lone pair | O | 1 | double bond | CH | CH |
| 73 | A | CH₃ | CH₃ | H | -C(CH₃)=CH₂ | H | 8-CH₃ | lone pair | O | 1 | double bond | CH | CH |
| 74 | B | CH₃ | CH₃ | CH₃ | CH₃ | H | 8-CH₃ | lone pair | O | 1 | double bond | N | CH |
| 75 | A | CH₃ | CH₃ | CH₃ | CH₃ | H | 8-CH₃ | lone pair | S | 1 | double bond | CH | CH |
| 76 | A | CH₃ | CH₃ | CH₃ | CH₃ | H | 8-CH₃ | lone pair | SO₂ | 1 | double bond | CH | CH |
| 77 | A | CH₃ | CH₃ | CH₃ | CH₃ | H | 8-CH₃ | lone pair | SO | 1 | double bond | CH | CH |
| 78 | A | CH₃ | CH₃ | CH₃ | CH₃ | H | 8-F | lone pair | S | 1 | double bond | CH | CH |
| 79 | A | CH₃ | CH₃ | CH₃ | CH₃ | H | 8-F | lone pair | SO₂ | 1 | double bond | CH | CH |
| 80 | A | CH₃ | CH₃ | CH₃ | CH₃ | H | 8-F | lone pair | SO | 1 | double bond | CH | CH |
| 81 | A | CH₃ | CH₃ | H | CH₃ | H | 8-F | lone pair | S | 1 | double bond | CH | CH |
| 82 | A | CH₃ | CH₃ | H | CH₃ | H | 8-F | lone pair | SO₂ | 1 | double bond | CH | CH |
| 83 | A | CH₃ | CH₃ | H | CH₃ | H | 8-F | lone pair | SO | 1 | double bond | CH | CH |
| 84 | A | CH₃ | CH₃ | Cl | CH₃ | H | 8-F | lone pair | S | 1 | double bond | CH | CH |
| 85 | A | CH₃ | CH₃ | CH₃ | CH₃ | H | 7-Cl | lone pair | O | 1 | double bond | CH | CH |
| 86 | A | CH₃ | CH₃ | H | CH₃ | H | 7-Br | lone pair | O | 1 | double bond | CH | CH |
| 87 | A | CH₃ | CH₃ | H | F | H | 7-Br | lone pair | O | 1 | double bond | CH | CH |
| 88 | A | CH₃ | CH₃ | H | Cl | H | 7-Br | lone pair | O | 1 | double bond | CH | CH |
| 89 | A | CH₃ | CH₃ | Cl | CH₃ | H | 8-F | lone pair | SO₂ | 1 | double bond | CH | CH |
| 90 | A | CH₃ | CH₃ | CH₃ | H | H | 8-CH₃ | lone pair | O | 1 | double bond | CH | CH |
| 91 | A | CH₃ | CH₃ | CH₃ | H | H | 7-CH₃ | lone pair | O | 1 | double bond | CH | CH |
| 92 | A | CH₃ | CH₃ | CH₃ | CH₃ | H | 6-Cl | lone pair | O | 1 | double bond | CH | CH |
| 93 | C | CH₃ | CH₃ | H | CH₃ | H | - | lone pair | O | 0 | double bond | CH | CH |
| 94 | A | CH₃ | CH₃ | CH₃ | CH₃ | H | 8-Br | lone pair | O | 1 | double bond | CH | CH |
| 95 | A | CH₃ | CH₃ | CH₃ | CH₃ | H | 8-cyclopropyl | lone pair | O | 1 | double bond | CH | CH |
| 96 | A | CH₃ | CH₃ | CH₃ | CH₃ | H | 7-CH₃ | lone pair | S | 1 | double bond | CH | CH |
| 97 | A | CH₃ | CH₃ | CH₃ | CH₃ | H | 7-CH₃ | lone pair | SO₂ | 1 | double bond | CH | CH |
| 98 | A | CH₃ | CH₃ | CH₃ | CH₃ | H | 7-F | lone pair | S | 1 | double bond | CH | CH |
| 99 | A | CH₃ | CH₃ | CH₃ | CH₃ | H | 7-F | lone pair | SO₂ | 1 | double bond | CH | CH |
| 100 | A | CH₃ | CH₃ | CH₃ | CH₃ | H | 7-F | lone pair | SO | 1 | double bond | CH | CH |
| 101 | A | CH₃ | CH₃ | CH₃ | CH₃ | H | 7-CH₂CH₃ | lone pair | O | 1 | double bond | CH | CH |
| 102 | A | COCH₃ | CH₃ | CH₃ | CH₃ | H | 8-CH₃ | lone pair | O | 1 | double bond | CH | CH |
| 103 | A | COCH₃ | CH₃ | H | Cl | H | 8-CH₃ | lone pair | O | 1 | double bond | CH | CH |
| 104 | A | COCH₃ | CH₃ | H | CH₃ | H | 8-CH₃ | lone pair | O | 1 | double bond | CH | CH |

**[Table 7-4]**

| | | | | | | | | | | | | | |
|---|---|---|---|---|---|---|---|---|---|---|---|---|---|
| 105 | A | COCH₃ | CH₃ | H | F | H | 8-CH₃ | lone pair | O | 1 | double bond | CH | CH |
| 106 | A | CH₃ | CH₃ | H | CH₃ | H | 7-F | lone pair | S | 1 | double bond | CH | CH |
| 107 | A | CH₃ | CH₃ | Cl | CH₃ | H | 8-F | lone pair | S | 1 | double bond | N | CH |
| 108 | A | CH₃ | CH₃ | Cl | CH₃ | H | 7-F | lone pair | S | 1 | double bond | N | CH |
| 109 | A | CH₃ | CH₃ | Cl | CH₃ | H | 7-OCH₃ | lone pair | O | 1 | double bond | N | CH |
| 110 | A | CH₃ | CH₃ | Cl | CH₃ | H | 7-CH₃ | lone pair | O | 1 | double bond | N | CH |
| 111 | A | CH₃ | CH₃ | Cl | CH₃ | H | 8-OCH₃ | lone pair | O | 1 | double bond | N | CH |
| 112 | A | CH₃ | CH₃ | CH₃ | CH₃ | H | 7-NHC(=O)OtBu | lone pair | O | 1 | double bond | CH | CH |
| 113 | A | CH₂CH₃ | CH₃ | CH₃ | CH₃ | H | 8-CH₃ | lone pair | O | 1 | double bond | CH | CH |
| 114 | A | CH₂CH₂CH₃ | CH₃ | CH₃ | CH₃ | H | 8-CH₃ | lone pair | O | 1 | double bond | CH | CH |
| 115 | A | CH₂CH₃ | CH₃ | H | Cl | H | 8-CH₃ | lone pair | O | 1 | double bond | CH | CH |
| 116 | A | CH₂CH₃ | CH₃ | H | CH₃ | H | 8-CH₃ | lone pair | O | 1 | double bond | CH | CH |
| 117 | A | CH₂CH₂CH₃ | CH₃ | H | Cl | H | 8-CH₃ | lone pair | O | 1 | double bond | CH | CH |
| 118 | A | CH₂CH₂CH₃ | CH₃ | H | CH₃ | H | 8-CH₃ | lone pair | O | 1 | double bond | CH | CH |
| 119 | A | -CH₂CH₂CH₂- | | CH₃ | CH₃ | H | 8-CH₃ | lone pair | O | 1 | double bond | CH | CH |
| 120 | A | -CH₂CH₂CH₂- | | H | CH₃ | H | 8-CH₃ | lone pair | O | 1 | double bond | CH | CH |
| 121 | A | -CH₂CH₂CH₂- | | H | Cl | H | 8-CH₃ | lone pair | O | 1 | double bond | CH | CH |
| 122 | A | CH₃ | CH₃ | CH₃ | CH₃ | H | 7-N(CH₃)₂ | lone pair | O | 1 | double bond | CH | CH |
| 123 | A | -CH₂CH₂CH₂CH₂- | | H | CH₃ | H | 8-CH₃ | lone pair | O | 1 | double bond | CH | CH |
| 124 | A | -CH₂CH₂CH₂CH₂- | | CH₃ | CH₃ | H | 8-CH₃ | lone pair | O | 1 | double bond | CH | CH |
| 125 | A | CH₃ | CH₃ | CH₃ | CH₃ | H | 7-NHCH₃ | lone pair | O | 1 | double bond | CH | CH |
| 126 | A | CH₃ | CH₃ | CH₃ | CH₃ | H | 7-NH(CH₂CH₃) | lone pair | O | 1 | double bond | CH | CH |
| 127 | A | CH₃ | CH₃ | CH₃ | CH₃ | H | 7-N(CH₃)(CH₂CH₃) | lone pair | O | 1 | double bond | CH | CH |
| 128 | C | CH₃ | CH₃ | H | CH₃ | H | 8-CH₃ | lone pair | O | 1 | double bond | CH | CH |
| 129 | C | CH₃ | CH₃ | CH₃ | CH₃ | H | 8-CH₃ | lone pair | O | 1 | double bond | CH | CH |
| 130 | A | CH₃ | CH₃ | CH₃ | CH₃ | H | 8-F,7-F | lone pair | O | 2 | double bond | CH | CH |
| 131 | A | CH₂CH₃ | CH₃ | CH₃ | CH₃ | H | 7-CH₃ | lone pair | O | 1 | double bond | CH | CH |
| 132 | A | -CH₂CH₂CH₂CH₂CH₂- | | CH₃ | CH₃ | H | 8-CH₃ | lone pair | O | 1 | double bond | CH | CH |
| 133 | A | CH₂CH₃ | CH₃ | H | CH₃ | H | 7-CH₃ | lone pair | O | 1 | double bond | CH | CH |
| 134 | A | -CH₂CH₂CH₂CH₂CH₂- | | H | CH₃ | H | 8-CH₃ | lone pair | O | 1 | double bond | CH | CH |
| 135 | A | CH₂CH₂CH₂CH₃ | CH₃ | CH₃ | CH₃ | H | 8-CH₃ | lone pair | O | 1 | double bond | CH | CH |
| 136 | A | CH₃ | CH₃ | H | CH₃ | H | 8-F,7-F | lone pair | O | 1 | double bond | CH | CH |
| 137 | A | CH₃ | CH₃ | H | Cl | H | 8-F,7-F | lone pair | O | 1 | double bond | CH | CH |
| 138 | A | CH₂CH₂CH₃ | CH₃ | CH₃ | CH₃ | H | 7-CH₃ | lone pair | O | 1 | double bond | CH | CH |
| 139 | A | CH₂CH₂CH₂CH₃ | CH₃ | CH₃ | CH₃ | H | 7-CH₃ | lone pair | O | 1 | double bond | CH | CH |

**[Table 7-5]**

| | | | | | | | | | | | | | |
|---|---|---|---|---|---|---|---|---|---|---|---|---|---|
| 140 | A | -CH₂CH₂CH₂CH₂- | | CH₃ | CH₃ | H | 7-CH₃ | lone pair | O | 1 | double bond | CH | CH |
| 141 | A | -CH₂CH₂CH₂CH₂CH₂- | | CH₃ | CH₃ | H | 7-CH₃ | lone pair | O | 1 | double bond | CH | CH |
| 142 | B | CH₃ | CH₃ | H | CH₃ | H | 8-CH₃ | lone pair | O | 1 | double bond | CH | CH |
| 143 | A | CH₃ | CH₃ | CH₃ | CH₃ | H | 8-F,7-CH₃ | lone pair | O | 2 | double bond | CH | CH |
| 144 | C | CH₃ | CH₃ | H | Cl | H | 8-CH₃ | lone pair | O | 1 | double bond | CH | CH |
| 145 | A | CH₃ | CH₃ | CH₃ | CH₃ | H | 7-OCH₃ | lone pair | S | 1 | double bond | CH | CH |
| 146 | A | -CH₂CH₂CH₂- | | CH₃ | CH₃ | H | 7-CH₃ | lone pair | O | 1 | double bond | CH | CH |
| 147 | A | CH₂OCH₃ | CH₃ | CH₃ | CH₃ | H | 7-CH₃ | lone pair | O | 1 | double bond | CH | CH |
| 148 | A | CH₂CH₂CH₂CH₃ | CH₃ | CH₃ | CH₃ | H | 8-F,7-CH₃ | lone pair | O | 2 | double bond | CH | CH |
| 149 | A | CH₃ | CH₃ | CH₃ | CH₃ | H | 8-CH₃,7-CH₃ | lone pair | O | 2 | double bond | CH | CH |
| 150 | A | CH₃ | CH₃ | H | Cl | H | 8-CH₃,7-CH₃ | lone pair | O | 2 | double bond | CH | CH |
| 151 | A | CH₂(CH₂)₃CH₃ | CH₃ | CH₃ | CH₃ | H | 7-CH₃ | lone pair | O | 1 | double bond | CH | CH |
| 152 | A | CH₂CH(CH₃)CH₃ | CH₃ | CH₃ | CH₃ | H | 7-CH₃ | lone pair | O | 1 | double bond | CH | CH |
| 153 | B | CH₃ | CH₃ | CH₃ | CH₃ | H | 8-CH₃ | lone pair | O | 1 | double bond | CH | CH |
| 154 | B | CH₃ | CH₃ | H | CH₃ | H | 7-CH₃ | lone pair | O | 1 | double bond | CH | CH |
| 155 | A | CH₂CH₃ | CH₃ | CH₃ | CH₃ | H | 8-F | lone pair | S | 1 | double bond | CH | CH |
| 156 | A | CH₂CH₂CH₂CH₃ | CH₃ | CH₃ | CH₃ | H | 7-Cl | lone pair | O | 1 | double bond | CH | CH |
| 157 | A | CH₃ | CH₃ | H | OCH₃ | H | 8-CH₃,7-CH₃ | lone pair | O | 2 | double bond | CH | CH |
| 158 | A | CH₃ | CH₃ | H | F | H | 8-CH₃,7-CH₃ | lone pair | O | 2 | double bond | CH | CH |
| 159 | B | CH₃ | CH₃ | H | CH₃ | H | 8-F | lone pair | O | 1 | double bond | CH | CH |
| 160 | B | CH₃ | CH₃ | CH₃ | CH₃ | H | 7-CH₃ | lone pair | O | 1 | double bond | CH | CH |
| 161 | B | CH₃ | CH₃ | H | H | H | 7-CH₃ | lone pair | O | 1 | double bond | CH | CH |
| 162 | B | CH₃ | CH₃ | CH₃ | CH₃ | H | 8-F | lone pair | O | 1 | double bond | CH | CH |
| 163 | A | cyclobutyl | CH₃ | CH₃ | CH₃ | H | 7-CH₃ | lone pair | O | 1 | double bond | CH | CH |
| 164 | A | cyclopropyl | CH₃ | CH₃ | CH₃ | H | 7-CH₃ | lone pair | O | 1 | double bond | CH | CH |
| 165 | A | CH(CH₃)₂ | CH₃ | CH₃ | CH₃ | H | 7-CH₃ | lone pair | O | 1 | double bond | CH | CH |
| 166 | A | C(CH₃)₃ | CH₃ | CH₃ | CH₃ | H | 7-CH₃ | lone pair | O | 1 | double bond | CH | CH |
| 167 | A | CH₂Cl | CH₃ | CH₃ | CH₃ | H | 7-CH₃ | lone pair | O | 1 | double bond | CH | CH |
| 168 | A | CH₂CH₂CH₃ | CH₃ | CH₃ | CH₃ | H | 7-F | lone pair | O | 1 | double bond | CH | CH |
| 169 | A | CH₂CH₃ | CH₃ | CH₃ | CH₃ | H | 7-F | lone pair | O | 1 | double bond | CH | CH |
| 170 | A | CH₃ | CH₃ | CH₃ | CH₃ | H | 8-CH₃,6-CH₃ | lone pair | O | 2 | double bond | CH | CH |
| 171 | B | CH₃ | CH₃ | CH₃ | CH₃ | H | 8-Cl | lone pair | O | 1 | double bond | CH | CH |
| 172 | B | CH₃ | CH₃ | CH₃ | CH₃ | H | - | lone pair | O | 0 | double bond | CH | CH |
| 173 | B | CH₃ | CH₃ | CH₃ | CH₃ | H | 7-F | lone pair | O | 1 | double bond | CH | CH |
| 174 | B | CH₃ | CH₃ | CH₃ | CH₃ | H | 8-Br | lone pair | O | 1 | double bond | CH | CH |

**[Table 7-6]**

| | | | | | | | | | | | | | |
|---|---|---|---|---|---|---|---|---|---|---|---|---|---|
| 175 | B | CH₃ | CH₃ | CH₃ | CH₃ | H | 8-cyclopropyl | lone pair | O | 1 | double bond | CH | CH |
| 176 | A | CH₂C(O)OCH₂CH₃ | CH₃ | CH₃ | CH₃ | H | 7-CH₃ | lone pair | O | 1 | double bond | CH | CH |
| 177 | A | CH₂C(O)OH | CH₃ | CH₃ | CH₃ | H | 7-CH₃ | lone pair | O | 1 | double bond | CH | CH |
| 178 | B | CH₃ | CH₃ | CH₃ | CH₃ | H | 7-OCH₃ | lone pair | O | 1 | double bond | CH | CH |
| 179 | B | CH₃ | CH₃ | CH₃ | CH₃ | H | 8-CH₂CH₃ | lone pair | O | 1 | double bond | CH | CH |
| 180 | B | CH₃ | CH₃ | H | CH₂CH₃ | H | 7-CH₃ | lone pair | O | 1 | double bond | CH | CH |
| 181 | B | CH₃ | CH₃ | | -CH₂CH₂CH₂- | H | 7-CH₃ | lone pair | O | 1 | double bond | CH | CH |
| 182 | B | CH₃ | CH₃ | CH₃ | CH₃ | H | 8-CH₃,7-CH₃ | lone pair | O | 2 | double bond | CH | CH |
| 183 | A | CH₂CH(CH₃)CH₃ | CH₃ | CH₃ | CH₃ | H | 7-F | lone pair | O | 1 | double bond | CH | CH |
| 184 | A | CH₃ | CH₃ | CH₃ | CH₃ | H | 8-F,7-Cl | lone pair | O | 2 | double bond | CH | CH |
| 185 | B | CH₃ | CH₃ | CH₃ | CH₃ | H | 8-CH=CH₂ | lone pair | O | 1 | double bond | CH | CH |
| 186 | B | CH₃ | CH₃ | CH₃ | CH₃ | H | 6-F | lone pair | O | 1 | double bond | CH | CH |
| 187 | B | CH₃ | CH₃ | H | CH₃ | H | 8-CH₃,7-CH₃ | lone pair | O | 2 | double bond | CH | CH |
| 188 | A | CH₂C(O)NH₂ | CH₃ | CH₃ | CH₃ | H | 7-CH₃ | lone pair | O | 1 | double bond | CH | CH |
| 189 | A | CH(CH₃)CH₂CH₃ | CH₃ | CH₃ | CH₃ | H | 7-CH₃ | lone pair | O | 1 | double bond | CH | CH |
| 190 | A | CH₂C(O)OCH₃ | CH₃ | CH₃ | CH₃ | H | 7-CH₃ | lone pair | O | 1 | double bond | CH | CH |
| 191 | A | CH₃ | CH₃ | H | Cl | H | 8-CH₃,6-CH₃ | lone pair | O | 2 | double bond | CH | CH |
| 192 | A | CH₃ | CH₃ | CH₃ | CH₃ | H | 8-CH₃,5-CH₃ | lone pair | O | 2 | double bond | CH | CH |
| 193 | A | CH₂(CH₂)₃CH₃ | CH₃ | CH₃ | CH₃ | H | 7-F | lone pair | O | 1 | double bond | CH | CH |
| 194 | B | CH₃ | CH₃ | CH₃ | CH₃ | H | 8-CH₂CH₂CH₃ | lone pair | O | 1 | double bond | CH | CH |
| 195 | B | CH₃ | CH₃ | CH₃ | CH₃ | H | 8-C(CH₃)=CH₂ | lone pair | O | 1 | double bond | CH | CH |
| 196 | A | CH₂C(O)NHCH₃ | CH₃ | CH₃ | CH₃ | H | 7-CH₃ | lone pair | O | 1 | double bond | CH | CH |
| 197 | A | CH₂CH₂OCH₃ | CH₃ | CH₃ | CH₃ | H | 7-CH₃ | lone pair | O | 1 | double bond | CH | CH |
| 198 | B | CH₃ | CH₃ | H | CH₃ | H | 8-CH₃,7-Cl | lone pair | O | 2 | double bond | CH | CH |
| 199 | A | CH₂C(O)OCH₃ | CH₃ | CH₃ | CH₃ | H | 7-CH₃ | lone pair | O | 1 | double bond | CH | CH |
| 200 | A | CH₂C(O)OCH₂CH₃ | CH₃ | CH₃ | CH₃ | H | 7-CH₃ | lone pair | O | 1 | double bond | CH | CH |
| 201 | A | CH₂SCH₃ | CH₃ | CH₃ | CH₃ | H | 7-CH₃ | lone pair | O | 1 | double bond | CH | CH |
| 202 | B | CH₃ | CH₃ | CH₃ | CH₃ | H | 7-Cl | lone pair | O | 1 | double bond | CH | CH |
| 203 | B | CH₃ | CH₃ | CH₃ | CH₃ | H | B-CH₃,7-Cl | lone pair | O | 2 | double bond | CH | CH |
| 204 | B | CH₃ | CH₃ | CH₃ | CH₃ | H | 8-F,7-F | lone pair | O | 2 | double bond | CH | CH |
| 205 | B | CH₃ | CH₃ | H | CH₃ | H | 8-F,7-F | lone pair | O | 2 | double bond | CH | CH |
| 206 | B | CH₃ | CH₃ | CH₃ | CH₃ | H | 8-F | lone pair | S | 1 | double bond | CH | CH |
| 207 | B | CH₃ | CH₃ | CH₃ | CH₃ | H | - | lone pair | S | 0 | double bond | CH | CH |
| 208 | B | CH₃ | CH₃ | H | CH₃ | H | - | lone pair | S | 0 | double bond | CH | CH |
| 209 | B | CH₃ | CH₃ | H | H | H | - | lone pair | O | 0 | double bond | CH | CH |

**[Table 7-7]**

| | | | | | | | | | | | | | |
|---|---|---|---|---|---|---|---|---|---|---|---|---|---|
| 210 | B | CH₃ | CH₃ | CH₃ | H | H | 7-CH₃ | lone pair | O | 1 | double bond | CH | CH |
| 211 | A | CH₂S(O)2CH₃ | CH₃ | CH₃ | CH₃ | H | 7-CH₃ | lone pair | O | 1 | double bond | CH | CH |
| 212 | B | CH₂OCH₃ | CH₃ | CH₃ | CH₃ | H | 7-CH₃ | lone pair | O | 1 | double bond | CH | CH |
| 213 | B | CH₂CH₂CH₂CH₃ | CH₃ | CH₃ | CH₃ | H | 8-CH₃ | lone pair | O | 1 | double bond | CH | CH |
| 214 | B | CH₃ | CH₃ | CH₃ | CH₃ | H | - | lone pair | S | 0 | double bond | CH | N |
| 215 | B | CH₃ | CH₃ | CH₃ | CH₃ | H | - | lone pair | SO₂ | 0 | double bond | CH | CH |
| 216 | B | CH₃ | CH₃ | CH₃ | CH₃ | H | - | lone pair | SO₂ | 0 | double bond | CH | N |
| 217 | B | CH₂CH₂CH₃ | CH₃ | CH₃ | CH₃ | H | 8-CH₃ | lone pair | O | 1 | double bond | CH | CH |
| 218 | B | CH₃ | CH₃ | CH₃ | CH₃ | H | 8-F,7-CH₃ | lone pair | O | 2 | double bond | CH | CH |
| 219 | B | CH₃ | CH₃ | H | CH₃ | H | 8-F,7-CH₃ | lone pair | O | 2 | double bond | CH | CH |
| 220 | B | CH₂OCH₃ | CH₃ | CH₃ | CH₃ | H | - | lone pair | S | 0 | double bond | CH | CH |
| 221 | B | CH₂OCH₃ | CH₃ | CH₃ | CH₃ | H | - | lone pair | SO₂ | 0 | double bond | CH | CH |
| 222 | B | CH₃ | CH₃ | CH₃ | CH₃ | H | 7-Br | lone pair | O | 1 | double bond | CH | CH |
| 223 | B | CH₂CH₂CH₃ | CH₃ | CH₃ | CH₃ | H | - | lone pair | O | 0 | double bond | CH | CH |
| 224 | B | CH₂CH₂CH₂CH₃ | CH₃ | CH₃ | CH₃ | H | - | lone pair | O | 0 | double bond | CH | CH |
| 225 | B | CH₂CH₂CH₂CH₃ | CH₃ | H | CH₃ | H | - | lone pair | O | 0 | double bond | CH | CH |
| 226 | B | CH₂CH₂CH₂CH₃ | CH₃ | CH₃ | H | H | - | lone pair | O | 0 | double bond | CH | CH |
| 227 | B | CH₃ | CH₃ | H | CH₃ | H | 8-F,7-OCH₃ | lone pair | O | 2 | double bond | CH | CH |
| 228 | B | CH₃ | CH₃ | CH₃ | CH₃ | H | 8-F,7-OCH₃ | lone pair | O | 2 | double bond | CH | CH |
| 229 | B | CH₃ | CH₃ | CH₃ | CH₃ | H | 7-CH₃ | lone pair | O | 1 | double bond | CH | N |
| 230 | B | CH₂CH₂CH₃ | CH₃ | H | CH₃ | H | - | lone pair | O | 0 | double bond | CH | CH |
| 231 | B | CH₂CH₂CH₃ | CH₃ | CH₃ | H | H | - | lone pair | O | 0 | double bond | CH | CH |
| 232 | B | CH₃ | CH₃ | CH₃ | CH₃ | H | 8-F,7-Br | lone pair | O | 2 | double bond | CH | CH |
| 233 | B | CH₂CH₂CH₂CH₃ | CH₃ | CH₃ | CH₃ | H | - | lone pair | S | 0 | double bond | CH | CH |
| 234 | B | CH₂CH₂CH₂CH₃ | CH₃ | H | H | H | - | lone pair | S | 0 | double bond | CCH₃ | CH |
| 235 | B | CH₂CH₂CH₂CH₃ | CH₃ | CH₃ | CH₃ | H | - | lone pair | S | 0 | double bond | CH | N |
| 236 | B | CH₂CH₂CH₂CH₃ | CH₃ | H | H | H | - | lone pair | S | 0 | double bond | CCH₃ | N |
| 237 | B | CH₂CH₂CH₂CH₃ | CH₃ | CH₃ | CH₃ | H | - | lone pair | SO₂ | 0 | double bond | CH | CH |
| 238 | B | CH₂CH₂CH₂CH₃ | CH₃ | CH₃ | CH₃ | H | - | lone pair | SO₂ | 0 | double bond | CH | N |
| 239 | A | CH₂OCH₃ | CH₃ | CH₃ | CH₃ | H | - | lone pair | O | 0 | double bond | CH | CH |
| 240 | B | CH₂CH₂CH₂CH₃ | CH₃ | H | H | H | - | lone pair | O | 0 | double bond | CCH₃ | CH |
| 241 | B | CH₂CH₂CH₂CH₃ | CH₃ | CH₃ | CH₃ | H | - | lone pair | O | 0 | double bond | CH | N |
| 242 | B | CH₂CH₂CH₂CH₃ | CH₃ | H | H | H | - | lone pair | O | 0 | double bond | CCH₃ | N |
| 243 | B | CH₂CH₂CH₃ | CH₃ | CH₃ | CH₃ | H | - | lone pair | O | 0 | double bond | CH | N |
| 244 | B | CH₂CH₂CH₂CH₃ | CH₃ | CH₃ | H | H | - | lone pair | O | 0 | double bond | N | CH |

**[Table 7-8]**

| | | | | | | | | | | | | | |
|---|---|---|---|---|---|---|---|---|---|---|---|---|---|
| 245 | B | CH₃ | CH₃ | CH₃ | CH₃ | H | 8-CH₃,7-F | lone pair | O | 2 | double bond | CH | CH |
| 246 | B | CH₃ | CH₃ | CH₃ | CH₃ | H | 8-F,7-Cl | lone pair | O | 2 | double bond | CH | CH |
| 247 | B | CH₃ | CH₃ | H | CH₃ | H | 8-CH₃,7-F | lone pair | O | 2 | double bond | CH | CH |
| 248 | B | CH₃ | CH₃ | CH₃ | H | H | - | lone pair | O | 0 | double bond | N | CH |
| 249 | A | CH₂CH₂CH₂CH₃ | CH₃ | CH₃ | CH₃ | H | - | lone pair | O | 0 | double bond | CH | CH |
| 250 | B | CH₂CH₃ | CH₃ | CH₃ | CH₃ | H | - | lone pair | O | 0 | double bond | CH | CH |
| 251 | B | CH₂CH(CH₃)CH₃ | CH₃ | CH₃ | CH₃ | H | - | lone pair | O | 0 | double bond | CH | CH |
| 252 | B | CH₂CH(CH₃)CH₃ | CH₃ | H | H | H | - | lone pair | O | 0 | double bond | CCH₃ | CH |
| 253 | B | CH₂CH(CH₃)CH₃ | CH₃ | H | CH₃ | H | - | lone pair | O | 0 | double bond | CH | CH |
| 254 | B | CH₂CH(CH₃)CH₃ | CH₃ | H | H | H | - | lone pair | O | 0 | double bond | CH | CH |
| 255 | B | CH₂CH(CH₃)CH₃ | CH₃ | CH₃ | H | H | - | lone pair | O | 0 | double bond | CH | CH |
| 256 | B | CH₂CH₂CH₂CH₃ | CH₃ | CH₃ | CH₃ | H | 8-F,7-CH₃ | lone pair | O | 2 | double bond | CH | CH |
| 257 | B | CH₂CH₂CH₂CH₃ | CH₃ | H | CH₃ | H | 8-F,7-CH₃ | lone pair | O | 2 | double bond | CH | CH |
| 258 | B | CH₂CH₂CH₂CH₃ | CH₃ | CH₃ | CH₃ | H | 8-F,7-CH₃ | lone pair | O | 2 | double bond | CH | N |
| 259 | B | CH₂CH₂CH₂CH₃ | CH₃ | H | H | H | 8-F,7-CH₃ | lone pair | O | 2 | double bond | CCH₃ | N |
| 260 | B | CH₂CH₃ | CH₃ | H | H | H | - | lone pair | O | 0 | double bond | CCH₃ | CH |
| 261 | B | CH₂CH₃ | CH₃ | H | CH₃ | H | - | lone pair | O | 0 | double bond | CH | CH |
| 262 | B | CH₂CH₃ | CH₃ | H | H | H | - | lone pair | O | 0 | double bond | CH | CH |
| 263 | B | CH₂CH₃ | CH₃ | CH₃ | CH₃ | H | - | lone pair | S | 0 | double bond | CH | CH |
| 264 | B | CH₂CH₃ | CH₃ | H | H | H | - | lone pair | S | 0 | double bond | CCH₃ | CH |
| 265 | B | CH₂CH₃ | CH₃ | H | CH₃ | H | - | lone pair | S | 0 | double bond | CH | CH |
| 266 | B | CH₂CH₃ | CH₃ | H | H | H | - | lone pair | S | 0 | double bond | CH | CH |
| 267 | B | CH₂CH(CH₃)CH₃ | CH₃ | CH₃ | H | H | 7-CH₃ | lone pair | O | 1 | double bond | N | CH |
| 268 | B | CH₂CH(CH₃)CH₃ | CH₃ | CH₃ | CH₃ | H | - | lone pair | O | 0 | double bond | CH | N |
| 269 | B | CH₂CH₃ | CH₃ | CH₃ | CH₃ | H | - | lone pair | O | 0 | double bond | CH | N |
| 270 | B | CH₂CH₃ | CH₃ | CH₃ | H | H | - | lone pair | O | 0 | double bond | CH | CH |
| 271 | B | CH₂CH₃ | CH₃ | CH₃ | CH₃ | H | - | lone pair | SO₂ | 0 | double bond | CH | CH |
| 272 | B | CH₂CH₃ | CH₃ | CH₃ | CH₃ | H | - | lone pair | S | 0 | double bond | CH | N |
| 273 | B | CH₂CH₃ | CH₃ | H | H | H | - | lone pair | S | 0 | double bond | CCH₃ | N |
| 274 | B | CH₂CH₃ | CH₃ | H | CH₃ | H | - | lone pair | SO₂ | 0 | double bond | CH | CH |
| 275 | B | CH₃ | CH₃ | H | H | H | 7-CH₃ | lone pair | O | 1 | double bond | CCH₃ | CH |
| 276 | B | CH₂CH₃ | CH₃ | CH₃ | CH₃ | H | - | lone pair | SO2 | 0 | double bond | CH | N |
| 277 | B | CH₂CH₃ | CH₃ | CH₃ | H | H | - | lone pair | S | 0 | double bond | CH | CH |
| 278 | B | CH₂CH₂CH₃ | CH₃ | CH₃ | CH₃ | H | - | lone pair | S | 0 | double bond | CH | CH |
| 279 | B | CH₂CH₂CH₃ | CH₃ | H | H | H | - | lone pair | S | 0 | double bond | CCH₃ | CH |

**[Table 7-9]**

| | | | | | | | | | | | | | |
|---|---|---|---|---|---|---|---|---|---|---|---|---|---|
| 280 | B | CH₂CH₂CH₂CH₃ | CH₃ | CH₃ | CH₃ | H | 8-F | lone pair | O | 1 | double bond | CH | CH |
| 281 | B | CH₂CH₂CH₂CH₃ | CH₃ | H | H | H | 8-F | lone pair | O | 1 | double bond | CCH₃ | CH |
| 282 | B | CH₂OCH₃ | CH₃ | CH₃ | CH₃ | H | 8-F | lone pair | O | 1 | double bond | CH | CH |
| 283 | B | CH₂OCH₃ | CH₃ | H | H | H | 8-F | lone pair | O | 1 | double bond | CCH₃ | CH |
| 284 | B | CH₂OCH₃ | CH₃ | CH₃ | CH₃ | H | - | lone pair | O | 0 | double bond | CH | CH |
| 285 | B | CH₂OCH₃ | CH₃ | H | H | H | - | lone pair | O | 0 | double bond | CCH₃ | CH |
| 286 | B | CH₂CH₂CH₂CH₃ | CH₃ | H | CH₃ | H | 8-F | lone pair | O | 1 | double bond | CH | CH |
| 287 | B | CH₂CH₂CH₂CH₃ | CH₃ | H | H | H | 8-F | lone pair | O | 1 | double bond | CH | CH |
| 288 | B | CH₂OCH₃ | CH₃ | H | CH₃ | H | 8-F | lone pair | O | 1 | double bond | CH | CH |
| 289 | B | CH₂OCH₃ | CH₃ | H | H | H | 8-F | lone pair | O | 1 | double bond | CH | CH |
| 290 | B | CH₂OCH₃ | CH₃ | H | CH₃ | H | - | lone pair | O | 0 | double bond | CH | CH |
| 291 | B | CH₂OCH₃ | CH₃ | H | H | H | - | lone pair | O | 0 | double bond | CH | CH |
| 292 | C | CH₂CH₂CH₂CH₃ | CH₃ | CH₃ | CH₃ | H | - | lone pair | O | 0 | double bond | CH | CH |
| 293 | B | CH₂CH₂CH₂CH₃ | CH₃ | H | CH₃ | H | - | lone pair | O | 0 | double bond | N | CH |
| 294 | C | CH₂CH₂OCH₃ | CH₃ | CH₃ | CH₃ | H | - | lone pair | O | 0 | double bond | CH | CH |
| 295 | B | CH₂CH₂CH₂CH₃ | CH₃ | CH₃ | CH₃ | H | - | lone pair | O | 0 | double bond | N | CH |
| 296 | B | CH₃ | CH₃ | CH₃ | H | H | 8-F | lone pair | S | 1 | double bond | N | CH |
| 297 | B | CH₂CH₂CH₃ | CH₃ | CH₃ | H | H | - | lone pair | O | 0 | double bond | N | CH |
| 298 | B | CH₂CH₂CH₃ | CH₃ | H | CH₃ | H | - | lone pair | S | 0 | double bond | CH | CH |
| 299 | B | CH₂CH₂CH₃ | CH₃ | H | H | H | - | lone pair | S | 0 | double bond | CH | CH |
| 300 | B | CH₂CH₃ | CH₃ | CH₃ | H | H | - | lone pair | SO₂ | 0 | double bond | CH | CH |
| 301 | B | CH₂CH₂CH₃ | CH₃ | CH₃ | CH₃ | H | - | lone pair | SO₂ | 0 | double bond | CH | CH |
| 302 | B | CH₂CH₂CH₃ | CH₃ | CH₃ | H | H | - | lone pair | S | 0 | double bond | CH | CH |
| 303 | B | CH₂CH₂CH₃ | CH₃ | CH₃ | CH₃ | H | - | lone pair | O | 0 | double bond | N | CH |
| 304 | B | CH₂CH₂CH₃ | CH₃ | CH₃ | CH₃ | H | - | lone pair | S | 0 | double bond | CH | N |
| 305 | B | CH₂CH₂CH₃ | CH₃ | H | H | H | - | lone pair | S | 0 | double bond | CCH₃ | N |
| 306 | B | CH₂CH₂CH₃ | CH₃ | H | CH₃ | H | - | lone pair | SO₂ | 0 | double bond | CH | CH |
| 307 | B | CH₂CH₂CH₃ | CH₃ | CH₃ | H | H | - | lone pair | SO₂ | 0 | double bond | CH | CH |
| 308 | B | CH₂CH(CH₃)CH₃ | CH₃ | CH₃ | H | H | - | lone pair | O | 0 | double bond | N | CH |
| 309 | B | CH₂CH(CH₃)CH₃ | CH₃ | CH₃ | CH₃ | H | - | lone pair | O | 0 | double bond | N | CH |
| 310 | C | CH₂CH₂CH₂CH₃ | CH₃ | H | H | H | - | lone pair | O | 0 | double bond | N | CH |
| 311 | B | CH₂CH₃ | CH₃ | CH₃ | CH₃ | H | 8-F | lone pair | S | 1 | double bond | CH | CH |
| 312 | B | CH₂CH₃ | CH₃ | H | H | H | 8-F | lone pair | S | 1 | double bond | CCH₃ | CH |
| 313 | B | CH₂CH₃ | CH₃ | H | CH₃ | H | 8-F | lone pair | S | 1 | double bond | CH | CH |
| 314 | B | CH₃ | CH₃ | CH₃ | H | H | 7-F | lone pair | S | 1 | double bond | N | CH |

**[Table 7-10]**

| | | | | | | | | | | | | | |
|---|---|---|---|---|---|---|---|---|---|---|---|---|---|
| 315 | B | CH₂OCH₃ | CH₃ | CH₃ | H | H | - | lone pair | O | 0 | double bond | CH | CH |
| 316 | B | CH₂CH₂CH₂CH₃ | CH₃ | CH₃ | H | H | 8-F | lone pair | O | 1 | double bond | CH | CH |
| 317 | B | CH₂CH₂CH₃ | CH₃ | CH₃ | CH₃ | H | - | lone pair | SO₂ | 0 | double bond | CH | N |
| 318 | B | CH₂CH₃ | CH₃ | CH₃ | H | H | 8-F | lone pair | S | 1 | double bond | CH | CH |
| 319 | B | CH₂CH₃ | CH₃ | CH₃ | CH₃ | H | 8-F | lone pair | S | 1 | double bond | CH | N |
| 320 | B | CH₂CH₂CH₂CH₃ | CH₃ | CH₃ | CH₃ | H | 8-F | lone pair | O | 1 | double bond | CH | N |
| 321 | B | CH₃ | CH₃ | CH₃ | CH₃ | H | 8-F | lone pair | S | 1 | double bond | N | CH |
| 322 | B | CH₃ | CH₃ | CH₃ | CH₃ | H | 7-F | lone pair | S | 1 | double bond | N | CH |
| 323 | B | CH₂CH₃ | CH₃ | CH₃ | H | H | - | lone pair | O | 0 | double bond | N | CH |
| 324 | B | CH₂CH₂CH₃ | CH₃ | CH₃ | H | H | 7-CH₃ | lone pair | O | 1 | double bond | N | CH |
| 325 | B | CH₃ | CH₃ | H | CH₃ | H | 8-F | lone pair | S | 1 | double bond | CH | CH |
| 326 | B | CH₃ | CH₃ | CH₃ | H | H | 8-F | lone pair | S | 1 | double bond | CH | CH |
| 327 | B | CH₃ | CH₃ | H | H | H | 8-F | lone pair | S | 1 | double bond | CCH₃ | CH |
| 328 | B | CH₃ | CH₃ | CH₃ | CH₃ | H | 8-F | lone pair | S | 1 | double bond | CH | N |
| 329 | B | CH₃ | CH₃ | H | H | H | 8-F | lone pair | S | 1 | double bond | CCH₃ | N |
| 330 | B | CH₂(CH₂)₃CH₃ | CH₃ | CH₃ | CH₃ | H | - | lone pair | O | 0 | double bond | CH | CH |
| 331 | B | CH₂(CH₂)₃CH₃ | CH₃ | H | H | H | - | lone pair | O | 0 | double bond | CCH₃ | CH |
| 332 | B | CH₂OCH₂CH₃ | CH₃ | CH₃ | CH₃ | H | - | lone pair | O | 0 | double bond | CH | CH |
| 333 | B | CH₂OCH₂CH₃ | CH₃ | H | H | H | - | lone pair | O | 0 | double bond | CCH₃ | CH |
| 334 | B | CH₂(CH₂)₃CH₃ | CH₃ | CH₃ | H | H | - | lone pair | O | 0 | double bond | CH | CH |
| 335 | B | CH₂OCH₂CH₃ | CH₃ | CH₃ | H | H | - | lone pair | O | 0 | double bond | CH | CH |
| 336 | B | CH₂(CH₂)₃CH₃ | CH₃ | H | CH₃ | H | - | lone pair | O | 0 | double bond | CH | CH |
| 337 | B | CH₂(CH₂)₃CH₃ | CH₃ | H | H | H | - | lone pair | O | 0 | double bond | CH | CH |
| 338 | B | CH₂OCH₂CH₃ | CH₃ | H | CH₃ | H | - | lone pair | O | 0 | double bond | CH | CH |
| 339 | B | CH₂OCH₂CH₃ | CH₃ | H | H | H | - | lone pair | O | 0 | double bond | CH | CH |
| 340 | B | CH₂CH₂CH₂CH₃ | CH₃ | CH₃ | CH₃ | H | 8-F | lone pair | S | 1 | double bond | CH | CH |
| 341 | B | CH₂CH₂CH₂CH₃ | CH₃ | H | H | H | 8-F | lone pair | S | 1 | double bond | CCH₃ | CH |
| 342 | B | CH₂CH₂CH₂CH₃ | CH₃ | H | CH₃ | H | 8-F | lone pair | S | 1 | double bond | CH | CH |
| 343 | B | CH₂CH₂CH₂CH₃ | CH₃ | H | H | H | 8-F | lone pair | S | 1 | double bond | CH | CH |
| 344 | B | CH₂CH₂CH₃ | CH₃ | CH₃ | CH₃ | H | 7-F | lone pair | O | 1 | double bond | N | CH |
| 345 | B | CH₂CH₃ | CH₂CH₃ | CH₃ | CH₃ | H | - | lone pair | O | 0 | double bond | CH | CH |
| 346 | B | CH₂CH₂CH₃ | CH₂CH₃ | CH₃ | CH₃ | H | - | lone pair | O | 0 | double bond | CH | CH |
| 347 | B | CH₂CH₃ | CH₂CH₃ | CH₃ | H | H | - | lone pair | O | 0 | double bond | CH | CH |
| 348 | B | CH₂CH₃ | CH₂CH₃ | H | H | H | - | lone pair | O | 0 | double bond | CCH₃ | CH |
| 349 | B | CH₂CH₂CH₃ | CH₂CH₃ | CH₃ | H | H | - | lone pair | O | 0 | double bond | CH | CH |

**[Table 7-11]**

| | | | | | | | | | | | | | |
|---|---|---|---|---|---|---|---|---|---|---|---|---|---|
| 350 | B | CH₂CH₂CH₃ | CH₂CH₃ | H | H | H | - | lone pair | O | 0 | double bond | CCH₃ | CH |
| 351 | B | CH₂CH₃ | CH₂CH₃ | H | CH₃ | H | - | lone pair | O | 0 | double bond | CH | CH |
| 352 | B | CH₂CH₃ | CH₂CH₃ | H | H | H | - | lone pair | O | 0 | double bond | CH | CH |
| 353 | B | CH₂CH₂CH₃ | CH₂CH₃ | H | CH₃ | H | - | lone pair | O | 0 | double bond | CH | CH |
| 354 | B | CH₂CH₂CH₃ | CH₂CH₃ | H | H | H | - | lone pair | O | 0 | double bond | CH | CH |
| 355 | B | CH₂CH₃ | CH₃ | CH₃ | CH₃ | H | 7-F | lone pair | O | 1 | double bond | N | CH |
| 356 | B | CH₂CH(CH₃)CH₃ | CH₃ | CH₃ | CH₃ | H | 7-F | lone pair | O | 1 | double bond | N | CH |
| 357 | B | CH₂CH₂CH₂CH₃ | CH₃ | CH₃ | H | H | 8-F | lone pair | S | 1 | double bond | CH | CH |
| 358 | B | CH₂CH₂CH₂CH₃ | CH₃ | CH₃ | CH₃ | H | 8-F | lone pair | S | 1 | double bond | CH | N |
| 359 | B | CH₂CH₂CH₂CH₃ | CH₃ | H | H | H | 8-F | lone pair | S | 1 | double bond | CCH₃ | N |
| 360 | B | CH₃ | CH₃ | H | H | H | - | lone pair | S | 0 | double bond | CCH₃ | CH |
| 361 | B | CH₃ | CH₃ | H | H | H | - | lone pair | S | 0 | double bond | CH | CH |
| 362 | B | CH₃ | CH₃ | H | CH₃ | H | - | lone pair | SO₂ | 0 | double bond | CH | CH |
| 363 | B | CH₂CH(CH₃)CH₃ | CH₃ | CH₃ | CH₃ | H | - | lone pair | S | 0 | double bond | CH | CH |
| 364 | B | CH₂CH(CH₃)CH₃ | CH₃ | H | H | H | - | lone pair | S | 0 | double bond | CCH₃ | CH |
| 365 | B | CH₂CH₃ | CH₃ | CH₃ | CH₃ | H | - | lone pair | O | 0 | double bond | N | CH |
| 366 | B | CH₃ | CH₃ | CH₃ | CH₃ | H | - | lone pair | O | 0 | double bond | N | CH |
| 367 | B | -CH₂OCH₂- | | CH₃ | CH₃ | H | - | lone pair | O | 0 | double bond | N | CH |
| 368 | B | CH₂CH₂CH₂CH₃ | CH₃ | CH₃ | CH₃ | H | 7-F | lone pair | O | 1 | double bond | N | CH |
| 369 | B | CH₂OCH₃ | CH₃ | CH₃ | CH₃ | H | - | lone pair | O | 0 | double bond | CH | N |
| 370 | B | CH₂OCH₃ | CH₃ | H | H | H | - | lone pair | O | 0 | double bond | CCH₃ | N |
| 371 | B | CH₂OCH₂CH₃ | CH₃ | CH₃ | CH₃ | H | - | lone pair | O | 0 | double bond | CH | N |
| 372 | B | CH₂OCH₂CH₃ | CH₃ | H | H | H | - | lone pair | O | 0 | double bond | CCH₃ | N |
| 373 | B | CH₂CH₂CH₂CH₃ | CH₂CH₃ | CH₃ | CH₃ | H | - | lone pair | O | 0 | double bond | CH | CH |
| 374 | B | CH₂CH₂CH₂CH₃ | CH₂CH₃ | H | H | H | - | lone pair | O | 0 | double bond | CCH₃ | CH |
| 375 | B | CH₂CH₂CH₂CH₃ | CH₂CH₃ | CH₃ | H | H | - | lone pair | O | 0 | double bond | CH | CH |
| 376 | B | CH₂CH₂CH₂CH₃ | CH₂CH₃ | H | CH₃ | H | - | lone pair | O | 0 | double bond | CH | CH |
| 377 | B | CH₂CH₂CH₂CH₃ | CH₂CH₃ | H | H | H | - | lone pair | O | 0 | double bond | CH | CH |
| 378 | B | CH₂CH(CH₃)CH₃ | CH₃ | CH₃ | H | H | - | lone pair | S | 0 | double bond | CH | CH |
| 379 | B | CH₂CH(CH₃)CH₃ | CH₃ | H | CH₃ | H | - | lone pair | S | 0 | double bond | CH | CH |
| 380 | B | CH₂CH(CH₃)CH₃ | CH₃ | H | H | H | - | lone pair | S | 0 | double bond | CH | CH |
| 381 | B | -CH₂OCH₂- | | CH₃ | CH₃ | H | 8-F | lone pair | O | 1 | double bond | CH | CH |
| 382 | B | CH₃ | CH₃ | CH₃ | CH₃ | H | 7-F | lone pair | O | 1 | double bond | N | CH |
| 383 | B | CH₂OCH₃ | CH₃ | CH₃ | CH₃ | H | - | lone pair | O | 0 | double bond | N | CH |
| 384 | B | CH₂OCH₂CH₃ | CH₃ | CH₃ | CH₃ | H | - | lone pair | O | 0 | double bond | N | CH |

**[Table 7-12]**

| | | | | | | | | | | | | | |
|---|---|---|---|---|---|---|---|---|---|---|---|---|---|
| 385 | B | CH₂CH(CH₃)CH₃ | CH₃ | CH₃ | CH₃ | H | - | lone pair | S | 0 | double bond | CH | N |
| 386 | B | CH₂(CH₂)₃CH₃ | CH₃ | CH₃ | CH₃ | H | - | lone pair | O | 0 | double bond | CH | N |
| 387 | B | CH₂CH(CH₃)CH₃ | CH₃ | CH₃ | CH₃ | H | - | lone pair | SO₂ | 0 | double bond | CH | CH |
| 388 | B | CH₂CH(CH₃)CH₃ | CH₃ | H | CH₃ | H | - | lone pair | SO₂ | 0 | double bond | CH | CH |
| 389 | B | CH₂CH(CH₃)CH₃ | CH₃ | CH₃ | H | H | - | lone pair | SO₂ | 0 | double bond | CH | CH |
| 390 | B | CH₂CH₂CH₂CH₃ | CH₃ | CH₃ | CH₃ | H | 7-CH₃ | lone pair | O | 1 | double bond | N | CH |
| 391 | B | CH₂OCH₃ | CH₃ | H | CH₃ | H | - | lone pair | S | 0 | double bond | CH | CH |
| 392 | B | CH₂OCH₃ | CH₃ | H | H | H | - | lone pair | S | 0 | double bond | CH | CH |
| 393 | B | CH₂OCH₃ | CH₃ | CH₃ | H | H | - | lone pair | S | 0 | double bond | CH | CH |
| 394 | B | CH₂OCH₃ | CH₃ | CH₃ | CH₃ | H | - | lone pair | S | 0 | double bond | CH | N |
| 395 | B | CH₂OCH₃ | CH₃ | H | CH₃ | H | - | lone pair | SO₂ | 0 | double bond | CH | CH |
| 396 | B | CH₂OCH₃ | CH₃ | CH₃ | H | H | - | lone pair | SO₂ | 0 | double bond | CH | CH |
| 397 | B | CH₂OCH₃ | CH₃ | CH₃ | CH₃ | H | - | lone pair | SO₂ | 0 | double bond | CH | N |
| 398 | B | CH₂CH(CH₃)CH₃ | CH₃ | CH₃ | CH₃ | H | 8-F | lone pair | O | 1 | double bond | N | CH |
| 399 | B | CH₂CH₂CH₃ | CH₃ | CH₃ | CH₃ | H | 8-F | lone pair | O | 1 | double bond | N | CH |
| 400 | B | CH₂CH₂CH₂CH₃ | CH₃ | CH₃ | CH₃ | H | 8-F | lone pair | O | 1 | double bond | N | CH |
| 401 | B | c-propyl | CH₃ | CH₃ | CH₃ | H | 8-F | lone pair | O | 1 | double bond | N | CH |
| 402 | B | CH₂(CH₂)₃CH₃ | CH₃ | CH₃ | CH₃ | H | - | lone pair | S | 0 | double bond | CH | CH |
| 403 | B | CH₂(CH₂)₃CH₃ | CH₃ | H | H | H | - | lone pair | S | 0 | double bond | CCH₃ | CH |
| 404 | B | CH₂(CH₂)₃CH₃ | CH₃ | H | CH₃ | H | - | lone pair | S | 0 | double bond | CH | CH |
| 405 | B | CH₂(CH₂)₃CH₃ | CH₃ | H | H | H | - | lone pair | S | 0 | double bond | CH | CH |
| 406 | B | CH₂(CH₂)₃CH₃ | CH₃ | CH₃ | CH₃ | H | - | lone pair | SO₂ | 0 | double bond | CH | CH |
| 407 | B | CH₂(CH₂)₃CH₃ | CH₃ | CH₃ | H | H | - | lone pair | S | 0 | double bond | CH | CH |
| 408 | B | CH₂(CH₂)₃CH₃ | CH₃ | CH₃ | CH₃ | H | - | lone pair | S | 0 | double bond | CH | N |
| 409 | B | CH₂(CH₂)₃CH₃ | CH₃ | H | H | H | - | lone pair | S | 0 | double bond | CCH₃ | N |
| 410 | B | CH₂(CH₂)₃CH₃ | CH₃ | H | CH₃ | H | - | lone pair | SO₂ | 0 | double bond | CH | CH |
| 411 | B | CH₂(CH₂)₃CH₃ | CH₃ | CH₃ | CH₃ | H | - | lone pair | O | 0 | double bond | N | CH |
| 412 | A | CH₂CH₂CH₂CH₃ | CH₃ | Cl | CH₃ | H | - | lone pair | O | 0 | double bond | N | CH |
| 413 | B | CH₂CH(CH₃)CH₃ | CH₃ | H | CH₃ | H | - | lone pair | O | 0 | double bond | N | CH |
| 414 | B | CH(CH₃)CH₃ | CH₃ | CH₃ | CH₃ | H | - | lone pair | O | 0 | double bond | N | CH |
| 415 | B | CH₂CH₂CH₃ | CH₃ | H | CH₃ | H | - | lone pair | O | 0 | double bond | N | CH |
| 416 | B | CH₂(CH₂)₃CH₃ | CH₃ | CH₃ | H | H | - | lone pair | SO₂ | 0 | double bond | CH | CH |
| 417 | B | CH₂(CH₂)₃CH₃ | CH₃ | CH₃ | CH₃ | H | - | lone pair | SO₂ | 0 | double bond | CH | N |
| 418 | B | CH₂CH(CH₃)CH₃ | CH₃ | CH₃ | CH₃ | H | 8-F | lone pair | S | 1 | double bond | CH | CH |
| 419 | A | CH₂CH₂CH₂CH₃ | CH₃ | CH₃ | CH₃ | H | 8-F | lone pair | S | 1 | double bond | CH | CH |

**[Table 7-13]**

| | | | | | | | | | | | | | |
|---|---|---|---|---|---|---|---|---|---|---|---|---|---|
| 420 | A | CH₂CH(CH₃)CH₃ | CH₃ | CH₃ | CH₃ | H | 8-F | lone pair | S | 1 | double bond | CH | CH |
| 421 | B | CH₂CH(CH₃)CH₃ | CH₃ | CH₃ | CH₃ | H | 8-F | lone pair | S | 1 | double bond | N | CH |
| 422 | B | c-propyl | CH₃ | H | CH₃ | H | - | lone pair | O | 0 | double bond | N | CH |
| 423 | B | CH₂CH₂CH₂CH₃ | CH₃ | H | CH₃ | H | 8-F | lone pair | O | 1 | double bond | N | CH |
| 424 | A | CH₂CH₃ | CH₃ | CH₃ | CH₃ | H | 8-F | lone pair | SO₂ | 1 | double bond | CH | CH |
| 425 | B | CH₂CH(CH₃)CH₃ | CH₃ | H | CH₃ | H | 7-CH₃ | lone pair | O | 1 | double bond | N | CH |
| 426 | B | CH₂CH(CH₃)CH₃ | CH₃ | H | CH₃ | H | 7-F | lone pair | O | 1 | double bond | N | CH |
| 427 | B | CH₂CH(CH₃)CH₃ | CH₃ | CH₃ | CH₃ | H | 7-CH₃ | lone pair | O | 1 | double bond | N | CH |
| 428 | B | CH₂CH₂CH₂CH₃ | CH₃ | H | CH₃ | H | 7-CH₃ | lone pair | O | 1 | double bond | N | CH |
| 429 | B | CH₂CH(CH₃)CH₃ | CH₃ | H | CH₃ | H | 8-F | lone pair | S | 1 | double bond | CH | CH |
| 430 | B | CH₂CH(CH₃)CH₃ | CH₃ | H | H | H | 8-F | lone pair | S | 1 | double bond | CH | CH |
| 431 | B | CH₂CH(CH₃)CH₃ | CH₃ | H | CH₃ | H | 8-F | lone pair | O | 1 | double bond | N | CH |
| 432 | B | CH₂CH₂CH₂CH₃ | CH₃ | CH₃ | H | H | 8-CH₃ | lone pair | O | 1 | double bond | N | CH |
| 433 | B | CH₂CH₂CH₂CH₃ | CH₃ | CH₃ | CH₃ | H | 8-CH₃ | lone pair | O | 1 | double bond | N | CH |
| 434 | B | CH₂CH(CH₃)CH₃ | CH₃ | CH₃ | H | H | 8-F | lone pair | S | 1 | double bond | CH | CH |
| 435 | B | CH₂CH(CH₃)CH₃ | CH₃ | H | H | H | 8-F | lone pair | S | 1 | double bond | CCH₃ | CH |
| 436 | B | CH₂CH(CH₃)CH₃ | CH₃ | CH₃ | CH₃ | H | 8-F | lone pair | S | 1 | double bond | CH | N |
| 437 | B | CH₂CH₂CH₂CH₃ | CH₃ | CH₃ | CH₃ | H | 8-F | lone pair | SO₂ | 1 | double bond | CH | CH |
| 438 | A | CH₂CH₃ | CH₃ | Br | CH₃ | H | 8-F | lone pair | S | 1 | double bond | N | CH |
| 439 | B | CH₂CH₂CH₂CH₃ | CH₃ | H | CH₃ | H | 8-CH₃ | lone pair | O | 1 | double bond | N | CH |
| 440 | B | CH₂CH₂CH₂CH₃ | CH₃ | CH₃ | H | H | 7-CH₃ | lone pair | O | 1 | double bond | N | CH |
| 441 | B | CH₂CH(CH₃)CH₃ | CH₃ | CH₃ | H | H | 8-CH₃ | lone pair | O | 1 | double bond | N | CH |
| 442 | B | CH₂CH(CH₃)CH₃ | CH₃ | H | CH₃ | H | 8-CH₃ | lone pair | O | 1 | double bond | N | CH |
| 443 | B | CH₂CH(CH₃)CH₃ | CH₃ | CH₃ | CH₃ | H | 8-CH₃ | lone pair | O | 1 | double bond | N | CH |
| 444 | B | CH₂CH₂CH₂CH₃ | CH₃ | CH₃ | CH₃ | H | 8-F | lone pair | S | 1 | double bond | N | CH |
| 445 | B | CH₂CH₂CH₂CH₃ | CH₃ | H | CH₃ | H | 8-F | lone pair | S | 1 | double bond | N | CH |
| 446 | B | CH₂(CH₂)₃CH₃ | CH₃ | H | CH₃ | H | 8-CH₃ | lone pair | O | 1 | double bond | N | CH |
| 447 | B | CH₂(CH₂)₃CH₃ | CH₃ | CH₃ | H | H | 8-CH₃ | lone pair | O | 1 | double bond | N | CH |
| 448 | B | CH₂(CH₂)₃CH₃ | CH₃ | CH₃ | CH₃ | H | 8-CH₃ | lone pair | O | 1 | double bond | N | CH |
| 449 | A | CH₂CH₃ | CH₃ | CH₃ | CH₃ | H | 8-F | lone pair | S | 1 | double bond | N | CH |
| 450 | B | CH₂CH₂CH₃ | CH₃ | CH₃ | H | H | 8-CH₃ | lone pair | O | 1 | double bond | N | CH |
| 451 | B | CH₂CH₂CH₃ | CH₃ | H | CH₃ | H | 8-CH₃ | lone pair | O | 1 | double bond | N | CH |
| 452 | B | CH₂CH₃ | CH₃ | H | CH₃ | H | 8-F | lone pair | O | 1 | double bond | N | CH |
| 453 | A | CH₃ | CH₃ | CH₃ | CH₃ | H | 8-CH₃,7-F | lone pair | O | 2 | double bond | CH | CH |
| 454 | A | CH₃ | CH₃ | H | CH₃ | H | 8-CH₃,7-F | lone pair | O | 2 | double bond | CH | CH |

**[Table 7-14]**

| | | | | | | | | | | | | | |
|---|---|---|---|---|---|---|---|---|---|---|---|---|---|
| 455 | B | CH₂CH₃ | CH₃ | CH₃ | CH₃ | H | 8-F | lone pair | O | 1 | double bond | CH | CH |
| 456 | B | CH₂CH₃ | CH₃ | H | H | H | 8-F | lone pair | O | 1 | double bond | CCH₃ | CH |
| 457 | B | CH₂CH₃ | CH₃ | H | CH₃ | H | 8-F | lone pair | O | 1 | double bond | CH | CH |
| 458 | B | CH₂CH₃ | CH₃ | H | H | H | 8-F | lone pair | O | 1 | double bond | CH | CH |
| 459 | B | CH₂CH₃ | CH₃ | CH₃ | H | H | 8-F | lone pair | O | 1 | double bond | CH | CH |
| 460 | B | CH₂CH₃ | CH₃ | CH₃ | CH₃ | H | 8-F | lone pair | O | 1 | double bond | CH | N |
| 461 | B | CH₂CH₃ | CH₃ | H | H | H | 8-F | lone pair | O | 1 | double bond | CCH₃ | N |
| 462 | A | CH₃ | CH₃ | CH₃ | CH₃ | H | 8-F | lone pair | O | 1 | double bond | N | CH |
| 463 | A | CH₃ | CH₃ | CH₃ | CH₃ | H | 8-F,7-F | lone pair | O | 2 | double bond | N | CH |
| 464 | B | CH₂CH₂CH₂CH₃ | CH₃ | CH₃ | CH₃ | H | 8-CH₃,7-F | lone pair | O | 2 | double bond | CH | CH |
| 465 | B | CH₂CH₂CH₂CH₃ | CH₃ | H | H | H | 8-CH₃,7-F | lone pair | O | 2 | double bond | CCH₃ | CH |
| 466 | B | CH₂CH₂CH₂CH₃ | CH₃ | H | CH₃ | H | 8-CH₃,7-F | lone pair | O | 2 | double bond | CH | CH |
| 467 | B | CH₂CH₂CH₂CH₃ | CH₃ | H | H | H | 8-CH₃,7-F | lone pair | O | 2 | double bond | CH | CH |
| 468 | B | CH₂CH₂CH₂CH₃ | CH₃ | CH₃ | H | H | 8-CH_{3,} 7-F | lone pair | O | 2 | double bond | CH | CH |
| 469 | B | CH₂CH₂CH₂CH₃ | CH₃ | CH₃ | CH₃ | H | 8-CH₃,7-F | lone pair | O | 2 | double bond | CH | N |
| 470 | B | CH₂CH₂CH₂CH₃ | CH₃ | H | H | H | 8-CH₃,7-F | lone pair | O | 2 | double bond | CCH₃ | N |
| 471 | B | CH₂CH₃ | CH₃ | CH₃ | H | H | 8-F | lone pair | O | 1 | double bond | N | CH |
| 472 | B | CH₂CH₂CH₂CH₃ | CH₃ | CH₃ | H | H | 8-F | lone pair | O | 1 | double bond | N | CH |
| 473 | B | CH₃ | CH₃ | CH₃ | H | H | 8-F | lone pair | O | 1 | double bond | N | CH |
| 474 | B | CH₃ | CH₃ | H | CH₃ | H | 8-F | lone pair | O | 1 | double bond | N | CH |
| 475 | B | CH₃ | CH₃ | CH₃ | CH₃ | H | 8-F | lone pair | O | 1 | double bond | N | CH |
| 476 | B | CH₃ | CH₃ | CH₃ | H | H | 8-CH₃,7-F | lone pair | O | 2 | double bond | N | CH |
| 477 | B | CH₃ | CH₃ | H | CH₃ | H | 8-CH_{3,} 7-F | lone pair | O | 2 | double bond | N | CH |
| 478 | B | CH₃ | CH₃ | CH₃ | CH₃ | H | 8-CH₃,7-F | lone pair | O | 2 | double bond | N | CH |
| 479 | B | CH₃ | CH₃ | CH₃ | H | H | 8-F,7-F | lone pair | O | 2 | double bond | N | CH |
| 480 | B | CH₃ | CH₃ | H | CH₃ | H | 8-F,7-F | lone pair | O | 2 | double bond | N | CH |
| 481 | B | CH₃ | CH₃ | CH₃ | CH₃ | H | 8-F,7-F | lone pair | O | 2 | double bond | N | CH |
| 482 | B | CH₂CH₂CH₂CH₃ | CH₃ | CH₃ | CH₃ | H | 8-F,7-F | lone pair | O | 2 | double bond | CH | CH |
| 483 | B | CH₂CH₂CH₂CH₃ | CH₃ | H | H | H | 8-F,7-F | lone pair | O | 2 | double bond | CCH₃ | CH |
| 484 | B | CH₂CH₂CH₂CH₃ | CH₃ | H | CH₃ | H | 8-F,7-F | lone pair | O | 2 | double bond | CH | CH |
| 485 | B | CH₂CH₂CH₂CH₃ | CH₃ | H | H | H | 8-F,7-F | lone pair | O | 2 | double bond | CH | CH |
| 486 | A | CH₂CH₃ | CH₃ | CH₃ | CH₃ | H | 8-CH₃,7-F | lone pair | O | 2 | double bond | CH | CH |
| 487 | A | CH₂CH₂CH₃ | CH₃ | CH₃ | CH₃ | H | 8-CH₃,7-F | lone pair | O | 2 | double bond | CH | CH |
| 488 | A | CH₂CH₂CH₂CH₃ | CH₃ | CH₃ | CH₃ | H | 8-CH₃,7-F | lone pair | O | 2 | double bond | CH | CH |
| 489 | A | CH₂CH₃ | CH₃ | CH₃ | CH₃ | H | 8-F,7-F | lone pair | O | 2 | double bond | CH | CH |

**[Table 7-15]**

| | | | | | | | | | | | | | |
|---|---|---|---|---|---|---|---|---|---|---|---|---|---|
| 490 | B | CH₂CH₂CH₂CH₃ | CH₃ | CH₃ | H | H | 8-F,7-F | lone pair | O | 2 | double bond | CH | CH |
| 491 | B | CH₂CH₂CH₂CH₃ | CH₃ | CH₃ | CH₃ | H | 8-F,7-F | lone pair | O | 2 | double bond | CH | N |
| 492 | B | CH₂CH₂CH₂CH₃ | CH₃ | H | H | H | 8-F,7-F | lone pair | O | 2 | double bond | CCH₃ | N |
| 493 | B | CH₂CH₃ | CH₃ | CH₃ | CH₃ | H | 8-F,7-F | lone pair | O | 2 | double bond | CH | CH |
| 494 | B | CH₂CH₂CH₃ | CH₃ | CH₃ | CH₃ | H | 8-F | lone pair | O | 1 | double bond | CH | CH |
| 495 | B | CH₂CH₂CH₃ | CH₃ | H | H | H | 8-F | lone pair | O | 1 | double bond | CCH₃ | CH |
| 496 | B | CH₂CH₂CH₂CH₃ | CH₃ | H | CH₃ | H | 8-F,7-F | lone pair | O | 2 | double bond | N | CH |
| 497 | B | CH₂CH₂CH₃ | CH₃ | H | CH₃ | H | 8-F | lone pair | O | 1 | double bond | CH | CH |
| 498 | B | CH₂CH₂CH₃ | CH₃ | H | H | H | 8-F | lone pair | O | 1 | double bond | CH | CH |
| 499 | B | CH₂CH₂CH₃ | CH₃ | CH₃ | CH₃ | H | 8-F | lone pair | O | 1 | double bond | CH | N |
| 500 | B | CH₂CH₂CH₃ | CH₃ | H | H | H | 8-F | lone pair | O | 1 | double bond | CCH₃ | N |
| 501 | B | CH₂CH₂CH₂CH₃ | CH₃ | CH₃ | H | H | 8-F,7-F | lone pair | O | 2 | double bond | N | CH |
| 502 | B | CH₂CH₂CH₂CH₃ | CH₃ | CH₃ | CH₃ | H | 8-F,7-F | lone pair | O | 2 | double bond | N | CH |
| 503 | B | CH₂CH₂CH₂CH₃ | CH₃ | CH₃ | H | H | 8-CH₃,7-F | lone pair | O | 2 | double bond | N | CH |
| 504 | A | CH₃ | CH₃ | Br | CH₃ | H | 8-CH₃,7-F | lone pair | O | 2 | double bond | N | CH |
| 505 | A | CH₂CH₂CH₂CH₃ | CH₃ | Br | CH₃ | H | 8-F | lone pair | O | 1 | double bond | N | CH |
| 506 | A | CH₃ | CH₃ | CH₃ | CH₃ | H | 8-CH₃,7-F | lone pair | O | 2 | double bond | N | CH |
| 507 | A | CH₂CH₂CH₂CH₃ | CH₃ | CH₃ | CH₃ | H | 8-F | lone pair | O | 1 | double bond | N | CH |
| 508 | B | CH₂CH₂CH₂CH₃ | CH₃ | H | CH₃ | H | 8-CH₃,7-F | lone pair | O | 2 | double bond | N | CH |
| 509 | B | CH₂CH₂CH₂CH₃ | CH₃ | CH₃ | CH₃ | H | 8-CH₃,7-F | lone pair | O | 2 | double bond | N | CH |
| 510 | B | CH₂CH₂CH₃ | CH₃ | CH₃ | H | H | 8-CH₃,7-F | lone pair | O | 2 | double bond | N | CH |
| 511 | B | CH₂CH₂CH₃ | CH₃ | H | CH₃ | H | 8-CH₃,7-F | lone pair | O | 2 | double bond | N | CH |
| 512 | B | CH₂CH(CH₃)CH₃ | CH₃ | CH₃ | CH₃ | H | 8-F | lone pair | O | 1 | double bond | CH | CH |
| 513 | B | CH₂CH(CH₃)CH₃ | CH₃ | H | H | H | 8-F | lone pair | O | 1 | double bond | CCH₃ | CH |
| 514 | B | CH₂CH(CH₃)CH₃ | CH₃ | H | CH₃ | H | 8-F | lone pair | O | 1 | double bond | CH | CH |
| 515 | B | CH₂CH(CH₃)CH₃ | CH₃ | H | H | H | 8-F | lone pair | O | 1 | double bond | CH | CH |
| 516 | B | CH₂CH(CH₃)CH₃ | CH₃ | CH₃ | CH₃ | H | 8-F | lone pair | O | 1 | double bond | CH | N |
| 517 | B | CH₂CH(CH₃)CH₃ | CH₃ | H | H | H | 8-F | lone pair | O | 1 | double bond | CCH₃ | N |
| 518 | A | CH₂CH₂CH₃ | CH₃ | CH₃ | CH₃ | H | 8-F,7-F | lone pair | O | 2 | double bond | CH | CH |
| 519 | A | CH₂CH₂CH₂CH₃ | CH₃ | CH₃ | CH₃ | H | 8-F,7-F | lone pair | O | 2 | double bond | CH | CH |
| 520 | A | CH₂CH₃ | CH₃ | CH₃ | CH₃ | H | 8-F | lone pair | O | 1 | double bond | CH | CH |
| 521 | A | CH₂CH₂CH₃ | CH₃ | CH₃ | CH₃ | H | 8-F | lone pair | O | 1 | double bond | CH | CH |
| 522 | A | CH₂CH₂CH₂CH₃ | CH₃ | CH₃ | CH₃ | H | 8-F | lone pair | O | 1 | double bond | CH | CH |
| 523 | C | CH₃ | CH₃ | H | H | H | 8-F | lone pair | O | 1 | double bond | N | CH |
| 524 | C | CH₂CH₂CH₂CH₃ | CH₃ | H | H | H | 8-F | lone pair | O | 1 | double bond | N | CH |

**[Table 7-16]**

| | | | | | | | | | | | | | |
|---|---|---|---|---|---|---|---|---|---|---|---|---|---|
| 525 | C | CH₂CH₂CH₂CH₃ | CH₃ | H | CH₃ | H | 8-F | lone pair | O | 1 | double bond | N | CH |
| 526 | C | CH₃ | CH₃ | H | CH₃ | H | 8-CH₃,7-F | lone pair | O | 2 | double bond | N | CH |
| 527 | B | CH₂CH₂CH₃ | CH₃ | CH₃ | CH₃ | H | 8-CH₃,7-F | lone pair | O | 2 | double bond | N | CH |
| 528 | B | CH₂CH₃ | CH₃ | CH₃ | H | H | 8-CH₃,7-F | lone pair | O | 2 | double bond | N | CH |
| 529 | B | CH₂CH₃ | CH₃ | CH₃ | CH₃ | H | 8-CH₃,7-F | lone pair | O | 2 | double bond | N | CH |
| 530 | B | CH₂CH₃ | CH₃ | CH₃ | CH₃ | H | 8-F,7-F | lone pair | O | 2 | double bond | N | CH |
| 531 | B | CH₂CH₂CH₃ | CH₃ | CH₃ | CH₃ | H | 8-CH₃,7-F | lone pair | O | 2 | double bond | CH | CH |
| 532 | B | CH₂CH₂CH₃ | CH₃ | H | CH₃ | H | 8-CH₃,7-F | lone pair | O | 2 | double bond | CH | CH |
| 533 | B | CH₂CH₂CH₃ | CH₃ | CH₃ | H | H | 8-CH₃ | lone pair | O | 2 | double bond | CH | CH |
| 534 | B | CH₂CH₂CH₃ | CH₃ | CH₃ | CH₃ | H | 8-CH₃,7-F | lone pair | O | 2 | double bond | CH | N |
| 535 | B | CH₂CH₂CH₃ | CH₃ | H | H | H | 8-CH₃,7-F | lone pair | O | 2 | double bond | CCH₃ | N |
| 536 | B | -CH₂CH₂CH₂- | | CH₃ | CH₃ | H | 8-F | lone pair | O | 1 | double bond | CH | CH |
| 537 | B | -CH₂CH₂CH₂- | | H | H | H | 8-F | lone pair | O | 1 | double bond | CCH₃ | CH |
| 538 | B | CH₂CH₃ | CH₃ | CH₃ | CH₃ | H | 8-CH₃,7-F | lone pair | O | 2 | double bond | CH | CH |
| 539 | B | CH₂CH₃ | CH₃ | H | H | H | 8-CH₃,7-F | lone pair | O | 2 | double bond | CCH₃ | CH |
| 540 | B | CH₂CH₃ | CH₃ | H | CH₃ | H | 8-CH₃,7-F | lone pair | O | 2 | double bond | CH | CH |
| 541 | B | CH₂CH₂CH₃ | CH₃ | CH₃ | H | H | 8-F | lone pair | O | 1 | double bond | N | CH |
| 542 | B | CH₂CH₂CH₃ | CH₃ | H | CH₃ | H | 8-F | lone pair | O | 1 | double bond | N | CH |
| 543 | B | CH₂CH₂CH₃ | CH₃ | CH₃ | CH₃ | H | 8-F,7-F | lone pair | O | 2 | double bond | N | CH |
| 544 | C | CH₃ | CH₃ | H | H | H | 8-CH₃,7-F | lone pair | O | 2 | double bond | N | CH |
| 545 | C | CH₂CH₂CH₂CH₃ | CH₃ | H | CH₃ | H | 8-F | lone pair | S | 1 | double bond | N | CH |
| 546 | C | CH₂CH₂CH₂CH₃ | CH₃ | CH₃ | H | H | 8-F | lone pair | O | 1 | double bond | N | Cl |
| 547 | B | CH₂CH₂CH₃ | CH₃ | CH₃ | CF3 | H | 8-F | lone pair | O | 1 | double bond | N | CH |
| 548 | B | CH₂CH₂CH₃ | CH₃ | CH₃ | H | H | 8-F,7-F | lone pair | O | 2 | double bond | N | CH |
| 549 | B | CH₂CH₂CH₃ | CH₃ | H | CH₃ | H | 8-F,7-F | lone pair | O | 2 | double bond | N | CH |
| 550 | B | CH₃ | CH₃ | CH₃ | CHF2 | H | 8-F | lone pair | O | 1 | double bond | N | CH |
| 551 | B | CH₃ | CH₃ | CH₃ | CF3 | H | 8-F | lone pair | O | 1 | double bond | N | CH |
| 552 | C | CH₃ | CH₃ | CH₃ | CH₃ | H | 8-F | lone pair | O | 1 | double bond | N | Cl |
| 553 | B | CH₃ | CH₃ | CH₃ | CF3 | H | 8-CH₃,7-F | lone pair | O | 2 | double bond | N | CH |
| 554 | B | CH₃ | CH₃ | CH₃ | CHF2 | H | 8-CH₃,7-F | lone pair | O | 2 | double bond | N | CH |
| 555 | B | CH₂CH₂CH₃ | CH₃ | H | H | H | 8-CH₃ | lone pair | O | 2 | double bond | CCH₃ | CH |
| 556 | B | CH₂CH₂CH₃ | CH₃ | H | H | H | 8-CH₃,7-F | lone pair | O | 2 | double bond | CH | CH |
| 557 | B | CH₂CH₃ | CH₃ | H | H | H | 8-CH₃,7-F | lone pair | O | 2 | double bond | CH | CH |
| 558 | C | CH₃ | CH₃ | CH₃ | CH₃ | H | 8-F | lone pair | O | 1 | double bond | N | CH |
| 559 | C | CH₃ | CH₃ | CH₃ | CH₃ | H | 8-CH₃, 7-F | lone pair | O | 2 | double bond | N | CH |

**[Table 7-17]**

| | | | | | | | | | | | | | |
|---|---|---|---|---|---|---|---|---|---|---|---|---|---|
| 560 | B | CH₃ | CH₃ | CH₃ | CF3 | H | 8-F,7-F | lone pair | O | 2 | double bond | N | CH |
| 561 | B | CH₂CH₃ | CH₃ | CH₃ | CF3 | H | 8-F | lone pair | O | 1 | double bond | N | CH |
| 562 | B | CH₃ | CH₃ | Cl | CH₃ | H | I 8-F | lone pair | O | 1 | double bond | N | CH |
| 563 | A | CH₂CH₂CH₃ | CH₃ | Br | CH₃ | H | 8-F | lone pair | O | 1 | double bond | N | CH |
| 564 | A | CH₂CH₂CH₃ | CH₃ | CH₃ | CH₃ | H | 8-CH₃,7-F | lone pair | O | 2 | double bond | N | CH |
| 565 | C | CH₃ | CH₃ | CH₃ | H | H | 8-F | lone pair | O | 1 | double bond | N | CH |
| 566 | C | CH₃ | CH₃ | CH₃ | H | H | 8-CH₃,7-F | lone pair | O | 2 | double bond | N | CH |
| 567 | C | CH₃ | CH₃ | H | CH₃ | H | 8-F | lone pair | O | 1 | double bond | N | CH |
| 568 | C | CH₂CH₂CH₂CH₃ | CH₃ | CH₃ | CH₃ | H | 8-F | lone pair | O | 1 | double bond | N | CH |
| 569 | B | CH₂CH₂CH₃ | CH₃ | CH₃ | CHF2 | H | 8-F | lone pair | O | 1 | double bond | N | CH |
| 570 | B | CH₂CH₂CH₂CH₃ | CH₃ | CH₃ | CF3 | H | 8-F | lone pair | O | 1 | double bond | N | CH |
| 571 | B | CH₂CH₂CH₂CH₃ | CH₃ | Cl | CH₃ | H | 8-F | lone pair | O | 1 | double bond | N | CH |
| 572 | B | CH₂(CH₂)₃CH₃ | CH₃ | H | CH₃ | H | 8-F | lone pair | O | 1 | double bond | N | CH |
| 573 | B | CH₂(CH₂)₃CH₃ | CH₃ | CH₃ | CH₃ | H | 8-F | lone pair | O | 1 | double bond | N | CH |
| 574 | C | CH₃ | CH₃ | H | CH₃ | H | 8-Cl | lone pair | S | 1 | double bond | N | CH |
| 575 | C | CH₃ | CH₃ | CH₃ | CH₃ | H | 8-Cl | lone pair | S | 1 | double bond | N | CH |
| 576 | B | CH₂(CH₂)₃CH₃ | CH₃ | CH₃ | H | H | 8-F | lone pair | O | 1 | double bond | N | CH |
| 577 | B | CH₂CH₃ | CH₃ | CH₃ | CH₃ | H | 8-F | lone pair | O | 1 | double bond | N | CH |
| 578 | B | CH₃ | CH₃ | H | CH₃ | H | - | lone pair | O | 0 | double bond | N | CH |
| 579 | B | CH₂CH₃ | CH₃ | H | CH₃ | H | - | lone pair | O | 0 | double bond | N | CH |
| 580 | B | CH₃ | CH₃ | CH₃ | H | H | 7-CH₃ | lone pair | O | 1 | double bond | N | CH |
| 581 | B | CH₃ | CH₃ | H | CH₃ | H | 7-CH₃ | lone pair | O | 1 | double bond | N | CH |
| 582 | B | CH₃ | CH₃ | CH₃ | CH₃ | H | 7-CH₃ | lone pair | O | 1 | double bond | N | CH |
| 583 | B | CH₃ | CH₃ | CH₃ | H | H | 8-CH₃ | lone pair | O | 1 | double bond | N | CH |
| 584 | B | CH₃ | CH₃ | H | CH₃ | H | 8-CH₃ | lone pair | O | 1 | double bond | N | CH |
| 585 | B | CH₂(CH₂)₃CH₃ | CH₃ | CH₃ | H | H | - | lone pair | O | 0 | double bond | N | CH |
| 586 | A | CH₂CH₃ | CH₃ | Br | CH₃ | H | 8-F | lone pair | O | 1 | double bond | N | CH |
| 587 | A | CH₂CH₂CH₃ | CH₃ | Br | CH₃ | H | 8-F, 7-Cl | lone pair | O | 2 | double bond | N | CH |
| 588 | A | CH₂CH₂CH₂CH₃ | CH₃ | Br | CH₃ | H | 8-F, 7-Cl | lone pair | O | 2 | double bond | N | CH |
| 589 | A | CH₂CH₂CH₂CH₃ | CH₃ | Br | CH₃ | H | 8-CH₃,7-F | lone pair | O | 2 | double bond | N | CH |
| 590 | A | CH₂CH₃ | CH₃ | CH₃ | CH₃ | H | 8-F | lone pair | O | 1 | double bond | N | CH |
| 591 | A | CH₂CH₂CH₃ | CH₃ | CH₃ | CH₃ | H | 8-F, 7-Cl | lone pair | O | 2 | double bond | N | CH |
| 592 | A | CH₂CH₂CH₂CH₃ | CH₃ | CH₃ | CH₃ | H | 8-F, 7-Cl | lone pair | O | 2 | double bond | N | CH |
| 593 | A | CH₂CH₂CH₂CH₃ | CH₃ | CH₃ | CH₃ | H | 8-CH₃,7-F | lone pair | O | 2 | double bond | N | CH |
| 594 | A | CH₃ | CH₃ | Br | CH₃ | H | - | lone pair | O | 0 | double bond | N | CH |

**[Table 7-18]**

| | | | | | | | | | | | | | |
|---|---|---|---|---|---|---|---|---|---|---|---|---|---|
| 595 | A | CH₃ | CH₃ | H | CH₃ | H | - | lone pair | O | 0 | double bond | N | CH |
| 596 | C | CH₃ | CH₃ | H | CH₃ | H | - | lone pair | S | 0 | double bond | N | CH |
| 597 | C | CH₃ | CH₃ | CH₃ | H | H | - | lone pair | S | 0 | double bond | N | CH |
| 598 | C | CH₃ | CH₃ | CH₃ | CH₃ | H | - | lone pair | S | 0 | double bond | N | CH |
| 599 | A | CH₂CH₃ | CH₃ | Br | CH₃ | H | - | lone pair | O | 0 | double bond | N | CH |
| 600 | A | CH₂CH₃ | CH₃ | H | CH₃ | H | - | lone pair | O | 0 | double bond | N | CH |
| 601 | A | CH₂CH₂CH₃ | CH₃ | Br | CH₃ | H | - | lone pair | O | 0 | double bond | N | CH |
| 602 | A | CH₂CH₂CH₃ | CH₃ | H | CH₃ | H | - | lone pair | O | 0 | double bond | N | CH |
| 603 | A | CH₃ | CH₃ | CH₃ | CH₃ | H | - | lone pair | O | 0 | double bond | N | CH |
| 604 | A | CH₂CH₃ | CH₃ | CH₃ | CH₃ | H | - | lone pair | O | 0 | double bond | N | CH |
| 605 | B | CH₂(CH₂)₃CH₃ | CH₃ | H | CH₃ | H | - | lone pair | O | 0 | double bond | N | CH |
| 606 | C | CH₂CH(CH₃)CH₃ | CH₃ | CH₃ | H | H | 8-F | lone pair | O | 1 | double bond | N | CH |
| 607 | B | CH₂CH(CH₃)CH₃ | CH₃ | CH₃ | H | H | 8-F | lone pair | O | 1 | double bond | N | CH |
| 608 | C | CH₂CH(CH₃)CH₃ | CH₃ | CH₃ | CH₃ | H | 8-F | lone pair | O | 1 | double bond | N | CH |
| 609 | B | CH₂CH₃ | CH₃ | CH₃ | H | H | 8-F,7-F | lone pair | O | 2 | double bond | N | CH |
| 610 | B | CH₂CH₃ | CH₃ | H | CH₃ | H | 8-F,7-F | lone pair | O | 2 | double bond | N | CH |
| 611 | C | CH₂(CH₂)₃CH₃ | CH₃ | CH₃ | H | H | - | lone pair | O | 0 | double bond | N | CH |
| 612 | C | CH₂(CH₂)₃CH₃ | CH₃ | H | CH₃ | H | - | lone pair | O | 0 | double bond | N | CH |
| 613 | C | CH₂(CH₂)₃CH₃ | CH₃ | CH₃ | CH₃ | H | - | lone pair | O | 0 | double bond | N | CH |
| 614 | C | CH₂CH₂CH₃ | CH₃ | H | CH₃ | H | - | lone pair | O | 0 | double bond | N | CH |
| 615 | C | CH₂CH₂CH₃ | CH₃ | CH₃ | CH₃ | H | - | lone pair | O | 0 | double bond | N | CH |
| 616 | B | CH₃ | CH₃ | H | CH₃ | H | - | lone pair | S | 0 | double bond | N | CH |
| 617 | C | CH₂CH₂CH₃ | CH₃ | CH₃ | H | H | - | lone pair | O | 0 | double bond | N | CH |
| 618 | B | CH₃ | CH₃ | CH₃ | H | H | - | lone pair | S | 0 | double bond | N | CH |
| 619 | B | CH₃ | CH₃ | CH₃ | CH₃ | H | - | lone pair | S | 0 | double bond | N | CH |
| 620 | A | CH₂CH₂CH₃ | CH₃ | Cl | CH₃ | H | - | lone pair | O | 0 | double bond | N | CH |
| 621 | C | CH₂CH₂CH₃ | CH₃ | CH₃ | H | H | 8-CH₃ | lone pair | O | 1 | double bond | N | CH |
| 622 | C | CH₂CH₂CH₃ | CH₃ | H | CH₃ | H | 8-CH₃ | lone pair | O | 1 | double bond | N | CH |
| 623 | C | CH₂CH₂CH₃ | CH₃ | CH₃ | CH₃ | H | 8-CH₃ | lone pair | O | 1 | double bond | N | CH |
| 624 | B | CH₂CH₂CH₃ | CH₃ | CH₃ | CH₃ | H | 8-CH₃ | lone pair | O | 1 | double bond | N | CH |
| 625 | A | CH₃ | CH₃ | Cl | CH₃ | H | 8-F | lone pair | O | 1 | double bond | N | CH |
| 626 | A | CH₂CH₃ | CH₃ | Cl | CH₃ | H | - | lone pair | O | 0 | double bond | N | CH |
| 627 | C | CH₂CH₃ | CH₃ | H | CH₃ | H | - | lone pair | O | 0 | double bond | N | CH |
| 628 | C | CH₂CH₃ | CH₃ | CH₃ | CH₃ | H | - | lone pair | O | 0 | double bond | N | CH |
| 629 | C | CH₂CH₃ | CH₃ | CH₃ | H | H | 8-CH₃ | lone pair | O | 1 | double bond | N | CH |

**[Table 7-19]**

| | | | | | | | | | | | | | |
|---|---|---|---|---|---|---|---|---|---|---|---|---|---|
| 630 | C | CH₂CH₃ | CH₃ | H | CH₃ | H | 8-CH₃ | lone pair | O | 1 | double bond | N | CH |
| 631 | C | CH₂CH₃ | CH₃ | CH₃ | CH₃ | H | 8-CH₃ | lone pair | O | 1 | double bond | N | CH |
| 632 | B | CH₂CH₃ | CH₃ | CH₃ | H | H | 8-CH₃ | lone pair | O | 1 | double bond | N | CH |
| 633 | B | CH₂CH₃ | CH₃ | H | CH₃ | H | 8-CH₃ | lone pair | O | 1 | double bond | N | CH |
| 634 | A | CH₃ | CH₃ | Cl | CH₃ | H | - | lone pair | O | 0 | double bond | N | CH |
| 635 | C | CH₃ | CH₃ | CH₃ | H | H | - | lone pair | O | 0 | double bond | N | CH |
| 636 | C | CH₃ | CH₃ | H | CH₃ | H | - | lone pair | O | 0 | double bond | N | CH |
| 637 | C | CH₃ | CH₃ | CH₃ | CH₃ | H | - | lone pair | O | 0 | double bond | N | CH |
| 638 | A | CH₃ | CH₃ | Br | CH₃ | H | 8-CH₃ | lone pair | O | 1 | double bond | N | CH |
| 639 | A | CH₂CH₂CH₃ | CH₃ | CH₃ | CH₃ | H | - | lone pair | O | 0 | double bond | N | CH |
| 640 | A | CH₂CH₂CH₃ | CH₃ | CH₃ | CH₃ | H | 8-F | lone pair | O | 1 | double bond | N | CH |
| 641 | A | CH₃ | CH₃ | Br | CH₃ | H | 7-CH₃ | lone pair | O | 1 | double bond | N | CH |
| 642 | A | CH₃ | CH₃ | H | CH₃ | H | 7-CH₃ | lone pair | O | 1 | double bond | N | CH |
| 643 | A | CH₃ | CH₃ | Cl | CH₃ | H | 8-CH₃ | lone pair | O | 1 | double bond | N | CH |
| 644 | C | CH₃ | CH₃ | CH₃ | CH₃ | H | 8-CH₃ | lone pair | O | 1 | double bond | N | CH |
| 645 | C | CH₃ | CH₃ | CH₃ | H | H | 8-CH₃ | lone pair | O | 1 | double bond | N | CH |
| 646 | C | CH₃ | CH₃ | H | CH₃ | H | 8-CH₃ | lone pair | O | 1 | double bond | N | CH |
| 647 | C | CH₂CH₂CH₃ | CH₃ | CH₃ | CH₃ | H | - | lone pair | S | 0 | double bond | N | CH |
| 648 | C | CH₂CH₂CH₃ | CH₃ | CH₃ | H | H | - | lone pair | S | 0 | double bond | N | CH |
| 649 | C | CH₂CH₂CH₃ | CH₃ | H | CH₃ | H | - | lone pair | S | 0 | double bond | N | CH |
| 650 | A | CH₃ | CH₃ | H | CH₃ | H | 8-F | lone pair | O | 1 | double bond | N | CH |
| 651 | A | CH₂CH₂CH₂CH₃ | CH₃ | H | CH₃ | H | 8-F | lone pair | O | 1 | double bond | N | CH |
| 652 | A | CH₂CH₂CH₂CH₃ | CH₃ | Cl | CH₃ | H | 8-F | lone pair | O | 1 | double bond | N | CH |
| 653 | C | CH₂CH₂CH₂CH₃ | CH₃ | CH₃ | CH₃ | H | 8-CH₃ | lone pair | O | 1 | double bond | N | CH |
| 654 | C | CH₂CH₂CH₂CH₃ | CH₃ | H | CH₃ | H | 8-CH₃ | lone pair | O | 1 | double bond | N | CH |
| 655 | A | CH₂CH₂CH₃ | CH₃ | H | CH₃ | H | 8-F | lone pair | O | 1 | double bond | N | CH |
| 656 | A | CH₂CH₂CH₃ | CH₃ | Cl | CH₃ | H | 8-F | lone pair | O | 1 | double bond | N | CH |
| 657 | B | CH₂CH₂CH₃ | CH₃ | CH₃ | H | H | - | lone pair | S | 0 | double bond | N | CH |
| 658 | B | CH₂CH₂CH₃ | CH₃ | H | CH₃ | H | - | lone pair | S | 0 | double bond | N | CH |
| 659 | B | CH₂CH(CH₃)CH₃ | CH₃ | CH₃ | H | H | 8-CH₃,7-F | lone pair | O | 2 | double bond | N | CH |
| 660 | B | CH₂CH(CH₃)CH₃ | CH₃ | H | CH₃ | H | 8-CH₃,7-F | lone pair | O | 2 | double bond | N | CH |
| 661 | B | CH₂CH(CH₃)CH₃ | CH₃ | CH₃ | CH₃ | H | 8-CH₃,7-F | lone pair | O | 2 | double bond | N | CH |
| 662 | C | CH₂CH₂CH₂CH₃ | CH₃ | CH₃ | H | H | 7-F | lone pair | O | 1 | double bond | N | CH |
| 663 | C | CH₂CH₂CH₂CH₃ | CH₃ | H | CH₃ | H | 7-F | lone pair | O | 1 | double bond | N | CH |
| 664 | C | CH₂CH(CH₃)CH₃ | CH₃ | CH₃ | H | H | 7-F | lone pair | O | 1 | double bond | N | CH |

**[Table 7-20]**

| | | | | | | | | | | | | | |
|---|---|---|---|---|---|---|---|---|---|---|---|---|---|
| 665 | C | CH₂CH(CH₃)CH₃ | CH₃ | H | CH₃ | H | 7-F | lone pair | O | 1 | double bond | N | CH |
| 666 | C | CH₂CH(CH₃)CH₃ | CH₃ | CH₃ | CH₃ | H | 7-F | lone pair | O | 1 | double bond | N | CH |
| 667 | B | CH₂CH₂CH₂CH₃ | CH₃ | CH₃ | H | H | 7-F | lone pair | O | 1 | double bond | N | CH |
| 668 | B | CH₂CH(CH₃)CH₃ | CH₃ | CH₃ | H | H | 7-F | lone pair | O | 1 | double bond | N | CH |
| 669 | A | CH₂CH₂CH₃ | CH₃ | Br | CH₃ | H | 8-CH₃ | lone pair | O | 1 | double bond | N | CH |
| 670 | A | CH₂CH₂CH₃ | CH₃ | Cl | CH3 | H | 8-CH₃ | lone pair | O | 1 | double bond | N | CH |
| 671 | A | CH₂CH₂CH₃ | CH₃ | H | CH₃ | H | 8-CH₃ | lone pair | O | 1 | double bond | N | CH |
| 672 | A | CH₂CH₂CH₂CH₃ | CH₃ | Br | CH₃ | H | 8-CH₃ | lone pair | O | 1 | double bond | N | CH |
| 673 | A | CH₂CH₂CH₂CH₃ | CH₃ | Cl | CH₃ | H | 8-CH₃ | lone pair | O | 1 | double bond | N | CH |
| 674 | A | CH₂CH₂CH₂CH₃ | CH₃ | H | CH₃ | H | 8-CH₃ | lone pair | O | 1 | double bond | N | CH |
| 675 | A | CH₃ | CH₃ | CH₃ | CH₃ | H | 8-CH₃ | lone pair | O | 1 | double bond | N | CH |
| 676 | A | CH₃ | CH₃ | CH₃ | CH₃ | H | 7-CH₃ | lone pair | O | 1 | double bond | N | CH |
| 677 | A | CH₂(CH₂)₃CH₃ | CH₃ | Cl | CH₃ | H | 8-F | lone pair | O | 1 | double bond | N | CH |
| 678 | A | CH₂(CH₂)₃CH₃ | CH₃ | H | CH₃ | H | 8-F | lone pair | O | 1 | double bond | N | CH |
| 679 | A | CH₂CH₂CH₃ | CH₃ | CH₃ | CH₃ | H | 8-CH₃ | lone pair | O | 1 | double bond | N | CH |
| 680 | A | CH₂CH₂CH₂CH₃ | CH₃ | CH₃ | CH₃ | H | 8-CH₃ | lone pair | O | 1 | double bond | N | CH |
| 681 | B | CH₂CH₂CH₃ | CH₃ | H | CH₃ | H | 7-CH₃ | lone pair | O | 1 | double bond | N | CH |
| 682 | B | CH₂CH₂CH₃ | CH₃ | CH₃ | CH₃ | H | 7-CH₃ | lone pair | O | 1 | double bond | N | CH |
| 683 | C | CH₃ | CH₃ | CH₃ | H | H | 7-F | lone pair | O | 1 | double bond | N | CH |
| 684 | C | CH₃ | CH₃ | H | CH₃ | H | 7-F | lone pair | O | 1 | double bond | N | CH |
| 685 | C | CH₃ | CH₃ | CH₃ | CH₃ | H | 7-F | lone pair | O | 1 | double bond | N | CH |
| 686 | B | CH₃ | CH₃ | CH₃ | H | H | 7-F | lone pair | O | 1 | double bond | N | CH |
| 687 | B | CH₃ | CH₃ | H | CH₃ | H | 7-F | lone pair | O | 1 | double bond | N | CH |
| 688 | B | CH₂CH₂CH₃ | CH₃ | CH₃ | H | H | 7-F | lone pair | O | 1 | double bond | N | CH |
| 689 | B | CH₂CH₂CH₃ | CH₃ | H | CH₃ | H | 7-F | lone pair | O | 1 | double bond | N | CH |
| 690 | C | CH₂CH₂CH₃ | CH₃ | CH₃ | H | H | 7-F | lone pair | O | 1 | double bond | N | CH |
| 691 | C | CH₂CH₂CH₃ | CH₃ | H | CH₃ | H | 7-F | lone pair | O | 1 | double bond | N | CH |
| 692 | C | CH₂CH₂CH₂CH₃ | CH₃ | H | CH₃ | H | - | lone pair | O | 0 | double bond | N | CH |
| 693 | A | CH₂CH₂CH₂CH₃ | CH₃ | Br | CH₃ | H | - | lone pair | O | 0 | double bond | N | CH |
| 694 | A | CH₂CH₂CH₂CH₃ | CH₃ | H | CH₃ | H | - | lone pair | O | 0 | double bond | N | CH |
| 695 | C | CH₂CH₂CH₂CH₃ | CH₃ | H | CH₃ | H | - | lone pair | S | 0 | double bond | N | CH |
| 696 | C | CH₂CH₂CH₂CH₃ | CH₃ | CH₃ | CH₃ | H | - | lone pair | S | 0 | double bond | N | CH |
| 697 | A | CH₃ | CH₃ | Cl | CH₃ | H | 8-CH₃,7-F | lone pair | O | 2 | double bond | N | CH |
| 698 | A | CH₂CH₂CH₃ | CH₃ | Cl | CH₃ | H | 8-CH₃,7-F | lone pair | O | 2 | double bond | N | CH |
| 699 | A | CH₂CH₂CH₂CH₃ | CH₃ | Cl | CH₃ | H | 8-CH₃,7-F | lone pair | O | 2 | double bond | N | CH |

**[Table 7-21]**

| | | | | | | | | | | | | | |
|---|---|---|---|---|---|---|---|---|---|---|---|---|---|
| 700 | A | CH₂CH₂CH₂CH₃ | CH₃ | Cl | CH₃ | H | 8-CH₃, 7-Cl | lone pair | O | 2 | double bond | N | CH |
| 701 | A | CH₂CH(CH₃)CH₃ | CH₃ | Cl | CH₃ | H | 8-CH₃, 7-F | lone pair | O | 2 | double bond | N | CH |
| 702 | A | CH₂CH(CH₃)CH₃ | CH₃ | Cl | CH₃ | H | 8-CH₃, 7-Cl | lone pair | O | 2 | double bond | N | CH |
| 703 | C | CH₂(CH₂)₃CH₃ | CH₃ | CH₃ | CH₃ | H | - | lone pair | S | 0 | double bond | N | CH |
| 704 | C | CH₂(CH₂)₃CH₃ | CH₃ | CH₃ | H | H | - | lone pair | S | 0 | double bond | N | CH |
| 705 | C | CH₂(CH₂)₃CH₃ | CH₃ | H | CH₃ | H | - | lone pair | S | 0 | double bond | N | CH |
| 706 | C | CH₂CH₃ | CH₃ | CH₃ | CH₃ | H | - | lone pair | S | 0 | double bond | N | CH |
| 707 | C | CH₂CH₃ | CH₃ | CH₃ | H | H | - | lone pair | S | 0 | double bond | N | CH |
| 708 | C | CH₂CH₃ | CH₃ | H | CH₃ | H | - | lone pair | S | 0 | double bond | N | CH |
| 709 | C | CH₂CH₂CH₃ | CH₃ | CH₃ | CH₃ | H | 7-CH₃ | lone pair | O | 1 | double bond | N | CH |
| 710 | C | CH₂CH₂CH₃ | CH₃ | CH₃ | H | H | 7-CH₃ | lone pair | O | 1 | double bond | N | CH |
| 711 | C | CH₂CH₂CH₃ | CH₃ | H | CH₃ | H | 7-CH₃ | lone pair | O | 1 | double bond | N | CH |
| 712 | C | CH₂CH₂CH₂CH₃ | CH₃ | CH₃ | CH₃ | H | 7-CH₃ | lone pair | O | 1 | double bond | N | CH |
| 713 | C | CH₂CH₂CH₂CH₃ | CH₃ | CH₃ | H | H | 7-CH₃ | lone pair | O | 1 | double bond | N | CH |
| 714 | C | CH₂CH₂CH₂CH₃ | CH₃ | H | CH₃ | H | 7-CH₃ | lone pair | O | 1 | double bond | N | CH |
| 715 | A | CH₃ | CH₃ | Cl | CH₃ | H | 7-Cl | lone pair | O | 1 | double bond | N | CH |
| 716 | A | CH₂CH₃ | CH₃ | Cl | CH₃ | H | 8-F | lone pair | O | 1 | double bond | N | CH |
| 717 | A | CH₂CH₃ | CH₃ | H | CH₃ | H | 7-Cl | lone pair | O | 1 | double bond | N | CH |
| 718 | A | CH₂CH₃ | CH₃ | Cl | CH₃ | H | 7-Cl | lone pair | O | 1 | double bond | N | CH |
| 719 | B | CH₂CH₂CH₂CH₃ | CH₃ | H | CH₃ | H | - | lone pair | S | 0 | double bond | N | CH |
| 720 | B | CH₂CH₂CH₂CH₃ | CH₃ | CH₃ | CH₃ | H | - | lone pair | S | 0 | double bond | N | CH |
| 721 | B | CH₂CH₃ | CH₃ | CH₃ | H | H | - | lone pair | S | 0 | double bond | N | CH |
| 722 | B | CH₂CH₃ | CH₃ | H | CH₃ | H | - | lone pair | S | 0 | double bond | N | CH |
| 723 | B | CH₂CH₃ | CH₃ | CH₃ | CH₃ | H | - | lone pair | S | 0 | double bond | N | CH |
| 724 | B | CH₂CH₂CH₃ | CH₃ | CH₃ | H | H | 7-Cl | lone pair | O | 1 | double bond | N | CH |
| 725 | B | CH₂CH₂CH₃ | CH₃ | H | CH₃ | H | 7-Cl | lone pair | O | 1 | double bond | N | CH |
| 726 | B | CH₂CH₂CH₃ | CH₃ | CH₃ | CH₃ | H | 7-Cl | lone pair | O | 1 | double bond | N | CH |
| 727 | B | CH₂(CH₂)₃CH₃ | CH₃ | CH₃ | H | H | - | lone pair | S | 0 | double bond | N | CH |
| 728 | B | CH₂(CH₂)₃CH₃ | CH₃ | H | CH₃ | H | - | lone pair | S | 0 | double bond | N | CH |
| 729 | B | CH₂(CH₂)₃CH₃ | CH₃ | CH₃ | CH₃ | H | - | lone pair | S | 0 | double bond | N | CH |
| 730 | B | CH₃ | CH₃ | CH₃ | H | H | 7-Cl | lone pair | O | 1 | double bond | N | CH |
| 731 | B | CH₃ | CH₃ | CH₃ | CH₃ | H | 7-Cl | lone pair | O | 1 | double bond | N | CH |
| 732 | A | CH₂(CH₂)₃CH₃ | CH₃ | Br | CH₃ | H | 8-F | lone pair | O | 1 | double bond | N | CH |
| 733 | A | CH₂(CH₂)₃CH₃ | CH₃ | Br | CH₃ | H | - | lone pair | O | 0 | double bond | N | CH |
| 734 | A | CH₂(CH₂)₃CH₃ | CH₃ | Cl | CH₃ | H | - | lone pair | O | 0 | double bond | N | CH |

**[Table 7-22]**

| | | | | | | | | | | | | | |
|---|---|---|---|---|---|---|---|---|---|---|---|---|---|
| 735 | A | CH₂(CH₂)₃CH₃ | CH₃ | H | CH₃ | H | - | lone pair | O | 0 | double bond | N | CH |
| 736 | A | CH₃ | CH₃ | Br | CH₃ | H | - | lone pair | S | 0 | double bond | N | CH |
| 737 | A | CH₃ | CH₃ | Cl | CH₃ | H | - | lone pair | S | 0 | double bond | N | CH |
| 738 | A | CH₃ | CH₃ | H | CH₃ | H | - | lone pair | S | 0 | double bond | N | CH |
| 739 | A | CH₂CH₂CH₃ | CH₃ | Br | CH₃ | H | 7-CH₃ | lone pair | O | 1 | double bond | N | CH |
| 740 | A | CH₂CH₂CH₃ | CH₃ | Cl | CH₃ | H | 7-CH₃ | lone pair | O | 1 | double bond | N | CH |
| 741 | A | CH₂CH₂CH₃ | CH₃ | H | CH₃ | H | 7-CH₃ | lone pair | O | 1 | double bond | N | CH |
| 742 | A | CH₃ | CH₃ | Br | CH₃ | H | 7-Cl | lone pair | O | 1 | double bond | N | CH |
| 743 | A | CH₃ | CH₃ | H | CH₃ | H | 7-Cl | lone pair | O | 1 | double bond | N | CH |
| 744 | A | CH₂CH₂CH₂CH₃ | CH₃ | CH₃ | CH₃ | H | - | lone pair | O | 0 | double bond | N | CH |
| 745 | A | CH₂(CH₂)₃CH₃ | CH₃ | CH₃ | CH₃ | H | - | lone pair | O | 0 | double bond | N | CH |
| 746 | A | CH₃ | CH₃ | CH₃ | CH₃ | H | - | lone pair | S | 0 | double bond | N | CH |
| 747 | A | CH₂CH₂CH₃ | CH₃ | CH₃ | CH₃ | H | 7-CH₃ | lone pair | O | 1 | double bond | N | CH |
| 748 | B | CH₂CH₂CH₂CH₃ | CH₃ | CH₃ | H | H | - | lone pair | S | 0 | double bond | N | CH |
| 749 | B | CH₃ | CH₃ | H | CH₃ | H | 7-Cl | lone pair | O | 1 | double bond | N | CH |
| 750 | B | CH₂CH₂CH₂CH₃ | CH₃ | CH₃ | H | H | 7-Cl | lone pair | O | 1 | double bond | N | CH |
| 751 | B | CH₂CH₂CH₂CH₃ | CH₃ | H | CH₃ | H | 7-Cl | lone pair | O | 1 | double bond | N | CH |
| 752 | B | CH₂CH(CH₃)CH₃ | CH₃ | CH₃ | H | H | 7-Cl | lone pair | O | 1 | double bond | N | CH |
| 753 | B | CH₂CH(CH₃)CH₃ | CH₃ | H | CH₃ | H | 7-Cl | lone pair | O | 1 | double bond | N | CH |
| 754 | B | CH₂CH(CH₃)CH₃ | CH₃ | CH₃ | CH₃ | H | 7-Cl | lone pair | O | 1 | double bond | N | CH |
| 755 | B | CH₃ | CH₃ | CH₃ | H | H | 8-Cl | lone pair | O | 1 | double bond | N | CH |
| 756 | B | CH₃ | CH₃ | H | CH₃ | H | 8-Cl | lone pair | O | 1 | double bond | N | CH |
| 757 | B | CH₃ | CH₃ | CH₃ | CH₃ | H | 8-Cl | lone pair | O | 1 | double bond | N | CH |
| 758 | B | CH₂CH₂CH₃ | CH₃ | CH₃ | H | H | 8-Cl | lone pair | O | 1 | double bond | N | CH |
| 759 | B | CH₂CH₂CH₃ | CH₃ | H | CH₃ | H | 8-Cl | lone pair | O | 1 | double bond | N | CH |
| 760 | B | CH₂CH₂CH₃ | CH₃ | CH₃ | CH₃ | H | 8-Cl | lone pair | O | 1 | double bond | N | CH |
| 761 | B | CH₂CH₂CH₂CH₃ | CH₃ | CH₃ | H | H | 8-F | lone pair | S | 1 | double bond | N | CH |
| 762 | C | CH₂CH₂CH₂CH₃ | CH₃ | CH₃ | H | H | 8-F | lone pair | S | 1 | double bond | N | CH |
| 763 | C | CH₂CH₂CH₂CH₃ | CH₃ | CH₃ | CH₃ | H | 8-F | lone pair | S | 1 | double bond | N | CH |
| 764 | B | CH₂CH₃ | CH₃ | H | CH₃ | H | 7-Cl | lone pair | O | 1 | double bond | N | CH |
| 765 | B | CH₂CH₃ | CH₃ | CH₃ | CH₃ | H | 7-Cl | lone pair | O | 1 | double bond | N | CH |
| 766 | B | CH₂CH₂CH(CH₃)CH₃ | CH₃ | CH₃ | H | H | - | lone pair | O | 0 | double bond | N | CH |
| 767 | B | CH₂CH₂CH(CH₃)CH₃ | CH₃ | H | CH₃ | H | - | lone pair | O | 0 | double bond | N | CH |
| 768 | B | CH₂CH₂CH(CH₃)CH₃ | CH₃ | CH₃ | CH₃ | H | - | lone pair | O | 0 | double bond | N | CH |
| 769 | B | CH₂CH₂CH₂CH₃ | CH₃ | CH₃ | CH₃ | H | 7-Cl | lone pair | O | 1 | double bond | N | CH |

**[Table 7-23]**

| | | | | | | | | | | | | | |
|---|---|---|---|---|---|---|---|---|---|---|---|---|---|
| 770 | C | CH₂CH₂CH₂CH₃ | CH₃ | CH₃ | H | H | 7-Cl | lone pair | O | 1 | double bond | N | CH |
| 771 | C | CH₂CH₂CH₂CH₃ | CH₃ | H | CH₃ | H | 7-Cl | lone pair | O | 1 | double bond | N | CH |
| 772 | C | CH₂CH₂CH₂CH₃ | CH₃ | CH₃ | CH₃ | H | 7-Cl | lone pair | O | 1 | double bond | N | CH |
| 773 | C | CH₂CH(CH₃)CH₃ | CH₃ | CH₃ | H | H | - | lone pair | O | 0 | double bond | N | CH |
| 774 | C | CH₂CH(CH₃)CH₃ | CH₃ | H | CH₃ | H | - | lone pair | O | 0 | double bond | N | CH |
| 775 | C | CH₂CH(CH₃)CH₃ | CH₃ | CH₃ | CH₃ | H | - | lone pair | O | 0 | double bond | N | CH |
| 776 | C | CH₂CH₂CH₂CH₃ | CH₃ | CH₃ | H | H | - | lone pair | O | 0 | double bond | N | CH |
| 777 | C | CH₂CH₂CH₂CH₃ | CH₃ | CH₃ | CH₃ | H | - | lone pair | O | 0 | double bond | N | CH |
| 778 | A | CH₂CH₂CH₃ | CH₃ | H | CH₃ | H | 7-Cl | lone pair | O | 1 | double bond | N | CH |
| 779 | A | CH₂CH₂CH₃ | CH₃ | Cl | CH₃ | H | 7-Cl | lone pair | O | 1 | double bond | N | CH |
| 780 | C | CH₃ | CH₃ | CH₃ | CH₃ | H | 7-CH₃ | lone pair | O | 1 | double bond | N | CH |
| 781 | C | CH₃ | CH₃ | H | CH₃ | H | 7-CH₃ | lone pair | O | 1 | double bond | N | CH |
| 782 | C | CH₃ | CH₃ | CH₃ | CH₃ | H | 7-Cl | lone pair | O | 1 | double bond | N | CH |
| 783 | C | CH₃ | CH₃ | CH₃ | H | H | 7-Cl | lone pair | O | 1 | double bond | N | CH |
| 784 | C | CH₃ | CH₃ | H | CH₃ | H | 7-Cl | lone pair | O | 1 | double bond | N | CH |
| 785 | A | CH₂CH₃ | CH₃ | Br | CH₃ | H | 7-Cl | lone pair | O | 1 | double bond | N | CH |
| 786 | A | CH₂CH₂CH₃ | CH₃ | Br | CH₃ | H | 7-Cl | lone pair | O | 1 | double bond | N | CH |
| 787 | A | CH₃ | CH₃ | CH₃ | CH₃ | H | 7-Cl | lone pair | O | 1 | double bond | N | CH |
| 788 | A | CH₂CH₂CH₂CH₃ | CH₃ | Br | CH₃ | H | - | lone pair | S | 0 | double bond | N | CH |
| 789 | A | CH₂CH₂CH₂CH₃ | CH₃ | Cl | CH₃ | H | - | lone pair | S | 0 | double bond | N | CH |
| 790 | A | CH₂CH₂CH₂CH₃ | CH₃ | H | CH₃ | H | - | lone pair | S | 0 | double bond | N | CH |
| 791 | A | CH₂CH₂CH₃ | CH₃ | Br | CH₃ | H | - | lone pair | S | 0 | double bond | N | CH |
| 792 | A | CH₂CH₂CH₃ | CH₃ | Cl | CH₃ | H | - | lone pair | S | 0 | double bond | N | CH |
| 793 | A | CH₂CH₂CH₃ | CH₃ | H | CH₃ | H | - | lone pair | S | 0 | double bond | N | CH |
| 794 | A | CH₂CH₃ | CH₃ | Br | CH₃ | H | - | lone pair | S | 0 | double bond | N | CH |
| 795 | A | CH₂CH₃ | CH₃ | Cl | CH₃ | H | - | lone pair | S | 0 | double bond | N | CH |
| 796 | A | CH₂CH₃ | CH₃ | H | CH₃ | H | - | lone pair | S | 0 | double bond | N | CH |
| 797 | A | CH₂CH₂CH₃ | CH₃ | CH₃ | CH₃ | H | - | lone pair | S | 0 | double bond | N | CH |
| 798 | A | CH₂CH₃ | CH₃ | CH₃ | CH₃ | H | - | lone pair | S | 0 | double bond | N | CH |
| 799 | A | CH₂CH₂CH₃ | CH₃ | H | CH₃ | H | 8-CH_{3,}7-F | lone pair | O | 2 | double bond | N | CH |
| 800 | A | CH₂CH₂CH₂CH₃ | CH₃ | H | CH₃ | H | 8-CH₃,7-F | lone pair | O | 2 | double bond | N | CH |
| 801 | A | CH₂CH₂CH₂CH₃ | CH₃ | H | CH₃ | H | 8-CH₃, 7-Cl | lone pair | O | 2 | double bond | N | CH |
| 802 | A | CH₂CH(CH₃)CH₃ | CH₃ | H | CH₃ | H | 8-CH₃,7-F | lone pair | O | 2 | double bond | N | CH |
| 803 | A | CH₂CH(CH₃)CH₃ | CH₃ | H | CH₃ | H | 8-CH₃, 7-Cl | lone pair | O | 2 | double bond | N | CH |
| 804 | A | CH₂CH₃ | CH₃ | H | CH₃ | H | 8-F | lone pair | O | 1 | double bond | N | CH |

**[Table 7-24]**

| | | | | | | | | | | | | | |
|---|---|---|---|---|---|---|---|---|---|---|---|---|---|
| 805 | A | CH₃ | CH₃ | Br | CH₃ | H | 8-Cl | lone pair | O | 1 | double bond | N | CH |
| 806 | A | CH₃ | CH₃ | Cl | CH₃ | H | 8-Cl | lone pair | O | 1 | double bond | N | CH |
| 807 | A | CH₃ | CH₃ | H | CH₃ | H | 8-Cl | lone pair | O | 1 | double bond | N | CH |
| 808 | A | CH₂CH₂CH₃ | CH₃ | Br | CH₃ | H | 8-Cl | lone pair | O | 1 | double bond | N | CH |
| 809 | A | CH₂CH₂CH₃ | CH₃ | Cl | CH₃ | H | 8-Cl | lone pair | O | 1 | double bond | N | CH |
| 810 | A | CH₂CH₂CH₃ | CH₃ | H | CH₃ | H | 8-Cl | lone pair | O | 1 | double bond | N | CH |
| 811 | A | CH₂CH₂CH₂CH₃ | CH₃ | CH₃ | CH₃ | H | - | lone pair | S | 0 | double bond | N | CH |
| 812 | A | CH₃ | CH₃ | H | CH₃ | H | 7-F | lone pair | O | 1 | double bond | N | CH |
| 813 | B | CH₂CH₂CH(CH₃)CH₃ | CH₃ | CH₃ | H | H | 8-F | lone pair | O | 1 | double bond | N | CH |
| 814 | B | CH₂CH₂CH(CH₃)CH₃ | CH₃ | H | CH₃ | H | 8-F | lone pair | O | 1 | double bond | N | CH |
| 815 | B | CH₂CH₂CH(CH₃)CH₃ | CH₃ | CH₃ | CH₃ | H | 8-F | lone pair | O | 1 | double bond | N | CH |
| 816 | C | CH₂CH₂CH(CH₃)CH₃ | CH₃ | H | CH₃ | H | - | lone pair | O | 0 | double bond | N | CH |
| 817 | C | CH₂CH₂CH(CH₃)CH₃ | CH₃ | CH₃ | CH₃ | H | - | lone pair | O | 0 | double bond | N | CH |
| 818 | C | CH₂CH₂CH₃ | CH₃ | CH₃ | CH₃ | H | 7-F | lone pair | O | 1 | double bond | N | CH |
| 819 | A | CH₂CH₂CH₃ | CH₃ | H | CH₃ | H | 7-F | lone pair | O | 1 | double bond | N | CH |
| 820 | A | CH₂CH₂CH₃ | CH₃ | Cl | CH₃ | H | 7-F | lone pair | O | 1 | double bond | N | CH |
| 821 | A | CH₂CH₂CH₃ | CH₃ | Br | CH₃ | H | 7-F | lone pair | O | 1 | double bond | N | CH |
| 822 | A | CH₂CH₂CH₃ | CH₃ | Br | CH₃ | H | 7-Br | lone pair | O | 1 | double bond | N | CH |
| 823 | A | CH₃ | CH₃ | H | CH₃ | H | 8-F,7-F | lone pair | O | 2 | double bond | N | CH |
| 824 | A | CH₃ | CH₃ | Cl | CH₃ | H | 8-F,7-F | lone pair | O | 2 | double bond | N | CH |
| 825 | C | CH₂CH₂CH₂CH₃ | CH₃ | CH₃ | CH₃ | H | 7-F | lone pair | O | 1 | double bond | N | CH |
| 826 | A | CH₂CH₂CH₂CH₃ | CH₃ | H | CH₃ | H | 7-F | lone pair | O | 1 | double bond | N | CH |
| 827 | A | CH₂CH₂CH₂CH₃ | CH₃ | Cl | CH₃ | H | 7-F | lone pair | O | 1 | double bond | N | CH |
| 828 | A | CH₂CH₂CH₂CH₃ | CH₃ | Br | CH₃ | H | 7-F | lone pair | O | 1 | double bond | N | CH |
| 829 | A | CH₂CH₂CH₂CH₃ | CH₃ | CH₃ | CH₃ | H | 7-F | lone pair | O | 1 | double bond | N | CH |
| 830 | A | CH₂CH₂CH₃ | CH₃ | CH₃ | CH₃ | H | 7-F | lone pair | O | 1 | double bond | N | CH |
| 831 | C | CH₂CH₃ | CH₃ | CH₃ | CH₃ | H | 7-Cl | lone pair | O | 1 | double bond | N | CH |
| 832 | C | CH₂CH₃ | CH₃ | CH₃ | H | H | 7-Cl | lone pair | O | 1 | double bond | N | CH |
| 833 | C | CH₂CH₃ | CH₃ | H | CH₃ | H | 7-Cl | lone pair | O | 1 | double bond | N | CH |
| 834 | C | CH₂CH₂CH₃ | CH₃ | CH₃ | CH₃ | H | 7-Cl | lone pair | O | 1 | double bond | N | CH |
| 835 | C | CH₂CH₂CH₃ | CH₃ | CH₃ | H | H | 7-Cl | lone pair | O | 1 | double bond | N | CH |
| 836 | C | CH₂CH₂CH₃ | CH₃ | H | CH₃ | H | 7-Cl | lone pair | O | 1 | double bond | N | CH |
| 837 | C | CH₂(CH₂)₃CH₃ | CH₃ | CH₃ | CH₃ | H | 8-CH₃ | lone pair | O | 1 | double bond | N | CH |
| 838 | C | CH₂(CH₂)₃CH₃ | CH₃ | CH₃ | H | H | 8-CH₃ | lone pair | O | 1 | double bond | N | CH |
| 839 | C | CH₂(CH₂)₃CH₃ | CH₃ | H | CH₃ | H | 8-CH₃ | lone pair | O | 1 | double bond | N | CH |

**[Table 7-25]**

| | | | | | | | | | | | | | |
|---|---|---|---|---|---|---|---|---|---|---|---|---|---|
| 840 | C | CH₂CH(CH₃)CH₃ | CH₃ | CH₃ | CH₃ | H | 8-CH₃ | lone pair | O | 1 | double bond | N | CH |
| 841 | C | CH₂CH(CH₃)CH₃ | CH₃ | CH₃ | H | H | 8-CH₃ | lone pair | O | 1 | double bond | N | CH |
| 842 | C | CH₂CH(CH₃)CH₃ | CH₃ | H | CH₃ | H | 8-CH₃ | lone pair | O | 1 | double bond | N | CH |
| 843 | C | CH₂CH₂CH₂CH₃ | CH₃ | CH₃ | CH₃ | H | 8-CH₃,7-F | lone pair | O | 2 | double bond | N | CH |
| 844 | C | CH₂CH₂CH₂CH₃ | CH₃ | CH₃ | H | H | 8-CH₃,7-F | lone pair | O | 2 | double bond | N | CH |
| 845 | C | CH₂CH₂CH₂CH₃ | CH₃ | H | CH₃ | H | 8-CH₃,7-F | lone pair | O | 2 | double bond | N | CH |
| 846 | C | CH₂CH(CH₃)CH₃ | CH₃ | CH₃ | CH₃ | H | 8-CH₃,7-F | lone pair | O | 2 | double bond | N | CH |
| 847 | C | CH₂CH(CH₃)CH₃ | CH₃ | CH₃ | H | H | 8-CH₃,7-F | lone pair | O | 2 | double bond | N | CH |
| 848 | C | CH₂CH(CH₃)CH₃ | CH₃ | H | CH₃ | H | 8-CH₃ | lone pair | O | 2 | double bond | N | CH |
| 849 | B | CH₂CH(CH₃)CH₃ | CH₃ | CH₃ | H | H | - | lone pair | S | 0 | double bond | N | CH |
| 850 | B | CH₂CH(CH₃)CH₃ | CH₃ | H | CH₃ | H | - | lone pair | S | 0 | double bond | N | CH |
| 851 | B | CH₂CH(CH₃)CH₃ | CH₃ | CH₃ | CH₃ | H | - | lone pair | S | 0 | double bond | N | CH |
| 852 | A | CH₃ | CH₃ | H | CH₃ | H | 8-CH₃,7-F | lone pair | O | 2 | double bond | N | CH |
| 853 | A | CH₂CH₂CH(CH₃)CH₃ | CH₃ | H | CH₃ | H | 8-F | lone pair | O | 1 | double bond | N | CH |
| 854 | A | CH₂CH₂CH(CH₃)CH₃ | CH₃ | Cl | CH₃ | H | 8-F | lone pair | O | 1 | double bond | N | CH |
| 855 | A | CH₂CH₂CH₂CH₃ | CH₃ | H | CH₃ | H | 7-Cl | lone pair | O | 1 | double bond | N | CH |
| 856 | A | CH₂CH₂CH₂CH₃ | CH₃ | Cl | CH₃ | H | 7-Cl | lone pair | O | 1 | double bond | N | CH |
| 857 | A | CH₂CH₃ | CH₃ | H | CH₃ | H | 8-CH₃ | lone pair | O | 1 | double bond | N | CH |
| 858 | A | CH₂CH₃ | CH₃ | Cl | CH₃ | H | 8-CH₃ | lone pair | O | 1 | double bond | N | CH |
| 859 | A | CH₂CH(CH₃)CH₃ | CH₃ | H | CH₃ | H | 8-CH₃ | lone pair | O | 1 | double bond | N | CH |
| 860 | A | CH₂CH(CH₃)CH₃ | CH₃ | Cl | CH₃ | H | 8-CH₃ | lone pair | O | 1 | double bond | N | CH |
| 861 | A | CH₂CH(CH₃)CH₃ | CH₃ | Br | CH₃ | H | - | lone pair | O | 0 | double bond | N | CH |
| 862 | A | CH₂CH(CH₃)CH₃ | CH₃ | Cl | CH₃ | H | - | lone pair | O | 0 | double bond | N | CH |
| 863 | A | CH₂CH(CH₃)CH₃ | CH₃ | H | CH₃ | H | - | lone pair | O | 0 | double bond | N | CH |
| 864 | A | CH₂CH₂CH(CH₃)CH₃ | CH₃ | Br | CH₃ | H | 8-F | lone pair | O | 1 | double bond | N | CH |
| 865 | A | CH₂CH₂OCH₂CH₃ | CH₃ | Br | CH₃ | H | - | lone pair | O | 0 | double bond | N | CH |
| 866 | A | CH₂CH₂OCH₂CH₃ | CH₃ | Cl | CH₃ | H | - | lone pair | O | 0 | double bond | N | CH |
| 867 | A | CH₂CH₂OCH₂CH₃ | CH₃ | H | CH₃ | H | - | lone pair | O | 0 | double bond | N | CH |
| 868 | A | CH₂CH₂CH₂CH₃ | CH₃ | Br | CH₃ | H | 7-Cl | lone pair | O | 1 | double bond | N | CH |
| 869 | B | CH₂CH₂CH₂CH₃ | CH₃ | CH₃ | H | H | 8-Cl | lone pair | O | 1 | double bond | N | CH |
| 870 | B | CH₂CH₂CH₂CH₃ | CH₃ | H | CH₃ | H | 8-Cl | lone pair | O | 1 | double bond | N | CH |
| 871 | B | CH₂CH₂CH₂CH₃ | CH₃ | CH₃ | CH₃ | H | 8-Cl | lone pair | O | 1 | double bond | N | CH |
| 872 | C | CH₂(CH₂)₃CH₃ | CH₃ | CH₃ | H | H | 8-F | lone pair | O | 1 | double bond | N | CH |
| 873 | C | CH₂(CH₂)₃CH₃ | CH₃ | H | CH₃ | H | 8-F | lone pair | O | 1 | double bond | N | CH |
| 874 | C | CH₂(CH₂)₃CH₃ | CH₃ | CH₃ | CH₃ | H | 8-F | lone pair | O | 1 | double bond | N | CH |

**[Table 7-26]**

| | | | | | | | | | | | | | |
|---|---|---|---|---|---|---|---|---|---|---|---|---|---|
| 875 | C | CH₂CH₂CH₃ | CH₃ | CH₃ | H | H | 8-F | lone pair | O | 1 | double bond | N | CH |
| 876 | C | CH₂CH₂CH₃ | CH₃ | H | CH₃ | H | 8-F | lone pair | O | 1 | double bond | N | CH |
| 877 | C | CH₂CH₂CH₃ | CH₃ | CH₃ | CH₃ | H | 8-F | lone pair | O | 1 | double bond | N | CH |
| 878 | C | CH₂CH(CH₃)CH₃ | CH₃ | CH₃ | CH₃ | H | - | lone pair | S | 0 | double bond | N | CH |
| 879 | C | CH₂CH(CH₃)CH₃ | CH₃ | CH₃ | H | H | - | lone pair | S | 0 | double bond | N | CH |
| 880 | C | CH₂CH(CH₃)CH₃ | CH₃ | H | CH₃ | H | - | lone pair | S | 0 | double bond | N | CH |
| 881 | A | CH₂(CH₂)₃CH₃ | CH₃ | H | CH₃ | H | 8-CH₃ | lone pair | O | 1 | double bond | N | CH |
| 882 | A | CH₂(CH₂)₃CH₃ | CH₃ | Cl | CH₃ | H | 8-CH₃ | lone pair | O | 1 | double bond | N | CH |
| 883 | A | CH₂CH₂CH(CH₃)CH₃ | CH₃ | H | CH₃ | H | - | lone pair | O | 0 | double bond | N | CH |
| 884 | A | CH₂CH₂CH(CH₃)CH₃ | CH₃ | Cl | CH₃ | H | - | lone pair | O | 0 | double bond | N | CH |
| 885 | A | CH₂CH₂CH(CH₃)CH₃ | CH₃ | Br | CH₃ | H | - | lone pair | O | 0 | double bond | N | CH |
| 886 | A | CH₂CH(CH₃)CH₃ | CH₃ | H | CH₃ | H | - | lone pair | S | 0 | double bond | N | CH |
| 887 | A | CH₂CH(CH₃)CH₃ | CH₃ | Cl | CH₃ | H | - | lone pair | S | 0 | double bond | N | CH |
| 888 | A | CH₂CH(CH₃)CH₃ | CH₃ | Br | CH₃ | H | - | lone pair | S | 0 | double bond | N | CH |
| 889 | A | CH₂CH₂CH(CH₃)CH₃ | CH₃ | CH₃ | CH₃ | H | - | lone pair | O | 0 | double bond | N | CH |
| 890 | A | CH₂CH(CH₃)CH₃ | CH₃ | CH₃ | CH₃ | H | - | lone pair | S | 0 | double bond | N | CH |
| 891 | A | CH₂(CH₂)₃CH₃ | CH₃ | H | CH₃ | H | - | lone pair | S | 0 | double bond | N | CH |
| 892 | A | CH₂CH₃ | CH₃ | CH₃ | CH₃ | H | 7-Cl | lone pair | O | 1 | double bond | N | CH |
| 893 | A | CH₂CH₃ | CH₃ | Br | CH₃ | H | 8-CH₃ | lone pair | O | 1 | double bond | N | CH |
| 894 | A | CH₂(CH₂)₃CH₃ | CH₃ | Br | CH₃ | H | 8-CH₃ | lone pair | O | 1 | double bond | N | CH |
| 895 | A | CH₂CH₂CH₂CH₃ | CH₃ | Cl | CH₃ | H | 7-CH₃ | lone pair | O | 1 | double bond | N | CH |
| 896 | A | CH₂CH₂CH₂CH₃ | CH₃ | H | CH₃ | H | 7-CH₃ | lone pair | O | 1 | double bond | N | CH |
| 897 | A | CH₂CH₂CH₂CH₃ | CH₃ | Cl | CH₃ | H | 8-F | lone pair | S | 1 | double bond | N | CH |
| 898 | A | CH₂CH₂CH₂CH₃ | CH₃ | H | CH₃ | H | 8-F | lone pair | S | 1 | double bond | N | CH |
| 899 | A | CH₃ | CH₃ | CH₃ | CH₃ | H | 8-Cl | lone pair | O | 1 | double bond | N | CH |
| 900 | A | CH₂CH₂CH₃ | CH₃ | CH₃ | CH₃ | H | 8-Cl | lone pair | O | 1 | double bond | N | CH |
| 901 | A | CH₂CH(CH₃)CH₃ | CH₃ | CH₃ | CH₃ | H | - | lone pair | O | 0 | double bond | N | CH |
| 902 | A | CH₂CH₂CH(CH₃)CH₃ | CH₃ | CH₃ | CH₃ | H | 8-F | lone pair | O | 1 | double bond | N | CH |
| 903 | A | CH₂(CH₂)₃CH₃ | CH₃ | Cl | CH₃ | H | - | lone pair | S | 0 | double bond | N | CH |
| 904 | A | CH₂(CH₂)₃CH₃ | CH₃ | Br | CH₃ | H | - | lone pair | S | 0 | double bond | N | CH |
| 905 | C | CH₃ | CH₃ | CH₃ | CH₃ | H | 8-Cl | lone pair | O | 1 | double bond | N | CH |
| 906 | C | CH₃ | CH₃ | CH₃ | H | H | 8-Cl | lone pair | O | 1 | double bond | N | CH |
| 907 | C | CH₃ | CH₃ | H | CH₃ | H | 8-Cl | lone pair | O | 1 | double bond | N | CH |
| 908 | C | CH₂CH₂CH₃ | CH₃ | CH₃ | CH₃ | H | 8-Cl | lone pair | O | 1 | double bond | N | CH |
| 909 | C | CH₂CH₂CH₃ | CH₃ | CH₃ | H | H | 8-Cl | lone pair | O | 1 | double bond | N | CH |

**[Table 7-27]**

| | | | | | | | | | | | | | |
|---|---|---|---|---|---|---|---|---|---|---|---|---|---|
| 910 | C | CH₂CH₂CH₃ | CH₃ | H | CH₃ | H | 8-Cl | lone pair | O | 1 | double bond | N | CH |
| 911 | C | CH₂CH₂CH₂CH₃ | CH₃ | CH₃ | CH₃ | H | 8-Cl | lone pair | O | 1 | double bond | N | CH |
| 912 | C | CH₂CH₂CH₂CH₃ | CH₃ | CH₃ | H | H | 8-Cl | lone pair | O | 1 | double bond | N | CH |
| 913 | C | CH₂CH₂CH₂CH₃ | CH₃ | H | CH₃ | H | 8-Cl | lone pair | O | 1 | double bond | N | CH |
| 914 | C | CH₂CH(CH₃)CH₃ | CH₃ | CH₃ | CH₃ | H | 8-Cl | lone pair | O | 1 | double bond | N | CH |
| 915 | C | CH₂CH(CH₃)CH₃ | CH₃ | CH₃ | H | H | 8-Cl | lone pair | O | 1 | double bond | N | CH |
| 916 | C | CH₂CH(CH₃)CH₃ | CH₃ | H | CH₃ | H | 8-Cl | lone pair | O | 1 | double bond | N | CH |
| 917 | C | CH₂CH₂CH(CH₃)CH₃ | CH₃ | CH₃ | H | H | - | lone pair | O | 0 | double bond | N | CH |
| 918 | B | CH₂CH(CH₃)CH₃ | CH₃ | CH₃ | H | H | 8-Cl | lone pair | O | 1 | double bond | N | CH |
| 919 | B | CH₂CH(CH₃)CH₃ | CH₃ | H | CH₃ | H | 8-Cl | lone pair | O | 1 | double bond | N | CH |
| 920 | B | CH₂CH(CH₃)CH₃ | CH₃ | CH₃ | CH₃ | H | 8-Cl | lone pair | O | 1 | double bond | N | CH |
| 921 | B | CH₂C(O)OCH₂CH₃ | CH₃ | CH₃ | CH₃ | H | - | lone pair | O | 0 | double bond | N | CH |
| 922 | B | CH₂C(O)OCH₂CH₃ | CH₃ | CH₃ | H | H | - | lone pair | O | 0 | double bond | N | CH |
| 923 | B | CH₂C(O)OCH₂CH₃ | CH₃ | H | CH₃ | H | - | lone pair | O | 0 | double bond | N | CH |
| 924 | C | CH₂C(O)OCH₂CH₃ | CH₃ | H | CH₃ | H | - | lone pair | O | 0 | double bond | N | CH |
| 925 | C | CH₂C(O)OCH₂CH₃ | CH₃ | CH₃ | CH₃ | H | - | lone pair | O | 0 | double bond | N | CH |
| 926 | B | CH₂C(O)OCH₂CH₃ | CH₃ | CH₃ | H | H | - | lone pair | S | 0 | double bond | N | CH |
| 927 | B | CH₂C(O)OCH₂CH₃ | CH₃ | H | CH₃ | H | - | lone pair | S | 0 | double bond | N | CH |
| 928 | B | CH₂C(O)OCH₂CH₃ | CH₃ | CH₃ | CH₃ | H | - | lone pair | S | 0 | double bond | N | CH |
| 929 | A | CH₂CH(CH₃)CH₃ | CH₃ | Br | CH₃ | H | 8-CH₃ | lone pair | O | 1 | double bond | N | CH |
| 930 | A | CH₂CH₂CH₂CH₃ | CH₃ | Br | CH₃ | H | 7-CH₃ | lone pair | O | 1 | double bond | N | CH |
| 931 | A | CH₂CH₂CH₂CH₃ | CH₃ | Br | CH₃ | H | 8-F | lone pair | S | 1 | double bond | N | CH |
| 932 | A | CH₂CH₂OCH₂CH₃ | CH₃ | CH₃ | CH₃ | H | - | lone pair | O | 0 | double bond | N | CH |
| 933 | A | CH₂CH₂CH₂CH₃ | CH₃ | CH₃ | CH₃ | H | 7-Cl | lone pair | O | 1 | double bond | N | CH |
| 934 | A | CH₂CH₂CH₂CH₃ | CH₃ | CH₃ | CH₃ | H | 7-CH₃ | lone pair | O | 1 | double bond | N | CH |
| 935 | A | CH₂CH₃ | CH₃ | CH₃ | CH₃ | H | 8-CH₃ | lone pair | O | 1 | double bond | N | CH |
| 936 | A | CH₂(CH₂)₃CH₃ | CH₃ | CH₃ | CH₃ | H | 8-CH₃ | lone pair | O | 1 | double bond | N | CH |
| 937 | A | CH₂CH₂CH₂CH₃ | CH₃ | Cl | CH₃ | H | 8-Cl | lone pair | O | 1 | double bond | N | CH |
| 938 | A | CH₂CH₂CH₂CH₃ | CH₃ | H | CH₃ | H | 8-Cl | lone pair | O | 1 | double bond | N | CH |
| 939 | A | CH₂CH(CH₃)CH₃ | CH₃ | CH₃ | CH₃ | H | 8-CH₃ | lone pair | O | 1 | double bond | N | CH |
| 940 | A | CH₂CH₂CH₂CH₃ | CH₃ | CH₃ | CH₃ | H | 8-F | lone pair | S | 1 | double bond | N | CH |
| 941 | C | CH₂CH₂CH₂CH₃ | CH₃ | CH₃ | H | H | 8-CH₃ | lone pair | O | 1 | double bond | N | CH |
| 942 | C | CH₂CH₂CH₂CH₃ | CH₃ | CH₃ | H | H | 8-F | lone pair | O | 1 | double bond | N | CH |
| 943 | C | CH₂CH₂CH₂CH₃ | CH₃ | CH₃ | H | H | - | lone pair | S | 0 | double bond | N | CH |
| 944 | C | CH₂C(O)OCH₂CH₃ | CH₃ | CH₃ | CH₃ | H | - | lone pair | S | 0 | double bond | N | CH |

**[Table 7-28]**

| | | | | | | | | | | | | | |
|---|---|---|---|---|---|---|---|---|---|---|---|---|---|
| 945 | C | CH₂C(O)OCH₂CH₃ | CH₃ | CH₃ | H | H | - | lone pair | S | 0 | double bond | N | CH |
| 946 | C | CH₂C(O)OCH₂CH₃ | CH₃ | H | CH₃ | H | - | lone pair | S | 0 | double bond | N | CH |
| 947 | C | C(O)OCH₂CH₃ | CH₃ | CH₃ | CH₃ | H | - | lone pair | O | 0 | double bond | N | CH |
| 948 | C | C(O)OCH₂CH₃ | CH₃ | CH₃ | H | H | - | lone pair | O | 0 | double bond | N | CH |
| 949 | C | C(O)OCH₂CH₃ | CH₃ | H | CH₃ | H | - | lone pair | O | 0 | double bond | N | CH |
| 950 | A | CH₂C(O)OCH₂CH₃ | CH₃ | H | CH₃ | H | - | lone pair | O | 0 | double bond | N | CH |
| 951 | A | CH₂C(O)OCH₂CH₃ | CH₃ | Cl | CH₃ | H | - | lone pair | O | 0 | double bond | N | CH |
| 952 | A | CH₂(CH₂)₃CH₃ | CH₃ | CH₃ | CH₃ | H | - | lone pair | S | 0 | double bond | N | CH |
| 953 | A | CH₂C(O)OCH₂CH₃ | CH₃ | CH₃ | CH₃ | H | - | lone pair | O | 0 | double bond | N | CH |
| 954 | B | CH₂(CH₂)4CH₃ | CH₃ | CH₃ | H | H | - | lone pair | O | 0 | double bond | N | CCH₃ |
| 955 | B | CH₂(CH₂)4CH₃ | CH₃ | CH₃ | CH₃ | H | - | lone pair | O | 0 | double bond | N | CH |
| 956 | B | CH₂CH₂C(O)OCH₃ | CH₃ | CH₃ | CH₃ | H | - | lone pair | O | 0 | double bond | N | CH |
| 957 | B | CH₂-4-F-Ph | CH₃ | CH₃ | CH₃ | H | - | lone pair | O | 0 | double bond | N | CH |
| 958 | B | CH₂CH₂CH=CH₂ | CH₃ | CH₃ | CH₃ | H | - | lone pair | O | 0 | double bond | N | CH |
| 959 | B | CH₂CH₂Ph | CH₃ | CH₃ | CH₃ | H | - | lone pair | O | 0 | double bond | N | CH |
| 960 | B | C(O)OCH₂CH₃ | CH₃ | CH₃ | CH₃ | H | - | lone pair | O | 0 | double bond | N | CH |
| 961 | B | CH₂CH₂CH=C(CH₃)CH₃ | CH₃ | CH₃ | CH₃ | H | - | lone pair | O | 0 | double bond | N | CH |
| 962 | C | CH₃ | CH₃ | CH₃ | H | H | 7-CH₃ | lone pair | O | 1 | double bond | N | CH |
| 963 | C | CH₂C(O)OCH₂CH₃ | CH₃ | CH₃ | CH₃ | H | 8-F | lone pair | O | 1 | double bond | N | CH |
| 964 | C | CH₂C(O)OCH₂CH₃ | CH₃ | CH₃ | H | H | 8-F | lone pair | O | 1 | double bond | N | CH |
| 965 | C | CH₂C(O)OCH₂CH₃ | CH₃ | H | CH₃ | H | 8-F | lone pair | O | 1 | double bond | N | CH |
| 966 | C | CH₂C(O)OCH₂CH₃ | CH₃ | CH₃ | CH₃ | H | 8-F | lone pair | S | 1 | double bond | N | CH |
| 967 | C | CH₂C(O)OCH₂CH₃ | CH₃ | H | CH₃ | H | 8-F | lone pair | S | 1 | double bond | N | CH |
| 968 | C | CH₃ | CH₃ | CH₃ | CH₃ | H | 8-CH₃O | lone pair | O | 1 | double bond | N | CH |
| 969 | C | CH₃ | CH₃ | CH₃ | H | H | 8-CH₃O | lone pair | O | 1 | double bond | N | CH |
| 970 | C | CH₃ | CH₃ | H | CH₃ | H | 8-CH₃O | lone pair | O | 1 | double bond | N | CH |
| 971 | C | CH₂CH₂CH₂CH₃ | CH₃ | CH₃ | CH₃ | H | 8-CH₃O | lone pair | O | 1 | double bond | N | CH |
| 972 | C | CH₂CH₂CH₂CH₃ | CH₃ | CH₃ | H | H | 8-CH₃O | lone pair | O | 1 | double bond | N | CH |
| 973 | C | CH₂CH₂CH₂CH₃ | CH₃ | H | CH₃ | H | 8-CH₃O | lone pair | O | 1 | double bond | N | CH |
| 974 | B | CH₂CH₂CH₂CN | CH₃ | CH₃ | CH₃ | H | - | lone pair | O | 0 | double bond | N | CH |
| 975 | B | CH₂-2,6-diCl-Ph | CH₃ | CH₃ | CH₃ | H | - | lone pair | O | 0 | double bond | N | CH |
| 976 | B | Ph | CH₃ | CH₃ | CH₃ | H | - | lone pair | O | 0 | double bond | N | CH |
| 977 | B | C(O)OCH₂CH₃ | CH₃ | CH₃ | H | H | 8-F | lone pair | O | 1 | double bond | N | CH |
| 978 | B | 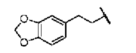 | CH₃ | CH₃ | CH₃ | H | - | lone pair | O | 0 | double bond | N | CH |

**[Table 7-29]**

| | | | | | | | | | | | | | |
|---|---|---|---|---|---|---|---|---|---|---|---|---|---|
| 979 | A | CH₂CH₂CH₂CH₃ | CH₃ | Br | CH₃ | H | 8-Cl | lone pair | O | 1 | double bond | N | CH |
| 980 | A | CH₂CH(CH₃)CH₃ | CH₃ | Br | CH₃ | H | 8-CH₃,7-F | lone pair | O | 2 | double bond | N | CH |
| 981 | A | CH₃ | CH₃ | Cl | CH₃ | H | 7-F | lone pair | O | 1 | double bond | N | CH |
| 982 | A | CH₃ | CH₃ | Br | CH₃ | H | 7-F | lone pair | O | 1 | double bond | N | CH |
| 983 | A | C(O)OCH₂CH₃ | CH₃ | Cl | CH₃ | H | - | lone pair | O | 0 | double bond | N | CH |
| 984 | A | C(O)OCH₂CH₃ | CH₃ | H | CH₃ | H | - | lone pair | O | 0 | double bond | N | CH |
| 985 | A | CH₂CH₂CH₂CH₃ | CH₃ | CH₃ | CH₃ | H | 8-Cl | lone pair | O | 1 | double bond | N | CH |
| 986 | A | CH₂CH(CH₃)CH₃ | CH₃ | CH₃ | CH₃ | H | 8-CH₃,7-F | lone pair | O | 2 | double bond | N | CH |
| 987 | A | CH₃ | CH₃ | CH₃ | CH₃ | H | 7-F | lone pair | O | 1 | double bond | N | CH |
| 988 | A | CH₂C(O)OCH₂CH₃ | CH₃ | H | CH₃ | H | - | lone pair | S | 0 | double bond | N | CH |
| 989 | A | CH₂C(O)OCH₂CH₃ | CH₃ | Cl | CH₃ | H | - | lone pair | S | 0 | double bond | N | CH |
| 990 | A | CH₂C(O)OCH₂CH₃ | CH₃ | Br | CH₃ | H | - | lone pair | S | 0 | double bond | N | CH |
| 991 | A | C(O)OCH₂CH₃ | CH₃ | H | CH₃ | H | 8-F | lone pair | O | 1 | double bond | N | CH |
| 992 | A | C(O)OCH₂CH₃ | CH₃ | Cl | CH₃ | H | 8-F | lone pair | O | 1 | double bond | N | CH |
| 993 | A | C(O)OCH_{z}CH₃ | CH₃ | Br | CH₃ | H | 8-F | lone pair | O | 1 | double bond | N | CH |
| 994 | A | CH₂C(O)OCH₂CH₃ | CH₃ | H | CH₃ | H | 8-F | lone pair | O | 1 | double bond | N | CH |
| 995 | A | CH₂C(O)OCH₂CH₃ | CH₃ | Cl | CH₃ | H | 8-F | lone pair | O | 1 | double bond | N | CH |
| 996 | A | CH₂C(O)OCH₂CH₃ | CH₃ | Br | CH₃ | H | 8-F | lone pair | O | 1 | double bond | N | CH |
| 997 | B | CH₂CH₂CH₂CH₃ | CH₃ | H | CH₃ | H | 8-OMe | lone pair | O | 1 | double bond | N | CH |
| 998 | B | CH₂CH₂-4-MeO-Ph | CH₃ | CH₃ | CH₃ | H | - | lone pair | O | 0 | double bond | N | CH |
| 999 | B | CH₂CH₂-4-MeO-Ph | CH₃ | CH₃ | H | H | - | lone pair | O | 0 | double bond | N | CCH₃ |
| 1000 | B | 3-MeO-Ph | CH₃ | CH₃ | CH₃ | H | - | lone pair | O | 0 | double bond | N | CH |
| 1001 | B | 3-MeO-Ph | CH₃ | CH₃ | H | H | - | lone pair | O | 0 | double bond | N | CCH₃ |
| 1002 | B | CH₂-2-MeO-Ph | CH₃ | CH₃ | CH₃ | H | - | lone pair | O | 0 | double bond | N | CH |
| 1003 | B | CH₂-4-MeO-Ph | CH₃ | CH₃ | CH₃ | H | - | lone pair | O | 0 | double bond | N | CH |
| 1004 | B | CH₂-4-MeO-Ph | CH₃ | CH₃ | H | H | - | lone pair | O | 0 | double bond | N | CCH₃ |
| 1005 | B | CH₂CH₂C(CH₂)CH₃ | CH₃ | CH₃ | CH₃ | H | - | lone pair | O | 0 | double bond | N | CH |
| 1006 | B | CH_{2C}H_{2C}(CH₂)CH₃ | CH₃ | CH₃ | H | H | - | lone pair | O | 0 | double bond | N | CCH₃ |
| 1007 | A | C(O)OCH₂CH₃ | CH₃ | Br | CH₃ | H | - | lone pair | O | 0 | double bond | N | CH |
| 1008 | A | C(O)OCH₂CH₃ | CH₃ | Br | CH₃ | H | 8-F | lone pair | S | 1 | double bond | N | CH |
| 1009 | A | C(O)OCH₂CH₃ | CH₃ | Cl | CH₃ | H | B-F | lone pair | S | 1 | double bond | N | CH |
| 1010 | A | C(O)OCH₂CH₃ | CH₃ | H | CH₃ | H | 8-F | lone pair | S | 1 | double bond | N | CH |
| 1011 | A | CH₂C(O)OCH₂CH₃ | CH₃ | Br | CH₃ | H | 8-F | lone pair | S | 2 | double bond | N | CH |
| 1012 | A | CH₂C(O)OCH₂CH₃ | CH₃ | Cl | CH₃ | H | 8-F | lone pair | S | 1 | double bond | N | CH |
| 1013 | A | C(O)OCH₂CH₂CH₃ | CH₃ | Br | CH₃ | H | - | lone pair | O | 0 | double bond | N | CH |

**[Table 7-30]**

| | | | | | | | | | | | | | |
|---|---|---|---|---|---|---|---|---|---|---|---|---|---|
| 1014 | A | CH₂CH₂CH₂CH₃ | CH₃ | Br | CH₃ | H | 8-OMe | lone pair | O | 1 | double bond | N | CH |
| 1015 | A | CH₂CH₂CH₂CH₃ | CH₃ | Cl | CH₃ | H | 8-OMe | lone pair | O | 1 | double bond | N | CH |
| 1016 | A | CH₂CH₂CH₂CH₃ | CH₃ | H | CH₃ | H | 8-OMe | lone pair | O | 1 | double bond | N | CH |
| 1017 | A | C(O)OCH₂CH₃ | CH₃ | CH₃ | CH₃ | H | - | lone pair | O | 0 | double bond | N | CH |
| 1018 | A | C(O)OCH₂CH₃ | CH₃ | CH₃ | CH₃ | H | 8-F | lone pair | S | 1 | double bond | N | CH |
| 1019 | A | CH₂C(O)OCH₂CH₃ | CH₃ | CH₃ | CH₃ | H | 8-F | lone pair | S | 1 | double bond | N | CH |
| 1020 | A | CH₂C(O)OCH₂CH₃ | CH₃ | CH₃ | CH₃ | H | - | lone pair | S | 0 | double bond | N | CH |
| 1021 | A | C(O)OCH₂CH₃ | CH₃ | CH₃ | CH₃ | H | 8-F | lone pair | O | 1 | double bond | N | CH |
| 1022 | A | CH₂C(O)OCH₂CH₃ | CH₃ | CH₃ | CH₃ | H | 8-F | lone pair | O | 1 | double bond | N | CH |
| 1023 | A | CH₃ | CH₃ | H | CH₃ | H | 8-Cl | lone pair | S | 1 | double bond | N | CH |
| 1024 | A | CH₃ | CH₃ | Cl | CH₃ | H | 8-Cl | lone pair | S | 1 | double bond | N | CH |
| 1025 | A | CH₃ | CH₃ | Br | CH₃ | H | 8-Cl | lone pair | S | 1 | double bond | N | CH |
| 1026 | A | CH₃ | CH₃ | CH₃ | CH₃ | H | 8-Cl | lone pair | S | 1 | double bond | N | CH |
| 1027 | C | CH₃ | CH₃ | CH₃ | H | H | 8-Cl | lone pair | S | 1 | double bond | N | CH |
| 1028 | C | C(O)OCH₂CH₃ | CH₃ | CH₃ | CH₃ | H | 8-F | lone pair | O | 1 | double bond | N | CH |
| 1029 | C | C(O)OCH₂CH₃ | CH₃ | CH₃ | CH₃ | H | 8-F | lone pair | S | 1 | double bond | N | CH |
| 1030 | A | CH₂CH₂OCH₂CH₃ | CH₃ | Cl | CH₃ | H | 8-F | lone pair | O | 1 | double bond | N | CH |
| 1031 | A | C(O)OCH₂CH₂CH₃ | CH₃ | CH₃ | CH₃ | H | - | lone pair | O | 0 | double bond | N | CH |
| 1032 | A | CH₂CH₂CH₂CH₃ | CH₃ | CH₃ | CH₃ | H | 8-OMe | lone pair | O | 1 | double bond | N | CH |
| 1033 | B | 4-nBu-Ph | CH₃ | CH₃ | CH₃ | H | - | lone pair | O | 0 | double bond | N | CH |
| 1034 | B | 4-nBu-Ph | CH₃ | CH₃ | H | H | - | lone pair | O | 0 | double bond | N | CCH₃ |
| 1035 | B | CH₂-4-Me-Ph | CH₃ | CH₃ | CH₃ | H | - | lone pair | O | 0 | double bond | N | CH |
| 1036 | B | CH₂-4-Me-Ph | CH₃ | CH₃ | H | H | - | lone pair | O | 0 | double bond | N | CCH₃ |
| 1037 | B | 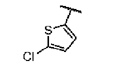 | CH₃ | CH₃ | CH₃ | H | - | lone pair | O | 0 | double bond | N | CH |
| 1038 | B | 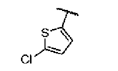 | CH₃ | CH₃ | H | H | - | lone pair | O | 0 | double bond | N | CCH₃ |
| 1039 | B | CH₂CH₂Ph | CH₃ | CH₃ | H | H | 8-F | lone pair | O | 1 | double bond | N | CCH₃ |
| 1040 | B | CH₂CH₂Ph | CH₃ | CH₃ | CH₃ | H | 8-F | lone pair | O | 1 | double bond | N | CH |
| 1041 | B | 4-F-Ph | CH₃ | CH₃ | CH₃ | H | - | lone pair | O | 0 | double bond | N | CH |
| 1042 | B | 4-F-Ph | CH₃ | CH₃ | H | H | - | lone pair | O | 0 | double bond | N | CCH₃ |
| 1043 | B | 4-Me-Ph | CH₃ | CH₃ | CH₃ | H | - | lone pair | O | 0 | double bond | N | CH |
| 1044 | B | 4-Me-Ph | CH₃ | CH₃ | H | H | - | lone pair | O | 0 | double bond | N | CCH₃ |

**[Table 7-31]**

| | | | | | | | | | | | | | |
|---|---|---|---|---|---|---|---|---|---|---|---|---|---|
| 1045 | B | 4-MeO-Ph | CH₃ | CH₃ | CH₃ | H | - | lone pair | O | 0 | double bond | N | CH |
| 1046 | B | 4-MeO-Ph | CH₃ | CH₃ | H | H | - | lone pair | O | 0 | double bond | N | CCH₃ |
| 1047 | A | CH₂C(O)OCH₂CH₃ | CH₃ | Cl | CH₃ | H | 8-CH₃ | lone pair | O | 1 | double bond | N | CH |
| 1048 | A | CH₂C(O)OCH₂CH₃ | CH₃ | Br | CH₃ | H | 8-CH₃ | lone pair | O | 1 | double bond | N | CH |
| 1049 | A | CH₂C(O)OCH₂CH₃ | CH₃ | CH₃ | CH₃ | H | 8-CH₃ | lone pair | O | 1 | double bond | N | CH |
| 1050 | C | CH₂C(O)OCH₂CH₂CH₃ | CH₃ | CH₃ | CH₃ | H | 8-CH₃ | lone pair | O | 1 | double bond | N | CH |
| 1051 | C | CH₂C(O)OCH₂CH₂CH₃ | CH₃ | H | CH₃ | H | 8-CH₃ | lone pair | O | 1 | double bond | N | CH |
| 1052 | C | CH₂C(O)OCH₂CH₃ | CH₃ | CH₃ | CH₃ | H | 8-CH₃ | lone pair | O | 1 | double bond | N | CH |
| 1053 | B | CH₂CH₂CH=CH₂ | CH₃ | CH₃ | CH₃ | H | 8-Cl | lone pair | O | 1 | double bond | N | CH |
| 1054 | B | CH₂CH₂Ph | CH₃ | CH₃ | CH₃ | H | 8-Cl | lone pair | O | 1 | double bond | N | CH |
| 1055 | B | CH₂CH₂Ph | CH₃ | CH₃ | H | H | 8-Cl | lone pair | O | 1 | double bond | N | CCH₃ |
| 1056 | A | CH₂CH₂OCH₂CH₃ | CH₃ | Br | CH₃ | H | 8-F | lone pair | O | 1 | double bond | N | CH |
| 1057 | A | CH₂C(O)OCH₂Ph | CH₃ | Br | CH₃ | H | 8-F | lone pair | O | 1 | double bond | N | CH |
| 1058 | A | CH₂SPh | CH₃ | Br | CH₃ | H | 8-F | lone pair | O | 1 | double bond | N | CH |
| 1059 | A | CH₂SPh | CH₃ | Cl | CH₃ | H | 8-F | lone pair | O | 1 | double bond | N | CH |
| 1060 | A | CH₂CH(CH₃)CH₃ | CH₃ | Br | CH₃ | H | 8-F | lone pair | O | 1 | double bond | N | CH |
| 1061 | A | CH₂C(O)OCH₂CH₂CH₃ | CH₃ | Br | CH₃ | H | 8-F | lone pair | O | 1 | double bond | N | CH |
| 1062 | A | CH₂C(O) OCH₂CH₂CH₂CH₃ | CH₃ | Br | CH₃ | H | 8-F | lone pair | O | 1 | double bond | N | CH |
| 1063 | A | CH₂CH₂OCH₂CH₃ | CH₃ | CH₃ | CH₃ | H | 8-F | lone pair | O | 1 | double bond | N | CH |
| 1064 | A | CH₂C(O)OCH₂Ph | CH₃ | CH₃ | CH₃ | H | 8-F | lone pair | O | 1 | double bond | N | CH |
| 1065 | A | CH₂SPh | CH₃ | CH₃ | CH₃ | H | 8-F | lone pair | O | 1 | double bond | N | CH |
| 1066 | A | CH₂CH(CH₃)CH₃ | CH₃ | CH₃ | CH₃ | H | 8-F | lone pair | O | 1 | double bond | N | CH |
| 1067 | A | CH₂C(O)OCH₂CH₂CH₃ | CH₃ | CH₃ | CH₃ | H | 8-F | lone pair | O | 1 | double bond | N | CH |
| 1068 | A | CH₂C(O)OCH₂CH₂CH₂CH₃ | CH₃ | CH₃ | CH₃ | H | 8-F | lone pair | O | 1 | double bond | N | CH |
| 1069 | C | 3-CH₃O-Ph | CH₃ | CH₃ | CH₃ | H | 8-F | lone pair | O | 1 | double bond | N | CH |
| 1070 | B | 3-CH₃O-Ph | CH₃ | CH₃ | CH₃ | H | 8-F | lone pair | O | 1 | double bond | N | CH |
| 1071 | C | Ph | CH₃ | CH₃ | CH₃ | H | 8-Cl | lone pair | O | 1 | double bond | N | CH |
| 1072 | B | Ph | CH₃ | CH₃ | CH₃ | H | 8-Cl | lone pair | O | 1 | double bond | N | CH |
| 1073 | A | Ph | CH₃ | Br | CH₃ | H | 8-Cl | lone pair | O | 1 | double bond | N | CH |
| 1074 | C | 4-F-Ph | CH₃ | CH₃ | CH₃ | H | 8-Cl | lone pair | O | 1 | double bond | N | CH |
| 1075 | C | 4-Cl-Ph | CH₃ | CH₃ | CH₃ | H | 8-Cl | lone pair | O | 1 | double bond | N | CH |
| 1076 | C | Ph | CH₃ | CH₃ | CH₃ | H | 8-F | lone pair | O | 1 | double bond | N | CH |
| 1077 | B | Ph | CH₃ | CH₃ | CH₃ | H | 8-F | lone pair | O | 1 | double bond | N | CH |
| 1078 | C | 4-F-Ph | CH₃ | CH₃ | CH₃ | H | 8-F | lone pair | O | 1 | double bond | N | CH |
| 1079 | B | 4-F-Ph | CH₃ | CH₃ | CH₃ | H | 8-F | lone pair | O | 1 | double bond | N | CH |

**[Table 7-32]**

| | | | | | | | | | | | | | |
|---|---|---|---|---|---|---|---|---|---|---|---|---|---|
| 1080 | C | 4-Cl-Ph | CH₃ | CH₃ | CH₃ | H | 8-F | lone pair | O | 1 | double bond | N | CH |
| 1081 | B | 4-Cl-Ph | CH₃ | CH₃ | CH₃ | H | 8-F | lone pair | O | 1 | double bond | N | CH |
| 1082 | C | 4-CH₃-Ph | CH₃ | CH₃ | CH₃ | H | 8-F | lone pair | O | 1 | double bond | N | CH |
| 1083 | B | 4-CH₃-Ph | CH₃ | CH₃ | CH₃ | H | 8-F | lone pair | O | 1 | double bond | N | CH |
| 1084 | B | CH₂CH₂CH₂CH₃ | CH₃ | CH₃ | CH₃ | H | 8-Cl | lone pair | S | 1 | double bond | N | CH |
| 1085 | B | 2-F-Ph | CH₃ | CH₃ | CH₃ | H | 8-F | lone pair | O | 1 | double bond | N | CH |
| 1086 | C | CH₂CH₂CH₂CH₃ | CH₃ | CH₃ | CH₃ | H | 8-Cl | lone pair | S | 1 | double bond | N | CH |
| 1087 | C | 4-F-Ph | CH₃ | CH₃ | CH₃ | H | - | lone pair | O | 0 | double bond | N | CH |
| 1088 | C | 3-Cl-Ph | CH₃ | CH₃ | CH₃ | H | 8-F | lone pair | O | 1 | double bond | N | CH |
| 1089 | B | 3-Cl-Ph | CH₃ | CH₃ | CH₃ | H | 8-F | lone pair | O | 1 | double bond | N | CH |
| 1090 | C | 3-CH₃-Ph | CH₃ | CH₃ | CH₃ | H | 8-F | lone pair | O | 1 | double bond | N | CH |
| 1091 | B | 3-CH₃-Ph | CH₃ | CH₃ | CH₃ | H | 8-F | lone pair | O | 1 | double bond | N | CH |
| 1092 | C | 3-F-Ph | CH₃ | CH₃ | CH₃ | H | 8-F | lone pair | O | 1 | double bond | N | CH |
| 1093 | B | 3-F-Ph | CH₃ | CH₃ | CH₃ | H | 8-F | lone pair | O | 1 | double bond | N | CH |
| 1094 | B | 2-Cl-Ph | CH₃ | CH₃ | CH₃ | H | 8-F | lone pair | O | 1 | double bond | N | CH |
| 1095 | C | 4-Cl-Ph | CH₃ | CH₃ | CH₃ | H | - | lone pair | O | 0 | double bond | N | CH |
| 1096 | B | 4-Cl-Ph | CH₃ | CH₃ | CH₃ | H | - | lone pair | O | 0 | double bond | N | CH |
| 1097 | C | Ph | CH₃ | CH₃ | CH₃ | H | 8-CH₃ | lone pair | O | 1 | double bond | N | CH |
| 1098 | B | Ph | CH₃ | CH₃ | CH₃ | H | 8-CH₃ | lone pair | O | 1 | double bond | N | CH |
| 1099 | C | 4-F-Ph | CH₃ | CH₃ | CH₃ | H | 8-CH₃ | lone pair | O | 1 | double bond | N | CH |
| 1100 | B | 4-F-Ph | CH₃ | CH₃ | CH₃ | H | 8-CH₃ | lone pair | O | 1 | double bond | N | CH |
| 1101 | C | 3-F-Ph | CH₃ | CH₃ | CH₃ | H | 8-CH₃ | lone pair | O | 1 | double bond | N | CH |
| 1102 | B | 3-F-Ph | CH₃ | CH₃ | CH₃ | H | 8-CH₃ | lone pair | O | 1 | double bond | N | CH |
| 1103 | C | 4-CH₃-Ph | CH₃ | CH₃ | CH₃ | H | 8-CH₃ | lone pair | O | 1 | double bond | N | CH |
| 1104 | B | 4-CH₃-Ph | CH₃ | CH₃ | CH₃ | H | 8-CH₂ | lone pair | O | 1 | double bond | N | CH |
| 1105 | B | 4-Cl-Ph | CH₃ | CH₃ | CH₃ | H | 8-CH₃ | lone pair | O | 1 | double bond | N | CH |
| 1106 | B | 2-F-Ph | CH₃ | CH₃ | CH₃ | H | 8-CH₃ | lone pair | O | 1 | double bond | N | CH |
| 1107 | C | 4-Cl-Ph | CH₃ | H | CH₃ | H | 8-CH₃ | lone pair | O | 1 | double bond | N | CH |
| 1108 | C | 2-F-Ph | CH₃ | H | CH₃ | H | 8-CH₃ | lone pair | O | 1 | double bond | N | CH |
| 1109 | C | 4-CH₃-Ph | CH₃ | CH₃ | CH₃ | H | 8-Cl | lone pair | O | 1 | double bond | N | CH |
| 1110 | B | 4-CH₃-Ph | CH₃ | CH₃ | CH₃ | H | 8-Cl | lone pair | O | 1 | double bond | N | CH |
| 1111 | A | 4-CH₃-Ph | CH₃ | Br | CH₃ | H | 8-Cl | lone pair | O | 1 | double bond | N | CH |
| 1112 | C | 4-CH₃O-Ph | CH₃ | CH₃ | CH₃ | H | 8-Cl | lone pair | O | 1 | double bond | N | CH |
| 1113 | B | 4-CH₃O-Ph | CH₃ | CH₃ | CH₃ | H | 8-Cl | lone pair | O | 1 | double bond | N | CH |
| 1114 | C | 2-F-Ph | CH₃ | CH₃ | CH₃ | H | 8-Cl | lone pair | O | 1 | double bond | N | CH |

**[Table 7-33]**

| | | | | | | | | | | | | | |
|---|---|---|---|---|---|---|---|---|---|---|---|---|---|
| 1115 | B | 2-F-Ph | CH₃ | CH₃ | CH₃ | H | 8-Cl | lone pair | O | 1 | double bond | N | CH |
| 1116 | C | 2-CH₃-Ph | CH₃ | CH₃ | CH₃ | H | 8-F | lone pair | O | 1 | double bond | N | CH |
| 1117 | B | 2-CH₃-Ph | CH₃ | CH₃ | CH₃ | H | 8-F | lone pair | O | 1 | double bond | N | CH |
| 1118 | C | 3-Cl-Ph | CH₃ | CH₃ | CH₃ | H | - | lone pair | O | 0 | double bond | N | CH |
| 1119 | B | 3-Cl-Ph | CH₃ | CH₃ | CH₃ | H | - | lone pair | O | 0 | double bond | N | CH |
| 1120 | C | 2-F-Ph | CH₃ | CH₃ | CH₃ | H | - | lone pair | O | 0 | double bond | N | CH |
| 1121 | B | 2-F-Ph | CH₃ | CH₃ | CH₃ | H | - | lone pair | O | 0 | double bond | N | CH |
| 1122 | C | 2-Cl-Ph | CH₃ | CH₃ | CH₃ | H | - | lone pair | O | 0 | double bond | N | CH |
| 1123 | B | 2-Cl-Ph | CH₃ | CH₃ | CH₃ | H | - | lone pair | O | 0 | double bond | N | CH |
| 1124 | C | 2-CH₃-Ph | CH₃ | CH₃ | CH₃ | H | - | lone pair | O | 0 | double bond | N | CH |
| 1125 | B | 2-CH₃-Ph | CH₃ | CH₃ | CH₃ | H | - | lone pair | O | 0 | double bond | N | CH |
| 1126 | C | 2-Cl-Ph | CH₃ | CH₃ | CH₃ | H | 8-CH₃ | lone pair | O | 1 | double bond | N | CH |
| 1127 | B | 2-Cl-Ph | CH₃ | CH₃ | CH₃ | H | 8-CH₃ | lone pair | O | 1 | double bond | N | CH |
| 1128 | C | 3-CH₃-Ph | CH₃ | CH₃ | CH₃ | H | 8-CH₃ | lone pair | O | 1 | double bond | N | CH |
| 1129 | B | 3-CH₃-Ph | CH₃ | CH₃ | CH₃ | H | 8-CH₃ | lone pair | O | 1 | double bond | N | CH |
| 1130 | C | 3-Cl-Ph | CH₃ | CH₃ | CH₃ | H | 8-CH₃ | lone pair | O | 1 | double bond | N | CH |
| 1131 | B | 3-Cl-Ph | CH₃ | CH₃ | CH₃ | H | 8-CH₃ | lone pair | O | 1 | double bond | N | CH |
| 1132 | C | 2-F-Ph | CH₃ | CH₃ | CH₃ | H | 8-CH₃ | lone pair | O | 1 | double bond | N | CH |
| 1133 | C | 4-CH₃-Ph | CH₃ | CH₃ | CH₃ | H | - | lone pair | O | 0 | double bond | N | CH |
| 1134 | C | Ph | CH₃ | CH₃ | CH₃ | H | - | lone pair | O | 0 | double bond | N | CH |
| 1135 | C | 3-F-Ph | CH₃ | CH₃ | CH₃ | H | - | lone pair | O | 0 | double bond | N | CH |
| 1136 | B | 3-F-Ph | CH₃ | CH₃ | CH₃ | H | - | lone pair | O | 0 | double bond | N | CH |
| 1137 | C | 3-CH₃-Ph | CH₃ | CH₃ | CH₃ | H | - | lone pair | O | 0 | double bond | N | CH |
| 1138 | B | 3-CH₃-Ph | CH₃ | CH₃ | CH₃ | H | - | lone pair | O | 0 | double bond | N | CH |
| 1139 | A | Ph | CH₃ | CH₃ | CH₃ | H | 8-Cl | lone pair | O | 1 | double bond | N | CH |
| 1140 | B | 4-Cl-Ph | CH₃ | CH₃ | CH₃ | H | 8-Cl | lone pair | O | 1 | double bond | N | CH |
| 1141 | A | 4-CH₃-Ph | CH₃ | CH₃ | CH₃ | H | 8-Cl | lone pair | O | 1 | double bond | N | CH |
| 1142 | C | 3-F-Ph | CH₃ | CH₃ | CH₃ | H | 8-Cl | lone pair | O | 1 | double bond | N | CH |
| 1143 | B | 3-F-Ph | CH₃ | CH₃ | CH₃ | H | 8-Cl | lone pair | O | 1 | double bond | N | CH |
| 1144 | A | 3-F-Ph | CH₃ | Br | CH₃ | H | 8-Cl | lone pair | O | 1 | double bond | N | CH |
| 1145 | C | 3-F-Ph | CH₃ | H | CH₃ | H | 8-Cl | lone pair | O | 1 | double bond | N | CH |
| 1146 | A | Ph | CH₃ | Cl | CH₃ | H | 8-F | lone pair | O | 1 | double bond | N | CH |
| 1147 | A | Ph | CH₃ | Br | CH₃ | H | 8-F | lone pair | O | 1 | double bond | N | CH |
| 1148 | A | Ph | CH₃ | CH₃ | CH₃ | H | 8-F | lone pair | O | 1 | double bond | N | CH |
| 1149 | A | 2-F-Ph | CH₃ | Cl | CH₃ | H | 8-Cl | lone pair | O | 1 | double bond | N | CH |

**[Table 7-34]**

| | | | | | | | | | | | | | |
|---|---|---|---|---|---|---|---|---|---|---|---|---|---|
| 1150 | A | 2-F-Ph | CH₃ | Br | CH₃ | H | 8-Cl | lone pair | O | 1 | double bond | N | CH |
| 1151 | A | 2-F-Ph | CH₃ | CH₃ | CH₃ | H | 8-Cl | lone pair | O | 1 | double bond | N | CH |
| 1152 | A | 2-F-Ph | CH₃ | Cl | CH₃ | H | - | lone pair | O | 0 | double bond | N | CH |
| 1153 | A | 2-F-Ph | CH₃ | CH₃ | CH₃ | H | - | lone pair | O | 0 | double bond | N | CH |
| 1154 | A | 3-F-Ph | CH₃ | Cl | CH₃ | H | - | lone pair | O | 0 | double bond | N | CH |
| 1155 | A | 3-F-Ph | CH₃ | Br | CH₃ | H | - | lone pair | O | 0 | double bond | N | CH |

Next, ¹H-NMR data on the compounds described in the table above is shown below.
1 ¹H-NMR (CDCl₃) δ: 8.26 (1H, s), 7.87-7.85 (1H, m), 7.65-7.63 (1H, m), 7.51-7.48 (1H, m), 7.39-7.31 (2H, m), 7.24 (1H, dd, J = 7.6, 1.5 Hz), 7.03-6.98 (2H, m), 1.72 (6H, s).
2 ¹H-NMR (CDCl₃) δ: 8.14 (1H, s), 7.60 (1H, s), 7.48 (1H, ddd, J = 10.0, 5.6, 2.0 Hz), 7.37 (1H, br s), 7.23 (1H, dd, J = 7.6, 1.8 Hz), 7.02-6.96 (2H, m), 2.39 (3H, s), 2.35 (3H, s), 1.72 (6H, s).
3 ¹H-NMR (CDCl₃) δ: 8.15 (1H, s), 7.60 (1H, s), 7.49-7.45 (2H, m), 7.24 (1H, dd, J = 7.6, 1.5 Hz), 7.05-6.96 (2H, m), 2.39 (3H, s), 2.36 (3H, s), 2.06-1.94 (4H, m), 1.78-1.73 (4H, m), 1.66-1.52 (2H, m).
4 ¹H-NMR (CDCl₃) δ: 8.25 (1H, s), 7.87-7.85 (1H, m), 7.65-7.63 (1H, m), 7.37-7.31 (3H, m), 7.07-7.05 (1H, m), 6.89 (1H, t, J = 7.6 Hz), 2.28 (3H, s), 1.72 (6H, s).
5 ¹H-NMR (CDCl₃) δ: 8.27 (1H, s), 7.87 (1H, dd, J = 7.1, 2.0 Hz), 7.66 (1H, dd, J = 7.1, 2.0 Hz), 7.46 (1H, ddd, J = 8.6, 7.1, 1.1 Hz), 7.37-7.31 (2H, m), 7.21 (1H, dd, J = 8.2, 1.0 Hz), 6.99 (1H, dd, J = 8.2, 1.0 Hz), 6.95 (1H, td, J = 8.2, 1.0 Hz), 1.99 (4H, q, J = 7.4 Hz), 1.04 (6H, t, J = 7.4 Hz).
6 ¹H-NMR (CDCl₃) δ: 8.22 (1H, s), 7.49-7.46 (1H, m), 7.44 (1H, d, J = 8.4 Hz), 7.23-7.20 (2H, m), 7.15 (1H, d, J = 7.3 Hz), 7.01 (1H, dd, J = 7.3, 1.2 Hz), 6.98 (1H, td, J = 7.3, 1.2 Hz), 2.72 (3H, s), 1.72 (6H, s).
7 ¹H-NMR (CDCl₃) δ: 8.22 (1H, s), 7.84 (1H, dd, J = 7.3, 2.0 Hz), 7.83-7.80 (1H, m), 7.60 (1H, ddd, J = 7.6, 7.3, 2.0 Hz), 7.23 (1H, d, J = 8.0 Hz), 7.11 (1H, ddd, J = 7.6, 7.3, 1.0 Hz), 7.02 (1H, d, J = 8.0 Hz), 2.60 (3H, s), 2.41 (3H, s), 2.00 (6H, s).
8 ¹H-NMR (CDCl₃) δ: 8.18 (1H, s), 7.32-7.28 (2H, m), 7.12 (1H, d, J = 8.3 Hz), 7.04 (1H, dt, J = 7.8, 1.2 Hz), 6.92 (1H, td, J = 8.1, 4.6 Hz), 2.63 (3H, s), 2.41 (3H, s), 1.76 (6H, s).
9 ¹H-NMR (CDCl₃) δ: 8.18 (1H, s), 7.33-7.32 (2H, m), 7.11 (1H, d, J = 7.4 Hz), 7.05 (1H, d, J = 7.4 Hz), 6.86 (1H, t, J = 7.4 Hz), 2.63 (3H, s), 2.41 (3H, s), 2.27 (3H, s), 1.70 (6H, s).
10 ¹H-NMR (CDCl₃) δ: 8.21 (1H, s), 7.80 (1H, d, J = 8.1 Hz), 7.71 (1H, d, J = 8.1 Hz), 7.22 (1H, d, J = 8.3 Hz), 6.92-6.90 (1H, m), 6.82 (1H, s), 2.60 (3H, s), 2.40 (3H, s), 2.41 (3H, s), 1.99 (6H, s).
11 ¹H-NMR (CDCl₃) δ: 8.30 (1H, s), 8.15-8.13 (1H, m), 7.86-7.82 (1H, m), 7.71 (1H, d, J = 8.0 Hz), 7.47-7.42 (2H, m), 6.92 (1H, d, J = 8.0 Hz), 6.83 (1H, s), 2.40 (3H, s), 2.00 (6H, s).
12 ¹H-NMR (CDCl₃) δ: 8.22 (1H, s), 7.39 (1H, d, J = 8.0 Hz), 7.30 (1H, d, J = 8.0 Hz), 7.06 (1H, d, J = 8.0 Hz), 6.83 (1H, s), 6.80-6.78 (1H, m), 2.73 (3H, s), 2.39 (3H, s), 1.70 (6H, s).
13 ¹H-NMR (CDCl₃) δ: 8.21 (1H, s), 7.37 (1H, d, J = 9.2 Hz), 7.29 (1H, d, J = 8.8 Hz), 7.09 (1H, d, J = 8.4 Hz), 6.53-6.50 (2H, m), 3.86 (3H, s), 2.73 (3H, s), 1.70 (6H, s).
14 ¹H-NMR (CDCl₃) δ: 8.17 (1H, s), 7.29 (1H, d, J = 8.6 Hz), 7.13 (1H, d, J = 8.4 Hz), 7.10 (1H, d, J = 8.4 Hz), 6.51 (1H, d, J = 2.3 Hz), 6.49 (1H, dd, J = 8.6, 2.5 Hz), 3.86 (3H, s), 2.62 (3H, s), 2.41 (3H, s), 1.70 (6H, s).
15 ¹H-NMR (CDCl₃) δ: 8.22 (1H, s), 7.39 (1H, d, J = 8.4 Hz), 7.32 (1H, d, J = 8.4 Hz), 7.31-7.29 (1H, m), 7.02 (1H, dt, J = 8.0, 1.2 Hz), 6.94 (1H, td, J = 8.0, 4.6 Hz), 2.73 (3H, s), 1.76 (6H, s).
16 ¹H-NMR (CDCl₃) δ: 8.15 (1H, s), 7.32-7.23 (2H, m), 7.19 (1H, dd, J = 8.3, 1.0 Hz), 7.03 (1H, dt, J = 7.8, 1.2 Hz), 6.93 (1H, td, J = 8.1, 4.6 Hz), 6.79 (1H, dd, J = 7.8, 1.0 Hz), 4.06 (3H, s), 1.77 (6H, s).
17 ¹H-NMR (CDCl₃) δ: 8.15 (1H, s), 7.27-7.24 (1H, m), 7.19 (1H, d, J = 1.9 Hz), 7.13-7.05 (3H, m), 2.62 (3H, s), 2.41 (3H, s), 1.70 (6H, s).
18 ¹H-NMR (CDCl₃) δ: 8.20 (1H, s), 7.33 (2H, q, J = 8.8 Hz), 7.21 (1H, d, J = 1.9 Hz), 7.13 (1H, dd, J = 8.4, 1.9 Hz), 7.05 (1H, d, J = 8.4 Hz), 2.73 (3H, s), 1.71 (6H, s).
19 ¹H-NMR (CDCl₃) δ: 8.23 (1H, s), 7.35 (1H, ddd, J = 9.0, 4.0, 1.5 Hz), 7.17 (1H, ddd, J = 11.0, 9.0, 7.0 Hz), 7.10 (1H, d, J = 9.0 Hz), 6.55-6.52 (2H, m), 3.87 (3H, s), 1.71 (6H, s).
20 ¹H-NMR (CDCl₃) δ: 8.22 (1H, s), 7.40 (1H, d, J = 8.4 Hz), 7.36-7.34 (1H, m), 7.30 (1H, d, J = 8.8 Hz), 7.03-7.01 (1H, m), 6.88 (1H, dd, J = 7.6, 7.6 Hz), 2.73 (3H, s), 2.28 (3H, s), 1.71 (6H, s).
21 ¹H-NMR (CDCl₃) δ: 8.25 (1H, s), 7.39-7.33 (2H, m), 7.17 (1H, ddd, J = 13.6, 6.3, 4.4 Hz), 7.02 (1H, dd, J = 7.8, 1.0 Hz), 6.91 (1H, t, J = 7.6 Hz), 2.28 (3H, s), 1.72 (6H, s).
22 ¹H-NMR (CDCl₃) δ: 8.23 (1H, s), 7.42 (1H, dd, J = 9.0, 5.2 Hz), 7.36-7.34 (1H, m), 7.07-7.02 (2H, m), 6.89 (1H, t, J = 7.6 Hz), 2.61 (3H, d, J = 1.9 Hz), 2.28 (3H, s), 1.71 (6H, s).
23 ¹H-NMR (CDCl₃) δ: 8.17 (1H, s), 7.54 (1H, dd, J = 8.0, 1.1 Hz), 7.28 (1H, d, J = 8.4 Hz), 7.16-7.11 (2H, m), 6.92 (1H, t, J = 8.0 Hz), 2.63 (3H, s), 2.41 (3H, s), 1.76 (6H, s).
24 ¹H-NMR (CDCl₃) δ: 8.21 (1H, s), 7.55 (1H, dd, J = 8.0, 1.5 Hz), 7.36 (1H, d, J = 8.4 Hz), 7.31 (1H, d, J = 8.4 Hz), 7.13-7.11 (1H, m), 6.94 (1H, t, J = 8.4 Hz), 2.73 (3H, s), 1.76 (6H, s).
25 ¹H-NMR (CDCl₃) δ: 8.21 (1H, s), 7.38-7.35 (1H, m), 7.21 (1H, d, J = 1.9 Hz), 7.14 (1H, dd, J = 8.4, 1.9 Hz), 7.09-7.03 (2H, m), 2.61 (3H, d, J = 1.5 Hz), 1.71 (6H, s).
26 ¹H-NMR (CDCl₃) δ: 8.13 (1H, s), 7.24 (1H, t, J = 8.4 Hz), 7.20 (1H, d, J = 1.9 Hz), 7.15-7.11 (2H, m), 7.06 (1H, d, J = 8.4 Hz), 6.78 (1H, d, J = 7.3 Hz), 4.06 (3H, s), 1.71 (6H, s).
27 ¹H-NMR (CDCl₃) δ: 8.22 (1H, s), 7.56 (1H, dd, J = 7.8, 1.3 Hz), 7.40-7.37 (1H, m), 7.14 (1H, dd, J = 7.8, 1.3 Hz), 7.06 (1H, t, J = 9.0 Hz), 6.95 (1H, t, J = 8.4 Hz), 2.61 (3H, d, J = 1.5 Hz), 1.76 (6H, s).
28 ¹H-NMR (CDCl₃) δ: 8.14 (1H, s), 7.54 (1H, dd, J = 8.0, 1.5 Hz), 7.25 (1H, t, J = 7.3 Hz), 7.16 (1H, dd, J = 8.0, 0.8 Hz), 7.13 (1H, dd, J = 7.6, 1.5 Hz), 6.93 (1H, t, J = 8.0 Hz), 6.79 (1H, d, J = 8.0 Hz), 4.06 (3H, s), 1.77 (6H, s).
29 ¹H-NMR (CDCl₃) δ: 8.19 (1H, s), 7.37 (1H, d, J = 8.4 Hz), 7.29-7.26 (1H, m), 7.12 (1H, d, J = 8.4 Hz), 7.03 (1H, d, J = 1.5 Hz), 6.90 (1H, d, J = 8.4 Hz), 2.63 (3H, s), 2.42 (3H, s), 2.24 (3H, s), 1.69 (6H, s).
30 ¹H-NMR (CDCl₃) δ: 8.23 (1H, s), 7.43 (1H, d, J = 8.4 Hz), 7.33-7.28 (2H, m), 6.99 (1H, d, J = 1.5 Hz), 6.91 (1H, d, J = 8.4 Hz), 2.74 (3H, s), 2.25 (3H, s), 1.69 (6H, s).
31 ¹H-NMR (CDCl₃) δ: 8.05 (1H, d, J = 5.1 Hz), 7.43 (1H, td, J = 8.3, 6.0 Hz), 7.33 (1H, d, J = 8.3 Hz), 7.09 (1H, d, J = 8.3 Hz), 6.83 (1H, d, J = 8.3 Hz), 6.70 (1H, ddd, J = 10.2, 8.3, 0.9 Hz), 2.59 (3H, s), 2.38 (3H, s), 1.67 (6H, s).
32 ¹H-NMR (CDCl₃) δ: 8.10 (1H, d, J = 5.7 Hz), 7.47-7.40 (2H, m), 7.02 (1H, dd, J = 11.3, 7.8 Hz), 6.84 (1H, d, J = 8.4 Hz), 6.72 (1H, ddd, J = 10.1, 8.4, 1.0 Hz), 2.58 (3H, d, J = 1.5 Hz), 1.68 (6H, s).
33 ¹H-NMR (CDCl₃) δ: 8.06 (1H, d, J = 5.7 Hz), 7.44-7.39 (1H, m), 7.31 (1H, ddd, J = 8.8, 3.8, 1.5 Hz), 7.13-7.06 (1H, m), 6.80 (1H, d, J = 8.0 Hz), 6.69 (1H, ddd, J = 10.3, 8.4, 1.1 Hz), 1.64 (6H, s).
34 ¹H-NMR (CDCl₃) δ: 8.18 (1H, s), 7.33 (1H, d, J = 8.4 Hz), 7.11 (1H, d, J = 8.4 Hz), 7.08 (1H, dd, J = 8.0, 1.5 Hz), 6.92 (1H, t, J = 8.0 Hz), 6.84 (1H, dd, J = 7.8, 1.3 Hz), 3.94 (3H, s), 2.63 (3H, s), 2.41 (3H, s), 1.76 (6H, s).
35 ¹H-NMR (CDCl₃) δ: 8.32 (1H, s), 8.24 (1H, d, J = 4.9 Hz), 7.30-7.28 (1H, m), 7.10 (1H, dd, J = 4.9, 0.7 Hz), 6.87 (1H, dd, J = 7.8, 1.2 Hz), 6.78 (1H, t, J = 7.6 Hz), 2.72 (3H, d, J = 0.5 Hz), 2.24 (3H, s), 1.72 (6H, s).
36 ¹H-NMR (CDCl₃) δ: 8.35 (1H, s), 7.87 (1H, d, J = 8.0 Hz), 7.64 (1H, d, J = 7.6 Hz), 7.40 (1H, t, J = 8.0 Hz), 7.37 (1H, d, J = 7.3 Hz), 7.03 (1H, dd, J = 7.4, 1.3 Hz), 6.90 (1H, t, J = 7.6 Hz), 2.29 (3H, s), 1.73 (6H, s).
37 ¹H-NMR (CDCl₃) δ: 8.09 (1H, d, J = 5.3 Hz), 7.45 (1H, td, J = 8.3, 6.0 Hz), 7.39 (1H, d, J = 8.8 Hz), 7.28 (1H, d, J = 8.8 Hz), 6.84 (1H, d, J = 8.0 Hz), 6.74-6.70 (1H, m), 2.70 (3H, s), 1.68 (6H, s).
38 ¹H-NMR (CDCl₃) δ: 8.34 (1H, s), 8.21 (1H, d, J = 5.0 Hz), 7.40 (1H, td, J = 8.4, 6.1 Hz), 7.10 (1H, d, J = 4.6 Hz), 6.80 (1H, d, J = 8.4 Hz), 6.62-6.58 (1H, m), 2.71 (3H, s), 1.69 (6H, s).
39 ¹H-NMR (CDCl₃) δ: 8.16 (1H, s), 7.26 (1H, d, J = 8.0 Hz), 7.22 (1H, dd, J = 8.8, 6.5 Hz), 7.12 (1H, d, J = 8.0 Hz), 6.72 (1H, dd, J = 9.5, 2.3 Hz), 6.67 (1H, td, J = 8.6, 2.5 Hz), 2.63 (3H, s), 2.41 (3H, s), 1.71 (6H, s).
40 ¹H-NMR (CDCl₃) δ: 8.19 (1H, s), 7.48 (1H, d, J = 8.4 Hz), 7.28 (1H, d, J = 9.2 Hz), 7.19 (1H, dd, J = 8.4, 5.7 Hz), 6.75-6.67 (2H, m), 2.76 (3H, s), 1.72 (6H, s).
41 ¹H-NMR (CDCl₃) δ: 8.20 (1H, s), 7.36-7.30 (2H, m), 7.20 (1H, dd, J = 8.6, 5.9 Hz), 6.75-6.68 (2H, m), 2.73 (3H, s), 1.72 (6H, s).
42 ¹H-NMR (CDCl₃) δ: 8.23 (1H, s, ), 7.40 (1H, dd, J = 8.8, 0.8 Hz, ), 7.31 (1H, d, J = 8.4 Hz, ), 7.10 (1H, dd, J = 8.2, 1.3 Hz, ), 6.94 (1H, t, J = 8.0 Hz, ), 6.81 (1H, dd, J = 8.2, 1.3 Hz, ), 3.95 (3H, s, ), 2.73 (3H, s, ), 1.76 (6H, s, ).
43 ¹H-NMR (CDCl₃) δ: 8.36 (1H, s), 7.88 (1H, d, J = 8.4 Hz), 7.65 (1H, d, J = 7.6 Hz), 7.41 (1H, t, J = 7.8 Hz), 7.12 (1H, dd, J = 8.2, 1.3 Hz), 6.96 (1H, t, J = 8.2 Hz), 6.82 (1H, dd, J = 8.0, 1.5 Hz), 3.95 (3H, s), 1.78 (6H, s).
44 ¹H-NMR (CDCl₃) δ: 8.20 (1H, s), 7.46 (1H, d, J = 8.8 Hz), 7.36-7.33 (2H, m), 7.02-7.00 (1H, m), 6.88 (1H, t, J = 7.6 Hz), 2.76 (3H, s), 2.28 (3H, s), 1.71 (6H, s).
45 ¹H-NMR (CDCl₃) δ: 8.15 (1H, s), 7.33 (1H, d, J = 8.3 Hz), 7.26-7.20 (2H, m), 7.03 (1H, dd, J = 7.8, 1.5 Hz), 6.87 (1H, t, J = 7.7 Hz), 6.77 (1H, dd, J = 7.1, 1.7 Hz), 4.06 (3H, s), 2.27 (3H, s), 1.71 (6H, s).
46 ¹H-NMR (CDCl₃) δ: 8.28 (1H, s), 7.56 (1H, dd, J = 8.2, 1.0 Hz), 7.38-7.34 (2H, m), 7.25 (1H, t, J = 8.0 Hz), 7.01 (1H, dd, J = 7.4, 1.3 Hz), 6.89 (1H, t, J = 7.6 Hz), 2.28 (3H, s), 1.72 (6H, s).
47 ¹H-NMR (CDCl₃) δ: 8.22 (1H, s), 7.42 (1H, dd, J = 8.4, 0.8 Hz), 7.35 (1H, d, J = 7.6 Hz), 7.27-7.24 (1H, m), 7.07-7.02 (2H, m), 6.89 (1H, t, J = 7.6 Hz), 2.28 (3H, s), 1.73 (6H, s).
48 ¹H-NMR (CDCl₃) δ: 8.28 (1H, s), 7.56 (1H, dd, J = 8.2, 1.0 Hz), 7.38 (1H, dd, J = 8.0, 1.0 Hz), 7.26 (1H, t, J = 8.2 Hz), 7.11 (1H, dd, J = 8.2, 1.3 Hz), 6.96 (1H, t, J = 8.0 Hz), 6.80 (1H, dd, J = 7.8, 1.3 Hz), 3.94 (3H, s), 1.77 (6H, s).
49 ¹H-NMR (CDCl₃) δ: 8.15 (1H, s), 7.25-7.22 (2H, m), 7.09 (1H, dd, J = 8.2, 1.3 Hz), 6.93 (1H, t, J = 8.1 Hz), 6.82 (1H, dd, J = 8.1, 1.5 Hz), 6.77 (1H, dd, J = 8.1, 1.3 Hz), 4.06 (3H, s), 3.94 (3H, s), 1.76 (6H, s).
50 ¹H-NMR (CDCl₃) δ: 8.22 (1H, s), 7.43 (1H, dd, J = 8.3, 0.7 Hz), 7.28-7.22 (1H, m), 7.11 (1H, dd, J = 8.2, 1.3 Hz), 7.05 (1H, ddd, J = 10.2, 8.1, 0.9 Hz), 6.96 (1H, t, J = 8.1 Hz), 6.81 (1H, dd, J = 7.9, 1.3 Hz), 3.95 (3H, s), 1.77 (6H, s).
51 ¹H-NMR (CDCl₃) δ: 8.11 (1H, s), 7.46-7.43 (2H, m), 7.28-7.25 (1H, m), 7.18-7.14 (2H, m), 7.09-7.06 (1H, m), 2.62 (3H, s), 2.40 (3H, s), 1.71 (6H, s).
52 ¹H-NMR (CDCl₃) δ: 8.13 (1H, s), 7.47-7.45 (2H, m), 7.43 (1H, d, J = 9.0 Hz), 7.24-7.21 (1H, m), 7.20-7.15 (2H, m), 2.75 (3H, s), 1.71 (6H, s).
53 ¹H-NMR (CDCl₃) δ: 8.22 (1H, s), 7.45 (1H, d, J = 8.0 Hz), 7.34-7.33 (1H, m), 7.21 (1H, t, J = 8.0 Hz), 7.15-7.13 (1H, m), 7.04 (1H, dd, J = 7.4, 1.3 Hz), 6.87 (1H, t, J = 7.4 Hz), 2.71 (3H, s), 2.28 (3H, s), 1.72 (6H, s).
54 ¹H-NMR (CDCl₃) δ: 8.22 (1H, s), 7.45 (1H, d, J = 8.1 Hz), 7.22 (1H, t, J = 7.7 Hz), 7.15 (1H, d, J = 7.3 Hz), 7.09 (1H, dd, J = 8.2, 1.3 Hz), 6.93 (1H, t, J = 8.1 Hz), 6.83 (1H, dd, J = 7.9, 1.3 Hz), 3.94 (3H, s), 2.71 (3H, s), 1.77 (6H, d, J = 4.4 Hz).
55 ¹H-NMR (CDCl₃) δ: 7.28-7.26 (1H, m), 6.95 (1H, d, J = 8.3 Hz), 6.77 (1H, d, J = 7.6 Hz), 6.72 (1H, t, J = 7.6 Hz), 6.63 (1H, dd, J = 8.3, 1.3 Hz), 2.60 (3H, s), 2.37 (3H, s), 2.26 (3H, s), 1.98 (3H, s), 1.79 (3H, s), 1.69 (3H, s).
56 ¹H-NMR (CDCl₃) δ: 8.18 (1H, s), 7.32 (1H, d, J = 8.4 Hz), 7.11 (1H, d, J = 8.2 Hz), 7.10 (1H, d, J = 8.0 Hz), 6.81 (1H, s), 6.78-6.76 (1H, m), 2.63 (3H, s), 2.41 (3H, s), 2.38 (3H, s), 1.69 (6H, s).
57 ¹H-NMR (CDCl₃) δ: 8.28 (1H, s), 7.55 (1H, dd, J = 8.1, 1.0 Hz), 7.37 (1H, dd, J = 7.8, 1.0 Hz), 7.25 (1H, t, J = 7.8 Hz), 7.06 (1H, d, J = 7.8 Hz), 6.84-6.83 (1H, m), 6.81-6.79 (1H, m), 2.39 (3H, s), 1.71 (6H, s).
58 ¹H-NMR (CDCl₃) δ: 8.21 (1H, s), 7.44 (1H, d, J = 8.0 Hz), 7.21 (1H, t, J = 7.6 Hz), 7.14 (1H, d, J = 7.3 Hz), 7.09 (1H, d, J = 8.0 Hz), 6.82 (1H, s), 6.78 (1H, d, J = 8.0 Hz), 2.71 (3H, s), 2.39 (3H, s), 1.70 (6H, s).
59 ¹H-NMR (CDCl₃) δ: 8.22 (1H, s), 7.42 (1H, dd, J = 8.3, 0.7 Hz), 7.25 (1H, td, J = 8.3, 5.0 Hz), 7.07 (1H, d, J = 8.3 Hz), 7.05 (1H, ddd, J = 10.5, 8.3, 0.7 Hz), 6.83-6.83 (1H, m), 6.81-6.79 (1H, m), 2.40 (3H, s), 1.71 (6H, s).
60 ¹H-NMR (CDCl₃) δ: 8.25 (1H, dd, J = 7.8, 1.2 Hz, ), 8.21 (1H, s, ), 7.89 (1H, td, J = 7.8, 1.2 Hz, ), 7.77 (1H, td, J = 7.8, 1.2 Hz, ), 7.48 (1H, d, J = 8.5 Hz, ), 7.43 (1H, dd, J = 7.8, 0.7 Hz, ), 7.26 (1H, dd, J = 8.5, 0.7 Hz, ), 2.76 (3H, s, ), 1.75 (6H, s, ).
61 ¹H-NMR (CDCl₃) δ: 8.16 (1H, s, ), 7.47-7.44 (2H, m, ), 7.29-7.25 (2H, m, ), 7.19-7.13 (3H, m, ), 2.71 (3H, s, ), 1.72 (6H, s, ).
62 ¹H-NMR (CDCl₃) δ: 8.24 (1H, dd, J = 7.6, 1.1 Hz, ), 8.19 (1H, s, ), 7.87 (1H, td, J = 5.5, 1.1 Hz, ), 7.75 (1H, td, J = 5.5, 1.1 Hz, ), 7.46 (1H, dd, J = 7.6, 1.1 Hz, ), 7.25 (1H, d, J = 8.4 Hz, ), 7.11 (1H, d, J = 8.4 Hz, ), 2.63 (3H, s, ), 2.41 (3H, s, ), 1.74 (6H, s, ).
63 ¹H-NMR (CDCl₃) δ: 8.24 (1H, s), 8.00 (1H, dd, J = 7.6, 1.1 Hz), 7.81 (1H, td, J = 5.5, 1.1 Hz), 7.65 (1H, td, J = 5.5, 1.1 Hz), 7.42 (1H, dd, J = 7.6, 1.1 Hz), 7.38 (1H, d, J = 8.4 Hz), 7.12 (1H, d, J = 8.4 Hz), 2.63 (3H, s), 2.42 (3H, s), 1.62 (3H, s), 1.61 (3H, s).
64 ¹H-NMR (CDCl₃) δ: 8.29 (1H, s), 7.60 (1H, dd, J = 8.4, 0.8 Hz), 7.54 (1H, dd, J = 7.6, 0.8 Hz), 7.20 (1H, t, J = 8.0 Hz), 7.05 (1H, d, J = 8.0 Hz), 6.83 (1H, s), 6.81-6.79 (1H, m), 2.39 (3H, s), 1.70 (6H, s).
65 ¹H-NMR (CDCl₃) δ: 8.30 (1H, s), 7.60 (1H, dd, J = 8.3, 1.0 Hz), 7.54 (1H, dd, J = 8.3, 1.0 Hz), 7.36-7.33 (1H, m), 7.20 (1H, t, J = 7.9 Hz), 7.01-7.00 (1H, m), 6.89 (1H, t, J = 7.9 Hz), 2.28 (3H, s), 1.72 (6H, s).
66 ¹H-NMR (CDCl₃) δ: 8.20 (1H, s), 7.42 (1H, d, J = 8.1 Hz), 7.21 (1H, t, J = 8.1 Hz), 7.15-7.11 (2H, m), 6.52-6.48 (2H, m), 3.86 (3H, s), 2.71 (3H, s), 1.71 (6H, s).
67 ¹H-NMR (CDCl₃) δ: 8.27 (1H, s), 7.53 (1H, dd, J = 8.1, 1.0 Hz), 7.37 (1H, dd, J = 7.8, 1.0 Hz), 7.24 (1H, t, J = 7.8 Hz), 7.10-7.07 (1H, m), 6.53-6.50 (2H, m), 3.86 (3H, s), 1.71 (6H, s).
68 ¹H-NMR (CDCl₃) δ: 8.21 (1H, s), 7.40 (1H, dd, J = 8.2, 1.0 Hz), 7.20 (1H, t, J = 8.0 Hz), 7.10 (1H, d, J = 8.0 Hz), 6.86 (1H, d, J = 7.6 Hz), 6.82 (1H, s), 6.78 (1H, d, J = 8.0 Hz), 2.67-2.62 (1H, m), 2.39 (3H, s), 1.70 (6H, s), 1.14-1.11 (2H, m), 1.00-0.97 (2H, m).
69 ¹H-NMR (CDCl₃) δ: 8.21 (1H, s), 7.45 (1H, dd, J = 8.0, 1.1 Hz), 7.35-7.33 (1H, m), 7.24 (1H, d, J = 8.0 Hz), 7.16 (1H, d, J = 7.3 Hz), 7.07 (1H, d, J = 7.3 Hz), 6.87 (1H, t, J = 7.6 Hz), 3.14 (2H, q, J = 7.6 Hz), 2.28 (3H, s), 1.71 (6H, s), 1.40 (3H, t, J = 7.6 Hz).
70 ¹H-NMR (CDCl₃) δ: 8.22 (1H, s), 7.40 (1H, dd, J = 8.2, 1.0 Hz), 7.34-7.32 (1H, m), 7.20 (1H, t, J = 7.8 Hz), 7.06-7.04 (1H, m), 6.88-6.85 (2H, m), 2.68-2.63 (1H, m), 2.28 (3H, s), 1.71 (6H, s), 1.14-1.12 (2H, m), 1.00-0.97 (2H, m).
71 ¹H-NMR (CDCl₃) δ: 8.39 (1H, d, J = 1.1 Hz, ), 8.24 (1H, s, ), 7.21 (1H, d, J = 7.6 Hz, ), 7.19 (1H, d, J = 7.6 Hz, ), 6.77 (1H, t, J = 7.6 Hz, ), 2.53 (3H, s, ), 2.31 (3H, d, J = 0.8 Hz, ), 2.24 (3H, s, ), 1.69 (6H, s, ).
72 ¹H-NMR (CDCl₃) δ: 8.24 (1H, s), 7.53 (1H, d, J = 7.7 Hz), 7.43 (1H, d, J = 7.3 Hz), 7.39-7.27 (3H, m), 7.04 (1H, d, J = 6.8 Hz), 6.87 (1H, t, J = 7.6 Hz), 6.33 (1H, dd, J = 17.7, 1.6 Hz), 5.54 (1H, dd, J = 11.1, 1.6 Hz), 2.28 (3H, s), 1.72 (6H, s).
73 ¹H-NMR (CDCl₃) δ: 8.23 (1H, s), 7.55 (1H, dd, J = 7.6, 1.5 Hz), 7.35-7.28 (3H, m), 7.06-7.04 (1H, m), 6.87 (1H, t, J = 7.6 Hz), 5.90-5.90 (1H, m), 5.45-5.44 (1H, m), 2.37 (3H, s), 2.28 (3H, s), 1.72 (6H, s).
74 ¹H-NMR (CDCl₃) δ: 8.38 (1H, s), 8.32 (1H, s), 7.23-7.21 (1H, m), 7.14-7.13 (1H, m), 6.78 (1H, t, J = 7.6 Hz), 2.81 (3H, s), 2.39 (3H, s), 2.23 (3H, s), 1.70 (6H, s).
75 ¹H-NMR (CDCl₃) δ: 8.07 (1H, s), 7.31 (1H, d, J = 7.3 Hz), 7.11 (2H, t, J = 8.5 Hz), 7.05-7.02 (2H, m), 2.60 (3H, s), 2.39 (3H, s), 2.38 (3H, s), 1.68 (6H, s).
76 ¹H-NMR (CDCl₃) δ: 8.12 (1H, s, ), 7.60-7.53 (2H, m, ), 7.24 (1H, dd, J = 7.3, 1.1 Hz, ), 7.15 (1H, d, J = 8.4 Hz, ), 7.08 (1H, d, J = 8.4 Hz, ), 2.86 (3H, s, ), 2.62 (3H, s, ), 2.40 (3H, s, ), 1.76 (6H, s, ).
77 ¹H-NMR (CDCl₃) δ: 8.22 (1H, s), 7.59-7.53 (2H, m), 7.34 (1H, dd, J = 7.3, 1.9 Hz), 7.28 (1H, d, J = 8.4 Hz), 7.09 (1H, d, J = 8.4 Hz), 2.81 (3H, s), 2.63 (3H, s), 2.41 (3H, s), 2.04 (3H, s), 1.22 (3H, s).
78 ¹H-NMR (CDCl₃) δ: 8.09 (1H, s), 7.28-7.24 (1H, m), 7.18-7.11 (3H, m), 7.08 (1H, d, J = 8.4 Hz), 2.62 (3H, s), 2.40 (3H, s), 1.73 (6H, s).
79 ¹H-NMR (CDCl₃) δ: 8.13 (1H, s, ), 7.75-7.70 (1H, m, ), 7.56-7.52 (1H, m, ), 7.27-7.25 (1H, m, ), 7.17 (1H, d, J = 8.4 Hz, ), 7.11 (1H, d, J = 8.4 Hz, ), 2.62 (3H, s, ), 2.41 (3H, s, ), 1.79 (6H, s, ).
80 ¹H-NMR (CDCl₃) δ: 8.22 (1H, s, ), 7.70 (1H, td, J = 7.2, 5.6 Hz, ), 7.53-7.50 (1H, m, ), 7.36 (1H, d, J = 7.2 Hz, ), 7.28 (1H, d, J = 8.4 Hz, ), 7.11 (1H, d, J = 8.4 Hz, ), 2.63 (3H, s, ), 2.41 (3H, s, ), 2.05 (3H, s, ), 1.26 (3H, s, ).
81 ¹H-NMR (CDCl₃) δ: 8.13 (1H, s, ), 7.29-7.25 (2H, m, ), 7.21-7.10 (4H, m, ), 2.71 (3H, s, ), 1.74 (6H, s, ).
82 ¹H-NMR (CDCl₃) δ: 8.17 (1H, s, ), 7.74 (1H, td, J = 7.1, 5.0 Hz, ), 7.57-7.53 (1H, m, ), 7.29-7.23 (2H, m, ), 7.23-7.17 (2H, m, ), 2.71 (3H, s, ), 1.80 (6H, s, ).
83 ¹H-NMR (CDCl₃) δ: 8.27 (1H, s, ), 7.73-7.69 (1H, m, ), 7.54-7.51 (1H, m, ), 7.40-7.35 (2H, m, ), 7.24-7.16 (2H, m, ), 2.72 (3H, s, ), 2.06 (3H, s, ), 1.27 (3H, s, ).
84 ¹H-NMR (CDCl₃) δ: 8.13 (1H, s), 7.30-7.26 (2H, m), 7.24-7.21 (1H, m), 7.20-7.15 (1H, m), 7.10 (1H, dd, J = 7.8, 1.0 Hz), 2.73 (3H, s), 1.73 (6H, s).
85 ¹H-NMR (CDCl₃) δ: 8.16 (1H, s), 7.27-7.25 (1H, m), 7.16 (1H, d, J = 8.4 Hz), 7.12 (1H, d, J = 8.0 Hz), 7.02 (1H, d, J = 2.3 Hz), 6.95 (1H, dd, J = 8.4, 1.9 Hz), 2.62 (3H, s), 2.41 (3H, s), 1.71 (6H, s).
86 ¹H-NMR (CDCl₃) δ: 8.19 (1H, s), 7.38 (1H, d, J = 7.8 Hz), 7.22 (1H, t, J = 7.8 Hz), 7.20 (1H, d, J = 1.7 Hz), 7.17-7.14 (1H, m), 7.12 (1H, dd, J = 8.2, 1.7 Hz), 7.07 (1H, t, J = 8.2 Hz), 2.71 (3H, s), 1.71 (6H, s).
87 ¹H-NMR (CDCl₃) δ: 8.20 (1H, s), 7.36 (1H, dd, J = 8.2, 1.0 Hz), 7.28-7.24 (1H, m), 7.21 (1H, d, J = 1.9 Hz), 7.14 (1H, dd, J = 8.2, 1.9 Hz), 7.09-7.04 (2H, m), 1.72 (6H, s).
88 ¹H-NMR (CDCl₃) δ: 8.26 (1H, s), 7.49 (1H, dd, J = 8.2, 1.0 Hz), 7.39 (1H, dd, J = 7.8, 1.0 Hz), 7.28-7.24 (1H, m), 7.21 (1H, d, J = 1.9 Hz), 7.14 (1H, dd, J = 8.2, 1.9 Hz), 7.04 (1H, d, J = 7.8 Hz), 1.72 (6H, s).
89 ¹H-NMR (CDCl₃) δ: 8.17 (1H, s, ), 7.78-7.72 (1H, m, ), 7.59-7.54 (1H, m, ), 7.31 (1H, d, J = 8.8 Hz, ), 7.28-7.24 (1H, m, ), 7.24-7.21 (1H, m, ), 2.73 (3H, s, ), 1.79 (6H, s, ).
90 ¹H-NMR (CDCl₃) δ:, 8.16 (1H, s,), 7.72 (1H, d, J = 8.0 Hz,), 7.41 (1H, s,), 7.16 (1H, dd, J = 8.6, 1.3 Hz,), 7.12-7.09 (1H, m,), 6.84-6.82 (1H, m,), 6.80-6.78 (1H, m,), 2.47 (3H, s,), 2.39 (3H, s,), 1.71 (6H, s,),
91 ¹H-NMR (CDCl₃) δ: 8.20 (1H, s), 7.63 (1H, d, J = 0.8 Hz), 7.49 (1H, d, J = 8.4 Hz), 7.35-7.33 (1H, m,), 7.17-7.12 (1H, m,), 7.07-7.03 (1H, m), 6.90-6.86 (1H, m), 2.49 (3H, s), 2.28 (3H, s), 1.71 (6H, s).
92 ¹H-NMR (CDCl₃) δ: 8.18 (1H, s), 7.43 (1H, dd, J = 8.6, 2.5 Hz), 7.30 (1H, d, J = 8.4 Hz), 7.23 (1H, d, J = 2.7 Hz), 7.14 (1H, d, J = 8.4 Hz), 6.96 (1H, d, J = 8.4 Hz), 2.63 (3H, s), 2.42 (3H, s), 1.70 (6H, s).
93 ¹H-NMR (CDCl₃) δ: 9.29 (1H, d, J = 6.9 Hz), 8.01 (1H, s), 7.64 (1H, dd, J = 7.8, 1.7 Hz), 7.43 (1H, ddd, J = 8.0, 7.0, 1.6 Hz), 7.16-7.14 (1H, m), 7.03 (1H, td, J = 7.4, 1.0 Hz), 6.96 (1H, dd, J = 8.2, 1.0 Hz), 6.88 (1H, t, J = 6.9 Hz), 2.68 (3H, s), 1.69 (6H, s).
94 ¹H-NMR (CDCl₃) δ: 8.16 (1H, s), 7.70 (1H, dd, J = 8.1, 1.5 Hz), 7.27 (1H, d, J = 8.1 Hz), 7.19 (1H, dd, J = 8.0, 1.5 Hz), 7.12 (1H, d, J = 8.0 Hz), 6.87 (1H, t, J = 8.1 Hz), 2.63 (3H, s), 2.41 (3H, s), 1.75 (6H, s).
95 ¹H-NMR (CDCl₃) δ: 8.18 (1H, s), 7.33 (1H, d, J = 8.0 Hz), 7.11 (1H, d, J = 8.0 Hz), 7.02-7.00 (2H, m), 6.86 (1H, t, J = 8.0 Hz), 2.62 (3H, s), 2.41 (3H, s), 2.22-2.15 (1H, m), 1.72 (6H, s), 1.04-0.98 (2H, m), 0.76-0.72 (2H, m).
96 ¹H-NMR (CDCl₃) δ: 8.08 (1H, s, ), 7.24-7.23 (1H, m, ), 7.13 (2H, dd, J = 14.5, 8.0 Hz, ), 7.05 (1H, d, J = 8.0 Hz, ), 6.95-6.91 (1H, m, ), 2.60 (3H, s, ), 2.38 (6H, s, ), 1.68 (6H, s, ).
97 ¹H-NMR (CDCl₃) δ: 8.25 (1H, s), 8.04 (1H, d, J = 1.1 Hz), 7.53-7.50 (1H, m), 7.33 (1H, d, J = 8.0 Hz), 7.24 (1H, d, J = 8.0 Hz), 7.11 (1H, d, J = 8.0 Hz), 2.63 (3H, s), 2.57 (3H, s), 2.41 (3H, s), 1.73 (6H, s).
98 ¹H-NMR (CDCl₃) δ: 8.10 (1H, s, ), 7.28-7.25 (1H, m, ), 7.17 (1H, dd, J = 8.6, 2.5 Hz, ), 7.08 (2H, dd, J = 9.5, 8.5 Hz, ), 6.84 (1H, td, J = 6.5, 2.5 Hz, ), 2.62 (3H, s, ), 2.40 (3H, s, ), 1.71 (6H, s, ).
99 ¹H-NMR (CDCl₃) δ: 8.18 (1H, s, ), 7.93 (1H, dd, J = 7.0, 2.5 Hz, ), 7.50 (1H, dd, J = 8.5, 4.6 Hz, ), 7.42-7.37 (1H, m, ), 7.18 (1H, d, J = 8.5 Hz, ), 7.11 (1H, d, J = 8.5 Hz, ), 2.63 (3H, s, ), 2.41 (3H, s, ), 1.74 (6H, s, ).
100 ¹H-NMR (CDCl₃) δ: 8.22 (1H, s), 7.72 (1H, dd, J = 7.3, 2.7 Hz), 7.42 (1H, dd, J = 8.4, 5.0 Hz), 7.34 (1H, d, J = 8.4 Hz), 7.30-7.25 (1H, m), 7.13 (1H, d, J = 8.4 Hz), 2.62 (3H, s), 2.41 (3H, s), 1.72 (3H, s), 1.52 (3H, s).
101 ¹H-NMR (CDCl₃) δ: 8.18 (1H, s), 7.34 (1H, d, J = 8.1 Hz), 7.12 (1H, d, J = 8.1 Hz), 7.12 (1H, d, J = 8.1 Hz), 6.84 (1H, d, J = 1.6 Hz), 6.79 (1H, dd, J = 8.1, 1.6 Hz), 2.67 (2H, q, J = 7.5 Hz), 2.63 (3H, s), 2.41 (3H, s), 1.70 (6H, s), 1.27 (3H, t, J = 7.5 Hz).
102 ¹H-NMR (CDCl₃) δ: 8.20 (1H, s), 7.47 (1H, d, J = 8.4 Hz), 7.39-7.37 (1H, m), 7.16-7.12 (2H, m), 6.94 (1H, t, J = 8.4 Hz), 2.63 (3H, s), 2.42 (3H, s), 2.36 (3H, s), 2.34 (3H, s), 1.79 (3H, s).
103 ¹H-NMR (CDCl₃) δ: 8.30 (1H, s), 7.73 (1H, dd, J = 8.0, 1.0 Hz), 7.42-7.39 (2H, m), 7.29 (1H, t, J = 8.0 Hz), 7.10-7.09 (1H, m), 6.97 (1H, t, J = 8.0 Hz), 2.36 (3H, s), 2.36 (3H, s), 1.82 (3H, s).
104 ¹H-NMR (CDCl₃) δ: 8.24 (1H, s), 7.59 (1H, d, J = 8.0 Hz), 7.40-7.39 (1H, m), 7.24 (1H, t, J = 8.0 Hz), 7.17-7.17 (1H, m), 7.13 (1H, dd, J = 8.0, 1.0 Hz), 6.95 (1H, t, J = 8.0 Hz), 2.71 (3H, s), 2.37 (3H, s), 2.35 (3H, s), 1.80 (3H, s).
105 ¹H-NMR (CDCl₃) δ: 8.24 (1H, s), 7.59 (1H, dd, J = 8.0, 0.8 Hz), 7.42-7.40 (1H, m), 7.29 (1H, td, J = 8.0, 4.7 Hz), 7.11 (1H, dd, J = 8.0, 0.8 Hz), 7.07 (1H, ddd, J = 10.3, 8.0, 0.8 Hz), 6.97 (1H, t, J = 8.0 Hz), 2.37 (3H, s), 2.36 (3H, br s), 1.82 (3H, s).
106 ¹H-NMR (CDCl₃) δ: 8.14 (1H, s, ), 7.28-7.24 (1H, m, ), 7.22-7.20 (1H, m, ), 7.19-7.16 (2H, m, ), 7.15-7.13 (1H, m, ), 6.88-6.83 (1H, m, ), 2.71 (3H, s, ), 1.72 (6H, s, ).
107 ¹H-NMR (CDCl₃) δ: 8.36 (1H, s, ), 8.21 (1H, s, ), 7.24 (1H, td, J = 8.8, 1.1 Hz, ), 7.09-7.05 (1H, m, ), 6.90 (1H, dd, J = 7.6, 1.1 Hz, ), 2.74 (3H, s, ), 1.73 (6H, s, ).
108 ¹H-NMR (CDCl₃) δ: 8.30 (1H, s), 8.15 (1H, s), 7.06 (1H, dd, J = 8.8, 2.5 Hz), 7.01 (1H, dd, J = 8.8, 5.3 Hz), 6.70 (1H, td, J = 8.8, 2.5 Hz), 2.67 (3H, s), 1.65 (6H, s).
109 ¹H-NMR (CDCl₃) δ: 8.34 (1H, s), 8.30 (1H, s), 6.95 (1H, d, J = 8.5 Hz), 6.47 (1H, d, J = 2.4 Hz), 6.42 (1H, dd, J = 10.5, 1.7 Hz), 3.84 (3H, s), 2.75 (3H, s), 1.71 (6H, s).
110 ¹H-NMR (CDCl₃) δ: 8.34 (1H, s), 8.30 (1H, s), 6.90 (1H, d, J = 8.0 Hz), 6.78 (1H, s), 6.70 (1H, d, J = 8.4 Hz), 2.75 (3H, s), 2.36 (3H, s), 1.70 (6H, s).
111 ¹H-NMR (CDCl₃) δ: 8.33 (1H, s), 8.30 (1H, s), 7.07-7.05 (1H, m), 6.84 (1H, t, J = 8.2 Hz), 6.63 (1H, dd, J = 8.0, 1.1 Hz), 3.92 (3H, s), 2.75 (3H, s), 1.77 (6H, s).
112 ¹H-NMR (CDCl₃) δ: 8.16 (1H, s), 7.29 (1H, d, J = 8.4 Hz), 7.18 (1H, d, J = 2.3 Hz), 7.10 (2H, t, J = 7.8 Hz), 6.87 (1H, dd, J = 8.6, 2.1 Hz), 6.66 (1H, br s), 2.62 (3H, s), 2.40 (3H, s), 1.68 (6H, s), 1.53 (9H, s).
113 ¹H-NMR (CDCl₃) δ: 8.18 (1H, s), 7.34 (1H, d, J = 7.8 Hz), 7.33-7.30 (1H, m), 7.11 (1H, d, J = 7.8 Hz), 7.05 (1H, dd, J = 7.8, 1.0 Hz), 6.85 (1H, t, J = 7.8 Hz), 2.63 (3H, s), 2.41 (3H, s), 2.27 (3H, s), 2.01 (2H, q, J = 7.4 Hz), 1.62 (3H, s), 1.07 (3H, t, J = 7.4 Hz).
114 ¹H-NMR (CDCl₃) δ: 8.18 (1H, s), 7.33 (1H, d, J = 8.0 Hz), 7.32-7.30 (1H, m), 7.11 (1H, d, J = 8.0 Hz), 7.04 (1H, dd, J = 8.0, 1.1 Hz), 6.85 (1H, t, J = 8.0 Hz), 2.63 (3H, s), 2.41 (3H, s), 2.26 (3H, s), 1.96 (2H, dd, J = 9.2, 7.4 Hz), 1.66-1.50 (2H, m), 1.63 (3H, s), 0.95 (3H, t, J = 7.4 Hz).
115 ¹H-NMR (CDCl₃) δ: 8.28 (1H, s), 7.56 (1H, dd, J = 8.2, 1.0 Hz), 7.37 (1H, dd, J = 7.8, 1.0 Hz), 7.35-7.33 (1H, m), 7.25 (1H, t, J = 8.2 Hz), 7.01-6.99 (1H, m), 6.88 (1H, t, J = 7.8 Hz), 2.28 (3H, s), 2.05-2.00 (2H, m), 1.64 (3H, s), 1.08 (3H, t, J = 7.4 Hz).
116 ¹H-NMR (CDCl₃) δ: 8.22 (1H, s), 7.46 (1H, d, J = 7.6 Hz), 7.32 (1H, d, J = 7.6 Hz), 7.21 (1H, t, J = 7.6 Hz), 7.15-7.14 (1H, m), 7.04 (1H, dd, J = 7.6, 1.3 Hz), 6.85 (1H, t, J = 7.6 Hz), 2.71 (3H, s), 2.28 (3H, s), 2.05-2.00 (2H, m), 1.64 (3H, s), 1.08 (3H, t, J = 7.4 Hz).
117 ¹H-NMR (CDCl₃) δ: 8.28 (1H, s), 7.56 (1H, dd, J = 8.0, 0.8 Hz), 7.37 (1H, dd, J = 8.0, 0.8 Hz), 7.34 (1H, d, J = 8.0 Hz), 7.25 (1H, t, J = 8.0 Hz), 7.00 (1H, d, J = 8.0 Hz), 6.87 (1H, t, J = 8.0 Hz), 2.27 (3H, s), 1.97 (2H, dd, J = 7.4, 7.4 Hz), 1.64 (3H, s), 1.63-1.49 (2H, m), 0.96 (3H, t, J = 7.4 Hz).
118 ¹H-NMR (CDCl₃) δ: 8.22 (1H, s), 7.45 (1H, d, J = 7.6 Hz), 7.33-7.31 (1H, m), 7.21 (1H, t, J = 7.6 Hz), 7.15-7.14 (1H, m), 7.03 (1H, dd, J = 7.6, 1.1 Hz), 6.85 (1H, t, J = 7.6 Hz), 2.71 (3H, s), 2.27 (3H, s), 1.97 (2H, dd, J = 9.0, 7.4 Hz), 1.64 (3H, s), 1.59-1.50 (2H, m), 0.96 (3H, t, J = 7.4 Hz).
119 ¹H-NMR (CDCl₃) δ: 8.19 (1H, s), 7.44 (1H, d, J = 8.4 Hz), 7.34 (1H, dd, J = 8.4, 0.8 Hz), 7.13 (1H, d, J = 8.4 Hz), 7.07 (1H, dd, J = 8.4, 0.8 Hz), 6.90 (1H, t, J = 8.4 Hz), 2.63 (3H, s), 2.62-2.50 (4H, m), 2.42 (3H, s), 2.33 (3H, s), 2.07-1.97 (2H, m).
120 ¹H-NMR (CDCl₃) δ: 8.23 (1H, s), 7.56 (1H, d, J = 7.8 Hz), 7.35 (1H, dd, J = 7.8, 1.0 Hz), 7.24 (1H, t, J = 7.8 Hz), 7.16 (1H, d, J = 7.8 Hz), 7.07 (1H, dd, J = 7.8, 1.0 Hz), 6.91 (1H, t, J = 7.8 Hz), 2.72 (3H, s), 2.63-2.54 (4H, m), 2.34 (3H, s), 2.10-1.98 (2H, m).
121 ¹H-NMR (CDCl₃) δ: 8.29 (1H, s), 7.67 (1H, dd, J = 8.0, 1.0 Hz), 7.39-7.36 (2H, m), 7.29 (1H, t, J = 8.0 Hz), 7.03 (1H, dd, J = 8.0, 1.0 Hz), 6.93 (1H, t, J = 8.0 Hz), 2.64-2.59 (2H, m), 2.56-2.53 (2H, m), 2.34 (3H, s), 2.10-1.97 (2H, m).
122 ¹H-NMR (CDCl₃) δ: 8.18 (1H, s, ), 7.33 (1H, d, J = 8.5 Hz, ), 7.09 (1H, d, J = 8.5 Hz, ), 7.01 (1H, d, J = 10.0 Hz, ), 6.23-6.20 (2H, m, ), 3.05 (6H, s, ), 2.62 (3H, s, ), 2.40 (3H, s, ), 1.69 (6H, s, ).
123 ¹H-NMR (CDCl₃) δ: 8.22 (1H, s), 7.50-7.47 (1H, m), 7.34-7.32 (1H, m), 7.21 (1H, t, J = 7.7 Hz), 7.15-7.13 (1H, m), 7.05 (1H, dd, J = 7.7, 0.7 Hz), 6.88 (1H, t, J = 7.7 Hz), 2.71 (3H, s), 2.28 (3H, s), 2.26-2.23 (2H, m), 2.10-2.06 (2H, m), 1.97-1.90 (4H, m).
124 ¹H-NMR (CDCl₃) δ: 8.18 (1H, s, ), 7.36 (1H, d, J = 8.3 Hz, ), 7.33-7.31 (1H, m, ), 7.11 (1H, d, J = 8.3 Hz, ), 7.05 (1H, dd, J = 8.3, 1.0 Hz, ), 6.87 (1H, t, J = 8.3 Hz, ), 2.62 (3H, s, ), 2.41 (3H, s, ), 2.28 (3H, s, ), 2.27-2.23 (2H, m, ), 2.09-2.03 (2H, m, ), 1.95-1.90 (4H, m, ).
125 ¹H-NMR (CDCl₃) δ: 8.16 (1H, s), 7.31 (1H, d, J = 8.3 Hz), 7.09 (1H, d, J = 8.3 Hz), 6.96 (1H, d, J = 8.3 Hz), 6.14-6.08 (2H, m), 4.29 (1H, q, J = 5.0 Hz), 2.89 (3H, d, J = 5.0 Hz), 2.62 (3H, s), 2.40 (3H, s), 1.69 (6H, s).
126 ¹H-NMR (CDCl₃) δ: 8.16 (1H, s), 7.31 (1H, d, J = 8.3 Hz), 7.09 (1H, d, J = 8.3 Hz), 6.94 (1H, d, J = 8.3 Hz), 6.14-6.08 (2H, m), 4.19-4.13 (1H, m), 3.25-3.18 (2H, m), 2.62 (3H, s), 2.40 (3H, s), 1.69 (6H, s), 1.29 (3H, t, J = 7.1 Hz).
127 ¹H-NMR (CDCl₃) δ: 8.17 (1H, s), 7.34 (1H, d, J = 8.3 Hz), 7.09 (1H, d, J = 8.3 Hz), 6.99 (1H, d, J = 9.5 Hz), 6.23-6.19 (2H, m), 3.44 (2H, q, J = 7.2 Hz), 3.00 (3H, s), 2.62 (3H, s), 2.40 (3H, s), 1.69 (6H, s), 1.19 (3H, t, J = 7.1 Hz).
128 ¹H-NMR (CDCl₃) δ: 9.30-9.28 (1H, m), 7.99 (1H, s), 7.48-7.46 (1H, m), 7.30-7.28 (1H, m), 7.15-7.14 (1H, m), 6.92 (1H, t, J = 7.6 Hz), 6.87 (1H, t, J = 7.0 Hz), 2.67 (3H, s), 2.26 (3H, s), 1.68 (6H, s).
129 ¹H-NMR (CDCl₃) δ: 9.20 (1H, d, J = 7.1 Hz), 7.95 (1H, s), 7.48 (1H, dd, J = 7.8, 1.0 Hz), 7.29-7.26 (1H, m), 6.92 (1H, t, J = 7.6 Hz), 6.76 (1H, d, J = 7.1 Hz), 2.60 (3H, s), 2.39 (3H, s), 2.25 (3H, s), 1.67 (6H, s).
130 ¹H-NMR (CDCl₃) δ: 8.15 (1H, s), 7.26 (1H, d, J = 8.4 Hz), 7.12 (1H, d, J = 8.0 Hz), 7.02 (1H, ddd, J = 8.9, 5.3, 2.0 Hz), 6.79-6.74 (1H, m), 2.62 (3H, s), 2.41 (3H, s), 1.77 (6H, s).
131 ¹H-NMR (CDCl₃) δ: 8.18 (1H, s), 7.34 (1H, d, J = 8.1 Hz), 7.11 (1H, d, J = 8.1 Hz), 7.09 (1H, d, J = 8.1 Hz), 6.81 (1H, br s), 6.77-6.74 (1H, m), 2.63 (3H, s), 2.41 (3H, s), 2.37 (3H, s), 2.00 (2H, q, J = 7.4 Hz), 1.61 (3H, s), 1.06 (3H, t, J = 7.4 Hz).
132 ¹H-NMR (CDCl₃) δ: 8.18 (1H, s), 7.39 (1H, d, J = 8.3 Hz), 7.33-7.31 (1H, m), 7.12 (1H, d, J = 8.3 Hz), 7.07-7.04 (1H, m), 6.86 (1H, t, J = 8.3 Hz), 2.62 (3H, s), 2.41 (3H, s), 2.32 (3H, s), 2.08-2.06 (2H, m), 1.96-1.89 (2H, m), 1.77-1.66 (6H, m).
133 ¹H-NMR (CDCl₃) δ: 8.22 (1H, s), 7.45 (1H, d, J = 7.7 Hz), 7.21 (1H, t, J = 7.7 Hz), 7.14 (1H, dd, J = 7.7, 1.0 Hz), 7.08 (1H, d, J = 7.7 Hz), 6.81 (1H, br s), 6.76 (1H, dd, J = 7.7, 1.0 Hz), 2.71 (3H, s), 2.38 (3H, s), 2.01 (2H, q, J = 7.3 Hz), 1.62 (3H, s), 1.07 (3H, t, J = 7.3 Hz).
134 ¹H-NMR (CDCl₃) δ: 8.21 (1H, s), 7.51 (1H, d, J = 7.6 Hz), 7.35-7.31 (1H, m), 7.22 (1H, t, J = 7.8 Hz), 7.16-7.13 (1H, m), 7.05 (1H, dd, J = 7.6, 1.0 Hz), 6.87 (1H, t, J = 7.6 Hz), 2.71 (3H, s), 2.32 (3H, s), 2.13-2.05 (2H, m), 1.98-1.88 (2H, m), 1.79-1.70 (6H, m).
135 ¹H-NMR (CDCl₃) δ: 8.18 (1H, s), 7.34 (1H, d, J = 7.8 Hz), 7.32-7.30 (1H, m), 7.11 (1H, d, J = 7.8 Hz), 7.04 (1H, dd, J = 7.8, 1.2 Hz), 6.84 (1H, t, J = 7.8 Hz), 2.63 (3H, s), 2.41 (3H, s), 2.27 (3H, s), 1.97 (2H, dd, J = 8.9, 7.4 Hz), 1.63 (3H, s), 1.54-1.44 (2H, br m), 1.38-1.31 (2H, m), 0.91 (3H, t, J = 7.4 Hz).
136 ¹H-NMR (CDCl₃) δ: 8.19 (1H, s), 7.38 (1H, d, J = 8.5 Hz), 7.22 (1H, dd, J = 7.3, 7.3 Hz), 7.16 (1H, d, J = 7.3 Hz), 7.02 (1H, ddd, J = 8.9, 5.2, 2.1 Hz), 6.78 (1H, td, J = 8.5, 6.8 Hz), 2.71 (3H, s), 1.77 (6H, s).
137 ¹H-NMR (CDCl₃) δ: 8.25 (1H, s), 7.49 (1H, dd, J = 8.4, 1.1 Hz), 7.39 (1H, dd, J = 8.0, 1.1 Hz), 7.29-7.25 (1H, m), 6.99 (1H, ddd, J = 9.2, 5.3, 2.3 Hz), 6.83-6.78 (1H, m), 1.78 (6H, s).
138 ¹H-NMR (CDCl₃) δ: 8.18 (1H, s), 7.33 (1H, d, J = 8.0 Hz), 7.11 (1H, d, J = 8.0 Hz), 7.09 (1H, d, J = 8.0 Hz), 6.79 (1H, s), 6.76-6.75 (1H, m), 2.63 (3H, s), 2.41 (3H, s), 2.37 (3H, s), 1.94 (2H, dd, J = 9.2, 7.4 Hz), 1.63-1.50 (2H, m), 1.62 (3H, s), 0.94 (3H, t, J = 7.4 Hz).
139 ¹H-NMR (CDCl₃) δ: 8.18 (1H, s), 7.33 (1H, d, J = 8.4 Hz), 7.11 (1H, d, J = 8.4 Hz), 7.09 (1H, d, J = 8.4 Hz), 6.80 (1H, s), 6.76-6.74 (1H, m), 2.63 (3H, s), 2.41 (3H, s), 2.37 (3H, s), 1.95 (2H, dd, J = 9.0, 7.4 Hz), 1.61 (3H, s), 1.57-1.47 (2H, m), 1.35-1.32 (2H, m), 0.90 (3H, t, J = 7.4 Hz).
140 ¹H-NMR (CDCl₃) δ: 8.18 (1H, s), 7.36 (1H, d, J = 8.0 Hz), 7.11 (1H, d, J = 8.0 Hz), 7.10 (1H, d, J = 8.0 Hz), 6.83 (1H, br s), 6.80-6.76 (1H, m), 2.62 (3H, s), 2.41 (3H, s), 2.38 (3H, s), 2.27-2.22 (2H, m), 2.09-2.03 (2H, m), 1.94-1.87 (4H, m).
141 ¹H-NMR (CDCl₃) δ: 8.18 (1H, s), 7.40 (1H, d, J = 8.6 Hz), 7.12 (1H, d, J = 8.6 Hz), 7.11 (1H, d, J = 8.6 Hz), 6.85 (1H, s), 6.78-6.77 (1H, m), 2.62 (3H, s), 2.41 (3H, s), 2.38 (3H, s), 2.05-1.91 (4H, m), 1.76-1.70 (4H, m), 1.65-1.50 (2H, m).
142 ¹H-NMR (CDCl₃) δ: 8.24 (1H, s), 7.97 (1H, d, J = 8.4 Hz), 7.36-7.34 (1H, m), 7.27-7.22 (2H, m), 6.87 (1H, t, J = 7.6 Hz), 6.76-6.75 (1H, m), 2.80 (3H, s), 2.26 (3H, s), 1.68 (6H, s).
143 ¹H-NMR (CDCl₃) δ: 8.17 (1H, s), 7.32 (1H, d, J = 8.4 Hz), 7.12 (1H, d, J = 8.4 Hz), 6.92 (1H, dd, J = 8.0, 1.1 Hz), 6.77 (1H, t, J = 7.3 Hz), 2.62 (3H, s), 2.41 (3H, s), 2.35 (3H, d, J = 1.9 Hz), 1.75 (6H, s).
144 ¹H-NMR (CDCl₃) δ: 9.38 (1H, dd, J = 7.1, 1.0 Hz), 8.06 (1H, s), 7.48 (1H, d, J = 8.0 Hz), 7.43 (1H, dd, J = 7.4, 1.0 Hz), 6.91-6.89 (1H, m), 6.86-6.83 (1H, m), 6.78 (1H, s), 2.38 (3H, s), 1.68 (6H, s).
145 ¹H-NMR (CDCl₃) δ: 8.14 (1H, s, ), 7.16 (1H, d, J = 8.8 Hz, ), 7.12 (1H, d, J = 8.4 Hz, ), 7.06 (1H, d, J = 8.4 Hz, ), 6.93 (1H, d, J = 2.7 Hz, ), 6.65 (1H, dd, J = 8.8, 2.7 Hz, ), 3.88 (3H, s, ), 2.61 (3H, s, ), 2.40 (3H, s, ), 1.70 (6H, s, ).
146 ¹H-NMR (CDCl₃) δ: 8.19 (1H, s), 7.43 (1H, d, J = 8.0 Hz), 7.13 (1H, d, J = 8.6 Hz), 7.12 (1H, d, J = 8.6 Hz), 6.90 (1H, s), 6.82-6.80 (1H, m), 2.63 (3H, s), 2.63-2.57 (2H, m), 2.54-2.49 (2H, m), 2.42 (3H, s), 2.40 (3H, s), 2.07-1.94 (2H, m).
147 ¹H-NMR (CDCl₃) δ: 8.19 (1H, s), 7.33 (1H, d, J = 8.4 Hz), 7.12 (1H, d, J = 8.4 Hz), 7.11 (1H, d, J = 8.4 Hz), 6.88 (1H, br s), 6.79-6.77 (1H, m), 3.77 (1H, d, J = 10.3 Hz), 3.70 (1H, d, J = 10.3 Hz), 3.48 (3H, s), 2.62 (3H, s), 2.41 (3H, s), 2.38 (3H, s), 1.64 (3H, s).
148 ¹H-NMR (CDCl₃) δ: 8.18 (1H, s), 7.33 (1H, d, J = 8.8 Hz), 7.12 (1H, d, J = 8.8 Hz), 6.91 (1H, dd, J = 7.9, 1.3 Hz), 6.77-6.73 (1H, m), 2.63 (3H, s), 2.41 (3H, s), 2.34 (3H, d, J = 2.0 Hz), 2.03-1.96 (2H, m), 1.67 (3H, s), 1.63-1.43 (2H, m), 1.41-1.30 (2H, m), 0.90 (3H, t, J = 7.3 Hz).
149 ¹H-NMR (CDCl₃) δ: 8.18 (1H, s), 7.32 (1H, d, J = 8.0 Hz), 7.10 (1H, d, J = 8.0 Hz), 6.95 (1H, d, J = 8.0 Hz), 6.76 (1H, d, J = 8.0 Hz), 2.62 (3H, s), 2.41 (3H, s), 2.33 (3H, s), 2.19 (3H, s), 1.69 (6H, s).
150 ¹H-NMR (CDCl₃) δ: 8.28 (1H, s), 7.55 (1H, dd, J = 8.0, 0.8 Hz), 7.36 (1H, dd, J = 7.8, 1.0 Hz), 7.24 (1H, t, J = 8.0 Hz), 6.91 (1H, d, J = 8.0 Hz), 6.79 (1H, d, J = 8.0 Hz), 2.33 (3H, s), 2.20 (3H, s), 1.71 (6H, s).
151 ¹H-NMR (CDCl₃) δ: 8.18 (1H, s), 7.33 (1H, d, J = 8.0 Hz), 7.11 (1H, d, J = 8.4 Hz), 7.08 (1H, d, J = 8.0 Hz), 6.80 (1H, br s), 6.76-6.74 (1H, m), 2.63 (3H, s), 2.41 (3H, s), 2.37 (3H, s), 1.95 (2H, t, J = 8.4 Hz), 1.61 (3H, s), 1.52-1.47 (2H, m), 1.32-1.27 (4H, m), 0.87 (3H, t, J = 7.3 Hz).
152 ¹H-NMR (CDCl₃) δ: 8.19 (1H, s), 7.31 (1H, d, J = 8.0 Hz), 7.11 (1H, d, J = 8.0 Hz), 7.08 (1H, d, J = 8.0 Hz), 6.78 (1H, s), 6.76-6.74 (1H, m), 2.63 (3H, s), 2.41 (3H, s), 2.37 (3H, s), 2.04-2.00 (1H, m), 1.90-1.87 (2H, m), 1.64 (3H, s), 0.99 (3H, d, J = 7.1 Hz), 0.97 (3H, d, J = 7.1 Hz).
153 ¹H-NMR (CDCl₃) δ: 8.18 (1H, s), 7.87 (1H, d, J = 9.0 Hz), 7.36-7.35 (1H, m), 7.27-7.24 (1H, m), 7.14 (1H, d, J = 9.0 Hz), 6.86 (1H, t, J = 7.6 Hz), 2.77 (3H, s), 2.40 (3H, s), 2.26 (3H, s), 1.67 (6H, s).
154 ¹H-NMR (CDCl₃) δ: 8.24 (1H, s), 7.97 (1H, d, J = 8.8 Hz), 7.39 (1H, d, J = 8.0 Hz), 7.26-7.22 (1H, m), 6.79-6.75 (3H, m), 2.80 (3H, s), 2.38 (3H, s), 1.67 (6H, s).
155 ¹H-NMR (CDCl₃) δ: 8.09 (1H, s), 7.26-7.22 (1H, m), 7.19-7.08 (4H, m), 2.62 (3H, s), 2.41 (3H, s), 2.06-1.98 (2H, m), 1.63 (3H, s), 1.11 (3H, t, J = 7.4 Hz).
156 ¹H-NMR (CDCl₃) δ: 8.16 (1H, s), 7.27 (1H, d, J = 8.3 Hz), 7.15 (1H, d, J = 8.5 Hz), 7.12 (1H, d, J = 8.3 Hz), 7.01 (1H, d, J = 2.4 Hz), 6.93 (1H, dd, J = 8.1, 2.4 Hz), 2.63 (3H, s), 2.41 (3H, s), 2.00-1.93 (2H, m), 1.63 (3H, s), 1.59-1.42 (2H, m), 1.41-1.30 (2H, m), 0.91 (3H, t, J = 7.5 Hz).
157 ¹H-NMR (CDCl₃) δ: 8.13 (1H, s), 7.23-7.18 (2H, m), 6.91 (1H, d, J = 7.6 Hz), 6.76-6.72 (2H, m), 4.04 (3H, s), 2.31 (3H, s), 2.17 (3H, s), 1.68 (6H, s).
158 ¹H-NMR (CDCl₃) δ: 8.22 (1H, s), 7.42 (1H, dd, J = 8.2, 1.0 Hz), 7.24 (1H, td, J = 7.1, 4.8 Hz), 7.04 (1H, ddd, J = 10.2, 8.1, 1.0 Hz), 6.92 (1H, d, J = 8.0 Hz), 6.79 (1H, d, J = 8.0 Hz), 2.34 (3H, s), 2.20 (3H, s), 1.71 (6H, s).
159 ¹H-NMR (CDCl₃) δ: 8.24 (1H, s), 7.99 (1H, d, J = 8.8 Hz), 7.34-7.32 (1H, m), 7.28-7.25 (1H, m), 7.23-7.19 (1H, m), 6.91 (1H, td, J = 6.9, 4.6 Hz), 6.79 (1H, d, J = 6.9 Hz), 2.81 (3H, s), 1.73 (6H, s).
160 ¹H-NMR (CDCl₃) δ: 8.18 (1H, s), 7.87 (1H, d, J = 8.8 Hz), 7.40 (1H, d, J = 8.0 Hz), 7.14 (1H, d, J = 9.2 Hz), 6.78 (1H, d, J = 8.0 Hz), 6.75 (1H, s), 2.77 (3H, s), 2.40 (3H, s), 2.36 (3H, s), 1.67 (6H, s).
161 ¹H-NMR (CDCl₃) δ: 8.53-8.51 (1H, m), 8.20 (1H, s), 8.07-8.05 (1H, m), 7.39 (1H, d, J = 8.0 Hz), 7.28 (1H, ddd, J = 8.8, 6.5, 1.1 Hz), 6.90-6.87 (1H, m), 6.80-6.78 (1H, m), 6.76 (1H, s), 2.37 (3H, s), 1.67 (6H, s).
162 ¹H-NMR (CDCl₃) δ: 8.18 (1H, s), 7.89 (1H, d, J = 9.2 Hz), 7.35-7.33 (1H, m), 7.22-7.16 (2H, m), 6.93-6.88 (1H, m), 2.78 (3H, s), 2.41 (3H, s), 1.73 (6H, s).
163 ¹H-NMR (CDCl₃) δ: 8.20 (1H, s), 7.39 (1H, d, J = 8.3 Hz), 7.13 (1H, d, J = 8.3 Hz), 7.09 (1H, d, J = 8.3 Hz), 6.81 (1H, br s), 6.76-6.73 (1H, m), 2.92 (1H, tt, J = 8.5, 8.5 Hz), 2.64 (3H, s), 2.41 (3H, s), 2.37 (3H, s), 2.22-2.16 (1H, m), 2.13-2.08 (1H, m), 1.99-1.94 (2H, m), 1.88-1.82 (1H, m), 1.79-1.72 (1H, m), 1.49 (3H, s).
164 ¹H-NMR (CDCl₃) δ: 8.16 (1H, s), 7.33 (1H, d, J = 8.3 Hz), 7.12 (1H, d, J = 8.3 Hz), 7.10 (1H, d, J = 8.3 Hz), 6.82 (1H, br s), 6.78-6.75 (1H, m), 2.63 (3H, s), 2.41 (3H, s), 2.38 (3H, s), 1.65 (3H, s), 1.48-1.43 (1H, m), 0.70-0.64 (1H, m), 0.58-0.49 (3H, m).
165 ¹H-NMR (CDCl₃) δ: 8.19 (1H, s), 7.38 (1H, d, J = 8.1 Hz), 7.12 (1H, d, J = 8.1 Hz), 7.10 (1H, d, J = 8.1 Hz), 6.80 (1H, br s), 6.75 (1H, dd, J = 8.1, 0.9 Hz), 2.63 (3H, s), 2.41 (3H, s), 2.37 (3H, s), 2.25-2.19 (1H, m), 1.54 (3H, s), 1.10 (3H, d, J = 4.2 Hz), 1.08 (3H, d, J = 4.2 Hz).
166 ¹H-NMR (CDCl₃) δ: 8.21 (1H, s), 7.42 (1H, d, J = 8.3 Hz), 7.13 (1H, d, J = 8.3 Hz), 7.12 (1H, d, J = 8.3 Hz), 6.80 (1H, br s), 6.74 (1H, dd, J = 8.3, 1.0 Hz), 2.64 (3H, s), 2.42 (3H, s), 2.37 (3H, s), 1.50 (3H, s), 1.11 (9H, s).
167 ¹H-NMR (CDCl₃) δ: 8.19 (1H, s), 7.39 (1H, d, J = 8.4 Hz), 7.17 (1H, d, J = 7.6 Hz), 7.13 (1H, d, J = 8.4 Hz), 6.89 (1H, br s), 6.83-6.82 (1H, m), 3.86 (1H, d, J = 11.5 Hz), 3.83 (1H, d, J = 11.5 Hz), 2.63 (3H, s), 2.41 (3H, s), 2.40 (3H, s), 1.76 (3H, s).
168 ¹H-NMR (CDCl₃) δ: 8.16 (1H, s), 7.27 (1H, d, J = 8.4 Hz), 7.21 (1H, dd, J = 8.4, 6.1 Hz), 7.12 (1H, d, J = 8.4 Hz), 6.70 (1H, dd, J = 9.5, 2.3 Hz), 6.65 (1H, td, J = 8.4, 2.3 Hz), 2.63 (3H, s), 2.41 (3H, s), 1.95 (2H, t, J = 8.4 Hz), 1.64 (3H, s), 1.59-1.46 (2H, m), 0.95 (3H, t, J = 7.3 Hz).
169 ¹H-NMR (CDCl₃) δ: 8.17 (1H, s), 7.28 (1H, d, J = 8.4 Hz), 7.22 (1H, dd, J = 8.4, 6.1 Hz), 7.12 (1H, d, J = 8.4 Hz), 6.71 (1H, dd, J = 9.5, 2.7 Hz), 6.65 (1H, td, J = 8.8, 2.7 Hz), 2.63 (3H, s), 2.41 (3H, s), 2.01 (2H, q, J = 7.5 Hz), 1.63 (3H, s), 1.06 (3H, t, J = 7.4 Hz).
170 ¹H-NMR (CDCl₃) δ: 8.18 (1H, s), 7.36 (1H, d, J = 8.0 Hz), 7.15 (1H, br s), 7.11 (1H, d, J = 8.4 Hz), 6.85 (1H, br s), 2.63 (3H, s), 2.41 (3H, s), 2.24 (3H, s), 2.20 (3H, s), 1.69 (6H, s).
171 ¹H-NMR (CDCl₃) δ: 8.17 (1H, s), 7.87 (1H, d, J = 9.0 Hz), 7.63-7.63 (2H, m), 7.16 (1H, d, J = 9.0 Hz), 6.91 (1H, t, J = 7.8 Hz), 2.78 (3H, s), 2.40 (3H, s), 1.72 (6H, s).
172 ¹H-NMR (CDCl₃) δ: 8.19 (1H, s), 7.88 (1H, d, J = 9.0 Hz), 7.54-7.52 (1H, m), 7.41-7.37 (1H, m), 7.15 (1H, d, J = 9.0 Hz), 6.99-6.92 (2H, m), 2.78 (3H, s), 2.40 (3H, s), 1.68 (6H, s).
173 ¹H-NMR (CDCl₃) δ: 8.15 (1H, s), 7.85 (1H, d, J = 8.8 Hz), 7.53-7.50 (1H, m), 7.16 (1H, d, J = 9.0 Hz), 6.68-6.63 (2H, m), 2.77 (3H, s), 2.40 (3H, s), 1.68 (6H, s).
174 ¹H-NMR (CDCl₃) δ: 8.16 (1H, s), 7.86 (1H, d, J = 8.8 Hz), 7.61 (1H, dd, J = 8.0, 1.5 Hz), 7.49 (1H, dd, J = 7.6, 1.5 Hz), 7.17 (1H, d, J = 9.2 Hz), 6.87 (1H, t, J = 7.8 Hz), 2.78 (3H, s), 2.41 (3H, s), 1.72 (6H, s).
175 ¹H-NMR (CDCl₃) δ: 8.17 (1H, s), 7.87 (1H, d, J = 9.0 Hz), 7.31 (1H, dd, J = 7.7, 1.6 Hz), 7.13 (1H, d, J = 8.8 Hz), 6.95 (1H, dd, J = 7.7, 1.6 Hz), 6.88-6.85 (1H, m), 2.77 (3H, s), 2.40 (3H, s), 2.23-2.17 (1H, m), 1.69 (6H, s), 0.99-0.96 (2H, m), 0.74-0.70 (2H, m).
176 ¹H-NMR (CDCl₃) δ: 8.18 (1H, s), 7.37 (1H, d, J = 8.0 Hz), 7.14 (1H, d, J = 8.0 Hz), 7.11 (1H, d, J = 8.0 Hz), 6.83 (1H, br s), 6.81 (1H, d, J = 8.0 Hz), 4.20-4.09 (2H, m), 2.96 (2H, q, J = 13.7 Hz), 2.62 (3H, s), 2.41 (3H, s), 2.39 (3H, s), 1.83 (3H, s), 1.24 (3H, t, J = 7.3 Hz).
177 ¹H-NMR (CDCl₃) δ: 8.29 (1H, s), 7.28 (1H, d, J = 8.0 Hz), 7.22 (1H, d, J = 8.0 Hz), 7.16 (1H, d, J = 8.0 Hz), 6.90-6.86 (2H, m), 3.11 (1H, d, J = 14.1 Hz), 3.03 (1H, d, J = 14.1 Hz), 2.62 (3H, s), 2.42 (6H, s), 1.80 (3H, s).
178 ¹H-NMR (CDCl₃) δ: 8.17 (1H, s), 7.84 (1H, d, J = 9.2 Hz), 7.43 (1H, d, J = 8.4 Hz), 7.13 (1H, d, J = 9.2 Hz), 6.50 (1H, dd, J = 8.6, 2.5 Hz), 6.47 (1H, d, J = 2.7 Hz), 3.84 (3H, s), 2.77 (3H, s), 2.40 (3H, s), 1.68 (6H, s).
179 ¹H-NMR (CDCl₃) δ: 8.18 (1H, s), 7.87 (1H, d, J = 9.0 Hz), 7.36 (1H, dd, J = 7.7, 1.6 Hz), 7.27-7.26 (1H, m), 7.13 (1H, d, J = 9.0 Hz), 6.92-6.87 (1H, m), 2.77 (3H, s), 2.67 (2H, q, J = 7.6 Hz), 2.40 (3H, d, J = 3.2 Hz), 1.67 (6H, s), 1.24 (3H, t, J = 7.6 Hz).
180 ¹H-NMR (CDCl₃) δ: 8.22 (1H, s), 7.96 (1H, d, J = 9.2 Hz), 7.40 (1H, d, J = 7.6 Hz), 7.28-7.25 (1H, m), 6.79-6.75 (3H, m), 3.23 (2H, q, J = 7.5 Hz), 2.37 (3H, s), 1.67 (6H, s), 1.44 (3H, t, J = 7.4 Hz).
181 ¹H-NMR (CDCl₃) δ: 8.19 (1H, s), 7.91 (1H, d, J = 8.8 Hz), 7.39 (1H, d, J = 7.8 Hz), 7.25 (1H, d, J = 10.0 Hz), 6.80-6.76 (2H, m), 3.41-3.37 (2H, m), 3.10-3.05 (2H, m), 2.40-2.28 (5H, m), 1.67 (6H, s).
182 ¹H-NMR (CDCl₃) δ: 8.17 (1H, s), 7.86 (1H, d, J = 9.0 Hz), 7.25 (1H, d, J = 8.3 Hz), 7.13 (1H, d, J = 9.0 Hz), 6.77 (1H, d, J = 7.8 Hz), 2.77 (3H, s), 2.39 (3H, s), 2.31 (3H, s), 2.18 (3H, s), 1.66 (6H, s).
183 ¹H-NMR (CDCl₃) δ: 8.17 (1H, s), 7.25 (1H, d, J = 8.4 Hz), 7.21 (1H, dd, J = 8.8, 6.1 Hz), 7.11 (1H, d, J = 8.4 Hz), 6.69 (1H, dd, J = 9.5, 2.7 Hz), 6.64 (1H, td, J = 6.4, 2.5 Hz), 2.63 (3H, s), 2.41 (3H, s), 2.05-1.97 (1H, m), 1.91-1.88 (2H, m), 1.66 (3H, s), 0.99 (3H, d, J = 6.9 Hz), 0.97 (3H, d, J = 6.9 Hz).
184 ¹H-NMR (CDCl₃) δ: 8.16 (1H, s), 7.26 (1H, d, J = 8.4 Hz), 7.12 (1H, d, J = 8.4 Hz), 7.00-6.98 (2H, m), 2.62 (3H, s), 2.41 (3H, s), 1.76 (6H, s).
185 ¹H-NMR (CDCl₃) δ: 8.17 (1H, s), 7.86 (1H, d, J = 8.8 Hz), 7.58 (1H, dd, J = 7.4, 1.3 Hz), 7.43 (1H, dd, J = 7.6, 1.9 Hz), 7.15 (1H, d, J = 9.2 Hz), 7.06-7.00 (1H, m), 6.96-6.93 (1H, m), 5.85 (1H, dd, J = 17.9, 1.5 Hz), 5.34 (1H, dd, J = 11.1, 1.5 Hz), 2.78 (3H, s), 2.40 (3H, s), 1.69 (6H, s).
186 ¹H-NMR (CDCl₃) δ: 8.19 (1H, s), 7.87 (1H, d, J = 8.8 Hz), 7.26-7.24 (1H, m), 7.17 (1H, d, J = 8.8 Hz), 7.10 (1H, td, J = 6.7, 3.1 Hz), 6.90 (1H, dd, J = 8.8, 4.6 Hz), 2.78 (3H, s), 2.41 (3H, s), 1.66 (6H, s).
187 ¹H-NMR (CDCl₃) δ: 8.23 (1H, s), 7.97 (1H, d, J = 8.3 Hz), 7.26-7.20 (2H, m), 6.76 (2H, dd, J = 13.4, 7.3 Hz), 2.79 (3H, s), 2.32 (3H, s), 2.18 (3H, s), 1.67 (6H, s).
188 ¹H-NMR (CDCl₃) δ: 8.18 (1H, s), 7.36 (1H, d, J = 8.4 Hz), 7.20 (1H, d, J = 7.6 Hz), 7.14 (1H, d, J = 8.4 Hz), 6.88 (1H, br s), 6.87-6.85 (1H, m), 6.67 (1H, br s), 5.40 (1H, br s), 3.01 (1H, d, J = 14.2 Hz), 2.96 (1H, d, J = 14.2 Hz), 2.64 (3H, s), 2.42 (3H, s), 2.41 (3H, s), 1.71 (3H, s).
189 ¹H-NMR (CDCl₃) δ: 8.19 (1H, s), 7.37 (1H, dd, J = 8.2, 2.1 Hz), 7.13-7.09 (2H, m), 6.80 (1H, s), 6.77-6.73 (1H, m), 2.63 (3H, s), 2.41 (3H, s), 2.37 (3H, s), 1.95-1.81 (2H, m), 1.54 (3H, d, J = 2.3 Hz), 1.23-1.17 (1H, m), 1.09 (3H, dd, J = 12.2, 7.2 Hz), 0.93 (3H, q, J = 7.2 Hz).
190 ¹H-NMR (CDCl₃) δ: 8.18 (1H, s), 7.37 (1H, d, J = 8.0 Hz), 7.15 (1H, d, J = 8.0 Hz), 7.12 (1H, d, J = 8.0 Hz), 6.84 (1H, br s), 6.81 (1H, dd, J = 8.0, 1.1 Hz), 3.69 (3H, s), 3.02 (1H, d, J = 14.0 Hz), 2.94 (1H, d, J = 14.0 Hz), 2.62 (3H, s), 2.41 (3H, s), 2.39 (3H, s), 1.83 (3H, s).
191 ¹H-NMR (CDCl₃) δ: 8.26 (1H, s), 7.57 (1H, d, J = 7.6 Hz), 7.35 (1H, dd, J = 7.6, 0.8 Hz), 7.23 (1H, t, J = 8.0 Hz), 7.15 (1H, br s), 6.78 (1H, br s), 2.22 (3H, s), 2.19 (3H, s), 1.68 (6H, s).
192 ¹H-NMR (CDCl₃) δ: 8.01 (1H, s), 7.20 (1H, d, J = 8.0 Hz), 7.08-7.03 (2H, m), 6.67 (1H, d, J = 8.0 Hz), 2.61 (3H, s), 2.39 (3H, s), 2.24 (3H, s), 1.72 (3H, s), 1.65 (6H, s).
193 ¹H-NMR (CDCl₃) δ: 8.17 (1H, s), 7.27 (1H, d, J = 8.0 Hz), 7.21 (1H, dd, J = 8.8, 6.1 Hz), 7.12 (1H, d, J = 8.4 Hz), 6.70 (1H, dd, J = 9.5, 2.7 Hz), 6.65 (1H, td, J = 8.4, 2.7 Hz), 2.63 (3H, s), 2.41 (3H, s), 1.97-1.94 (2H, m), 1.63 (3H, s), 1.59-1.44 (2H, m), 1.36-1.23 (4H, m), 0.92-0.84 (3H, m).
194 ¹H-NMR (CDCl₃) δ: 8.18 (1H, s), 7.87 (1H, d, J = 9.2 Hz), 7.36 (1H, dd, J = 7.6, 1.5 Hz), 7.24 (1H, dd, J = 7.4, 1.7 Hz), 7.13 (1H, d, J = 9.2 Hz), 6.88 (1H, t, J = 7.4 Hz), 2.77 (3H, s), 2.62 (2H, t, J = 7.6 Hz), 2.40 (3H, s), 1.69-1.63 (2H, m), 1.66 (6H, s), 0.98 (3H, t, J = 7.4 Hz).
195 ¹H-NMR (CDCl₃) δ: 8.17 (1H, s, ), 7.86 (1H, d, J = 9.2 Hz, ), 7.43 (1H, dd, J = 7.8, 1.7 Hz, ), 7.34 (1H, dd, J = 7.6, 1.5 Hz, ), 7.14 (1H, d, J = 9.2 Hz, ), 6.92 (1H, t, J = 7.6 Hz, ), 5.23-5.20 (2H, m, ), 2.77 (3H, s, ), 2.40 (3H, s, ), 2.19-2.18 (3H, m, ), 1.68 (6H, s, ).
196 ¹H-NMR (CDCl₃) δ: 8.18 (1H, s), 7.37 (1H, d, J = 8.4 Hz), 7.21 (1H, d, J = 8.0 Hz), 7.16 (1H, d, J = 8.4 Hz), 6.89-6.84 (2H, m), 6.75 (1H, br s), 2.96 (1H, d, J = 14.3 Hz), 2.92 (1H, d, J = 14.3 Hz), 2.82 (3H, d, J = 4.6 Hz), 2.64 (3H, s), 2.43 (3H, s), 2.41 (3H, s), 1.68 (3H, s).
197 ¹H-NMR (CDCl₃) δ: 8.19 (1H, s), 7.35 (1H, d, J = 8.4 Hz), 7.12-7.11 (2H, m), 6.82 (1H, br s), 6.78 (1H, d, J = 8.0 Hz), 3.67-3.58 (2H, m), 3.33 (3H, s), 2.63 (3H, s), 2.41 (3H, s), 2.38 (3H, s), 2.29 (2H, t, J = 7.1 Hz), 1.65 (3H, s).
198 ¹H-NMR (CDCl₃) δ: 8.20 (1H, s), 7.95 (1H, d, J = 8.8 Hz), 7.29 (1H, d, J = 8.4 Hz), 7.25 (1H, dd, J = 9.0, 7.1 Hz), 6.99 (1H, d, J = 8.4 Hz), 6.77 (1H, d, J = 6.9 Hz), 2.80 (3H, s), 2.30 (3H, s), 1.68 (6H, s).
199 ¹H-NMR (CDCl₃) δ: 8.19 (1H, s), 7.49 (1H, d, J = 8.2 Hz), 7.18 (1H, d, J = 8.2 Hz), 7.15 (1H, d, J = 8.2 Hz), 6.95 (1H, s), 6.85 (1H, dd, J = 8.2, 0.9 Hz), 3.79 (3H, s), 2.62 (3H, s), 2.41 (3H, s), 2.41 (3H, s), 1.95 (3H, s).
200 ¹H-NMR (CDCl₃) δ: 8.18 (1H, s), 7.53 (1H, d, J = 8.1 Hz), 7.17 (1H, d, J = 8.1 Hz), 7.15 (1H, d, J = 8.1 Hz), 6.96 (1H, br s), 6.84 (1H, dd, J = 8.1, 1.0 Hz), 4.25-4.18 (2H, m), 2.62 (3H, s), 2.42 (3H, s), 2.40 (3H, s), 1.96 (3H, s), 1.25 (3H, t, J = 7.1 Hz).
201 ¹H-NMR (CDCl₃) δ: 8.20 (1H, s), 7.40 (1H, d, J = 8.4 Hz), 7.16-7.10 (2H, m), 6.83 (1H, br s), 6.79 (1H, dd, J = 8.4, 1.0 Hz), 3.06 (2H, q, J = 13.7 Hz), 2.63 (3H, s), 2.41 (3H, s), 2.38 (3H, s), 2.20 (3H, s), 1.75 (3H, s).
202 ¹H-NMR (CDCl₃) δ: 8.15 (1H, s, ), 7.85 (1H, d, J = 9.2 Hz, ), 7.46 (1H, d, J = 8.8 Hz, ), 7.16 (1H, d, J = 9.2 Hz, ), 6.96-6.93 (2H, m, ), 2.77 (3H, s, ), 2.40 (3H, s, ), 1.67 (6H, s, ).
203 ¹H-NMR (CDCl₃) δ: 8.15 (1H, s), 7.84 (1H, d, J = 9.2 Hz), 7.30 (1H, d, J = 8.4 Hz), 7.15 (1H, d, J = 8.8 Hz), 6.98 (1H, d, J = 8.4 Hz), 2.77 (3H, s), 2.40 (3H, s), 2.30 (3H, s), 1.67 (6H, s).
204 ¹H-NMR (CDCl₃) δ: 8.14 (1H, s), 7.86 (1H, d, J = 8.8 Hz), 7.33-7.29 (1H, m), 7.18 (1H, d, J = 8.8 Hz), 6.79-6.72 (1H, m), 2.78 (3H, s), 2.41 (3H, s), 1.73 (6H, s).
205 ¹H-NMR (CDCl₃) δ: 8.21 (1H, s), 7.96 (1H, d, J = 9.5 Hz), 7.33-7.23 (2H, m), 6.82-6.71 (2H, m), 2.80 (3H, s), 1.74 (6H, s).
206 ¹H-NMR (CDCl₃) δ: 8.01 (1H, s, ), 7.93 (1H, d, J = 8.8 Hz, ), 7.48 (1H, dd, J = 6.5, 2.7 Hz, ), 7.21-7.14 (3H, m, ), 2.77 (3H, s, ), 2.41 (3H, s, ), 1.69 (6H, s, ).
207 ¹H-NMR (CDCl₃) δ: 8.04 (1H, s, ), 7.92 (1H, d, J = 8.8 Hz, ), 7.62 (1H, dd, J = 7.8, 1.0 Hz, ), 7.40-7.34 (2H, m, ), 7.20-7.16 (1H, m, ), 7.15 (1H, d, J = 8.8 Hz, ), 2.77 (3H, s, ), 2.40 (3H, s, ), 1.67 (6H, s, ).
208 ¹H-NMR (CDCl₃) δ: 8.09 (1H, s), 8.02 (1H, d, J = 8.8 Hz), 7.62 (1H, dd, J = 7.8, 1.0 Hz), 7.41-7.35 (2H, m), 7.25-7.23 (1H, m), 7.21-7.17 (1H, m), 6.77 (1H, d, J = 6.5 Hz), 2.80 (3H, s), 1.68 (6H, s).
209 ¹H-NMR (CDCl₃) δ: 8.54-8.52 (1H, m), 8.22 (1H, s), 8.08-8.06 (1H, m), 7.52 (1H, dd, J = 7.6, 1.5 Hz), 7.42-7.38 (1H, m), 7.29 (1H, ddd, J = 9.0, 6.7, 1.1 Hz), 6.99 (1H, td, J = 5.5, 1.1 Hz), 6.94 (1H, dd, J = 8.2, 1.0 Hz), 6.90 (1H, td, J = 5.1, 1.5 Hz), 1.68 (6H, s).
210 ¹H-NMR (CDCl₃) δ: 8.31-8.31 (1H, m, ), 8.14 (1H, s, ), 7.95 (1H, d, J = 8.8 Hz, ), 7.40 (1H, d, J = 7.6 Hz, ), 7.12 (1H, dd, J = 9.0, 1.3 Hz, ), 6.79-6.78 (1H, m, ), 6.75 (1H, s, ), 2.38 (3H, s, ), 2.37 (3H, s, ), 1.66 (6H, s, ).
211 ¹H-NMR (CDCl₃) δ: 8.19 (1H, s), 7.46 (1H, d, J = 8.5 Hz), 7.23 (1H, d, J = 7.8 Hz), 7.16 (1H, d, J = 8.5 Hz), 6.91-6.87 (2H, m), 3.70 (1H, d, J = 15.4 Hz), 3.66 (1H, d, J = 15.4 Hz), 3.05 (3H, s), 2.63 (3H, s), 2.42 (6H, s), 1.88 (3H, s).
212 ¹H-NMR (CDCl₃) δ: 8.19 (1H, s), 7.87 (1H, d, J = 9.2 Hz), 7.42 (1H, d, J = 7.6 Hz), 7.15 (1H, d, J = 9.2 Hz), 6.83 (1H, br s), 6.80-6.78 (1H, m), 3.77 (1H, d, J = 10.3 Hz), 3.70 (1H, d, J = 10.3 Hz), 3.49 (3H, s), 2.77 (3H, s), 2.40 (3H, s), 2.36 (3H, s), 1.63 (3H, s).
213 ¹H-NMR (CDCl₃) δ: 8.19 (1H, s), 7.88 (1H, d, J = 9.0 Hz), 7.36 (1H, d, J = 7.4 Hz), 7.24 (1H, d, J = 7.4 Hz), 7.14 (1H, d, J = 9.0 Hz), 6.85 (1H, t, J = 7.4 Hz), 2.77 (3H, s), 2.40 (3H, s), 2.25 (3H, s), 1.97-1.92 (2H, m), 1.60 (3H, s), 1.56-1.50 (2H, m), 1.38-1.31 (2H, m), 0.91 (3H, t, J = 7.3 Hz).
214 ¹H-NMR (CDCl₃) δ: 9.39 (1H, s, ), 8.10 (1H, s, ), 7.63-7.60 (1H, m, ), 7.43-7.37 (2H, m, ), 7.21 (1H, ddd, J = 8.0, 6.9, 2.3 Hz, ), 2.80 (3H, s, ), 2.65 (3H, s, ), 1.68 (6H, s, ).
215 ¹H-NMR (CDCl₃) δ: 8.17-8.14 (1H, m, ), 8.12 (1H, s, ), 7.98 (1H, d, J = 9.2 Hz, ), 7.81-7.79 (1H, m, ), 7.75-7.71 (2H, m, ), 7.21 (1H, d, J = 9.2 Hz, ), 2.77 (3H, s, ), 2.40 (3H, s, ), 1.69 (6H, s, ).
216 ¹H-NMR (CDCl₃) δ: 9.43 (1H, s), 8.19 (1H, s), 8.19-8.16 (1H, m), 7.82-7.75 (3H, m), 2.80 (3H, s), 2.64 (3H, s), 1.70 (6H, s).
217 ¹H-NMR (CDCl₃) δ: 8.18 (1H, s), 7.87 (1H, d, J = 9.2 Hz), 7.36 (1H, d, J = 7.6 Hz), 7.24 (1H, d, J = 7.6 Hz), 7.14 (1H, d, J = 9.2 Hz), 6.85 (1H, t, J = 7.6 Hz), 2.77 (3H, s), 2.40 (3H, s), 2.25 (3H, s), 1.93 (2H, dd, J = 9.2, 7.4 Hz), 1.66-1.60 (2H, m), 1.60 (3H, s), 0.95 (3H, t, J = 7.4 Hz).
218 ¹H-NMR (CDCl₃) δ: 8.17 (1H, s), 7.88 (1H, d, J = 9.2 Hz), 7.21 (1H, dd, J = 8.0, 1.1 Hz), 7.16 (1H, d, J = 8.8 Hz), 6.78-6.73 (1H, m), 2.77 (3H, s), 2.40 (3H, s), 2.33 (3H, d, J = 2.3 Hz), 1.72 (6H, s).
219 ¹H-NMR (CDCl₃) δ: 8.23 (1H, s), 8.00-7.96 (1H, m), 7.28-7.18 (2H, m), 6.80-6.74 (2H, m), 2.80 (3H, s), 2.34 (3H, d, J = 2.2 Hz), 1.72 (6H, s).
220 ¹H-NMR (CDCl₃) δ: 8.05 (1H, s, ), 7.92 (1H, d, J = 8.8 Hz, ), 7.63 (1H, dd, J = 7.8, 1.3 Hz, ), 7.41 (1H, dd, J = 7.8, 1.3 Hz, ), 7.36 (1H, td, J = 7.8, 1.3 Hz, ), 7.21-7.15 (2H, m, ), 3.74 (2H, s, ), 3.46 (3H, s, ), 2.77 (3H, s, ), 2.41 (3H, s, ), 1.63 (3H, s, ).
221 ¹H-NMR (CDCl₃) δ: 8.19-8.16 (1H, m), 8.15 (1H, s), 7.99 (1H, d, J = 8.8 Hz), 7.85-7.81 (1H, m), 7.76-7.71 (2H, m), 7.23 (1H, d, J = 8.8 Hz), 4.12 (1H, d, J = 10.3 Hz), 3.93 (1H, d, J = 10.7 Hz), 3.40 (3H, s), 2.79 (3H, s), 2.43 (3H, s), 1.68 (3H, s).
222 ¹H-NMR (CDCl₃) δ: 8.15 (1H, s), 7.85 (1H, d, J = 8.8 Hz), 7.40-7.38 (1H, m), 7.16 (1H, d, J = 9.2 Hz), 7.11-7.10 (2H, m), 2.77 (3H, s), 2.40 (3H, s), 1.67 (6H, s).
223 ¹H-NMR (CDCl₃) δ: 8.20 (1H, s), 7.88 (1H, d, J = 9.0 Hz), 7.53 (1H, dd, J = 7.7, 1.5 Hz), 7.37 (1H, td, J = 7.7, 1.5 Hz), 7.15 (1H, d, J = 9.0 Hz), 6.95 (1H, td, J = 7.7, 1.0 Hz), 6.91 (1H, dd, J = 7.7, 1.0 Hz), 2.78 (3H, s), 2.40 (3H, s), 1.93 (2H, t, J = 8.3 Hz), 1.69-1.50 (2H, m), 1.61 (3H, s), 0.94 (3H, t, J = 8.3 Hz).
224 ¹H-NMR (CDCl₃) δ: 8.20 (1H, s), 7.89 (1H, d, J = 9.0 Hz), 7.53 (1H, dd, J = 7.8, 1.5 Hz), 7.37 (1H, td, J = 7.8, 1.5 Hz), 7.15 (1H, d, J = 9.0 Hz), 6.95 (1H, td, J = 7.8, 1.1 Hz), 6.91 (1H, dd, J = 7.8, 1.1 Hz), 2.78 (3H, s), 2.40 (3H, s), 1.95 (2H, dd, J = 8.8, 7.4 Hz), 1.61 (3H, s), 1.57-1.52 (2H, m), 1.38-1.30 (2H, m), 0.90 (3H, t, J = 7.4 Hz).
225 ¹H-NMR (CDCl₃) δ: 8.26 (1H, s), 7.98 (1H, d, J = 9.0 Hz), 7.52 (1H, dd, J = 7.7, 1.6 Hz), 7.38 (1H, td, J = 7.7, 1.6 Hz), 7.25 (1H, dd, J = 6.8, 9.0 Hz), 6.96 (1H, td, J = 7.7, 1.1 Hz), 6.92 (1H, dd, J = 7.7, 1.1 Hz), 6.77 (1H, d, J = 6.8 Hz), 2.80 (3H, s), 1.95 (2H, t, J = 8.3 Hz), 1.62 (3H, s), 1.55-1.51 (2H, m), 1.38-1.30 (2H, m), 0.91 (3H, t, J = 7.3 Hz).
226 ¹H-NMR (CDCl₃) δ: 8.33-8.31 (1H, m), 8.16 (1H, s), 7.96 (1H, d, J = 9.0 Hz), 7.52 (1H, dd, J = 7.6, 1.5 Hz), 7.38 (1H, td, J = 8.2, 1.5 Hz), 7.14 (1H, dd, J = 9.0, 1.5 Hz), 6.96 (1H, td, J = 7.6, 1.1 Hz), 6.91 (1H, dd, J = 8.2, 1.1 Hz), 2.38 (3H, d, J = 1.1 Hz), 1.94 (2H, dd, J = 7.4, 7.4 Hz), 1.60 (3H, s), 1.56-1.54 (2H, m), 1.38-1.31 (2H, m), 0.91 (3H, t, J = 7.4 Hz).
227 ¹H-NMR (CDCl₃) δ: 8.22 (1H, s), 7.97 (1H, d, J = 9.2 Hz), 7.29-7.23 (2H, m), 6.77 (1H, d, J = 6.9 Hz), 6.56 (1H, dd, J = 8.8, 7.3 Hz), 3.94 (3H, s), 2.80 (3H, s), 1.73 (6H, s).
228 ¹H-NMR (CDCl₃) δ: 8.16 (1H, s), 7.87 (1H, d, J = 8.8 Hz), 7.28 (1H, dd, J = 8.8, 2.3 Hz), 7.16 (1H, d, J = 9.2 Hz), 6.55 (1H, dd, J = 8.6, 7.4 Hz), 3.95 (3H, s), 2.77 (3H, s), 2.40 (3H, s), 1.72 (6H, s).
229 ¹H-NMR (CDCl₃) δ: 9.32 (1H, s), 8.24 (1H, s), 7.38 (1H, d, J = 7.8 Hz), 6.81-6.79 (1H, m), 6.77-6.77 (1H, m), 2.80 (3H, s), 2.64 (3H, s), 2.37 (3H, s), 1.67 (6H, s).
230 ¹H-NMR (CDCl₃) δ: 8.26 (1H, s), 7.98 (1H, d, J = 9.2 Hz), 7.52 (1H, d, J = 7.6 Hz), 7.38 (1H, t, J = 7.6 Hz), 7.25 (1H, t, J = 8.2 Hz), 6.96 (1H, t, J = 7.4 Hz), 6.92 (1H, d, J = 8.0 Hz), 6.77 (1H, d, J = 6.5 Hz), 2.80 (3H, s), 1.94 (2H, t, J = 8.2 Hz), 1.70-1.52 (2H, m), 1.62 (3H, s), 0.95 (3H, t, J = 7.3 Hz).
231 ¹H-NMR (CDCl₃) δ: 8.32 (1H, s), 8.16 (1H, s), 7.96 (1H, d, J = 8.8 Hz), 7.53 (1H, dd, J = 7.6, 1.4 Hz), 7.38 (1H, td, J = 7.6, 1.4 Hz), 7.14 (1H, dd, J = 8.8, 1.4 Hz), 6.96 (1H, td, J = 7.6, 1.4 Hz), 6.91 (1H, d, J = 7.6 Hz), 2.38 (3H, s), 1.93 (2H, t, J = 8.4 Hz), 1.67-1.53 (2H, m), 1.61 (3H, s), 0.94 (3H, t, J = 7.4 Hz).
232 ¹H-NMR (CDCl₃) δ: 8.15 (1H, s), 7.87 (1H, d, J = 9.2 Hz), 7.23 (1H, dd, J = 8.4, 1.5 Hz), 7.18 (1H, d, J = 9.2 Hz), 7.14 (1H, dd, J = 8.4, 6.1 Hz), 2.78 (3H, s), 2.41 (3H, s), 1.72 (6H, s).
233 ¹H-NMR (CDCl₃) δ: 8.04 (1H, s), 7.94 (1H, d, J = 9.0 Hz), 7.62 (1H, dd, J = 7.8, 1.0 Hz), 7.39-7.32 (2H, m), 7.20-7.13 (2H, m), 2.77 (3H, s), 2.40 (3H, s), 2.00-1.87 (2H, m), 1.65-1.55 (2H, m), 1.61 (3H, s), 1.39-1.28 (2H, m), 0.90 (3H, t, J = 7.3 Hz).
234 ¹H-NMR (CDCl₃) δ: 8.38 (1H, d, J = 6.8 Hz), 7.94 (1H, s), 7.36-7.28 (2H, m), 7.22-7.19 (1H, m), 7.04 (1H, ddd, J = 8.2, 6.3, 1.5 Hz), 6.90 (1H, dt, J = 6.9, 1.0 Hz), 6.74 (1H, t, J = 7.0 Hz), 2.20 (3H, s), 1.98-1.94 (2H, m), 1.61 (3H, s), 1.59-1.50 (2H, m), 1.37-1.30 (2H, m), 0.90 (3H, t, J = 7.3 Hz).
235 ¹H-NMR (DMSO-d₆) δ: 9.33 (1H, s, ), 8.25 (1H, s, ), 7.71-7.68 (1H, m, ), 7.51-7.45 (2H, m, ), 7.32 (1H, ddd, J = 8.2, 6.3, 1.5 Hz, ), 2.76 (3H, s, ), 2.59 (3H, s, ), 1.96-1.84 (2H, m, ), 1.56-1.48 (5H, m, ), 1.34-1.25 (2H, m, ), 0.86 (3H, t, J = 7.3 Hz, ).
236 ¹H-NMR (CDCl₃) δ: 8.30 (1H, t, J = 4.6 Hz, ), 8.03 (1H, s, ), 7.82 (1H, d, J = 4.9 Hz, ), 7.39-7.33 (2H, m, ), 7.15 (1H, d, J = 7.3 Hz, ), 7.10-7.05 (1H, m, ), 2.51 (3H, s, ), 2.01-1.95 (2H, m, ), 1.64-1.51 (5H, m, ), 1.37-1.29 (2H, m, ), 0.91 (3H, t, J = 7.3 Hz, ).
237 ¹H-NMR (CDCl₃) δ: 8.16 (1H, dt, J = 9.5, 3.6 Hz, ), 8.14 (1H, s, ), 8.00 (1H, d, J = 9.2 Hz, ), 7.83 (1H, dt, J = 9.5, 3.6 Hz, ), 7.75 (2H, dt, J = 9.5, 3.6 Hz, ), 7.25 (1H, d, J = 8.8 Hz, ), 2.79 (3H, s, ), 2.43 (3H, s, ), 2.12-1.99 (2H, m, ), 1.76-1.64 (2H, m, ), 1.62 (3H, s, ), 1.42-1.35 (2H, m, ), 0.93 (3H, t, J = 7.3 Hz, ).
238 ¹H-NMR (CDCl₃) δ: 9.45 (1H, s, ), 8.20 (1H, s, ), 8.20-8.18 (1H, m, ), 7.83-7.80 (1H, m, ), 7.80-7.77 (2H, m, ), 2.82 (3H, s, ), 2.67 (3H, s, ), 2.12-2.01 (2H, m, ), 1.76-1.65 (2H, m, ), 1.63 (3H, s, ), 1.42-1.34 (2H, m, ), 0.93 (3H, t, J = 7.4 Hz, ).
239 ¹H-NMR (CDCl₃) δ: 8.20 (1H, s), 7.48 (1H, td, J = 7.6, 1.5 Hz), 7.33 (1H, d, J = 8.4 Hz), 7.24 (1H, dd, J = 7.6, 1.5 Hz), 7.12 (1H, d, J = 8.4 Hz), 7.06 (1H, dd, J = 7.6, 1.5 Hz), 6.98 (1H, td, J = 7.6, 1.5 Hz), 3.78 (1H, d, J = 10.3 Hz), 3.72 (1H, d, J = 10.3 Hz), 3.47 (3H, s), 2.63 (3H, s), 2.41 (3H, s), 1.66 (3H, s).
240 ¹H-NMR (CDCl₃) δ: 8.39 (1H, d, J = 6.8 Hz), 7.99 (1H, s), 7.35 (1H, td, J = 7.6, 1.7 Hz), 7.10 (1H, dd, J = 7.6, 1.7 Hz), 6.94 (1H, dt, J = 7.6, 1.7 Hz), 6.89-6.83 (2H, m), 6.76 (1H, t, J = 7.6 Hz), 2.38 (3H, s), 2.03-1.91 (2H, m), 1.62 (3H, s), 1.55-1.51 (2H, m), 1.39-1.31 (2H, m), 0.91 (3H, t, J = 7.3 Hz).
241 ¹H-NMR (CDCl₃) δ: 9.36 (1H, s), 8.27 (1H, s), 7.52 (1H, dd, J = 7.6, 1.5 Hz), 7.40 (1H, td, J = 7.6, 1.5 Hz), 6.98 (1H, t, J = 7.6 Hz), 6.93 (1H, d, J = 7.6 Hz), 2.81 (3H, s), 2.65 (3H, s), 1.95 (2H, t, J = 8.2 Hz), 1.60 (3H, s), 1.55-1.48 (2H, m), 1.36-1.32 (2H, m), 0.90 (3H, t, J = 7.4 Hz).
242 ¹H-NMR (CDCl₃) δ: 8.31 (1H, d, J = 4.6 Hz), 8.07 (1H, s), 7.84 (1H, d, J = 4.6 Hz), 7.40-7.37 (1H, m), 7.04 (1H, dd, J = 7.6, 1.5 Hz), 6.91 (1H, d, J = 7.6 Hz), 6.87 (1H, t, J = 7.6 Hz), 2.68 (3H, s), 2.03-1.94 (2H, m), 1.64 (3H, s), 1.53-1.48 (2H, m), 1.38-1.33 (2H, m), 0.91 (3H, t, J = 7.3 Hz).
243 ¹H-NMR (CDCl₃) δ: 9.36 (1H, s), 8.27 (1H, s), 7.52 (1H, dd, J = 7.6, 1.5 Hz), 7.40 (1H, td, J = 7.6, 1.5 Hz), 6.98 (1H, td, J = 7.6, 1.5 Hz), 6.93 (1H, dd, J = 7.6, 1.5 Hz), 2.81 (3H, s), 2.65 (3H, s), 1.96-1.92 (2H, m), 1.67-1.52 (2H, m), 1.61 (3H, s), 0.94 (3H, t, J = 7.4 Hz).
244 ¹H-NMR (CDCl₃) δ: 8.52 (1H, dd, J = 2.3, 1.1 Hz), 8.45 (1H, d, J = 2.2 Hz), 8.32 (1H, s), 7.37-7.33 (1H, m), 7.32-7.30 (1H, m), 6.89-6.85 (2H, m), 2.41 (3H, d, J = 1.0 Hz), 1.99-1.95 (2H, m), 1.64 (3H, s), 1.55-1.51 (2H, m), 1.34 (2H, q, J = 7.3 Hz), 0.90 (3H, t, J = 7.3 Hz).
245 ¹H-NMR (CDCl₃) δ: 8.15 (1H, s), 7.84 (1H, d, J = 8.8 Hz), 7.35 (1H, dd, J = 8.6, 6.3 Hz), 7.14 (1H, d, J = 9.2 Hz), 6.64 (1H, t, J = 9.0 Hz), 2.77 (3H, s), 2.40 (3H, s), 2.17 (3H, d, J = 1.9 Hz), 1.67 (6H, s).
246 ¹H-NMR (CDCl₃) δ: 8.15 (1H, s), 7.87 (1H, d, J = 8.8 Hz), 7.29 (1H, dd, J = 8.6, 1.7 Hz), 7.18 (1H, d, J = 9.2 Hz), 6.98 (1H, dd, J = 8.0, 6.1 Hz), 2.78 (3H, s), 2.41 (3H, s), 1.73 (6H, s).
247 ¹H-NMR (CDCl₃) δ: 8.21 (1H, s), 7.96-7.93 (1H, m), 7.34 (1H, dd, J = 8.4, 6.1 Hz), 7.24 (1H, dd, J = 8.8, 6.9 Hz), 6.76 (1H, d, J = 6.9 Hz), 6.65 (1H, t, J = 8.8 Hz), 2.80 (3H, s), 2.17 (3H, d, J = 1.9 Hz), 1.68 (6H, s).
248 ¹H-NMR (CDCl₃) δ: 8.53-8.53 (1H, m, ), 8.46 (1H, d, J = 2.2 Hz, ), 8.32 (1H, s, ), 7.37 (1H, ddd, J = 9.3, 6.1, 1.8 Hz, ), 7.33-7.31 (1H, m, ), 6.91-6.88 (2H, m, ), 2.41 (3H, d, J = 1.0 Hz, ), 1.70 (6H, s, ).
249 ¹H-NMR (CDCl₃) δ: 8.19 (1H, s), 7.48-7.43 (1H, m), 7.33 (1H, d, J = 8.3 Hz), 7.21 (1H, dd, J = 7.8, 1.2 Hz), 7.12 (1H, d, J = 8.3 Hz), 7.00-6.93 (2H, m), 2.63 (3H, s), 2.41 (3H, s), 1.97 (2H, dd, J = 8.8, 7.6 Hz), 1.63 (3H, s), 1.58-1.44 (2H, m), 1.38-1.31 (2H, m), 0.90 (3H, t, J = 7.3 Hz).
250 ¹H-NMR (CDCl₃) δ: 8.20 (1H, s), 7.89 (1H, d, J = 9.0 Hz), 7.53 (1H, dd, J = 7.8, 1.5 Hz), 7.40-7.35 (1H, m), 7.15 (1H, d, J = 9.0 Hz), 6.98-6.90 (2H, m), 2.78 (3H, s), 2.40 (3H, s), 1.99 (2H, q, J = 7.5 Hz), 1.60 (3H, s), 1.08 (3H, t, J = 7.5 Hz).
251 ¹H-NMR (CDCl₃) δ: 8.21 (1H, s), 7.90 (1H, d, J = 9.0 Hz), 7.53 (1H, dd, J = 7.8, 1.5 Hz), 7.37 (1H, td, J = 7.8, 1.5 Hz), 7.14 (1H, d, J = 9.0 Hz), 6.95 (1H, td, J = 7.8, 1.5 Hz), 6.90 (1H, dd, J = 7.8, 1.5 Hz), 2.78 (3H, s), 2.40 (3H, s), 2.08-2.02 (1H, m), 1.86 (2H, d, J = 6.3 Hz), 1.65 (3H, s), 0.99 (6H, d, J = 6.3 Hz).
252 ¹H-NMR (CDCl₃) δ: 8.39 (1H, d, J = 7.1 Hz), 7.99 (1H, s), 7.34 (1H, td, J = 7.7, 1.3 Hz), 7.07 (1H, dd, J = 7.7, 1.3 Hz), 6.94-6.92 (1H, m), 6.88-6.82 (2H, m), 6.76 (1H, t, J = 7.0 Hz), 2.36 (3H, s), 2.07-2.02 (1H, m), 1.94 (1H, dd, J = 14.3, 6.6 Hz), 1.84 (1H, dd, J = 14.3, 5.7 Hz), 1.66 (3H, s), 1.00 (6H, d, J = 6.6 Hz).
253 ¹H-NMR (CDCl₃) δ: 8.26 (1H, s), 7.99 (1H, d, J = 8.9 Hz), 7.52 (1H, dd, J = 7.7, 1.1 Hz), 7.40-7.36 (1H, m), 7.24 (1H, dd, J = 8.9, 7.0 Hz), 6.96 (1H, td, J = 7.7, 1.1 Hz), 6.91 (1H, dd, J = 7.7, 1.1 Hz), 6.76 (1H, d, J = 7.0 Hz), 2.80 (3H, s), 2.09-2.02 (1H, m), 1.87 (2H, dd, J = 6.1, 1.2 Hz), 1.66 (3H, s), 0.99 (6H, d, J = 6.8 Hz).
254 ¹H-NMR (CDCl₃) δ: 8.53 (1H, dt, J = 7.1, 1.2 Hz), 8.23 (1H, s), 8.08 (1H, dt, J = 7.1, 1.2 Hz), 7.52 (1H, dd, J = 7.1, 1.2 Hz), 7.38 (1H, td, J = 7.1, 1.2 Hz), 7.30-7.28 (1H, m), 6.97 (1H, td, J = 7.1, 1.2 Hz), 6.92-6.87 (2H, m), 2.09-2.02 (1H, m), 1.87 (2H, d, J = 6.1 Hz), 1.66 (3H, s), 0.99 (6H, d, J = 6.6 Hz).
255 ¹H-NMR (CDCl₃) δ: 8.32 (1H, s), 8.16 (1H, s), 7.98 (1H, d, J = 9.0 Hz), 7.53 (1H, dd, J = 7.7, 1.6 Hz), 7.37 (1H, td, J = 7.7, 1.6 Hz), 7.13 (1H, dd, J = 9.0, 1.6 Hz), 6.96 (1H, td, J = 7.7, 1.6 Hz), 6.90 (1H, dd, J = 7.7, 1.6 Hz), 2.38 (3H, s), 2.08-2.01 (1H, m), 1.86 (2H, d, J = 6.1 Hz), 1.65 (3H, s), 0.99 (6H, d, J = 6.6 Hz).
256 ¹H-NMR (CDCl₃) δ: 8.18 (1H, s), 7.89 (1H, d, J = 9.0 Hz), 7.22 (1H, dd, J = 7.8, 1.5 Hz), 7.16 (1H, d, J = 9.0 Hz), 6.76-6.72 (1H, m), 2.77 (3H, s), 2.40 (3H, s), 2.32 (3H, d, J = 2.0 Hz), 2.02-1.95 (2H, m), 1.64 (3H, s), 1.61-1.48 (2H, m), 1.41-1.29 (2H, m), 0.91 (3H, t, J = 7.3 Hz).
257 ¹H-NMR (CDCl₃) δ: 8.24 (1H, s), 7.99 (1H, d, J = 8.8 Hz), 7.28-7.23 (1H, m), 7.21 (1H, dd, J = 7.8, 1.0 Hz), 6.79-6.72 (2H, m), 2.80 (3H, s), 2.33 (3H, d, J = 2.3 Hz), 2.02-1.96 (2H, m), 1.65 (3H, s), 1.63-1.49 (2H, m), 1.41-1.31 (2H, m), 0.91 (3H, t, J = 7.4 Hz).
258 ¹H-NMR (CDCl₃) δ: 9.36 (1H, s), 8.25 (1H, s), 7.21 (1H, dd, J = 8.0, 1.1 Hz), 6.79-6.74 (1H, m), 2.80 (3H, s), 2.64 (3H, s), 2.33 (3H, d, J = 2.3 Hz), 2.02-1.95 (2H, m), 1.64 (3H, s), 1.62-1.49 (2H, m), 1.39-1.30 (2H, m), 0.90 (3H, t, J = 7.4 Hz).
259 ¹H-NMR (CDCl₃) δ: 8.30 (1H, d, J = 5.0 Hz), 8.05 (1H, s), 7.84 (1H, d, J = 4.6 Hz), 6.72 (1H, dd, J = 8.0, 0.8 Hz), 6.66 (1H, t, J = 7.4 Hz), 2.68 (3H, s), 2.32 (3H, d, J = 1.9 Hz), 2.06-2.00 (2H, m), 1.68 (3H, s), 1.62-1.46 (2H, m), 1.42-1.33 (2H, m), 0.91 (3H, t, J = 7.3 Hz).
260 ¹H-NMR (CDCl₃) δ: 8.39 (1H, d, J = 6.9 Hz), 7.99 (1H, s), 7.38-7.33 (1H, m), 7.11 (1H, dd, J = 8.0, 1.1 Hz), 6.95 (1H, dt, J = 7.0, 1.1 Hz), 6.90 (1H, dd, J = 8.0, 1.1 Hz), 6.86 (1H, td, J = 8.0, 1.1 Hz), 6.76 (1H, t, J = 7.0 Hz), 2.39 (3H, s), 2.10-1.94 (2H, m), 1.62 (3H, s), 1.07 (3H, t, J = 7.3 Hz).
261 ¹H-NMR (CDCl₃) δ: 8.26 (1H, s), 7.99 (1H, d, J = 8.8 Hz), 7.52 (1H, dd, J = 7.6, 1.5 Hz), 7.38 (1H, td, J = 7.6, 1.5 Hz), 7.25-7.22 (1H, m), 6.96 (1H, td, J = 7.6, 1.5 Hz), 6.93 (1H, d, J = 8.8 Hz), 6.77 (1H, d, J = 7.6 Hz), 2.81 (3H, s), 2.01-1.98 (2H, m), 1.61 (3H, s), 1.09 (3H, t, J = 7.4 Hz).
262 ¹H-NMR (CDCl₃) δ: 8.54-8.52 (1H, m), 8.23 (1H, s), 8.09-8.07 (1H, m), 7.52 (1H, dd, J = 7.6, 1.5 Hz), 7.41-7.37 (1H, m), 7.31-7.27 (1H, m), 6.99-6.88 (3H, m), 2.02-1.97 (2H, m), 1.60 (3H, s), 1.09 (3H, t, J = 7.4 Hz).
263 ¹H-NMR (CDCl₃) δ: 8.04 (1H, s), 7.94 (1H, d, J = 8.8 Hz), 7.62 (1H, dd, J = 7.8, 1.0 Hz), 7.39-7.33 (2H, m), 7.20-7.14 (2H, m), 2.77 (3H, s), 2.40 (3H, s), 2.03-1.90 (2H, m), 1.59 (3H, s), 1.11 (3H, t, J = 7.4 Hz).
264 ¹H-NMR (CDCl₃) δ: 8.38 (1H, d, J = 6.9 Hz), 7.93 (1H, s), 7.35-7.29 (2H, m), 7.21 (1H, dd, J = 8.0, 1.1 Hz), 7.07-7.02 (1H, m), 6.92-6.89 (1H, m), 6.74 (1H, t, J = 6.9 Hz), 2.21 (3H, s), 2.03-1.94 (2H, m), 1.62 (3H, s), 1.09 (3H, t, J = 7.4 Hz).
265 ¹H-NMR (CDCl₃) δ: 8.10 (1H, s), 8.06-8.02 (1H, m), 7.63-7.60 (1H, m), 7.40-7.32 (2H, m), 7.27-7.21 (1H, m), 7.20-7.14 (1H, m), 6.75 (1H, d, J = 6.8 Hz), 2.79 (3H, s), 2.04-1.91 (2H, m), 1.59 (3H, s), 1.12 (3H, t, J = 7.3 Hz).
266 ¹H-NMR (CDCl₃) δ: 8.52 (1H, dt, J = 7.0, 1.0 Hz), 8.13 (1H, dt, J = 9.0, 1.2 Hz), 8.07 (1H, s), 7.63-7.61 (1H, m), 7.40-7.34 (2H, m), 7.29 (1H, ddd, J = 8.9, 6.7, 1.1 Hz), 7.19 (1H, ddd, J = 8.2, 6.5, 1.3 Hz), 6.89 (1H, td, J = 6.8, 1.5 Hz), 2.04-1.89 (2H, m), 1.59 (3H, s), 1.12 (3H, t, J = 7.4 Hz).
267 ¹H-NMR (CDCl₃) δ: 8.52-8.51 (1H, m), 8.44 (1H, d, J = 2.3 Hz), 8.31 (1H, s), 7.20-7.18 (1H, m), 6.69-6.67 (2H, m), 2.40 (3H, d, J = 1.1 Hz), 2.33 (3H, s), 2.09-2.01 (1H, m), 1.92-1.84 (2H, m), 1.66 (3H, s), 0.99 (6H, d, J = 6.5 Hz).
268 ¹H-NMR (CDCl₃) δ: 9.37 (1H, s), 8.27 (1H, s), 7.53 (1H, dd, J = 7.8, 1.7 Hz), 7.40 (1H, td, J = 7.8, 1.7 Hz), 6.98 (1H, td, J = 7.8, 1.7 Hz), 6.92 (1H, dd, J = 7.8, 1.7 Hz), 2.81 (3H, s), 2.64 (3H, s), 2.08-2.01 (1H, m), 1.88 (2H, d, J = 6.1 Hz), 1.64 (3H, s), 0.99 (6H, d, J = 6.5 Hz).
269 ¹H-NMR (CDCl₃) δ: 9.36 (1H, s), 8.27 (1H, s), 7.52 (1H, dd, J = 7.6, 1.5 Hz), 7.40 (1H, td, J = 7.6, 1.5 Hz), 6.98 (1H, td, J = 7.6, 1.0 Hz), 6.94 (1H, dd, J = 7.6, 1.0 Hz), 2.81 (3H, s), 2.64 (3H, s), 1.99 (2H, q, J = 7.5 Hz), 1.59 (3H, s), 1.08 (3H, t, J = 7.5 Hz).
270 ¹H-NMR (CDCl₃) δ: 8.33-8.32 (1H, m), 8.16 (1H, s), 7.98 (1H, d, J = 9.0 Hz), 7.53 (1H, dd, J = 7.8, 1.2 Hz), 7.38 (1H, td, J = 7.8, 1.2 Hz), 7.14 (1H, dd, J = 9.0, 1.2 Hz), 6.96 (1H, td, J = 7.8, 1.2 Hz), 6.92 (1H, dd, J = 7.8, 1.2 Hz), 2.38 (3H, s), 1.98 (2H, q, J = 7.4 Hz), 1.60 (3H, s), 1.08 (3H, t, J = 7.4 Hz).
271 ¹H-NMR (CDCl₃) δ: 8.18-8.15 (1H, m), 8.14 (1H, s), 8.00 (1H, d, J = 8.8 Hz), 7.85-7.82 (1H, m), 7.77-7.72 (2H, m), 7.24 (1H, d, J = 8.8 Hz), 2.79 (3H, s), 2.43 (3H, s), 2.13 (2H, q, J = 7.4 Hz), 1.61 (3H, s), 1.24 (3H, t, J = 7.6 Hz).
272 ¹H-NMR (CDCl₃) δ: 9.42 (1H, s), 8.10 (1H, s), 7.63-7.61 (1H, m), 7.42-7.36 (2H, m), 7.22-7.19 (1H, m), 2.80 (3H, s), 2.65 (3H, s), 2.03-1.91 (2H, m), 1.58 (3H, s), 1.11 (3H, t, J = 7.4 Hz).
273 ¹H-NMR (CDCl₃) δ: 8.29 (1H, dd, J = 4.8, 4.0 Hz), 8.01 (1H, s), 7.81 (1H, d, J = 5.0 Hz), 7.38-7.33 (2H, m), 7.15 (1H, dd, J = 7.8, 1.0 Hz), 7.09-7.04 (1H, m), 2.51 (3H, s), 2.05-1.97 (2H, m), 1.64 (3H, s), 1.10 (3H, t, J = 7.4 Hz).
274 ¹H-NMR (CDCl₃) δ: 8.20 (1H, s), 8.19-8.15 (1H, m), 8.10 (1H, d, J = 8.5 Hz), 7.85-7.79 (1H, m), 7.79-7.73 (2H, m), 7.34 (1H, dd, J = 8.9, 7.0 Hz), 6.84 (1H, d, J = 6.8 Hz), 2.82 (3H, s), 2.13 (2H, q, J = 7.8 Hz), 1.62 (3H, s), 1.24 (3H, t, J = 7.8 Hz).
275 ¹H-NMR (CDCl₃) δ: 8.38 (1H, d, J = 6.8 Hz), 7.98 (1H, s), 7.04 (1H, d, J = 7.6 Hz), 6.95 (1H, dt, J = 6.9, 1.1 Hz), 6.76 (1H, t, J = 7.0 Hz), 6.72 (1H, s), 6.69 (1H, d, J = 7.8 Hz), 2.41 (3H, s), 2.35 (3H, s), 1.69 (6H, s).
276 ¹H-NMR (CDCl₃) δ: 9.46 (1H, s), 8.21 (1H, s), 8.20-8.16 (1H, m), 7.85-7.76 (3H, m), 2.83 (3H, s), 2.67 (3H, s), 2.14 (2H, q, J = 7.5 Hz), 1.62 (3H, s), 1.24 (3H, t, J = 7.4 Hz).
277 ¹H-NMR (CDCl₃) δ: 8.32 (1H, d, J = 1.1 Hz), 8.02 (1H, d, J = 9.2 Hz), 8.00 (1H, s), 7.62 (1H, dd, J = 7.8, 1.0 Hz), 7.39-7.33 (2H, m), 7.21-7.17 (1H, m), 7.14 (1H, dd, J = 9.2, 1.5 Hz), 2.38 (3H, s), 2.03-1.90 (2H, m), 1.58 (3H, s), 1.11 (3H, t, J = 7.3 Hz).
278 ¹H-NMR (CDCl₃) δ: 8.03 (1H, s), 7.94 (1H, d, J = 8.8 Hz), 7.63-7.61 (1H, m), 7.39-7.33 (2H, m), 7.19-7.15 (2H, m), 2.77 (3H, s), 2.41 (3H, s), 1.98-1.85 (2H, m), 1.69-1.60 (2H, m), 1.59 (3H, s), 0.94 (3H, t, J = 7.3 Hz).
279 ¹H-NMR (CDCl₃) δ: 8.38 (1H, d, J = 6.9 Hz), 7.94 (1H, s), 7.34-7.29 (2H, m), 7.21 (1H, dd, J = 7.8, 1.0 Hz), 7.06-7.02 (1H, m), 6.92-6.88 (1H, m), 6.74 (1H, t, J = 6.9 Hz), 2.20 (3H, s), 1.97-1.92 (2H, m), 1.61 (3H, s), 1.60-1.55 (2H, m), 0.94 (3H, t, J = 7.4 Hz).
280 ¹H-NMR (CDCl₃) δ: 8.19 (1H, s), 7.90 (1H, d, J = 9.2 Hz), 7.36-7.32 (1H, m), 7.21-7.16 (2H, m), 6.89 (1H, td, J = 7.8, 5.0 Hz), 2.78 (3H, s), 2.41 (3H, s), 1.99 (2H, t, J = 8.4 Hz), 1.65 (3H, s), 1.63-1.50 (2H, m), 1.39-1.31 (2H, m), 0.91 (3H, t, J = 7.3 Hz).
281 ¹H-NMR (CDCl₃) δ: 8.40 (1H, d, J = 6.9 Hz), 7.99 (1H, s), 7.19-7.15 (1H, m), 6.98-6.95 (1H, m), 6.93-6.89 (1H, m), 6.80-6.76 (2H, m), 2.39 (3H, s), 2.05-1.99 (2H, m), 1.67 (3H, s), 1.62-1.49 (2H, m), 1.38-1.33 (2H, m), 0.91 (3H, t, J = 7.3 Hz).
282 ¹H-NMR (CDCl₃) δ: 8.19 (1H, s), 7.90 (1H, d, J = 8.8 Hz), 7.37-7.35 (1H, m), 7.22-7.17 (2H, m), 6.92 (1H, td, J = 7.8, 5.0 Hz), 3.79 (1H, d, J = 10.7 Hz), 3.76 (1H, d, J = 10.7 Hz), 3.49 (3H, s), 2.78 (3H, s), 2.41 (3H, s), 1.67 (3H, s).
283 ¹H-NMR (CDCl₃) δ: 8.40 (1H, d, J = 6.9 Hz), 8.00 (1H, s), 7.20-7.16 (1H, m), 7.00-6.97 (2H, m), 6.84-6.77 (2H, m), 3.80 (1H, d, J = 10.7 Hz), 3.77 (1H, d, J = 10.7 Hz), 3.47 (3H, s), 2.42 (3H, s), 1.69 (3H, s).
284 ¹H-NMR (CDCl₃) δ: 8.20 (1H, s), 7.88 (1H, d, J = 9.2 Hz), 7.55 (1H, dd, J = 7.6, 1.5 Hz), 7.40 (1H, td, J = 7.6, 1.5 Hz), 7.16 (1H, d, J = 9.2 Hz), 7.02-6.97 (2H, m), 3.78 (1H, d, J = 10.3 Hz), 3.71 (1H, d, J = 10.3 Hz), 3.49 (3H, s), 2.78 (3H, s), 2.41 (3H, s), 1.64 (3H, s).
285 ¹H-NMR (CDCl₃) δ: 8.39 (1H, d, J = 6.9 Hz), 8.00 (1H, s), 7.38 (1H, td, J = 7.8, 1.7 Hz), 7.18 (1H, dd, J = 7.8, 1.7 Hz), 6.98-6.96 (2H, m), 6.89 (1H, td, J = 7.8, 1.1 Hz), 6.78 (1H, t, J = 6.9 Hz), 3.80 (1H, d, J = 10.3 Hz), 3.72 (1H, d, J = 10.3 Hz), 3.47 (3H, s), 2.41 (3H, s), 1.65 (3H, s).
286 ¹H-NMR (CDCl₃) δ: 8.25 (1H, s), 8.00 (1H, d, J = 8.8 Hz), 7.34-7.32 (1H, m), 7.28-7.25 (1H, m), 7.21-7.17 (1H, m), 6.90 (1H, td, J = 7.8, 4.6 Hz), 6.79 (1H, d, J = 6.9 Hz), 2.81 (3H, s), 1.99 (2H, t, J = 8.4 Hz), 1.67 (3H, s), 1.63-1.51 (2H, m), 1.39-1.31 (2H, m), 0.91 (3H, t, J = 7.3 Hz).
287 ¹H-NMR (CDCl₃) δ: 8.54 (1H, d, J = 8.0 Hz), 8.22 (1H, s), 8.09 (1H, d, J = 9.2 Hz), 7.35-7.29 (2H, m), 7.22-7.18 (1H, m), 6.94-6.88 (2H, m), 1.99 (2H, t, J = 8.4 Hz), 1.66 (3H, s), 1.63-1.50 (2H, m), 1.39-1.33 (2H, m), 0.91 (3H, t, J = 7.4 Hz).
288 ¹H-NMR (CDCl₃) δ: 8.25 (1H, s), 8.00 (1H, d, J = 8.8 Hz), 7.35 (1H, d, J = 7.6 Hz), 7.29-7.26 (1H, m), 7.23-7.19 (1H, m), 6.92 (1H, td, J = 7.6, 4.6 Hz), 6.79 (1H, d, J = 7.6 Hz), 3.79 (1H, d, J = 10.7 Hz), 3.77 (1H, d, J = 10.7 Hz), 3.49 (3H, s), 2.81 (3H, s), 1.68 (3H, s).
289 ¹H-NMR (CDCl₃) δ: 8.54 (1H, d, J = 6.9 Hz), 8.22 (1H, s), 8.09 (1H, d, J = 8.8 Hz), 7.36-7.34 (1H, m), 7.34-7.30 (1H, m), 7.24-7.19 (1H, m), 6.95-6.91 (2H, m), 3.79 (1H, d, J = 10.7 Hz), 3.77 (1H, d, J = 10.7 Hz), 3.49 (3H, s), 1.68 (3H, s).
290 ¹H-NMR (CDCl₃) δ: 8.26 (1H, s), 7.99 (1H, d, J = 8.4 Hz), 7.54 (1H, dd, J = 7.8, 1.7 Hz), 7.42-7.39 (1H, m), 7.28-7.24 (1H, m), 7.03-6.97 (2H, m), 6.78 (1H, d, J = 6.9 Hz), 3.79 (1H, d, J = 10.3 Hz), 3.72 (1H, d, J = 10.3 Hz), 3.49 (3H, s), 2.81 (3H, s), 1.64 (3H, s).
291 ¹H-NMR (CDCl₃) δ: 8.53 (1H, d, J = 7.6 Hz), 8.23 (1H, s), 8.08 (1H, d, J = 8.8 Hz), 7.54 (1H, dd, J = 7.6, 1.5 Hz), 7.43-7.39 (1H, m), 7.32-7.28 (1H, m), 7.02-6.98 (2H, m), 6.92-6.89 (1H, m), 3.78 (1H, d, J = 10.3 Hz), 3.72 (1H, d, J = 10.3 Hz), 3.49 (3H, s), 1.64 (3H, s).
292 ¹H-NMR (CDCl₃) δ: 9.21 (1H, d, J = 7.1 Hz), 7.98 (1H, s), 7.65 (1H, dd, J = 7.8, 1.5 Hz), 7.43-7.38 (1H, m), 7.03-6.99 (1H, m), 6.93 (1H, dd, J = 8.1, 1.2 Hz), 6.78 (1H, d, J = 7.1 Hz), 2.61 (3H, s), 2.40 (3H, s), 1.97-1.92 (2H, m), 1.59-1.59 (2H, m), 1.59 (3H, s), 1.38-1.29 (2H, m), 0.90 (3H, t, J = 7.3 Hz).
293 ¹H-NMR (CDCl₃) δ: 8.48 (1H, d, J = 4.2 Hz), 8.40 (1H, s), 7.35 (1H, td, J = 5.7, 1.7 Hz), 7.33-7.30 (1H, m), 6.89-6.85 (2H, m), 6.78 (1H, d, J = 4.2 Hz), 2.85 (3H, d, J = 0.7 Hz), 2.00-1.96 (2H, m), 1.64 (3H, s), 1.59-1.53 (2H, m), 1.37-1.31 (2H, m), 0.90 (3H, t, J = 7.3 Hz).
294 ¹H-NMR (CDCl₃) δ: 9.24 (1H, d, J = 6.9 Hz), 7.99 (1H, s), 7.66 (1H, dd, J = 7.6, 1.5 Hz), 7.43-7.40 (1H, m), 7.03 (1H, td, J = 5.5, 1.1 Hz), 6.96-6.93 (1H, m), 6.78 (1H, d, J = 7.3 Hz), 3.68-3.61 (2H, m), 3.34 (3H, s), 2.61 (3H, s), 2.40 (3H, s), 2.29 (2H, t, J = 7.3 Hz), 1.62 (3H, s).
295 ¹H-NMR (CDCl₃) δ: 9.24 (1H, d, J = 6.9 Hz), 7.99 (1H, s), 7.66 (1H, dd, J = 7.6, 1.5 Hz), 7.43-7.40 (1H, m), 7.03 (1H, td, J = 5.5, 1.1 Hz), 6.96-6.93 (1H, m), 6.78 (1H, d, J = 7.3 Hz), 3.68-3.61 (2H, m), 3.34 (3H, d, J = 1.5 Hz), 2.61 (3H, s), 2.40 (3H, s), 2.29 (3H, t, J = 7.3 Hz), 1.62 (3H, s).
296 ¹H-NMR (CDCl₃) δ: 8.52-8.50 (1H, m), 8.41 (1H, d, J = 2.3 Hz), 8.28 (1H, s), 7.23 (1H, dd, J = 7.6, 1.1 Hz), 7.16 (1H, td, J = 6.0, 1.1 Hz), 7.10-7.05 (1H, m), 2.40 (3H, d, J = 1.1 Hz), 1.71 (6H, s).
297 ¹H-NMR (CDCl₃) δ: 8.53-8.52 (1H, m), 8.45 (1H, d, J = 2.2 Hz), 8.32 (1H, s), 7.37-7.30 (2H, m), 6.88-6.85 (2H, m), 2.41 (3H, d, J = 1.0 Hz), 1.98-1.93 (2H, m), 1.64 (3H, s), 1.62-1.52 (2H, m), 0.94 (3H, t, J = 7.3 Hz).
298 ¹H-NMR (CDCl₃) δ: 8.09 (1H, s), 8.03 (1H, d, J = 8.8 Hz), 7.61 (1H, dd, J = 7.6, 1.1 Hz), 7.39-7.34 (2H, m), 7.27-7.24 (1H, m), 7.20-7.16 (1H, m), 6.77 (1H, d, J = 6.9 Hz), 2.80 (3H, s), 1.99-1.86 (2H, m), 1.69-1.61 (2H, m), 1.60 (3H, s), 0.94 (3H, t, J = 7.4 Hz).
299 ¹H-NMR (CDCl₃) δ: 8.53-8.50 (1H, m), 8.14-8.10 (1H, m), 8.06 (1H, s), 7.61 (1H, dd, J = 7.6, 0.8 Hz), 7.39-7.34 (2H, m), 7.32-7.27 (1H, m), 7.22-7.17 (1H, m), 6.89 (1H, td, J = 6.9, 1.5 Hz), 2.00-1.84 (2H, m), 1.67-1.61 (2H, m), 1.60 (3H, s), 0.94 (3H, t, J = 7.3 Hz).
300 ¹H-NMR (CDCl₃) δ: 8.36 (1H, s), 8.18-8.14 (1H, m), 8.10 (1H, s), 8.09 (1H, d, J = 9.2 Hz), 7.85-7.81 (1H, m), 7.78-7.73 (2H, m), 7.24 (1H, dd, J = 9.0, 1.3 Hz), 2.41 (3H, s), 2.12 (2H, q, J = 7.6 Hz), 1.61 (3H, s), 1.23 (3H, t, J = 7.6 Hz).
301 ¹H-NMR (CDCl₃) δ: 8.18-8.14 (1H, m), 8.13 (1H, s), 7.99 (1H, d, J = 9.2 Hz), 7.85-7.81 (1H, m), 7.77-7.73 (2H, m), 7.25 (1H, d, J = 9.2 Hz), 2.79 (3H, s), 2.42 (3H, s), 2.11-1.96 (2H, m), 1.80-1.69 (2H, m), 1.63 (3H, s), 1.00 (3H, t, J = 7.3 Hz).
302 ¹H-NMR (CDCl₃) δ: 8.31 (1H, br s), 8.02 (1H, d, J = 8.8 Hz), 7.99 (1H, s), 7.62 (1H, dd, J = 7.8, 1.0 Hz), 7.39-7.33 (2H, m), 7.21-7.16 (1H, m), 7.15 (1H, dd, J = 9.2, 1.5 Hz), 2.38 (3H, d, J = 0.8 Hz), 1.98-1.85 (2H, m), 1.67-1.60 (2H, m), 1.59 (3H, s), 0.94 (3H, t, J = 7.4 Hz).
303 ¹H-NMR (CDCl₃) δ: 8.38 (1H, s), 8.33 (1H, s), 7.36-7.30 (2H, m), 6.88-6.84 (2H, m), 2.81 (3H, s), 2.40 (3H, s), 1.98-1.93 (2H, m), 1.64 (3H, s), 1.63-1.52 (2H, m), 0.93 (3H, t, J = 7.3 Hz).
304 ¹H-NMR (CDCl₃) δ: 9.42 (1H, s), 8.10 (1H, s), 7.62 (1H, d, J = 7.6 Hz), 7.42-7.35 (2H, m), 7.23-7.18 (1H, m), 2.80 (3H, s), 2.65 (3H, s), 1.99-1.86 (2H, m), 1.68-1.60 (2H, m), 1.58 (3H, s), 0.93 (3H, t, J = 7.3 Hz).
305 ¹H-NMR (CDCl₃) δ: 8.29 (1H, d, J = 4.6 Hz), 8.01 (1H, s), 7.81 (1H, d, J = 4.6 Hz), 7.38-7.32 (2H, m), 7.15 (1H, d, J = 8.0 Hz), 7.09-7.04 (1H, m), 2.51 (3H, s), 1.99-1.91 (2H, m), 1.63 (3H, s), 1.62-1.55 (2H, m), 0.95 (3H, t, J = 7.3 Hz).
306 ¹H-NMR (CDCl₃) δ: 8.19 (1H, s), 8.17 (1H, dd, J = 5.7, 3.4 Hz), 8.08 (1H, d, J = 8.8 Hz), 7.84-7.79 (1H, m), 7.78-7.74 (2H, m), 7.34 (1H, dd, J = 8.8, 6.9 Hz), 6.84 (1H, d, J = 6.9 Hz), 2.82 (3H, s), 2.12-1.98 (2H, m), 1.82-1.67 (2H, m), 1.64 (3H, s), 1.00 (3H, t, J = 7.3 Hz).
307 ¹H-NMR (CDCl₃) δ: 8.36 (1H, br s), 8.18-8.14 (1H, m), 8.10 (1H, s), 8.07 (1H, d, J = 9.2 Hz), 7.85-7.81 (1H, m), 7.78-7.73 (2H, m), 7.24 (1H, dd, J = 9.2, 1.5 Hz), 2.41 (3H, s), 2.09-1.98 (2H, m), 1.79-1.69 (2H, m), 1.63 (3H, s), 1.00 (3H, t, J = 7.3 Hz).
308 ¹H-NMR (CDCl₃) δ: 8.53-8.52 (1H, m), 8.45 (1H, d, J = 2.3 Hz), 8.32 (1H, s), 7.36-7.31 (2H, m), 6.88-6.86 (2H, m), 2.41 (3H, d, J = 1.1 Hz), 2.09-2.02 (1H, m), 1.93-1.85 (2H, m), 1.68 (3H, s), 0.99 (6H, dd, J = 6.5, 2.3 Hz).
309 ¹H-NMR (CDCl₃) δ: 8.38 (1H, s), 8.34 (1H, s), 7.36-7.31 (2H, m), 6.88-6.85 (2H, m), 2.81 (3H, s), 2.40 (3H, s), 2.08-2.02 (1H, m), 1.93-1.85 (2H, m), 1.68 (3H, s), 0.99 (6H, dd, J = 6.5, 2.7 Hz).
310 ¹H-NMR (CDCl₃) δ: 8.36 (1H, dd, J = 4.4, 1.7 Hz), 8.05-8.02 (1H, m), 8.01 (1H, s), 7.39-7.35 (1H, m), 7.11 (1H, dd, J = 9.2, 4.6 Hz), 7.01 (1H, dd, J = 7.6, 1.5 Hz), 6.90 (1H, dd, J = 8.2, 1.0 Hz), 6.86-6.83 (1H, m), 2.03-1.98 (2H, m), 1.68 (3H, s), 1.63-1.49 (2H, m), 1.39-1.31 (2H, m), 0.91 (3H, t, J = 7.4 Hz).
311 ¹H-NMR (CDCl₃) δ: 8.02 (1H, s), 7.96 (1H, d, J = 9.0 Hz), 7.52-7.43 (1H, m), 7.19-7.12 (3H, m), 2.77 (3H, s), 2.41 (3H, s), 2.05-1.92 (2H, m), 1.59 (3H, s), 1.13 (3H, t, J = 7.4 Hz).
312 ¹H-NMR (CDCl₃) δ: 8.38 (1H, d, J = 6.8 Hz), 7.92 (1H, s), 7.16-7.11 (1H, m), 7.09-6.99 (2H, m), 6.93 (1H, dt, J = 6.8, 1.0 Hz), 6.75 (1H, t, J = 6.8 Hz), 2.22 (3H, s), 2.02 (2H, q, J = 7.4 Hz), 1.63 (3H, s), 1.11 (3H, t, J = 7.4 Hz).
313 ¹H-NMR (CDCl₃) δ: 8.08 (1H, s), 8.05 (1H, d, J = 9.0 Hz), 7.49-7.44 (1H, m), 7.29-7.25 (1H, m), 7.20-7.13 (2H, m), 6.78 (1H, d, J = 6.8 Hz), 2.80 (3H, s), 2.07-1.93 (2H, m), 1.61 (3H, s), 1.14 (3H, t, J = 7.4 Hz).
314 ¹H-NMR (CDCl₃) δ: 8.51-8.51 (1H, m), 8.40 (1H, d, J = 2.3 Hz), 8.29 (1H, s), 7.39 (1H, dd, J = 8.6, 5.9 Hz), 7.07 (1H, dd, J = 8.6, 2.5 Hz), 6.78 (1H, td, J = 6.5, 2.7 Hz), 2.40 (3H, d, J = 1.1 Hz), 1.69 (6H, s).
315 ¹H-NMR (CDCl₃) δ: 8.33 (1H, d, J = 1.1 Hz), 8.16 (1H, s), 7.97 (1H, d, J = 9.2 Hz), 7.55 (1H, dd, J = 7.6, 1.5 Hz), 7.42-7.38 (1H, m), 7.15 (1H, dd, J = 8.8, 1.5 Hz), 7.02-6.97 (2H, m), 3.78 (1H, d, J = 10.3 Hz), 3.71 (1H, d, J = 10.3 Hz), 3.49 (3H, s), 2.39 (3H, s), 1.63 (3H, s).
316 ¹H-NMR (CDCl₃) δ: 8.33 (1H, br s), 8.15 (1H, s), 7.98 (1H, d, J = 9.2 Hz), 7.34 (1H, dt, J = 7.8, 1.2 Hz), 7.22-7.15 (2H, m), 6.90 (1H, td, J = 7.8, 5.0 Hz), 2.39 (3H, s), 1.98 (2H, t, J = 8.4 Hz), 1.65 (3H, s), 1.62-1.52 (2H, m), 1.38-1.33 (2H, m), 0.91 (3H, t, J = 7.4 Hz).
317 ¹H-NMR (CDCl₃) δ: 9.45 (1H, s), 8.20 (1H, s), 8.20-8.16 (1H, m), 7.84-7.77 (3H, m), 2.82 (3H, s), 2.67 (3H, s), 2.11-2.03 (2H, m), 1.82-1.70 (2H, m), 1.63 (3H, s), 1.00 (3H, t, J = 7.4 Hz).
318 ¹H-NMR (CDCl₃) δ: 8.32 (1H, s), 8.03 (1H, d, J = 8.8 Hz), 7.98 (1H, s), 7.50-7.45 (1H, m), 7.19-7.13 (3H, m), 2.38 (3H, s), 2.05-1.93 (2H, m), 1.59 (3H, s), 1.13 (3H, t, J = 7.3 Hz).
319 ¹H-NMR (CDCl₃) δ: 9.43 (1H, s), 8.09 (1H, s), 7.51-7.45 (1H, m), 7.22-7.18 (2H, m), 2.80 (3H, s), 2.65 (3H, s), 2.07-1.93 (2H, m), 1.59 (3H, s), 1.13 (3H, t, J = 7.3 Hz).
320 ¹H-NMR (CDCl₃) δ: 9.37 (1H, s), 8.26 (1H, s), 7.34-7.32 (1H, m), 7.26-7.18 (1H, m), 6.95-6.91 (1H, m), 2.81 (3H, s), 2.65 (3H, s), 2.00-1.98 (2H, m), 1.65 (3H, s), 1.62-1.50 (2H, m), 1.37-1.33 (2H, m), 0.90 (3H, t, J = 7.3 Hz).
321 ¹H-NMR (CDCl₃) δ: 8.34 (1H, s), 8.30 (1H, s), 7.23 (1H, dd, J = 8.0, 1.1 Hz), 7.17-7.14 (1H, m), 7.06 (1H, td, J = 7.3, 5.7 Hz), 2.80 (3H, s), 2.39 (3H, s), 1.71 (6H, s).
322 ¹H-NMR (CDCl₃) δ: 8.33 (1H, s), 8.31 (1H, s), 7.41-7.38 (1H, m), 7.06 (1H, dd, J = 8.8, 2.7 Hz), 6.76 (1H, td, J = 6.5, 2.7 Hz), 2.80 (3H, s), 2.39 (3H, s), 1.69 (6H, s).
323 ¹H-NMR (CDCl₃) δ: 8.53-8.52 (1H, m), 8.45 (1H, d, J = 1.9 Hz), 8.32 (1H, s), 7.37-7.34 (1H, m), 7.31 (1H, dd, J = 7.6, 1.5 Hz), 6.89-6.86 (2H, m), 2.41 (3H, d, J = 0.8 Hz), 2.04-1.98 (2H, m), 1.63 (3H, s), 1.07 (3H, t, J = 7.4 Hz).
324 ¹H-NMR (CDCl₃) δ: 8.52-8.51 (1H, m), 8.44 (1H, d, J = 2.3 Hz), 8.31 (1H, s), 7.18 (1H, d, J = 7.6 Hz), 6.69-6.67 (2H, m), 2.40 (3H, d, J = 0.8 Hz), 2.33 (3H, s), 1.98-1.89 (2H, m), 1.62 (3H, s), 1.61-1.49 (2H, m), 0.94 (3H, t, J = 7.4 Hz).
325 ¹H-NMR (CDCl₃) δ: 8.07 (1H, s), 8.04 (1H, d, J = 9.2 Hz), 7.49-7.44 (1H, m), 7.29-7.26 (1H, m), 7.21-7.14 (2H, m), 6.78 (1H, d, J = 6.9 Hz), 2.80 (3H, s), 1.70 (6H, s).
326 ¹H-NMR (CDCl₃) δ: 8.32 (1H, d, J = 1.0 Hz, ), 8.02 (1H, d, J = 9.0 Hz, ), 7.98 (1H, s, ), 7.51-7.44 (1H, m, ), 7.21-7.12 (3H, m, ), 2.39 (3H, s, ), 1.69 (6H, s, ).
327 ¹H-NMR (CDCl₃) δ: 8.39 (1H, d, J = 6.8 Hz), 7.92 (1H, s), 7.18-7.11 (2H, m), 7.07-7.02 (1H, m), 6.95 (1H, dt, J = 7.0, 1.1 Hz), 6.77 (1H, t, J = 7.0 Hz), 2.25 (3H, s), 1.72 (6H, s).
328 ¹H-NMR (CDCl₃) δ: 9.40 (1H, s), 8.09 (1H, s), 7.49-7.45 (1H, m), 7.23-7.17 (2H, m), 2.80 (3H, s), 2.65 (3H, s), 1.69 (6H, s).
329 ¹H-NMR (CDCl₃) δ: 8.30 (1H, d, J = 4.6 Hz), 7.99 (1H, s), 7.84 (1H, d, J = 4.6 Hz), 7.21-7.16 (1H, m), 7.10-7.04 (2H, m), 2.55 (3H, s), 1.74 (6H, s).
330 ¹H-NMR (CDCl₃) δ: 8.20 (1H, s), 7.89 (1H, d, J = 9.0 Hz), 7.53 (1H, dd, J = 7.6, 1.4 Hz), 7.37 (1H, td, J = 7.6, 1.4 Hz), 7.15 (1H, d, J = 9.0 Hz), 6.95 (1H, td, J = 7.6, 1.0 Hz), 6.91 (1H, dd, J = 7.6, 1.0 Hz), 2.78 (3H, s), 2.40 (3H, s), 1.95-1.93 (2H, m), 1.61 (3H, s), 1.60-1.52 (2H, m), 1.34-1.27 (4H, m), 0.87 (3H, t, J = 7.1 Hz).
331 ¹H-NMR (CDCl₃) δ: 8.39 (1H, d, J = 7.3 Hz), 7.99 (1H, s), 7.37-7.33 (1H, m), 7.10 (1H, dd, J = 7.6, 1.1 Hz), 6.94 (1H, dt, J = 7.3, 1.1 Hz), 6.88 (1H, dd, J = 7.6, 1.1 Hz), 6.85 (1H, td, J = 7.6, 1.1 Hz), 6.76 (1H, t, J = 7.3 Hz), 2.38 (3H, s), 2.03-1.90 (2H, m), 1.62 (3H, s), 1.55-1.50 (2H, m), 1.34-1.29 (4H, m), 0.88 (3H, t, J = 7.1 Hz).
332 ¹H-NMR (CDCl₃) δ: 8.21 (1H, s), 7.89 (1H, d, J = 9.2 Hz), 7.55 (1H, dd, J = 7.6, 1.5 Hz), 7.41-7.37 (1H, m), 7.16 (1H, d, J = 9.2 Hz), 7.00-6.96 (2H, m), 3.80 (1H, d, J = 10.7 Hz), 3.73 (1H, d, J = 10.7 Hz), 3.69-3.60 (2H, m), 2.78 (3H, s), 2.40 (3H, s), 1.66 (3H, s), 1.22 (3H, t, J = 6.9 Hz).
333 ¹H-NMR (CDCl₃) δ: 8.39 (1H, d, J = 6.9 Hz), 8.00 (1H, s), 7.38-7.35 (1H, m), 7.16 (1H, dd, J = 7.6, 1.1 Hz), 6.96 (2H, tt, J = 6.9, 1.1 Hz), 6.88 (1H, td, J = 7.6, 1.1 Hz), 6.77 (1H, t, J = 6.9 Hz), 3.83 (1H, d, J = 10.7 Hz), 3.74 (1H, d, J = 10.7 Hz), 3.69-3.56 (2H, m), 2.41 (3H, s), 1.67 (3H, s), 1.20 (3H, t, J = 7.1 Hz).
334 ¹H-NMR (CDCl₃) δ: 8.32 (1H, s), 8.16 (1H, s), 7.97 (1H, d, J = 9.0 Hz), 7.53 (1H, dd, J = 7.6, 1.5 Hz), 7.38 (1H, td, J = 7.6, 1.5 Hz), 7.14 (1H, d, J = 9.0 Hz), 6.96 (1H, td, J = 7.6, 1.1 Hz), 6.92 (1H, dd, J = 7.6, 1.1 Hz), 2.38 (3H, s), 1.95-1.91 (2H, m), 1.61-1.53 (2H, m), 1.61 (3H, s), 1.34-1.28 (4H, m), 0.88 (3H, t, J = 7.1 Hz).
335 ¹H-NMR (CDCl₃) δ: 8.33 (1H, br s), 8.16 (1H, s), 7.97 (1H, d, J = 9.0 Hz), 7.54 (1H, dd, J = 7.6, 1.5 Hz), 7.41-7.38 (1H, m), 7.15 (1H, dd, J = 9.0, 1.5 Hz), 7.00-6.97 (2H, m), 3.80 (1H, d, J = 10.7 Hz), 3.73 (1H, d, J = 10.7 Hz), 3.70-3.57 (2H, m), 2.38 (3H, s), 1.65 (3H, s), 1.22 (3H, t, J = 6.9 Hz).
336 ¹H-NMR (CDCl₃) δ: 8.26 (1H, s), 7.98 (1H, d, J = 8.5 Hz), 7.52 (1H, dd, J = 7.6, 1.0 Hz), 7.41-7.36 (1H, m), 7.24 (1H, dd, J = 8.5, 6.8 Hz), 6.96 (1H, td, J = 7.6, 1.0 Hz), 6.92 (1H, dd, J = 7.6, 1.0 Hz), 6.77 (1H, d, J = 6.8 Hz), 2.80 (3H, s), 1.97-1.92 (2H, m), 1.62 (3H, s), 1.61-1.53 (2H, m), 1.32-1.29 (4H, m), 0.87 (3H, t, J = 8.2 Hz).
337 ¹H-NMR (CDCl₃) δ: 8.53 (1H, dt, J = 7.8, 1.1 Hz), 8.23 (1H, s), 8.07 (1H, dt, J = 8.9, 1.1 Hz), 7.52 (1H, dd, J = 7.8, 1.1 Hz), 7.40-7.38 (1H, m), 7.31-7.27 (1H, m), 6.97 (1H, td, J = 7.8, 1.1 Hz), 6.93-6.88 (2H, m), 1.97-1.91 (2H, m), 1.61 (3H, s), 1.60-1.54 (2H, m), 1.32-1.30 (4H, m), 0.88 (3H, t, J = 7.1 Hz).
338 ¹H-NMR (CDCl₃) δ: 8.26 (1H, s), 7.99 (1H, d, J = 8.3 Hz), 7.54 (1H, dd, J = 7.7, 1.6 Hz), 7.43-7.37 (1H, m), 7.25 (1H, dd, J = 8.3, 6.8 Hz), 7.01-6.96 (2H, m), 6.78 (1H, d, J = 6.8 Hz), 3.81 (1H, d, J = 10.5 Hz), 3.74 (1H, d, J = 10.5 Hz), 3.71-3.58 (2H, m), 2.81 (3H, s), 1.67 (3H, s), 1.22 (3H, t, J = 7.1 Hz).
339 ¹H-NMR (CDCl₃) δ: 8.53 (1H, dt, J = 7.0, 1.1 Hz), 8.23 (1H, s), 8.08 (1H, dt, J = 7.8, 1.1 Hz), 7.54 (1H, dd, J = 7.8, 1.1 Hz), 7.43-7.37 (1H, m), 7.32-7.28 (1H, m), 7.01-6.97 (2H, m), 6.93-6.88 (1H, m), 3.81 (1H, d, J = 10.7 Hz), 3.74 (1H, d, J = 10.5 Hz), 3.71-3.58 (2H, m), 1.66 (3H, s), 1.22 (3H, t, J = 7.0 Hz).
340 ¹H-NMR (CDCl₃) δ: 8.02 (1H, s), 7.95 (1H, d, J = 9.0 Hz), 7.51-7.43 (1H, m), 7.21-7.13 (3H, m), 2.77 (3H, s), 2.41 (3H, s), 2.02-1.88 (2H, m), 1.66-1.54 (2H, m), 1.60 (3H, s), 1.40-1.27 (2H, m), 0.91 (3H, t, J = 7.3 Hz).
341 ¹H-NMR (CDCl₃) δ: 8.38 (1H, d, J = 6.8 Hz), 7.93 (1H, s), 7.16-7.10 (1H, m), 7.08-6.99 (2H, m), 6.94-6.90 (1H, m), 6.75 (1H, t, J = 7.0 Hz), 2.21 (3H, s), 2.00-1.96 (2H, m), 1.62 (3H, s), 1.61-1.51 (2H, m), 1.40-1.28 (2H, m), 0.91 (3H, t, J = 7.2 Hz).
342 ¹H-NMR (CDCl₃) δ: 8.08 (1H, s), 8.05 (1H, d, J = 8.8 Hz), 7.48-7.44 (1H, m), 7.29-7.26 (1H, m), 7.20-7.14 (2H, m), 6.78 (1H, d, J = 6.9 Hz), 2.80 (3H, s), 2.02-1.91 (2H, m), 1.63-1.56 (2H, m), 1.61 (3H, s), 1.38-1.29 (2H, m), 0.91 (3H, t, J = 7.3 Hz).
343 ¹H-NMR (CDCl₃) δ: 8.53 (1H, d, J = 6.9 Hz), 8.14 (1H, d, J = 8.0 Hz), 8.05 (1H, s), 7.49-7.45 (1H, m), 7.35-7.30 (1H, m), 7.21-7.15 (2H, m), 6.93-6.90 (1H, m), 2.02-1.90 (2H, m), 1.65-1.57 (2H, m), 1.60 (3H, s), 1.39-1.30 (2H, m), 0.91 (3H, t, J = 7.3 Hz).
344 ¹H-NMR (CDCl₃) δ: 8.38 (1H, s), 8.33 (1H, s), 7.32 (1H, dd, J = 8.8, 6.5 Hz), 6.59-6.53 (2H, m), 2.81 (3H, s), 2.40 (3H, s), 1.98-1.89 (2H, m), 1.63 (3H, s), 1.62-1.51 (2H, m), 0.94 (3H, t, J = 7.5 Hz).
345 ¹H-NMR (CDCl₃) δ: 8.22 (1H, s), 7.91 (1H, d, J = 8.8 Hz), 7.54 (1H, dd, J = 7.6, 1.5 Hz), 7.36 (1H, td, J = 7.6, 1.5 Hz), 7.15 (1H, d, J = 8.8 Hz), 6.95-6.90 (2H, m), 2.78 (3H, s), 2.40 (3H, s), 1.98-1.93 (4H, m), 1.03 (6H, t, J = 7.4 Hz).
346 ¹H-NMR (CDCl₃) δ: 8.21 (1H, s), 7.90 (1H, d, J = 9.2 Hz), 7.53 (1H, dd, J = 7.6, 1.5 Hz), 7.36 (1H, td, J = 7.6, 1.5 Hz), 7.15 (1H, d, J = 9.2 Hz), 6.93 (1H, td, J = 7.6, 1.5 Hz), 6.90 (1H, dd, J = 7.6, 1.5 Hz), 2.78 (3H, s), 2.40 (3H, s), 1.96-1.94 (2H, m), 1.90-1.88 (2H, m), 1.59-1.48 (2H, m), 1.03 (3H, t, J = 7.4 Hz), 0.92 (3H, t, J = 7.3 Hz).
347 ¹H-NMR (CDCl₃) δ: 8.33 (1H, br s), 8.17 (1H, s), 7.98 (1H, d, J = 9.2 Hz), 7.53 (1H, dd, J = 7.6, 1.5 Hz), 7.37 (1H, td, J = 7.6, 1.5 Hz), 7.14 (1H, dd, J = 9.2, 1.5 Hz), 6.94 (1H, td, J = 7.6, 1.5 Hz), 6.91 (1H, dd, J = 7.6, 1.5 Hz), 2.38 (3H, s), 1.98-1.92 (4H, m), 1.03 (6H, t, J = 7.4 Hz).
348 ¹H-NMR (CDCl₃) δ: 8.39 (1H, d, J = 6.9 Hz), 7.99 (1H, s), 7.35-7.32 (1H, m), 7.05 (1H, dd, J = 7.6, 1.0 Hz), 6.94 (1H, dt, J = 6.9, 1.0 Hz), 6.88 (1H, dd, J = 7.6, 1.0 Hz), 6.82 (1H, td, J = 7.6, 1.0 Hz), 6.76 (1H, t, J = 6.9 Hz), 2.37 (3H, s), 2.02-1.94 (4H, m), 1.04 (6H, t, J = 7.4 Hz).
349 ¹H-NMR (CDCl₃) δ: 8.32 (1H, br s), 8.17 (1H, s), 7.97 (1H, d, J = 9.0 Hz), 7.53 (1H, dd, J = 8.1, 1.2 Hz), 7.36-7.34 (1H, m), 7.14 (1H, dd, J = 9.0, 1.2 Hz), 6.94 (1H, td, J = 8.1, 1.2 Hz), 6.90 (1H, dd, J = 8.1, 1.2 Hz), 2.38 (3H, s), 1.96-1.87 (4H, m), 1.56-1.49 (2H, m), 1.03 (3H, t, J = 7.4 Hz), 0.93 (3H, t, J = 7.4 Hz).
350 ¹H-NMR (CDCl₃) δ: 8.39 (1H, d, J = 6.8 Hz), 7.99 (1H, s), 7.35-7.31 (1H, m), 7.03 (1H, dd, J = 7.7, 1.6 Hz), 6.93 (1H, d, J = 6.8 Hz), 6.86 (1H, dd, J = 7.7, 1.6 Hz), 6.81 (1H, td, J = 7.7, 1.6 Hz), 6.76 (1H, t, J = 6.8 Hz), 2.36 (3H, s), 2.04-1.90 (4H, m), 1.55-1.49 (2H, m), 1.04 (3H, t, J = 7.4 Hz), 0.94 (3H, t, J = 7.4 Hz).
351 ¹H-NMR (CDCl₃) δ: 8.27 (1H, s), 8.00 (1H, d, J = 8.4 Hz), 7.53 (1H, dd, J = 7.6, 1.5 Hz), 7.37 (1H, td, J = 7.6, 1.5 Hz), 7.24 (1H, dd, J = 8.4, 6.9 Hz), 6.96-6.91 (2H, m), 6.77 (1H, d, J = 6.9 Hz), 2.81 (3H, s), 1.98-1.95 (4H, m), 1.04 (6H, t, J = 7.4 Hz).
352 ¹H-NMR (CDCl₃) δ: 8.53 (1H, dt, J = 8.8, 1.1 Hz), 8.24 (1H, s), 8.09 (1H, dt, J = 8.8, 1.1 Hz), 7.52 (1H, dd, J = 7.6, 1.5 Hz), 7.37 (1H, td, J = 7.6, 1.5 Hz), 7.30-7.27 (1H, m), 6.95 (1H, td, J = 7.6, 1.5 Hz), 6.93-6.88 (2H, m), 1.97-1.94 (4H, m), 1.04 (6H, t, J = 7.4 Hz).
353 ¹H-NMR (CDCl₃) δ: 8.27 (1H, s), 7.99 (1H, d, J = 8.8 Hz), 7.52 (1H, dd, J = 7.6, 1.5 Hz), 7.37 (1H, td, J = 7.6, 1.5 Hz), 7.24 (1H, dd, J = 8.8, 6.9 Hz), 6.94 (1H, td, J = 7.6, 1.5 Hz), 6.90 (1H, dd, J = 7.6, 1.5 Hz), 6.77 (1H, d, J = 6.9 Hz), 2.81 (3H, s), 1.98-1.95 (2H, m), 1.91-1.89 (2H, m), 1.61-1.50 (2H, m), 1.04 (3H, t, J = 7.4 Hz), 0.93 (3H, t, J = 7.4 Hz).
354 ¹H-NMR (CDCl₃) δ: 8.53 (1H, dt, J = 7.0, 1.1 Hz), 8.24 (1H, s), 8.08 (1H, dt, J = 7.0, 1.1 Hz), 7.52 (1H, dd, J = 7.6, 1.5 Hz), 7.37 (1H, td, J = 7.6, 1.5 Hz), 7.30-7.27 (1H, m), 6.95 (1H, td, J = 7.6, 1.5 Hz), 6.91-6.89 (2H, m), 1.97-1.93 (2H, m), 1.91-1.89 (2H, m), 1.62-1.50 (2H, m), 1.04 (3H, t, J = 7.4 Hz), 0.93 (3H, t, J = 7.4 Hz).
355 ¹H-NMR (CDCl₃) δ: 8.38 (1H, s), 8.34 (1H, s), 7.33 (1H, dd, J = 8.5, 6.6 Hz), 6.61-6.53 (2H, m), 2.81 (3H, s), 2.40 (3H, s), 2.04-1.96 (2H, m), 1.63 (3H, s), 1.06 (3H, t, J = 7.3 Hz).
356 ¹H-NMR (CDCl₃) δ: 8.38 (1H, s), 8.34 (1H, s), 7.34 (1H, dd, J = 8.6, 6.3 Hz), 6.58-6.53 (2H, m), 2.81 (3H, s), 2.40 (3H, s), 1.92-1.84 (2H, m), 1.67 (3H, s), 1.27-1.25 (1H, m), 0.99 (6H, dd, J = 6.7, 2.1 Hz).
357 ¹H-NMR (CDCl₃) δ: 8.32 (1H, s), 8.03 (1H, d, J = 9.2 Hz), 7.98 (1H, s), 7.49-7.46 (1H, m), 7.20-7.14 (3H, m), 2.39 (3H, s), 2.01-1.89 (2H, m), 1.66-1.56 (2H, m), 1.59 (3H, s), 1.39-1.29 (2H, m), 0.91 (3H, t, J = 7.3 Hz).
358 ¹H-NMR (CDCl₃) δ: 9.43 (1H, s), 8.08 (1H, s), 7.49-7.43 (1H, m), 7.21-7.17 (2H, m), 2.80 (3H, s), 2.65 (3H, s), 2.02-1.91 (2H, m), 1.65-1.55 (5H, m), 1.38-1.29 (2H, m), 0.90 (3H, t, J = 8.0 Hz).
359 ¹H-NMR (CDCl₃) δ: 8.30 (1H, d, J = 5.0 Hz), 8.00 (1H, s), 7.83 (1H, d, J = 5.0 Hz), 7.18-7.15 (1H, m), 7.07-7.00 (2H, m), 2.53 (3H, s), 2.01-1.98 (2H, m), 1.64 (3H, s), 1.59-1.51 (2H, m), 1.41-1.29 (2H, m), 0.91 (3H, t, J = 7.3 Hz).
360 ¹H-NMR (CDCl₃) δ: 8.38 (1H, d, J = 6.9 Hz), 7.93 (1H, s), 7.37-7.30 (2H, m), 7.28-7.25 (1H, m), 7.08-7.05 (1H, m), 6.93-6.92 (1H, m), 6.75 (1H, t, J = 6.9 Hz), 2.24 (3H, s), 1.71 (6H, s).
361 ¹H-NMR (CDCl₃) δ: 8.52 (1H, d, J = 6.9 Hz), 8.12 (1H, d, J = 9.2 Hz), 8.06 (1H, s), 7.62 (1H, dd, J = 8.0, 0.8 Hz), 7.41-7.34 (2H, m), 7.29 (1H, ddd, J = 9.1, 6.8, 1.1 Hz), 7.23-7.18 (1H, m), 6.89 (1H, td, J = 7.3, 1.5 Hz), 1.68 (6H, s).
362 ¹H-NMR (CDCl₃) δ: 8.20 (1H, s), 8.19-8.16 (1H, m), 8.10 (1H, d, J = 8.8 Hz), 7.83-7.79 (1H, m), 7.78-7.74 (2H, m), 7.33 (1H, dd, J = 8.8, 6.9 Hz), 6.84 (1H, d, J = 6.9 Hz), 2.82 (3H, s), 1.72 (6H, s).
363 ¹H-NMR (CDCl₃) δ: 8.04 (1H, s), 7.96 (1H, d, J = 9.0 Hz), 7.63 (1H, dd, J = 7.9, 0.9 Hz), 7.39-7.31 (2H, m), 7.21-7.13 (2H, m), 2.77 (3H, s), 2.40 (3H, s), 2.13-2.05 (1H, m), 1.92-1.77 (2H, m), 1.64 (3H, s), 1.00 (3H, d, J = 6.6 Hz), 0.97 (3H, d, J = 6.8 Hz).
364 ¹H-NMR (CDCl₃) δ: 8.38 (1H, d, J = 6.8 Hz), 7.96 (1H, s), 7.35-7.29 (2H, m), 7.20-7.18 (1H, m), 7.07-7.02 (1H, m), 6.91-6.86 (1H, m), 6.73 (1H, t, J = 7.0 Hz), 2.14 (3H, s), 2.07-1.97 (1H, m), 1.89-1.85 (2H, m), 1.68 (3H, s), 1.02 (3H, d, J = 6.6 Hz), 0.96 (3H, d, J = 6.8 Hz).
365 ¹H-NMR (CDCl₃) δ: 8.38 (1H, d, J = 2.3 Hz), 8.34 (1H, s), 7.36-7.31 (2H, m), 6.89-6.84 (2H, m), 2.81 (3H, s), 1.63 (3H, s), 2.40 (3H, s), 2.04-1.97 (2H, m), 1.07 (3H, t, J = 7.4 Hz).
366 ¹H-NMR (CDCl₃) δ: 8.39 (1H, s), 8.34 (1H, s), 7.38-7.34 (1H, m), 7.33-7.32 (1H, m), 6.91-6.87 (2H, m), 2.81 (3H, s), 2.40 (3H, s), 1.70 (6H, s).
367 ¹H-NMR (CDCl₃) δ: 8.44 (1H, s), 8.43 (1H, s), 7.47-7.42 (2H, m), 7.13-7.10 (1H, m), 7.00 (1H, td, J = 5.5, 1.2 Hz), 5.06-4.99 (4H, m), 2.83 (3H, s), 2.42 (3H, s).
368 ¹H-NMR (CDCl₃) δ: 8.38 (1H, s), 8.34 (1H, s), 7.32 (1H, dd, J = 8.8, 6.5 Hz), 6.60-6.53 (2H, m), 2.81 (3H, s), 2.40 (3H, s), 1.98-1.94 (2H, m), 1.63 (3H, s), 1.55-1.46 (2H, m), 1.39-1.28 (2H, m), 0.90 (3H, t, J = 7.3 Hz).
369 ¹H-NMR (CDCl₃) δ: 9.34 (1H, s), 8.28 (1H, s), 7.53 (1H, dd, J = 8.0, 1.5 Hz), 7.42 (1H, td, J = 8.0, 1.5 Hz), 7.03-6.99 (2H, m), 3.78 (1H, d, J = 10.3 Hz), 3.72 (1H, d, J = 10.3 Hz), 3.48 (3H, s), 2.81 (3H, s), 2.64 (3H, s), 1.63 (3H, s).
370 ¹H-NMR (CDCl₃) δ: 8.31 (1H, d, J = 4.6 Hz), 8.07 (1H, s), 7.85 (1H, d, J = 4.6 Hz), 7.40 (1H, td, J = 7.8, 1.0 Hz), 7.10 (1H, dd, J = 7.8, 1.0 Hz), 6.99 (1H, dd, J = 7.8, 1.0 Hz), 6.90 (1H, td, J = 7.8, 1.0 Hz), 3.81 (1H, d, J = 10.3 Hz), 3.73 (1H, d, J = 10.3 Hz), 3.48 (3H, s), 2.71 (3H, s), 1.67 (3H, s).
371 ¹H-NMR (CDCl₃) δ: 9.34 (1H, s), 8.27 (1H, s), 7.52 (1H, dd, J = 7.6, 1.5 Hz), 7.41 (1H, td, J = 7.6, 1.5 Hz), 7.03-6.97 (2H, m), 3.81 (1H, d, J = 10.7 Hz), 3.74 (1H, d, J = 10.7 Hz), 3.69-3.64 (1H, m), 3.62-3.59 (1H, m), 2.81 (3H, s), 2.64 (3H, s), 1.65 (3H, s), 1.21 (3H, t, J = 7.4 Hz).
372 ¹H-NMR (CDCl₃) δ: 8.30 (1H, d, J = 5.0 Hz), 8.07 (1H, s), 7.85 (1H, d, J = 5.0 Hz), 7.39 (1H, td, J = 7.8, 1.0 Hz), 7.09 (1H, dd, J = 7.8, 1.0 Hz), 6.97 (1H, dd, J = 7.8, 1.0 Hz), 6.89 (1H, td, J = 7.8, 1.0 Hz), 3.84 (1H, d, J = 10.3 Hz), 3.75 (1H, d, J = 10.3 Hz), 3.67-3.64 (1H, m), 3.61-3.57 (1H, m), 2.71 (3H, s), 1.68 (3H, s), 1.18 (3H, t, J = 7.1 Hz).
373 ¹H-NMR (CDCl₃) δ: 8.21 (1H, s), 7.90 (1H, d, J = 9.0 Hz), 7.53 (1H, dd, J = 7.6, 1.5 Hz), 7.36 (1H, td, J = 7.6, 1.5 Hz), 7.15 (1H, d, J = 9.0 Hz), 6.93 (1H, td, J = 7.6, 1.5 Hz), 6.90 (1H, dd, J = 7.6, 1.5 Hz), 2.78 (3H, s), 2.40 (3H, s), 1.99-1.88 (4H, m), 1.55-1.44 (2H, m), 1.36-1.29 (2H, m), 1.03 (3H, t, J = 7.4 Hz), 0.89 (3H, t, J = 7.3 Hz).
374 ¹H-NMR (CDCl₃) δ: 8.39 (1H, dd, J = 7.0, 1.1 Hz), 7.99 (1H, s), 7.35-7.31 (1H, m), 7.03 (1H, dd, J = 7.6, 1.5 Hz), 6.93 (1H, dt, J = 7.0, 1.1 Hz), 6.87 (1H, dd, J = 7.6, 1.5 Hz), 6.81 (1H, td, J = 7.6, 1.5 Hz), 6.76 (1H, t, J = 7.0 Hz), 2.36 (3H, s), 2.00-1.93 (4H, m), 1.54-1.45 (2H, m), 1.35-1.31 (2H, m), 1.04 (3H, t, J = 7.3 Hz), 0.90 (3H, t, J = 7.3 Hz).
375 ¹H-NMR (CDCl₃) δ: 8.33 (1H, br s), 8.17 (1H, s), 7.98 (1H, d, J = 9.2 Hz), 7.53 (1H, dd, J = 7.6, 1.5 Hz), 7.36 (1H, td, J = 7.6, 1.5 Hz), 7.14 (1H, d, J = 9.2 Hz), 6.94 (1H, td, J = 7.6, 1.5 Hz), 6.90 (1H, dd, J = 7.6, 1.5 Hz), 2.38 (3H, s), 1.98-1.89 (4H, m), 1.53-1.47 (2H, m), 1.34-1.31 (2H, m), 1.03 (3H, t, J = 7.4 Hz), 0.89 (3H, t, J = 7.3 Hz).
376 ¹H-NMR (CDCl₃) δ: 8.27 (1H, s), 8.00 (1H, d, J = 8.9 Hz), 7.53-7.51 (1H, m), 7.39-7.34 (1H, m), 7.26-7.22 (1H, m), 6.96-6.89 (2H, m), 6.78-6.76 (1H, m), 2.81 (3H, s), 1.97-1.90 (4H, m), 1.53-1.45 (2H, m), 1.37-1.29 (2H, m), 1.04 (3H, t, J = 7.4 Hz), 0.89 (3H, t, J = 7.3 Hz).
377 ¹H-NMR (CDCl₃) δ: 8.53 (1H, dt, J = 6.9, 1.3 Hz), 8.24 (1H, s), 8.08 (1H, dt, J = 8.8, 1.3 Hz), 7.52 (1H, dd, J = 7.6, 1.3 Hz), 7.39-7.35 (1H, m), 7.31-7.26 (1H, m), 6.95 (1H, td, J = 7.6, 1.3 Hz), 6.92-6.88 (2H, m), 1.99-1.88 (4H, m), 1.54-1.47 (2H, m), 1.35-1.30 (2H, m), 1.04 (3H, t, J = 7.4 Hz), 0.89 (3H, t, J = 7.3 Hz).
378 ¹H-NMR (CDCl₃) δ: 8.31 (1H, d, J = 0.8 Hz), 8.04 (1H, d, J = 9.2 Hz), 8.00 (1H, s), 7.63 (1H, dd, J = 7.8, 1.0 Hz), 7.39-7.34 (2H, m), 7.21-7.18 (1H, m), 7.14 (1H, dd, J = 9.0, 1.3 Hz), 2.38 (3H, s), 2.11-2.03 (1H, m), 1.89 (1H, dd, J = 14.1, 5.3 Hz), 1.81 (1H, dd, J = 14.1, 5.7 Hz), 1.64 (3H, s), 1.00 (3H, d, J = 6.9 Hz), 0.97 (3H, d, J = 6.9 Hz).
379 ¹H-NMR (CDCl₃) δ: 8.10 (1H, s), 8.06 (1H, d, J = 8.4 Hz), 7.62 (1H, dd, J = 7.6, 0.8 Hz), 7.40-7.34 (2H, m), 7.26-7.22 (1H, m), 7.21-7.17 (1H, m), 6.76 (1H, d, J = 6.9 Hz), 2.79 (3H, s), 2.11-2.04 (1H, m), 1.90 (1H, dd, J = 14.1, 5.3 Hz), 1.82 (1H, dd, J = 14.1, 5.3 Hz), 1.65 (3H, s), 1.00 (3H, d, J = 6.5 Hz), 0.98 (3H, d, J = 6.5 Hz).
380 ¹H-NMR (CDCl₃) δ: 8.52 (1H, d, J = 7.3 Hz), 8.14 (1H, d, J = 8.8 Hz), 8.07 (1H, s), 7.62 (1H, d, J = 8.0 Hz), 7.40-7.35 (2H, m), 7.31-7.27 (1H, m), 7.22-7.19 (1H, m), 6.89 (1H, td, J = 6.9, 1.1 Hz), 2.11-2.03 (1H, m), 1.89 (1H, dd, J = 14.1, 5.3 Hz), 1.82 (1H, dd, J = 14.1, 5.7 Hz), 1.65 (3H, s), 1.00 (3H, d, J = 6.9 Hz), 0.98 (3H, d, J = 6.5 Hz).
381 ¹H-NMR (CDCl₃) δ: 8.23-8.21 (2H, m), 7.44-7.42 (1H, m), 7.30-7.26 (2H, m), 7.03 (1H, td, J = 7.0, 4.8 Hz), 5.08 (2H, dd, J = 6.7, 0.9 Hz), 5.01 (2H, dd, J = 7.0, 0.9 Hz), 2.80 (3H, s), 2.44 (3H, s).
382 ¹H-NMR (CDCl₃) δ: 8.39 (1H, s), 8.34 (1H, s), 7.33 (1H, dd, J = 8.5, 6.6 Hz), 6.63-6.55 (2H, m), 2.81 (3H, s), 2.40 (3H, s), 1.69 (6H, s).
383 ¹H-NMR (CDCl₃) δ: 8.40 (1H, s), 8.34 (1H, s), 7.39-7.35 (2H, m), 6.97 (1H, dd, J = 8.0, 0.8 Hz), 6.92-6.89 (1H, m), 3.82-3.73 (2H, m), 3.49 (3H, s), 2.81 (3H, s), 2.40 (3H, s), 1.67 (3H, s).
384 ¹H-NMR (CDCl₃) δ: 8.39 (1H, s), 8.34 (1H, s), 7.38-7.34 (2H, m), 6.96 (1H, dd, J = 8.0, 0.8 Hz), 6.89 (1H, td, J = 5.5, 1.1 Hz), 3.84-3.75 (2H, m), 3.72-3.58 (2H, m), 2.81 (3H, s), 2.40 (3H, s), 1.69 (3H, s), 1.21 (3H, t, J = 6.9 Hz).
385 ¹H-NMR (CDCl₃) δ: 9.44 (1H, s), 8.11 (1H, s), 7.64-7.60 (1H, m), 7.42-7.35 (2H, m), 7.25-7.17 (1H, m), 2.80 (3H, s), 2.65 (3H, s), 2.14-2.05 (1H, m), 1.91 (1H, dd, J = 14.2, 5.4 Hz), 1.84 (1H, dd, J = 14.2, 5.4 Hz), 1.63 (3H, s), 0.98 (6H, t, J = 6.8 Hz).
386 ¹H-NMR (CDCl₃) δ: 9.36 (1H, s), 8.27 (1H, s), 7.52 (1H, dd, J = 7.6, 1.5 Hz), 7.40 (1H, td, J = 7.6, 1.5 Hz), 6.98 (1H, td, J = 7.6, 1.5 Hz), 6.93 (1H, dd, J = 7.6, 1.5 Hz), 2.81 (3H, s), 2.65 (3H, s), 1.94 (2H, dd, J = 9.2, 7.6 Hz), 1.60 (3H, s), 1.60-1.52 (2H, m), 1.32-1.28 (4H, m), 0.87 (3H, t, J = 7.1 Hz).
387 ¹H-NMR (CDCl₃) δ: 8.17-8.13 (2H, m), 8.02 (1H, d, J = 8.8 Hz), 7.84-7.80 (1H, m), 7.77-7.72 (2H, m), 7.23 (1H, d, J = 8.8 Hz), 2.79 (3H, s), 2.42 (3H, s), 2.22-2.12 (1H, m), 1.99 (1H, dd, J = 13.9, 4.8 Hz), 1.82 (1H, dd, J = 14.1, 6.9 Hz), 1.73 (3H, s), 1.05 (3H, d, J = 6.9 Hz), 1.01 (3H, d, J = 6.9 Hz).
388 ¹H-NMR (CDCl₃) δ: 8.20 (1H, s), 8.18-8.13 (1H, m), 8.11 (1H, d, J = 9.0 Hz), 7.83-7.70 (3H, m), 7.32 (1H, dd, J = 8.9, 7.0 Hz), 6.84 (1H, d, J = 6.8 Hz), 2.82 (3H, s), 2.23-2.12 (1H, m), 2.00 (1H, dd, J = 13.9, 4.9 Hz), 1.83 (1H, dd, J = 13.9, 6.8 Hz), 1.74 (3H, s), 1.05 (3H, d, J = 6.8 Hz), 1.02 (3H, d, J = 6.6 Hz).
389 ¹H-NMR (CDCl₃) δ: 8.37-8.34 (1H, m), 8.17-8.13 (1H, m), 8.12-8.07 (2H, m), 7.84-7.70 (3H, m), 7.23 (1H, dd, J = 9.2, 1.6 Hz), 2.41 (3H, s), 2.21-2.10 (1H, m), 1.99 (1H, dd, J = 13.9, 4.6 Hz), 1.81 (1H, dd, J = 14.4, 6.8 Hz), 1.73 (3H, s), 1.05 (3H, d, J = 6.8 Hz), 1.02 (3H, d, J = 6.6 Hz).
390 ¹H-NMR (CDCl₃) δ: 8.38 (1H, s), 8.33 (1H, s), 7.18 (1H, d, J = 8.0 Hz), 6.69 (1H, s), 6.68-6.66 (1H, m), 2.80 (3H, s), 2.39 (3H, s), 2.33 (3H, s), 2.01-1.91 (2H, m), 1.62 (3H, s), 1.59-1.47 (2H, m), 1.38-1.30 (2H, m), 0.90 (3H, t, J = 8.4 Hz).
391 ¹H-NMR (CDCl₃) δ: 8.11 (1H, s), 8.03 (1H, d, J = 8.4 Hz), 7.62 (1H, dd, J = 7.8, 1.3 Hz), 7.41 (1H, dd, J = 7.6, 1.1 Hz), 7.36 (1H, td, J = 7.6, 1.4 Hz), 7.25 (1H, dd, J = 8.8, 6.9 Hz), 7.19 (1H, td, J = 7.6, 1.5 Hz), 6.77 (1H, d, J = 6.9 Hz), 3.75 (2H, s), 3.46 (3H, s), 2.79 (3H, s), 1.64 (3H, s).
392 ¹H-NMR (CDCl₃) δ: 8.53-8.51 (1H, m), 8.14-8.10 (1H, m), 8.08 (1H, s), 7.63 (1H, dd, J = 7.8, 1.3 Hz), 7.42 (1H, dd, J = 7.8, 1.3 Hz), 7.37 (1H, td, J = 7.3, 1.4 Hz), 7.32-7.28 (1H, m), 7.21 (1H, td, J = 7.6, 1.5 Hz), 6.90 (1H, td, J = 7.3, 1.5 Hz), 3.74 (2H, s), 3.46 (3H, s), 1.64 (3H, s).
393 ¹H-NMR (CDCl₃) δ: 8.33-8.30 (1H, m), 8.02-7.99 (2H, m), 7.63 (1H, dd, J = 8.0, 1.1 Hz), 7.42-7.40 (1H, m), 7.36 (1H, td, J = 8.0, 1.5 Hz), 7.20 (1H, td, J = 7.3, 1.4 Hz), 7.15 (1H, dd, J = 9.0, 1.3 Hz), 3.74 (2H, s), 3.46 (3H, s), 2.38 (3H, d, J = 1.1 Hz), 1.63 (3H, s).
394 ¹H-NMR (CDCl₃) δ: 9.39 (1H, s, ), 8.11 (1H, s, ), 7.63 (1H, dd, J = 7.8, 1.2 Hz, ), 7.45-7.36 (2H, m, ), 7.22 (1H, ddd, J = 8.2, 6.7, 1.0 Hz, ), 3.75 (2H, s, ), 3.46 (3H, s, ), 2.80 (3H, s, ), 2.64 (3H, s, ), 1.61 (3H, s, ).
395 ¹H-NMR (CDCl₃) δ: 8.21 (1H, s), 8.20-8.16 (1H, m), 8.09 (1H, d, J = 9.2 Hz), 7.85-7.80 (1H, m), 7.77-7.72 (2H, m), 7.33 (1H, dd, J = 8.8, 6.9 Hz), 6.84 (1H, d, J = 6.9 Hz), 4.12 (1H, d, J = 10.3 Hz), 3.94 (1H, d, J = 10.3 Hz), 3.39 (3H, s), 2.82 (3H, s), 1.69 (3H, s).
396 ¹H-NMR (CDCl₃) δ: 8.37-8.34 (1H, m), 8.20-8.14 (1H, m), 8.12 (1H, s), 8.08 (1H, d, J = 9.0 Hz), 7.86-7.81 (1H, m), 7.78-7.72 (2H, m), 7.23 (1H, dd, J = 9.0, 1.5 Hz), 4.11 (1H, d, J = 10.5 Hz), 3.93 (1H, d, J = 10.5 Hz), 3.39 (3H, s), 2.41 (3H, d, J = 0.7 Hz), 1.68 (3H, s).
397 ¹H-NMR (CDCl₃) δ: 9.44 (1H, s), 8.22 (1H, s), 8.21-8.17 (1H, m), 7.86-7.74 (3H, m), 4.09 (1H, d, J = 10.5 Hz), 3.95 (1H, d, J = 10.5 Hz), 3.36 (3H, s), 2.82 (3H, s), 2.67 (3H, s), 1.70 (3H, s).
398 ¹H-NMR (CDCl₃) δ: 8.38 (1H, s), 8.34 (1H, s), 7.18-7.12 (2H, m), 6.79 (1H, td, J = 6.9, 4.6 Hz), 2.81 (3H, s), 2.40 (3H, s), 2.09-2.05 (1H, m), 1.92 (2H, d, J = 6.1 Hz), 1.73 (3H, s), 1.00 (6H, dd, J = 6.7, 1.7 Hz).
399 ¹H-NMR (CDCl₃) δ: 8.38 (1H, s), 8.34 (1H, s), 7.18-7.11 (2H, m), 6.79 (1H, td, J = 6.9, 4.7 Hz), 2.81 (3H, s), 2.40 (3H, s), 2.02-1.97 (2H, m), 1.69 (3H, s), 1.66-1.52 (2H, m), 0.95 (3H, t, J = 7.4 Hz).
400 ¹H-NMR (CDCl₃) δ: 8.41 (1H, d, J = 0.7 Hz), 8.25 (1H, s), 7.20-7.13 (2H, m), 6.79 (1H, td, J = 6.9, 4.7 Hz), 2.53 (3H, s), 2.32 (3H, d, J = 1.0 Hz), 2.07-1.94 (2H, m), 1.69 (3H, s), 1.68-1.50 (2H, m), 1.41-1.31 (2H, m), 0.91 (3H, t, J = 7.3 Hz).
401 ¹H-NMR (CDCl₃) δ: 8.40 (1H, d, J = 1.2 Hz), 8.23 (1H, s), 7.21-7.12 (2H, m), 6.81-6.75 (1H, m), 2.54 (3H, s), 2.32 (3H, d, J = 1.0 Hz), 1.75 (3H, s), 1.48-1.42 (1H, m), 0.82-0.75 (1H, m), 0.64-0.46 (3H, m).
402 ¹H-NMR (CDCl₃) δ: 8.04 (1H, s), 7.95 (1H, d, J = 9.2 Hz), 7.62 (1H, dd, J = 7.6, 1.1 Hz), 7.39-7.31 (2H, m), 7.20-7.14 (2H, m), 2.77 (3H, s), 2.40 (3H, s), 1.98-1.87 (2H, m), 1.64-1.61 (2H, m), 1.59 (3H, s), 1.36-1.25 (4H, m), 0.87 (3H, t, J = 7.1 Hz).
403 ¹H-NMR (CDCl₃) δ: 8.38 (1H, d, J = 6.9 Hz), 7.94 (1H, s), 7.35-7.29 (2H, m), 7.21 (1H, dd, J = 8.0, 1.1 Hz), 7.06-7.02 (1H, m), 6.90 (1H, dt, J = 6.9, 1.0 Hz), 6.74 (1H, t, J = 6.9 Hz), 2.20 (3H, s), 1.99-1.91 (2H, m), 1.61 (3H, s), 1.60-1.52 (2H, m), 1.36-1.25 (4H, m), 0.87 (3H, t, J = 6.9 Hz).
404 ¹H-NMR (CDCl₃) δ: 8.10 (1H, s), 8.04 (1H, d, J = 8.4 Hz), 7.61 (1H, dd, J = 7.8, 1.0 Hz), 7.39-7.34 (2H, m), 7.25 (1H, dd, J = 9.5, 6.9 Hz), 7.18 (1H, ddd, J = 8.4, 6.5, 1.1 Hz), 6.77 (1H, d, J = 6.9 Hz), 2.80 (3H, s), 1.99-1.87 (2H, m), 1.67-1.61 (2H, m), 1.59 (3H, s), 1.35-1.26 (4H, m), 0.87 (3H, t, J = 7.1 Hz).
405 ¹H-NMR (CDCl₃) δ: 8.52 (1H, dt, J = 7.0, 1.0 Hz), 8.13 (1H, dt, J = 8.9, 1.2 Hz), 8.07 (1H, s), 7.63-7.60 (1H, m), 7.40-7.34 (2H, m), 7.29 (1H, ddd, J = 9.1, 6.8, 1.1 Hz), 7.20 (1H, ddd, J = 8.2, 6.5, 1.3 Hz), 6.89 (1H, td, J = 6.9, 1.5 Hz), 1.99-1.87 (2H, m), 1.66-1.60 (2H, m), 1.59 (3H, s), 1.36-1.26 (4H, m), 0.88 (3H, t, J = 7.1 Hz).
406 ¹H-NMR (CDCl₃) δ: 8.19-8.15 (1H, m), 8.14 (1H, s), 8.00 (1H, d, J = 9.0 Hz), 7.87-7.80 (1H, m), 7.78-7.71 (2H, m), 7.24 (1H, d, J = 9.0 Hz), 2.79 (3H, s), 2.43 (3H, s), 2.14-1.98 (2H, m), 1.79-1.65 (2H, m), 1.62 (3H, s), 1.39-1.24 (4H, m), 0.91-0.83 (3H, m).
407 ¹H-NMR (CDCl₃) δ: 8.31 (1H, br s), 8.02 (1H, d, J = 8.8 Hz), 8.00 (1H, s), 7.62 (1H, dd, J = 7.8, 1.3 Hz), 7.40-7.32 (2H, m), 7.21-7.17 (1H, m), 7.15 (1H, dd, J = 9.2, 1.5 Hz), 2.38 (3H, d, J = 0.8 Hz), 1.98-1.86 (2H, m), 1.64-1.59 (2H, m), 1.58 (3H, s), 1.36-1.24 (4H, m), 0.88 (3H, t, J = 7.3 Hz).
408 ¹H-NMR (CDCl₃) δ: 9.42 (1H, s), 8.10 (1H, s), 7.62 (1H, dd, J = 7.8, 1.0 Hz), 7.41-7.36 (2H, m), 7.23-7.18 (1H, m), 2.80 (3H, s), 2.65 (3H, s), 1.99-1.87 (2H, m), 1.64-1.59 (2H, m), 1.58 (3H, s), 1.35-1.25 (4H, m), 0.87 (3H, t, J = 6.9 Hz).
409 ¹H-NMR (CDCl₃) δ: 8.29 (1H, d, J = 5.3 Hz), 8.01 (1H, s), 7.81 (1H, d, J = 5.0 Hz), 7.38-7.32 (2H, m), 7.17-7.14 (1H, m), 7.08-7.05 (1H, m), 2.51 (3H, s), 2.00-1.92 (2H, m), 1.63 (3H, s), 1.60-1.53 (2H, m), 1.35-1.25 (4H, m), 0.88 (3H, t, J = 6.9 Hz).
410 ¹H-NMR (CDCl₃) δ: 8.20 (1H, s), 8.18-8.15 (1H, m), 8.10 (1H, d, J = 8.8 Hz), 7.84-7.80 (1H, m), 7.79-7.73 (2H, m), 7.34 (1H, dd, J = 8.9, 7.0 Hz), 6.84 (1H, d, J = 7.1 Hz), 2.82 (3H, s), 2.13-1.98 (2H, m), 1.79-1.65 (2H, m), 1.63 (3H, s), 1.41-1.31 (4H, m), 0.91-0.86 (3H, m).
411 ¹H-NMR (CDCl₃) δ: 8.38 (1H, s), 8.33 (1H, s), 7.36-7.30 (2H, m), 6.88-6.85 (2H, m), 2.81 (3H, s), 2.40 (3H, s), 1.98-1.93 (2H, m), 1.64 (3H, s), 1.61-1.50 (2H, m), 1.33-1.26 (4H, m), 0.87 (3H, t, J = 7.1 Hz).
412 ¹H-NMR (CDCl₃) δ: 8.34 (1H, s), 8.31 (1H, s), 7.45-7.42 (1H, m), 7.02 (1H, dd, J = 8.0, 1.5 Hz), 6.94 (1H, dd, J = 8.2, 1.0 Hz), 6.87 (1H, td, J = 5.3, 0.9 Hz), 2.75 (3H, s), 2.00-1.96 (2H, m), 1.65 (3H, s), 1.53-1.47 (2H, m), 1.36-1.33 (2H, m), 0.90 (3H, t, J = 7.4 Hz).
413 ¹H-NMR (CDCl₃) δ: 8.47 (1H, d, J = 4.2 Hz), 8.40 (1H, s), 7.37-7.31 (2H, m), 6.89-6.85 (2H, m), 6.78 (1H, dd, J = 4.2, 1.0 Hz), 2.85 (3H, d, J = 1.0 Hz), 2.10-2.02 (1H, m), 1.92-1.89 (2H, m), 1.69 (3H, s), 1.00 (6H, dd, J = 6.6, 1.7 Hz).
414 ¹H-NMR (CDCl₃) δ: 8.38 (1H, s), 8.33 (1H, s), 7.35-7.32 (2H, m), 6.87-6.83 (2H, m), 2.81 (3H, s), 2.40 (3H, s), 2.29-2.26 (1H, m), 1.58 (3H, s), 1.15 (3H, d, J = 6.9 Hz), 1.08 (3H, d, J = 6.9 Hz).
415 ¹H-NMR (CDCl₃) δ: 8.48 (1H, d, J = 4.4 Hz), 8.40 (1H, s), 7.37-7.28 (2H, m), 6.88-6.84 (2H, m), 6.78 (1H, dd, J = 4.3, 0.9 Hz), 2.84 (3H, d, J = 0.7 Hz), 2.00-1.94 (2H, m), 1.65-1.48 (2H, m), 1.65 (3H, s), 0.94 (3H, t, J = 7.3 Hz).
416 ¹H-NMR (CDCl₃) δ: 8.37-8.35 (1H, m), 8.19-8.13 (1H, m), 8.11-8.06 (2H, m), 7.86-7.81 (1H, m), 7.78-7.73 (2H, m), 7.24 (1H, dd, J = 9.0, 1.5 Hz), 2.41 (3H, d, J = 0.7 Hz), 2.13-1.97 (2H, m), 1.79-1.67 (2H, m), 1.62 (3H, s), 1.39-1.31 (4H, m), 0.91-0.87 (3H, m).
417 ¹H-NMR (CDCl₃) δ: 9.45 (1H, s), 8.24-8.16 (2H, m), 7.85-7.77 (3H, m), 2.83 (3H, s), 2.67 (3H, s), 2.12-1.98 (2H, m), 1.79-1.65 (2H, m), 1.64 (3H, s), 1.37-1.29 (4H, m), 0.91-0.84 (3H, m).
418 ¹H-NMR (CDCl₃) δ: 8.02 (1H, s), 7.98 (1H, d, J = 8.8 Hz), 7.50-7.46 (1H, m), 7.19-7.15 (3H, m), 2.77 (3H, s), 2.41 (3H, s), 2.13-2.03 (1H, m), 1.90 (1H, dd, J = 14.1, 5.3 Hz), 1.84 (1H, dd, J = 14.1, 5.7 Hz), 1.65 (3H, s), 1.00 (3H, d, J = 6.9 Hz), 0.98 (3H, d, J = 6.5 Hz).
419 ¹H-NMR (CDCl₃) δ: 8.09 (1H, s, ), 7.28-7.23 (1H, m, ), 7.17 (1H, d, J = 8.0 Hz, ), 7.16-7.11 (2H, m, ), 7.09 (1H, d, J = 8.0 Hz, ), 2.63 (3H, s, ), 2.41 (3H, s, ), 1.99-1.96 (2H, m, ), 1.63 (3H, s, ), 1.60-1.50 (2H, m, ), 1.40-1.30 (2H, m, ), 0.91 (3H, t, J = 7.3 Hz, ).
420 ¹H-NMR (CDCl₃) δ: 8.11 (1H, s), 7.27-7.23 (1H, m), 7.17-7.11 (3H, m), 7.08 (1H, d, J = 8.4 Hz), 2.63 (3H, s), 2.41 (3H, s), 2.11-2.04 (1H, m), 1.94-1.87 (2H, m), 1.66 (3H, s), 1.02 (3H, d, J = 7.0 Hz), 0.97 (3H, d, J = 7.0 Hz).
421 ¹H-NMR (CDCl₃) δ: 8.33 (1H, s), 8.31 (1H, s), 7.23 (1H, dd, J = 7.7, 1.3 Hz), 7.14 (1H, td, J = 6.0, 1.3 Hz), 7.07-7.04 (1H, m), 2.80 (3H, s), 2.38 (3H, s), 2.10-2.03 (1H, m), 1.94-1.81 (2H, m), 1.70 (3H, s), 1.03 (3H, d, J = 6.6 Hz), 0.96 (3H, d, J = 6.6 Hz).
422 ¹H-NMR (CDCl₃) δ: 8.47 (1H, d, J = 4.2 Hz), 8.39 (1H, s), 7.36-7.31 (2H, m), 6.89-6.84 (2H, m), 6.78 (1H, dd, J = 4.2, 0.7 Hz), 2.84 (3H, d, J = 0.7 Hz), 1.67 (3H, s), 1.49-1.41 (1H, m), 0.77-0.72 (1H, m), 0.63-0.58 (1H, m), 0.55-0.46 (2H, m).
423 ¹H-NMR (CDCl₃) δ: 8.48 (1H, d, J = 4.2 Hz), 8.40 (1H, d, J = 2.3 Hz), 7.18-7.11 (2H, m), 6.82-6.78 (2H, m), 2.85 (3H, s), 2.03-2.01 (2H, m), 1.69 (3H, s), 1.61-1.50 (2H, m), 1.37-1.33 (2H, m), 0.90 (3H, t, J = 7.3 Hz).
424 ¹H-NMR (CDCl₃) δ: 8.12 (1H, s, ), 7.73 (1H, td, J = 7.1, 4.7 Hz, ), 7.57-7.50 (1H, m, ), 7.28 (1H, d, J = 8.3 Hz, ), 7.24 (1H, d, J = 8.3 Hz, ), 7.13 (1H, d, J = 8.5 Hz, ), 2.63 (3H, s, ), 2.41 (3H, s, ), 2.18 (2H, q, J = 7.1 Hz, ), 1.69 (3H, s, ), 1.23 (3H, t, J = 7.1 Hz, ).
425 ¹H-NMR (CDCl₃) δ: 8.55 (1H, d, J = 6.9 Hz), 8.30 (1H, s), 7.26-7.24 (1H, m), 6.73 (1H, d, J = 7.3 Hz), 6.68-6.67 (2H, m), 2.58 (3H, s), 2.33 (3H, s), 2.12-2.04 (1H, m), 1.88 (2H, d, J = 6.1 Hz), 1.68 (3H, s), 1.01 (6H, dd, J = 6.7, 2.5 Hz).
426 ¹H-NMR (CDCl₃) δ: 8.57 (1H, d, J = 7.3 Hz), 8.32 (1H, s), 7.19-7.14 (2H, m), 6.80 (1H, td, J = 6.9, 4.6 Hz), 6.76 (1H, d, J = 7.3 Hz), 2.59 (3H, s), 2.13-2.06 (1H, m), 1.96-1.89 (2H, m), 1.74 (3H, s), 1.02 (6H, dd, J = 6.5, 3.1 Hz).
427 ¹H-NMR (CDCl₃) δ: 8.38 (1H, s), 8.33 (1H, s), 7.20 (1H, d, J = 7.6 Hz), 6.68-6.66 (2H, m), 2.80 (3H, s), 2.39 (3H, s), 2.33 (3H, s), 2.09-2.01 (1H, m), 1.92-1.84 (2H, m), 1.67 (3H, s), 0.99 (6H, dd, J = 6.7, 1.0 Hz).
428 ¹H-NMR (CDCl₃) δ: 8.47 (1H, d, J = 2.9 Hz), 8.39 (1H, s), 7.18 (1H, d, J = 8.0 Hz), 6.77 (1H, dd, J = 4.2, 1.1 Hz), 6.71-6.67 (2H, m), 2.84 (3H, d, J = 0.8 Hz), 2.33 (3H, s), 1.63 (3H, s), 2.02-1.92 (2H, m), 1.60-1.48 (2H, m), 1.37-1.30 (2H, m), 0.90 (3H, t, J = 7.3 Hz).
429 ¹H-NMR (CDCl₃) δ: 8.10-8.04 (2H, m), 7.49-7.45 (1H, m), 7.29-7.24 (1H, m), 7.20-7.15 (2H, m), 6.78 (1H, d, J = 6.9 Hz), 2.80 (3H, s), 2.14-2.06 (1H, m), 1.91 (1H, dd, J = 14.5, 5.3 Hz), 1.85 (1H, dd, J = 14.1, 5.7 Hz), 1.66 (3H, s), 1.01 (3H, d, J = 6.9 Hz), 0.98 (3H, d, J = 6.9 Hz).
430 ¹H-NMR (CDCl₃) δ: 8.53 (1H, dt, J = 7.0, 1.0 Hz), 8.16 (1H, dt, J = 8.9, 1.2 Hz), 8.05 (1H, s), 7.50-7.45 (1H, m), 7.34-7.29 (1H, m), 7.21-7.16 (2H, m), 6.91 (1H, td, J = 7.3, 1.5 Hz), 2.13-2.04 (1H, m), 1.91 (1H, dd, J = 14.1, 5.3 Hz), 1.84 (1H, dd, J = 14.1, 5.7 Hz), 1.66 (3H, s), 1.01 (3H, d, J = 6.5 Hz), 0.99 (3H, d, J = 6.9 Hz).
431 ¹H-NMR (CDCl₃) δ: 8.48 (1H, d, J = 4.4 Hz), 8.41 (1H, s), 7.19-7.13 (2H, m), 6.83-6.78 (2H, m), 2.85 (3H, d, J = 1.0 Hz), 2.10-2.02 (1H, m), 1.93 (2H, d, J = 6.1 Hz), 1.73 (3H, s), 1.01 (6H, dd, J = 6.6, 1.0 Hz).
432 ¹H-NMR (CDCl₃) δ: 8.51-8.51 (1H, m), 8.44 (1H, d, J = 1.9 Hz), 8.30 (1H, s), 7.23-7.20 (1H, m), 7.13-7.10 (1H, m), 6.77 (1H, t, J = 7.4 Hz), 2.40 (3H, d, J = 1.1 Hz), 2.23 (3H, s), 1.99-1.94 (2H, m), 1.63 (3H, s), 1.58-1.48 (2H, m), 1.38-1.30 (2H, m), 0.90 (3H, t, J = 7.4 Hz).
433 ¹H-NMR (CDCl₃) δ: 8.37 (1H, s), 8.32 (1H, s), 7.20 (1H, d, J = 7.3 Hz), 7.13-7.11 (1H, m), 6.76 (1H, t, J = 7.6 Hz), 2.80 (3H, s), 2.39 (3H, s), 2.23 (3H, s), 1.99-1.93 (2H, m), 1.63 (3H, s), 1.60-1.45 (2H, m), 1.38-1.29 (2H, m), 0.90 (3H, t, J = 7.3 Hz).
434 ¹H-NMR (CDCl₃) δ: 8.33-8.31 (1H, m), 8.06 (1H, d, J = 8.8 Hz), 7.99 (1H, s), 7.50-7.46 (1H, m), 7.21-7.15 (3H, m), 2.38 (3H, d, J = 1.1 Hz), 2.12-2.06 (1H, m), 1.90 (1H, dd, J = 14.5, 5.3 Hz), 1.84 (1H, dd, J = 14.3, 5.5 Hz), 1.65 (3H, s), 1.00 (3H, d, J = 6.5 Hz), 0.98 (3H, d, J = 6.5 Hz).
435 ¹H-NMR (CDCl₃) δ: 8.38 (1H, d, J = 6.9 Hz), 7.95 (1H, s), 7.15-7.11 (1H, m), 7.06-7.00 (2H, m), 6.91 (1H, dt, J = 6.9, 1.1 Hz), 6.75 (1H, t, J = 6.9 Hz), 2.16 (3H, s), 2.10-2.01 (1H, m), 1.94-1.85 (2H, m), 1.69 (3H, s), 1.03 (3H, d, J = 6.5 Hz), 0.97 (3H, d, J = 6.5 Hz).
436 ¹H-NMR (CDCl₃) δ: 9.45 (1H, s), 8.09 (1H, s), 7.50-7.45 (1H, m), 7.23-7.18 (2H, m), 2.80 (3H, s), 2.65 (3H, s), 2.14-2.07 (1H, m), 1.92 (1H, dd, J = 14.1, 5.3 Hz), 1.87 (1H, dd, J = 14.1, 6.1 Hz), 1.64 (3H, s), 1.02-0.96 (6H, m).
437 ¹H-NMR (CDCl₃) δ: 8.04 (1H, s), 7.98 (1H, d, J = 9.0 Hz), 7.73 (1H, td, J = 8.1, 5.0 Hz), 7.63 (1H, dd, J = 7.9, 1.1 Hz), 7.45-7.39 (1H, m), 7.26 (1H, d, J = 8.8 Hz), 2.78 (3H, s), 2.43 (3H, s), 2.16-2.06 (2H, m), 1.77-1.57 (5H, m), 1.45-1.34 (2H, m), 0.94 (3H, t, J = 7.3 Hz).
438 ¹H-NMR (CDCl₃) δ: 7.87 (1H, s, ), 7.87 (1H, s, ), 6.76 (1H, td, J = 8.0, 1.1 Hz, ), 6.61-6.56 (1H, m, ), 6.42 (1H, dd, J = 8.0, 0.8 Hz, ), 2.29 (3H, s, ), 1.55 (2H, q, J = 7.3 Hz, ), 1.21-1.18 (3H, m, ), 0.63 (3H, t, J = 7.3 Hz, ).
439 ¹H-NMR (CDCl₃) δ: 8.47 (1H, d, J = 4.2 Hz), 8.39 (1H, s), 7.22-7.21 (1H, m), 7.12 (1H, dd, J = 7.6, 1.1 Hz), 6.78-6.75 (2H, m), 2.84 (3H, d, J = 0.8 Hz), 2.23 (3H, s), 1.62-1.48 (2H, m), 2.00-1.96 (2H, m), 1.64 (3H, s), 1.37-1.31 (2H, m), 0.90 (3H, t, J = 7.4 Hz).
440 ¹H-NMR (CDCl₃) δ: 8.52-8.51 (1H, m), 8.45 (1H, d, J = 2.2 Hz), 8.31 (1H, s), 7.18 (1H, d, J = 7.8 Hz), 6.70-6.67 (2H, m), 2.41 (3H, d, J = 2.0 Hz), 2.33 (3H, s), 2.00-1.91 (2H, m), 1.63 (3H, s), 1.60-1.46 (2H, m), 1.38-1.31 (2H, m), 0.90 (3H, t, J = 7.3 Hz).
441 ¹H-NMR (CDCl₃) δ: 8.52-8.52 (1H, m), 8.44-8.44 (1H, m), 8.31 (1H, s), 7.22-7.20 (1H, m), 7.14-7.12 (1H, m), 6.78-6.75 (1H, m), 2.40 (3H, d, J = 1.1 Hz), 2.22 (3H, s), 2.10-2.02 (1H, m), 1.92 (2H, d, J = 6.1 Hz), 1.66 (3H, s), 1.03-0.98 (6H, m).
442 ¹H-NMR (CDCl₃) δ: 8.47 (1H, d, J = 4.2 Hz), 8.39 (1H, s), 7.21 (1H, d, J = 7.6 Hz), 7.13 (1H, dd, J = 7.6, 1.1 Hz), 6.78-6.75 (2H, m), 2.84 (3H, d, J = 0.8 Hz), 2.23 (3H, s), 2.10-2.03 (1H, m), 1.93 (2H, d, J = 6.5 Hz), 1.67 (3H, s), 1.01 (6H, dd, J = 12.4, 6.7 Hz).
443 ¹H-NMR (CDCl₃) δ: 8.37 (1H, s), 8.32 (1H, s), 7.21-7.19 (1H, m), 7.15-7.12 (1H, m), 6.76 (1H, t, J = 7.6 Hz), 2.80 (3H, s), 2.39 (3H, s), 2.22 (3H, s), 2.09-1.99 (1H, m), 1.92 (2H, d, J = 6.3 Hz), 1.66 (3H, s), 1.00 (6H, dd, J = 10.4, 6.7 Hz).
444 ¹H-NMR (CDCl₃) δ: 8.33 (1H, s), 8.30 (1H, s), 7.22 (1H, dd, J = 8.0, 1.1 Hz), 7.15-7.12 (1H, m), 7.06-7.02 (1H, m), 2.80 (3H, s), 2.38 (3H, s), 2.02-1.90 (2H, m), 1.63 (3H, s), 1.61-1.54 (2H, m), 1.36-1.30 (2H, m), 0.90 (3H, t, J = 7.4 Hz).
445 ¹H-NMR (CDCl₃) δ: 8.42 (1H, d, J = 4.2 Hz), 8.37 (1H, s), 7.22 (1H, dd, J = 7.8, 1.2 Hz), 7.14 (1H, td, J = 6.1, 1.2 Hz), 7.08-7.02 (1H, m), 6.76 (1H, dd, J = 4.3, 0.9 Hz), 2.84 (3H, d, J = 0.7 Hz), 2.01-1.92 (2H, m), 1.64 (3H, s), 1.59-1.55 (2H, m), 1.37-1.30 (2H, m), 0.90 (3H, t, J = 7.3 Hz).
446 ¹H-NMR (CDCl₃) δ: 8.47 (1H, d, J = 4.2 Hz), 8.39 (1H, s), 7.21 (1H, d, J = 7.3 Hz), 7.13-7.11 (1H, m), 6.78-6.75 (2H, m), 2.84 (3H, d, J = 0.8 Hz), 2.23 (3H, s), 1.98-1.95 (2H, m), 1.62-1.49 (2H, m), 1.59 (3H, s), 1.35-1.26 (4H, m), 0.88 (3H, t, J = 7.1 Hz).
447 ¹H-NMR (CDCl₃) δ: 8.51-8.51 (1H, m), 8.44 (1H, s), 8.30 (1H, s), 7.22-7.20 (1H, m), 7.12-7.11 (1H, m), 6.77 (1H, t, J = 7.6 Hz), 2.40 (3H, d, J = 1.1 Hz), 2.23 (3H, s), 1.97-1.94 (2H, m), 1.64 (3H, s), 1.59-1.52 (2H, m), 1.32-1.27 (4H, m), 0.88 (3H, t, J = 6.9 Hz).
448 ¹H-NMR (CDCl₃) δ: 8.37 (1H, s), 8.32 (1H, s), 7.21-7.20 (1H, m), 7.13-7.11 (1H, m), 6.76-6.75 (1H, m), 2.80 (3H, s), 2.39 (3H, s), 2.23 (3H, s), 1.98-1.92 (2H, m), 1.64 (3H, s), 1.60-1.51 (2H, m), 1.34-1.26 (4H, m), 0.87 (3H, t, J = 7.1 Hz).
449 ¹H-NMR (CDCl₃) δ: 8.24 (1H, s), 7.99 (1H, s), 7.16-7.11 (1H, m), 6.99-6.94 (1H, m), 6.87-6.85 (1H, m), 2.56 (3H, s), 2.28 (3H, s), 1.94 (2H, q, J = 7.5 Hz), 1.57 (3H, s), 1.03 (3H, t, J = 7.5 Hz).
450 ¹H-NMR (CDCl₃) δ: 8.51-8.51 (1H, m), 8.44 (1H, d, J = 2.2 Hz), 8.30 (1H, s), 7.22-7.20 (1H, m), 7.13-7.11 (1H, m), 6.77 (1H, t, J = 7.6 Hz), 2.40 (3H, d, J = 1.0 Hz), 2.23 (3H, s), 1.98-1.93 (2H, m), 1.64 (3H, s), 1.66-1.52 (2H, m), 0.94 (3H, t, J = 7.3 Hz).
451 ¹H-NMR (CDCl₃) δ: 8.47 (1H, d, J = 4.2 Hz), 8.38 (1H, s), 7.22-7.20 (1H, m), 7.13-7.11 (1H, m), 6.78-6.75 (2H, m), 2.84 (3H, d, J = 0.8 Hz), 2.23 (3H, s), 1.98-1.93 (2H, m), 1.65-1.49 (2H, m), 1.64 (3H, s), 0.94 (3H, t, J = 7.4 Hz).
452 ¹H-NMR (CDCl₃) δ: 8.48 (1H, d, J = 4.4 Hz), 8.41 (1H, s), 7.19-7.11 (2H, m), 6.83-6.77 (2H, m), 2.85 (3H, d, J = 0.7 Hz), 2.06 (2H, q, J = 7.4 Hz), 1.69 (3H, s), 1.09 (3H, t, J = 7.4 Hz).
453 ¹H-NMR (CDCl₃) δ: 8.16 (1H, s), 7.27 (1H, d, J = 8.3 Hz), 7.11 (1H, d, J = 8.3 Hz), 7.06 (1H, dd, J = 8.3, 6.3 Hz), 6.65 (1H, t, J = 8.3 Hz), 2.62 (3H, s), 2.41 (3H, s), 2.19 (3H, d, J = 1.7 Hz), 1.71 (6H, s).
454 ¹H-NMR (CDCl₃) δ: 8.19 (1H, s), 7.40-7.38 (1H, m), 7.21 (1H, t, J = 7.7 Hz), 7.16-7.13 (1H, m), 7.07-7.03 (1H, m), 6.66 (1H, t, J = 8.9 Hz), 2.71 (3H, s), 2.19 (3H, d, J = 2.0 Hz), 1.72 (6H, s).
455 ¹H-NMR (CDCl₃) δ: 8.19 (1H, s), 7.91 (1H, d, J = 9.0 Hz), 7.35 (1H, dt, J = 7.8, 1.3 Hz), 7.21-7.16 (2H, m), 6.89 (1H, td, J = 8.1, 4.9 Hz), 2.78 (3H, s), 2.40 (3H, s), 2.03 (2H, q, J = 7.4 Hz), 1.65 (3H, s), 1.10 (3H, t, J = 7.4 Hz).
456 ¹H-NMR (CDCl₃) δ: 8.40 (1H, d, J = 6.8 Hz), 7.99 (1H, s), 7.20-7.15 (1H, m), 6.99-6.90 (2H, m), 6.81-6.76 (2H, m), 2.40 (3H, s), 2.14-1.98 (2H, m), 1.67 (3H, s), 1.09 (3H, t, J = 7.4 Hz).
457 ¹H-NMR (CDCl₃) δ: 8.25 (1H, s), 8.03-7.98 (1H, m), 7.36-7.31 (1H, m), 7.28-7.24 (1H, m), 7.23-7.16 (1H, m), 6.90 (1H, td, J = 7.8, 4.9 Hz), 6.78 (1H, d, J = 6.8 Hz), 2.80 (3H, s), 2.04 (2H, q, J = 7.5 Hz), 1.66 (3H, s), 1.11 (3H, t, J = 7.5 Hz).
458 ¹H-NMR (CDCl₃) δ: 8.54 (1H, dt, J = 6.9, 1.0 Hz), 8.22 (1H, s), 8.10 (1H, dt, J = 9.0, 1.2 Hz), 7.35-7.29 (2H, m), 7.23-7.14 (1H, m), 6.93-6.88 (2H, m), 2.03 (2H, q, J = 7.4 Hz), 1.66 (3H, s), 1.11 (3H, t, J = 7.4 Hz).
459 ¹H-NMR (CDCl₃) δ: 8.35-8.31 (1H, m), 8.15 (1H, s), 8.01-7.96 (1H, m), 7.36-7.32 (1H, m), 7.22-7.15 (2H, m), 6.90 (1H, td, J = 8.1, 4.6 Hz), 2.39 (3H, d, J = 1.0 Hz), 2.02 (2H, q, J = 7.6 Hz), 1.65 (3H, s), 1.10 (3H, t, J = 7.6 Hz).
460 ¹H-NMR (CDCl₃) δ: 9.37 (1H, s), 8.26 (1H, s), 7.33 (1H, dt, J = 7.8, 1.2 Hz), 7.25-7.18 (1H, m), 6.92 (1H, td, J = 7.8, 4.6 Hz), 2.81 (3H, s), 2.65 (3H, s), 2.04 (2H, q, J = 7.4 Hz), 1.65 (3H, s), 1.10 (3H, t, J = 7.4 Hz).
461 ¹H-NMR (CDCl₃) δ: 8.32 (1H, dd, J = 4.8, 0.6 Hz), 8.07 (1H, s), 7.86 (1H, d, J = 5.2 Hz), 7.24-7.16 (1H, m), 6.89-6.86 (1H, m), 6.85-6.78 (1H, m), 2.69 (3H, s), 2.16-1.98 (2H, m), 1.69 (3H, s), 1.10 (3H, t, J = 7.5 Hz).
462 ¹H-NMR (CDCl₃) δ: 8.30 (1H, s, ), 8.12 (1H, s, ), 7.25-7.22 (1H, m, ), 6.90 (1H, d, J = 8.1 Hz, ), 6.82 (1H, td, J = 6.8, 4.4 Hz, ), 2.64 (3H, s, ), 2.38 (3H, s, ), 1.76 (6H, s, ).
463 ¹H-NMR (CDCl₃) δ: 8.30 (1H, s, ), 8.11 (1H, s, ), 6.93 (1H, ddd, J = 9.0, 5.4, 2.2 Hz, ), 6.71-6.64 (1H, m, ), 2.64 (3H, s, ), 2.38 (3H, s, ), 1.77 (6H, d, J = 3.2 Hz, ).
464 ¹H-NMR (CDCl₃) δ: 8.15 (1H, s), 7.86 (1H, d, J = 9.0 Hz), 7.35 (1H, ddd, J = 8.5, 6.3, 0.5 Hz), 7.14 (1H, d, J = 9.0 Hz), 6.63 (1H, t, J = 8.9 Hz), 2.77 (3H, s), 2.40 (3H, s), 2.16 (3H, d, J = 1.7 Hz), 1.97-1.91 (2H, m), 1.61 (3H, s), 1.59-1.43 (2H, m), 1.40-1.29 (2H, m), 0.91 (3H, t, J = 7.8 Hz).
465 ¹H-NMR (CDCl₃) δ: 8.38 (1H, d, J = 6.8 Hz), 7.96 (1H, s), 6.98-6.91 (2H, m), 6.76 (1H, t, J = 6.8 Hz), 6.53 (1H, t, J = 8.8 Hz), 2.38 (3H, s), 2.16 (3H, d, J = 1.7 Hz), 2.04-1.89 (2H, m), 1.62 (3H, s), 1.59-1.45 (2H, m), 1.41-1.29 (2H, m), 0.92 (3H, t, J = 6.6 Hz).
466 ¹H-NMR (CDCl₃) δ: 8.21 (1H, s), 7.95 (1H, d, J = 9.5 Hz), 7.34 (1H, dd, J = 9.3, 6.1 Hz), 7.24 (1H, dd, J = 8.8, 6.8 Hz), 6.76 (1H, d, J = 6.8 Hz), 6.64 (1H, t, J = 8.9 Hz), 2.78 (3H, d, J = 14.9 Hz), 2.17 (3H, d, J = 1.7 Hz), 2.00-1.92 (2H, m), 1.61 (3H, s), 1.59-1.44 (2H, m), 1.41-1.29 (2H, m), 0.91 (3H, t, J = 7.6 Hz).
467 ¹H-NMR (CDCl₃) δ: 8.52 (1H, dt, J = 7.0, 1.0 Hz), 8.18 (1H, br s), 8.04 (1H, dt, J = 9.0, 1.2 Hz), 7.37-7.32 (1H, m), 7.31-7.26 (1H, m), 6.89 (1H, td, J = 6.8, 1.4 Hz), 6.64 (1H, t, J = 8.9 Hz), 2.16 (3H, d, J = 2.0 Hz), 1.95 (2H, dd, J = 8.9, 7.7 Hz), 1.61 (3H, s), 1.59-1.45 (2H, m), 1.40-1.30 (2H, m), 0.92 (3H, t, J = 7.3 Hz).
468 ¹H-NMR (CDCl₃) δ: 8.32-8.31 (1H, m), 8.11 (1H, s), 7.94 (1H, d, J = 8.8 Hz), 7.35 (1H, dd, J = 8.6, 6.3 Hz), 7.13 (1H, dd, J = 9.0, 1.3 Hz), 6.64 (1H, t, J = 9.0 Hz), 2.38 (3H, d, J = 1.1 Hz), 2.16 (3H, d, J = 1.5 Hz), 1.98-1.90 (2H, m), 1.60 (3H, s), 1.58-1.46 (2H, m), 1.39-1.30 (2H, m), 0.91 (3H, t, J = 7.3 Hz).
469 ¹H-NMR (CDCl₃) δ: 9.34 (1H, s), 8.22 (1H, s), 7.34 (1H, dd, J = 8.6, 6.3 Hz), 6.66 (1H, t, J = 9.2 Hz), 2.80 (3H, s), 2.64 (3H, s), 2.17 (3H, d, J = 1.9 Hz), 1.99-1.91 (2H, m), 1.61 (3H, s), 1.60-1.45 (2H, m), 1.38-1.30 (2H, m), 0.91 (3H, t, J = 7.3 Hz).
470 ¹H-NMR (CDCl₃) δ: 8.30 (1H, d, J = 5.0 Hz), 8.04 (1H, s), 7.84 (1H, d, J = 4.6 Hz), 6.89 (1H, dd, J = 8.6, 6.3 Hz), 6.55 (1H, t, J = 8.8 Hz), 2.68 (3H, s), 2.17 (3H, d, J = 1.9 Hz), 2.06-1.94 (2H, m), 1.65 (3H, s), 1.62-1.45 (2H, m), 1.41-1.31 (2H, m), 0.92 (3H, t, J = 7.4 Hz).
471 ¹H-NMR (CDCl₃) δ: 8.54-8.53 (1H, m), 8.46 (1H, d, J = 1.9 Hz), 8.32 (1H, s), 7.19-7.11 (2H, m), 6.80 (1H, td, J = 6.9, 4.6 Hz), 2.42 (3H, d, J = 1.1 Hz), 2.05 (2H, q, J = 7.4 Hz), 1.68 (3H, s), 1.09 (3H, t, J = 7.4 Hz).
472 ¹H-NMR (CDCl₃) δ: 8.53-8.53 (1H, m, ), 8.46 (1H, d, J = 2.2 Hz, ), 8.32 (1H, s, ), 7.19-7.11 (2H, m, ), 6.80 (1H, td, J = 7.0, 4.7 Hz, ), 2.42 (3H, d, J = 1.0 Hz, ), 2.04-2.00 (2H, m, ), 1.68 (3H, s, ), 1.62-1.50 (2H, m, ), 1.39-1.30 (2H, m, ), 0.90 (3H, t, J = 7.3 Hz, ).
473 ¹H-NMR (CDCl₃) δ: 8.54-8.53 (1H, m), 8.46 (1H, d, J = 2.3 Hz), 8.33 (1H, s), 7.21-7.16 (1H, m), 7.15-7.12 (1H, m), 6.83 (1H, td, J = 6.9, 4.6 Hz), 2.42 (3H, d, J = 1.1 Hz), 1.75 (6H, s).
474 ¹H-NMR (CDCl₃) δ: 8.49 (1H, d, J = 4.2 Hz), 8.40 (1H, s), 7.20-7.16 (1H, m), 7.14-7.13 (1H, m), 6.85-6.80 (2H, m), 2.85 (3H, d, J = 0.8 Hz), 1.75 (6H, s).
475 ¹H-NMR (CDCl₃) δ: 8.39 (1H, s), 8.34 (1H, s), 7.20-7.12 (2H, m), 6.81 (1H, td, J = 6.9, 4.6 Hz), 2.81 (3H, s), 2.40 (3H, s), 1.75 (6H, s).
476 ¹H-NMR (CDCl₃) δ: 8.52-8.52 (1H, m), 8.45-8.44 (1H, m), 8.31 (1H, s), 7.16-7.12 (1H, m), 6.57 (1H, t, J = 9.0 Hz), 2.41 (3H, d, J = 0.8 Hz), 2.14 (3H, d, J = 1.9 Hz), 1.69 (6H, s).
477 ¹H-NMR (CDCl₃) δ: 8.48 (1H, t, J = 2.9 Hz), 8.40 (1H, s), 7.15-7.13 (1H, m), 6.78 (1H, d, J = 4.2 Hz), 6.57 (1H, t, J = 9.0 Hz), 2.84 (3H, d, J = 0.8 Hz), 2.15 (3H, d, J = 1.9 Hz), 1.70 (6H, s).
478 ¹H-NMR (CDCl₃) δ: 8.37 (1H, s), 8.33 (1H, s), 7.14 (1H, dd, J = 8.6, 6.3 Hz), 6.56 (1H, t, J = 9.0 Hz), 2.81 (3H, s), 2.39 (3H, s), 2.14 (3H, d, J = 1.9 Hz), 1.69 (6H, s).
479 ¹H-NMR (CDCl₃) δ: 8.54-8.53 (1H, m), 8.46 (1H, d, J = 2.1 Hz), 8.33 (1H, s), 7.14-7.11 (1H, m), 6.70-6.65 (1H, m), 2.42 (3H, d, J = 1.1 Hz), 1.75 (6H, s).
480 ¹H-NMR (CDCl₃) δ: 8.48 (1H, d, J = 4.2 Hz), 8.41 (1H, s), 7.13 (1H, ddd, J = 8.8, 5.5, 2.1 Hz), 6.81 (1H, dd, J = 4.2, 0.8 Hz), 6.67 (1H, ddd, J = 12.9, 5.8, 3.9 Hz), 2.85 (3H, d, J = 0.8 Hz), 1.75 (6H, s).
481 ¹H-NMR (CDCl₃) δ: 8.38 (1H, s), 8.34 (1H, s), 7.14-7.11 (1H, m), 6.69-6.64 (1H, m), 2.82 (3H, s), 2.41 (3H, s), 1.75 (6H, s).
482 ¹H-NMR (CDCl₃) δ: 8.15 (1H, s), 7.87 (1H, d, J = 8.8 Hz), 7.33-7.29 (1H, m), 7.18 (1H, d, J = 9.0 Hz), 6.77-6.71 (1H, m), 2.78 (3H, s), 2.41 (3H, s), 1.60-1.51 (2H, m), 2.01-1.96 (2H, m), 1.66 (3H, s), 1.35 (2H, dd, J = 14.9, 7.3 Hz), 0.91 (3H, t, J = 7.3 Hz).
483 ¹H-NMR (CDCl₃) δ: 8.41-8.39 (1H, m), 7.97 (1H, s), 6.99-6.97 (1H, m), 6.90 (1H, ddd, J = 8.8, 5.6, 2.2 Hz), 6.79 (1H, t, J = 7.0 Hz), 6.67-6.60 (1H, m), 2.39 (3H, s), 2.04-2.00 (2H, m), 1.67 (3H, s), 1.59-1.47 (2H, m), 1.41-1.33 (2H, m), 0.91 (3H, t, J = 7.3 Hz).
484 ¹H-NMR (CDCl₃) δ: 8.21 (1H, s), 7.97 (1H, d, J = 8.8 Hz), 7.30-7.27 (2H, m), 6.80-6.72 (2H, m), 2.80 (3H, s), 2.01-1.98 (2H, m), 1.66 (3H, s), 1.62-1.50 (2H, m), 1.39-1.32 (2H, m), 0.91 (3H, t, J = 7.3 Hz).
485 ¹H-NMR (CDCl₃) δ: 8.54-8.53 (1H, m), 8.18 (1H, s), 8.06 (1H, dt, J = 9.2, 1.1 Hz), 7.33-7.28 (2H, m), 6.92 (1H, td, J = 5.1, 1.5 Hz), 6.78-6.73 (1H, m), 2.01-1.98 (2H, m), 1.66 (3H, s), 1.61-1.51 (2H, m), 1.40-1.33 (2H, m), 0.92 (3H, t, J = 7.3 Hz).
486 ¹H-NMR (CDCl₃) δ: 8.16 (1H, s), 7.29 (1H, d, J = 8.5 Hz), 7.11 (1H, d, J = 8.5 Hz), 7.08-7.02 (1H, m), 6.63 (1H, t, J = 8.8 Hz), 2.63 (3H, s), 2.41 (3H, s), 2.18 (3H, d, J = 2.0 Hz), 2.02 (2H, q, J = 7.4 Hz), 1.63 (3H, s), 1.07 (3H, t, J = 7.4 Hz).
487 ¹H-NMR (CDCl₃) δ: 8.16 (1H, s), 7.28 (1H, d, J = 8.3 Hz), 7.11 (1H, d, J = 8.3 Hz), 7.07-7.03 (1H, m), 6.63 (1H, t, J = 8.8 Hz), 2.63 (3H, s), 2.41 (3H, s), 2.18 (3H, d, J = 1.7 Hz), 1.96 (2H, t, J = 8.3 Hz), 1.63 (3H, s), 1.60-1.48 (2H, m), 0.96 (3H, t, J = 7.4 Hz).
488 ¹H-NMR (CDCl₃) δ: 8.16 (1H, s), 7.28 (1H, d, J = 8.3 Hz), 7.11 (1H, d, J = 8.3 Hz), 7.05 (1H, dd, J = 8.9, 6.1 Hz), 6.63 (1H, t, J = 8.9 Hz), 2.63 (3H, s), 2.41 (3H, s), 2.18 (3H, d, J = 1.7 Hz), 1.99-1.95 (2H, m), 1.63 (3H, s), 1.57-1.44 (2H, m), 1.38-1.32 (2H, m), 0.91 (3H, t, J = 7.3 Hz).
489 ¹H-NMR (CDCl₃) δ: 8.16 (1H, s), 7.28 (1H, d, J = 8.3 Hz), 7.13 (1H, d, J = 8.3 Hz), 7.04-7.00 (1H, m), 6.79-6.72 (1H, m), 2.63 (3H, s), 2.41 (3H, s), 2.06 (2H, q, J = 7.4 Hz), 1.68 (3H, s), 1.08 (3H, t, J = 7.4 Hz).
490 ¹H-NMR (CDCl₃) δ: 8.33-8.33 (1H, m), 8.12 (1H, s), 7.96 (1H, d, J = 8.3 Hz), 7.31 (1H, ddd, J = 8.8, 5.6, 2.2 Hz), 7.17 (1H, dd, J = 9.0, 1.5 Hz), 6.79-6.71 (1H, m), 2.39 (3H, d, J = 1.0 Hz), 2.01-1.95 (2H, m), 1.65 (3H, s), 1.62-1.49 (2H, m), 1.40-1.32 (2H, m), 0.91 (3H, t, J = 7.2 Hz).
491 ¹H-NMR (CDCl₃) δ: 9.35 (1H, s), 8.22 (1H, s), 7.31 (1H, ddd, J = 8.9, 5.4, 2.1 Hz), 6.80-6.74 (1H, m), 2.81 (3H, s), 2.65 (3H, s), 2.02-1.98 (2H, m), 1.66 (3H, s), 1.58-1.45 (2H, m), 1.38-1.32 (2H, m), 0.91 (3H, t, J = 5.4 Hz).
492 ¹H-NMR (CDCl₃) δ: 8.31 (1H, dd, J = 4.8, 0.6 Hz), 8.04 (1H, s), 7.87 (1H, d, J = 4.6 Hz), 6.85 (1H, ddd, J = 8.7, 5.6, 2.1 Hz), 6.69-6.63 (1H, m), 2.68 (3H, d, J = 0.5 Hz), 2.06-2.01 (2H, m), 1.69 (3H, s), 1.62-1.47 (2H, m), 1.41-1.33 (2H, m), 0.92 (3H, t, J = 7.1 Hz).
493 ¹H-NMR (CDCl₃) δ: 8.16 (1H, s), 7.88 (1H, d, J = 9.2 Hz), 7.33-7.30 (1H, m), 7.17 (1H, d, J = 9.2 Hz), 6.77-6.71 (1H, m), 2.78 (3H, s), 2.41 (3H, s), 2.03 (2H, q, J = 7.4 Hz), 1.65 (3H, s), 1.10 (3H, t, J = 7.4 Hz).
494 ¹H-NMR (CDCl₃) δ: 8.19 (1H, s), 7.90 (1H, d, J = 9.2 Hz), 7.35-7.34 (1H, m), 7.21-7.16 (2H, m), 6.89 (1H, td, J = 7.0, 4.8 Hz), 2.78 (3H, s), 2.41 (3H, s), 1.99-1.95 (2H, m), 1.68-1.53 (2H, m), 1.66 (3H, s), 0.95 (3H, t, J = 5.9 Hz).
495 ¹H-NMR (CDCl₃) δ: 8.41-8.39 (1H, m), 7.99 (1H, s), 7.19-7.15 (1H, m), 6.97-6.96 (1H, m), 6.92 (1H, dt, J = 7.9, 1.2 Hz), 6.81-6.76 (2H, m), 2.39 (3H, s), 2.03-1.98 (2H, m), 1.67 (3H, s), 1.66-1.52 (2H, m), 0.96 (3H, t, J = 7.4 Hz).
496 ¹H-NMR (CDCl₃) δ: 8.47 (1H, d, J = 4.2 Hz), 8.40 (1H, s), 7.14-7.10 (1H, m), 6.80 (1H, dd, J = 4.2, 0.8 Hz), 6.67-6.62 (1H, m), 2.85 (3H, d, J = 0.8 Hz), 2.03-2.00 (2H, m), 1.68 (3H, s), 1.61-1.47 (2H, m), 1.39-1.32 (2H, m), 0.91 (3H, t, J = 7.4 Hz).
497 ¹H-NMR (CDCl₃) δ: 8.25 (1H, s), 8.00 (1H, d, J = 8.8 Hz), 7.34-7.33 (1H, m), 7.28-7.25 (1H, m), 7.22-7.18 (1H, m), 6.90 (1H, td, J = 7.0, 5.0 Hz), 6.79-6.78 (1H, m), 2.81 (3H, s), 2.01-1.96 (2H, m), 1.67 (3H, s), 1.65-1.55 (2H, m), 0.96 (3H, t, J = 7.4 Hz).
498 ¹H-NMR (CDCl₃) δ: 8.54 (1H, dt, J = 7.0, 1.0 Hz), 8.22 (1H, s), 8.09 (1H, dt, J = 8.8, 1.1 Hz), 7.34-7.29 (2H, m), 7.22-7.18 (1H, m), 6.93-6.89 (2H, m), 1.99-1.96 (2H, m), 1.67-1.56 (2H, m), 1.66 (3H, s), 0.96 (3H, t, J = 7.4 Hz).
499 ¹H-NMR (CDCl₃) δ: 9.37 (1H, s), 8.26 (1H, s), 7.33 (1H, dt, J = 7.8, 1.2 Hz), 7.24-7.19 (1H, m), 6.92 (1H, td, J = 6.9, 4.6 Hz), 2.81 (3H, d, J = 0.7 Hz), 2.65 (3H, s), 2.01-1.96 (2H, m), 1.68-1.51 (2H, m), 1.65 (3H, s), 0.95 (3H, t, J = 7.8 Hz).
500 ¹H-NMR (CDCl₃) δ: 8.32-8.30 (1H, m), 8.06 (1H, s), 7.86 (1H, d, J = 4.9 Hz), 7.23-7.18 (1H, m), 6.87-6.78 (2H, m), 2.68 (3H, d, J = 0.5 Hz), 2.04-1.99 (2H, m), 1.69 (3H, s), 1.66-1.52 (2H, m), 0.97 (3H, t, J = 7.3 Hz).
501 ¹H-NMR (CDCl₃) δ: 8.54-8.53 (1H, m), 8.45 (1H, d, J = 2.1 Hz), 8.32 (1H, s), 7.14-7.10 (1H, m), 6.67-6.62 (1H, m), 2.42 (3H, d, J = 0.8 Hz), 2.02-1.99 (2H, m), 1.68 (3H, s), 1.60-1.48 (2H, m), 1.39-1.33 (2H, m), 0.91 (3H, t, J = 7.3 Hz).
502 ¹H-NMR (CDCl₃) δ: 8.37 (1H, s), 8.34 (1H, s), 7.14-7.11 (1H, m), 6.66-6.61 (1H, m), 2.81 (3H, s), 2.40 (3H, s), 2.02-1.99 (2H, m), 1.68 (3H, s), 1.61-1.48 (2H, m), 1.39-1.31 (2H, m), 0.91 (3H, t, J = 7.3 Hz).
503 ¹H-NMR (CDCl₃) δ: 8.52-8.51 (1H, m), 8.44-8.43 (1H, m), 8.31 (1H, d, J = 1.1 Hz), 7.14-7.11 (1H, m), 6.55 (1H, t, J = 8.8 Hz), 2.41 (3H, s), 2.14 (3H, s), 1.97-1.94 (2H, m), 1.63 (3H, s), 1.59-1.46 (2H, m), 1.37-1.30 (2H, m), 0.91 (3H, t, J = 7.3 Hz).
504 ¹H-NMR (CDCl₃) δ: 8.38 (1H, s, ), 8.31 (1H, d, J = 5.3 Hz, ), 6.85 (1H, dd, J = 8.4, 5.7 Hz, ), 6.56 (1H, t, J = 9.0 Hz, ), 2.74 (3H, s, ), 2.13 (3H, d, J = 1.9 Hz, ), 1.69 (6H, s, ).
505 1H-NMR (CDCl₃) δ: 8.39 (1H, s), 8.30 (1H, s), 7.25-7.20 (1H, m), 6.84-6.77 (2H, m), 2.75 (3H, s), 2.06-1.95 (2H, m), 1.67 (3H, s), 1.55-1.41 (2H, m), 1.37-1.30 (2H, m), 0.88 (3H, t, J = 7.6 Hz).
506 ¹H-NMR (CDCl₃) δ: 8.22 (1H, s, ), 8.05 (1H, s, ), 6.85 (1H, dd, J = 8.8, 6.1 Hz, ), 6.49 (1H, t, J = 8.8 Hz, ), 2.56 (3H, s, ), 2.30 (3H, s, ), 2.08 (3H, d, J = 1.9 Hz, ), 1.64 (6H, s, ).
507 ¹H-NMR (CDCl₃) δ: 8.22 (1H, s, ), 8.06 (1H, s, ), 7.18-7.14 (1H, m, ), 6.83 (1H, dt, J = 7.9, 1.2 Hz, ), 6.77-6.72 (1H, m, ), 2.58 (3H, s, ), 2.32 (3H, s, ), 1.97-1.93 (2H, m, ), 1.62 (3H, s, ), 1.48-1.38 (2H, m, ), 1.33-1.27 (2H, m, ), 0.84 (3H, t, J = 7.6 Hz, ).
508 ¹H-NMR (CDCl₃) δ: 8.46 (1H, d, J = 4.2 Hz), 8.39 (1H, s), 7.13 (1H, dd, J = 8.4, 6.5 Hz), 6.78 (1H, dd, J = 4.2, 1.1 Hz), 6.55 (1H, t, J = 8.8 Hz), 2.84 (3H, d, J = 0.8 Hz), 2.14 (3H, d, J = 1.9 Hz), 2.02-1.94 (2H, m), 1.64 (3H, s), 1.61-1.45 (2H, m), 1.38-1.31 (2H, m), 0.91 (3H, t, J = 7.4 Hz).
509 ¹H-NMR (CDCl₃) δ: 8.37 (1H, s), 8.33 (1H, s), 7.13 (1H, ddd, J = 8.7, 6.5, 0.5 Hz), 6.53 (1H, t, J = 8.9 Hz), 2.81 (3H, d, J = 0.5 Hz), 2.39 (3H, s), 2.13 (3H, d, J = 2.0 Hz), 1.99-1.94 (2H, m), 1.63 (3H, s), 1.58-1.46 (2H, m), 1.39-1.31 (2H, m), 0.90 (3H, t, J = 7.3 Hz).
510 ¹H-NMR (CDCl₃) δ: 8.52-8.51 (1H, m), 8.44 (1H, d, J = 2.2 Hz), 8.31 (1H, s), 7.13 (1H, ddd, J = 8.7, 6.4, 0.5 Hz), 6.55 (1H, t, J = 8.9 Hz), 2.41 (3H, d, J = 1.0 Hz), 2.13 (3H, d, J = 2.0 Hz), 1.97-1.93 (2H, m), 1.63 (3H, s), 1.62-1.50 (2H, m), 0.95 (3H, t, J = 7.4 Hz).
511 ¹H-NMR (CDCl₃) δ: 8.46 (1H, d, J = 4.2 Hz), 8.39 (1H, s), 7.13 (1H, ddd, J = 8.7, 6.5, 0.6 Hz), 6.78 (1H, dd, J = 4.2, 0.7 Hz), 6.55 (1H, t, J = 8.9 Hz), 2.84 (3H, d, J = 1.0 Hz), 2.14 (3H, d, J = 2.0 Hz), 1.98-1.93 (2H, m), 1.66-1.49 (2H, m), 1.63 (3H, s), 0.95 (3H, t, J = 7.4 Hz).
512 ¹H-NMR (CDCl₃) δ: 8.19 (1H, s), 7.91 (1H, d, J = 9.0 Hz), 7.34 (1H, dt, J = 7.8, 1.2 Hz), 7.21-7.14 (2H, m), 6.89 (1H, td, J = 6.9, 4.6 Hz), 2.78 (3H, s), 2.41 (3H, s), 2.09-2.01 (1H, m), 1.90 (2H, d, J = 5.9 Hz), 1.69 (3H, s), 1.01-0.99 (6H, m).
513 ¹H-NMR (CDCl₃) δ: 8.25 (1H, s), 8.01 (1H, d, J = 8.8 Hz), 7.34-7.32 (1H, m), 7.28-7.24 (1H, m), 7.21-7.17 (1H, m), 6.90 (1H, td, J = 7.0, 5.0 Hz), 6.78 (1H, d, J = 6.9 Hz), 2.81 (3H, s), 2.10-2.04 (1H, m), 1.91 (2H, d, J = 6.1 Hz), 1.70 (3H, s), 1.00 (6H, dd, J = 6.7, 2.5 Hz).
514 ¹H-NMR (CDCl₃) δ: 8.25 (1H, s), 8.01 (1H, d, J = 8.8 Hz), 7.34-7.32 (1H, m), 7.28-7.24 (1H, m), 7.21-7.17 (1H, m), 6.90 (1H, td, J = 7.0, 5.0 Hz), 6.78 (1H, d, J = 6.9 Hz), 2.81 (3H, s), 2.10-2.04 (1H, m), 1.91 (2H, d, J = 6.1 Hz), 1.70 (3H, s), 1.00 (6H, dd, J = 6.7, 2.5 Hz).
515 ¹H-NMR (CDCl₃) δ: 8.54 (1H, d, J = 7.6 Hz), 8.22 (1H, s), 8.11-8.09 (1H, m), 7.35-7.29 (2H, m), 7.22-7.18 (1H, m), 6.93-6.88 (2H, m), 2.10-2.02 (1H, m), 1.91 (2H, d, J = 5.7 Hz), 1.70 (3H, s), 1.00 (6H, dd, J = 6.7, 2.9 Hz).
516 ¹H-NMR (CDCl₃) δ: 9.37 (1H, s), 8.26 (1H, s), 7.33 (1H, dt, J = 7.8, 1.2 Hz), 7.24-7.19 (1H, m), 6.92 (1H, td, J = 6.9, 4.6 Hz), 2.81 (3H, s), 2.65 (3H, s), 2.09-2.01 (1H, m), 1.92 (2H, dd, J = 6.2, 2.1 Hz), 1.68 (3H, s), 0.99 (6H, d, J = 6.6 Hz).
517 ¹H-NMR (CDCl₃) δ: 8.31 (1H, dd, J = 4.8, 0.6 Hz), 8.06 (1H, s), 7.85 (1H, d, J = 4.6 Hz), 7.22-7.16 (1H, m), 6.85-6.78 (2H, m), 2.67 (3H, s), 2.09-2.03 (1H, m), 2.00-1.89 (2H, m), 1.73 (3H, s), 1.01 (6H, d, J = 6.6 Hz).
518 ¹H-NMR (CDCl₃) δ: 8.15 (1H, s), 7.28 (1H, d, J = 8.5 Hz), 7.13 (1H, d, J = 8.5 Hz), 7.03-7.00 (1H, m), 6.78-6.72 (1H, m), 2.63 (3H, s), 2.41 (3H, s), 2.02-1.97 (2H, m), 1.69 (3H, s), 1.63-1.50 (2H, m), 0.96 (3H, t, J = 7.3 Hz).
519 ¹H-NMR (CDCl₃) δ: 8.15 (1H, s), 7.28 (1H, d, J = 8.3 Hz), 7.13 (1H, d, J = 8.3 Hz), 7.03-7.00 (1H, m), 6.77-6.73 (1H, m), 2.63 (3H, s), 2.42 (3H, s), 2.02-1.99 (2H, m), 1.69 (3H, s), 1.55-1.43 (2H, m), 1.37-1.34 (2H, m), 0.91 (3H, t, J = 7.3 Hz).
520 ¹H-NMR (CDCl₃) δ: 8.18 (1H, s), 7.34 (1H, d, J = 8.3 Hz), 7.30-7.25 (1H, m), 7.13 (1H, d, J = 8.3 Hz), 7.05-7.03 (1H, m), 6.90 (1H, td, J = 8.0, 4.4 Hz), 2.63 (3H, s), 2.42 (3H, s), 2.05 (2H, t, J = 7.4 Hz), 1.68 (3H, s), 1.09 (3H, t, J = 7.4 Hz).
521 ¹H-NMR (CDCl₃) δ: 8.18 (1H, s), 7.33 (1H, d, J = 8.0 Hz), 7.29-7.25 (1H, m), 7.13 (1H, d, J = 8.0 Hz), 7.04-7.03 (1H, m), 6.90 (1H, td, J = 7.8, 4.6 Hz), 2.63 (3H, s), 2.42 (3H, s), 2.02-1.98 (2H, m), 1.68 (3H, s), 1.63-1.52 (2H, m), 0.96 (3H, t, J = 7.4 Hz).
522 ¹H-NMR (CDCl₃) δ: 8.18 (1H, s), 7.33 (1H, d, J = 8.4 Hz), 7.29-7.27 (1H, m), 7.13 (1H, d, J = 8.4 Hz), 7.04-7.03 (1H, m), 6.90 (1H, td, J = 7.8, 4.7 Hz), 2.63 (3H, s), 2.42 (3H, s), 2.04-2.01 (2H, m), 1.68 (3H, s), 1.56-1.46 (2H, m), 1.39-1.33 (2H, m), 0.90 (3H, t, J = 7.4 Hz).
523 ¹H-NMR (CDCl₃) δ: 8.37 (1H, dd, J = 4.4, 1.7 Hz, ), 8.04 (1H, dd, J = 9.6, 2.1 Hz, ), 8.02 (1H, s, ), 7.20 (1H, ddd, J = 10.3, 7.9, 1.9 Hz, ), 7.13 (1H, dd, J = 9.3, 4.4 Hz, ), 6.87-6.78 (2H, m, ), 1.79 (6H, s, ).
524 ¹H-NMR (CDCl₃) δ: 8.36 (1H, dd, J = 4.5, 1.6 Hz, ), 8.04 (1H, dd, J = 9.3, 1.7 Hz, ), 8.02 (1H, s, ), 7.21-7.16 (1H, m, ), 7.13 (1H, dd, J = 9.3, 4.4 Hz, ), 6.84-6.75 (2H, m, ), 2.07-2.01 (2H, m, ), 1.72 (3H, s, ), 1.68-1.49 (2H, m, ), 1.42-1.32 (2H, m, ), 0.92 (3H, t, J = 7.3 Hz, ).
525 ¹H-NMR (CDCl₃) δ: 8.23 (1H, d, J = 4.6 Hz, ), 7.96 (1H, s, ), 7.19-7.15 (1H, m, ), 6.94-6.92 (1H, m, ), 6.82-6.75 (2H, m, ), 2.72 (3H, d, J = 0.8 Hz, ), 2.06-2.02 (2H, m, ), 1.72 (3H, s, ), 1.63-1.52 (2H, m, ), 1.41-1.32 (2H, m, ), 0.91 (3H, t, J = 7.3 Hz, ).
526 ¹H-NMR (CDCl₃) δ: 8.22 (1H, d, J = 4.4 Hz), 7.95 (1H, s), 6.92 (1H, dq, J = 4.6, 1.0 Hz), 6.83 (1H, dd, J = 8.8, 6.3 Hz), 6.53 (1H, t, J = 8.9 Hz), 2.71 (3H, d, J = 1.0 Hz), 2.15 (3H, d, J = 1.7 Hz), 1.73 (6H, s).
527 ¹H-NMR (CDCl₃) δ: 8.37 (1H, s), 8.32 (1H, s), 7.14-7.12 (1H, m), 6.53 (1H, t, J = 9.0 Hz), 2.81 (3H, s), 2.39 (3H, s), 2.13 (3H, d, J = 1.5 Hz), 1.98-1.92 (2H, m), 1.63 (3H, s), 1.60-1.49 (2H, m), 0.94 (3H, t, J = 7.4 Hz).
528 ¹H-NMR (CDCl₃) δ: 8.46 (1H, d, J = 4.4 Hz), 8.39 (1H, s), 7.13 (1H, dd, J = 8.7, 6.5 Hz), 6.78 (1H, d, J = 2.1 Hz), 6.55 (1H, t, J = 8.9 Hz), 2.84 (3H, s), 2.14 (3H, d, J = 1.7 Hz), 2.05-1.96 (2H, m), 1.63 (3H, s), 1.06 (3H, t, J = 7.4 Hz).
529 ¹H-NMR (CDCl₃) δ: 8.37 (1H, s), 8.33 (1H, s), 7.13 (1H, dd, J = 8.2, 6.3 Hz), 6.54 (1H, t, J = 9.0 Hz), 2.81 (3H, s), 2.40 (3H, s), 2.14 (3H, d, J = 1.9 Hz), 2.04-1.99 (2H, m), 1.63 (3H, s), 1.06 (3H, t, J = 7.4 Hz).
530 ¹H-NMR (CDCl₃) δ: 8.38 (1H, s), 8.34 (1H, s), 7.14-7.11 (1H, m), 6.67-6.59 (1H, m), 2.82 (3H, s), 2.41 (3H, s), 2.07-2.02 (2H, m), 1.68 (3H, s), 1.08 (3H, t, J = 7.4 Hz).
531 ¹H-NMR (CDCl₃) δ: 8.15 (1H, s), 7.85 (1H, d, J = 9.2 Hz), 7.36-7.34 (1H, m), 7.14 (1H, d, J = 9.2 Hz), 6.63 (1H, t, J = 8.8 Hz), 2.77 (3H, s), 2.40 (3H, s), 2.16 (3H, d, J = 1.5 Hz), 1.94-1.92 (2H, m), 1.64-1.55 (5H, m), 0.95 (3H, t, J = 7.3 Hz).
532 ¹H-NMR (CDCl₃) δ: 8.21 (1H, s), 7.95 (1H, d, J = 9.0 Hz), 7.34 (1H, dd, J = 8.7, 6.5 Hz), 7.23-7.22 (1H, m), 6.76 (1H, d, J = 6.5 Hz), 6.63 (1H, t, J = 9.0 Hz), 2.80 (3H, s), 2.16 (3H, s), 1.96-1.92 (2H, m), 1.67-1.50 (5H, m), 0.95 (3H, t, J = 7.3 Hz).
533 ¹H-NMR (CDCl₃) δ: 8.32 (1H, br s), 8.11 (1H, s), 7.93 (1H, d, J = 9.2 Hz), 7.35 (1H, dd, J = 8.4, 6.1 Hz), 7.13 (1H, dd, J = 8.4, 1.9 Hz), 6.64 (1H, t, J = 9.0 Hz), 2.38 (3H, s), 2.16 (3H, d, J = 1.9 Hz), 1.92 (2H, t, J = 8.4 Hz), 1.61-1.60 (5H, m), 0.95 (3H, t, J = 7.3 Hz).
534 ¹H-NMR (CDCl₃) δ: 9.34 (1H, s), 8.22 (1H, s), 7.34 (1H, dd, J = 8.6, 6.3 Hz), 6.66 (1H, t, J = 8.6 Hz), 2.80 (3H, s), 2.64 (3H, s), 2.16 (3H, d, J = 1.9 Hz), 1.96-1.92 (2H, m), 1.64-1.63 (1H, m), 1.61 (3H, s), 1.54-1.53 (1H, m), 0.95 (3H, t, J = 7.4 Hz).
535 ¹H-NMR (CDCl₃) δ: 8.30 (1H, d, J = 4.6 Hz), 8.03 (1H, s), 7.84 (1H, d, J = 4.6 Hz), 6.88 (1H, dd, J = 8.8, 6.3 Hz), 6.55 (1H, t, J = 8.8 Hz), 2.67 (3H, s), 2.16 (3H, d, J = 1.9 Hz), 2.01-1.95 (2H, m), 1.65-1.49 (2H, m), 1.64 (3H, s), 0.97 (3H, t, J = 7.4 Hz).
536 ¹H-NMR (CDCl₃) δ: 8.18 (1H, s), 8.05 (1H, d, J = 8.8 Hz), 7.36 (1H, dt, J = 7.9, 1.2 Hz), 7.23-7.20 (2H, m), 6.95 (1H, td, J = 7.9, 4.6 Hz), 2.79 (3H, s), 2.69-2.63 (2H, m), 2.55-2.50 (2H, m), 2.42 (3H, s), 2.07-1.99 (2H, m).
537 ¹H-NMR (CDCl₃) δ: 8.41 (1H, d, J = 6.9 Hz), 7.99 (1H, s), 7.22-7.18 (1H, m), 7.03-6.99 (2H, m), 6.85 (1H, td, J = 6.9, 4.6 Hz), 6.81 (1H, t, J = 6.9 Hz), 2.72-2.66 (2H, m), 2.59-2.53 (2H, m), 2.50 (3H, s), 2.05-1.96 (2H, m).
538 ¹H-NMR (CDCl₃) δ: 8.16 (1H, s), 7.86 (1H, d, J = 9.0 Hz), 7.35 (1H, dd, J = 8.4, 6.5 Hz), 7.14 (1H, d, J = 9.0 Hz), 6.63 (1H, t, J = 8.4 Hz), 2.77 (3H, s), 2.40 (3H, s), 2.17 (3H, s), 1.98 (2H, q, J = 7.2 Hz), 1.60 (3H, s), 1.08 (3H, t, J = 7.2 Hz).
539 ¹H-NMR (CDCl₃) δ: 8.38 (1H, dt, J = 7.0, 0.9 Hz), 7.96 (1H, s), 6.97-6.93 (2H, m), 6.76 (1H, t, J = 7.0 Hz), 6.53 (1H, t, J = 8.9 Hz), 2.38 (3H, s), 2.16 (3H, d, J = 2.0 Hz), 2.09-1.92 (2H, m), 1.62 (3H, s), 1.07 (3H, t, J = 7.4 Hz).
540 ¹H-NMR (CDCl₃) δ: 8.21 (1H, s), 7.96 (1H, d, J = 8.8 Hz), 7.34 (1H, dd, J = 8.8, 6.9 Hz), 7.25-7.23 (1H, m), 6.76 (1H, d, J = 6.9 Hz), 6.64 (1H, t, J = 8.8 Hz), 2.80 (3H, s), 2.17 (3H, d, J = 1.9 Hz), 2.00-1.97 (2H, m), 1.61 (3H, s), 1.08 (3H, t, J = 7.4 Hz).
541 ¹H-NMR (CDCl₃) δ: 8.54-8.53 (1H, m, ), 8.46-8.45 (1H, m, ), 8.32 (1H, s, ), 7.18-7.14 (1H, m, ), 7.13-7.11 (1H, m, ), 6.80 (1H, td, J = 6.9, 4.6 Hz, ), 2.42 (3H, d, J = 0.8 Hz, ), 2.01-1.97 (2H, m, ), 1.69 (3H, s, ), 1.66-1.53 (2H, m, ), 0.95 (3H, t, J = 7.4 Hz, ).
542 ¹H-NMR (CDCl₃) δ: 8.48 (1H, d, J = 4.2 Hz), 8.40 (1H, s), 7.18-7.11 (2H, m), 6.82-6.77 (2H, m), 2.77 (3H, s), 2.01-1.99 (2H, m), 1.69 (3H, s), 1.68-1.53 (2H, m), 0.97-0.94 (3H, m).
543 ¹H-NMR (CDCl₃) δ: 8.37 (1H, s), 8.34 (1H, s), 7.14-7.10 (1H, m), 6.66-6.60 (1H, m), 2.81 (3H, s), 2.40 (3H, s), 2.01-1.97 (2H, m), 1.68 (3H, s), 1.65-1.53 (2H, m), 0.95 (3H, t, J = 7.3 Hz).
544 ¹H-NMR (CDCl₃) δ: 8.29 (1H, dd, J = 4.5, 1.7 Hz, ), 7.96 (1H, dd, J = 9.2, 1.5 Hz, ), 7.93 (1H, s, ), 7.04 (1H, dd, J = 9.2, 4.5 Hz, ), 6.78 (1H, dd, J = 8.8, 6.5 Hz, ), 6.48 (1H, t, J = 9.0 Hz, ), 2.09 (3H, d, J = 1.9 Hz, ), 1.66 (6H, s, ).
545 ¹H-NMR (CDCl₃) δ: 8.15 (1H, d, J = 4.6 Hz, ), 7.93 (1H, s, ), 7.16-7.12 (1H, m, ), 7.00 (1H, td, J = 7.1, 5.3 Hz, ), 6.90-6.88 (2H, m, ), 2.71 (3H, d, J = 1.1 Hz, ), 2.04-1.93 (2H, m, ), 1.67 (3H, s, ), 1.62-1.53 (2H, m, ), 1.37-1.29 (2H, m, ), 0.90 (3H, t, J = 7.3 Hz, ).
546 ¹H-NMR (CDCl₃) δ: 7.86 (1H, s), 7.76 (1H, q, J = 1.1 Hz), 7.14-7.10 (1H, m), 6.87-6.85 (1H, m), 6.74 (1H, td, J = 8.0, 4.6 Hz), 2.40 (3H, d, J = 0.8 Hz), 2.05-1.99 (1H, m), 1.96-1.89 (1H, m), 1.67 (3H, s), 1.65-1.57 (2H, m), 1.30 (2H, td, J = 12.4, 7.4 Hz), 0.86 (3H, t, J = 7.4 Hz).
547 ¹H-NMR (CDCl₃) δ: 8.49 (1H, s), 8.41 (1H, s), 7.19-7.16 (1H, m), 7.05-7.03 (1H, m), 6.80 (1H, td, J = 7.0, 5.0 Hz), 2.60 (3H, q, J = 3.6 Hz), 2.01-1.98 (2H, m), 1.69 (3H, s), 1.67-1.54 (2H, m), 0.95 (3H, t, J = 7.4 Hz).
548 ¹H-NMR (CDCl₃) δ: 8.54-8.53 (1H, m), 8.45-8.44 (1H, m), 8.32 (1H, s), 7.13-7.10 (1H, m), 6.66-6.62 (1H, m), 2.42 (3H, d, J = 1.1 Hz), 2.00-1.97 (2H, m), 1.68 (3H, s), 1.66-1.52 (2H, m), 0.95 (3H, t, J = 7.4 Hz).
549 ¹H-NMR (CDCl₃) δ: 8.47 (1H, d, J = 4.2 Hz), 8.40 (1H, s), 7.14-7.10 (1H, m), 6.80 (1H, dd, J = 4.2, 1.1 Hz), 6.67-6.62 (1H, m), 2.85 (3H, d, J = 0.8 Hz), 2.01-1.97 (2H, m), 1.69 (3H, s), 1.66-1.53 (2H, m), 0.96 (3H, t, J = 7.3 Hz).
550 ¹H-NMR (CDCl₃) δ: 8.50 (1H, s), 8.35 (1H, s), 7.82 (1H, t, J = 52.5 Hz), 7.21-7.17 (1H, m), 7.08 (1H, dt, J = 7.8, 1.2 Hz), 6.83 (1H, td, J = 7.0, 5.0 Hz), 2.59 (3H, t, J = 2.9 Hz), 1.75 (6H, s).
551 ¹H-NMR (CDCl₃) δ: 8.50 (1H, s), 8.41 (1H, s), 7.20 (1H, ddd, J = 10.3, 8.0, 1.5 Hz), 7.06 (1H, dt, J = 7.8, 1.2 Hz), 6.83 (1H, td, J = 6.9, 4.6 Hz), 2.61 (3H, q, J = 3.6 Hz), 1.75 (6H, s).
552 ¹H-NMR (CDCl₃) δ: 7.90 (1H, s, ), 7.21-7.17 (1H, m, ), 6.89 (1H, dt, J = 7.6, 1.1 Hz, ), 6.81 (1H, td, J = 6.9, 4.6 Hz, ), 2.70 (3H, d, J = 0.8 Hz, ), 2.43 (3H, d, J = 0.8 Hz, ), 1.78 (6H, s, ).
553 ¹H-NMR (CDCl₃) δ: 8.48 (1H, s), 8.40 (1H, s), 7.07 (1H, dd, J = 8.4, 6.5 Hz), 6.57 (1H, t, J = 8.8 Hz), 2.60 (3H, q, J = 3.6 Hz), 2.15 (3H, d, J = 1.9 Hz), 1.70 (6H, s).
554 ¹H-NMR (CDCl₃) δ: 8.48 (1H, s), 8.34 (1H, s), 7.82 (1H, t, J = 50.8 Hz), 7.09 (1H, dd, J = 8.5, 6.3 Hz), 6.57 (1H, t, J = 8.9 Hz), 2.58 (3H, t, J = 3.1 Hz), 2.15 (3H, d, J = 1.7 Hz), 1.69 (6H, s).
555 ¹H-NMR (CDCl₃) δ: 8.39 (1H, d, J = 6.9 Hz), 7.96 (1H, s), 6.95-6.93 (2H, m), 6.76 (1H, t, J = 6.9 Hz), 6.53 (1H, t, J = 8.8 Hz), 2.38 (3H, s), 2.15 (3H, s), 2.01-1.91 (2H, m), 1.62 (3H, s), 1.54 (2H, dd, J = 12.8, 5.9 Hz), 0.96 (3H, dd, J = 8.0, 6.9 Hz).
556 ¹H-NMR (CDCl₃) δ: 8.52 (1H, d, J = 6.9 Hz), 8.18 (1H, s), 8.04 (1H, d, J = 9.5 Hz), 7.35-7.34 (1H, m), 7.30-7.28 (1H, m), 6.89 (1H, t, J = 6.5 Hz), 6.64 (1H, t, J = 9.0 Hz), 2.16 (3H, d, J = 1.5 Hz), 1.94-1.92 (2H, m), 1.63-1.59 (5H, m), 0.96 (3H, t, J = 7.4 Hz).
557 ¹H-NMR (CDCl₃) δ: 8.53-8.52 (1H, m), 8.18 (1H, s), 8.06-8.04 (1H, m), 7.35 (1H, dd, J = 8.8, 6.3 Hz), 7.29-7.28 (1H, m), 6.89 (1H, td, J = 7.0, 1.5 Hz), 6.65 (1H, t, J = 8.8 Hz), 2.17 (3H, d, J = 1.9 Hz), 2.00-1.97 (2H, m), 1.61 (3H, s), 1.08 (3H, t, J = 7.4 Hz).
558 ¹H-NMR (CDCl₃) δ: 8.11 (1H, s, ), 7.89 (1H, s, ), 7.16 (1H, ddd, J = 10.4, 7.9, 1.7 Hz, ), 6.83 (1H, dq, J = 7.9, 0.9 Hz, ), 6.77 (1H, td, J = 6.8, 4.5 Hz, ), 2.61 (3H, s, ), 2.34 (3H, s, ), 1.75 (6H, s, ).
559 ¹H-NMR (CDCl₃) δ: 8.09 (1H, s, ), 7.87 (1H, s, ), 6.84-6.80 (1H, m, ), 6.51 (1H, t, J = 8.9 Hz, ), 2.61 (3H, d, J = 0.7 Hz, ), 2.33 (3H, s, ), 2.13 (3H, d, J = 2.0 Hz, ), 1.69 (6H, s, ).
560 ¹H-NMR (CDCl₃) δ: 8.49 (1H, s), 8.41 (1H, s), 7.04 (1H, ddd, J = 8.8, 5.5, 2.1 Hz), 6.68 (1H, ddd, J = 12.8, 5.9, 4.0 Hz), 2.61 (3H, q, J = 3.6 Hz), 1.75 (6H, s).
561 ¹H-NMR (CDCl₃) δ: 8.49 (1H, s), 8.41 (1H, s), 7.20-7.14 (1H, m), 7.05 (1H, dt, J = 7.8, 1.2 Hz), 6.84-6.78 (1H, m), 2.61 (3H, q, J = 3.4 Hz), 2.05 (2H, q, J = 7.5 Hz), 1.68 (3H, s), 1.09 (3H, t, J = 7.4 Hz).
562 ¹H-NMR (CDCl₃) δ: 8.49 (1H, s), 8.39 (1H, s), 7.21-7.17 (1H, m), 7.08 (1H, dt, J = 7.8, 1.2 Hz), 6.83 (1H, td, J = 6.9, 4.6 Hz), 2.95 (3H, s), 1.75 (6H, s).
563 ¹H-NMR (CDCl₃) δ: 8.41 (1H, s), 8.31 (1H, s), 7.25-7.23 (1H, m), 6.85-6.81 (2H, m), 2.77 (3H, s), 2.02-1.98 (2H, m), 1.69 (3H, s), 1.65-1.52 (2H, m), 0.96 (3H, t, J = 7.4 Hz).
564 ¹H-NMR (CDCl₃) δ: 8.28 (1H, s), 8.12 (1H, s), 6.93-6.89 (1H, m), 6.54 (1H, t, J = 8.9 Hz), 2.63 (3H, s), 2.37 (3H, s), 2.14 (3H, d, J = 2.0 Hz), 1.98-1.94 (2H, m), 1.64 (3H, s), 1.61-1.51 (2H, m), 0.95 (3H, t, J = 7.4 Hz).
565 ¹H-NMR (CDCl₃) δ: 8.21 (1H, s, ), 7.94 (1H, s, ), 7.77 (1H, s, ), 7.22-7.17 (1H, m, ), 6.87 (1H, d, J = 7.6 Hz, ), 6.81 (1H, td, J = 6.8, 4.5 Hz, ), 2.45 (3H, s, ), 1.77 (6H, s, ).
566 ¹H-NMR (CDCl₃) δ: 8.13 (1H, d, J = 2.0 Hz, ), 7.85 (1H, s, ), 7.70-7.67 (1H, m, ), 6.80 (1H, dd, J = 8.5, 6.3 Hz, ), 6.47 (1H, t, J = 8.9 Hz, ), 2.37 (3H, d, J = 1.2 Hz, ), 2.09 (3H, d, J = 2.0 Hz, ), 1.65 (6H, s, ).
567 ¹H-NMR (CDCl₃) δ: 8.21 (1H, d, J = 4.6 Hz, ), 7.95 (1H, s, ), 7.16 (1H, ddd, J = 10.4, 7.7, 2.0 Hz, ), 6.91 (1H, dd, J = 4.5, 1.1 Hz, ), 6.83-6.74 (2H, m, ), 2.69 (3H, d, J = 1.2 Hz, ), 1.76 (6H, s, ).
568 1H-NMR (CDCl₃) δ: 8.22 (1H, d, J = 4.6 Hz), 7.96 (1H, s), 7.17 (1H, ddd, J = 10.4, 7.9, 1.6 Hz), 6.94-6.92 (1H, m), 6.82-6.74 (1H, m), 2.72 (3H, d, J = 1.1 Hz), 2.06-2.01 (2H, m), 1.71 (3H, s), 1.58 (3H, s), 1.61-1.52 (2H, m), 1.40-1.32 (2H, m), 0.91 (3H, t, J = 7.4 Hz).
569 ¹H-NMR (CDCl₃) δ: 8.49 (1H, s), 8.34 (1H, s), 7.82 (1H, t, J = 52.5 Hz), 7.17 (1H, ddd, J = 10.4, 8.2, 1.5 Hz), 7.07 (1H, dt, J = 7.8, 1.2 Hz), 6.80 (1H, td, J = 6.9, 4.6 Hz), 2.58 (3H, t, J = 3.1 Hz), 2.01-1.97 (2H, m), 1.68 (3H, s), 1.66-1.49 (2H, m), 0.95 (3H, t, J = 7.3 Hz).
570 ¹H-NMR (CDCl₃) δ: 8.49 (1H, s), 8.41 (1H, s), 7.19-7.15 (1H, m), 7.04 (1H, dt, J = 8.0, 1.1 Hz), 6.80 (1H, td, J = 7.0, 5.0 Hz), 2.60 (3H, q, J = 3.4 Hz), 2.03-2.00 (2H, m), 1.68 (3H, s), 1.62-1.49 (2H, m), 1.39-1.31 (2H, m), 0.90 (3H, t, J = 7.4 Hz).
571 ¹H-NMR (CDCl₃) δ: 8.48 (1H, s), 8.39 (1H, s), 7.17 (1H, ddd, J = 10.4, 8.2, 1.4 Hz), 7.07 (1H, dt, J = 7.7, 1.2 Hz), 6.80 (1H, td, J = 6.9, 4.6 Hz), 2.95 (3H, s), 2.02-1.99 (2H, m), 1.68 (3H, s), 1.64-1.46 (2H, m), 1.36-1.33 (2H, m), 0.90 (3H, t, J = 7.3 Hz).
572 ¹H-NMR (CDCl₃) δ: 8.47 (1H, d, J = 4.2 Hz), 8.40 (1H, s), 7.18-7.16 (1H, m), 7.14-7.12 (1H, m), 6.82-6.78 (2H, m), 2.85 (3H, d, J = 1.0 Hz), 2.02-1.99 (2H, m), 1.69 (3H, s), 1.58-1.54 (2H, m), 1.32-1.29 (4H, m), 0.89-0.86 (3H, m).
573 ¹H-NMR (CDCl₃) δ: 8.38 (1H, s), 8.34 (1H, s), 7.18-7.11 (2H, m), 6.79 (1H, td, J = 6.9, 4.8 Hz), 2.81 (3H, s), 2.40 (3H, s), 2.02-1.97 (2H, m), 1.68 (3H, s), 1.65-1.49 (2H, m), 1.34-1.24 (4H, m), 0.89-0.85 (3H, m).
574 ¹H-NMR (CDCl₃) δ: 8.15 (1H, d, J = 4.6 Hz, ), 7.93 (1H, s, ), 7.43 (1H, dd, J = 7.1, 2.2 Hz, ), 7.03-6.97 (2H, m, ), 6.91-6.88 (1H, m, ), 2.70 (3H, d, J = 1.0 Hz, ), 1.74 (6H, s, ).
575 ¹H-NMR (CDCl₃) δ: 8.09 (1H, s), 7.87 (1H, s), 7.43 (1H, dd, J = 7.6, 1.5 Hz), 7.04-6.98 (2H, m), 2.62 (3H, s), 2.60 (3H, s), 1.73 (6H, s).
576 ¹H-NMR (CDCl₃) δ: 8.53-8.52 (1H, m), 8.45 (1H, d, J = 2.7 Hz), 8.32 (1H, s), 7.18-7.14 (1H, m), 7.12 (1H, dt, J = 7.8, 1.2 Hz), 6.80 (1H, td, J = 6.9, 4.6 Hz), 2.41 (3H, d, J = 1.1 Hz), 2.01-1.98 (2H, m), 1.68 (3H, s), 1.64-1.51 (2H, m), 1.32-1.29 (4H, m), 0.89-0.86 (3H, m).
577 ¹H-NMR (CDCl₃) δ: 8.38 (1H, s), 8.34 (1H, s), 7.18-7.12 (2H, m), 6.79 (1H, td, J = 6.9, 4.8 Hz), 2.81 (3H, d, J = 0.5 Hz), 2.40 (3H, s), 2.08-2.02 (2H, m), 1.68 (3H, s), 1.09 (3H, t, J = 7.4 Hz).
578 ¹H-NMR (CDCl₃) δ: 8.49 (1H, d, J = 4.2 Hz), 8.40 (1H, s), 7.39-7.35 (1H, m), 7.35-7.31 (1H, m), 6.91-6.85 (2H, m), 6.79 (1H, dd, J = 4.2, 0.8 Hz), 2.85 (3H, d, J = 0.8 Hz), 1.71 (6H, s).
579 ¹H-NMR (CDCl₃) δ: 8.48 (1H, d, J = 2.9 Hz), 8.40 (1H, s), 7.37-7.33 (1H, m), 7.31 (1H, dd, J = 7.6, 1.5 Hz), 6.90-6.85 (2H, m), 6.78 (1H, d, J = 4.2 Hz), 2.84 (3H, s), 2.04-1.97 (2H, m), 1.64 (3H, s), 1.08 (3H, t, J = 8.0 Hz).
580 ¹H-NMR (CDCl₃) δ: 8.52-8.51 (1H, m), 8.45 (1H, d, J = 1.9 Hz), 8.31 (1H, s), 7.19 (1H, d, J = 7.6 Hz), 6.72-6.70 (2H, m), 2.41 (3H, d, J = 1.5 Hz), 2.34 (3H, s), 1.68 (6H, s).
581 ¹H-NMR (CDCl₃) δ: 8.48 (1H, d, J = 3.1 Hz), 8.40 (1H, s), 7.19 (1H, d, J = 8.0 Hz), 6.78 (1H, dd, J = 4.2, 1.1 Hz), 6.72-6.69 (2H, m), 2.84 (3H, d, J = 0.8 Hz), 2.34 (3H, s), 1.69 (6H, s).
582 ¹H-NMR (CDCl₃) δ: 8.39 (1H, s), 8.33 (1H, s), 7.20 (1H, d, J = 7.8 Hz), 6.72-6.68 (2H, m), 2.81 (3H, s), 2.40 (3H, s), 2.34 (3H, s), 1.69 (6H, s).
583 ¹H-NMR (CDCl₃) δ: 7.33-7.30 (2H, m), 7.24-7.22 (1H, m), 7.14-7.12 (1H, m), 6.86-6.84 (1H, m), 6.79 (1H, t, J = 7.7 Hz), 2.43 (3H, d, J = 1.0 Hz), 2.24 (3H, s), 1.69 (6H, s).
584 ¹H-NMR (CDCl₃) δ: 8.48 (1H, d, J = 4.2 Hz), 7.32 (1H, d, J = 2.3 Hz), 7.24-7.22 (1H, m), 7.13 (1H, dd, J = 8.0, 1.1 Hz), 6.86-6.77 (2H, m), 2.84 (3H, d, J = 0.8 Hz), 2.24 (3H, s), 1.70 (6H, s).
585 ¹H-NMR (CDCl₃) δ: 8.52-8.52 (1H, m), 8.45 (1H, d, J = 2.2 Hz), 8.31 (1H, s), 7.37-7.33 (1H, m), 7.32-7.30 (1H, m), 6.89-6.85 (2H, m), 2.41 (3H, d, J = 1.0 Hz), 1.98-1.94 (2H, m), 1.64 (3H, s), 1.62-1.51 (2H, m), 1.33-1.27 (4H, m), 0.89-0.85 (3H, m).
586 ¹H-NMR (CDCl₃) δ: 8.41 (1H, s), 8.32 (1H, s), 7.25-7.23 (1H, m), 6.84-6.81 (2H, m), 2.77 (3H, s), 2.09-2.04 (2H, m), 1.69 (3H, s), 1.08 (3H, t, J = 7.4 Hz).
587 ¹H-NMR (CDCl₃) δ: 8.49 (1H, s), 8.28 (1H, d, J = 3.1 Hz), 7.58 (1H, dd, J = 8.6, 1.7 Hz), 7.45 (1H, dd, J = 8.6, 6.3 Hz), 2.78 (3H, s), 2.00-1.95 (2H, m), 1.67 (3H, s), 1.56-1.49 (2H, m), 0.97 (3H, t, J = 7.4 Hz).
588 ¹H-NMR (CDCl₃) δ: 8.49 (1H, s), 8.28 (1H, d, J = 3.1 Hz), 7.58 (1H, dd, J = 8.4, 1.9 Hz), 7.45 (1H, dd, J = 8.6, 6.3 Hz), 2.78 (3H, s), 2.00-1.97 (2H, m), 1.67 (3H, s), 1.55-1.43 (2H, m), 1.38-1.35 (2H, m), 0.92 (3H, t, J = 7.4 Hz).
589 ¹H-NMR (CDCl₃) δ: 8.40 (1H, s), 8.31 (1H, s), 6.88-6.84 (1H, m), 6.56 (1H, t, J = 8.9 Hz), 2.76 (3H, s), 2.15 (3H, d, J = 2.0 Hz), 1.99-1.97 (2H, m), 1.64 (3H, s), 1.52-1.49 (2H, m), 1.37-1.33 (2H, m), 0.91 (3H, t, J = 7.2 Hz).
590 ¹H-NMR (CDCl₃) δ: 8.29 (1H, s), 8.13 (1H, s), 7.23-7.21 (1H, m), 6.91-6.89 (1H, m), 6.81-6.79 (1H, m), 2.64 (3H, s), 2.38 (3H, s), 2.07-2.04 (2H, m), 1.69 (3H, s), 1.09 (3H, t, J = 7.6 Hz).
591 ¹H-NMR (CDCl₃) δ: 8.26 (1H, d, J = 3.4 Hz), 8.21 (1H, s), 7.57 (1H, dd, J = 8.7, 1.5 Hz), 7.52 (1H, dd, J = 8.7, 6.0 Hz), 2.66 (3H, s), 2.41 (3H, s), 1.97 (2H, dd, J = 9.4, 7.4 Hz), 1.66 (3H, s), 1.60-1.50 (2H, m), 0.96 (3H, t, J = 7.4 Hz).
592 ¹H-NMR (CDCl₃) δ: 8.26 (1H, d, J = 3.4 Hz), 8.21 (1H, s), 7.58-7.57 (1H, m), 7.53-7.51 (1H, m), 2.66 (3H, s), 2.41 (3H, s), 2.01-1.97 (2H, m), 1.66 (3H, s), 1.50-1.34 (4H, m), 0.92 (3H, dd, J = 9.9, 4.5 Hz).
593 ¹H-NMR (CDCl₃) δ: 8.28 (1H, s), 8.12 (1H, s), 6.91 (1H, dd, J = 9.0, 6.3 Hz), 6.54 (1H, t, J = 9.0 Hz), 2.63 (3H, s), 2.37 (3H, s), 2.14 (3H, d, J = 1.5 Hz), 1.97 (2H, dd, J = 9.0, 7.4 Hz), 1.64 (3H, s), 1.54-1.48 (2H, m), 1.36-1.34 (2H, m), 0.91 (3H, t, J = 7.6 Hz).
594 ¹H-NMR (CDCl₃) δ: 8.41 (1H, s), 8.32 (1H, s), 7.47-7.43 (1H, m), 7.02 (1H, dd, J = 8.0, 1.1 Hz), 6.96 (1H, d, J = 8.0 Hz), 6.89 (1H, td, J = 8.0, 1.1 Hz), 2.77 (3H, s), 1.72 (6H, s).
595 ¹H-NMR (CDCl₃) δ: 8.33 (1H, s), 8.24 (1H, d, J = 5.0 Hz), 7.45-7.42 (1H, m), 7.11 (1H, d, J = 5.0 Hz), 7.06 (1H, dd, J = 7.6, 1.5 Hz), 6.95-6.95 (1H, m), 6.89 (1H, td, J = 7.6, 1.1 Hz), 2.73 (3H, s), 1.72 (6H, s).
596 ¹H-NMR (CDCl₃) δ: 8.08 (1H, d, J = 4.4 Hz, ), 7.87 (1H, s, ), 7.31-7.25 (2H, m, ), 7.01-6.93 (2H, m, ), 6.82-6.81 (1H, m, ), 2.63 (3H, d, J = 1.0 Hz, ), 1.65 (6H, s, ).
597 ¹H-NMR (CDCl₃) δ: 8.06 (1H, d, J = 2.2 Hz, ), 7.83 (1H, s, ), 7.69-7.66 (1H, m, ), 7.32-7.25 (2H, m, ), 7.04-6.96 (2H, m, ), 2.35 (3H, s, ), 1.64 (6H, s, ).
598 ¹H-NMR (CDCl₃) δ: 8.03 (1H, s), 7.85 (1H, s), 7.34-7.30 (2H, m), 7.07-6.98 (2H, m), 2.60 (3H, s), 2.31 (3H, s), 1.69 (6H, s).
599 ¹H-NMR (CDCl₃) δ: 8.42 (1H, s), 8.32 (1H, s), 7.43 (1H, td, J = 7.6, 1.0 Hz), 7.02 (1H, dd, J = 7.6, 1.0 Hz), 6.95 (1H, dd, J = 7.6, 1.0 Hz), 6.87 (1H, td, J = 7.6, 1.0 Hz), 2.77 (3H, s), 2.01 (2H, q, J = 7.3 Hz), 1.64 (3H, s), 1.07 (3H, t, J = 7.3 Hz).
600 ¹H-NMR (CDCl₃) δ: 8.33 (1H, s), 8.24 (1H, d, J = 5.0 Hz), 7.44-7.41 (1H, m), 7.11-7.11 (1H, m), 7.06 (1H, dd, J = 7.6, 1.0 Hz), 6.94 (1H, dd, J = 7.6, 1.0 Hz), 6.87 (1H, td, J = 7.6, 1.0 Hz), 2.73 (3H, s), 2.03-2.01 (2H, m), 1.65 (3H, s), 1.07 (3H, t, J = 7.4 Hz).
601 ¹H-NMR (CDCl₃) δ: 8.42 (1H, s), 8.31 (1H, s), 7.44-7.43 (1H, m), 7.01 (1H, dd, J = 7.6, 1.1 Hz), 6.94 (1H, dd, J = 7.6, 1.1 Hz), 6.87 (1H, td, J = 7.6, 1.1 Hz), 2.77 (3H, s), 1.98-1.93 (2H, m), 1.64 (3H, s), 1.62-1.49 (2H, m), 0.95 (3H, t, J = 7.3 Hz).
602 ¹H-NMR (CDCl₃) δ: 8.32 (1H, s), 8.24 (1H, d, J = 5.0 Hz), 7.43-7.41 (1H, m), 7.11 (1H, dd, J = 5.0, 0.8 Hz), 7.06-7.04 (1H, m), 6.93 (1H, dd, J = 8.2, 1.0 Hz), 6.87 (1H, td, J = 8.2, 1.0 Hz), 2.73 (3H, s), 1.98-1.95 (2H, m), 1.65 (3H, s), 1.63-1.50 (2H, m), 0.95 (3H, t, J = 7.4 Hz).
603 ¹H-NMR (CDCl₃) δ: 8.29 (1H, s), 8.13 (1H, s), 7.45-7.41 (1H, m), 7.07 (1H, dd, J = 7.6, 1.0 Hz), 6.95 (1H, dd, J = 7.6, 1.0 Hz), 6.88 (1H, td, J = 7.6, 1.0 Hz), 2.64 (3H, s), 2.38 (3H, s), 1.72 (6H, s).
604 ¹H-NMR (CDCl₃) δ: 8.29 (1H, s), 8.14 (1H, s), 7.43-7.40 (1H, m), 7.07 (1H, dd, J = 7.6, 1.0 Hz), 6.94-6.92 (1H, m), 6.86 (1H, td, J = 7.6, 1.0 Hz), 2.64 (3H, s), 2.38 (3H, s), 2.05-1.99 (2H, m), 1.65 (3H, s), 1.06 (3H, t, J = 7.5 Hz).
605 ¹H-NMR (CDCl₃) δ: 8.47 (1H, d, J = 2.9 Hz), 8.40 (1H, s), 7.37-7.30 (2H, m), 6.88-6.85 (2H, m), 6.78 (1H, dd, J = 4.2, 0.8 Hz), 2.84 (3H, d, J = 0.8 Hz), 2.01-1.93 (2H, m), 1.65 (3H, s), 1.63-1.51 (2H, m), 1.33-1.28 (4H, m), 0.87 (3H, t, J = 7.1 Hz).
606 ¹H-NMR (CDCl₃) δ: 8.22 (1H, d, J = 4.6 Hz), 7.96 (1H, s), 7.18-7.15 (1H, m), 6.92 (1H, dd, J = 4.6, 1.1 Hz), 6.81-6.74 (2H, m), 2.72 (3H, d, J = 1.1 Hz), 2.11-2.06 (1H, m), 1.96 (2H, d, J = 6.1 Hz), 1.75 (3H, s), 1.01 (6H, dd, J = 6.7, 2.5 Hz).
607 ¹H-NMR (CDCl₃) δ: 8.53-8.53 (1H, m), 8.45 (1H, d, J = 2.3 Hz), 8.32 (1H, s), 7.18-7.12 (2H, m), 6.81 (1H, td, J = 6.9, 4.6 Hz), 2.41 (3H, d, J = 0.8 Hz), 2.09-2.02 (1H, m), 1.92 (2H, d, J = 7.6 Hz), 1.73 (3H, s), 1.01 (6H, dd, J = 6.7, 1.0 Hz).
608 ¹H-NMR (CDCl₃) δ: 8.12 (1H, s), 7.90 (1H, s), 7.18-7.14 (1H, m), 6.82-6.81 (1H, m), 6.76 (1H, td, J = 6.8, 4.5 Hz), 2.63 (3H, s), 2.36 (3H, s), 2.10-2.05 (1H, m), 1.96-1.94 (2H, m), 1.75 (3H, s), 1.01 (6H, dd, J = 6.9, 2.7 Hz).
609 ¹H-NMR (CDCl₃) δ: 8.47 (1H, d, J = 4.2 Hz), 8.40 (1H, s), 7.13 (1H, ddd, J = 8.8, 5.5, 2.1 Hz), 6.80 (1H, dd, J = 4.2, 0.8 Hz), 6.68-6.67 (1H, m), 2.85 (3H, d, J = 0.8 Hz), 2.05 (2H, q, J = 7.4 Hz), 1.68 (3H, s), 1.09 (3H, t, J = 7.4 Hz).
610 ¹H-NMR (CDCl₃) δ: 8.54-8.53 (1H, m), 8.45-8.45 (1H, m), 8.33 (1H, s), 7.14-7.11 (1H, m), 6.65 (1H, ddd, J = 12.8, 5.9, 4.0 Hz), 2.42 (3H, d, J = 0.8 Hz), 2.04 (2H, q, J = 8.8 Hz), 1.68 (3H, s), 1.09 (3H, t, J = 7.4 Hz).
611 ¹H-NMR (CDCl₃) δ: 8.19 (1H, d, J = 2.3 Hz), 7.93 (1H, s), 7.76-7.75 (1H, m), 7.37-7.35 (1H, m), 7.03 (1H, dd, J = 7.6, 1.5 Hz), 6.89 (1H, dd, J = 8.2, 1.0 Hz), 6.84 (1H, td, J = 5.5, 1.1 Hz), 2.44 (3H, d, J = 1.1 Hz), 1.98 (2H, dd, J = 9.4, 7.4 Hz), 1.67 (3H, s), 1.65-1.51 (2H, m), 1.35-1.27 (4H, m), 0.88 (3H, t, J = 7.1 Hz).
612 ¹H-NMR (CDCl₃) δ: 8.22 (1H, d, J = 4.6 Hz), 7.95 (1H, s), 7.38-7.34 (1H, m), 7.00 (1H, dd, J = 7.6, 1.5 Hz), 6.92-6.88 (2H, m), 6.83 (1H, td, J = 5.3, 1.1 Hz), 2.71 (3H, d, J = 1.1 Hz), 1.99 (2H, dd, J = 8.8, 7.6 Hz), 1.67 (3H, s), 1.65-1.51 (2H, m), 1.34-1.29 (4H, m), 0.88 (3H, t, J = 7.1 Hz).
613 ¹H-NMR (CDCl₃) δ: 8.11 (1H, s), 7.90 (1H, d, J = 1.5 Hz), 7.37-7.33 (1H, m), 7.02 (1H, dd, J = 7.8, 1.7 Hz), 6.89 (1H, d, J = 9.2 Hz), 6.83 (1H, td, J = 5.3, 1.0 Hz), 2.63 (3H, s), 2.36 (3H, s), 1.98 (2H, dd, J = 9.2, 7.6 Hz), 1.66 (3H, s), 1.64-1.54 (2H, m), 1.33-1.26 (4H, m), 0.88 (3H, t, J = 7.1 Hz).
614 ¹H-NMR (CDCl₃) δ: 8.22 (1H, d, J = 4.6 Hz), 7.96 (1H, s), 7.38-7.34 (1H, m), 7.00 (1H, dd, J = 7.6, 1.5 Hz), 6.92-6.88 (2H, m), 6.83 (1H, td, J = 5.3, 1.0 Hz), 2.71 (3H, d, J = 1.1 Hz), 1.67 (3H, s), 2.00-1.97 (2H, m), 1.66-1.53 (2H, m), 0.95 (3H, t, J = 7.4 Hz).
615 ¹H-NMR (CDCl₃) δ: 8.12 (1H, s), 7.90 (1H, s), 7.37-7.33 (1H, m), 7.01 (1H, dd, J = 7.6, 1.5 Hz), 6.89-6.87 (1H, m), 6.83 (1H, td, J = 5.3, 1.0 Hz), 1.67 (3H, s), 2.63 (3H, s), 2.36 (3H, s), 1.99-1.96 (2H, m), 1.65-1.53 (2H, m), 0.94 (3H, t, J = 7.4 Hz).
616 ¹H-NMR (CDCl₃) δ: 8.51-8.50 (1H, m), 8.41 (1H, d, J = 2.0 Hz), 8.27 (1H, s), 7.40-7.34 (3H, m), 7.10 (1H, ddd, J = 8.3, 5.9, 2.0 Hz), 2.39 (3H, d, J = 1.0 Hz), 1.69 (6H, s).
617 ¹H-NMR (CDCl₃) δ: 8.20 (1H, d, J = 2.3 Hz), 7.92 (1H, s), 7.76-7.74 (1H, m), 7.37-7.34 (1H, m), 7.03 (1H, dd, J = 7.8, 1.7 Hz), 6.89 (1H, d, J = 8.0 Hz), 6.85-6.82 (1H, m), 2.44 (3H, d, J = 1.1 Hz), 1.99-1.96 (2H, m), 1.67 (3H, s), 1.65-1.55 (2H, m), 0.95 (3H, t, J = 7.4 Hz).
618 ¹H-NMR (CDCl₃) δ: 8.44 (1H, d, J = 4.2 Hz), 8.35 (1H, s), 7.40-7.32 (3H, m), 7.11-7.07 (1H, m), 6.76 (1H, dd, J = 4.3, 0.9 Hz), 2.83 (3H, d, J = 0.7 Hz), 1.70 (6H, s).
619 ¹H-NMR (CDCl₃) δ: 8.34 (1H, s), 8.28 (1H, s), 7.40-7.38 (1H, m), 7.35-7.33 (2H, m), 7.08 (1H, ddd, J = 8.3, 6.1, 1.7 Hz), 2.80 (3H, s), 2.38 (3H, s), 1.69 (6H, s).
620 ¹H-NMR (CDCl₃) δ: 8.33 (1H, s), 8.30 (1H, s), 7.43 (1H, ddd, J = 8.6, 7.0, 1.3 Hz), 7.02 (1H, dd, J = 8.3, 1.5 Hz), 6.94 (1H, dd, J = 8.3, 0.7 Hz), 6.87 (1H, td, J = 7.6, 1.1 Hz), 2.75 (3H, s), 1.96 (2H, dd, J = 9.0, 7.6 Hz), 1.65 (3H, s), 1.63-1.46 (2H, m), 0.95 (3H, t, J = 7.4 Hz).
621 ¹H-NMR (CDCl₃) δ: 8.19 (1H, d, J = 2.0 Hz), 7.91 (1H, s), 7.75-7.74 (1H, m), 7.23-7.21 (1H, m), 6.85 (1H, dd, J = 7.7, 1.1 Hz), 6.74 (1H, t, J = 7.6 Hz), 2.43 (3H, d, J = 1.2 Hz), 2.24 (3H, s), 2.00-1.95 (2H, m), 1.66 (3H, s), 1.64-1.51 (2H, m), 0.95 (3H, t, J = 7.4 Hz).
622 ¹H-NMR (CDCl₃) δ: 8.21 (1H, d, J = 4.6 Hz), 7.94 (1H, s), 7.23-7.21 (1H, m), 6.90-6.89 (1H, m), 6.83-6.81 (1H, m), 6.73 (1H, t, J = 7.6 Hz), 2.71 (3H, d, J = 1.2 Hz), 2.23 (3H, s), 2.01-1.97 (2H, m), 1.67 (3H, s), 1.65-1.55 (2H, m), 0.95 (3H, t, J = 7.3 Hz).
623 ¹H-NMR (CDCl₃) δ: 8.11 (1H, s), 7.88 (1H, s), 7.22-7.20 (1H, m), 6.83 (1H, d, J = 7.6 Hz), 6.72 (1H, t, J = 7.6 Hz), 2.63 (3H, s), 2.35 (3H, s), 2.23 (3H, s), 1.99-1.96 (2H, m), 1.66 (3H, s), 1.64-1.57 (2H, m), 0.95 (3H, t, J = 7.4 Hz).
624 ¹H-NMR (CDCl₃) δ: 8.37 (1H, s), 8.32 (1H, s), 7.21-7.19 (1H, m), 7.14-7.12 (1H, m), 6.75 (1H, t, J = 7.6 Hz), 2.80 (3H, s), 2.39 (3H, s), 2.22 (3H, s), 1.98-1.93 (2H, m), 1.63 (3H, s), 1.61-1.54 (2H, m), 0.94 (3H, t, J = 8.1 Hz).
625 ¹H-NMR (CDCl₃) δ: 8.35 (1H, s), 8.29 (1H, s), 7.28-7.23 (1H, m), 6.86-6.81 (2H, m), 2.75 (3H, s), 1.76 (6H, s).
626 ¹H-NMR (CDCl₃) δ: 8.34 (1H, s), 8.30 (1H, s), 7.45-7.42 (1H, m), 7.02 (1H, dd, J = 7.6, 1.5 Hz), 6.95 (1H, dd, J = 8.2, 1.0 Hz), 6.87 (1H, td, J = 5.3, 0.9 Hz), 2.75 (3H, s), 2.02 (2H, q, J = 8.4 Hz), 1.64 (3H, s), 1.07 (3H, t, J = 7.4 Hz).
627 ¹H-NMR (CDCl₃) δ: 8.22 (1H, d, J = 4.6 Hz), 7.96 (1H, s), 7.36 (1H, ddd, J = 8.6, 6.9, 1.2 Hz), 7.01 (1H, dd, J = 7.7, 1.3 Hz), 6.93-6.89 (2H, m), 6.83 (1H, td, J = 5.5, 1.2 Hz), 2.71 (3H, d, J = 1.0 Hz), 2.07-2.01 (2H, m), 1.67 (3H, s), 1.09 (3H, t, J = 7.4 Hz).
628 ¹H-NMR (CDCl₃) δ: 8.12 (1H, s), 7.90 (1H, s), 7.35 (1H, ddd, J = 8.6, 7.0, 1.3 Hz), 7.02 (1H, dd, J = 7.8, 1.5 Hz), 6.91-6.88 (1H, m), 6.83 (1H, td, J = 5.5, 1.1 Hz), 2.63 (3H, s), 2.35 (3H, s), 2.06-2.00 (2H, m), 1.66 (3H, s), 1.08 (3H, t, J = 7.4 Hz).
629 ¹H-NMR (CDCl₃) δ: 8.19 (1H, d, J = 2.3 Hz), 7.92 (1H, s), 7.75 (1H, s), 7.23-7.21 (1H, m), 6.86 (1H, d, J = 7.6 Hz), 6.74 (1H, t, J = 7.4 Hz), 2.43 (3H, d, J = 0.8 Hz), 2.24 (3H, s), 2.04-2.01 (2H, m), 1.66 (3H, s), 1.09 (3H, t, J = 7.4 Hz).
630 ¹H-NMR (CDCl₃) δ: 8.22 (1H, d, J = 4.2 Hz), 7.95 (1H, s), 7.23-7.21 (1H, m), 6.91-6.89 (1H, m), 6.83 (1H, dd, J = 7.6, 1.1 Hz), 6.73 (1H, t, J = 7.4 Hz), 2.71 (3H, d, J = 1.1 Hz), 2.24 (3H, s), 2.08-1.99 (2H, m), 1.67 (3H, s), 1.09 (3H, t, J = 7.4 Hz).
631 ¹H-NMR (CDCl₃) δ: 8.11 (1H, s), 7.89 (1H, s), 7.22-7.20 (1H, m), 6.84 (1H, dd, J = 8.2, 1.3 Hz), 6.73 (1H, t, J = 7.6 Hz), 2.63 (3H, s), 2.35 (3H, s), 2.24 (3H, s), 2.07-2.00 (2H, m), 1.66 (3H, s), 1.08 (3H, t, J = 9.0 Hz).
632 ¹H-NMR (CDCl₃) δ: 8.51-8.50 (1H, m), 8.44 (1H, d, J = 2.3 Hz), 8.30 (1H, s), 7.21 (1H, d, J = 7.3 Hz), 7.12 (1H, dd, J = 7.6, 1.1 Hz), 6.77 (1H, t, J = 7.4 Hz), 2.40 (3H, d, J = 1.1 Hz), 2.23 (3H, s), 2.04-1.96 (2H, m), 1.63 (3H, s), 1.07 (3H, t, J = 7.4 Hz).
633 ¹H-NMR (CDCl₃) δ: 8.47 (1H, d, J = 4.2 Hz), 8.39 (1H, s), 7.22-7.21 (1H, m), 7.12 (1H, dd, J = 7.8, 1.0 Hz), 6.78-6.73 (2H, m), 2.84 (3H, d, J = 0.8 Hz), 2.24 (3H, s), 2.06-1.97 (2H, m), 1.64 (3H, s), 1.08 (3H, t, J = 8.0 Hz).
634 ¹H-NMR (CDCl₃) δ: 8.34 (1H, s), 8.30 (1H, s), 7.45 (1H, ddd, J = 8.7, 7.0, 1.2 Hz), 7.03 (1H, dd, J = 7.6, 1.5 Hz), 6.97-6.95 (1H, m), 6.89 (1H, td, J = 5.5, 1.0 Hz), 2.75 (3H, s), 1.72 (6H, s).
635 ¹H-NMR (CDCl₃) δ: 8.21 (1H, d, J = 2.3 Hz), 7.94 (1H, s), 7.76-7.76 (1H, m), 7.39-7.37 (1H, m), 7.06 (1H, dd, J = 7.6, 1.5 Hz), 6.92 (1H, dd, J = 8.2, 1.0 Hz), 6.87 (1H, td, J = 5.5, 1.1 Hz), 2.44 (3H, d, J = 0.8 Hz), 1.72 (6H, s).
636 ¹H-NMR (CDCl₃) δ: 8.23 (1H, d, J = 4.6 Hz), 7.96 (1H, s), 7.39-7.37 (1H, m), 7.03 (1H, dd, J = 7.6, 1.5 Hz), 6.93-6.91 (2H, m), 6.86 (1H, td, J = 8.0, 1.0 Hz), 2.71 (3H, d, J = 0.8 Hz), 1.73 (6H, s).
637 ¹H-NMR (CDCl₃) δ: 8.12 (1H, s), 7.90 (1H, s), 7.39-7.35 (1H, m), 7.04 (1H, dd, J = 7.8, 1.7 Hz), 6.91 (1H, dd, J = 8.2, 1.0 Hz), 6.85 (1H, td, J = 5.5, 1.1 Hz), 2.63 (3H, s), 2.36 (3H, s), 1.72 (6H, s).
638 ¹H-NMR (CDCl₃) δ: 8.40 (1H, s), 8.31 (1H, s), 7.31-7.30 (1H, m), 6.84-6.77 (2H, m), 2.76 (3H, s), 2.25 (3H, s), 1.71 (6H, s).
639 ¹H-NMR (CDCl₃) δ: 8.28 (1H, s), 8.14 (1H, s), 7.42-7.40 (1H, m), 7.07-7.06 (1H, m), 6.92-6.91 (1H, m), 6.86-6.85 (1H, m), 2.64 (3H, s), 2.38 (3H, s), 1.97-1.95 (2H, m), 1.65 (3H, s), 1.60-1.52 (2H, m), 0.95 (3H, t, J = 7.4 Hz).
640 ¹H-NMR (CDCl₃) δ: 8.28 (1H, s), 8.13 (1H, s), 7.24-7.20 (1H, m), 6.90-6.89 (1H, m), 6.80-6.79 (1H, m), 2.64 (3H, s), 2.38 (3H, s), 2.02-1.98 (2H, m), 1.69 (3H, s), 1.62-1.53 (2H, m), 0.96 (3H, t, J = 7.5 Hz).
641 ¹H-NMR (CDCl₃) δ: 8.41 (1H, s), 8.31 (1H, s), 6.90 (1H, d, J = 8.0 Hz), 6.78 (1H, br s), 6.71-6.69 (1H, m), 2.76 (3H, s), 2.36 (3H, s), 1.70 (6H, s).
642 ¹H-NMR (CDCl₃) δ: 8.32 (1H, s), 8.24 (1H, d, J = 4.9 Hz), 7.10 (1H, dd, J = 4.9, 0.7 Hz), 6.94 (1H, d, J = 7.8 Hz), 6.77 (1H, br s), 6.71-6.68 (1H, m), 2.72 (3H, s), 2.35 (3H, s), 1.71 (6H, s).
643 ¹H-NMR (CDCl₃) δ: 8.33 (1H, s), 8.29 (1H, s), 7.31-7.29 (1H, m), 6.84-6.77 (2H, m), 2.75 (3H, s), 2.25 (3H, s), 1.72 (6H, s).
644 ¹H-NMR (CDCl₃) δ: 8.11 (1H, s, ), 7.90 (1H, s, ), 7.23 (1H, dd, J = 7.6, 0.8 Hz, ), 6.86 (1H, dd, J = 7.6, 1.1 Hz, ), 6.75 (1H, t, J = 7.6 Hz, ), 2.63 (3H, s, ), 2.35 (3H, s, ), 2.24 (3H, s, ), 1.72 (6H, s, ).
645 ¹H-NMR (CDCl₃) δ: 8.20 (1H, d, J = 2.3 Hz, ), 7.93 (1H, s, ), 7.76-7.74 (1H, m, ), 7.25-7.22 (1H, m, ), 6.88 (1H, dd, J = 7.6, 1.0 Hz, ), 6.76 (1H, t, J = 7.6 Hz, ), 2.43 (3H, d, J = 1.5 Hz, ), 2.25 (3H, s, ), 1.72 (6H, s, ).
646 ¹H-NMR (CDCl₃) δ: 8.22 (1H, d, J = 4.6 Hz, ), 7.95 (1H, s, ), 7.24-7.22 (1H, m, ), 6.90 (1H, dq, J = 4.6, 1.2 Hz, ), 6.84 (1H, dd, J = 7.6, 1.2 Hz, ), 6.75 (1H, t, J = 7.6 Hz, ), 2.71 (3H, d, J = 0.8 Hz, ), 2.24 (3H, s, ), 1.72 (6H, s, ).
647 ¹H-NMR (CDCl₃) δ: 8.05 (1H, s, ), 7.86 (1H, s, ), 7.35-7.30 (2H, m, ), 7.07-7.00 (2H, m, ), 2.62 (3H, s, ), 2.34 (3H, s, ), 1.99-1.88 (2H, m, ), 1.65 (3H, s, ), 1.62-1.57 (2H, m, ), 0.93 (3H, t, J = 7.4 Hz, ).
648 ¹H-NMR (CDCl₃) δ: 8.13 (1H, d, J = 2.3 Hz), 7.89 (1H, s), 7.75-7.74 (1H, m), 7.36-7.31 (2H, m), 7.08-7.02 (2H, m), 2.42 (3H, s), 2.00-1.88 (2H, m), 1.65 (3H, s), 1.62-1.56 (2H, m), 0.93 (3H, t, J = 7.4 Hz).
649 ¹H-NMR (CDCl₃) δ: 8.15 (1H, d, J = 4.6 Hz), 7.92 (1H, s), 7.35-7.31 (2H, m), 7.04-7.02 (2H, m), 6.88 (1H, dd, J = 4.6, 1.1 Hz), 2.70 (3H, d, J = 1.1 Hz), 1.99-1.89 (2H, m), 1.66 (3H, s), 1.64-1.61 (2H, m), 0.93 (3H, t, J = 7.4 Hz).
650 ¹H-NMR (CDCl₃) δ: 8.34 (1H, s), 8.23 (1H, d, J = 4.9 Hz), 7.27-7.22 (1H, m), 7.12 (1H, dd, J = 5.0, 0.9 Hz), 6.91-6.89 (1H, m), 6.84-6.82 (1H, m), 2.73 (3H, s), 1.77 (6H, s).
651 ¹H-NMR (CDCl₃) δ: 8.33 (1H, s), 8.24 (1H, d, J = 5.0 Hz), 7.25-7.21 (1H, m), 7.12 (1H, dd, J = 5.0, 2.5 Hz), 6.89 (1H, dt, J = 7.8, 1.2 Hz), 6.81 (1H, td, J = 6.8, 4.5 Hz), 2.73 (3H, s), 2.02 (2H, td, J = 5.7, 3.1 Hz), 1.70 (3H, s), 1.65-1.45 (2H, m), 1.36 (2H, td, J = 12.2, 7.4 Hz), 0.91 (3H, t, J = 7.4 Hz).
652 ¹H-NMR (CDCl₃) δ: 8.34 (1H, s), 8.30 (1H, s), 7.25-7.24 (1H, m), 6.86-6.80 (2H, m), 2.75 (3H, s), 2.04-2.00 (2H, m), 1.69 (3H, s), 1.60-1.40 (2H, m), 1.36 (2H, td, J = 12.0, 7.0 Hz), 0.91 (3H, t, J = 7.4 Hz).
653 ¹H-NMR (CDCl₃) δ: 8.11 (1H, s, ), 7.88 (1H, s, ), 7.21 (1H, dd, J = 7.3, 0.8 Hz, ), 6.83 (1H, dd, J = 7.8, 1.0 Hz, ), 6.72 (1H, t, J = 7.6 Hz, ), 2.63 (3H, s, ), 2.35 (3H, s, ), 2.23 (3H, s, ), 1.99 (2H, dd, J = 8.8, 7.6 Hz, ), 1.66 (3H, s, ), 1.55-1.47 (2H, m, ), 1.39-1.31 (2H, m, ), 0.91 (3H, t, J = 7.3 Hz, ).
654 ¹H-NMR (CDCl₃) δ: 8.21 (1H, d, J = 4.6 Hz), 7.94 (1H, s), 7.23-7.20 (1H, m), 6.90 (1H, dd, J = 4.6, 1.1 Hz), 6.82 (1H, dd, J = 7.6, 1.1 Hz), 6.73 (1H, t, J = 7.6 Hz), 2.71 (3H, d, J = 1.1 Hz), 2.24 (3H, s), 2.03-1.96 (2H, m), 1.67 (3H, s), 1.57-1.48 (2H, m), 1.39-1.30 (2H, m), 1.26 (3H, t, J = 7.3 Hz).
655 ¹H-NMR (CDCl₃) δ: 8.32 (1H, s), 8.23 (1H, d, J = 5.0 Hz), 7.25-7.21 (1H, m), 7.12 (1H, d, J = 4.6 Hz), 6.88 (1H, dt, J = 8.0, 1.1 Hz), 6.81 (1H, td, J = 6.9, 4.6 Hz), 2.73 (3H, s), 2.03-1.99 (2H, m), 1.70 (3H, s), 1.66-1.59 (1H, m), 1.57-1.51 (1H, m), 0.96 (3H, t, J = 7.4 Hz).
656 ¹H-NMR (CDCl₃) δ: 8.33 (1H, s), 8.30 (1H, s), 7.25-7.22 (1H, m), 6.85-6.79 (2H, m), 2.75 (3H, s), 2.01-1.97 (2H, m), 1.69 (3H, s), 1.66-1.58 (1H, m), 1.53-1.52 (1H, m), 0.96 (3H, t, J = 7.4 Hz).
657 ¹H-NMR (CDCl₃) δ: 8.51-8.50 (1H, m), 8.44 (1H, d, J = 2.3 Hz), 8.30 (1H, s), 7.21 (1H, d, J = 7.3 Hz), 7.13-7.11 (1H, m), 6.77 (1H, t, J = 7.6 Hz), 2.40 (3H, d, J = 1.1 Hz), 2.23 (3H, s), 2.03-1.95 (2H, m), 1.63 (3H, s), 1.07 (3H, t, J = 7.4 Hz).
658 ¹H-NMR (CDCl₃) δ: 8.42 (1H, d, J = 4.2 Hz), 8.35 (1H, s), 7.37 (1H, d, J = 8.5 Hz), 7.35-7.30 (2H, m), 7.09-7.05 (1H, m), 6.75 (1H, dd, J = 4.2, 0.7 Hz), 2.83 (3H, d, J = 0.7 Hz), 1.98-1.89 (2H, m), 1.65-1.57 (2H, m), 1.63 (3H, s), 0.93 (3H, t, J = 7.3 Hz).
659 ¹H-NMR (CDCl₃) δ: 8.52-8.51 (1H, m), 8.43 (1H, d, J = 1.9 Hz), 8.31 (1H, s), 7.14 (1H, dd, J = 8.6, 6.3 Hz), 6.55 (1H, t, J = 9.0 Hz), 2.40 (3H, d, J = 0.8 Hz), 2.13 (3H, d, J = 1.9 Hz), 2.08-2.01 (1H, m), 1.91 (2H, d, J = 6.1 Hz), 1.66 (3H, s), 1.00 (6H, dd, J = 10.3, 6.9 Hz).
660 ¹H-NMR (CDCl₃) δ: 8.45 (1H, d, J = 4.6 Hz), 8.39 (1H, s), 7.15 (1H, dd, J = 8.6, 6.3 Hz), 6.77 (1H, dd, J = 4.2, 1.1 Hz), 6.54 (1H, t, J = 9.0 Hz), 2.84 (3H, d, J = 0.8 Hz), 2.13 (3H, d, J = 1.9 Hz), 2.09-2.01 (1H, m), 1.92 (2H, d, J = 6.5 Hz), 1.66 (3H, s), 1.00 (6H, dd, J = 11.1, 8.4 Hz).
661 ¹H-NMR (CDCl₃) δ: 8.36 (1H, s), 8.33 (1H, s), 7.15 (1H, dd, J = 8.8, 6.5 Hz), 6.53 (1H, t, J = 8.8 Hz), 2.80 (3H, s), 2.39 (3H, s), 2.13 (3H, d, J = 1.9 Hz), 2.08-2.01 (1H, m), 1.91 (2H, d, J = 6.1 Hz), 1.66 (3H, s), 1.00 (6H, dd, J = 11.3, 6.7 Hz).
662 ¹H-NMR (CDCl₃) δ: 8.20 (1H, d, J = 1.9 Hz), 7.92 (1H, s), 7.77-7.75 (1H, m), 7.01 (1H, dd, J = 8.8, 6.5 Hz), 6.62-6.60 (1H, m), 6.53 (1H, td, J = 8.8, 2.5 Hz), 2.44 (3H, d, J = 1.1 Hz), 2.01-1.97 (2H, m), 1.66 (3H, s), 1.58-1.50 (2H, m), 1.39-1.31 (2H, m), 0.91 (3H, t, J = 7.3 Hz).
663 ¹H-NMR (CDCl₃) δ: 8.22 (1H, d, J = 4.6 Hz), 7.95 (1H, s), 6.98 (1H, dd, J = 8.7, 6.5 Hz), 6.93-6.91 (1H, m), 6.60 (1H, dd, J = 9.8, 2.4 Hz), 6.52 (1H, td, J = 6.5, 2.4 Hz), 2.71 (3H, d, J = 1.0 Hz), 2.02-1.97 (2H, m), 1.67 (3H, s), 1.60-1.48 (2H, m), 1.39-1.30 (2H, m), 0.91 (3H, t, J = 7.3 Hz).
664 ¹H-NMR (CDCl₃) δ: 8.22 (1H, d, J = 4.6 Hz), 7.94 (1H, s), 6.97 (1H, dd, J = 8.6, 6.3 Hz), 6.93-6.90 (1H, m), 6.59 (1H, dd, J = 9.7, 2.5 Hz), 6.52 (1H, td, J = 6.5, 2.4 Hz), 2.71 (3H, d, J = 1.1 Hz), 2.08-2.03 (1H, m), 1.92 (2H, ddd, J = 19.6, 13.5, 5.3 Hz), 1.70 (3H, s), 1.00 (6H, dd, J = 6.7, 3.2 Hz).
665 ¹H-NMR (CDCl₃) δ: 8.19 (1H, d, J = 2.3 Hz), 7.91 (1H, s), 7.76 (1H, s), 7.00 (1H, dd, J = 8.6, 6.3 Hz), 6.59 (1H, dd, J = 9.7, 2.5 Hz), 6.55-6.52 (1H, m), 2.44 (3H, d, J = 0.8 Hz), 2.07-2.03 (1H, m), 1.92-1.90 (2H, m), 1.70 (3H, s), 1.00 (6H, dd, J = 6.7, 3.2 Hz).
666 ¹H-NMR (CDCl₃) δ: 8.11 (1H, s), 7.88 (1H, s), 7.00-6.97 (1H, m), 6.58 (1H, dd, J = 9.8, 2.4 Hz), 6.52 (1H, td, J = 6.5, 2.4 Hz), 2.63 (3H, s), 2.36 (3H, s), 2.07-2.02 (1H, m), 1.91 (2H, dd, J = 6.2, 2.1 Hz), 1.69 (3H, s), 1.00 (6H, dd, J = 6.7, 2.8 Hz).
667 ¹H-NMR (CDCl₃) δ: 8.53-8.52 (1H, m), 8.45 (1H, d, J = 2.2 Hz), 8.32 (1H, s), 7.32 (1H, dd, J = 8.4, 6.2 Hz), 6.60-6.53 (2H, m), 2.41 (3H, d, J = 1.0 Hz), 1.98-1.94 (2H, m), 1.63 (3H, s), 1.55-1.50 (2H, m), 1.39-1.30 (2H, m), 0.90 (3H, t, J = 7.2 Hz).
668 ¹H-NMR (CDCl₃) δ: 8.45 (1H, d, J = 1.9 Hz), 8.32 (1H, s), 7.35-7.32 (1H, m), 6.66-6.65 (1H, m), 6.58-6.54 (2H, m), 2.41 (3H, d, J = 0.8 Hz), 2.07-2.00 (1H, m), 1.88 (2H, dd, J = 6.1, 3.1 Hz), 1.67 (3H, s), 0.99 (6H, dd, J = 6.9, 1.5 Hz).
669 ¹H-NMR (CDCl₃) δ: 8.41 (1H, s), 8.30 (1H, s), 7.29-7.28 (1H, m), 6.82 (1H, dd, J = 7.6, 1.7 Hz), 6.77 (1H, t, J = 7.6 Hz), 2.76 (3H, s), 2.24 (3H, s), 1.97-1.96 (2H, m), 1.64 (3H, s), 1.63-1.59 (1H, m), 1.53-1.50 (1H, m), 0.96 (3H, t, J = 7.4 Hz).
670 ¹H-NMR (CDCl₃) δ: 8.41 (1H, s), 8.30 (1H, s), 7.29-7.28 (1H, m), 6.82 (1H, dd, J = 7.6, 1.7 Hz), 6.77 (1H, t, J = 7.6 Hz), 2.76 (3H, s), 2.24 (3H, s), 1.97-1.96 (2H, m), 1.64 (3H, s), 1.63-1.59 (1H, m), 1.53-1.50 (1H, m), 0.96 (3H, t, J = 7.4 Hz).
671 ¹H-NMR (CDCl₃) δ: 8.31 (1H, s), 8.24 (1H, d, J = 5.0 Hz), 7.28-7.27 (1H, m), 7.10-7.09 (1H, m), 6.85 (1H, dd, J = 7.6, 1.3 Hz), 6.76 (1H, t, J = 7.6 Hz), 2.72 (3H, s), 2.23 (3H, s), 1.98-1.96 (2H, m), 1.65 (3H, s), 1.62-1.61 (1H, m), 1.54-1.52 (1H, m), 0.96 (3H, t, J = 7.4 Hz).
672 ¹H-NMR (CDCl₃) δ: 8.41 (1H, s), 8.30 (1H, s), 7.30-7.28 (1H, m), 6.83-6.81 (1H, m), 6.77 (1H, t, J = 7.6 Hz), 2.76 (3H, s), 2.24 (3H, s), 2.00-1.97 (2H, m), 1.64 (3H, s), 1.56-1.43 (2H, m), 1.39-1.31 (2H, m), 0.91 (3H, t, J = 7.3 Hz).
673 ¹H-NMR (CDCl₃) δ: 8.33 (1H, s), 8.30 (1H, s), 7.30-7.28 (1H, m), 6.83-6.81 (1H, m), 6.77 (1H, t, J = 7.4 Hz), 2.75 (3H, s), 2.24 (3H, s), 1.99-1.96 (2H, m), 1.64 (3H, s), 1.56-1.43 (2H, m), 1.37-1.33 (2H, m), 0.91 (3H, t, J = 7.3 Hz).
674 ¹H-NMR (CDCl₃) δ: 8.31 (1H, s), 8.24 (1H, d, J = 5.0 Hz), 7.28-7.27 (1H, m), 7.10-7.09 (1H, m), 6.86-6.85 (1H, m), 6.76 (1H, t, J = 7.6 Hz), 2.72 (3H, s), 2.24 (3H, s), 1.99-1.97 (2H, m), 1.65 (3H, s), 1.61-1.58 (1H, m), 1.55-1.45 (1H, m), 1.39-1.32 (2H, m), 0.91 (3H, dd, J = 8.4, 6.1 Hz).
675 ¹H-NMR (CDCl₃) δ: 8.28 (1H, s), 8.13 (1H, s), 7.29-7.28 (1H, m), 6.88-6.86 (1H, m), 6.77 (1H, t, J = 7.6 Hz), 2.63 (3H, s), 2.37 (3H, s), 2.24 (3H, s), 1.71 (6H, s).
676 ¹H-NMR (CDCl₃) δ: 8.28 (1H, s), 8.13 (1H, s), 6.95 (1H, d, J = 7.6 Hz), 6.76 (1H, s), 6.69 (1H, dd, J = 7.6, 1.0 Hz), 2.64 (3H, s), 2.38 (3H, s), 2.35 (3H, s), 1.70 (6H, s).
677 ¹H-NMR (CDCl₃) δ: 8.34 (1H, s), 8.30 (1H, s), 7.25-7.23 (1H, m), 6.86-6.79 (2H, m), 2.75 (3H, s), 2.03-1.99 (2H, m), 1.69 (3H, s), 1.62-1.46 (2H, m), 1.33-1.29 (4H, m), 0.90-0.86 (3H, m).
678 ¹H-NMR (CDCl₃) δ: 8.33 (1H, s), 8.23 (1H, d, J = 4.9 Hz), 7.24-7.22 (1H, m), 7.12 (1H, d, J = 4.9 Hz), 6.89-6.87 (1H, m), 6.82-6.80 (1H, m), 2.73 (3H, s), 2.04-2.00 (2H, m), 1.70 (3H, s), 1.65-1.52 (2H, m), 1.33-1.30 (4H, m), 0.89-0.87 (3H, m).
679 ¹H-NMR (CDCl₃) δ: 8.27 (1H, s), 8.13 (1H, s), 7.28-7.26 (1H, m), 6.86-6.86 (1H, m), 6.77-6.75 (1H, m), 2.64 (3H, s), 2.37 (3H, s), 2.23 (3H, s), 1.96-1.95 (2H, m), 1.64 (3H, s), 1.55-1.50 (2H, m), 0.95 (3H, t, J = 7.5 Hz).
680 ¹H-NMR (CDCl₃) δ: 8.28 (1H, s), 8.13 (1H, s), 7.28-7.27 (1H, m), 6.87-6.86 (1H, m), 6.76-6.75 (1H, m), 2.64 (3H, s), 2.37 (3H, s), 2.22 (3H, s), 2.00-1.96 (2H, m), 1.64 (3H, s), 1.57-1.45 (2H, m), 1.36-1.34 (2H, m), 0.91 (3H, t, J = 7.5 Hz).
681 ¹H-NMR (CDCl₃) δ: 8.47 (1H, d, J = 4.2 Hz), 8.39 (1H, s), 7.18 (1H, d, J = 8.0 Hz), 6.77 (1H, dd, J = 4.2, 0.8 Hz), 6.70-6.67 (2H, m), 2.84 (3H, d, J = 0.8 Hz), 2.33 (3H, s), 1.97-1.93 (2H, m), 1.63 (3H, s), 1.61-1.46 (2H, m), 0.94 (3H, t, J = 7.3 Hz).
682 ¹H-NMR (CDCl₃) δ: 8.38 (1H, s), 8.32 (1H, s), 7.18 (1H, d, J = 7.6 Hz), 6.70-6.66 (2H, m), 2.81 (3H, s), 2.39 (3H, s), 2.33 (3H, s), 2.00-1.89 (2H, m), 1.63 (3H, s), 1.65-1.50 (2H, m), 0.93 (3H, t, J = 7.3 Hz).
683 ¹H-NMR (CDCl₃) δ: 8.21 (1H, d, J = 1.9 Hz), 7.92 (1H, s), 7.77-7.76 (1H, m), 7.04 (1H, dd, J = 9.5, 6.9 Hz), 6.63 (1H, dd, J = 9.7, 2.5 Hz), 6.57 (1H, td, J = 6.5, 2.4 Hz), 2.45 (3H, d, J = 2.3 Hz), 1.72 (6H, s).
684 ¹H-NMR (CDCl₃) δ: 8.23 (1H, d, J = 4.6 Hz), 7.96 (1H, s), 7.01 (1H, dd, J = 8.8, 6.5 Hz), 6.93 (1H, dd, J = 4.6, 1.1 Hz), 6.63 (1H, dd, J = 9.7, 2.5 Hz), 6.55 (1H, td, J = 6.5, 2.4 Hz), 2.71 (3H, d, J = 0.8 Hz), 1.73 (6H, s).
685 ¹H-NMR (CDCl₃) δ: 8.12 (1H, s), 7.90 (1H, s), 7.02 (1H, dd, J = 8.8, 6.5 Hz), 6.63 (1H, dd, J = 9.7, 2.5 Hz), 6.55 (1H, td, J = 6.5, 2.4 Hz), 2.63 (3H, s), 2.36 (3H, s), 1.72 (6H, s).
686 ¹H-NMR (CDCl₃) δ: 8.85 (1H, s), 8.53-8.53 (1H, m), 8.32 (1H, s), 7.33 (1H, dd, J = 8.4, 6.5 Hz), 6.62-6.56 (2H, m), 2.41 (3H, s), 1.69 (6H, s).
687 ¹H-NMR (CDCl₃) δ: 8.48 (1H, d, J = 5.6 Hz), 8.40 (1H, s), 7.33 (1H, dd, J = 8.5, 6.3 Hz), 6.79 (1H, dd, J = 4.2, 1.0 Hz), 6.63-6.56 (2H, m), 2.85 (3H, d, J = 0.9 Hz), 1.70 (6H, s).
688 ¹H-NMR (CDCl₃) δ: 8.53-8.52 (1H, m), 8.45 (1H, d, J = 2.2 Hz), 8.32 (1H, s), 7.34-7.30 (1H, m), 6.60-6.53 (2H, m), 2.41 (3H, d, J = 1.2 Hz), 1.97-1.92 (2H, m), 1.65-1.51 (2H, m), 1.63 (3H, s), 0.94 (3H, t, J = 7.3 Hz).
689 ¹H-NMR (CDCl₃) δ: 8.47 (1H, d, J = 4.2 Hz), 8.40 (1H, s), 7.32 (1H, dd, J = 8.6, 6.3 Hz), 6.79 (1H, d, J = 2.1 Hz), 6.60-6.53 (2H, m), 2.85 (3H, s), 2.01-1.91 (2H, m), 1.64 (3H, s), 1.61-1.52 (2H, m), 0.94 (3H, t, J = 7.4 Hz).
690 ¹H-NMR (CDCl₃) δ: 8.22 (1H, d, J = 4.6 Hz), 7.94 (1H, s), 6.98 (1H, dd, J = 8.8, 6.3 Hz), 6.92 (1H, dd, J = 4.6, 1.2 Hz), 6.60 (1H, dd, J = 9.8, 2.4 Hz), 6.52 (1H, td, J = 6.6, 2.7 Hz), 2.71 (3H, d, J = 1.2 Hz), 2.00-1.96 (2H, m), 1.67 (3H, s), 1.65-1.52 (2H, m), 0.95 (3H, t, J = 7.3 Hz).
691 ¹H-NMR (CDCl₃) δ: 8.20 (1H, d, J = 2.2 Hz), 7.91 (1H, s), 7.76-7.76 (1H, m), 7.03-6.99 (1H, m), 6.60 (1H, dd, J = 9.5, 2.4 Hz), 6.56-6.51 (1H, m), 2.44 (3H, d, J = 1.2 Hz), 1.99-1.94 (2H, m), 1.66 (3H, s), 1.63-1.50 (2H, m), 0.95 (3H, t, J = 7.3 Hz).
692 ¹H-NMR (CDCl₃) δ: 8.22 (1H, d, J = 4.6 Hz), 7.95 (1H, s), 7.37-7.34 (1H, m), 7.00 (1H, dd, J = 8.0, 1.5 Hz), 6.92-6.88 (2H, m), 6.83 (1H, td, J = 8.0, 1.0 Hz), 2.71 (3H, d, J = 1.1 Hz), 2.02-1.98 (2H, m), 1.67 (3H, s), 1.60-1.51 (2H, m), 1.38-1.31 (2H, m), 0.91 (3H, t, J = 7.3 Hz).
693 ¹H-NMR (CDCl₃) δ: 8.42 (1H, s), 8.31 (1H, s), 7.44-7.42 (1H, m), 7.03-7.00 (1H, m), 6.94 (1H, dd, J = 8.3, 1.0 Hz), 6.87 (1H, td, J = 8.3, 1.0 Hz), 2.77 (3H, s), 1.99-1.97 (2H, m), 1.65 (3H, s), 1.55-1.47 (2H, m), 1.37-1.33 (2H, m), 0.90 (3H, t, J = 7.3 Hz).
694 ¹H-NMR (CDCl₃) δ: 8.33 (1H, s), 8.24 (1H, d, J = 4.9 Hz), 7.43-7.41 (1H, m), 7.11 (1H, dd, J = 4.9, 1.0 Hz), 7.06 (1H, dd, J = 7.8, 1.0 Hz), 6.93 (1H, dd, J = 7.8, 1.0 Hz), 6.87 (1H, td, J = 7.8, 1.0 Hz), 2.73 (3H, s), 2.01-1.96 (2H, m), 1.65 (3H, s), 1.57-1.45 (2H, m), 1.36-1.33 (2H, m), 0.91 (3H, t, J = 7.3 Hz).
695 ¹H-NMR (CDCl₃) δ: 8.13 (1H, d, J = 4.6 Hz, ), 7.90 (1H, s, ), 7.34-7.27 (2H, m, ), 7.03-6.98 (2H, m, ), 6.86 (1H, dd, J = 4.6, 1.1 Hz, ), 2.68 (3H, d, J = 1.1 Hz, ), 2.00-1.89 (2H, m, ), 1.63 (3H, s, ), 1.56-1.51 (2H, m, ), 1.34-1.27 (2H, m, ), 0.88 (3H, t, J = 7.4 Hz, ).
696 ¹H-NMR (CDCl₃) δ: 8.02 (1H, s, ), 7.84 (1H, s, ), 7.33-7.27 (2H, m, ), 7.04-6.98 (2H, m, ), 2.60 (3H, s, ), 2.31 (3H, s, ), 1.99-1.88 (2H, m, ), 1.63 (3H, s, ), 1.54-1.50 (2H, m, ), 1.35-1.26 (2H, m, ), 0.88 (3H, t, J = 7.3 Hz, ).
697 ¹H-NMR (CDCl₃) δ: 8.35 (1H, s), 8.29 (1H, s), 6.88 (1H, dd, J = 9.0, 6.3 Hz), 6.58 (1H, t, J = 9.2 Hz), 2.74 (3H, s), 2.16 (3H, d, J = 1.9 Hz), 1.72 (6H, s).
698 ¹H-NMR (CDCl₃) δ: 8.33 (1H, s), 8.29 (1H, s), 6.86 (1H, dd, J = 8.6, 6.3 Hz), 6.56 (1H, t, J = 8.6 Hz), 2.75 (3H, s), 2.15 (3H, d, J = 1.5 Hz), 1.96 (2H, dd, J = 9.0, 7.4 Hz), 1.64 (3H, s), 1.62-1.48 (2H, m), 0.96 (3H, t, J = 7.3 Hz).
699 ¹H-NMR (CDCl₃) δ: 8.33 (1H, s), 8.29 (1H, s), 6.87 (1H, dd, J = 9.0, 6.3 Hz), 6.56 (1H, t, J = 9.0 Hz), 2.75 (3H, s), 2.15 (3H, d, J = 1.9 Hz), 1.98 (2H, dd, J = 9.0, 7.8 Hz), 1.64 (3H, s), 1.60-1.41 (2H, m), 1.35 (2H, td, J = 12.1, 7.3 Hz), 0.91 (3H, t, J = 7.3 Hz).
700 ¹H-NMR (CDCl₃) δ: 8.34 (1H, s), 8.08 (1H, s), 7.62 (1H, d, J = 8.8 Hz), 6.97 (1H, d, J = 8.0 Hz), 2.78 (3H, s), 1.96 (2H, t, J = 10.0 Hz), 1.96 (3H, s), 1.62 (3H, s), 1.54-1.42 (2H, m), 1.36 (2H, td, J = 12.0, 7.0 Hz), 0.93 (3H, t, J = 7.3 Hz).
701 ¹H-NMR (CDCl₃) δ: 8.34 (1H, s), 8.29 (1H, s), 6.87 (1H, dd, J = 8.8, 6.1 Hz), 6.56 (1H, t, J = 8.8 Hz), 2.75 (3H, s), 2.15 (3H, d, J = 2.0 Hz), 2.06-1.97 (1H, m), 1.93 (2H, d, J = 5.9 Hz), 1.66 (3H, s), 1.01 (3H, d, J = 6.3 Hz), 0.99 (3H, d, J = 6.6 Hz).
702 ¹H-NMR (CDCl₃) δ: 8.34 (1H, s), 8.08 (1H, s), 7.62 (1H, d, J = 8.1 Hz), 6.97 (1H, d, J = 8.8 Hz), 2.77 (3H, s), 2.01-1.99 (1H, m), 1.96 (3H, s), 1.92-1.91 (2H, m), 1.63 (3H, s), 1.01-0.99 (6H, m).
703 '1H-NMR (CDCl₃) δ: 8.04 (1H, s), 7.86 (1H, s), 7.35-7.29 (2H, m), 7.07-7.00 (2H, m), 2.62 (3H, s), 2.34 (3H, s), 2.00-1.89 (2H, m), 1.65 (3H, s), 1.63-1.54 (4H, m), 1.34-1.27 (2H, m), 0.87 (3H, t, J = 7.1 Hz).
704 '1H-NMR (CDCl₃) δ: 8.13 (1H, d, J = 2.3 Hz), 7.89 (1H, s), 7.74 (1H, d, J = 0.8 Hz), 7.36-7.30 (2H, m), 7.05 (2H, tt, J = 11.3, 3.8 Hz), 2.42 (3H, s), 2.01-1.89 (2H, m), 1.65 (3H, s), 1.62-1.57 (2H, m), 1.34-1.25 (4H, m), 0.87 (3H, t, J = 7.1 Hz).
705 '1H-NMR (CDCl₃) δ: 8.15 (1H, d, J = 4.6 Hz), 7.92 (1H, s), 7.36-7.30 (2H, m), 7.03 (2H, tt, J = 10.9, 3.6 Hz), 6.88 (1H, dd, J = 4.6, 1.1 Hz), 2.70 (3H, d, J = 0.8 Hz), 2.01-1.90 (2H, m), 1.66 (3H, s), 1.58 (2H, dt, J = 18.6, 6.6 Hz), 1.34-1.27 (4H, m), 0.87 (3H, t, J = 7.1 Hz).
706 ¹H-NMR (CDCl₃) δ: 8.05 (1H, s), 7.86 (1H, s), 7.36-7.29 (2H, m), 7.08-7.00 (2H, m), 2.62 (3H, s), 2.34 (3H, s), 2.02-1.97 (2H, m), 1.65 (3H, s), 1.10 (3H, t, J = 7.3 Hz).
707 ¹H-NMR (CDCl₃) δ: 8.13 (1H, d, J = 1.9 Hz), 7.90 (1H, d, J = 3.1 Hz), 7.74 (1H, dd, J = 2.3, 1.1 Hz), 7.33 (2H, tt, J = 10.5, 3.8 Hz), 7.09-7.02 (2H, m), 2.42 (3H, s), 2.00 (2H, dq, J = 29.4, 7.0 Hz), 1.65 (3H, s), 1.10 (3H, t, J = 7.4 Hz).
708 '1H-NMR (CDCl₃) δ: 8.15 (1H, d, J = 4.6 Hz), 7.92 (1H, s), 7.36-7.30 (2H, m), 7.06-7.01 (2H, m), 6.88 (1H, dd, J = 4.6, 1.1 Hz), 2.70 (3H, d, J = 1.1 Hz), 2.04-1.96 (2H, m), 1.66 (3H, s), 1.10 (3H, t, J = 7.4 Hz).
709 ¹H-NMR (CDCl₃) δ: 8.12 (1H, s), 7.88 (1H, s), 6.89 (1H, d, J = 8.0 Hz), 6.70 (1H, s), 6.64-6.62 (1H, m), 2.63 (3H, s), 2.35 (3H, s), 2.33 (3H, s), 1.98-1.94 (2H, m), 1.65 (3H, s), 1.63-1.52 (2H, m), 0.94 (3H, t, J = 7.4 Hz).
710 '1H-NMR (CDCl₃) δ: 8.20 (1H, d, J = 1.9 Hz), 7.91 (1H, s), 7.75-7.74 (1H, br m), 6.90 (1H, d, J = 8.0 Hz), 6.71 (1H, s), 6.65 (1H, d, J = 6.9 Hz), 2.43 (3H, d, J = 0.8 Hz), 2.33 (3H, s), 1.99-1.93 (2H, m), 1.66 (3H, d, J = 5.7 Hz), 1.64-1.52 (2H, m), 0.95 (3H, t, J = 7.3 Hz).
711 ¹H-NMR (CDCl₃) δ: 8.22 (1H, d, J = 4.6 Hz), 7.94 (1H, s), 6.90 (1H, dd, J = 4.6, 1.1 Hz), 6.87 (1H, d, J = 7.6 Hz), 6.71 (1H, s), 6.65-6.62 (1H, m), 2.71 (3H, d, J = 0.8 Hz), 2.33 (3H, s), 1.99-1.95 (2H, m), 1.66 (3H, s), 1.64-1.52 (2H, m), 0.95 (3H, t, J = 7.3 Hz).
712 ¹H-NMR (CDCl₃) δ: 8.11 (1H, s), 7.88 (1H, s), 6.89 (1H, d, J = 7.6 Hz), 6.71 (1H, s), 6.65-6.62 (1H, m), 2.63 (3H, s), 2.35 (3H, s), 2.33 (3H, s), 2.01-1.95 (2H, m), 1.65 (3H, s), 1.61-1.49 (2H, m), 1.38-1.31 (2H, m), 0.91 (3H, t, J = 7.3 Hz).
713 ¹H-NMR (CDCl₃) δ: 8.19 (1H, d, J = 1.9 Hz), 7.91 (1H, s), 7.75 (1H, dd, J = 1.9, 1.1 Hz), 6.90 (1H, d, J = 8.0 Hz), 6.71 (1H, s), 6.65 (1H, t, J = 3.8 Hz), 2.43 (3H, d, J = 1.1 Hz), 2.33 (3H, s), 1.98 (2H, t, J = 8.4 Hz), 1.65 (3H, s), 1.60-1.49 (2H, m), 1.38-1.31 (2H, m), 0.91 (3H, t, J = 7.3 Hz).
714 ¹H-NMR (CDCl₃) δ: 8.22 (1H, d, J = 4.6 Hz), 7.94 (1H, s), 6.90 (1H, dd, J = 4.6, 1.1 Hz), 6.87 (1H, d, J = 8.0 Hz), 6.71 (1H, s), 6.65-6.63 (1H, m), 2.71 (3H, d, J = 1.1 Hz), 2.33 (3H, s), 2.02-1.96 (2H, m), 1.66 (3H, s), 1.60-1.49 (2H, m), 1.39-1.31 (2H, m), 0.91 (3H, t, J = 7.3 Hz).
715 ¹H-NMR (CDCl₃) δ: 8.35 (1H, s), 8.29 (1H, s), 7.00 (1H, d, J = 8.4 Hz), 6.98 (1H, d, J = 1.9 Hz), 6.87 (1H, dd, J = 8.4, 1.9 Hz), 2.75 (3H, s), 1.71 (6H, s).
716 ¹H-NMR (CDCl₃) δ: 8.35 (1H, s), 8.30 (1H, s), 7.27-7.23 (1H, m), 6.85 (1H, dd, J = 8.0, 1.5 Hz), 6.82 (1H, td, J = 6.6, 4.1 Hz), 2.75 (3H, s), 2.08-2.05 (2H, m), 1.69 (3H, s), 1.09 (3H, t, J = 7.4 Hz).
717 ¹H-NMR (CDCl₃) δ: 8.34 (1H, s), 8.23 (1H, d, J = 4.9 Hz), 7.12 (1H, dd, J = 5.0, 0.9 Hz), 7.04 (1H, d, J = 8.3 Hz), 6.96 (1H, d, J = 2.0 Hz), 6.85 (1H, dd, J = 8.5, 2.0 Hz), 2.72 (3H, d, J = 0.5 Hz), 2.02 (2H, q, J = 7.8 Hz), 1.64 (3H, s), 1.06 (3H, t, J = 7.4 Hz).
718 ¹H-NMR (CDCl₃) δ: 8.35 (1H, s), 8.29 (1H, s), 7.00 (1H, d, J = 8.3 Hz), 6.97 (1H, d, J = 2.0 Hz), 6.85 (1H, dd, J = 8.5, 2.0 Hz), 2.75 (3H, s), 2.01 (2H, q, J = 7.5 Hz), 1.63 (3H, s), 1.05 (3H, t, J = 7.4 Hz).
719 ¹H-NMR (CDCl₃) δ: 8.42 (1H, d, J = 4.2 Hz), 8.36 (1H, s), 7.38-7.30 (3H, m), 7.07 (1H, ddd, J = 8.1, 6.0, 1.8 Hz), 6.75 (1H, dd, J = 4.2, 1.0 Hz), 2.83 (3H, d, J = 0.7 Hz), 2.02-1.90 (2H, m), 1.63 (3H, s), 1.61-1.52 (2H, m), 1.37-1.26 (2H, m), 0.90 (3H, t, J = 7.3 Hz).
720 ¹H-NMR (CDCl₃) δ: 8.33 (1H, s), 8.29 (1H, s), 7.38-7.36 (1H, m), 7.33-7.31 (2H, m), 7.08-7.04 (1H, m), 2.80 (3H, d, J = 0.5 Hz), 2.38 (3H, s), 1.59-1.52 (2H, m), 1.99-1.90 (2H, m), 1.63 (3H, s), 1.34-1.28 (2H, m), 0.89 (3H, t, J = 7.3 Hz).
721 ¹H-NMR (CDCl₃) δ: 8.51-8.50 (1H, m), 8.40 (1H, d, J = 2.3 Hz), 8.28 (1H, s), 7.38-7.30 (3H, m), 7.09-7.07 (1H, m), 2.39 (3H, d, J = 0.7 Hz), 2.03-1.91 (2H, m), 1.63 (3H, s), 1.09 (3H, t, J = 7.3 Hz).
722 ¹H-NMR (CDCl₃) δ: 8.43 (1H, d, J = 4.2 Hz), 8.35 (1H, s), 7.38-7.31 (3H, m), 7.09-7.04 (1H, m), 6.75 (1H, d, J = 4.2 Hz), 2.83 (3H, d, J = 0.8 Hz), 2.02-1.96 (2H, m), 1.64 (3H, s), 1.10 (3H, t, J = 7.3 Hz).
723 ¹H-NMR (CDCl₃) δ: 8.33 (1H, s), 8.29 (1H, s), 7.38-7.30 (3H, m), 7.08-7.05 (1H, m), 2.80 (3H, s), 2.38 (3H, s), 2.03-1.94 (2H, m), 1.63 (3H, s), 1.09 (3H, t, J = 7.3 Hz).
724 ¹H-NMR (CDCl₃) δ: 8.44 (1H, d, J = 2.2 Hz), 8.32 (1H, s), 7.27-7.25 (1H, m), 6.89 (1H, d, J = 2.2 Hz), 6.84 (1H, dd, J = 8.3, 2.0 Hz), 6.66 (1H, t, J = 1.1 Hz), 2.41 (3H, d, J = 1.0 Hz), 1.97-1.91 (2H, m), 1.62-1.50 (2H, m), 1.62 (3H, s), 0.94 (3H, t, J = 7.4 Hz).
725 ¹H-NMR (CDCl₃) δ: 8.47 (1H, d, J = 4.2 Hz), 8.40 (1H, s), 7.28-7.26 (1H, m), 6.89 (1H, d, J = 1.9 Hz), 6.84 (1H, dd, J = 8.4, 1.9 Hz), 6.79 (1H, d, J = 4.2 Hz), 2.85 (3H, s), 2.00-1.90 (2H, m), 1.63 (3H, s), 1.61-1.51 (2H, m), 0.95 (3H, t, J = 9.4 Hz).
726 ¹H-NMR (CDCl₃) δ: 8.37 (1H, s), 8.33 (1H, s), 7.27 (1H, d, J = 7.6 Hz), 6.88 (1H, d, J = 1.9 Hz), 6.82 (1H, dd, J = 8.4, 1.9 Hz), 2.81 (3H, s), 2.40 (3H, s), 1.98-1.91 (2H, m), 1.62 (3H, s), 1.58-1.49 (2H, m), 0.94 (3H, t, J = 7.4 Hz).
727 ¹H-NMR (CDCl₃) δ: 8.42 (1H, d, J = 4.2 Hz), 8.35 (1H, s), 7.38-7.29 (3H, m), 7.09-7.05 (1H, m), 6.75 (1H, dd, J = 4.2, 0.8 Hz), 2.83 (3H, s), 1.99-1.91 (2H, m), 1.63 (3H, s), 1.62-1.54 (2H, m), 1.35-1.25 (4H, m), 0.87 (3H, t, J = 7.1 Hz).
728 ¹H-NMR (CDCl₃) δ: 8.50-8.50 (1H, m), 8.39 (1H, d, J = 2.3 Hz), 8.28 (1H, s), 7.37-7.30 (3H, m), 7.09-7.06 (1H, m), 2.39 (3H, d, J = 0.8 Hz), 1.99-1.87 (2H, m), 1.63 (3H, s), 1.60-1.56 (2H, m), 1.35-1.25 (4H, m), 0.87 (3H, t, J = 7.1 Hz).
729 ¹H-NMR (CDCl₃) δ: 8.33 (1H, s), 8.29 (1H, s), 7.38-7.31 (3H, m), 7.07-7.05 (1H, m), 2.80 (3H, s), 2.38 (3H, s), 1.97-1.90 (2H, m), 1.63 (3H, s), 1.61-1.54 (2H, m), 1.33-1.25 (4H, m), 0.87 (3H, t, J = 7.1 Hz).
730 ¹H-NMR (CDCl₃) δ: 8.53-8.52 (1H, m), 8.45 (1H, d, J = 2.3 Hz), 8.32 (1H, s), 7.27 (1H, d, J = 8.8 Hz), 6.92 (1H, d, J = 1.9 Hz), 6.87 (1H, dd, J = 8.4, 1.9 Hz), 2.42 (3H, d, J = 1.5 Hz), 1.69 (6H, s).
731 ¹H-NMR (CDCl₃) δ: 8.38 (1H, s), 8.34 (1H, s), 7.27 (1H, d, J = 6.5 Hz), 6.91 (1H, d, J = 1.9 Hz), 6.86 (1H, dd, J = 8.4, 1.9 Hz), 2.81 (3H, s), 2.40 (3H, s), 1.69 (6H, s).
732 ¹H-NMR (CDCl₃) δ: 8.41 (1H, s), 8.31 (1H, s), 7.24-7.23 (1H, m), 6.84-6.81 (2H, m), 2.77 (3H, s), 2.03-1.98 (2H, m), 1.69 (3H, s), 0.89-0.85 (9H, m).
733 ¹H-NMR (CDCl₃) δ: 8.41 (1H, s), 8.31 (1H, s), 7.43 (1H, td, J = 8.0, 1.0 Hz), 7.01 (1H, dd, J = 8.0, 1.0 Hz), 6.94 (1H, dd, J = 8.0, 1.0 Hz), 6.87 (1H, td, J = 8.0, 1.0 Hz), 2.77 (3H, s), 1.98-1.96 (2H, m), 1.64 (3H, s), 1.54-1.48 (2H, m), 1.31-1.30 (4H, m), 0.89-0.86 (3H, m).
734 ¹H-NMR (CDCl₃) δ: 8.34 (1H, s), 8.30 (1H, s), 7.44-7.41 (1H, m), 7.03-7.01 (1H, m), 6.95-6.93 (1H, m), 6.89-6.87 (1H, m), 2.75 (3H, s), 1.98-1.96 (2H, m), 1.65 (3H, s), 1.52-1.48 (2H, m), 1.32-1.30 (4H, m), 0.89-0.87 (3H, m).
735 ¹H-NMR (CDCl₃) δ: 8.32 (1H, s), 8.24 (1H, d, J = 4.9 Hz), 7.43-7.41 (1H, m), 7.11 (1H, dd, J = 4.9, 0.7 Hz), 7.06 (1H, dd, J = 8.0, 1.0 Hz), 6.93 (1H, dd, J = 8.0, 1.0 Hz), 6.86 (1H, td, J = 8.0, 1.0 Hz), 2.73 (3H, s), 1.99-1.96 (2H, m), 1.65 (3H, s), 1.53-1.51 (2H, m), 1.32-1.29 (4H, m), 0.89-0.87 (3H, m).
736 ¹H-NMR (CDCl₃) δ: 8.33 (1H, s), 8.33 (1H, s), 7.43-7.41 (2H, m), 7.10-7.02 (2H, m), 2.76 (3H, s), 1.71 (6H, s).
737 ¹H-NMR (CDCl₃) δ: 8.35 (1H, s), 8.23 (1H, s), 7.43-7.41 (2H, m), 7.08-7.05 (2H, m), 2.74 (3H, s), 1.71 (6H, s).
738 ¹H-NMR (CDCl₃) δ: 8.34 (1H, s), 8.17 (1H, d, J = 4.9 Hz), 7.41-7.40 (2H, m), 7.08-7.06 (3H, m), 2.72 (3H, s), 1.72 (6H, s).
739 ¹H-NMR (CDCl₃) δ: 8.42 (1H, s), 8.30 (1H, s), 6.89 (1H, d, J = 8.0 Hz), 6.75 (1H, br s), 6.68 (1H, dd, J = 8.0, 0.8 Hz), 2.77 (3H, s), 2.35 (3H, s), 1.95-1.94 (2H, m), 1.63 (3H, s), 1.60-1.50 (2H, m), 0.95 (3H, t, J = 7.4 Hz).
740 ¹H-NMR (CDCl₃) δ: 8.32 (1H, s), 8.31 (1H, s), 6.89 (1H, d, J = 8.0 Hz), 6.75 (1H, br s), 6.68-6.67 (1H, m), 2.75 (3H, s), 2.35 (3H, s), 1.96-1.94 (2H, m), 1.63 (3H, s), 1.60-1.45 (2H, m), 0.95 (3H, t, J = 7.3 Hz).
741 ¹H-NMR (CDCl₃) δ: 8.31 (1H, s), 8.25 (1H, d, J = 4.9 Hz), 7.10 (1H, dd, J = 4.9, 0.9 Hz), 6.93 (1H, d, J = 8.1 Hz), 6.74 (1H, br s), 6.68-6.66 (1H, m), 2.72 (3H, s), 2.34 (3H, s), 1.96-1.94 (2H, m), 1.64 (3H, s), 1.59-1.52 (2H, m), 0.95 (3H, t, J = 7.4 Hz).
742 ¹H-NMR (CDCl₃) δ: 8.40 (1H, s), 8.33 (1H, s), 7.00-6.98 (2H, m), 6.87 (1H, dd, J = 8.4, 1.9 Hz), 2.77 (3H, s), 1.71 (6H, s).
743 ¹H-NMR (CDCl₃) δ: 8.34 (1H, s), 8.23 (1H, d, J = 5.0 Hz), 7.12 (1H, dd, J = 5.0, 0.8 Hz), 7.04 (1H, d, J = 8.4 Hz), 6.97 (1H, d, J = 1.9 Hz), 6.87 (1H, dd, J = 8.4, 1.9 Hz), 2.73 (3H, s), 1.71 (6H, s).
744 ¹H-NMR (CDCl₃) δ: 8.28 (1H, s), 8.14 (1H, s), 7.42-7.40 (1H, m), 7.07 (1H, dd, J = 7.8, 1.3 Hz), 6.93-6.92 (1H, m), 6.86 (1H, td, J = 7.8, 1.3 Hz), 2.64 (3H, s), 2.38 (3H, s), 1.98-1.97 (2H, m), 1.65 (3H, s), 1.56-1.45 (2H, m), 1.36-1.33 (2H, m), 0.90 (3H, t, J = 7.4 Hz).
745 ¹H-NMR (CDCl₃) δ: 8.28 (1H, s), 8.14 (1H, s), 7.43-7.39 (1H, m), 7.07 (1H, dd, J = 7.6, 1.5 Hz), 6.94-6.91 (1H, m), 6.87-6.84 (1H, m), 2.64 (3H, s), 2.38 (3H, s), 1.97-1.96 (2H, m), 1.65 (3H, s), 1.60-1.49 (2H, m), 1.32-1.30 (4H, m), 0.88-0.86 (3H, m).
746 ¹H-NMR (CDCl₃) δ: 8.30 (1H, s), 8.06 (1H, s), 7.40-7.40 (2H, m), 7.08-7.06 (2H, m), 2.63 (3H, s), 2.35 (3H, s), 1.71 (6H, s).
747 ¹H-NMR (CDCl₃) δ: 8.27 (1H, s), 8.14 (1H, s), 6.94 (1H, d, J = 8.0 Hz), 6.74 (1H, br s), 6.66 (1H, dd, J = 8.0, 1.0 Hz), 2.64 (3H, s), 2.38 (3H, s), 2.34 (3H, s), 1.95-1.93 (2H, m), 1.63 (3H, s), 1.61-1.51 (2H, m), 0.94 (3H, t, J = 7.4 Hz).
748 ¹H-NMR (CDCl₃) δ: 8.22 (1H, d, J = 4.6 Hz), 7.95 (1H, s), 7.37-7.34 (1H, m), 7.00 (1H, dd, J = 8.0, 1.5 Hz), 6.92-6.88 (2H, m), 6.83 (1H, td, J = 8.0, 1.0 Hz), 2.71 (3H, d, J = 1.1 Hz), 2.02-1.98 (2H, m), 1.67 (3H, s), 1.60-1.51 (2H, m), 1.38-1.31 (2H, m), 0.91 (3H, t, J = 7.3 Hz).
749 ¹H-NMR (CDCl₃) δ: 8.48 (1H, d, J = 3.6 Hz), 8.40 (1H, s), 7.27 (1H, d, J = 8.0 Hz), 6.92 (1H, d, J = 1.9 Hz), 6.88-6.85 (1H, m), 6.79 (1H, d, J = 4.2 Hz), 2.85 (3H, s), 1.69 (6H, s).
750 ¹H-NMR (CDCl₃) δ: 8.53-8.52 (1H, m), 8.45 (1H, d, J = 1.9 Hz), 8.32 (1H, s), 7.26 (1H, d, J = 7.3 Hz), 6.89 (1H, d, J = 1.9 Hz), 6.84 (1H, dd, J = 8.4, 1.9 Hz), 2.41 (3H, d, J = 1.1 Hz), 1.98-1.93 (2H, m), 1.66 (3H, s), 1.56-1.48 (2H, m), 1.37-1.30 (2H, m), 0.91 (3H, t, J = 7.3 Hz).
751 ¹H-NMR (CDCl₃) δ: 8.47 (1H, d, J = 4.2 Hz), 8.40 (1H, s), 7.26 (1H, d, J = 8.4 Hz), 6.89 (1H, d, J = 2.3 Hz), 6.84 (1H, dd, J = 8.2, 2.1 Hz), 6.79 (1H, dd, J = 4.4, 1.0 Hz), 2.85 (3H, s), 1.99-1.95 (2H, m), 1.63 (3H, s), 1.57-1.48 (2H, m), 1.37-1.31 (2H, m), 0.90 (3H, t, J = 7.3 Hz).
752 ¹H-NMR (CDCl₃) δ: 8.53-8.52 (1H, m), 8.45 (1H, d, J = 1.9 Hz), 8.32 (1H, s), 7.28 (1H, d, J = 9.9 Hz), 6.88-6.83 (2H, m), 2.41 (3H, d, J = 0.8 Hz), 2.06-2.00 (1H, m), 1.88 (2H, dd, J = 6.1, 1.1 Hz), 1.66 (3H, s), 0.99 (6H, dd, J = 6.7, 1.0 Hz).
753 ¹H-NMR (CDCl₃) δ: 8.47 (1H, d, J = 4.2 Hz), 8.41 (1H, s), 7.28 (1H, d, J = 8.4 Hz), 6.88 (1H, d, J = 1.9 Hz), 6.84 (1H, dd, J = 8.2, 2.1 Hz), 6.79 (1H, d, J = 4.2 Hz), 2.85 (3H, s), 2.07-2.00 (1H, m), 1.89 (2H, dd, J = 6.3, 2.9 Hz), 1.66 (3H, s), 0.99 (6H, dd, J = 6.5, 1.5 Hz).
754 ¹H-NMR (CDCl₃) δ: 8.37 (1H, s), 8.34 (1H, s), 7.28 (1H, d, J = 8.0 Hz), 6.87 (1H, d, J = 2.3 Hz), 6.83 (1H, dd, J = 8.2, 2.1 Hz), 2.81 (3H, s), 2.40 (3H, s), 2.06-2.00 (1H, m), 1.88 (2H, dd, J = 6.1, 2.3 Hz), 1.66 (3H, s), 0.99 (6H, dd, J = 6.7, 1.7 Hz).
755 ¹H-NMR (CDCl₃) δ: 8.53-8.53 (1H, m), 8.46 (1H, d, J = 2.3 Hz), 8.32 (1H, s), 7.43 (1H, dd, J = 8.0, 1.5 Hz), 7.24 (1H, dd, J = 7.6, 1.5 Hz), 6.84 (1H, t, J = 7.8 Hz), 2.42 (3H, s), 1.74 (6H, s).
756 ¹H-NMR (CDCl₃) δ: 8.48 (1H, d, J = 4.2 Hz), 8.40 (1H, s), 7.43-7.42 (1H, m), 7.25 (1H, dd, J = 7.8, 1.3 Hz), 6.85-6.82 (1H, m), 6.80 (1H, dd, J = 4.2, 0.8 Hz), 2.85 (3H, s), 1.75 (6H, s).
757 ¹H-NMR (CDCl₃) δ: 8.38 (1H, s), 8.34 (1H, s), 7.42 (1H, dd, J = 8.0, 1.5 Hz), 7.26-7.24 (1H, m), 6.83 (1H, t, J = 7.8 Hz), 2.81 (3H, s), 2.40 (3H, s), 1.74 (6H, s).
758 ¹H-NMR (CDCl₃) δ: 8.53-8.52 (1H, m), 8.45 (1H, d, J = 1.9 Hz), 8.32 (1H, s), 7.41 (1H, dd, J = 8.0, 1.5 Hz), 7.23 (1H, dd, J = 7.6, 1.5 Hz), 6.81 (1H, t, J = 7.8 Hz), 2.41 (3H, s), 1.99-1.96 (2H, m), 1.69 (3H, s), 1.67-1.55 (2H, m), 0.95 (3H, t, J = 7.4 Hz).
759 ¹H-NMR (CDCl₃) δ: 8.47 (1H, d, J = 4.2 Hz), 8.40 (1H, s), 7.41-7.40 (1H, m), 7.23 (1H, dd, J = 7.6, 1.5 Hz), 6.83-6.78 (2H, m), 2.85 (3H, d, J = 0.8 Hz), 1.99-1.98 (2H, m), 1.69 (3H, s), 1.63-1.61 (2H, m), 0.95 (3H, t, J = 7.4 Hz).
760 ¹H-NMR (CDCl₃) δ: 8.37 (1H, s), 8.33 (1H, s), 7.40 (1H, dd, J = 8.0, 1.5 Hz), 7.23 (1H, dd, J = 7.6, 1.5 Hz), 6.80 (1H, t, J = 7.8 Hz), 2.81 (3H, s), 2.40 (3H, s), 2.00-1.96 (2H, m), 1.68 (3H, s), 1.65-1.56 (2H, m), 0.95 (3H, t, J = 7.4 Hz).
761 ¹H-NMR (CDCl₃) δ: 8.52-8.50 (1H, m), 8.39 (1H, d, J = 1.9 Hz), 8.29 (1H, s), 7.22 (1H, d, J = 7.3 Hz), 7.14 (1H, t, J = 8.6 Hz), 7.07-7.03 (1H, m), 2.40 (3H, s), 1.99-1.93 (2H, m), 1.64 (3H, s), 1.61-1.54 (2H, m), 1.34-1.31 (2H, m), 0.90 (3H, t, J = 7.4 Hz).
762 ¹H-NMR (CDCl₃) δ: 8.13 (1H, d, J = 2.3 Hz), 7.89 (1H, s), 7.76-7.74 (1H, m), 7.15-7.13 (1H, m), 7.01 (1H, td, J = 7.1, 5.3 Hz), 6.92 (1H, d, J = 6.5 Hz), 2.43-2.42 (3H, m), 2.02-1.93 (2H, m), 1.66 (3H, s), 1.62-1.52 (2H, m), 1.37-1.31 (2H, m), 0.91 (3H, t, J = 7.4 Hz).
763 ¹H-NMR (CDCl₃) δ: 8.05 (1H, s), 7.87 (1H, s), 7.15-7.11 (1H, m), 6.99 (1H, td, J = 7.1, 5.3 Hz), 6.92-6.90 (1H, m), 2.63 (3H, s), 2.34 (3H, s), 2.02-1.93 (2H, m), 1.66 (3H, s), 1.62-1.54 (2H, m), 1.37-1.31 (2H, m), 0.90 (3H, t, J = 7.4 Hz).
764 ¹H-NMR (CDCl₃) δ: 8.47 (1H, d, J = 4.2 Hz), 8.40 (1H, s), 7.27 (1H, d, J = 7.6 Hz), 6.90 (1H, d, J = 1.9 Hz), 6.84 (1H, dd, J = 8.2, 2.1 Hz), 6.79 (1H, d, J = 3.4 Hz), 2.85 (3H, d, J = 0.8 Hz), 2.03-1.97 (2H, m), 1.63 (3H, s), 1.06 (3H, t, J = 7.4 Hz).
765 ¹H-NMR (CDCl₃) δ: 8.37 (1H, s), 8.34 (1H, s), 7.27 (1H, d, J = 8.8 Hz), 6.89 (1H, d, J = 1.9 Hz), 6.83 (1H, dd, J = 8.2, 2.1 Hz), 2.81 (3H, s), 2.40 (3H, s), 2.02-1.96 (2H, m), 1.62 (3H, s), 1.05 (3H, t, J = 7.4 Hz).
766 ¹H-NMR (CDCl₃) δ: 8.49-8.48 (1H, m), 8.32 (1H, s), 7.36-7.30 (2H, m), 6.89-6.85 (2H, m), 6.65 (1H, d, J = 2.3 Hz), 2.41 (3H, d, J = 0.8 Hz), 1.99-1.95 (2H, m), 1.63 (3H, s), 1.57-1.41 (3H, m), 0.88 (6H, dd, J = 9.2, 6.5 Hz).
767 ¹H-NMR (CDCl₃) δ: 8.47 (1H, d, J = 3.8 Hz), 8.40 (1H, s), 7.36-7.30 (2H, m), 6.88-6.85 (2H, m), 6.77 (1H, dd, J = 4.2, 0.8 Hz), 2.84 (3H, s), 2.00-1.96 (2H, m), 1.64 (3H, s), 1.56-1.41 (3H, m), 0.88 (6H, dd, J = 9.2, 6.5 Hz).
768 ¹H-NMR (CDCl₃) δ: 8.38 (1H, s), 8.33 (1H, s), 7.35-7.31 (2H, m), 6.66 (2H, d, J = 2.3 Hz), 2.81 (3H, s), 2.40 (3H, s), 1.99-1.95 (2H, m), 1.63 (3H, s), 1.57-1.42 (3H, m), 0.88 (6H, dd, J = 9.5, 6.5 Hz).
769 ¹H-NMR (CDCl₃) δ: 8.37 (1H, s), 8.34 (1H, s), 7.27 (1H, d, J = 6.5 Hz), 6.89 (1H, d, J = 1.9 Hz), 6.83 (1H, dd, J = 8.4, 1.9 Hz), 2.81 (3H, s), 2.40 (3H, s), 1.98-1.94 (2H, m), 1.62 (3H, s), 1.56-1.49 (2H, m), 1.37-1.32 (2H, m), 0.90 (3H, t, J = 7.3 Hz).
770 ¹H-NMR (CDCl₃) δ: 8.20 (1H, d, J = 2.2 Hz), 7.92 (1H, s), 7.76-7.76 (1H, m), 6.95 (1H, d, J = 8.1 Hz), 6.91 (1H, d, J = 2.0 Hz), 6.81 (1H, dd, J = 8.2, 2.1 Hz), 2.44 (3H, d, J = 1.2 Hz), 1.98 (2H, t, J = 8.2 Hz), 1.66 (3H, s), 1.56-1.51 (2H, m), 1.39-1.32 (2H, m), 0.91 (3H, t, J = 7.3 Hz).
771 ¹H-NMR (CDCl₃) δ: 8.22 (1H, d, J = 4.6 Hz), 7.95 (1H, s), 6.93-6.91 (3H, m), 6.80 (1H, dd, J = 8.4, 1.9 Hz), 2.71 (3H, d, J = 1.1 Hz), 1.99 (2H, dd, J = 9.0, 7.8 Hz), 1.66 (3H, s), 1.60-1.49 (2H, m), 1.39-1.32 (2H, m), 0.91 (3H, t, J = 7.3 Hz).
772 ¹H-NMR (CDCl₃) δ: 8.12 (1H, s), 7.89 (1H, s), 6.93 (1H, d, J = 8.3 Hz), 6.91 (1H, d, J = 1.7 Hz), 6.80 (1H, dd, J = 8.3, 2.0 Hz), 2.63 (3H, d, J = 0.5 Hz), 2.36 (3H, s), 2.01-1.95 (2H, m), 1.65 (3H, s), 1.61-1.46 (2H, m), 1.40-1.31 (2H, m), 0.91 (3H, t, J = 7.3 Hz).
773 ¹H-NMR (CDCl₃) δ: 8.20 (1H, d, J = 1.9 Hz), 7.92 (1H, s), 7.76-7.76 (1H, m), 7.38-7.34 (1H, m), 7.03-7.01 (1H, m), 6.88 (1H, d, J = 8.0 Hz), 6.84 (1H, td, J = 5.3, 1.0 Hz), 2.44 (3H, d, J = 1.1 Hz), 2.10-2.03 (1H, m), 1.92 (2H, dd, J = 6.1, 1.9 Hz), 1.71 (3H, s), 1.00 (6H, dd, J = 6.7, 4.0 Hz).
774 ¹H-NMR (CDCl₃) δ: 8.22 (1H, d, J = 4.6 Hz), 7.95 (1H, s), 7.38-7.33 (1H, m), 6.99 (1H, dd, J = 7.6, 1.5 Hz), 6.91 (1H, dd, J = 4.6, 1.1 Hz), 6.88 (1H, dd, J = 8.0, 0.8 Hz), 6.83 (1H, td, J = 5.3, 1.0 Hz), 2.71 (3H, d, J = 0.8 Hz), 2.10-2.03 (1H, m), 1.96-1.88 (2H, m), 1.71 (3H, s), 1.00 (6H, dd, J = 6.9, 3.8 Hz).
775 ¹H-NMR (CDCl₃) δ: 8.11 (1H, s), 7.89 (1H, s), 7.37-7.33 (1H, m), 7.00 (1H, dd, J = 8.0, 1.5 Hz), 6.87 (1H, d, J = 8.4 Hz), 6.83 (1H, td, J = 5.5, 1.1 Hz), 2.63 (3H, s), 2.36 (3H, s), 2.09-2.04 (1H, m), 1.91 (2H, dd, J = 6.1, 3.4 Hz), 1.70 (3H, s), 1.00 (6H, dd, J = 6.7, 4.0 Hz).
776 ¹H-NMR (CDCl₃) δ: 8.20 (1H, d, J = 2.3 Hz), 7.93 (1H, s), 7.76 (1H, s), 7.36 (1H, ddd, J = 8.5, 7.0, 1.2 Hz), 7.03 (1H, dd, J = 7.8, 1.3 Hz), 6.89 (1H, d, J = 8.0 Hz), 6.84 (1H, td, J = 5.3, 1.1 Hz), 2.44 (3H, d, J = 1.1 Hz), 2.01-1.98 (2H, m), 1.67 (3H, s), 1.62-1.50 (2H, m), 1.38-1.32 (2H, m), 0.91 (3H, t, J = 7.3 Hz).
777 ¹H-NMR (CDCl₃) δ: 8.12 (1H, s), 7.90 (1H, s), 7.37-7.33 (1H, m), 7.01 (1H, dd, J = 7.6, 1.5 Hz), 6.90-6.87 (1H, m), 6.83 (1H, td, J = 5.3, 1.1 Hz), 2.63 (3H, s), 2.36 (3H, s), 2.01-1.97 (2H, m), 1.66 (3H, s), 1.62-1.49 (2H, m), 1.39-1.31 (2H, m), 0.91 (3H, t, J = 7.4 Hz).
778 ¹H-NMR (CDCl₃) δ: 8.33 (1H, s), 8.23 (1H, d, J = 5.0 Hz), 7.12 (1H, dd, J = 5.0, 0.8 Hz), 7.03 (1H, d, J = 8.4 Hz), 6.95 (1H, d, J = 1.9 Hz), 6.85 (1H, dd, J = 8.4, 1.9 Hz), 2.73 (3H, s), 1.95 (2H, dd, J = 9.0, 7.8 Hz), 1.64 (3H, s), 1.62-1.47 (2H, m), 0.95 (3H, t, J = 7.4 Hz).
779 ¹H-NMR (CDCl₃) δ: 8.34 (1H, s), 8.29 (1H, s), 6.99 (1H, d, J = 8.4 Hz), 6.96 (1H, d, J = 2.7 Hz), 6.85 (1H, dd, J = 8.2, 2.1 Hz), 2.75 (3H, s), 1.95 (2H, dd, J = 8.6, 7.4 Hz), 1.64 (3H, s), 1.62-1.47 (2H, m), 0.95 (3H, t, J = 7.3 Hz).
780 ¹H-NMR (CDCl₃) δ: 8.12 (1H, s), 7.89 (1H, s), 6.92 (1H, d, J = 7.8 Hz), 6.73 (1H, d, J = 0.5 Hz), 6.68-6.64 (1H, m), 2.63 (3H, s), 2.35 (6H, d, J = 5.9 Hz), 1.71 (6H, s).
781 ¹H-NMR (CDCl₃) δ: 8.23 (1H, d, J = 4.4 Hz), 7.95 (1H, s), 6.91-6.89 (2H, m), 6.74 (1H, d, J = 0.5 Hz), 6.68-6.65 (1H, m), 2.71 (3H, d, J = 1.0 Hz), 2.34 (3H, s), 1.71 (6H, s).
782 ¹H-NMR (CDCl₃) δ: 8.12 (1H, s), 7.90 (1H, s), 6.96 (1H, d, J = 8.0 Hz), 6.93 (1H, d, J = 2.3 Hz), 6.83 (1H, dd, J = 8.4, 1.9 Hz), 2.63 (3H, s), 2.36 (3H, s), 1.71 (6H, s).
783 ¹H-NMR (CDCl₃) δ: 8.20 (1H, d, J = 2.3 Hz), 7.93 (1H, s), 7.77-7.76 (1H, m), 6.98 (1H, d, J = 8.4 Hz), 6.94 (1H, d, J = 1.9 Hz), 6.84 (1H, dd, J = 8.4, 1.9 Hz), 2.45 (3H, d, J = 1.1 Hz), 1.72 (6H, s).
784 ¹H-NMR (CDCl₃) δ: 8.23 (1H, d, J = 4.6 Hz), 7.96 (1H, s), 6.95-6.92 (3H, m), 6.83 (1H, dd, J = 8.2, 2.1 Hz), 2.71 (3H, d, J = 1.1 Hz), 1.72 (6H, s).
785 ¹H-NMR (CDCl₃) δ: 8.41 (1H, s, ), 8.32 (1H, s, ), 7.00-6.97 (2H, m, ), 6.85 (1H, dd, J = 8.4, 2.3 Hz, ), 2.77 (3H, s, ), 2.01 (2H, q, J = 7.4 Hz, ), 1.63 (3H, s, ), 1.05 (3H, t, J = 7.4 Hz, ).
786 ¹H-NMR (CDCl₃) δ: 8.41 (1H, s), 8.31 (1H, s), 6.99-6.96 (2H, m), 6.85 (1H, dd, J = 8.4, 2.3 Hz), 2.77 (3H, s), 1.96-1.94 (2H, m), 1.64 (3H, s), 1.58-1.50 (2H, m), 0.95 (3H, t, J = 7.4 Hz).
787 ¹H-NMR (CDCl₃) δ: 8.30 (1H, s), 8.12 (1H, s), 7.05 (1H, d, J = 8.4 Hz), 6.96 (1H, d, J = 1.9 Hz), 6.86 (1H, dd, J = 8.4, 1.9 Hz), 2.64 (3H, s), 2.38 (3H, s), 1.70 (6H, s).
788 ¹H-NMR (CDCl₃) δ: 8.34 (1H, s), 8.33 (1H, s), 7.40-7.40 (2H, m), 7.07-7.06 (1H, m), 7.02-7.01 (1H, m), 2.76 (3H, s), 1.97-1.93 (2H, m), 1.63 (3H, s), 1.55-1.50 (2H, m), 1.37-1.31 (2H, m), 0.90 (3H, t, J = 7.3 Hz).
789 ¹H-NMR (CDCl₃) δ: 8.35 (1H, s), 8.23 (1H, s), 7.41-7.39 (2H, m), 7.08-7.01 (2H, m), 2.74 (3H, s), 1.96-1.94 (2H, m), 1.63 (3H, s), 1.56-1.50 (2H, m), 1.36-1.30 (2H, m), 0.90 (3H, t, J = 7.3 Hz).
790 ¹H-NMR (CDCl₃) δ: 8.34 (1H, s), 8.17 (1H, d, J = 4.6 Hz), 7.39-7.38 (2H, m), 7.08-7.04 (3H, m), 2.72 (3H, s), 1.97-1.95 (2H, m), 1.65 (3H, s), 1.57-1.50 (2H, m), 1.37-1.31 (2H, m), 0.90 (3H, t, J = 7.4 Hz).
791 ¹H-NMR (CDCl₃) δ: 8.34 (1H, s), 8.32 (1H, s), 7.41-7.39 (2H, m), 7.08-7.00 (2H, m), 2.76 (3H, s), 1.95-1.91 (2H, m), 1.63 (3H, s), 1.60-1.53 (2H, m), 0.94 (3H, t, J = 7.3 Hz).
792 ¹H-NMR (CDCl₃) δ: 8.34 (1H, s), 8.23 (1H, s), 7.40 (2H, dd, J = 4.6, 1.2 Hz), 7.07-7.01 (2H, m), 2.74 (3H, s), 1.94-1.92 (2H, m), 1.64 (3H, s), 1.59-1.55 (2H, m), 0.94 (3H, t, J = 7.3 Hz).
793 ¹H-NMR (CDCl₃) δ: 8.33 (1H, s), 8.17 (1H, d, J = 4.9 Hz), 7.39-7.38 (2H, m), 7.07-7.04 (3H, m), 2.72 (3H, s), 1.95-1.93 (2H, m), 1.64 (3H, s), 1.60-1.54 (2H, m), 0.94 (3H, t, J = 7.3 Hz).
794 ¹H-NMR (CDCl₃) δ: 8.34 (1H, s), 8.32 (1H, s), 7.41-7.39 (2H, m), 7.08-7.04 (1H, m), 7.03-7.01 (1H, m), 2.76 (3H, s), 1.99 (2H, q, J = 7.4 Hz), 1.63 (3H, s), 1.08 (3H, t, J = 7.4 Hz).
795 ¹H-NMR (CDCl₃) δ: 8.34 (1H, s), 8.23 (1H, s), 7.41-7.40 (2H, m), 7.08-7.01 (2H, m), 2.74 (3H, s), 1.99 (2H, q, J = 7.3 Hz), 1.64 (3H, s), 1.08 (3H, t, J = 7.3 Hz).
796 ¹H-NMR (CDCl₃) δ: 8.33 (1H, s), 8.17 (1H, d, J = 5.0 Hz), 7.40-7.38 (2H, m), 7.07-7.04 (3H, m), 2.72 (3H, s), 2.00 (2H, q, J = 7.4 Hz), 1.64 (3H, s), 1.09 (3H, t, J = 7.4 Hz).
797 ¹H-NMR (CDCl₃) δ: 8.30 (1H, s), 8.07 (1H, s), 7.40-7.38 (2H, m), 7.08-7.05 (2H, m), 2.63 (3H, s), 2.36 (3H, s), 1.96-1.91 (2H, m), 1.63 (3H, s), 1.61-1.53 (2H, m), 0.94 (3H, t, J = 7.3 Hz).
798 ¹H-NMR (CDCl₃) δ: 8.30 (1H, s), 8.07 (1H, s), 7.39-7.38 (2H, m), 7.07-7.04 (2H, m), 2.63 (3H, s), 2.36 (3H, s), 1.99 (2H, q, J = 7.3 Hz), 1.64 (3H, s), 1.08 (3H, t, J = 7.3 Hz).
799 ¹H-NMR (CDCl₃) δ: 8.32 (1H, s), 8.23 (1H, d, J = 4.9 Hz), 7.10 (1H, dd, J = 4.9, 0.7 Hz), 6.90 (1H, ddd, J = 8.8, 6.1, 0.5 Hz), 6.55 (1H, t, J = 8.8 Hz), 2.72 (3H, s), 2.14 (3H, d, J = 1.7 Hz), 1.97 (2H, dd, J = 9.0, 7.6 Hz), 1.65 (3H, s), 1.63-1.47 (2H, m), 0.96 (3H, t, J = 7.4 Hz).
800 ¹H-NMR (CDCl₃) δ: 8.33 (1H, s), 8.23 (1H, d, J = 4.6 Hz), 7.11 (1H, d, J = 5.0 Hz), 6.90 (1H, dd, J = 8.8, 6.1 Hz), 6.56 (1H, t, J = 8.8 Hz), 2.72 (3H, s), 2.15 (3H, d, J = 1.5 Hz), 1.98 (2H, dd, J = 9.0, 7.8 Hz), 1.65 (3H, s), 1.55-1.46 (2H, m), 1.35 (2H, td, J = 12.3, 7.4 Hz), 0.91 (3H, t, J = 7.1 Hz).
801 ¹H-NMR (CDCl₃) δ: 8.29 (1H, d, J = 5.0 Hz), 8.08 (1H, s), 7.61 (1H, dd, J = 8.2, 0.6 Hz), 7.13 (1H, dd, J = 5.0, 0.8 Hz), 7.00 (1H, d, J = 8.0 Hz), 2.76 (3H, s), 1.97-1.95 (5H, m), 1.62 (3H, s), 1.56-1.43 (2H, m), 1.36 (2H, td, J = 12.0, 7.3 Hz), 0.93 (3H, t, J = 7.3 Hz).
802 ¹H-NMR (CDCl₃) δ: 8.32 (1H, s), 8.23 (1H, d, J = 5.0 Hz), 7.10 (1H, dd, J = 5.0, 0.8 Hz), 6.91 (1H, dd, J = 8.4, 6.1 Hz), 6.56 (1H, d, J = 8.8 Hz), 2.72 (3H, s), 2.14 (3H, d, J = 1.9 Hz), 2.07-1.99 (1H, m), 1.94-1.92 (2H, m), 1.66 (3H, s), 1.02-0.99 (6H, m).
803 ¹H-NMR (CDCl₃) δ: 8.29 (1H, d, J = 5.0 Hz), 8.07 (1H, s), 7.61 (1H, d, J = 8.4 Hz), 7.13 (1H, dd, J = 5.0, 0.8 Hz), 7.00 (1H, d, J = 8.0 Hz), 2.76 (3H, s), 2.05-1.97 (4H, m), 1.91 (2H, dd, J = 6.1, 1.9 Hz), 1.62 (3H, s), 1.01-0.99 (6H, m).
804 ¹H-NMR (CDCl₃) δ: 8.33 (1H, s), 8.24 (1H, d, J = 5.0 Hz), 7.25-7.21 (1H, m), 7.12 (1H, dd, J = 5.0, 0.8 Hz), 6.89 (1H, dt, J = 7.9, 1.2 Hz), 6.81 (1H, td, J = 6.9, 4.6 Hz), 2.73 (3H, s), 2.07 (2H, q, J = 7.3 Hz), 1.70 (3H, s), 1.09 (3H, t, J = 7.3 Hz).
805 ¹H-NMR (CDCl₃) δ: 8.39 (1H, s), 8.33 (1H, s), 7.51 (1H, dd, J = 8.0, 1.5 Hz), 6.96 (1H, dd, J = 8.0, 1.3 Hz), 6.84 (1H, t, J = 8.0 Hz), 2.77 (3H, s), 1.76 (6H, s).
806 ¹H-NMR (CDCl₃) δ: 8.35 (1H, s), 8.28 (1H, s), 7.51 (1H, dd, J = 7.8, 1.5 Hz), 6.96 (1H, dd, J = 7.8, 1.5 Hz), 6.85 (1H, t, J = 7.8 Hz), 2.75 (3H, s), 1.76 (6H, s).
807 ¹H-NMR (CDCl₃) δ: 8.34 (1H, s), 8.22 (1H, d, J = 5.0 Hz), 7.50 (1H, dd, J = 8.0, 1.5 Hz), 7.12 (1H, d, J = 5.0 Hz), 7.00 (1H, dd, J = 8.0, 1.5 Hz), 6.86-6.82 (1H, m), 2.73 (3H, s), 1.77 (6H, s).
808 ¹H-NMR (CDCl₃) δ: 8.40 (1H, s), 8.32 (1H, s), 7.49 (1H, dd, J = 8.0, 1.5 Hz), 6.95 (1H, dd, J = 8.0, 1.5 Hz), 6.83 (1H, t, J = 8.0 Hz), 2.77 (3H, s), 1.99 (2H, tt, J = 9.7, 4.6 Hz), 1.69 (3H, s), 1.62-1.50 (2H, m), 0.96 (3H, t, J = 7.4 Hz).
809 ¹H-NMR (CDCl₃) δ: 8.34 (1H, s), 8.29 (1H, s), 7.49 (1H, dd, J = 8.0, 1.5 Hz), 6.95 (1H, dd, J = 8.0, 1.5 Hz), 6.83 (1H, t, J = 8.0 Hz), 2.75 (3H, s), 2.02-1.98 (2H, m), 1.69 (3H, s), 1.63-1.50 (2H, m), 0.97 (3H, t, J = 7.4 Hz).
810 ¹H-NMR (CDCl₃) δ: 8.33 (1H, s), 8.23 (1H, d, J = 5.0 Hz), 7.48 (1H, dd, J = 8.0, 1.5 Hz), 7.12 (1H, dd, J = 5.0, 0.8 Hz), 6.99 (1H, dd, J = 8.0, 1.5 Hz), 6.82 (1H, t, J = 8.0 Hz), 2.73 (3H, s), 2.02-1.98 (2H, m), 1.70 (3H, s), 1.63-1.55 (2H, m), 0.96 (3H, t, J = 7.5 Hz).
811 ¹H-NMR (CDCl₃) δ: 8.30 (1H, s), 8.07 (1H, s), 7.40-7.38 (2H, m), 7.06-7.05 (2H, m), 2.63 (3H, s), 2.36 (3H, s), 1.96-1.94 (2H, m), 1.64 (3H, s), 1.55-1.50 (2H, m), 1.34-1.31 (2H, m), 0.90 (3H, t, J = 7.4 Hz).
812 ¹H-NMR (CDCl₃) δ: 8.34 (1H, s, ), 8.23 (1H, d, J = 5.0 Hz, ), 7.12-7.11 (2H, m, ), 6.66 (1H, dd, J = 8.0, 2.5 Hz, ), 6.59 (1H, td, J = 8.0, 2.5 Hz, ), 2.73 (3H, s, ), 1.72 (6H, s, ).
813 ¹H-NMR (CDCl₃) δ: 8.53-8.53 (1H, m), 8.45 (1H, d, J = 2.2 Hz), 8.32 (1H, s), 7.19-7.11 (2H, m), 6.80 (1H, td, J = 6.9, 4.6 Hz), 2.41 (3H, d, J = 1.2 Hz), 2.02-1.98 (2H, m), 1.68 (3H, s), 1.58-1.42 (3H, m), 0.89 (6H, t, J = 6.6 Hz).
814 ¹H-NMR (CDCl₃) δ: 8.48 (1H, d, J = 4.2 Hz), 8.39 (1H, s), 7.19-7.11 (2H, m), 6.83-6.77 (2H, m), 2.85 (3H, d, J = 1.0 Hz), 2.03-1.99 (2H, m), 1.69 (3H, s), 1.57-1.43 (3H, m), 0.91-0.87 (6H, m).
815 ¹H-NMR (CDCl₃) δ: 8.38 (1H, s), 8.34 (1H, s), 7.18-7.12 (2H, m), 6.79 (1H, td, J = 6.9, 4.6 Hz), 2.81 (3H, s), 2.40 (3H, s), 2.02-1.98 (2H, m), 1.58-1.44 (3H, m), 1.69 (3H, s), 0.88 (6H, t, J = 6.8 Hz).
816 ¹H-NMR (CDCl₃) δ: 8.22 (1H, d, J = 4.6 Hz), 7.96 (1H, s), 7.36 (1H, ddd, J = 8.6, 7.0, 1.3 Hz), 7.01-6.99 (1H, m), 6.93-6.88 (2H, m), 6.83 (1H, td, J = 5.3, 1.1 Hz), 2.71 (3H, d, J = 1.2 Hz), 2.02-1.98 (2H, m), 1.67 (3H, s), 1.58-1.39 (3H, m), 0.89 (6H, dd, J = 7.3, 6.3 Hz).
817 ¹H-NMR (CDCl₃) δ: 8.12 (1H, s), 7.90 (1H, s), 7.37-7.33 (1H, m), 7.02 (1H, dd, J = 7.8, 1.5 Hz), 6.89 (1H, dd, J = 8.2, 1.1 Hz), 6.83 (1H, td, J = 5.4, 1.0 Hz), 2.63 (3H, s), 2.36 (3H, s), 2.01-1.96 (2H, m), 1.66 (3H, s), 1.59-1.43 (3H, m), 0.89 (6H, dd, J = 7.4, 6.5 Hz).
818 ¹H-NMR (CDCl₃) δ: 8.12 (1H, s), 7.89 (1H, s), 6.99 (1H, dd, J = 8.6, 6.3 Hz), 6.60 (1H, dd, J = 9.7, 2.5 Hz), 6.52 (1H, td, J = 6.6, 2.5 Hz), 2.63 (3H, s), 2.36 (3H, s), 1.98-1.94 (2H, m), 1.66 (3H, s), 1.64-1.52 (2H, m), 0.95 (3H, t, J = 7.4 Hz).
819 ¹H-NMR (CDCl₃) δ: 8.33 (1H, s), 8.24 (1H, d, J = 5.0 Hz), 7.12-7.08 (2H, m), 6.64 (1H, dd, J = 9.5, 2.3 Hz), 6.57 (1H, td, J = 6.5, 2.4 Hz), 2.73 (3H, s), 1.98-1.93 (2H, m), 1.65 (3H, s), 1.58-1.48 (2H, m), 0.95 (3H, t, J = 7.4 Hz).
820 ¹H-NMR (CDCl₃) δ: 8.34 (1H, s), 8.30 (1H, s), 7.06 (1H, dd, J = 8.8, 6.1 Hz), 6.65 (1H, dd, J = 9.5, 2.7 Hz), 6.58 (1H, td, J = 6.6, 2.5 Hz), 2.75 (3H, s), 1.95 (2H, dd, J = 8.8, 7.6 Hz), 1.64 (3H, s), 1.60-1.49 (2H, m), 0.95 (3H, t, J = 7.4 Hz).
821 ¹H-NMR (CDCl₃) δ: 8.41 (1H, s), 8.31 (1H, s), 7.05 (1H, dd, J = 8.5, 6.1 Hz), 6.65 (1H, dd, J = 9.5, 2.4 Hz), 6.58 (1H, td, J = 6.5, 2.4 Hz), 2.77 (3H, s), 1.98-1.93 (2H, m), 1.64 (3H, s), 1.63-1.46 (2H, m), 0.95 (3H, t, J = 7.3 Hz).
822 ¹H-NMR (CDCl₃) δ: 8.51 (1H, s), 8.32 (1H, s), 7.70 (1H, d, J = 8.3 Hz), 7.44 (1H, dd, J = 8.4, 2.1 Hz), 7.32 (1H, d, J = 2.0 Hz), 2.77 (3H, s), 1.96-1.87 (2H, m), 1.62-1.48 (2H, m), 1.62 (3H, s), 0.96 (3H, t, J = 7.4 Hz).
823 ¹H-NMR (CDCl₃) δ: 8.34-8.33 (2H, m), 7.56-7.51 (2H, m), 7.19 (1H, d, J = 5.0 Hz), 2.76 (3H, s), 1.74 (6H, s).
824 ¹H-NMR (CDCl₃) δ: 8.38 (1H, s), 8.30 (1H, d, J = 3.1 Hz), 7.55 (1H, dd, J = 8.6, 1.7 Hz), 7.48 (1H, dd, J = 8.6, 6.3 Hz), 2.77 (3H, s), 1.74 (6H, s).
825 ¹H-NMR (CDCl₃) δ: 8.12 (1H, s), 7.89 (1H, s), 7.00 (1H, dd, J = 8.7, 6.5 Hz), 6.60 (1H, dd, J = 9.8, 2.4 Hz), 6.52 (1H, td, J = 8.8, 2.4 Hz), 2.63 (3H, d, J = 0.7 Hz), 2.36 (3H, s), 1.98 (2H, dd, J = 8.8, 7.8 Hz), 1.66 (3H, s), 1.58-1.47 (2H, m), 1.39-1.31 (2H, m), 0.91 (3H, t, J = 7.3 Hz).
826 ¹H-NMR (CDCl₃) δ: 8.33 (1H, s), 8.24 (1H, d, J = 4.9 Hz), 7.12-7.08 (2H, m), 6.64 (1H, dd, J = 9.8, 2.4 Hz), 6.57 (1H, td, J = 6.5, 2.4 Hz), 2.73 (3H, d, J = 0.5 Hz), 2.00-1.95 (2H, m), 1.65 (3H, s), 1.57-1.45 (2H, m), 1.38-1.32 (2H, m), 0.91 (3H, t, J = 7.3 Hz).
827 ¹H-NMR (CDCl₃) δ: 8.34 (1H, s), 8.30 (1H, s), 7.07-7.04 (1H, m), 6.65 (1H, dd, J = 9.5, 2.7 Hz), 6.58 (1H, td, J = 6.6, 2.5 Hz), 2.75 (3H, s), 1.97 (2H, dd, J = 8.8, 7.6 Hz), 1.64 (3H, s), 1.56-1.43 (2H, m), 1.37-1.31 (2H, m), 0.91 (3H, t, J = 6.9 Hz).
828 ¹H-NMR (CDCl₃) δ: 8.41 (1H, s), 8.32 (1H, s), 7.06 (1H, dd, J = 8.8, 6.1 Hz), 6.65 (1H, dd, J = 9.5, 2.3 Hz), 6.58 (1H, td, J = 6.4, 2.4 Hz), 2.77 (3H, s), 1.97 (2H, dd, J = 8.8, 7.6 Hz), 1.64 (3H, s), 1.57-1.42 (2H, m), 1.38-1.33 (2H, m), 0.91 (3H, t, J = 7.3 Hz).
829 ¹H-NMR (CDCl₃) δ: 8.29 (1H, s), 8.13 (1H, s), 7.11 (1H, dd, J = 8.7, 6.2 Hz), 6.64 (1H, dd, J = 9.8, 2.4 Hz), 6.56 (1H, td, J = 6.6, 2.5 Hz), 2.64 (3H, s), 2.38 (3H, s), 1.97 (2H, t, J = 8.2 Hz), 1.64 (3H, s), 1.54-1.45 (2H, m), 1.39-1.32 (2H, m), 0.90 (3H, t, J = 7.3 Hz).
830 ¹H-NMR (CDCl₃) δ: 8.28 (1H, s), 8.13 (1H, s), 7.11 (1H, dd, J = 8.8, 6.1 Hz), 6.63 (1H, dd, J = 9.8, 2.4 Hz), 6.56 (1H, td, J = 6.6, 2.5 Hz), 2.64 (3H, s), 2.38 (3H, s), 1.97-1.93 (2H, m), 1.64 (3H, s), 1.61-1.48 (2H, m), 0.95 (3H, t, J = 7.3 Hz).
831 ¹H-NMR (CDCl₃) δ: 8.12 (1H, s), 7.89 (1H, s), 6.94 (1H, d, J = 8.2 Hz), 6.91 (1H, d, J = 2.3 Hz), 6.80 (1H, dd, J = 8.2, 2.3 Hz), 2.63 (3H, s), 2.36 (3H, s), 2.02 (2H, q, J = 7.3 Hz), 1.65 (3H, s), 1.07 (3H, t, J = 7.3 Hz).
832 ¹H-NMR (CDCl₃) δ: 8.20 (1H, d, J = 2.3 Hz), 7.92 (1H, s), 7.77-7.75 (1H, m), 6.96 (1H, d, J = 8.4 Hz), 6.92 (1H, d, J = 1.9 Hz), 6.82 (1H, dd, J = 8.2, 2.1 Hz), 2.44 (3H, d, J = 0.8 Hz), 2.03 (2H, q, J = 7.3 Hz), 1.66 (3H, s), 1.08 (3H, t, J = 7.4 Hz).
833 ¹H-NMR (CDCl₃) δ: 8.22 (1H, d, J = 4.6 Hz), 7.95 (1H, s), 6.94-6.92 (3H, m), 6.81 (1H, dd, J = 8.2, 2.1 Hz), 2.71 (3H, d, J = 1.1 Hz), 2.03 (2H, q, J = 7.4 Hz), 1.66 (3H, s), 1.08 (3H, t, J = 7.4 Hz).
834 ¹H-NMR (CDCl₃) δ: 8.12 (1H, s), 7.89 (1H, s), 6.93 (1H, d, J = 8.4 Hz), 6.90 (1H, d, J = 1.9 Hz), 6.80 (1H, dd, J = 8.4, 1.9 Hz), 2.63 (3H, s), 2.36 (3H, s), 1.99-1.94 (2H, m), 1.66 (3H, s), 1.60-1.50 (2H, m), 0.95 (3H, t, J = 7.4 Hz).
835 ¹H-NMR (CDCl₃) δ: 8.20 (1H, d, J = 2.2 Hz), 7.92 (1H, s), 7.77-7.76 (1H, m), 6.95 (1H, d, J = 8.3 Hz), 6.91 (1H, d, J = 2.2 Hz), 6.81 (1H, dd, J = 8.3, 2.0 Hz), 2.44 (3H, d, J = 1.2 Hz), 1.97 (2H, dd, J = 8.9, 7.7 Hz), 1.66 (3H, s), 1.63-1.50 (2H, m), 0.95 (3H, t, J = 7.4 Hz).
836 ¹H-NMR (CDCl₃) δ: 8.22 (1H, d, J = 4.6 Hz), 7.95 (1H, s), 6.94-6.89 (3H, m), 6.80 (1H, dd, J = 8.4, 1.8 Hz), 2.71 (3H, d, J = 1.2 Hz), 1.97 (2H, dd, J = 9.0, 7.6 Hz), 1.66 (3H, s), 1.64-1.52 (2H, m), 0.95 (3H, t, J = 7.4 Hz).
837 ¹H-NMR (CDCl₃) δ: 8.11 (1H, s), 7.88 (1H, s), 7.23-7.19 (1H, m), 6.84 (1H, dd, J = 7.8, 1.2 Hz), 6.72 (1H, t, J = 7.6 Hz), 2.63 (3H, s), 2.35 (3H, s), 2.23 (3H, s), 2.01-1.95 (2H, m), 1.66 (3H, s), 1.64-1.51 (2H, m), 1.35-1.27 (4H, m), 0.88 (3H, t, J = 7.0 Hz).
838 ¹H-NMR (CDCl₃) δ: 8.19 (1H, d, J = 2.2 Hz), 7.92 (1H, s), 7.76-7.74 (1H, m), 7.24-7.20 (1H, m), 6.87-6.83 (1H, m), 6.74 (1H, t, J = 7.6 Hz), 2.43 (3H, d, J = 1.2 Hz), 2.24 (3H, s), 2.00-1.96 (2H, m), 1.66 (3H, s), 1.62-1.50 (2H, m), 1.35-1.29 (4H, m), 0.88 (3H, t, J = 7.0 Hz).
839 ¹H-NMR (CDCl₃) δ: 8.21 (1H, d, J = 4.6 Hz), 7.94 (1H, s), 7.24-7.20 (1H, m), 6.91-6.89 (1H, m), 6.84-6.81 (1H, m), 6.73 (1H, t, J = 7.6 Hz), 2.71 (3H, d, J = 1.0 Hz), 2.24 (3H, s), 2.02-1.96 (2H, m), 1.67 (3H, s), 1.62-1.51 (2H, m), 1.36-1.29 (4H, m), 0.88 (3H, t, J = 7.0 Hz).
840 ¹H-NMR (CDCl₃) δ: 8.10 (1H, s), 7.88 (1H, s), 7.23-7.19 (1H, m), 6.84-6.82 (1H, m), 6.72 (1H, t, J = 7.6 Hz), 2.63 (3H, d, J = 0.5 Hz), 2.35 (3H, s), 2.23 (3H, s), 2.11-2.06 (1H, m), 1.94 (2H, d, J = 6.1 Hz), 1.68 (3H, s), 1.01 (6H, dd, J = 9.5, 6.6 Hz).
841 ¹H-NMR (CDCl₃) δ: 8.18 (1H, d, J = 2.0 Hz), 7.91 (1H, s), 7.76-7.73 (1H, m), 7.23-7.21 (1H, m), 6.86-6.82 (1H, m), 6.73 (1H, t, J = 7.6 Hz), 2.43 (3H, d, J = 1.2 Hz), 2.23 (3H, s), 2.11-2.05 (1H, m), 1.94 (2H, d, J = 6.1 Hz), 1.68 (3H, s), 1.01 (6H, dd, J = 9.0, 6.6 Hz).
842 ¹H-NMR (CDCl₃) δ: 8.21 (1H, d, J = 4.6 Hz), 7.94 (1H, s), 7.23-7.20 (1H, m), 6.91-6.88 (1H, m), 6.83-6.79 (1H, m), 6.72 (1H, t, J = 7.6 Hz), 2.71 (3H, d, J = 1.0 Hz), 2.23 (3H, s), 2.11-2.05 (1H, m), 1.95 (2H, t, J = 3.3 Hz), 1.69 (3H, s), 1.02 (6H, dd, J = 9.0, 6.6 Hz).
843 ¹H-NMR (CDCl₃) δ: 8.11 (1H, s), 7.88 (1H, s), 6.82 (1H, dd, J = 8.5, 6.3 Hz), 6.50 (1H, t, J = 8.9 Hz), 2.63 (3H, s), 2.35 (3H, s), 2.14 (3H, d, J = 2.0 Hz), 2.03-1.95 (2H, m), 1.66 (3H, s), 1.62-1.45 (2H, m), 1.39-1.31 (2H, m), 0.91 (3H, t, J = 7.3 Hz).
844 ¹H-NMR (CDCl₃) δ: 8.19 (1H, d, J = 2.2 Hz), 7.91 (1H, s), 7.75 (1H, dd, J = 2.1, 1.1 Hz), 6.84 (1H, dd, J = 8.7, 6.2 Hz), 6.52 (1H, t, J = 8.9 Hz), 2.44 (3H, d, J = 1.0 Hz), 2.15 (3H, d, J = 1.7 Hz), 2.03-1.95 (2H, m), 1.66 (3H, s), 1.61-1.47 (2H, m), 1.40-1.31 (2H, m), 0.91 (3H, t, J = 7.3 Hz).
845 ¹H-NMR (CDCl₃) δ: 8.21 (1H, d, J = 4.6 Hz), 7.94 (1H, s), 6.92-6.90 (1H, m), 6.81 (1H, ddd, J = 8.5, 6.3, 0.5 Hz), 6.51 (1H, t, J = 8.9 Hz), 2.71 (3H, d, J = 1.2 Hz), 2.15 (3H, d, J = 2.0 Hz), 2.00 (2H, dd, J = 8.5, 7.5 Hz), 1.66 (3H, s), 1.57-1.47 (2H, m), 1.40-1.31 (2H, m), 0.91 (3H, t, J = 7.3 Hz).
846 ¹H-NMR (CDCl₃) δ: 8.10 (1H, s), 7.87 (1H, s), 6.81 (1H, dd, J = 8.6, 6.3 Hz), 6.50 (1H, t, J = 8.8 Hz), 2.63 (3H, s), 2.35 (3H, s), 2.14 (3H, d, J = 1.5 Hz), 2.10-2.02 (1H, m), 1.94 (2H, d, J = 6.1 Hz), 1.68 (3H, s), 1.01 (6H, dd, J = 9.9, 6.5 Hz).
847 ¹H-NMR (CDCl₃) δ: 8.18 (1H, d, J = 1.9 Hz), 7.90 (1H, s), 7.76-7.74 (1H, m), 6.83 (1H, dd, J = 8.8, 6.5 Hz), 6.52 (1H, t, J = 8.8 Hz), 2.44 (3H, d, J = 0.8 Hz), 2.14 (3H, d, J = 1.5 Hz), 2.11-2.02 (1H, m), 1.94 (2H, d, J = 6.1 Hz), 1.69 (3H, s), 1.01 (6H, dd, J = 9.5, 6.9 Hz).
848 ¹H-NMR (CDCl₃) δ: 8.21 (1H, d, J = 4.6 Hz), 7.93 (1H, s), 6.91 (1H, dd, J = 4.6, 1.1 Hz), 6.80 (1H, dd, J = 8.6, 6.3 Hz), 6.51 (1H, t, J = 9.0 Hz), 2.71 (3H, d, J = 1.1 Hz), 2.14 (3H, d, J = 1.9 Hz), 2.09-2.04 (1H, m), 1.95 (2H, d, J = 6.1 Hz), 1.69 (3H, s), 1.01 (6H, dd, J = 9.5, 6.9 Hz).
849 ¹H-NMR (CDCl₃) δ: 8.50-8.50 (1H, m), 8.39 (1H, d, J = 2.2 Hz), 8.30 (1H, s), 7.39-7.33 (3H, m), 7.11-7.07 (1H, m), 2.39 (3H, d, J = 1.0 Hz), 2.08-2.01 (1H, m), 1.94-1.79 (2H, m), 1.69 (3H, s), 0.99 (6H, dd, J = 26.4, 6.6 Hz).
850 ¹H-NMR (CDCl₃) δ: 8.41 (1H, d, J = 4.4 Hz), 8.38 (1H, s), 7.39-7.32 (3H, m), 7.10-7.06 (1H, m), 6.74 (1H, d, J = 3.4 Hz), 2.83 (3H, d, J = 1.0 Hz), 2.09-2.01 (1H, m), 1.94-1.80 (2H, m), 1.69 (3H, s), 0.99 (6H, dd, J = 27.3, 6.3 Hz).
851 ¹H-NMR (CDCl₃) δ: 8.32 (1H, s), 8.32 (1H, s), 7.39-7.31 (3H, m), 7.09-7.05 (1H, m), 2.80 (3H, s), 2.38 (3H, s), 2.09-2.01 (1H, m), 1.93-1.79 (2H, m), 1.69 (3H, s), 0.99 (6H, dd, J = 27.0, 6.7 Hz).
852 ¹H-NMR (CDCl₃) δ: 8.33 (1H, s), 8.23 (1H, d, J = 5.0 Hz), 7.10 (1H, d, J = 5.0 Hz), 6.91 (1H, dd, J = 8.8, 6.1 Hz), 6.57 (1H, t, J = 9.0 Hz), 2.72 (3H, s), 2.15 (3H, d, J = 1.9 Hz), 1.72 (6H, s).
853 ¹H-NMR (CDCl₃) δ: 8.33 (1H, s), 8.24 (1H, d, J = 4.9 Hz), 7.23 (1H, ddd, J = 10.3, 8.1, 1.5 Hz), 7.12 (1H, dd, J = 4.9, 0.7 Hz), 6.89 (1H, dt, J = 7.8, 1.2 Hz), 6.81 (1H, td, J = 6.8, 4.5 Hz), 2.73 (3H, s), 2.05-1.99 (2H, m), 1.69 (3H, s), 1.59-1.35 (3H, m), 0.90-0.88 (6H, m).
854 ¹H-NMR (CDCl₃) δ: 8.34 (1H, s), 8.30 (1H, s), 7.26-7.24 (1H, m), 6.86-6.79 (2H, m), 2.75 (3H, s), 2.04-1.99 (2H, m), 1.69 (3H, s), 1.50-1.43 (3H, m), 0.90-0.88 (6H, m).
855 ¹H-NMR (CDCl₃) δ: 8.34 (1H, s), 8.24 (1H, d, J = 5.0 Hz), 7.12 (1H, dd, J = 5.0, 0.8 Hz), 7.04 (1H, d, J = 8.4 Hz), 6.95 (1H, d, J = 1.9 Hz), 6.85 (1H, dd, J = 8.4, 2.3 Hz), 2.73 (3H, s), 1.99-1.96 (2H, m), 1.64 (3H, s), 1.58-1.41 (2H, m), 1.35 (2H, td, J = 12.3, 7.4 Hz), 0.91 (3H, t, J = 7.3 Hz).
856 ¹H-NMR (CDCl₃) δ: 8.34 (1H, s), 8.30 (1H, s), 6.99 (1H, d, J = 8.4 Hz), 6.96 (1H, d, J = 1.9 Hz), 6.85 (1H, dd, J = 8.4, 1.9 Hz), 2.75 (3H, s), 1.97 (2H, dd, J = 8.8, 7.6 Hz), 1.64 (3H, s), 1.58-1.41 (2H, m), 1.35 (2H, td, J = 12.2, 7.4 Hz), 0.91 (3H, t, J = 7.3 Hz).
857 ¹H-NMR (CDCl₃) δ: 8.32 (1H, s), 8.24 (1H, d, J = 5.1 Hz), 7.28 (1H, dq, J = 7.4, 0.8 Hz), 7.10 (1H, dd, J = 5.0, 0.9 Hz), 6.86 (1H, dq, J = 7.8, 0.7 Hz), 6.76 (1H, t, J = 7.7 Hz), 2.72 (3H, d, J = 0.5 Hz), 2.24 (3H, s), 2.05-2.01 (2H, m), 1.65 (3H, s), 1.07 (3H, t, J = 7.4 Hz).
858 ¹H-NMR (CDCl₃) δ: 8.33 (1H, s), 8.30 (1H, s), 7.29 (1H, ddd, J = 7.3, 0.9, 0.4 Hz), 6.83 (1H, ddd, J = 7.8, 0.7, 0.4 Hz), 6.77 (1H, t, J = 7.6 Hz), 2.75 (3H, s), 2.24 (3H, s), 2.02 (2H, ddd, J = 14.9, 7.4, 2.2 Hz), 1.64 (3H, s), 1.07 (3H, t, J = 7.4 Hz).
859 ¹H-NMR (CDCl₃) δ: 8.32 (1H, s), 8.24 (1H, d, J = 5.3 Hz), 7.29-7.27 (1H, m), 7.09 (1H, dd, J = 5.0, 0.8 Hz), 6.86 (1H, dd, J = 7.8, 1.0 Hz), 6.76 (1H, t, J = 7.6 Hz), 2.72 (3H, s), 2.23 (3H, s), 2.08-2.01 (1H, m), 1.94-1.93 (2H, m), 1.66 (3H, s), 1.02 (3H, d, J = 6.5 Hz), 0.99 (3H, d, J = 6.5 Hz).
860 ¹H-NMR (CDCl₃) δ: 8.33 (1H, s), 8.29 (1H, s), 7.29 (1H, ddd, J = 7.4, 0.8, 0.4 Hz), 6.84-6.82 (1H, m), 6.77 (1H, t, J = 7.6 Hz), 2.75 (3H, s), 2.24 (3H, s), 2.08-2.00 (1H, m), 1.93-1.93 (2H, m), 1.66 (3H, s), 1.02 (3H, d, J = 6.5 Hz), 0.99 (3H, d, J = 6.9 Hz).
861 ¹H-NMR (CDCl₃) δ: 8.41 (1H, s), 8.32 (1H, s), 7.43 (1H, td, J = 7.6, 1.5 Hz), 7.02 (1H, dd, J = 7.6, 1.5 Hz), 6.93 (1H, dd, J = 7.6, 1.5 Hz), 6.87 (1H, td, J = 7.6, 1.5 Hz), 2.77 (3H, s), 2.05-1.99 (1H, m), 1.91-1.90 (2H, m), 1.68 (3H, s), 1.00 (3H, d, J = 6.9 Hz), 0.98 (3H, d, J = 6.9 Hz).
862 ¹H-NMR (CDCl₃) δ: 8.34 (1H, s), 8.30 (1H, s), 7.44-7.43 (1H, m), 7.03-7.02 (1H, m), 6.94-6.92 (1H, m), 6.88-6.87 (1H, m), 2.75 (3H, s), 2.04-2.02 (1H, m), 1.91-1.90 (2H, m), 1.68 (3H, s), 1.00 (3H, d, J = 6.5 Hz), 0.98 (3H, d, J = 6.5 Hz).
863 ¹H-NMR (CDCl₃) δ: 8.33 (1H, s), 8.24 (1H, d, J = 5.0 Hz), 7.44-7.40 (1H, m), 7.11 (1H, d, J = 5.0 Hz), 7.06 (1H, dd, J = 8.0, 1.5 Hz), 6.92 (1H, dd, J = 8.0, 1.5 Hz), 6.87 (1H, td, J = 8.0, 1.5 Hz), 2.73 (3H, s), 2.05-2.03 (1H, m), 1.92-1.90 (2H, m), 1.68 (3H, s), 1.00 (3H, d, J = 6.5 Hz), 0.99 (3H, d, J = 6.5 Hz).
864 ¹H-NMR (CDCl₃) δ: 8.41 (1H, s), 8.32 (1H, s), 7.27-7.17 (1H, m), 6.86-6.80 (2H, m), 2.77 (3H, s), 2.03-1.99 (2H, m), 1.69 (3H, s), 1.60-1.37 (3H, m), 0.89 (6H, t, J = 6.9 Hz).
865 ¹H-NMR (CDCl₃) δ: 8.41 (1H, s), 8.33 (1H, s), 7.46-7.42 (1H, m), 7.03 (1H, dd, J = 7.6, 1.5 Hz), 6.95 (1H, dd, J = 7.6, 1.5 Hz), 6.88 (1H, t, J = 7.6 Hz), 3.72-3.62 (2H, m), 3.48-3.42 (2H, m), 2.77 (3H, s), 2.31-2.30 (2H, m), 1.69 (3H, s), 1.16 (3H, t, J = 7.1 Hz).
866 ¹H-NMR (CDCl₃) δ: 8.36 (1H, s), 8.30 (1H, s), 7.45-7.43 (1H, m), 7.04-7.02 (1H, m), 6.96-6.94 (1H, m), 6.89-6.88 (1H, m), 3.73-3.61 (2H, m), 3.49-3.41 (2H, m), 2.75 (3H, s), 2.32-2.30 (2H, m), 1.69 (3H, s), 1.16 (3H, t, J = 7.1 Hz).
867 ¹H-NMR (CDCl₃) δ: 8.34 (1H, s), 8.24 (1H, d, J = 5.1 Hz), 7.44-7.42 (1H, m), 7.11 (1H, d, J = 5.1 Hz), 7.07 (1H, dd, J = 8.0, 1.0 Hz), 6.94 (1H, dd, J = 8.0, 1.0 Hz), 6.88 (1H, td, J = 8.0, 1.0 Hz), 3.70-3.66 (2H, m), 3.48-3.42 (2H, m), 2.73 (3H, s), 2.34-2.30 (2H, m), 1.69 (3H, s), 1.17 (3H, t, J = 7.0 Hz).
868 ¹H-NMR (CDCl₃) δ: 8.41 (1H, s), 8.32 (1H, s), 7.00-6.96 (2H, m), 6.85 (1H, dd, J = 8.3, 2.0 Hz), 2.77 (3H, s), 1.99-1.95 (2H, m), 1.64 (3H, s), 1.50-1.46 (2H, m), 1.37-1.33 (2H, m), 0.91 (3H, t, J = 7.3 Hz).
869 ¹H-NMR (CDCl₃) δ: 8.53-8.52 (1H, m), 8.45 (1H, d, J = 2.3 Hz), 8.32 (1H, s), 7.41 (1H, dd, J = 8.0, 1.5 Hz), 7.23 (1H, dd, J = 7.6, 1.5 Hz), 6.82 (1H, t, J = 7.8 Hz), 2.41 (3H, d, J = 0.8 Hz), 2.01-1.97 (2H, m), 1.68 (3H, s), 1.60-1.53 (2H, m), 1.38-1.33 (2H, m), 0.91 (3H, t, J = 7.4 Hz).
870 ¹H-NMR (CDCl₃) δ: 8.47 (1H, d, J = 4.2 Hz), 8.40 (1H, s), 7.41 (1H, dt, J = 8.0, 1.9 Hz), 7.23 (1H, dd, J = 7.8, 1.3 Hz), 6.83-6.79 (2H, m), 2.85 (3H, s), 2.02-1.98 (2H, m), 1.69 (3H, s), 1.60-1.53 (2H, m), 1.39-1.32 (2H, m), 0.91 (3H, t, J = 7.3 Hz).
871 ¹H-NMR (CDCl₃) δ: 8.38 (1H, s), 8.34 (1H, s), 7.40 (1H, dd, J = 7.8, 1.3 Hz), 7.23 (1H, dd, J = 8.0, 1.5 Hz), 6.80 (1H, t, J = 7.8 Hz), 2.81 (3H, s), 2.40 (3H, s), 2.01-1.98 (2H, m), 1.68 (3H, s), 1.61-1.51 (2H, m), 1.39-1.31 (2H, m), 0.90 (3H, t, J = 7.4 Hz).
872 ¹H-NMR (CDCl₃) δ: 8.20 (1H, d, J = 1.9 Hz), 7.93 (1H, s), 7.78-7.76 (1H, m), 7.19-7.15 (1H, m), 6.84 (1H, d, J = 7.6 Hz), 6.80-6.76 (1H, m), 2.45 (3H, d, J = 0.8 Hz), 2.05-2.00 (2H, m), 1.72 (3H, s), 1.66-1.57 (2H, m), 1.35-1.30 (4H, m), 0.88 (3H, t, J = 7.1 Hz).
873 ¹H-NMR (CDCl₃) δ: 8.22 (1H, d, J = 4.6 Hz), 7.96 (1H, s), 7.19-7.15 (1H, m), 6.93 (1H, dd, J = 4.6, 0.8 Hz), 6.82-6.75 (2H, m), 2.72 (3H, d, J = 1.1 Hz), 2.05-2.01 (2H, m), 1.72 (3H, s), 1.66-1.51 (2H, m), 1.33-1.30 (4H, m), 0.88 (3H, t, J = 7.1 Hz).
874 ¹H-NMR (CDCl₃) δ: 8.12 (1H, s), 7.90 (1H, s), 7.18-7.14 (1H, m), 6.83 (1H, d, J = 7.6 Hz), 6.78-6.75 (1H, m), 2.64 (3H, s), 2.36 (3H, s), 2.04-2.00 (2H, m), 1.71 (3H, s), 1.67-1.49 (2H, m), 1.34-1.30 (4H, m), 0.88 (3H, t, J = 7.1 Hz).
875 ¹H-NMR (CDCl₃) δ: 8.21 (1H, d, J = 2.3 Hz), 7.93 (1H, s), 7.76 (1H, s), 7.19-7.15 (1H, m), 6.84 (1H, d, J = 7.6 Hz), 6.79-6.76 (1H, m), 2.44 (3H, d, J = 1.1 Hz), 2.04-1.99 (2H, m), 1.72 (3H, s), 1.68-1.56 (2H, m), 0.96 (3H, t, J = 7.3 Hz).
876 ¹H-NMR (CDCl₃) δ: 8.23 (1H, d, J = 4.6 Hz), 7.96 (1H, s), 7.19-7.15 (1H, m), 6.93 (1H, dd, J = 4.6, 1.1 Hz), 6.81-6.74 (2H, m), 2.72 (3H, d, J = 0.8 Hz), 2.05-2.00 (2H, m), 1.72 (3H, s), 1.68-1.54 (2H, m), 0.96 (3H, t, J = 7.3 Hz).
877 ¹H-NMR (CDCl₃) δ: 8.12 (1H, s), 7.90 (1H, s), 7.18-7.14 (1H, m), 6.82 (1H, d, J = 7.3 Hz), 6.76 (1H, td, J = 6.9, 4.6 Hz), 2.64 (3H, s), 2.36 (3H, s), 2.04-1.99 (2H, m), 1.71 (3H, s), 1.68-1.54 (2H, m), 0.96 (3H, t, J = 7.3 Hz).
878 ¹H-NMR (CDCl₃) δ: 8.04 (1H, s), 7.87 (1H, s), 7.36-7.30 (2H, m), 7.07-7.01 (2H, m), 2.62 (3H, s), 2.33 (3H, s), 2.10-2.02 (1H, m), 1.96-1.81 (2H, m), 1.71 (3H, s), 1.02 (3H, d, J = 6.5 Hz), 0.96 (3H, d, J = 6.5 Hz).
879 ¹H-NMR (CDCl₃) δ: 8.12 (1H, d, J = 2.3 Hz), 7.90 (1H, s), 7.74 (1H, d, J = 0.8 Hz), 7.37-7.31 (2H, m), 7.08-7.03 (2H, m), 2.42 (3H, d, J = 0.8 Hz), 2.10-2.02 (1H, m), 1.96-1.81 (2H, m), 1.72 (3H, s), 1.02 (3H, d, J = 6.9 Hz), 0.96 (3H, d, J = 6.9 Hz).
880 ¹H-NMR (CDCl₃) δ: 8.14 (1H, d, J = 4.6 Hz), 7.93 (1H, s), 7.37-7.31 (2H, m), 7.05-7.01 (2H, m), 6.88 (1H, t, J = 2.3 Hz), 2.70 (3H, s), 2.11-2.02 (1H, m), 1.96-1.82 (2H, m), 1.72 (3H, s), 1.02 (3H, d, J = 6.5 Hz), 0.96 (3H, d, J = 6.5 Hz).
881 ¹H-NMR (CDCl₃) δ: 8.32 (1H, s), 8.24 (1H, d, J = 5.0 Hz), 7.29-7.27 (1H, m), 7.09 (1H, dd, J = 4.6, 0.8 Hz), 6.86 (1H, dd, J = 8.2, 1.3 Hz), 6.76 (1H, t, J = 7.6 Hz), 2.72 (3H, s), 2.24 (3H, s), 1.99-1.96 (2H, m), 1.65 (3H, s), 1.63-1.47 (2H, m), 1.32-1.29 (4H, m), 0.88 (3H, t, J = 7.1 Hz).
882 ¹H-NMR (CDCl₃) δ: 8.33 (1H, s), 8.30 (1H, s), 7.29 (1H, dq, J = 7.3, 0.8 Hz), 6.82 (1H, dd, J = 7.6, 1.5 Hz), 6.77 (1H, t, J = 7.6 Hz), 2.75 (3H, s), 2.24 (3H, s), 1.97 (2H, dd, J = 8.8, 7.6 Hz), 1.64 (3H, s), 1.60-1.45 (2H, m), 1.33-1.29 (4H, m), 0.88 (3H, t, J = 6.9 Hz).
883 ¹H-NMR (CDCl₃) δ: 8.33 (1H, s), 8.24 (1H, d, J = 4.9 Hz), 7.42 (1H, ddd, J = 8.6, 7.0, 1.3 Hz), 7.11 (1H, dd, J = 5.0, 0.9 Hz), 7.06 (1H, dd, J = 7.7, 1.6 Hz), 6.93 (1H, dd, J = 8.3, 1.0 Hz), 6.87 (1H, td, J = 5.5, 1.1 Hz), 2.73 (3H, s), 2.00-1.96 (2H, m), 1.65 (3H, s), 1.58-1.35 (3H, m), 0.89 (6H, t, J = 6.2 Hz).
884 ¹H-NMR (CDCl₃) δ: 8.34 (1H, s), 8.30 (1H, s), 7.43 (1H, ddd, J = 8.6, 7.0, 1.2 Hz), 7.02 (1H, dd, J = 7.7, 1.6 Hz), 6.94 (1H, dd, J = 8.2, 0.9 Hz), 6.87 (1H, td, J = 5.5, 1.1 Hz), 2.75 (3H, s), 1.98 (2H, dd, J = 9.2, 7.4 Hz), 1.64 (3H, s), 1.50-1.42 (3H, m), 0.89 (6H, t, J = 6.2 Hz).
885 ¹H-NMR (CDCl₃) δ: 8.41 (1H, s), 8.32 (1H, s), 7.43 (1H, ddd, J = 8.5, 7.1, 1.2 Hz), 7.02 (1H, dd, J = 7.8, 1.7 Hz), 6.94 (1H, dd, J = 8.2, 1.1 Hz), 6.87 (1H, td, J = 5.4, 1.1 Hz), 2.77 (3H, s), 1.98 (2H, dd, J = 9.2, 7.2 Hz), 1.64 (3H, s), 1.49-1.42 (3H, m), 0.89 (6H, t, J = 6.2 Hz).
886 ¹H-NMR (CDCl₃) δ: 8.36 (1H, s), 8.16 (1H, d, J = 5.0 Hz), 7.40-7.39 (2H, m), 7.07-7.06 (3H, m), 2.72 (3H, s), 2.10-2.02 (1H, m), 1.93-1.85 (2H, m), 1.68 (3H, s), 1.02 (3H, d, J = 6.9 Hz), 0.96 (3H, d, J = 6.9 Hz).
887 ¹H-NMR (CDCl₃) δ: 8.37 (1H, s), 8.22 (1H, s), 7.41-7.40 (2H, m), 7.07-7.05 (2H, m), 2.74 (3H, s), 2.07-2.03 (1H, m), 1.93-1.85 (2H, m), 1.67 (3H, s), 1.02 (3H, d, J = 6.9 Hz), 0.96 (3H, d, J = 6.5 Hz).
888 ¹H-NMR (CDCl₃) δ: 8.35 (1H, s), 8.33 (1H, s), 7.41-7.40 (2H, m), 7.09-7.02 (2H, m), 2.76 (3H, s), 2.09-2.01 (1H, m), 1.93-1.85 (2H, m), 1.67 (3H, s), 1.02 (3H, d, J = 6.5 Hz), 0.96 (3H, d, J = 6.9 Hz).
889 ¹H-NMR (CDCl₃) δ: 8.29 (1H, s), 8.14 (1H, s), 7.41 (1H, ddd, J = 8.6, 7.0, 1.3 Hz), 7.07 (1H, dd, J = 7.9, 1.6 Hz), 6.92 (1H, dd, J = 8.3, 0.7 Hz), 6.86 (1H, td, J = 5.5, 1.1 Hz), 2.64 (3H, s), 2.38 (3H, s), 1.97 (2H, dd, J = 9.0, 7.6 Hz), 1.64 (3H, s), 1.48-1.42 (3H, m), 0.89 (6H, t, J = 6.2 Hz).
890 ¹H-NMR (CDCl₃) δ: 8.32 (1H, s), 8.06 (1H, s), 7.39-7.38 (2H, m), 7.10-7.03 (2H, m), 2.63 (3H, s), 2.35 (3H, s), 2.10-2.01 (1H, m), 1.89 (2H, ddd, J = 19.0, 13.3, 4.8 Hz), 1.67 (3H, s), 1.02 (3H, d, J = 6.6 Hz), 0.96 (3H, d, J = 6.8 Hz).
891 ¹H-NMR (CDCl₃) δ: 8.34 (1H, s), 8.17 (1H, d, J = 5.0 Hz), 7.39-7.38 (2H, m), 7.07-7.05 (3H, m), 2.72 (3H, s), 1.95 (2H, dd, J = 10.1, 6.7 Hz), 1.64 (3H, s), 1.58-1.53 (2H, m), 1.35-1.25 (4H, m), 0.87 (3H, t, J = 6.9 Hz).
892 ¹H-NMR (CDCl₃) δ: 8.29 (1H, s), 8.13 (1H, s), 7.05 (1H, d, J = 8.4 Hz), 6.95-6.95 (1H, m), 6.84 (1H, dd, J = 8.4, 1.9 Hz), 2.64 (3H, s), 2.38 (3H, s), 2.01-2.00 (2H, m), 1.63 (3H, s), 1.05 (3H, t, J = 7.4 Hz).
893 ¹H-NMR (CDCl₃) δ: 8.41 (1H, s), 8.31 (1H, s), 7.29 (1H, d, J = 7.5 Hz), 6.82 (1H, d, J = 7.5 Hz), 6.77 (1H, t, J = 7.5 Hz), 2.77 (3H, s), 2.24 (3H, s), 2.03-2.00 (2H, m), 1.64 (3H, s), 1.07 (3H, t, J = 7.4 Hz).
894 ¹H-NMR (CDCl₃) δ: 8.41 (1H, s), 8.30 (1H, s), 7.30-7.28 (1H, m), 6.82-6.81 (1H, m), 6.77 (1H, t, J = 7.6 Hz), 2.76 (3H, s), 2.24 (3H, s), 1.98-1.96 (2H, m), 1.64 (3H, s), 1.53-1.47 (2H, m), 1.32-1.30 (4H, m), 0.88 (3H, t, J = 7.1 Hz).
895 ¹H-NMR (CDCl₃) δ: 8.33 (1H, s), 8.31 (1H, s), 6.89 (1H, d, J = 8.0 Hz), 6.76 (1H, s), 6.68 (1H, d, J = 8.0 Hz), 2.75 (3H, s), 2.35 (3H, s), 1.97-1.96 (2H, m), 1.63 (3H, s), 1.51-1.49 (2H, m), 1.36-1.33 (2H, m), 0.90 (3H, t, J = 7.3 Hz).
896 ¹H-NMR (CDCl₃) δ: 8.32 (1H, s), 8.25 (1H, d, J = 4.6 Hz), 7.10 (1H, d, J = 4.6 Hz), 6.93 (1H, d, J = 8.0 Hz), 6.75 (1H, s), 6.68-6.67 (1H, m), 2.73 (3H, s), 2.34 (3H, s), 1.98-1.96 (2H, m), 1.63 (3H, s), 1.50-1.47 (2H, m), 1.37-1.31 (2H, m), 0.90 (3H, t, J = 7.3 Hz).
897 ¹H-NMR (CDCl₃) δ: 8.36 (1H, s), 8.22 (1H, s), 7.22 (1H, t, J = 8.0 Hz), 7.06-7.04 (1H, m), 6.89 (1H, d, J = 8.0 Hz), 2.74 (3H, s), 1.98-1.96 (2H, m), 1.64 (3H, s), 1.56-1.52 (2H, m), 1.35-1.32 (2H, m), 0.91 (3H, t, J = 7.3 Hz).
898 ¹H-NMR (CDCl₃) δ: 8.35 (1H, s, ), 8.16 (1H, d, J = 5.0 Hz, ), 7.21 (1H, t, J = 8.4 Hz, ), 7.08 (1H, d, J = 5.0 Hz, ), 7.06-7.01 (1H, m, ), 6.92 (1H, d, J = 8.4 Hz, ), 2.72 (3H, s, ), 1.98-1.97 (2H, m, ), 1.65 (3H, s, ), 1.59-1.53 (2H, m, ), 1.35-1.32 (2H, m, ), 0.91 (3H, t, J = 7.4 Hz, ).
899 ¹H-NMR (CDCl₃) δ: 8.30 (1H, s), 8.12 (1H, s), 7.49 (1H, dd, J = 8.0, 1.5 Hz), 7.01 (1H, dd, J = 8.0, 1.5 Hz), 6.83 (1H, t, J = 8.0 Hz), 2.64 (3H, s), 2.38 (3H, s), 1.76 (6H, s).
900 ¹H-NMR (CDCl₃) δ: 8.29 (1H, s), 8.12 (1H, s), 7.47 (1H, dd, J = 7.8, 1.5 Hz), 7.00 (1H, dd, J = 7.8, 1.5 Hz), 6.81 (1H, t, J = 7.8 Hz), 2.64 (3H, s), 2.38 (3H, s), 2.00-1.98 (2H, m), 1.69 (3H, s), 1.60-1.50 (2H, m), 0.96 (3H, t, J = 7.4 Hz).
901 ¹H-NMR (CDCl₃) δ: 8.29 (1H, s), 8.13 (1H, s), 7.43-7.39 (1H, m), 7.08 (1H, dd, J = 7.8, 1.7 Hz), 6.91-6.90 (1H, m), 6.87-6.85 (1H, m), 2.64 (3H, s), 2.38 (3H, s), 2.05-2.02 (1H, m), 1.91-1.89 (2H, m), 1.68 (3H, s), 1.01-0.97 (6H, m).
902 ¹H-NMR (CDCl₃) δ: 8.29 (1H, s), 8.13 (1H, s), 7.24-7.21 (1H, m), 6.91-6.89 (1H, m), 6.82-6.80 (1H, m), 2.64 (3H, s), 2.38 (3H, s), 2.03-1.99 (2H, m), 1.90-1.89 (1H, m), 1.68 (3H, s), 1.51-1.40 (2H, m), 0.90-0.87 (6H, m).
903 ¹H-NMR (CDCl₃) δ: 8.35 (1H, s), 8.23 (1H, s), 7.41-7.40 (2H, m), 7.08-7.05 (1H, m), 7.02 (1H, dt, J = 7.9, 1.1 Hz), 2.74 (3H, s), 1.96-1.92 (2H, m), 1.64 (3H, s), 1.56-1.52 (2H, m), 1.34-1.25 (4H, m), 0.87 (3H, t, J = 7.1 Hz).
904 ¹H-NMR (CDCl₃) δ: 8.34 (1H, s), 8.33 (1H, s), 7.40-7.40 (2H, m), 7.08-7.05 (1H, m), 7.02 (1H, dt, J = 7.8, 1.1 Hz), 2.76 (3H, s), 1.96-1.92 (2H, m), 1.63 (3H, s), 1.57-1.52 (2H, m), 1.34-1.25 (4H, m), 0.87 (3H, t, J = 7.1 Hz).
905 ¹H-NMR (CDCl₃) δ: 8.12 (1H, s), 7.91 (1H, s), 7.43 (1H, dd, J = 7.8, 1.3 Hz), 6.97-6.93 (1H, m), 6.80 (1H, t, J = 8.0 Hz), 2.63 (3H, s), 2.36 (3H, s), 1.77 (6H, s).
906 ¹H-NMR (CDCl₃) δ: 8.21 (1H, d, J = 2.3 Hz), 7.94 (1H, s), 7.78-7.76 (1H, m), 7.44 (1H, dd, J = 8.0, 1.5 Hz), 6.97 (1H, dd, J = 8.0, 1.5 Hz), 6.82 (1H, t, J = 8.0 Hz), 2.45 (3H, d, J = 0.8 Hz), 1.77 (6H, s).
907 ¹H-NMR (CDCl₃) δ: 8.23 (1H, d, J = 4.6 Hz), 7.97 (1H, s), 7.43 (1H, dd, J = 8.0, 1.5 Hz), 6.95-6.92 (2H, m), 6.81 (1H, t, J = 7.8 Hz), 2.72 (3H, d, J = 1.1 Hz), 1.78 (6H, s).
908 ¹H-NMR (CDCl₃) δ: 8.12 (1H, s), 7.90 (1H, s), 7.41 (1H, dd, J = 7.8, 1.3 Hz), 6.92 (1H, dd, J = 8.0, 1.5 Hz), 6.77 (1H, t, J = 7.8 Hz), 2.63 (3H, s), 2.36 (3H, s), 2.03-1.98 (2H, m), 1.71 (3H, s), 1.67-1.58 (2H, m), 0.96 (3H, t, J = 7.3 Hz).
909 ¹H-NMR (CDCl₃) δ: 8.20 (1H, d, J = 2.3 Hz), 7.93 (1H, s), 7.76 (1H, dd, J = 2.3, 1.1 Hz), 7.42 (1H, dd, J = 8.0, 1.5 Hz), 6.94 (1H, dd, J = 7.6, 1.5 Hz), 6.79 (1H, t, J = 7.8 Hz), 2.44 (3H, d, J = 1.1 Hz), 2.02-1.98 (2H, m), 1.72 (3H, s), 1.66-1.58 (2H, m), 0.96 (3H, t, J = 7.3 Hz).
910 ¹H-NMR (CDCl₃) δ: 8.22 (1H, d, J = 4.6 Hz), 7.96 (1H, s), 7.41 (1H, dd, J = 7.8, 1.3 Hz), 6.91 (2H, ddd, J = 10.0, 6.2, 1.2 Hz), 6.78 (1H, t, J = 7.8 Hz), 2.71 (3H, d, J = 1.1 Hz), 2.03-1.99 (2H, m), 1.72 (3H, s), 1.67-1.59 (2H, m), 0.96 (3H, t, J = 7.4 Hz).
911 ¹H-NMR (CDCl₃) δ: 8.12 (1H, s), 7.90 (1H, s), 7.41 (1H, dd, J = 8.1, 1.5 Hz), 6.93 (1H, dd, J = 7.8, 1.5 Hz), 6.77 (1H, t, J = 7.8 Hz), 2.63 (3H, d, J = 0.7 Hz), 2.36 (3H, s), 2.05-1.99 (2H, m), 1.71 (3H, s), 1.63-1.53 (2H, m), 1.40-1.31 (2H, m), 0.91 (3H, t, J = 7.3 Hz).
912 ¹H-NMR (CDCl₃) δ: 8.20 (1H, d, J = 2.2 Hz), 7.93 (1H, s), 7.78-7.75 (1H, m), 7.42 (1H, dd, J = 8.1, 1.5 Hz), 6.94 (1H, dd, J = 7.7, 1.6 Hz), 6.79 (1H, t, J = 7.8 Hz), 2.44 (3H, d, J = 1.2 Hz), 2.05-1.98 (2H, m), 1.72 (3H, s), 1.62-1.53 (2H, m), 1.39-1.32 (2H, m), 0.91 (3H, t, J = 7.3 Hz).
913 ¹H-NMR (CDCl₃) δ: 8.22 (1H, d, J = 4.6 Hz), 7.96 (1H, s), 7.42 (1H, dd, J = 7.9, 1.6 Hz), 6.94-6.89 (2H, m), 6.78 (1H, t, J = 7.8 Hz), 2.71 (3H, d, J = 1.2 Hz), 2.05-2.00 (2H, m), 1.72 (3H, s), 1.62-1.53 (2H, m), 1.41-1.31 (2H, m), 0.91 (3H, t, J = 7.3 Hz).
914 ¹H-NMR (CDCl₃) δ: 8.11 (1H, s), 7.89 (1H, s), 7.41 (1H, dd, J = 8.0, 1.5 Hz), 6.92 (1H, dd, J = 7.6, 1.5 Hz), 6.77 (1H, t, J = 7.8 Hz), 2.63 (3H, s), 2.36 (3H, s), 2.11-2.05 (1H, m), 1.98-1.94 (2H, m), 1.74 (3H, s), 1.02 (6H, d, J = 6.5 Hz).
915 ¹H-NMR (CDCl₃) δ: 8.20 (1H, d, J = 2.3 Hz), 7.92 (1H, s), 7.78-7.75 (1H, m), 7.42 (1H, dd, J = 8.0, 1.5 Hz), 6.94 (1H, dd, J = 8.0, 1.5 Hz), 6.79 (1H, t, J = 7.8 Hz), 2.44 (3H, d, J = 1.1 Hz), 2.11-2.06 (1H, m), 1.99-1.94 (2H, m), 1.74 (3H, s), 1.03 (6H, d, J = 6.5 Hz).
916 ¹H-NMR (CDCl₃) δ: 8.22 (1H, d, J = 4.2 Hz), 7.95 (1H, s), 7.42 (1H, dd, J = 8.0, 1.5 Hz), 6.92 (1H, dd, J = 4.6, 1.1 Hz), 6.90 (1H, dd, J = 7.6, 1.5 Hz), 6.78 (1H, t, J = 7.8 Hz), 2.71 (3H, d, J = 0.8 Hz), 2.11-2.06 (1H, m), 2.00-1.93 (2H, m), 1.74 (3H, s), 1.03 (6H, d, J = 6.9 Hz).
917 ¹H-NMR (CDCl₃) δ: 8.20 (1H, d, J = 2.3 Hz), 7.93 (1H, s), 7.76 (1H, d, J = 1.1 Hz), 7.38-7.34 (1H, m), 7.03 (1H, dd, J = 7.6, 1.5 Hz), 6.90 (1H, d, J = 9.2 Hz), 6.84 (1H, td, J = 5.3, 1.0 Hz), 2.44 (3H, d, J = 1.1 Hz), 2.01-1.97 (2H, m), 1.66 (3H, s), 1.55-1.42 (3H, m), 0.89 (6H, dd, J = 9.2, 6.5 Hz).
918 ¹H-NMR (CDCl₃) δ: 8.53-8.52 (1H, m), 8.44 (1H, d, J = 2.2 Hz), 8.32 (1H, s), 7.41 (1H, dd, J = 7.9, 1.6 Hz), 7.24 (1H, dd, J = 7.8, 1.5 Hz), 6.82 (1H, t, J = 7.8 Hz), 2.41 (3H, d, J = 1.0 Hz), 2.10-2.04 (1H, m), 1.94-1.92 (2H, m), 1.71 (3H, s), 1.02 (6H, d, J = 6.8 Hz).
919 ¹H-NMR (CDCl₃) δ: 8.47 (1H, d, J = 4.2 Hz), 8.40 (1H, s), 7.41 (1H, dd, J = 8.0, 1.5 Hz), 7.24 (1H, dd, J = 7.6, 1.5 Hz), 6.83-6.79 (2H, m), 2.85 (3H, s), 2.10-2.04 (1H, m), 1.95-1.93 (2H, m), 1.72 (3H, s), 1.02 (6H, d, J = 6.9 Hz).
920 ¹H-NMR (CDCl₃) δ: 8.37 (1H, s), 8.34 (1H, s), 7.40 (1H, dd, J = 8.0, 1.5 Hz), 7.26-7.24 (1H, m), 6.80 (1H, t, J = 7.8 Hz), 2.81 (3H, s), 2.40 (3H, s), 2.08-2.05 (1H, m), 1.93 (2H, dd, J = 6.1, 1.5 Hz), 1.71 (3H, s), 1.02 (6H, dd, J = 6.5, 0.8 Hz).
921 ¹H-NMR (CDCl₃) δ: 8.38 (1H, s), 8.35 (1H, s), 7.40-7.36 (2H, m), 6.93-6.89 (2H, m), 4.20-4.11 (2H, m), 2.97 (2H, s), 2.81 (3H, s), 2.40 (3H, s), 1.86 (3H, s), 1.25 (3H, t, J = 7.1 Hz).
922 ¹H-NMR (CDCl₃) δ: 8.53-8.53 (1H, m), 8.45 (1H, d, J = 2.2 Hz), 8.33 (1H, s), 7.41-7.35 (2H, m), 6.94-6.90 (2H, m), 4.17-4.10 (2H, m), 2.96 (2H, s), 2.41 (3H, s), 1.86 (3H, s), 1.28-1.23 (3H, m).
923 ¹H-NMR (CDCl₃) δ: 8.47 (1H, d, J = 1.1 Hz), 8.41 (1H, s), 7.40-7.36 (2H, m), 6.93-6.90 (2H, m), 6.73 (1H, d, J = 4.2 Hz), 4.19-4.10 (2H, m), 2.97 (2H, s), 2.85 (3H, s), 1.86 (3H, s), 1.25 (3H, t, J = 7.3 Hz).
924 ¹H-NMR (CDCl₃) δ: 8.22 (1H, d, J = 4.4 Hz), 7.97 (1H, s), 7.41-7.37 (1H, m), 7.04 (1H, dd, J = 7.6, 1.5 Hz), 6.94-6.87 (3H, m), 4.20-4.08 (2H, m), 3.01 (2H, d, J = 2.0 Hz), 2.72 (3H, s), 1.88 (3H, s), 1.25 (3H, t, J = 7.3 Hz).
925 ¹H-NMR (CDCl₃) δ: 8.12 (1H, s), 7.91 (1H, s), 7.41-7.36 (1H, m), 7.07 (1H, dd, J = 7.7, 1.6 Hz), 6.94-6.86 (2H, m), 4.20-4.09 (2H, m), 3.00 (2H, d, J = 1.7 Hz), 2.64 (3H, s), 1.87 (3H, s), 2.36 (3H, s), 1.25 (3H, t, J = 7.2 Hz).
926 ¹H-NMR (CDCl₃) δ: 8.52-8.50 (1H, m), 8.39 (1H, d, J = 2.2 Hz), 8.30 (1H, s), 7.42 (1H, dt, J = 7.8, 1.0 Hz), 7.37-7.36 (2H, m), 7.15-7.11 (1H, m), 4.22-4.10 (2H, m), 2.98 (1H, d, J = 13.7 Hz), 2.82 (1H, d, J = 13.7 Hz), 2.39 (3H, d, J = 1.0 Hz), 1.89 (3H, s), 1.27 (3H, t, J = 7.3 Hz).
927 ¹H-NMR (CDCl₃) δ: 8.47 (1H, d, J = 4.2 Hz), 8.38 (1H, s), 7.43-7.41 (1H, m), 7.38-7.36 (2H, m), 7.15-7.10 (1H, m), 6.76 (1H, dd, J = 4.2, 0.7 Hz), 4.22-4.10 (2H, m), 2.99 (1H, d, J = 13.4 Hz), 2.85-2.84 (3H, m), 2.83 (1H, d, J = 8.8 Hz), 1.90 (3H, s), 1.27 (3H, t, J = 7.3 Hz).
928 ¹H-NMR (CDCl₃) δ: 8.32 (2H, s), 7.43-7.41 (1H, m), 7.36-7.35 (2H, m), 7.14-7.09 (1H, m), 4.23-4.12 (2H, m), 2.98 (1H, d, J = 13.4 Hz), 2.82 (1H, d, J = 14.9 Hz), 2.80 (3H, s), 2.38 (3H, s), 1.89 (3H, s), 1.29-1.25 (3H, m).
929 ¹H-NMR (CDCl₃) δ: 8.41 (1H, s), 8.31 (1H, s), 7.29 (1H, d, J = 7.4 Hz), 6.83-6.82 (1H, m), 6.77 (1H, t, J = 7.4 Hz), 2.77 (3H, s), 2.24 (3H, s), 2.06-2.01 (1H, m), 1.93 (2H, d, J = 6.1 Hz), 1.66 (3H, s), 1.02 (3H, d, J = 6.5 Hz), 0.99 (3H, d, J = 6.5 Hz).
930 ¹H-NMR (CDCl₃) δ: 8.42 (1H, s), 8.31 (1H, s), 6.89 (1H, d, J = 8.0 Hz), 6.76 (1H, s), 6.68 (1H, d, J = 8.0 Hz), 2.77 (3H, s), 2.35 (3H, s), 1.96 (2H, t, J = 8.2 Hz), 1.63 (3H, s), 1.51 (2H, td, J = 13.1, 7.8 Hz), 1.34 (2H, dd, J = 14.7, 7.4 Hz), 0.90 (3H, t, J = 7.3 Hz).
931 ¹H-NMR (CDCl₃) δ: 8.33 (1H, s), 8.33 (1H, s), 7.23-7.21 (1H, m), 7.05-7.04 (1H, m), 6.90-6.88 (1H, m), 2.76 (3H, s), 1.97-1.96 (2H, m), 1.64 (3H, s), 1.55-1.50 (2H, m), 1.35-1.33 (2H, m), 0.91 (3H, t, J = 7.3 Hz).
932 ¹H-NMR (CDCl₃) δ: 8.31 (1H, s), 8.13 (1H, s), 7.43-7.42 (1H, m), 7.09 (1H, d, J = 8.0 Hz), 6.94 (1H, d, J = 8.0 Hz), 6.87 (1H, t, J = 8.0 Hz), 3.70-3.64 (2H, m), 3.47-3.44 (2H, m), 2.64 (3H, s), 2.38 (3H, s), 2.32-2.30 (2H, m), 1.68 (3H, s), 1.17 (3H, t, J = 7.1 Hz).
933 ¹H-NMR (CDCl₃) δ: 8.29 (1H, s), 8.13 (1H, s), 7.05 (1H, d, J = 8.4 Hz), 6.95 (1H, d, J = 1.9 Hz), 6.84 (1H, dd, J = 8.4, 1.9 Hz), 2.64 (3H, s), 2.38 (3H, s), 1.96 (2H, t, J = 8.2 Hz), 1.63 (3H, s), 1.53-1.44 (2H, m), 1.36-1.33 (2H, m), 0.90 (3H, t, J = 7.3 Hz).
934 ¹H-NMR (CDCl₃) δ: 8.28 (1H, s), 8.14 (1H, s), 6.95 (1H, d, J = 7.8 Hz), 6.74 (1H, br s), 6.66 (1H, d, J = 7.8 Hz), 2.64 (3H, s), 2.38 (3H, s), 2.34 (3H, s), 1.96 (2H, t, J = 8.2 Hz), 1.63 (3H, s), 1.53-1.44 (2H, m), 1.36-1.33 (2H, m), 0.90 (3H, t, J = 7.3 Hz).
935 ¹H-NMR (CDCl₃) δ: 8.28 (1H, s), 8.13 (1H, s), 7.29-7.27 (1H, m), 6.87 (1H, d, J = 7.6 Hz), 6.76 (1H, t, J = 7.6 Hz), 2.64 (3H, s), 2.37 (3H, s), 2.24 (3H, s), 2.05-1.99 (2H, m), 1.64 (3H, s), 1.07 (3H, t, J = 7.4 Hz).
936 ¹H-NMR (CDCl₃) δ: 8.27 (1H, s), 8.13 (1H, s), 7.28-7.26 (1H, m), 6.86 (1H, d, J = 7.6 Hz), 6.75 (1H, t, J = 7.6 Hz), 2.64 (3H, s), 2.37 (3H, s), 2.23 (3H, s), 1.97 (2H, t, J = 8.2 Hz), 1.64 (3H, s), 1.55-1.50 (2H, m), 1.34-1.28 (4H, m), 0.88 (3H, t, J = 7.4 Hz).
937 ¹H-NMR (CDCl₃) δ: 8.34 (1H, s), 8.29 (1H, s), 7.49 (1H, dd, J = 7.8, 1.5 Hz), 6.95 (1H, dd, J = 7.8, 1.5 Hz), 6.83 (1H, t, J = 7.8 Hz), 2.75 (3H, s), 2.02-2.00 (2H, m), 1.69 (3H, s), 1.59-1.48 (2H, m), 1.38-1.34 (2H, m), 0.91 (3H, t, J = 7.3 Hz).
938 ¹H-NMR (CDCl₃) δ: 8.33 (1H, s), 8.23 (1H, d, J = 5.0 Hz), 7.48 (1H, dd, J = 8.0, 1.5 Hz), 7.12 (1H, d, J = 5.0 Hz), 6.99 (1H, dd, J = 8.0, 1.5 Hz), 6.82 (1H, t, J = 8.0 Hz), 2.73 (3H, s), 2.02-2.00 (2H, m), 1.70 (3H, s), 1.59-1.52 (2H, m), 1.37-1.35 (2H, m), 0.91 (3H, t, J = 7.4 Hz).
939 ¹H-NMR (CDCl₃) δ: 8.28 (1H, s), 8.13 (1H, s), 7.28-7.26 (1H, m), 6.88 (1H, d, J = 7.6 Hz), 6.75 (1H, t, J = 7.6 Hz), 2.64 (3H, s), 2.37 (3H, s), 2.23 (3H, s), 2.07-2.01 (1H, m), 1.93-1.92 (2H, m), 1.66 (3H, s), 1.02 (3H, d, J = 6.9 Hz), 0.98 (3H, d, J = 6.9 Hz).
940 ¹H-NMR (CDCl₃) δ: 8.31 (1H, s), 8.05 (1H, s), 7.21-7.19 (1H, m), 7.06-7.01 (1H, m), 6.93 (1H, d, J = 6.9 Hz), 2.63 (3H, s), 2.36 (3H, s), 1.98-1.96 (2H, m), 1.64 (3H, s), 1.55-1.53 (2H, m), 1.35-1.32 (2H, m), 0.91 (3H, t, J = 7.3 Hz).
941 ¹H-NMR (CDCl₃) δ: 8.19 (1H, d, J = 2.3 Hz), 7.92 (1H, s), 7.75 (1H, dd, J = 2.3, 1.1 Hz), 7.22 (1H, dt, J = 6.5, 0.8 Hz), 6.85 (1H, dd, J = 7.6, 1.1 Hz), 6.74 (1H, t, J = 7.4 Hz), 2.43 (3H, d, J = 1.1 Hz), 2.24 (3H, s), 2.01-1.97 (2H, m), 1.66 (3H, s), 1.62-1.49 (2H, m), 1.38-1.32 (2H, m), 0.91 (3H, t, J = 7.4 Hz).
942 ¹H-NMR (CDCl₃) δ: 8.21 (1H, d, J = 2.3 Hz), 7.93 (1H, s), 7.78-7.75 (1H, m), 7.19-7.15 (1H, m), 6.85-6.83 (1H, m), 6.80-6.75 (1H, m), 2.44 (3H, d, J = 1.1 Hz), 2.05-2.01 (2H, m), 1.71 (3H, s), 1.65-1.51 (2H, m), 1.40-1.32 (2H, m), 0.91 (3H, t, J = 7.4 Hz).
943 ¹H-NMR (CDCl₃) δ: 8.13 (1H, d, J = 2.3 Hz), 7.89 (1H, s), 7.74 (1H, s), 7.36-7.31 (2H, m), 7.08-7.02 (2H, m), 2.42 (3H, s), 2.02-1.90 (2H, m), 1.65 (3H, s), 1.60-1.53 (2H, m), 1.35-1.29 (2H, m), 0.90 (3H, t, J = 7.3 Hz).
944 ¹H-NMR (CDCl₃) δ: 8.04 (1H, s), 7.88 (1H, s), 7.39-7.34 (2H, m), 7.12-7.05 (2H, m), 4.23-4.12 (2H, m), 3.02-2.83 (2H, m), 2.63 (3H, s), 2.34 (3H, s), 1.91 (3H, s), 1.27 (3H, t, J = 7.1 Hz).
945 ¹H-NMR (CDCl₃) δ: 8.12 (1H, d, J = 1.9 Hz), 7.92 (1H, s), 7.75 (1H, d, J = 1.1 Hz), 7.39-7.35 (2H, m), 7.14-7.06 (2H, m), 4.23-4.12 (2H, m), 3.00-2.86 (2H, m), 2.42 (3H, s), 1.91 (3H, s), 1.28 (3H, t, J = 7.1 Hz).
946 ¹H-NMR (CDCl₃) δ: 8.14 (1H, d, J = 4.6 Hz), 7.95 (1H, s), 7.40-7.34 (2H, m), 7.11-7.06 (2H, m), 6.90 (1H, dd, J = 4.6, 1.1 Hz), 4.23-4.12 (2H, m), 3.02-2.85 (2H, m), 2.71 (3H, d, J = 1.1 Hz), 1.91 (3H, s), 1.27 (3H, t, J = 7.3 Hz).
947 ¹H-NMR (CDCl₃) δ: 8.15 (1H, s), 7.96 (1H, s), 7.44-7.40 (1H, m), 7.10 (1H, dd, J = 8.0, 1.5 Hz), 7.04 (1H, dd, J = 8.0, 0.8 Hz), 6.91 (1H, td, J = 5.5, 1.1 Hz), 4.23 (2H, qd, J = 7.1, 1.1 Hz), 2.64 (3H, s), 2.37 (3H, s), 1.98 (3H, s), 1.24 (3H, t, J = 7.1 Hz).
948 ¹H-NMR (CDCl₃) δ: 8.23 (1H, d, J = 1.9 Hz), 7.99 (1H, s), 7.79-7.76 (1H, m), 7.45-7.41 (1H, m), 7.12 (1H, dd, J = 8.0, 1.5 Hz), 7.05 (1H, dd, J = 8.0, 0.8 Hz), 6.93 (1H, td, J = 5.3, 1.0 Hz), 4.26-4.21 (2H, m), 2.45 (3H, d, J = 0.8 Hz), 1.99 (3H, s), 1.25 (3H, t, J = 7.3 Hz).
949 ¹H-NMR (CDCl₃) δ: 8.25 (1H, d, J = 4.6 Hz), 8.02 (1H, s), 7.42 (1H, td, J = 5.7, 1.3 Hz), 7.08 (1H, dd, J = 7.8, 1.3 Hz), 7.04 (1H, d, J = 8.0 Hz), 6.94 (1H, dd, J = 4.6, 1.1 Hz), 6.92 (1H, t, J = 7.6 Hz), 4.24 (2H, q, J = 7.1 Hz), 2.72 (3H, s), 1.99 (3H, s), 1.25 (3H, t, J = 7.1 Hz).
950 ¹H-NMR (CDCl₃) δ: 8.36 (1H, s), 8.23 (1H, d, J = 5.0 Hz), 7.45 (1H, ddd, J = 8.6, 7.1, 1.1 Hz), 7.13-7.11 (2H, m), 6.95 (1H, dd, J = 8.2, 1.0 Hz), 6.91 (1H, td, J = 5.5, 1.0 Hz), 4.18-4.06 (2H, m), 3.01 (1H, d, J = 13.7 Hz), 2.94 (1H, d, J = 13.7 Hz), 2.73 (3H, s), 1.87 (3H, s), 1.23 (3H, t, J = 7.1 Hz).
951 ¹H-NMR (CDCl₃) δ: 8.38 (1H, s), 8.29 (1H, s), 7.48-7.44 (1H, m), 7.08 (1H, dd, J = 7.6, 1.5 Hz), 6.96 (1H, dd, J = 8.4, 0.8 Hz), 6.91 (1H, td, J = 5.3, 1.0 Hz), 4.20-4.04 (2H, m), 3.00 (1H, d, J = 13.7 Hz), 2.94 (1H, d, J = 13.7 Hz), 2.75 (3H, s), 1.86 (3H, s), 1.22 (3H, t, J = 7.1 Hz).
952 ¹H-NMR (CDCl₃) δ: 8.30 (1H, s), 8.06 (1H, s), 7.38-7.38 (2H, m), 7.07-7.04 (2H, m), 2.63 (3H, s), 2.35 (3H, s), 1.95-1.93 (2H, m), 1.63 (3H, s), 1.59-1.51 (2H, m), 1.32-1.29 (4H, m), 0.87 (3H, t, J = 7.1 Hz).
953 ¹H-NMR (CDCl₃) δ: 8.32 (1H, s), 8.12 (1H, s), 7.44 (1H, ddd, J = 8.7, 7.1, 1.2 Hz), 7.14 (1H, dd, J = 7.8, 1.5 Hz), 6.95 (1H, dd, J = 8.2, 0.9 Hz), 6.90 (1H, td, J = 5.3, 1.1 Hz), 4.16-4.09 (2H, m), 3.00 (1H, d, J = 13.4 Hz), 2.93 (1H, d, J = 13.7 Hz), 2.64 (3H, s), 2.38 (3H, s), 1.86 (3H, s), 1.23 (3H, t, J = 7.2 Hz).
954 ¹H-NMR (CDCl₃) δ: 8.40 (1H, d, J = 1.0 Hz), 8.24 (1H, s), 7.39-7.32 (2H, m), 6.87-6.84 (2H, m), 2.53 (3H, s), 2.32 (3H, d, J = 1.2 Hz), 1.98-1.92 (2H, m), 1.65 (3H, s), 1.63-1.55 (2H, m), 1.33-1.26 (6H, m), 0.86 (3H, t, J = 6.8 Hz).
955 ¹H-NMR (CDCl₃) δ: 8.38 (1H, s), 8.33 (1H, s), 7.36-7.30 (2H, m), 6.88-6.84 (2H, m), 2.81 (3H, s), 2.40 (3H, s), 1.99-1.94 (2H, m), 1.64 (3H, s), 1.59-1.47 (2H, m), 1.33-1.24 (6H, m), 0.86 (3H, t, J = 6.6 Hz).
956 ¹H-NMR (CDCl₃) δ: 8.39 (1H, s), 8.34 (1H, s), 7.37-7.34 (2H, m), 6.90-6.86 (2H, m), 3.63 (3H, s), 2.82 (3H, s), 2.69-2.54 (2H, m), 2.40 (3H, s), 2.36 (2H, t, J = 7.8 Hz), 1.63 (3H, s).
957 ¹H-NMR (CDCl₃) δ: 8.41 (1H, s), 8.32 (1H, s), 7.38-7.35 (1H, m), 7.32-7.28 (2H, m), 6.95-6.84 (5H, m), 3.27 (1H, d, J = 13.7 Hz), 3.17 (1H, d, J = 14.1 Hz), 2.82 (3H, s), 2.41 (3H, s), 1.63 (3H, s).
958 ¹H-NMR (CDCl₃) δ: 8.39 (1H, s), 8.34 (1H, s), 7.37-7.32 (2H, m), 6.89-6.85 (2H, m), 5.91-5.83 (1H, m), 5.03 (1H, dd, J = 17.0, 1.7 Hz), 4.94-4.92 (1H, m), 2.81 (3H, s), 2.40 (3H, s), 2.39-2.29 (2H, m), 2.11-2.05 (2H, m), 1.65 (3H, s).
959 ¹H-NMR (CDCl₃) δ: 8.39 (1H, s), 8.34 (1H, s), 7.38-7.33 (2H, m), 7.27-7.13 (5H, m), 6.92-6.86 (2H, m), 2.96-2.84 (2H, m), 2.82 (3H, s), 2.40 (3H, s), 2.32-2.27 (2H, m), 1.71 (3H, s).
960 ¹H-NMR (CDCl₃) δ: 8.41 (1H, s), 8.40 (1H, s), 7.42-7.37 (2H, m), 7.02 (1H, d, J = 8.3 Hz), 6.96-6.91 (1H, m), 4.24-4.18 (2H, m), 2.82 (3H, s), 2.17 (3H, s), 1.97 (3H, s), 1.22 (3H, t, J = 7.2 Hz).
961 ¹H-NMR (CDCl₃) δ: 8.38 (1H, s), 8.34 (1H, s), 7.36-7.31 (2H, m), 6.89-6.85 (2H, m), 5.14-5.11 (1H, m), 2.81 (3H, s), 2.40 (3H, s), 2.30-2.21 (2H, m), 2.00-1.97 (2H, m), 1.67 (3H, s), 1.65 (3H, s), 1.59 (3H, s).
962 ¹H-NMR (CDCl₃) δ: 8.20 (1H, d, J = 1.9 Hz), 7.92 (1H, s), 7.75 (1H, s), 6.93 (1H, d, J = 7.6 Hz), 6.74 (1H, s), 6.67 (1H, d, J = 7.6 Hz), 2.44 (3H, s), 2.34 (3H, s), 1.71 (6H, s).
963 ¹H-NMR (CDCl₃) δ: 8.12 (1H, s), 7.92 (1H, s), 7.22-7.17 (1H, m), 6.89-6.79 (2H, m), 4.20-4.10 (2H, m), 3.04-3.00 (2H, m), 2.64 (3H, s), 2.37 (3H, s), 1.92 (3H, s), 1.24 (3H, t, J = 7.4 Hz).
964 ¹H-NMR (CDCl₃) δ: 8.20 (1H, d, J = 2.2 Hz), 7.95 (1H, s), 7.78-7.76 (1H, m), 7.20 (1H, ddd, J = 10.1, 8.1, 1.6 Hz), 6.92-6.88 (1H, m), 6.87-6.80 (1H, m), 4.21-4.09 (2H, m), 3.05-2.99 (2H, m), 2.45 (3H, d, J = 1.0 Hz), 1.92 (3H, s), 1.24 (3H, t, J = 7.1 Hz).
965 ¹H-NMR (CDCl₃) δ: 8.22 (1H, d, J = 4.6 Hz), 7.98 (1H, s), 7.23-7.17 (1H, m), 6.95-6.93 (1H, m), 6.86-6.79 (2H, m), 4.20-4.10 (2H, m), 3.05 (2H, t, J = 8.2 Hz), 2.72 (3H, d, J = 1.2 Hz), 1.92 (3H, s), 1.24 (3H, t, J = 7.2 Hz).
966 ¹H-NMR (CDCl₃) δ: 8.04 (1H, s), 7.88 (1H, s), 7.17 (1H, td, J = 8.0, 1.3 Hz), 7.05 (1H, td, J = 7.2, 5.5 Hz), 7.00-6.94 (1H, m), 4.25-4.10 (2H, m), 3.00-2.83 (2H, m), 2.63 (3H, d, J = 0.5 Hz), 2.34 (3H, s), 1.94 (3H, s), 1.27 (3H, t, J = 7.2 Hz).
967 ¹H-NMR (CDCl₃) δ: 8.14 (1H, d, J = 4.6 Hz), 7.95 (1H, s), 7.18 (1H, td, J = 8.5, 1.2 Hz), 7.06 (1H, td, J = 7.1, 5.5 Hz), 6.95 (1H, dd, J = 7.8, 1.2 Hz), 6.92-6.89 (1H, m), 4.25-4.10 (2H, m), 3.03-2.83 (2H, m), 2.71 (3H, d, J = 1.0 Hz), 1.94 (3H, s), 1.28 (3H, t, J = 7.1 Hz).
968 ¹H-NMR (CDCl₃) δ: 8.13 (1H, s), 7.89 (1H, s), 7.00 (1H, dd, J = 8.1, 1.5 Hz), 6.81 (1H, t, J = 7.9 Hz), 6.68 (1H, dd, J = 7.8, 1.5 Hz), 3.92 (3H, s), 2.63 (3H, d, J = 0.5 Hz), 2.36 (3H, s), 1.77 (6H, s).
969 ¹H-NMR (CDCl₃) δ: 8.21 (1H, d, J = 2.2 Hz), 7.92 (1H, s), 7.77-7.74 (1H, m), 7.01 (1H, dd, J = 8.2, 1.3 Hz), 6.82 (1H, t, J = 8.1 Hz), 6.69 (1H, dd, J = 7.9, 1.3 Hz), 3.92 (3H, s), 2.44 (3H, d, J = 1.0 Hz), 1.77 (6H, s).
970 ¹H-NMR (CDCl₃) δ: 8.23 (1H, d, J = 4.6 Hz), 7.95 (1H, s), 7.01 (1H, dd, J = 8.2, 1.3 Hz), 6.93-6.91 (1H, m), 6.81 (1H, t, J = 7.9 Hz), 6.66 (1H, dd, J = 7.9, 1.3 Hz), 3.92 (3H, s), 2.71 (3H, d, J = 1.2 Hz), 1.78 (6H, s).
971 ¹H-NMR (CDCl₃) δ: 8.12 (1H, s), 7.89 (1H, s), 6.98 (1H, dd, J = 8.2, 1.3 Hz), 6.78 (1H, t, J = 8.0 Hz), 6.65 (1H, dd, J = 8.0, 1.1 Hz), 3.91 (3H, s), 2.63 (3H, s), 2.35 (3H, s), 2.08-1.98 (2H, m), 1.71 (3H, s), 1.66-1.56 (1H, m), 1.56-1.50 (1H, m), 1.38-1.31 (2H, m), 0.90 (3H, t, J = 7.4 Hz).
972 ¹H-NMR (CDCl₃) δ: 8.20 (1H, d, J = 1.9 Hz), 7.92 (1H, s), 7.76-7.75 (1H, m), 6.99 (1H, dd, J = 8.2, 1.3 Hz), 6.79 (1H, t, J = 7.8 Hz), 6.66 (1H, dd, J = 7.8, 1.3 Hz), 3.91 (3H, s), 2.44 (3H, d, J = 0.8 Hz), 2.09-1.97 (2H, m), 1.72 (3H, s), 1.70-1.57 (1H, m), 1.57-1.49 (1H, m), 1.38-1.31 (2H, m), 0.90 (3H, t, J = 7.3 Hz).
973 ¹H-NMR (CDCl₃) δ: 8.22 (1H, d, J = 4.2 Hz), 7.95 (1H, s), 6.99 (1H, d, J = 7.6 Hz), 6.91 (1H, d, J = 4.6 Hz), 6.78 (1H, t, J = 8.0 Hz), 6.63 (1H, dd, J = 8.0, 0.8 Hz), 3.91 (3H, s), 2.71 (3H, s), 2.10-1.98 (2H, m), 1.72 (3H, s), 1.66-1.56 (1H, m), 1.56-1.49 (1H, m), 1.39-1.30 (2H, m), 0.90 (3H, t, J = 7.3 Hz).
974 ¹H-NMR (CDCl₃) δ: 8.39 (1H, s), 8.33 (1H, s), 7.39-7.34 (2H, m), 6.92-6.87 (2H, m), 2.82 (3H, s), 2.47-2.32 (2H, m), 2.40 (3H, s), 2.17-2.12 (2H, m), 2.03-1.93 (2H, m), 1.62 (3H, s).
975 ¹H-NMR (CDCl₃) δ: 8.41 (1H, s), 8.35 (1H, s), 7.30-7.22 (4H, m), 7.05 (1H, d, J = 8.3 Hz), 6.86 (1H, d, J = 7.8 Hz), 6.80 (1H, t, J = 8.3 Hz), 3.78 (1H, d, J = 13.9 Hz), 3.69 (1H, d, J = 13.9 Hz), 2.81 (3H, s), 2.40 (3H, s), 1.81 (3H, s).
976 ¹H-NMR (CDCl₃) δ: 8.45 (1H, s, ), 8.42 (1H, s, ), 7.80-7.78 (2H, m, ), 7.36-7.28 (4H, m, ), 7.22 (1H, d, J = 7.3 Hz, ), 7.00 (1H, d, J = 8.0 Hz, ), 6.83 (1H, t, J = 7.6 Hz, ), 2.83 (3H, s, ), 2.42 (3H, s, ), 2.04 (3H, s, ).
977 ¹H-NMR (CDCl₃) δ: 8.54-8.54 (1H, m), 8.48-8.48 (1H, m), 8.40 (1H, s), 7.30-7.18 (2H, m), 6.88 (1H, td, J = 7.1, 2.3 Hz), 4.27-4.19 (2H, m), 2.42 (3H, d, J = 1.0 Hz), 2.02 (3H, s), 1.23 (3H, t, J = 7.1 Hz).
978 ¹H-NMR (CDCl₃) δ: 8.39 (1H, s), 8.34 (1H, s), 7.38-7.33 (2H, m), 6.91-6.86 (2H, m), 6.71-6.65 (3H, m), 5.89 (2H, s), 2.88-2.76 (2H, m), 2.82 (3H, s), 2.40 (3H, s), 2.29-2.21 (2H, m), 1.69 (3H, s).
979 ¹H-NMR (CDCl₃) δ: 8.40 (1H, s), 8.32 (1H, s), 7.49 (1H, dd, J = 8.0, 1.5 Hz), 6.95 (1H, dd, J = 8.0, 1.5 Hz), 6.83 (1H, t, J = 8.0 Hz), 2.77 (3H, s), 2.02-2.00 (2H, m), 1.69 (3H, s), 1.60-1.42 (2H, m), 1.39-1.33 (2H, m), 0.91 (3H, t, J = 7.4 Hz).
980 ¹H-NMR (CDCl₃) δ: 8.40 (1H, s), 8.31 (1H, s), 6.87 (1H, dd, J = 8.8, 6.1 Hz), 6.56 (1H, t, J = 8.8 Hz), 2.76 (3H, s), 2.14 (3H, s), 2.03-2.00 (1H, m), 1.92 (2H, d, J = 6.1 Hz), 1.66 (3H, s), 1.01 (3H, d, J = 6.5 Hz), 0.98 (3H, d, J = 6.5 Hz).
981 ¹H-NMR (CDCl₃) δ: 8.35 (1H, s), 8.29 (1H, s), 7.08-7.06 (1H, m), 6.68-6.66 (1H, m), 6.61-6.59 (1H, m), 2.75 (3H, s), 1.71 (6H, s).
982 ¹H-NMR (CDCl₃) δ: 8.40 (1H, s), 8.33 (1H, s), 7.07-7.06 (1H, m), 6.68-6.66 (1H, m), 6.61-6.59 (1H, m), 2.77 (3H, s), 1.71 (6H, s).
983 ¹H-NMR (CDCl₃) δ: 8.43 (1H, s), 8.29 (1H, s), 7.51-7.49 (1H, m), 7.12-7.10 (1H, m), 7.08-7.06 (1H, m), 6.96-6.94 (1H, m), 4.23 (2H, q, J = 7.1 Hz), 2.75 (3H, s), 1.97 (3H, s), 1.24 (3H, t, J = 7.1 Hz).
984 ¹H-NMR (CDCl₃) δ: 8.42 (1H, s), 8.22 (1H, d, J = 5.1 Hz), 7.49-7.47 (1H, m), 7.15 (1H, dd, J = 8.0, 0.9 Hz), 7.12 (1H, dd, J = 5.1, 0.9 Hz), 7.06 (1H, dd, J = 8.0, 0.9 Hz), 6.97-6.93 (1H, m), 4.24 (2H, q, J = 7.1 Hz), 2.73 (3H, s), 1.97 (3H, s), 1.24 (3H, t, J = 7.1 Hz).
985 ¹H-NMR (CDCl₃) δ: 8.29 (1H, s), 8.12 (1H, s), 7.47 (1H, dd, J = 8.1, 1.5 Hz), 7.01-6.99 (1H, m), 6.81 (1H, t, J = 8.1 Hz), 2.64 (3H, s), 2.38 (3H, s), 2.02-1.99 (2H, m), 1.68 (3H, s), 1.56-1.47 (2H, m), 1.38-1.33 (2H, m), 0.91 (3H, t, J = 7.2 Hz).
986 ¹H-NMR (CDCl₃) δ: 8.28 (1H, s), 8.12 (1H, s), 6.94-6.91 (1H, m), 6.55 (1H, t, J = 8.9 Hz), 2.64 (3H, s), 2.37 (3H, s), 2.14 (3H, d, J = 2.0 Hz), 2.06-1.99 (1H, m), 1.92 (2H, d, J = 6.3 Hz), 1.66 (3H, s), 1.01 (3H, d, J = 6.3 Hz), 0.99 (3H, d, J = 6.3 Hz).
987 ¹H-NMR (CDCl₃) δ: 8.30 (1H, s), 8.12 (1H, s), 7.12 (1H, dd, J = 9.0, 6.1 Hz), 6.66 (1H, dd, J = 9.0, 2.4 Hz), 6.59-6.58 (1H, m), 2.64 (3H, s), 2.38 (3H, s), 1.71 (6H, s).
988 ¹H-NMR (CDCl₃) δ: 8.40 (1H, s), 8.15 (1H, d, J = 4.9 Hz), 7.45-7.40 (2H, m), 7.13-7.08 (3H, m), 4.23-4.08 (2H, m), 3.00 (1H, d, J = 13.4 Hz), 2.83 (1H, d, J = 13.7 Hz), 2.72 (3H, d, J = 0.5 Hz), 1.90 (3H, s), 1.28 (3H, t, J = 7.2 Hz).
989 ¹H-NMR (CDCl₃) δ: 8.42 (1H, s), 8.20 (1H, s), 7.47-7.40 (2H, m), 7.14-7.08 (2H, m), 4.23-4.08 (2H, m), 2.99 (1H, d, J = 13.4 Hz), 2.82 (1H, d, J = 13.4 Hz), 2.74 (3H, s), 1.90 (3H, s), 1.27 (3H, t, J = 7.1 Hz).
990 ¹H-NMR (CDCl₃) δ: 8.40 (1H, s), 8.32 (1H, s), 7.47-7.40 (2H, m), 7.14-7.07 (2H, m), 4.23-4.08 (2H, m), 2.99 (1H, d, J = 13.4 Hz), 2.82 (1H, d, J = 13.7 Hz), 2.76 (3H, s), 1.90 (3H, s), 1.27 (3H, t, J = 7.1 Hz).
991 ¹H-NMR (CDCl₃) δ: 8.42 (1H, s), 8.21 (1H, d, J = 4.9 Hz), 7.31-7.28 (1H, m), 7.13 (1H, dd, J = 4.9, 0.7 Hz), 6.98 (1H, dt, J = 7.9, 1.3 Hz), 6.89 (1H, td, J = 6.8, 4.4 Hz), 4.27-4.23 (2H, m), 2.73 (3H, d, J = 0.5 Hz), 2.02 (3H, s), 1.25 (3H, t, J = 7.2 Hz).
992 ¹H-NMR (CDCl₃) δ: 8.43 (1H, s), 8.28 (1H, s), 7.32-7.27 (1H, m), 6.91 (2H, tdd, J = 8.7, 4.8, 3.5 Hz), 4.30-4.21 (2H, m), 2.75 (3H, s), 2.02 (3H, s), 1.25 (3H, t, J = 7.1 Hz).
993 ¹H-NMR (CDCl₃) δ: 8.41 (1H, s), 8.39 (1H, s), 7.32-7.27 (1H, m), 6.92-6.90 (2H, m), 4.27-4.23 (2H, m), 2.77 (3H, s), 2.02 (3H, s), 1.25 (3H, t, J = 7.2 Hz).
994 ¹H-NMR (CDCl₃) δ: 8.36 (1H, s), 8.23 (1H, d, J = 4.9 Hz), 7.28-7.23 (1H, m), 7.12 (1H, dd, J = 5.0, 0.9 Hz), 6.95 (1H, dt, J = 7.9, 1.3 Hz), 6.85 (1H, td, J = 6.9, 4.6 Hz), 4.19-4.05 (2H, m), 3.04 (1H, d, J = 13.9 Hz), 2.98 (1H, d, J = 13.7 Hz), 2.73 (3H, d, J = 0.5 Hz), 1.91 (3H, s), 1.23 (3H, t, J = 7.0 Hz).
995 ¹H-NMR (CDCl₃) δ: 8.37 (1H, s), 8.29 (1H, s), 7.29-7.24 (1H, m), 6.91 (1H, ddd, J = 7.9, 0.8, 0.4 Hz), 6.86 (1H, td, J = 6.8, 4.3 Hz), 4.19-4.04 (2H, m), 3.04 (1H, d, J = 13.7 Hz), 2.97 (1H, d, J = 13.4 Hz), 2.75 (3H, s), 1.91 (3H, s), 1.22 (3H, t, J = 7.3 Hz).
996 ¹H-NMR (CDCl₃) δ: 8.40 (1H, s), 8.35 (1H, s), 7.29-7.24 (1H, m), 6.91 (1H, ddd, J = 7.9, 0.9, 0.4 Hz), 6.86 (1H, td, J = 6.8, 4.3 Hz), 4.19-4.04 (2H, m), 3.04 (1H, d, J = 13.9 Hz), 2.97 (1H, d, J = 13.9 Hz), 2.77 (3H, s), 1.91 (3H, s), 1.22 (3H, t, J = 7.2 Hz).
997 ¹H-NMR (CDCl₃) δ: 8.47 (1H, d, J = 4.2 Hz), 8.39 (1H, s), 6.99 (1H, dd, J = 7.8, 1.7 Hz), 6.93 (1H, dd, J = 7.9, 1.3 Hz), 6.84-6.77 (2H, m), 3.90 (3H, s), 2.84 (3H, d, J = 0.7 Hz), 2.04-1.99 (2H, m), 1.69 (3H, s), 1.66-1.50 (2H, m), 1.38-1.31 (2H, m), 0.89 (3H, t, J = 7.3 Hz).
998 ¹H-NMR (CDCl₃) δ: 8.39 (1H, s), 8.34 (1H, s), 7.39-7.32 (2H, m), 7.13-7.11 (2H, m), 6.91-6.86 (2H, m), 6.81-6.78 (2H, m), 3.77 (3H, s), 2.88-2.82 (2H, m), 2.82 (3H, s), 2.40 (3H, s), 2.29-2.23 (2H, m), 1.70 (3H, s).
999 ¹H-NMR (CDCl₃) δ: 8.41 (1H, d, J = 1.2 Hz), 8.25 (1H, s), 7.42-7.33 (2H, m), 7.16-7.10 (2H, m), 6.90-6.86 (2H, m), 6.81-6.78 (2H, m), 3.77 (3H, s), 2.92-2.85 (2H, m), 2.54 (3H, s), 2.32 (3H, d, J = 1.0 Hz), 2.28-2.23 (2H, m), 1.71 (3H, s).
1000 ¹H-NMR (CDCl₃) δ: 8.42 (1H, s), 8.39 (1H, s), 7.46-7.45 (1H, m), 7.37-7.34 (2H, m), 7.21 (1H, t, J = 7.9 Hz), 7.00 (1H, dd, J = 8.1, 0.7 Hz), 6.86-6.82 (1H, m), 7.30-7.24 (1H, m), 6.77-6.74 (1H, m), 3.82 (3H, s), 2.83 (3H, s), 2.42 (3H, s), 2.04 (3H, s).
1001 ¹H-NMR (CDCl₃) δ: 8.43 (1H, s), 8.27 (1H, s), 7.51-7.48 (1H, m), 7.44-7.43 (1H, m), 7.35-7.19 (3H, m), 7.01 (1H, d, J = 6.8 Hz), 6.84 (1H, t, J = 7.6 Hz), 6.75 (1H, dd, J = 8.1, 2.0 Hz), 3.80 (3H, s), 2.61 (3H, s), 2.35 (3H, d, J = 1.0 Hz), 2.05 (3H, s).
1002 1H-NMR (CDCl₃) δ: 8.39 (1H, s), 8.35 (1H, s), 7.38-7.34 (1H, m), 7.31-7.26 (2H, m), 7.20-7.17 (1H, m), 6.90-6.81 (4H, m), 3.78 (3H, s), 3.45 (1H, d, J = 12.0 Hz),3.17 (1H, d, J = 12.0 Hz), 2.82 (3H, s), 2.41 (3H, s), 1.67 (3H, s).
1003 ¹H-NMR (CDCl₃) δ: 8.40 (1H, s), 8.34 (1H, s), 7.38-7.31 (2H, m), 7.22 (2H, d, J = 8.8 Hz), 6.93-6.77 (4H, m), 3.78 (3H, s), 3.25 (1H, d, J = 13.7 Hz), 3.12 (1H, d, J = 14.1 Hz), 2.82 (3H, s), 2.41 (3H, s), 1.62 (3H, s).
1004 ¹H-NMR (CDCl₃) δ: 8.42 (1H, s), 8.25 (1H, s), 7.38-7.35 (2H, m), 7.29-7.26 (2H, m), 6.90-6.81 (4H, m), 3.78 (3H, s), 3.22 (1H, d, J = 14.1 Hz), 3.15 (1H, d, J = 13.7 Hz), 2.57 (3H, s), 2.33 (3H, s), 1.62 (3H, s).
1005 ¹H-NMR (CDCl₃) δ: 8.41 (1H, d, J = 1.1 Hz), 8.25 (1H, s), 7.40-7.38 (1H, m), 7.36-7.33 (1H, m), 6.88-6.85 (2H, m), 4.71-4.68 (2H, m), 2.54 (3H, s), 2.41-2.26 (2H, m), 2.32 (3H, d, J = 1.1 Hz), 2.14-2.10 (2H, m), 1.74 (3H, s), 1.67 (3H, s).
1006 ¹H-NMR (CDCl₃) δ: 8.39 (1H, s), 8.34 (1H, s), 7.37-7.32 (2H, m), 6.89-6.85 (2H, m), 4.69-4.68 (2H, m), 2.81 (3H, s), 2.40 (3H, s), 2.35-2.22 (2H, m), 2.18-2.08 (2H, m), 1.73 (3H, s), 1.66 (3H, s).
1007 ¹H-NMR (CDCl₃) δ: 8.41 (1H, s), 8.40 (1H, s), 7.51-7.49 (1H, m), 7.11-7.07 (2H, m), 6.95 (1H, td, J = 8.0, 1.1 Hz), 4.23 (2H, q, J = 7.1 Hz), 2.77 (3H, s), 1.97 (3H, s), 1.24 (3H, t, J = 7.1 Hz).
1008 ¹H-NMR (CDCl₃) δ: 8.46 (1H, s), 8.32 (1H, s), 7.23 (1H, td, J = 7.9, 1.1 Hz), 7.10-7.08 (1H, m), 6.95 (1H, dd, J = 7.9, 1.1 Hz), 4.22-4.15 (2H, m), 2.76 (3H, s), 2.02 (3H, s), 1.18 (3H, t, J = 7.1 Hz).
1009 ¹H-NMR (CDCl₃) δ: 8.48 (1H, s), 8.20 (1H, s), 7.22-7.21 (1H, m), 7.10-7.09 (1H, m), 6.95 (1H, d, J = 7.1 Hz), 4.19-4.17 (2H, m), 2.75 (3H, s), 2.02 (3H, s), 1.18 (3H, t, J = 7.2 Hz).
1010 ¹H-NMR (CDCl₃) δ: 8.47 (1H, s), 8.15 (1H, d, J = 4.9 Hz), 7.22-7.21 (1H, m), 7.09-7.08 (2H, m), 7.00-6.99 (1H, m), 4.19-4.17 (2H, m), 2.72 (3H, s), 2.03 (3H, s), 1.18 (3H, t, J = 7.2 Hz).
1011 ¹H-NMR (CDCl₃) δ: 8.40 (1H, s), 8.31 (1H, s), 7.28-7.23 (1H, m), 7.10 (1H, td, J = 7.8, 5.3 Hz), 6.96 (1H, dd, J = 7.8, 1.0 Hz), 4.24-4.11 (2H, m), 2.97 (1H, d, J = 13.4 Hz), 2.82 (1H, d, J = 13.4 Hz), 2.75 (3H, s), 1.93 (3H, s), 1.31-1.24 (3H, m).
1012 ¹H-NMR (CDCl₃) δ: 8.42 (1H, s), 8.19 (1H, s), 7.29-7.23 (1H, m), 7.11-7.09 (1H, m), 6.96 (1H, d, J = 7.8 Hz), 4.18-4.13 (2H, m), 2.97 (1H, d, J = 13.4 Hz), 2.82 (1H, d, J = 13.4 Hz), 2.74 (3H, s), 1.93 (3H, s), 1.28 (3H, t, J = 7.1 Hz).
1013 ¹H-NMR (CDCl₃) δ: 8.41 (1H, s), 8.40 (1H, s), 7.52-7.47 (1H, m), 7.11-7.07 (2H, m), 6.95 (1H, td, J = 8.0, 1.1 Hz), 4.13 (2H, t, J = 6.5 Hz), 2.78 (3H, s), 1.98 (3H, s), 1.67-1.60 (2H, m), 0.86 (3H, t, J = 7.4 Hz).
1014 ¹H-NMR (CDCl₃) δ: 8.41 (1H, s), 8.31 (1H, s), 7.05 (1H, dd, J = 7.9, 1.2 Hz), 6.82 (1H, t, J = 7.9 Hz), 6.61 (1H, dd, J = 7.9, 1.2 Hz), 3.91 (3H, s), 2.77 (3H, s), 2.07-1.95 (2H, m), 1.69 (3H, s), 1.63-1.45 (2H, m), 1.39-1.30 (2H, m), 0.89 (3H, t, J = 7.3 Hz).
1015 ¹H-NMR (CDCl₃) δ: 8.33 (1H, s), 8.30 (1H, s), 7.05 (1H, dd, J = 8.1, 1.3 Hz), 6.82 (1H, t, J = 8.1 Hz), 6.61 (1H, dd, J = 8.1, 1.3 Hz), 3.91 (3H, s), 2.74 (3H, s), 2.07-1.98 (2H, m), 1.69 (3H, s), 1.63-1.45 (2H, m), 1.39-1.31 (2H, m), 0.89 (3H, t, J = 7.5 Hz).
1016 ¹H-NMR (CDCl₃) δ: 8.32 (1H, s), 8.24 (1H, d, J = 5.1 Hz), 7.10 (1H, dd, J = 5.1, 0.9 Hz), 7.04 (1H, dd, J = 8.2, 1.3 Hz), 6.82 (1H, t, J = 8.2 Hz), 6.66 (1H, dd, J = 8.2, 1.3 Hz), 3.91 (3H, s), 2.73 (3H, s), 2.08-1.98 (2H, m), 1.70 (3H, s), 1.63-1.45 (2H, m), 1.38-1.33 (2H, m), 0.90 (3H, t, J = 7.3 Hz).
1017 ¹H-NMR (CDCl₃) δ: 8.38 (1H, s), 8.11 (1H, s), 7.50-7.45 (1H, m), 7.16 (1H, dd, J = 7.9, 1.0 Hz), 7.05 (1H, dd, J = 7.9, 1.0 Hz), 6.93 (1H, td, J = 7.9, 1.0 Hz), 4.23 (2H, q, J = 7.1 Hz), 2.64 (3H, s), 2.38 (3H, s), 1.96 (3H, s), 1.24 (3H, t, J = 7.1 Hz).
1018 ¹H-NMR (CDCl₃) δ: 8.42 (1H, s), 8.03 (1H, s), 7.21 (1H, td, J = 7.9, 1.3 Hz), 7.08 (1H, td, J = 7.9, 5.3 Hz), 6.99 (1H, dd, J = 7.9, 1.3 Hz), 4.20-4.13 (2H, m), 2.64 (3H, s), 2.36 (3H, s), 2.02 (3H, s), 1.17 (3H, t, J = 7.1 Hz).
1019 ¹H-NMR (CDCl₃) δ: 8.36 (1H, s), 8.03 (1H, s), 7.24-7.21 (1H, m), 7.08 (1H, td, J = 7.9, 5.2 Hz), 7.01-6.99 (1H, m), 4.22-4.16 (1H, m), 4.16-4.09 (1H, m), 2.97 (1H, d, J = 13.4 Hz), 2.82 (1H, d, J = 13.4 Hz), 2.63 (3H, s), 2.35 (3H, s), 1.92 (3H, s), 1.28 (3H, t, J = 7.2 Hz).
1020 ¹H-NMR (CDCl₃) δ: 8.36 (1H, s), 8.04 (1H, s), 7.45-7.38 (2H, m), 7.15-7.07 (2H, m), 4.22-4.08 (2H, m), 2.99 (1H, d, J = 13.2 Hz), 2.82 (1H, d, J = 13.7 Hz), 2.63 (3H, s), 2.35 (3H, s), 1.90 (3H, s), 1.27 (3H, t, J = 6.8 Hz).
1021 ¹H-NMR (CDCl₃) δ: 8.38 (1H, s), 8.11 (1H, s), 7.30-7.25 (1H, m), 6.99 (1H, dt, J = 7.8, 1.2 Hz), 6.88 (1H, td, J = 6.8, 4.4 Hz), 4.26-4.22 (2H, m), 2.64 (3H, s), 2.38 (3H, s), 2.02 (3H, s), 1.25 (3H, t, J = 7.1 Hz).
1022 ¹H-NMR (CDCl₃) δ: 8.32 (1H, s), 8.12 (1H, s), 7.25 (1H, ddd, J = 10.3, 8.3, 1.5 Hz), 6.97 (1H, dt, J = 8.0, 1.3 Hz), 6.85 (1H, td, J = 7.0, 4.6 Hz), 4.19-4.05 (2H, m), 3.03 (1H, d, J = 3.7 Hz), 2.97 (1H, d, J = 13.4 Hz), 2.64 (3H, s), 2.38 (3H, s), 1.91 (3H, s), 1.22 (3H, t, J = 7.2 Hz).
1023 ¹H-NMR (CDCl₃) δ: 8.35 (1H, s), 8.15 (1H, d, J = 4.9 Hz), 7.50 (1H, dt, J = 9.2, 4.1 Hz), 7.08-7.07 (1H, m), 7.03-7.03 (2H, m), 2.72 (3H, d, J = 0.5 Hz), 1.74 (6H, s).
1024 ¹H-NMR (CDCl₃) δ: 8.37 (1H, s), 8.21 (1H, s), 7.51 (1H, dd, J = 7.4, 1.6 Hz), 7.04 (1H, t, J = 7.7 Hz), 7.00 (1H, dd, J = 7.8, 1.7 Hz), 2.74 (3H, s), 1.74 (6H, s).
1025 ¹H-NMR (CDCl₃) δ: 8.34 (1H, s), 8.32 (1H, s), 7.51 (1H, dd, J = 7.6, 1.7 Hz), 7.04 (1H, t, J = 7.8 Hz), 6.99 (1H, dd, J = 7.9, 1.6 Hz), 2.76 (3H, s), 1.73 (6H, s).
1026 ¹H-NMR (CDCl₃) δ: 8.32 (1H, s), 8.04 (1H, s), 7.49 (1H, dd, J = 7.3, 2.3 Hz), 7.03-7.02 (2H, m), 2.63 (3H, s), 2.35 (3H, s), 1.73 (6H, s).
1027 ¹H-NMR (CDCl₃) δ: 8.12 (1H, d, J = 2.3 Hz), 7.90 (1H, s), 7.74 (1H, s), 7.44 (1H, dd, J = 7.6, 1.5 Hz), 7.05-6.99 (2H, m), 2.42 (3H, s), 1.73 (6H, s).
1028 ¹H-NMR (CDCl₃) δ: 8.15 (1H, s), 7.97 (1H, s), 7.24-7.20 (1H, m), 6.92-6.89 (1H, m), 6.87-6.82 (1H, m), 4.27-4.21 (2H, m), 2.64 (3H, s), 2.37 (3H, s), 2.04 (3H, s), 1.25 (3H, t, J = 7.1 Hz).
1029 ¹H-NMR (CDCl₃) δ: 8.07 (1H, s), 7.97 (1H, s), 7.17-7.12 (1H, m), 7.07-7.03 (1H, m), 6.98-6.95 (1H, m), 4.20-4.12 (2H, m), 2.63 (3H, s), 2.35 (3H, s), 2.02 (3H, s), 1.60 (3H, s).
1030 ¹H-NMR (CDCl₃) δ: 8.36 (1H, s), 8.29 (1H, s), 7.26-7.23 (1H, m), 6.85-6.83 (2H, m), 3.70-3.64 (2H, m), 3.48-3.41 (2H, m), 2.75 (3H, s), 2.36-2.32 (2H, m), 1.74 (3H, s), 1.16 (3H, t, J = 7.1 Hz).
1031 ¹H-NMR (CDCl₃) δ: 8.38 (1H, s), 8.11 (1H, s), 7.50-7.45 (1H, m), 7.16 (1H, dd, J = 7.6, 1.1 Hz), 7.06-7.04 (1H, m), 6.93 (1H, td, J = 7.6, 1.1 Hz), 4.13 (2H, t, J = 6.7 Hz), 2.64 (3H, s), 2.38 (3H, s), 1.98 (3H, s), 1.64-1.60 (2H, m), 0.86 (3H, t, J = 6.7 Hz).
1032 ¹H-NMR (CDCl₃) δ: 8.28 (1H, s), 8.14 (1H, s), 7.05-6.99 (1H, m), 6.83-6.80 (1H, m), 6.68-6.66 (1H, m), 3.91 (3H, s), 2.64 (3H, s), 2.38 (3H, s), 2.07-1.97 (2H, m), 1.69 (3H, s), 1.52-1.46 (2H, m), 1.38-1.29 (2H, m), 0.91-0.88 (3H, m).
1033 ¹H-NMR (CDCl₃) δ: 8.43 (1H, s), 8.41 (1H, s), 7.69-7.67 (2H, m), 7.35-7.26 (2H, m), 7.12-7.11 (2H, m), 6.99 (1H, dd, J = 8.2, 1.0 Hz), 6.83 (1H, td, J = 5.5, 1.1 Hz), 2.82 (3H, s), 2.56-2.51 (2H, m), 2.42 (3H, s), 2.02 (3H, s), 1.59-1.52 (2H, m), 1.35-1.29 (2H, m), 0.89 (3H, t, J = 7.3 Hz).
1034 ¹H-NMR (CDCl₃) δ: 8.42 (1H, d, J = 1.1 Hz), 8.28 (1H, s), 7.78-7.76 (2H, m), 7.35-7.28 (2H, m), 7.12-7.10 (2H, m), 7.02-6.98 (1H, m), 6.84-6.81 (1H, m), 2.61 (3H, s), 2.55-2.42 (2H, m), 2.35 (3H, d, J = 0.7 Hz), 2.03 (3H, s), 1.58-1.52 (2H, m), 1.36-1.30 (2H, m), 0.89 (3H, t, J = 7.4 Hz).
1035 ¹H-NMR (CDCl₃) δ: 8.40 (1H, s), 8.34 (1H, s), 7.39-7.34 (1H, m), 7.33 (1H, dd, J = 7.6, 1.5 Hz), 7.20-7.19 (2H, m), 7.09-7.08 (2H, m), 6.91-6.85 (2H, m), 3.27 (1H, d, J = 13.7 Hz), 3.14 (1H, d, J = 13.7 Hz), 2.82 (3H, s), 2.41 (3H, s), 2.32 (3H, s), 1.62 (3H, s).
1036 ¹H-NMR (CDCl₃) δ: 8.42 (1H, s), 8.25 (1H, s), 7.39-7.36 (2H, m), 7.25-7.23 (2H, m), 7.09-7.08 (2H, m), 6.91-6.85 (2H, m), 3.24 (1H, d, J = 13.4 Hz), 3.16 (1H, d, J = 13.7 Hz), 2.56 (3H, s), 2.33 (3H, d, J = 1.1 Hz), 2.32 (3H, s), 1.62 (3H, s).
1037 ¹H-NMR (CDCl₃) δ: 8.44 (1H, s), 8.41 (1H, s), 7.39-7.35 (2H, m), 7.06 (1H, d, J = 4.2 Hz), 6.95 (1H, d, J = 7.6 Hz), 6.90 (1H, td, J = 5.5, 1.1 Hz), 6.70 (1H, d, J = 3.8 Hz), 2.83 (3H, s), 2.42 (3H, s), 2.10 (3H, s).
1038 ¹H-NMR (CDCl₃) δ: 8.43 (1H, s), 8.31 (1H, s), 7.39-7.35 (2H, m), 7.15 (1H, d, J = 3.8 Hz), 6.97-6.89 (2H, m), 6.70 (1H, d, J = 3.8 Hz), 2.58 (3H, s), 2.34 (3H, d, J = 0.8 Hz), 2.10 (3H, s).
1039 ¹H-NMR (CDCl₃) δ: 8.42 (1H, s), 8.25 (1H, s), 7.26-7.15 (7H, m), 6.81 (1H, td, J = 8.1, 4.6 Hz), 3.05-2.86 (2H, m), 2.54 (3H, s), 2.40-2.28 (2H, m), 2.33 (3H, s), 1.77 (3H, s).
1040 ¹H-NMR (CDCl₃) δ: 8.39 (1H, s), 8.34 (1H, s), 7.26-7.13 (7H, m), 6.84-6.78 (1H, m), 3.01-2.84 (2H, m), 2.82 (3H, s), 2.41 (3H, s), 2.36-2.30 (2H, m), 1.75 (3H, s).
1041 ¹H-NMR (CDCl₃) δ: 8.45 (1H, s), 8.39 (1H, s), 7.81-7.76 (2H, m), 7.35 (1H, ddd, J = 8.5, 7.1, 1.2 Hz), 7.30 (1H, dd, J = 7.7, 1.6 Hz), 7.00-6.96 (3H, m), 6.85 (1H, td, J = 5.5, 1.1 Hz), 2.83 (3H, s), 2.42 (3H, s), 2.01 (3H, s).
1042 ¹H-NMR (CDCl₃) δ: 8.43 (1H, d, J = 0.7 Hz), 8.27 (1H, s), 7.90-7.85 (2H, m), 7.35-7.31 (2H, m), 6.99-6.97 (3H, m), 6.85 (1H, td, J = 5.5, 1.2 Hz), 2.61 (3H, s), 2.35 (3H, d, J = 1.0 Hz), 2.02 (3H, s).
1043 ¹H-NMR (CDCl₃) δ: 8.43 (1H, s), 8.41 (1H, s), 7.66 (2H, dt, J = 8.4, 2.0 Hz), 7.33 (1H, ddd, J = 8.4, 6.9, 1.1 Hz), 7.27-7.25 (1H, m), 7.11 (2H, d, J = 7.6 Hz), 6.99 (1H, dd, J = 8.2, 1.0 Hz), 6.82 (1H, td, J = 5.5, 1.1 Hz), 2.82 (3H, s), 2.41 (3H, s), 2.28 (3H, s), 2.03 (3H, s).
1044 ¹H-NMR (CDCl₃) δ: 8.42 (1H, d, J = 1.1 Hz), 8.28 (1H, s), 7.76 (2H, dt, J = 8.4, 1.9 Hz), 7.32 (1H, ddd, J = 8.5, 7.0, 1.2 Hz), 7.28 (1H, dd, J = 7.6, 1.5 Hz), 7.11 (2H, d, J = 8.0 Hz), 6.99 (1H, dd, J = 7.8, 1.0 Hz), 6.82 (1H, td, J = 5.3, 1.1 Hz), 2.61 (3H, s), 2.34 (3H, d, J = 0.8 Hz), 2.28 (3H, s), 2.04 (3H, s).
1045 ¹H-NMR (CDCl₃) δ: 8.44 (1H, s), 8.40 (1H, s), 7.70 (2H, dt, J = 9.7, 2.7 Hz), 7.33 (1H, ddd, J = 8.5, 7.0, 1.2 Hz), 7.28-7.27 (1H, m), 6.97 (1H, dd, J = 8.2, 1.0 Hz), 6.85-6.81 (3H, m), 3.76 (3H, s), 2.83 (3H, s), 2.42 (3H, s), 2.03 (3H, s).
1046 ¹H-NMR (CDCl₃) δ: 8.43 (1H, d, J = 1.1 Hz), 8.28 (1H, s), 7.80 (2H, dt, J = 9.5, 2.6 Hz), 7.33 (1H, ddd, J = 8.6, 7.1, 1.1 Hz), 7.29 (1H, dd, J = 7.6, 1.5 Hz), 6.98 (1H, dd, J = 8.2, 1.0 Hz), 6.83-6.82 (3H, m), 3.76 (3H, s), 2.61 (3H, s), 2.34 (3H, d, J = 1.1 Hz), 2.03 (3H, s).
1047 ¹H-NMR (CDCl₃) δ: 8.37 (1H, s), 8.29 (1H, s), 7.32 (1H, dq, J = 7.4, 0.9 Hz), 6.88 (1H, dd, J = 7.8, 1.3 Hz), 6.81 (1H, t, J = 7.4 Hz), 4.16-4.06 (2H, m), 2.99 (1H, d, J = 13.7 Hz), 2.95 (1H, d, J = 13.4 Hz), 2.75 (3H, s), 2.24 (3H, s), 1.87 (3H, s), 1.23 (3H, t, J = 7.3 Hz).
1048 ¹H-NMR (CDCl₃) δ: 8.40 (1H, s), 8.34 (1H, s), 7.32 (1H, dq, J = 7.4, 0.8 Hz), 6.88-6.86 (1H, m), 6.81 (1H, t, J = 7.6 Hz), 4.16-4.06 (2H, m), 2.98 (1H, d, J = 13.4 Hz), 2.94 (1H, d, J = 13.4 Hz), 2.77 (3H, s), 2.24 (3H, s), 1.87 (3H, s), 1.23 (3H, t, J = 7.3 Hz).
1049 ¹H-NMR (CDCl₃) δ: 8.31 (1H, s), 8.12 (1H, s), 7.30 (1H, dq, J = 7.4, 0.8 Hz), 6.93-6.92 (1H, m), 6.80 (1H, t, J = 7.6 Hz), 4.15-4.07 (2H, m), 2.98 (1H, d, J = 13.7 Hz), 2.95 (1H, d, J = 13.7 Hz), 2.64 (3H, s), 2.37 (3H, s), 2.23 (3H, s), 1.87 (3H, s), 1.23 (3H, t, J = 7.1 Hz).
1050 ¹H-NMR (CDCl₃) δ: 8.13 (1H, s), 7.96 (1H, s), 7.27-7.25 (1H, m), 6.92-6.89 (1H, m), 6.80 (1H, t, J = 7.6 Hz), 4.10 (2H, td, J = 4.8, 1.3 Hz), 2.63 (3H, d, J = 0.5 Hz), 2.36 (3H, s), 2.31 (3H, s), 2.02 (3H, s), 1.65-1.56 (2H, m), 0.85 (3H, t, J = 7.4 Hz).
1051 ¹H-NMR (CDCl₃) δ: 8.24 (1H, d, J = 4.6 Hz), 8.02 (1H, s), 7.28-7.26 (1H, m), 6.93-6.92 (1H, m), 6.89 (1H, dd, J = 8.2, 1.3 Hz), 6.80 (1H, t, J = 7.6 Hz), 4.12-4.09 (2H, m), 2.71 (3H, d, J = 1.1 Hz), 2.32 (3H, s), 2.03 (3H, s), 1.66-1.58 (2H, m), 0.88-0.84 (3H, m).
1052 ¹H-NMR (CDCl₃) δ: 8.11 (1H, s), 7.90 (1H, s), 7.26-7.23 (1H, m), 6.90-6.88 (1H, m), 6.78 (1H, t, J = 7.6 Hz), 4.16 (2H, q, J = 7.1 Hz), 3.04-2.94 (2H, m), 2.63 (3H, s), 2.36 (3H, s), 2.23 (3H, s), 1.88 (3H, s), 1.26 (3H, t, J = 7.3 Hz).
1053 ¹H-NMR (CDCl₃) δ: 8.38 (1H, s), 8.34 (1H, s), 7.41 (1H, dd, J = 8.0, 1.5 Hz), 7.30-7.23 (1H, m), 6.82 (1H, t, J = 7.8 Hz), 5.91-5.84 (1H, m), 5.07-4.93 (2H, m), 2.82 (3H, s), 2.40 (3H, s), 2.37-2.32 (2H, m), 2.14-2.09 (2H, m), 1.70 (3H, s).
1054 ¹H-NMR (CDCl₃) δ: 8.38 (1H, s), 8.33 (1H, s), 7.43 (1H, dd, J = 8.1, 1.5 Hz), 7.31-7.14 (6H, m), 6.83 (1H, t, J = 7.8 Hz), 2.96-2.89 (2H, m), 2.82 (3H, s), 2.40 (3H, s), 2.34-2.28 (2H, m), 1.77 (3H, s).
1055 ¹H-NMR (CDCl₃) δ: 8.41 (1H, s), 8.25 (1H, s), 7.44-7.41 (1H, m), 7.33-7.14 (6H, m), 6.82 (1H, t, J = 7.8 Hz), 3.00-2.89 (2H, m), 2.53 (3H, s), 2.34-2.27 (2H, m), 2.32 (3H, d, J = 1.0 Hz), 1.78 (3H, s).
1056 ¹H-NMR (CDCl₃) δ: 8.40 (1H, s), 8.34 (1H, s), 7.28-7.23 (1H, m), 6.85-6.82 (2H, m), 3.71-3.64 (2H, m), 3.47-3.41 (2H, m), 2.77 (3H, s), 2.37-2.32 (2H, m), 1.74 (3H, s), 1.15 (3H, t, J = 7.1 Hz).
1057 ¹H-NMR (CDCl₃) δ: 8.39 (1H, s), 8.22 (1H, s), 7.30 (5H, s), 7.26-7.23 (1H, m), 6.87-6.85 (2H, m), 5.13 (1H, d, J = 12.5 Hz), 5.08 (1H, d, J = 12.5 Hz), 3.12 (1H, d, J = 14.6 Hz), 3.05 (1H, d, J = 14.6 Hz), 2.77 (3H, s), 1.88 (3H, s).
1058 ¹H-NMR (CDCl₃) δ: 8.39 (1H, s), 7.88 (1H, s), 7.35-7.33 (2H, m), 7.24-7.18 (4H, m), 6.88-6.86 (1H, m), 6.82-6.80 (1H, m), 3.59 (1H, d, J = 14.1 Hz), 3.54 (1H, d, J = 14.1 Hz), 2.75 (3H, s), 1.87 (3H, s).
1059 ¹H-NMR (CDCl₃) δ: 8.28 (1H, s), 7.91 (1H, s), 7.35-7.33 (2H, m), 7.23-7.18 (4H, m), 6.88-6.86 (1H, m), 6.84-6.79 (1H, m), 3.59 (1H, d, J = 14.1 Hz), 3.54 (1H, d, J = 14.1 Hz), 2.74 (3H, s), 1.87 (3H, s).
1060 ¹H-NMR (CDCl₃) δ: 8.40 (1H, s), 8.32 (1H, s), 7.25-7.22 (1H, m), 6.84-6.81 (2H, m), 2.77 (3H, s), 2.08-2.00 (1H, m), 1.94-1.94 (2H, m), 1.72 (3H, s), 1.01-0.99 (6H, m).
1061 ¹H-NMR (CDCl₃) δ: 8.40 (1H, s), 8.35 (1H, s), 7.29-7.24 (1H, m), 6.91-6.89 (1H, m), 6.87-6.84 (1H, m), 4.06-4.04 (1H, m), 4.01-3.97 (1H, m), 3.05 (1H, d, J = 14.1 Hz), 2.99 (1H, d, J = 14.1 Hz), 2.77 (3H, s), 1.90 (3H, s), 1.64-1.59 (2H, m), 0.90 (3H, t, J = 7.4 Hz).
1062 ¹H-NMR (CDCl₃) δ: 8.40 (1H, s), 8.35 (1H, s), 7.29-7.24 (1H, m), 6.92-6.89 (1H, m), 6.88-6.84 (1H, m), 4.12-4.08 (1H, m), 4.05-4.01 (1H, m), 3.05 (1H, d, J = 14.1 Hz), 2.98 (1H, d, J = 14.1 Hz), 2.77 (3H, s), 1.90 (3H, s), 1.59-1.55 (2H, m), 1.35-1.30 (2H, m), 0.88 (3H, t, J = 7.4 Hz).
1063 ¹H-NMR (CDCl₃) δ: 8.31 (1H, s), 8.12 (1H, s), 7.25-7.21 (1H, m), 6.91-6.91 (1H, m), 6.81 (1H, td, J = 7.8, 4.6 Hz), 3.72-3.64 (2H, m), 3.46-3.41 (2H, m), 2.64 (3H, s), 2.38 (3H, s), 2.35-2.33 (2H, m), 1.74 (3H, s), 1.16 (3H, t, J = 7.1 Hz).
1064 ¹H-NMR (CDCl₃) δ: 8.21 (1H, s), 8.11 (1H, s), 7.30 (5H, s), 7.25-7.20 (1H, m), 6.94 (1H, d, J = 8.0 Hz), 6.85-6.82 (1H, m), 5.14 (1H, d, J = 12.2 Hz), 5.09 (1H, d, J = 12.2 Hz), 3.11 (1H, d, J = 14.1 Hz), 3.05 (1H, d, J = 14.1 Hz), 2.64 (3H, s), 2.38 (3H, s), 1.89 (3H, s).
1065 ¹H-NMR (CDCl₃) δ: 8.11 (1H, s), 7.90 (1H, s), 7.36-7.34 (2H, m), 7.24-7.18 (4H, m), 6.93 (1H, d, J = 8.4 Hz), 6.82-6.80 (1H, m), 3.59 (1H, d, J = 14.1 Hz), 3.54 (1H, d, J = 14.1 Hz), 2.63 (3H, s), 2.37 (3H, s), 1.87 (3H, s).
1066 ¹H-NMR (CDCl₃) δ: 8.29 (1H, s), 8.13 (1H, s), 7.25-7.21 (1H, m), 6.91-6.90 (1H, m), 6.80 (1H, td, J = 7.8, 4.6 Hz), 2.64 (3H, s), 2.38 (3H, s), 2.07-2.00 (1H, m), 1.94 (2H, d, J = 6.1 Hz), 1.71 (3H, s), 1.01-0.99 (6H, m).
1067 ¹H-NMR (CDCl₃) δ: 8.32 (1H, s), 8.12 (1H, s), 7.25-7.23 (1H, m), 6.97 (1H, dt, J = 8.0, 1.1 Hz), 6.85 (1H, td, J = 8.0, 4.6 Hz), 4.06 (1H, dt, J = 12.6, 5.3 Hz), 3.99 (1H, dt, J = 12.6, 5.3 Hz), 3.04 (1H, d, J = 13.7 Hz), 2.99 (1H, d, J = 13.7 Hz), 2.64 (3H, s), 2.38 (3H, s), 1.91 (3H, s), 1.66-1.60 (2H, m), 0.90 (3H, t, J = 7.4 Hz).
1068 ¹H-NMR (CDCl₃) δ: 8.31 (1H, s), 8.11 (1H, s), 7.25-7.23 (1H, m), 6.97 (1H, dt, J = 7.9, 1.2 Hz), 6.85 (1H, td, J = 7.9, 4.6 Hz), 4.10 (1H, dt, J = 12.6, 5.3 Hz), 4.03 (1H, dt, J = 12.6, 5.3 Hz), 3.04 (1H, d, J = 13.2 Hz), 2.98 (1H, d, J = 13.2 Hz), 2.64 (3H, s), 2.38 (3H, s), 1.90 (3H, s), 1.60-1.54 (2H, m), 1.34-1.32 (2H, m), 0.88 (3H, t, J = 7.4 Hz).
1069 ¹H-NMR (CDCl₃) δ: 8.24 (1H, s), 7.94 (1H, s), 7.48-7.47 (1H, m), 7.41-7.39 (1H, m), 7.23 (1H, t, J = 7.9 Hz), 7.18-7.13 (1H, m), 6.96-6.94 (1H, m), 6.81-6.75 (2H, m), 3.82 (3H, s), 2.65 (3H, s), 2.40 (3H, s), 2.09 (3H, s).
1070 ¹H-NMR (CDCl₃) δ: 8.43 (1H, s), 8.40 (1H, s), 7.45-7.45 (1H, m), 7.38-7.36 (1H, m), 7.22 (1H, t, J = 7.9 Hz), 7.16-7.11 (1H, m), 7.07 (1H, dt, J = 7.8, 1.2 Hz), 6.80-6.75 (2H, m), 3.82 (3H, s), 2.83 (3H, s), 2.42 (3H, s), 2.09 (3H, s).
1071 ¹H-NMR (CDCl₃) δ: 8.25 (1H, s), 7.94 (1H, s), 7.88-7.83 (2H, m), 7.40 (1H, dd, J = 8.0, 1.5 Hz), 7.34-7.31 (2H, m), 7.26-7.22 (1H, m), 7.02 (1H, dd, J = 7.6, 1.5 Hz), 6.78 (1H, t, J = 7.8 Hz), 2.65 (3H, s), 2.39 (3H, s), 2.10 (3H, s).
1072 ¹H-NMR (CDCl₃) δ: 8.44 (1H, s), 8.42 (1H, s), 7.81 (2H, dd, J = 8.2, 1.3 Hz), 7.38 (1H, dd, J = 8.0, 1.5 Hz), 7.33-7.30 (2H, m), 7.25-7.23 (1H, m), 7.18 (1H, dd, J = 7.6, 1.5 Hz), 6.78 (1H, t, J = 7.8 Hz), 2.83 (3H, s), 2.42 (3H, s), 2.09 (3H, s).
1073 ¹H-NMR (CDCl₃) δ: 8.42 (2H, d, J = 6.9 Hz), 7.74-7.72 (2H, m), 7.50 (1H, dd, J = 8.0, 1.5 Hz), 7.39-7.35 (2H, m), 7.32-7.28 (1H, m), 6.93 (1H, dd, J = 7.6, 1.5 Hz), 6.82 (1H, t, J = 8.0 Hz), 2.78 (3H, s), 2.07 (3H, s).
1074 ¹H-NMR (CDCl₃) δ: 8.26 (1H, s), 7.93 (1H, s), 7.86-7.81 (2H, m), 7.41 (1H, dd, J = 8.0, 1.5 Hz), 7.07 (1H, dd, J = 8.0, 1.5 Hz), 7.02-6.97 (2H, m), 6.81 (1H, t, J = 7.8 Hz), 2.65 (3H, s), 2.40 (3H, s), 2.08 (3H, s).
1075 ¹H-NMR (CDCl₃) δ: 8.26 (1H, s), 7.93 (1H, s), 7.82-7.78 (2H, m), 7.42 (1H, dd, J = 8.0, 1.5 Hz), 7.30-7.28 (2H, m), 7.07 (1H, dd, J = 7.6, 1.5 Hz), 6.81 (1H, t, J = 7.8 Hz), 2.65 (3H, s), 2.40 (3H, s), 2.07 (3H, s).
1076 ¹H-NMR (CDCl₃) δ: 8.26 (1H, s), 7.95 (1H, s), 7.84-7.82 (2H, m), 7.35-7.31 (2H, m), 7.25-7.23 (1H, m), 7.17-7.13 (1H, m), 6.91 (1H, dt, J = 7.6, 1.1 Hz), 6.77 (1H, td, J = 7.0, 5.0 Hz), 2.65 (3H, s), 2.40 (3H, s), 2.09 (3H, s).
1077 ¹H-NMR (CDCl₃) δ: 8.45 (1H, s), 8.42 (1H, s), 7.80-7.79 (2H, m), 7.34-7.31 (2H, m), 7.25-7.22 (1H, m), 7.14-7.12 (1H, m), 7.08-7.06 (1H, m), 6.77 (1H, td, J = 6.9, 4.6 Hz), 2.83 (3H, s), 2.42 (3H, s), 2.08 (3H, s).
1078 ¹H-NMR (CDCl₃) δ: 8.26 (1H, s), 7.94 (1H, s), 7.84-7.81 (2H, m), 7.18-7.14 (1H, m), 7.02-6.95 (3H, m), 6.79 (1H, td, J = 6.9, 4.6 Hz), 2.65 (3H, s), 2.40 (3H, s), 2.07 (3H, s).
1079 ¹H-NMR (CDCl₃) δ: 8.46 (1H, s), 8.40 (1H, s), 7.80-7.77 (2H, m), 7.16-7.12 (1H, m), 7.10-7.08 (1H, m), 7.01-6.97 (2H, m), 6.81-6.77 (1H, m), 2.83 (3H, s), 2.43 (3H, s), 2.06 (3H, s).
1080 ¹H-NMR (CDCl₃) δ: 8.26 (1H, s), 7.94 (1H, s), 7.80-7.78 (2H, m), 7.30-7.29 (1H, m), 7.19-7.15 (2H, m), 6.96-6.95 (1H, m), 6.80 (1H, td, J = 6.9, 4.6 Hz), 2.65 (3H, s), 2.40 (3H, s), 2.06 (3H, s).
1081 ¹H-NMR (CDCl₃) δ: 8.45 (1H, s), 8.39 (1H, s), 7.76-7.75 (2H, m), 7.30-7.27 (2H, m), 7.17-7.13 (1H, m), 7.10-7.08 (1H, m), 6.79 (1H, td, J = 6.9, 4.6 Hz), 2.83 (3H, s), 2.43 (3H, s), 2.05 (3H, s).
1082 ¹H-NMR (CDCl₃) δ: 8.24 (1H, s), 7.94 (1H, s), 7.71-7.70 (2H, m), 7.16-7.12 (3H, m), 6.90-6.89 (1H, m), 6.77-6.73 (1H, m), 2.65 (3H, s), 2.39 (3H, s), 2.29 (3H, s), 2.08 (3H, s).
1083 ¹H-NMR (CDCl₃) δ: 8.44 (1H, s), 8.42 (1H, s), 7.68-7.66 (2H, m), 7.14-7.10 (3H, m), 7.07-7.04 (1H, m), 6.75 (1H, td, J = 6.8, 4.7 Hz), 2.83 (3H, s), 2.42 (3H, s), 2.29 (3H, s), 2.07 (3H, s).
1084 ¹H-NMR (CDCl₃) δ: 8.32 (1H, s), 8.30 (1H, s), 7.41 (1H, dd, J = 7.8, 1.3 Hz), 7.32 (1H, dd, J = 7.8, 1.3 Hz), 7.01 (1H, t, J = 8.0 Hz), 2.80 (3H, s), 2.38 (3H, s), 2.02-1.91 (2H, m), 1.64 (3H, s), 1.60-1.54 (2H, m), 1.37-1.30 (2H, m), 0.90 (3H, t, J = 7.3 Hz).
1085 ¹H-NMR (CDCl₃) δ: 8.22 (1H, s), 7.97 (1H, s), 7.84 (1H, td, J = 6.0, 1.8 Hz), 7.28-7.23 (1H, m), 7.19-7.15 (1H, m), 7.09-7.04 (2H, m), 6.93-6.92 (1H, m), 6.79 (1H, td, J = 6.9, 4.6 Hz), 2.65 (3H, s), 2.39 (3H, s), 2.21 (3H, s).
1086 ¹H-NMR (CDCl₃) δ: 8.04 (1H, s), 7.86 (1H, s), 7.41 (1H, dd, J = 7.6, 1.9 Hz), 7.01-6.95 (2H, m), 2.63 (3H, s), 2.34 (3H, s), 2.02-1.94 (2H, m), 1.66 (3H, s), 1.59-1.55 (2H, m), 1.34-1.31 (2H, m), 0.90 (3H, t, J = 7.4 Hz).
1087 ¹H-NMR (CDCl₃) δ: 8.25 (1H, s), 7.93 (1H, s), 7.82-7.80 (2H, m), 7.36 (1H, ddd, J = 8.6, 7.1, 1.1 Hz), 7.15 (1H, dd, J = 7.6, 1.5 Hz), 7.01-6.96 (3H, m), 6.85 (1H, td, J = 5.3, 1.0 Hz), 2.65 (3H, s), 2.39 (3H, s), 2.02 (3H, s).
1088 ¹H-NMR (CDCl₃) δ: 8.29 (1H, s), 8.01-7.99 (1H, m), 7.94 (1H, s), 7.71 (1H, dt, J = 7.6, 1.5 Hz), 7.25-7.21 (2H, m), 7.20-7.16 (1H, m), 7.01-6.99 (1H, m), 6.81 (1H, td, J = 6.9, 4.6 Hz), 2.65 (3H, s), 2.41 (3H, s), 2.06 (3H, s).
1089 ¹H-NMR (CDCl₃) δ: 8.47 (1H, s), 8.40 (1H, s), 7.90-7.90 (1H, m), 7.69 (1H, dt, J = 7.6, 1.5 Hz), 7.28-7.20 (2H, m), 7.18-7.14 (1H, m), 7.12-7.10 (1H, m), 6.81 (1H, td, J = 6.9, 4.6 Hz), 2.83 (3H, s), 2.43 (3H, s), 2.05 (3H, s).
1090 ¹H-NMR (CDCl₃) δ: 8.22 (1H, s), 7.93 (1H, s), 7.89-7.87 (1H, m), 7.20-7.10 (4H, m), 6.92 (1H, d, J = 8.0 Hz), 6.79 (1H, td, J = 6.9, 4.6 Hz), 2.78 (3H, s), 2.65 (3H, s), 2.39 (3H, s), 2.15 (3H, s).
1091 ¹H-NMR (CDCl₃) δ: 8.42 (1H, s), 8.41 (1H, s), 7.83-7.81 (1H, m), 7.18-7.09 (5H, m), 6.79 (1H, td, J = 7.0, 5.0 Hz), 2.82 (3H, s), 2.78 (3H, s), 2.42 (3H, s), 2.13 (3H, s).
1092 ¹H-NMR (CDCl₃) δ: 8.29 (1H, s), 7.94 (1H, s), 7.67-7.63 (1H, m), 7.62-7.60 (1H, m), 7.29 (1H, td, J = 7.3, 5.7 Hz), 7.19-7.15 (1H, m), 7.00-6.98 (1H, m), 6.96-6.92 (1H, m), 6.81 (1H, td, J = 7.1, 4.7 Hz), 2.66 (3H, s), 2.40 (3H, s), 2.07 (3H, s).
1093 ¹H-NMR (CDCl₃) δ: 8.47 (1H, s), 8.41 (1H, s), 7.60-7.57 (2H, m), 7.31-7.27 (1H, m), 7.17-7.14 (1H, m), 7.11-7.10 (1H, m), 6.95-6.91 (1H, m), 6.80 (1H, td, J = 6.9, 4.6 Hz), 2.83 (3H, s), 2.43 (3H, s), 2.06 (3H, s).
1094 ¹H-NMR (CDCl₃) δ: 8.48 (1H, s), 8.42 (1H, s), 7.91-7.89 (1H, m), 7.40-7.38 (1H, m), 7.20-7.15 (3H, m), 7.12 (1H, d, J = 7.6 Hz), 6.81 (1H, td, J = 6.9, 4.7 Hz), 2.83 (3H, s), 2.42 (3H, s), 2.24 (3H, s).
1095 ¹H-NMR (CDCl₃) δ: 8.25 (1H, s), 7.93 (1H, s), 7.79-7.77 (2H, m), 7.37 (1H, ddd, J = 8.6, 7.1, 1.1 Hz), 7.29-7.27 (2H, m), 7.16 (1H, dd, J = 7.6, 1.5 Hz), 7.01 (1H, dd, J = 8.2, 1.0 Hz), 6.86 (1H, td, J = 5.4, 1.0 Hz), 2.65 (3H, s), 2.39 (3H, s), 2.02 (3H, s).
1096 ¹H-NMR (CDCl₃) δ: 8.45 (1H, s), 8.38 (1H, s), 7.76-7.74 (2H, m), 7.35 (1H, ddd, J = 8.6, 6.9, 1.2 Hz), 7.30-7.25 (3H, m), 6.99 (1H, dd, J = 8.1, 1.0 Hz), 6.86 (1H, td, J = 5.4, 1.0 Hz), 2.83 (3H, s), 2.42 (3H, s), 2.01 (3H, s)
1097 ¹H-NMR (CDCl₃) δ: 8.23 (1H, s), 7.93 (1H, s), 7.83-7.80 (2H, m), 7.33-7.29 (2H, m), 7.25-7.19 (2H, m), 6.96-6.93 (1H, m), 6.72 (1H, t, J = 7.7 Hz), 2.64 (3H, s), 2.38-2.38 (6H, m), 2.05 (3H, s).
1098 ¹H-NMR (CDCl₃) δ: 8.43 (1H, s), 8.40 (1H, s), 7.80-7.78 (2H, m), 7.32-7.17 (4H, m), 7.10 (1H, dd, J = 7.9, 1.1 Hz), 6.73 (1H, t, J = 7.9 Hz), 2.82 (3H, s), 2.41 (3H, s), 2.37 (3H, s), 2.03 (3H, s).
1099 ¹H-NMR (CDCl₃) δ: 8.24 (1H, s), 7.92 (1H, s), 7.82-7.78 (2H, m), 7.22-7.21 (1H, m), 7.01-6.95 (3H, m), 6.75 (1H, t, J = 7.6 Hz), 2.65 (3H, s), 2.39 (3H, s), 2.36 (3H, s), 2.02 (3H, s).
1100 ¹H-NMR (CDCl₃) δ: 8.44 (1H, s), 8.37 (1H, s), 7.80-7.76 (2H, m), 7.21-7.19 (1H, m), 7.13-7.10 (1H, m), 7.00-6.95 (2H, m), 6.75 (1H, t, J = 7.6 Hz), 2.82 (3H, s), 2.41 (3H, s), 2.35 (3H, s), 2.01 (3H, s).
1101 ¹H-NMR (CDCl₃) δ: 8.27 (1H, s), 7.92 (1H, s), 7.64-7.62 (1H, m), 7.60-7.58 (1H, m), 7.29-7.25 (1H, m), 7.23-7.22 (1H, m), 7.02 (1H, dd, J = 7.6, 1.1 Hz), 6.94-6.90 (1H, m), 6.77 (1H, t, J = 7.6 Hz), 2.65 (3H, s), 2.39 (3H, s), 2.38 (3H, s), 2.03 (3H, s).
1102 ¹H-NMR (CDCl₃) δ: 8.45 (1H, s), 8.38 (1H, s), 7.58-7.55 (2H, m), 7.28-7.23 (1H, m), 7.21-7.20 (1H, m), 7.13 (1H, dd, J = 7.8, 1.0 Hz), 6.91-6.90 (1H, m), 6.76 (1H, t, J = 7.8 Hz), 2.82 (3H, s), 2.42 (3H, s), 2.37 (3H, s), 2.01 (3H, s).
1103 ¹H-NMR (CDCl₃) δ: 8.23 (1H, s), 7.93 (1H, s), 7.69-7.68 (2H, m), 7.20-7.18 (1H, m), 7.11 (2H, d, J = 7.8 Hz), 6.93 (1H, dd, J = 7.8, 1.0 Hz), 6.71 (1H, t, J = 7.8 Hz), 2.64 (3H, s), 2.38 (3H, s), 2.37 (3H, s), 2.29 (3H, s), 2.04 (3H, s).
1104 ¹H-NMR (CDCl₃) δ: 8.43 (1H, s), 8.39 (1H, s), 7.66-7.66 (2H, m), 7.17 (1H, d, J = 7.6 Hz), 7.10-7.08 (3H, m), 6.72 (1H, t, J = 7.6 Hz), 2.82 (3H, s), 2.41 (3H, s), 2.36 (3H, s), 2.28 (3H, s), 2.03 (3H, s).
1105 ¹H-NMR (CDCl₃) δ: 8.44 (1H, s), 8.37 (1H, s), 7.75-7.73 (2H, m), 7.28-7.24 (2H, m), 7.21-7.19 (1H, m), 7.11 (1H, dd, J = 7.6, 1.1 Hz), 6.76 (1H, t, J = 7.6 Hz), 2.82 (3H, s), 2.42 (3H, s), 2.35 (3H, s), 2.01 (3H, s).
1106 ¹H-NMR (CDCl₃) δ: 8.44 (1H, s), 8.42 (1H, s), 7.83 (1H, td, J = 7.6, 1.8 Hz), 7.23-7.19 (2H, m), 7.13-7.12 (1H, m), 7.04-7.02 (2H, m), 6.75 (1H, t, J = 7.6 Hz), 2.83 (3H, s), 2.42 (3H, s), 2.33 (3H, s), 2.17 (3H, s).
1107 ¹H-NMR (CDCl₃) δ: 8.33 (1H, d, J = 1.9 Hz), 7.95 (1H, s), 7.78-7.76 (3H, m), 7.28-7.27 (2H, m), 7.24-7.22 (1H, m), 7.00 (1H, dd, J = 7.6, 1.0 Hz), 6.77 (1H, t, J = 7.6 Hz), 2.48 (3H, s), 2.37 (3H, s), 2.02 (3H, s).
1108 ¹H-NMR (CDCl₃) δ: 8.31 (1H, d, J = 1.9 Hz), 7.98 (1H, s), 7.89-7.88 (1H, m), 7.79-7.79 (1H, m), 7.23-7.21 (2H, m), 7.05-7.03 (2H, m), 6.98 (1H, dd, J = 7.6, 1.1 Hz), 6.76 (1H, t, J = 7.6 Hz), 2.47 (3H, d, J = 1.1 Hz), 2.34 (3H, s), 2.18 (3H, s).
1109 ¹H-NMR (CDCl₃) δ: 8.24 (1H, s), 7.94 (1H, s), 7.74-7.70 (2H, m), 7.39 (1H, dd, J = 8.1, 1.5 Hz), 7.15-7.11 (2H, m), 7.00 (1H, dd, J = 7.8, 1.5 Hz), 6.76 (1H, t, J = 7.8 Hz), 2.65 (3H, d, J = 0.5 Hz), 2.39 (3H, s), 2.29 (3H, s), 2.09 (3H, s).
1110 ¹H-NMR (CDCl₃) δ: 8.43 (1H, s), 8.42 (1H, s), 7.70-7.67 (2H, m), 7.37 (1H, dd, J = 8.1, 1.5 Hz), 7.17 (1H, dd, J = 7.7, 1.6 Hz), 7.14-7.10 (2H, m), 6.76 (1H, t, J = 7.8 Hz), 2.83 (3H, s), 2.42 (3H, s), 2.29 (3H, s), 2.08 (3H, s).
1111 ¹H-NMR (CDCl₃) δ: 8.42 (1H, s), 8.41 (1H, s), 7.62-7.58 (2H, m), 7.48 (1H, dd, J = 8.1, 1.5 Hz), 7.19-7.15 (2H, m), 6.91 (1H, dd, J = 7.8, 1.5 Hz), 6.80 (1H, t, J = 7.9 Hz), 2.77 (3H, s), 2.32 (3H, s), 2.05 (3H, s).
1112 ¹H-NMR (CDCl₃) δ: 8.43 (1H, s), 8.40 (1H, s), 7.66-7.63 (2H, m), 7.48 (1H, dd, J = 7.8, 1.5 Hz), 6.94-6.87 (3H, m), 6.81 (1H, t, J = 7.8 Hz), 3.78 (3H, s), 2.78 (3H, d, J = 2.0 Hz), 2.05 (3H, s), 1.57 (3H, s).
1113 ¹H-NMR (CDCl₃) δ: 8.44 (1H, s), 8.41 (1H, s), 7.74-7.71 (2H, m), 7.37 (1H, dd, J = 8.0, 1.5 Hz), 7.18 (1H, dd, J = 7.6, 1.5 Hz), 6.86-6.82 (2H, m), 6.77 (1H, t, J = 7.8 Hz), 3.76 (3H, s), 2.83 (3H, s), 2.42 (3H, s), 2.08 (3H, s).
1114 ¹H-NMR (CDCl₃) δ: 8.20 (1H, s), 7.96 (1H, s), 7.81 (1H, td, J = 5.9, 1.8 Hz), 7.42 (1H, dd, J = 8.1, 1.5 Hz), 7.29-7.23 (1H, m), 7.11-7.01 (3H, m), 6.80 (1H, t, J = 7.9 Hz), 2.65 (3H, d, J = 0.7 Hz), 2.38 (3H, d, J = 0.5 Hz), 2.22 (3H, d, J = 0.5 Hz).
1115 ¹H-NMR (CDCl₃) δ: 8.44 (1H, s), 8.41 (1H, s), 7.74 (1H, td, J = 5.9, 1.8 Hz), 7.41 (1H, dd, J = 8.0, 1.5 Hz), 7.25-7.22 (2H, m), 7.09-7.03 (2H, m), 6.81 (1H, t, J = 7.8 Hz), 2.83 (3H, s), 2.42 (3H, s), 2.20 (3H, s).
1116 ¹H-NMR (CDCl₃) δ: 8.21 (1H, s), 7.93 (1H, s), 7.88-7.87 (1H, m), 7.20-7.10 (4H, m), 6.92 (1H, d, J = 8.0 Hz), 6.80-6.77 (1H, m), 2.78 (3H, s), 2.65 (3H, s), 2.39 (3H, s), 2.14 (3H, s).
1117 ¹H-NMR (CDCl₃) δ: 8.42 (1H, s), 8.41 (1H, s), 7.83-7.80 (1H, m), 7.18-7.09 (5H, m), 6.79 (1H, td, J = 6.9, 4.6 Hz), 2.82 (3H, s), 2.78 (3H, s), 2.41 (3H, s), 2.13 (3H, s).
1118 ¹H-NMR (CDCl₃) δ: 8.28 (1H, s), 7.97 (1H, t, J = 1.7 Hz), 7.93 (1H, s), 7.70-7.68 (1H, m), 7.40-7.35 (1H, m), 7.24-7.18 (3H, m), 7.02 (1H, dd, J = 8.3, 0.7 Hz), 6.87 (1H, td, J = 5.5, 1.1 Hz), 2.65 (3H, s), 2.40 (3H, s), 2.02 (3H, s).
1119 ¹H-NMR (CDCl₃) δ: 8.46 (1H, s), 8.40 (1H, s), 7.88 (1H, t, J = 1.7 Hz), 7.69 (1H, dt, J = 7.6, 1.5 Hz), 7.39-7.34 (1H, m), 7.31 (1H, dd, J = 7.7, 1.6 Hz), 7.24-7.18 (2H, m), 7.01 (1H, dd, J = 8.2, 0.9 Hz), 6.87 (1H, td, J = 5.4, 1.1 Hz), 2.83 (3H, s), 2.42 (3H, s), 2.01 (3H, s).
1120 ¹H-NMR (CDCl₃) δ: 8.24 (1H, s), 7.96 (1H, s), 7.89 (1H, td, J = 5.9, 1.7 Hz), 7.36 (1H, td, J = 5.7, 1.9 Hz), 7.26-7.21 (1H, m), 7.14 (1H, dd, J = 7.6, 1.5 Hz), 7.06-7.02 (3H, m), 6.86 (1H, td, J = 5.5, 1.1 Hz), 2.66 (3H, s), 2.39 (3H, s), 2.17 (3H, s).
1121 ¹H-NMR (CDCl₃) δ: 8.44 (1H, s), 8.43 (1H, s), 7.83-7.80 (1H, m), 7.36-7.30 (2H, m), 7.23-7.19 (1H, m), 7.05-7.00 (3H, m), 6.87 (1H, td, J = 5.4, 1.0 Hz), 2.83 (3H, s), 2.42 (3H, s), 2.16 (3H, s).
1122 ¹H-NMR (CDCl₃) δ: 8.24 (1H, s), 8.09-8.07 (1H, m), 7.97 (1H, s), 7.39-7.34 (2H, m), 7.17-7.14 (3H, m), 7.10 (1H, dd, J = 8.2, 1.0 Hz), 6.87 (1H, td, J = 5.5, 1.1 Hz), 2.65 (3H, s), 2.39 (3H, s), 2.22 (3H, s).
1123 ¹H-NMR (CDCl₃) δ: 8.45 (1H, s), 8.45 (1H, s), 8.00-7.98 (1H, m), 7.37-7.30 (3H, m), 7.17-7.14 (2H, m), 7.08 (1H, d, J = 7.3 Hz), 6.87 (1H, t, J = 6.9 Hz), 2.83 (3H, s), 2.42 (3H, s), 2.21 (3H, s).
1124 ¹H-NMR (CDCl₃) δ: 8.21 (1H, s), 7.93 (1H, s), 7.89 (1H, d, J = 6.9 Hz), 7.37 (1H, ddd, J = 8.6, 7.1, 1.1 Hz), 7.15-7.09 (4H, m), 7.04 (1H, dd, J = 8.2, 1.0 Hz), 6.86 (1H, td, J = 5.3, 1.1 Hz), 2.74 (3H, s), 2.64 (3H, s), 2.38 (3H, s), 2.10 (3H, s).
1125 ¹H-NMR (CDCl₃) δ: 8.42 (1H, s), 8.40 (1H, s), 7.85-7.83 (1H, m), 7.38-7.35 (1H, m), 7.31-7.30 (1H, m), 7.15-7.09 (3H, m), 7.02 (1H, d, J = 7.3 Hz), 6.86 (1H, td, J = 5.5, 1.1 Hz), 2.82 (3H, s), 2.74 (3H, s), 2.41 (3H, s), 2.09 (3H, s).
1126 ¹H-NMR (CDCl₃) δ: 8.20 (1H, s), 8.04 (1H, dd, J = 6.1, 3.4 Hz), 7.95 (1H, s), 7.41-7.38 (1H, m), 7.25-7.17 (3H, m), 6.96 (1H, dd, J = 7.8, 1.0 Hz), 6.75 (1H, t, J = 7.6 Hz), 2.65 (3H, s), 2.40 (3H, s), 2.38 (3H, s), 2.23 (3H, s).
1127 ¹H-NMR (CDCl₃) δ: 8.44 (1H, s), 8.42 (1H, s), 7.98-7.95 (1H, m), 7.40-7.37 (1H, m), 7.22 (1H, dq, J = 7.4, 0.8 Hz), 7.19-7.13 (3H, m), 6.77 (1H, t, J = 7.6 Hz), 2.82 (3H, s), 2.41 (3H, s), 2.40 (3H, s), 2.21 (3H, s).
1128 ¹H-NMR (CDCl₃) δ: 8.23 (1H, s), 7.93 (1H, s), 7.68 (1H, s), 7.59 (1H, d, J = 8.0 Hz), 7.21-7.17 (2H, m), 7.04 (1H, d, J = 7.6 Hz), 6.94 (1H, d, J = 7.6 Hz), 6.72 (1H, t, J = 7.4 Hz), 2.65 (3H, s), 2.39 (3H, s), 2.38 (3H, s), 2.34 (3H, s), 2.04 (3H, s).
1129 ¹H-NMR (CDCl₃) δ: 8.43 (1H, s), 8.40 (1H, s), 7.65-7.63 (1H, m), 7.57 (1H, d, J = 7.6 Hz), 7.20-7.17 (2H, m), 7.10-7.08 (1H, m), 7.03 (1H, d, J = 7.6 Hz), 6.73 (1H, t, J = 7.6 Hz), 2.82 (3H, s), 2.41 (3H, s), 2.36 (3H, s), 2.33 (3H, s), 2.02 (3H, s).
1130 ¹H-NMR (CDCl₃) δ: 8.27 (1H, s), 7.98-7.98 (1H, m), 7.92 (1H, s), 7.68 (1H, dt, J = 7.5, 1.3 Hz), 7.23-7.21 (3H, m), 7.02 (1H, dd, J = 7.5, 1.3 Hz), 6.77 (1H, t, J = 7.5 Hz), 2.65 (3H, s), 2.40 (3H, s), 2.38 (3H, s), 2.03 (3H, s).
1131 ¹H-NMR (CDCl₃) δ: 8.46 (1H, s), 8.38 (1H, s), 7.89-7.88 (1H, m), 7.68 (1H, dt, J = 7.8, 1.4 Hz), 7.22-7.19 (3H, m), 7.13 (1H, dd, J = 7.8, 1.4 Hz), 6.77 (1H, t, J = 7.8 Hz), 2.82 (3H, s), 2.42 (3H, s), 2.37 (3H, s), 2.01 (3H, s).
1132 ¹H-NMR (CDCl₃) δ: 8.23 (1H, s), 7.95 (1H, s), 7.92-7.88 (1H, m), 7.24-7.19 (2H, m), 7.05-7.03 (2H, m), 6.97 (1H, dq, J = 7.8, 0.7 Hz), 6.74 (1H, t, J = 7.8 Hz), 2.65 (3H, s), 2.38 (3H, s), 2.33 (3H, s), 2.18 (3H, s).
1133 ¹H-NMR (CDCl₃) δ: 8.23 (1H, s), 7.94 (1H, s), 7.70-7.67 (2H, m), 7.34 (1H, ddd, J = 8.5, 7.0, 1.2 Hz), 7.12-7.09 (3H, m), 7.01 (1H, dd, J = 8.2, 1.1 Hz), 6.81 (1H, td, J = 5.5, 1.2 Hz), 2.65 (3H, s), 2.38 (3H, s), 2.29 (3H, s), 2.04 (3H, s).
1134 ¹H-NMR (CDCl₃) δ: 8.25 (1H, s), 7.94 (1H, s), 7.83-7.81 (2H, m), 7.37-7.29 (3H, m), 7.23 (1H, tt, J = 7.3, 1.5 Hz), 7.11 (1H, dd, J = 7.6, 1.5 Hz), 7.02 (1H, dd, J = 8.2, 1.0 Hz), 6.83 (1H, td, J = 5.5, 1.1 Hz), 2.65 (3H, s), 2.39 (3H, s), 2.05 (3H, s).
1135 ¹H-NMR (CDCl₃) δ: 8.27 (1H, s), 7.94 (1H, s), 7.64 (1H, ddd, J = 10.4, 2.6, 1.7 Hz), 7.59 (1H, ddd, J = 7.8, 0.9, 0.4 Hz), 7.37 (1H, ddd, J = 8.6, 6.9, 1.2 Hz), 7.30-7.24 (1H, m), 7.18 (1H, dd, J = 7.7, 1.6 Hz), 7.02 (1H, dd, J = 8.3, 1.0 Hz), 6.92 (1H, tdd, J = 8.4, 2.4, 0.9 Hz), 6.86 (1H, td, J = 5.4, 1.0 Hz), 2.65 (3H, s), 2.40 (3H, s), 2.03 (3H, s).
1136 ¹H-NMR (CDCl₃) δ: 8.46 (1H, s), 8.40 (1H, s), 7.59-7.55 (2H, m), 7.36 (1H, ddd, J = 8.6, 6.9, 1.2 Hz), 7.32-7.24 (2H, m), 7.01 (1H, dd, J = 8.2, 1.1 Hz), 6.93-6.84 (2H, m), 2.83 (3H, s), 2.42 (3H, s), 2.02 (3H, s).
1137 ¹H-NMR (CDCl₃) δ: 8.23 (1H, s), 7.94 (1H, s), 7.68-7.67 (1H, m), 7.61-7.59 (1H, m), 7.35 (1H, ddd, J = 8.5, 7.0, 1.0 Hz), 7.20 (1H, t, J = 7.7 Hz), 7.12-7.10 (1H, m), 7.06-7.00 (2H, m), 6.82 (1H, td, J = 5.4, 1.1 Hz), 2.65 (3H, s), 2.39 (3H, s), 2.34 (3H, s), 2.03 (3H, s).
1138 ¹H-NMR (CDCl₃) δ: 8.44 (1H, s), 8.41 (1H, s), 7.64-7.63 (1H, m), 7.59-7.56 (1H, m), 7.33 (1H, ddd, J = 8.6, 6.9, 1.2 Hz), 7.28 (1H, dd, J = 7.7, 1.3 Hz), 7.19 (1H, t, J = 7.7 Hz), 7.03-7.02 (1H, m), 7.00 (1H, dd, J = 8.3, 1.0 Hz), 6.83 (1H, td, J = 5.4, 1.2 Hz), 2.83 (3H, s), 2.41 (3H, s), 2.34 (3H, s), 2.02 (3H, s).
1139 ¹H-NMR (CDCl₃) δ: 8.40 (1H, s), 8.14 (1H, s), 7.75-7.73 (2H, m), 7.47 (1H, dd, J = 8.0, 1.5 Hz), 7.38-7.35 (2H, m), 7.31-7.27 (1H, m), 6.98 (1H, dd, J = 8.0, 1.5 Hz), 6.80 (1H, t, J = 8.0 Hz), 2.65 (3H, s), 2.38 (3H, s), 2.06 (3H, s).
1140 ¹H-NMR (CDCl₃) δ: 8.45 (1H, s), 8.39 (1H, s), 7.77-7.76 (2H, m), 7.40 (1H, dd, J = 8.0, 1.5 Hz), 7.30-7.26 (2H, m), 7.20 (1H, dd, J = 7.8, 1.3 Hz), 6.80 (1H, t, J = 8.0 Hz), 2.83 (3H, s), 2.42 (3H, s), 2.06 (3H, s).
1141 ¹H-NMR (CDCl₃) δ: 8.36 (1H, s), 8.16 (1H, s), 7.66-7.62 (2H, m), 7.27 (1H, s), 6.90-6.84 (3H, m), 6.74 (1H, t, J = 7.6 Hz), 2.65 (3H, s), 2.38 (3H, s), 2.36 (3H, s), 2.00 (3H, s).
1142 ¹H-NMR (CDCl₃) δ: 8.28 (1H, s), 7.93 (1H, s), 7.68-7.65 (1H, m), 7.63-7.61 (1H, m), 7.42 (1H, dd, J = 8.0, 1.5 Hz), 7.32-7.26 (1H, m), 7.10 (1H, dd, J = 7.6, 1.5 Hz), 6.93 (1H, tdd, J = 8.4, 2.3, 1.0 Hz), 6.82 (1H, t, J = 7.8 Hz), 2.65 (3H, s), 2.40 (3H, s), 2.08 (3H, s).
1143 ¹H-NMR (CDCl₃) δ: 8.46 (1H, s), 8.40 (1H, s), 7.61-7.58 (2H, m), 7.41 (1H, dd, J = 8.0, 1.5 Hz), 7.30-7.27 (1H, m), 7.22 (1H, dd, J = 7.6, 1.5 Hz), 6.92 (1H, tdd, J = 8.4, 2.2, 1.0 Hz), 6.81 (1H, t, J = 7.8 Hz), 2.83 (3H, s), 2.42 (3H, s), 2.07 (3H, s).
1144 ¹H-NMR (CDCl₃) δ: 8.44 (1H, s), 8.40 (1H, s), 7.54-7.48 (3H, m), 7.37-7.31 (1H, m), 7.01-6.96 (2H, m), 6.85 (1H, t, J = 7.8 Hz), 2.78 (3H, s), 2.05 (3H, s).
1145 ¹H-NMR (CDCl₃) δ: 8.37 (1H, d, J = 1.9 Hz), 7.97 (1H, s), 7.81-7.79 (1H, m), 7.68-7.64 (1H, m), 7.63-7.61 (1H, m), 7.43 (1H, dd, J = 8.0, 1.5 Hz), 7.31-7.27 (1H, m), 7.11 (1H, dd, J = 7.6, 1.5 Hz), 6.94 (1H, tdd, J = 8.4, 2.3, 1.0 Hz), 6.83 (1H, t, J = 8.0 Hz), 2.49 (3H, d, J = 1.1 Hz), 2.08 (3H, s).
1146 ¹H-NMR (CDCl₃) δ: 8.43 (1H, s), 8.33 (1H, s), 7.73-7.70 (2H, m), 7.39-7.36 (2H, m), 7.32-7.28 (1H, m), 7.26-7.22 (1H, m), 6.83-6.80 (2H, m), 2.76 (3H, s), 2.06 (3H, s).
1147 ¹H-NMR (CDCl₃) δ: 8.44 (1H, s), 8.41 (1H, s), 7.73-7.70 (2H, m), 7.39-7.35 (2H, m), 7.33-7.27 (1H, m), 7.27-7.21 (1H, m), 6.84-6.78 (2H, m), 2.78 (3H, s), 2.06 (3H, s).
1148 ¹H-NMR (CDCl₃) δ: 8.40 (1H, s), 8.15 (1H, s), 7.74-7.70 (2H, m), 7.39-7.35 (2H, m), 7.31-7.28 (1H, m), 7.25-7.20 (1H, m), 6.88-6.86 (1H, m), 6.82-6.76 (1H, m), 2.65 (3H, s), 2.39 (3H, s), 2.06 (3H, s).
1149 ¹H-NMR (CDCl₃) δ: 8.47 (1H, s), 8.29 (1H, s), 7.66 (1H, td, J = 6.7, 1.7 Hz), 7.52 (1H, dd, J = 8.0, 1.1 Hz), 7.33-7.28 (1H, m), 7.14-7.08 (2H, m), 6.99 (1H, dd, J = 7.6, 1.5 Hz), 6.85 (1H, t, J = 8.0 Hz), 2.75 (3H, s), 2.17 (3H, s).
1150 ¹H-NMR (CDCl₃) δ: 8.45 (1H, s), 8.40 (1H, s), 7.66 (1H, td, J = 5.7, 1.7 Hz), 7.52 (1H, dd, J = 8.0, 1.5 Hz), 7.33-7.28 (1H, m), 7.13-7.09 (2H, m), 6.99 (1H, dd, J = 7.6, 1.5 Hz), 6.85 (1H, t, J = 8.0 Hz), 2.77 (3H, s), 2.17 (3H, s).
1151 ¹H-NMR (CDCl₃) δ: 8.42 (1H, s), 8.11 (1H, s), 7.67 (1H, td, J = 5.9, 1.8 Hz), 7.50 (1H, dd, J = 8.1, 1.5 Hz), 7.33-7.27 (1H, m), 7.14-7.11 (1H, m), 7.11-7.07 (1H, m), 7.05-7.03 (1H, m), 6.84 (1H, t, J = 7.9 Hz), 2.64 (3H, s), 2.38 (3H, s), 2.16 (3H, s).
1152 ¹H-NMR (CDCl₃) δ: 8.41 (1H, s), 8.35 (1H, s), 7.53-7.51 (1H, m), 7.49-7.46 (2H, m), 7.36-7.30 (1H, m), 7.10 (1H, dd, J = 8.2, 1.0 Hz), 7.05 (1H, dd, J = 8.0, 1.5 Hz), 6.98 (1H, tdd, J = 8.4, 2.4, 0.8 Hz), 6.90 (1H, td, J = 7.5, 1.0 Hz), 2.77 (3H, s), 2.00 (3H, s).
1153 ¹H-NMR (CDCl₃) δ: 8.37 (1H, s), 8.17 (1H, s), 7.54-7.50 (1H, m), 7.49-7.43 (2H, m), 7.35-7.29 (1H, m), 7.11-7.07 (2H, m), 6.99-6.95 (1H, m), 6.88 (1H, td, J = 5.5, 1.0 Hz), 2.65 (3H, s), 2.39 (3H, s), 1.99 (3H, s).
1154 ¹H-NMR (CDCl₃) δ: 8.45 (1H, s), 8.32 (1H, s), 7.71 (1H, td, J = 7.5, 1.7 Hz), 7.49-7.45 (1H, m), 7.31-7.27 (1H, m), 7.12-7.05 (4H, m), 6.90 (1H, td, J = 7.5, 1.1 Hz), 2.76 (3H, s), 2.13 (3H, s).
1155 ¹H-NMR (CDCl₃) δ: 8.44 (1H, s), 8.43 (1H, s), 7.71 (1H, td, J = 5.7, 1.7 Hz), 7.50-7.44 (1H, m), 7.31-7.26 (1H, m), 7.12-7.04 (4H, m), 6.90 (1H, td, J = 7.5, 1.1 Hz), 2.78 (3H, s), 2.13 (3H, s).

### [Activity evaluation]

Next, the compounds are specifically shown to be effective for plant diseases, though the present invention is not limited by these examples.

### [Test Example A]

### Rice blast disease pot test

Seeds of test plants (rice variety: Sachikaze) were planted and then cultivated until the second leaves appeared. In the test, each synthesized compound was dissolved in a dimethyl sulfoxide-methanol mixed solution (volume ratio: 9/1) and diluted into a concentration of 250 ppm with ion exchange water to obtain a chemical. The obtained chemical was sprayed to the test plants (2.5 ml/pot). After drying of the chemical, the plants were inoculated by spraying of 3 to 5 × 10⁴ organisms/ml of a conidium suspension of *Magnaporthe grisea*. The plants thus inoculated were left at room temperature of 23 to 25°C in a moist chamber for approximately 24 hours to promote the onset of the disease. The degree of disease development was investigated 6 to 8 days after inoculation, and effects of the chemical were evaluated.

### [Test Example B] Tomato gray mold pot test

Seeds of test plants (tomato variety: Oogata Fukuju) were planted and then cultivated until three first leaves appeared. In the test, each compound was dissolved in a dimethyl sulfoxide-methanol mixed solution (volume ratio: 9/1) and diluted into a concentration of 250 ppm with ion exchange water to obtain a chemical. The obtained chemical was sprayed to the test plants (2.5 ml/pot). After drying of the chemical, the plants were inoculated by spraying of 3 to 6 × 10⁵ organisms/ml of a conidium suspension of *Botrytis cinerea*. The plants thus inoculated were left at room temperature of 23 to 25°C in a moist chamber for approximately 48 hours to promote the onset of the disease. The degree of disease development was investigated 2 to 3 days after inoculation, and effects of the chemical were evaluated.

The degrees of disease development in these Test Examples were evaluated by 0.05 when the degree of disease development in a plant having no onset of the disease was defined as 0 and the degree of disease development in a plant in a group without chemical treatment was defined as 3. From the degree of disease development, a control value was calculated according to the following expression.

### <Control value>

$Control value = 100 \left\{1-\left(n/3\right)\right\}$ N = Degree of disease development in each chemical treatment group.

### [Test Example C] Gray mold anti-germ test

Gray mold fungus (Botrytis cinerea) Conidia were suspended in Czapek-dox broth and a suspension of 1 × 10⁵ spores were prepared. The prepared spore suspension was seeded at 100 µl/well to a 96-well multiplate. In the test, each compound was dissolved in a dimethyl sulfoxide-methanol mixed solution (volume ratio: 9/1) and dispensed at 50 ppm/well. The absorbance of each well was measured at a wavelength of 600 nm using a photometer. The multiplate was incubated at 25°C for 3 days, followed by absorbance measurement again. The difference in absorbance between the treatment day and 3 days later was calculated, and the rate of inhibition was calculated from the ratio between the treatment group and the non-treatment group.

### <Rate of inhibition>

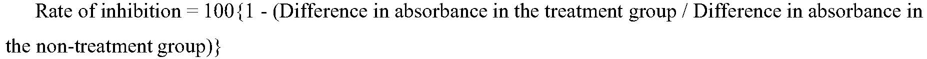

Rate of inhibition = 100{1 - (Difference in absorbance in the treatment group / Difference in absorbance in the non-treatment group)}

### [Test Example D] Fusarium blight anti-germ test

Fusarium blight fungus (Fusarium graminearum) Conidia were suspended in Czapek-dox broth and a suspension of 1 × 10⁵ spores were prepared. The prepared spore suspension was seeded at 100 µl/well to a 96-well multiplate. In the test, each compound was dissolved in a dimethyl sulfoxide-methanol mixed solution (volume ratio: 9/1) and dispensed at 50 ppm/well. The absorbance of each well was measured at a wavelength of 600 nm using a photometer. The multiplate was incubated at 25°C for 3 days, followed by absorbance measurement again. The difference in absorbance between the treatment day and 3 days later was calculated, and the rate of inhibition was calculated from the ratio between the treatment group and the non-treatment group.

### <Rate of inhibition>

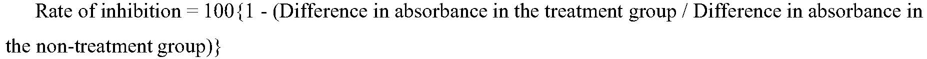

Rate of inhibition = 100{1 - (Difference in absorbance in the treatment group / Difference in absorbance in the non-treatment group)}

### [Test Example E] Anthracnose anti-germ test

Anthracnose fungus (Glomerella cingulata) Conidia were suspended in Czapek-dox broth and a suspension of 1 × 10⁵ spores were prepared. The prepared spore suspension was seeded at 100 µl/well to a 96-well multiplate. In the test, each compound was dissolved in a dimethyl sulfoxide-methanol mixed solution (volume ratio: 9/1) and dispensed at 50 ppm/well. The absorbance of each well was measured at a wavelength of 600 nm using a photometer. The multiplate was incubated at 25°C for 3 days, followed by absorbance measurement again. The difference in absorbance between the treatment day and 3 days later was calculated, and the rate of inhibition was calculated from the ratio between the treatment group and the non-treatment group.

### <Rate of inhibition>

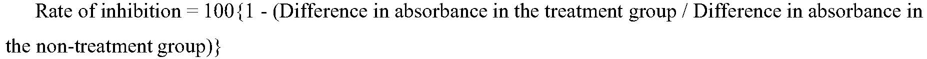

Rate of inhibition = 100{1 - (Difference in absorbance in the treatment group / Difference in absorbance in the non-treatment group)}

### [Test Example F] Speckled leaf blotch anti-germ test

Speckled leaf blotch fungus (Septoria tritici) Conidia were suspended in Czapek-dox broth and a suspension of 1 × 10⁵ spores were prepared. The prepared spore suspension was seeded at 100 µl/well to a 96-well multiplate. In the test, each compound was dissolved in a dimethyl sulfoxide-methanol mixed solution (volume ratio: 9/1) and dispensed at 50 ppm/well. The absorbance of each well was measured at a wavelength of 600 nm using a photometer. The multiplate was incubated at 25°C for 3 days, followed by absorbance measurement again. The difference in absorbance between the treatment day and 3 days later was calculated, and the rate of inhibition was calculated from the ratio between the treatment group and the non-treatment group.

### <Rate of inhibition>

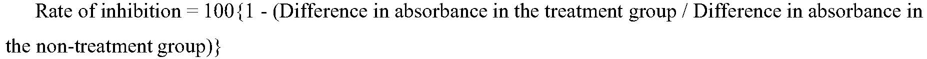

Rate of inhibition = 100{1 - (Difference in absorbance in the treatment group / Difference in absorbance in the non-treatment group)}

The results of the tests described above are summarized in Table 9 below. In the table, H represents a control value or rate of inhibition of larger than 50%, and L represents a control value or rate of inhibition of 50% or less. NT represents not tested.

### Table 8:

**[Table 8-1]**

| Compound | Rice blast disease pot test | Tomato gray mold pot test | Gray mold anti-germ test | Fusarium blight anti-germ test | Anthracnose anti-germ test | Speckled leaf blotch anti-germ test |
|---|---|---|---|---|---|---|
| 1 | H | H | L | L | H | L |
| 2 | L | H | H | L | H | H |
| 3 | L | L | H | H | L | L |
| 4 | H | H | H | L | H | L |
| 5 | H | L | H | H | H | H |
| 6 | H | H | H | H | H | H |
| 7 | H | H | H | H | H | H |
| 8 | H | H | H | H | H | L |
| 9 | H | H | H | H | H | H |
| 10 | L | H | H | H | L | L |
| 11 | L | L | L | L | L | L |
| 12 | L | H | H | H | H | L |
| 13 | L | H | H | H | H | L |
| 14 | H | H | H | H | H | H |
| 15 | L | L | H | L | H | L |
| 16 | L | L | L | L | H | L |
| 17 | L | H | L | H | L | L |
| 18 | L | L | L | L | L | L |
| 19 | L | H | H | H | H | H |
| 20 | H | L | H | H | H | H |
| 21 | H | H | H | L | H | L |
| 22 | H | H | H | H | H | H |
| 23 | H | H | H | H | H | L |
| 24 | H | H | L | L | H | L |
| 25 | L | H | H | L | L | L |
| 26 | L | L | H | H | H | H |
| 27 | H | H | H | H | H | L |
| 28 | L | L | H | H | H | L |
| 29 | H | H | H | H | H | H |

**[Table 8-2]**

| | | | | | | |
|---|---|---|---|---|---|---|
| 30 | L | L | H | L | H | L |
| 31 | H | H | H | H | H | L |
| 32 | H | H | H | L | H | L |
| 33 | L | L | L | L | L | L |
| 34 | H | H | H | H | H | L |
| 35 | H | H | H | H | H | L |
| 36 | L | H | H | L | H | L |
| 37 | H | H | H | L | H | L |
| 38 | H | H | L | L | H | L |
| 39 | H | H | H | H | H | L |
| 40 | L | H | L | L | H | L |
| 41 | L | L | L | L | H | L |
| 42 | L | H | H | L | H | L |
| 43 | H | H | L | L | L | L |
| 44 | H | L | L | L | H | L |
| 45 | H | H | H | L | H | L |
| 46 | H | H | H | L | H | H |
| 47 | H | H | H | H | H | H |
| 48 | L | H | H | L | H | L |
| 49 | L | L | L | L | L | L |
| 50 | L | H | H | L | L | L |
| 51 | H | H | H | H | H | L |
| 52 | L | H | H | L | H | L |
| 53 | H | H | H | H | H | H |
| 54 | H | H | H | L | H | H |
| 55 | H | H | H | H | H | H |
| 56 | H | H | H | H | H | H |
| 57 | H | H | H | H | H | H |
| 58 | H | H | H | H | H | H |
| 59 | H | H | H | H | H | L |
| 60 | L | H | H | H | H | L |

**[Table 8-3]**

| | | | | | | |
|---|---|---|---|---|---|---|
| 61 | H | H | H | H | H | H |
| 62 | H | H | H | H | H | L |
| 63 | H | H | H | L | H | L |
| 64 | H | H | H | H | H | H |
| 65 | H | H | H | L | H | H |
| 66 | H | H | H | H | H | H |
| 67 | H | H | H | L | H | L |
| 68 | L | H | H | L | L | L |
| 69 | H | H | H | L | H | H |
| 70 | H | H | L | L | H | H |
| 71 | H | L | L | H | H | L |
| 72 | H | L | H | L | H | H |
| 73 | H | L | H | L | H | L |
| 74 | N.T. | N.T. | H | H | H | H |
| 75 | H | H | H | H | H | H |
| 76 | H | H | H | H | H | H |
| 77 | H | H | H | H | H | L |
| 78 | H | H | H | H | H | H |
| 79 | H | H | H | H | H | L |
| 80 | H | H | H | H | H | L |
| 81 | H | H | H | H | H | L |
| 82 | H | H | H | L | H | L |
| 83 | H | H | L | L | H | L |
| 84 | H | H | H | H | H | L |
| 85 | L | H | H | H | H | L |
| 86 | L | H | H | H | H | L |
| 87 | L | L | H | H | H | H |
| 88 | H | L | L | L | L | L |
| 89 | H | H | H | H | H | L |
| 90 | H | H | H | H | H | H |
| 91 | H | H | H | L | H | L |

**[Table 8-4]**

| | | | | | | |
|---|---|---|---|---|---|---|
| 92 | H | H | H | H | H | L |
| 93 | H | H | L | H | H | H |
| 94 | L | H | H | H | H | L |
| 95 | H | H | H | H | H | H |
| 96 | H | H | H | H | H | H |
| 97 | H | H | H | H | H | H |
| 98 | H | H | H | H | H | H |
| 99 | H | H | H | L | H | L |
| 100 | H | H | H | H | H | L |
| 101 | H | H | H | H | H | H |
| 102 | H | H | H | H | H | H |
| 103 | H | H | H | L | H | L |
| 104 | L | H | H | L | H | L |
| 105 | L | H | L | L | H | L |
| 106 | H | H | H | L | H | L |
| 107 | H | H | H | H | H | L |
| 108 | H | H | H | H | H | H |
| 109 | H | H | H | H | H | L |
| 110 | H | H | H | H | H | L |
| 111 | L | H | H | H | H | H |
| 112 | L | L | H | L | H | H |
| 113 | H | H | H | H | H | H |
| 114 | H | H | H | H | H | H |
| 115 | H | H | H | L | H | H |
| 116 | H | H | H | L | H | H |
| 117 | H | H | H | L | H | L |
| 118 | H | H | H | H | H | H |
| 119 | H | H | H | H | H | H |
| 120 | H | H | H | H | H | H |
| 121 | H | H | H | L | H | H |
| 122 | H | H | H | H | H | H |

**[Table 8-5]**

| | | | | | | |
|---|---|---|---|---|---|---|
| 123 | H | H | H | L | H | L |
| 124 | H | H | H | L | H | L |
| 125 | H | H | H | H | H | H |
| 126 | H | H | H | H | H | H |
| 127 | H | H | H | H | H | H |
| 128 | H | H | H | H | H | H |
| 129 | H | H | H | H | H | H |
| 130 | H | H | H | H | H | L |
| 131 | H | H | H | H | H | H |
| 132 | H | L | H | L | H | L |
| 133 | H | H | H | H | H | H |
| 134 | H | L | L | L | H | L |
| 135 | H | H | H | H | H | H |
| 136 | H | H | H | H | H | L |
| 137 | H | H | H | L | L | L |
| 138 | H | H | H | H | H | H |
| 139 | H | H | H | H | H | H |
| 140 | H | H | H | L | H | L |
| 141 | H | H | H | L | H | L |
| 142 | H | H | H | H | H | H |
| 143 | H | H | H | H | H | L |
| 144 | L | L | L | L | H | L |
| 145 | H | H | H | H | H | H |
| 146 | H | H | H | H | H | H |
| 147 | H | H | H | H | H | H |
| 148 | H | H | H | H | H | H |
| 149 | H | H | H | H | H | H |
| 150 | H | H | H | H | H | H |
| 151 | H | H | H | H | H | H |
| 152 | H | H | H | H | H | H |
| 153 | H | H | H | H | H | H |

**[Table 8-6]**

| | | | | | | |
|---|---|---|---|---|---|---|
| 154 | H | H | H | H | H | H |
| 155 | H | H | H | H | H | H |
| 156 | H | H | L | H | H | L |
| 157 | H | H | H | H | H | H |
| 158 | H | H | H | H | H | H |
| 159 | H | H | H | H | H | L |
| 160 | H | H | H | H | H | H |
| 161 | H | H | H | H | H | H |
| 162 | H | H | H | H | H | H |
| 163 | H | H | H | H | H | H |
| 164 | H | H | H | H | H | H |
| 165 | H | H | H | H | H | L |
| 166 | L | H | H | L | H | L |
| 167 | H | H | H | H | H | L |
| 168 | H | H | H | H | H | L |
| 169 | H | H | H | H | H | H |
| 170 | H | H | H | H | H | H |
| 171 | H | H | H | H | H | L |
| 172 | H | H | H | H | H | H |
| 173 | H | H | H | H | H | H |
| 174 | H | H | H | H | H | L |
| 175 | H | H | H | H | H | H |
| 176 | H | H | H | H | H | L |
| 177 | H | L | L | L | L | L |
| 178 | H | H | H | H | H | L |
| 179 | H | H | H | H | H | H |
| 180 | L | H | H | H | L | L |
| 181 | H | H | H | H | H | H |
| 182 | L | H | H | H | H | H |
| 183 | H | H | H | H | H | L |
| 184 | H | H | L | L | H | L |

**[Table 8-7]**

| | | | | | | |
|---|---|---|---|---|---|---|
| 185 | H | H | H | H | H | L |
| 186 | H | H | H | H | H | H |
| 187 | H | H | H | H | H | L |
| 188 | L | L | L | L | L | L |
| 189 | H | H | H | H | H | L |
| 190 | L | H | H | H | H | L |
| 191 | H | L | L | L | L | L |
| 192 | L | H | H | L | H | L |
| 193 | H | H | H | H | H | L |
| 194 | H | H | H | H | H | L |
| 195 | H | H | H | H | H | L |
| 196 | L | H | L | H | L | L |
| 197 | H | H | H | H | H | H |
| 198 | H | H | H | H | H | H |
| 199 | L | H | H | H | H | H |
| 200 | H | H | H | H | H | H |
| 201 | H | H | H | H | H | H |
| 202 | L | H | H | H | H | L |
| 203 | H | H | H | H | H | H |
| 204 | H | H | H | H | H | L |
| 205 | H | H | H | H | H | L |
| 206 | H | H | H | H | H | H |
| 207 | H | H | H | H | H | H |
| 208 | H | H | H | H | H | H |
| 209 | H | H | H | H | H | H |
| 210 | H | H | H | H | H | H |
| 211 | H | H | H | H | H | L |
| 212 | H | H | H | H | H | H |
| 213 | H | H | H | H | H | H |
| 214 | H | H | H | H | H | H |
| 215 | H | H | H | H | H | L |

**[Table 8-8]**

| | | | | | | |
|---|---|---|---|---|---|---|
| 216 | L | H | L | L | L | L |
| 217 | H | H | H | H | H | H |
| 218 | H | H | H | H | H | L |
| 219 | H | H | H | H | H | L |
| 220 | H | H | H | H | H | L |
| 221 | H | H | H | H | H | L |
| 222 | L | H | H | H | H | L |
| 223 | H | H | H | H | H | H |
| 224 | H | H | H | H | H | H |
| 225 | H | H | H | H | H | H |
| 226 | H | H | H | H | H | H |
| 227 | L | H | H | H | H | H |
| 228 | L | H | H | H | H | H |
| 229 | H | H | H | H | H | L |
| 230 | H | H | H | H | H | H |
| 231 | H | H | H | H | H | H |
| 232 | L | H | H | H | H | H |
| 233 | H | H | H | H | H | H |
| 234 | H | H | H | H | H | H |
| 235 | H | H | H | H | H | H |
| 236 | L | H | H | H | H | H |
| 237 | H | H | H | H | H | L |
| 238 | H | H | H | H | H | L |
| 239 | H | H | H | H | H | H |
| 240 | H | H | H | H | H | H |
| 241 | H | H | H | H | H | H |
| 242 | L | H | H | L | H | L |
| 243 | H | H | H | H | H | H |
| 244 | H | H | H | H | H | L |
| 245 | H | H | H | H | H | H |
| 246 | L | H | H | H | H | L |

**[Table 8-9]**

| | | | | | | |
|---|---|---|---|---|---|---|
| 247 | H | H | H | H | H | H |
| 248 | H | H | L | L | H | L |
| 249 | H | H | H | H | H | H |
| 250 | H | H | H | H | H | L |
| 251 | H | H | H | H | H | H |
| 252 | H | H | H | H | H | H |
| 253 | H | H | H | H | H | H |
| 254 | H | H | H | H | H | H |
| 255 | H | H | H | H | H | H |
| 256 | H | H | H | H | H | L |
| 257 | H | H | H | H | H | H |
| 258 | H | H | H | H | H | L |
| 259 | H | H | H | H | L | H |
| 260 | H | H | H | L | H | H |
| 261 | H | H | H | H | H | H |
| 262 | H | H | H | L | H | H |
| 263 | H | H | H | H | H | H |
| 264 | H | H | H | H | H | H |
| 265 | H | H | H | H | H | H |
| 266 | H | H | H | H | H | H |
| 267 | H | H | H | H | H | H |
| 268 | H | H | H | H | H | H |
| 269 | H | H | H | H | H | H |
| 270 | H | H | H | H | H | H |
| 271 | H | H | H | H | H | L |
| 272 | H | H | H | H | H | H |
| 273 | H | L | L | L | L | L |
| 274 | H | H | H | H | H | L |
| 275 | H | H | H | H | L | H |
| 276 | H | H | H | H | H | L |
| 277 | H | H | H | H | H | H |

**[Table 8-10]**

| | | | | | | |
|---|---|---|---|---|---|---|
| 278 | H | H | H | H | H | H |
| 279 | H | H | H | H | H | H |
| 280 | H | H | H | H | H | H |
| 281 | H | H | H | H | H | H |
| 282 | H | H | H | H | H | L |
| 283 | H | L | L | L | H | L |
| 284 | H | H | H | H | H | H |
| 285 | H | L | L | L | H | L |
| 286 | H | H | H | H | H | H |
| 287 | H | H | H | H | H | H |
| 288 | H | H | H | H | H | L |
| 289 | H | L | L | H | H | L |
| 290 | H | H | H | H | H | H |
| 291 | H | L | H | H | H | H |
| 292 | H | H | H | H | H | H |
| 293 | H | H | H | H | H | H |
| 294 | H | H | H | H | H | H |
| 295 | H | H | H | H | H | H |
| 296 | H | H | H | H | H | H |
| 297 | H | H | H | H | H | L |
| 298 | H | H | H | H | H | H |
| 299 | H | H | H | H | H | H |
| 300 | H | H | H | H | H | L |
| 301 | H | H | H | H | H | L |
| 302 | H | H | H | H | H | H |
| 303 | H | H | H | H | H | H |
| 304 | H | H | H | H | H | H |
| 305 | H | H | H | L | L | H |
| 306 | H | H | H | H | H | L |
| 307 | H | H | H | H | H | H |
| 308 | H | H | H | H | H | H |

**[Table 8-11]**

| | | | | | | |
|---|---|---|---|---|---|---|
| 309 | H | H | H | H | H | H |
| 310 | H | H | H | H | H | L |
| 311 | H | H | H | H | H | H |
| 312 | H | H | H | L | H | H |
| 313 | H | H | H | H | H | H |
| 314 | H | H | H | H | H | H |
| 315 | H | H | H | H | H | H |
| 316 | H | H | H | H | H | H |
| 317 | H | H | H | H | H | L |
| 318 | H | H | H | H | H | H |
| 319 | H | H | H | H | H | H |
| 320 | H | H | H | H | H | H |
| 321 | H | H | H | H | H | H |
| 322 | H | H | H | H | H | H |
| 323 | H | H | H | H | H | H |
| 324 | H | H | H | H | H | H |
| 325 | H | H | H | H | H | H |
| 326 | H | H | H | H | H | H |
| 327 | L | H | H | H | H | H |
| 328 | H | H | H | H | H | H |
| 329 | L | L | L | L | L | L |
| 330 | H | H | H | H | H | H |
| 331 | H | H | H | H | H | L |
| 332 | H | H | H | H | H | H |
| 333 | H | L | L | L | H | L |
| 334 | H | H | H | H | H | H |
| 335 | H | H | H | H | H | H |
| 336 | H | H | H | H | H | H |
| 337 | H | H | H | H | H | H |
| 338 | H | H | H | H | H | H |
| 339 | H | H | H | H | H | H |

**[Table 8-12]**

| | | | | | | |
|---|---|---|---|---|---|---|
| 340 | H | H | H | H | H | L |
| 341 | H | H | H | H | H | L |
| 342 | H | H | H | H | H | L |
| 343 | H | H | H | H | H | L |
| 344 | H | H | H | H | H | L |
| 345 | H | H | H | H | H | L |
| 346 | H | H | H | H | H | H |
| 347 | H | H | H | H | H | H |
| 348 | H | L | H | H | H | H |
| 349 | H | H | H | H | H | H |
| 350 | H | L | H | L | H | H |
| 351 | H | H | H | H | H | H |
| 352 | H | H | H | H | H | H |
| 353 | H | H | H | H | H | H |
| 354 | H | H | H | H | H | H |
| 355 | H | H | H | H | H | H |
| 356 | H | H | H | H | H | L |
| 357 | H | H | H | H | H | H |
| 358 | H | H | H | H | H | L |
| 359 | H | H | H | L | H | H |
| 360 | H | H | H | H | H | H |
| 361 | H | H | H | H | H | H |
| 362 | H | H | H | H | H | L |
| 363 | H | H | H | H | H | H |
| 364 | H | H | H | H | H | H |
| 365 | H | H | H | H | H | H |
| 366 | H | H | H | H | H | H |
| 367 | H | H | H | H | H | H |
| 368 | H | H | H | H | H | H |
| 369 | H | H | H | H | H | H |
| 370 | L | L | L | L | L | H |

**[Table 8-13]**

| | | | | | | |
|---|---|---|---|---|---|---|
| 371 | H | H | H | H | H | H |
| 372 | L | L | L | L | L | H |
| 373 | H | H | H | H | H | L |
| 374 | H | H | H | L | H | L |
| 375 | H | H | H | H | H | H |
| 376 | H | H | H | H | H | H |
| 377 | H | H | H | L | H | H |
| 378 | H | H | H | H | H | H |
| 379 | H | H | H | H | H | L |
| 380 | H | H | H | H | H | H |
| 381 | H | H | H | H | H | L |
| 382 | H | H | H | H | H | H |
| 383 | H | H | H | H | H | L |
| 384 | H | H | H | H | H | L |
| 385 | H | H | H | H | H | L |
| 386 | H | H | H | H | H | H |
| 387 | H | H | H | H | H | H |
| 388 | H | H | H | H | H | L |
| 389 | H | H | H | H | H | L |
| 390 | H | H | H | H | H | H |
| 391 | H | H | H | H | H | H |
| 392 | H | H | H | H | H | H |
| 393 | H | H | H | H | H | H |
| 394 | H | H | H | H | H | H |
| 395 | H | H | H | H | H | L |
| 396 | H | H | H | H | H | L |
| 397 | H | H | L | H | H | L |
| 398 | H | H | H | H | H | H |
| 399 | H | H | H | H | H | L |
| 400 | H | H | H | H | H | H |
| 401 | H | H | L | L | H | L |

**[Table 8-14]**

| | | | | | | |
|---|---|---|---|---|---|---|
| 402 | H | H | H | H | H | L |
| 403 | H | H | H | H | H | H |
| 404 | H | H | H | H | H | H |
| 405 | H | H | H | H | H | H |
| 406 | H | H | H | H | H | L |
| 407 | H | H | H | H | H | H |
| 408 | H | H | H | H | H | L |
| 409 | H | H | H | H | H | H |
| 410 | H | H | H | H | H | L |
| 411 | H | H | H | H | H | H |
| 412 | H | H | H | H | H | L |
| 413 | H | H | H | H | H | L |
| 414 | H | H | H | H | H | L |
| 415 | H | H | H | H | H | L |
| 416 | H | H | H | H | H | H |
| 417 | H | H | H | H | H | H |
| 418 | H | H | H | H | H | L |
| 419 | H | H | H | H | H | H |
| 420 | H | H | H | H | H | L |
| 421 | H | H | H | H | H | H |
| 422 | H | H | H | H | H | L |
| 423 | H | H | H | H | H | L |
| 424 | H | H | H | H | H | L |
| 425 | H | H | H | L | L | L |
| 426 | H | H | L | L | L | H |
| 427 | H | H | H | H | H | H |
| 428 | H | H | H | H | H | H |
| 429 | H | H | H | H | H | H |
| 430 | H | H | H | H | H | H |
| 431 | H | H | H | H | H | H |
| 432 | H | H | H | H | H | H |

**[Table 8-15]**

| | | | | | | |
|---|---|---|---|---|---|---|
| 433 | H | H | H | H | H | H |
| 434 | H | H | H | H | H | H |
| 435 | H | H | H | L | H | H |
| 436 | H | H | H | H | H | L |
| 437 | H | H | H | H | H | H |
| 438 | H | H | H | H | H | H |
| 439 | H | H | H | H | H | H |
| 440 | H | H | H | H | H | H |
| 441 | H | H | H | H | H | H |
| 442 | H | H | H | H | H | H |
| 443 | H | H | H | H | H | H |
| 444 | H | H | H | H | H | H |
| 445 | H | H | H | H | H | H |
| 446 | H | H | H | H | H | H |
| 447 | H | H | H | H | H | H |
| 448 | H | H | H | H | H | H |
| 449 | H | H | H | H | H | H |
| 450 | H | H | H | H | H | H |
| 451 | H | H | H | H | H | H |
| 452 | H | H | H | H | H | H |
| 453 | H | H | H | H | H | H |
| 454 | H | H | H | H | H | H |
| 455 | H | H | H | H | H | H |
| 456 | H | H | H | L | H | H |
| 457 | H | H | H | H | H | H |
| 458 | H | H | H | L | H | H |
| 459 | H | H | H | H | H | H |
| 460 | H | H | H | L | H | H |
| 461 | H | L | L | L | L | H |
| 462 | H | H | H | H | H | H |
| 463 | H | H | H | H | H | H |

**[Table 8-16]**

| | | | | | | |
|---|---|---|---|---|---|---|
| 464 | H | H | H | H | H | H |
| 465 | H | H | H | H | H | H |
| 466 | H | H | H | H | H | H |
| 467 | H | H | H | H | H | H |
| 468 | H | H | H | H | H | H |
| 469 | H | H | H | H | H | H |
| 470 | H | H | H | H | H | H |
| 471 | H | H | H | H | H | H |
| 472 | H | H | H | H | H | H |
| 473 | H | H | H | H | H | H |
| 474 | H | H | L | H | H | L |
| 475 | H | H | L | H | H | H |
| 476 | H | H | H | H | H | H |
| 477 | H | H | H | H | H | H |
| 478 | H | H | H | H | H | H |
| 479 | H | H | H | H | H | H |
| 480 | H | H | H | H | H | H |
| 481 | H | H | H | H | H | H |
| 482 | H | H | H | H | H | H |
| 483 | H | H | H | H | H | H |
| 484 | H | H | H | H | H | L |
| 485 | H | H | H | H | H | H |
| 486 | H | H | H | H | H | H |
| 487 | H | H | H | H | H | H |
| 488 | H | H | H | H | H | H |
| 489 | H | H | H | H | H | H |
| 490 | H | H | H | H | H | H |
| 491 | H | H | L | H | H | L |
| 492 | H | H | H | H | H | H |
| 493 | H | H | H | H | H | L |
| 494 | H | H | H | H | H | H |

**[Table 8-17]**

| | | | | | | |
|---|---|---|---|---|---|---|
| 495 | H | H | H | L | H | H |
| 496 | H | H | H | H | H | H |
| 497 | H | H | H | H | H | H |
| 498 | H | H | H | H | H | H |
| 499 | H | H | H | H | H | H |
| 500 | H | H | L | H | H | H |
| 501 | H | H | H | H | H | H |
| 502 | H | H | H | H | H | H |
| 503 | H | H | H | H | H | H |
| 504 | H | H | H | H | H | H |
| 505 | H | H | H | H | H | H |
| 506 | H | H | H | H | H | H |
| 507 | H | H | H | H | H | H |
| 508 | H | H | H | H | H | H |
| 509 | H | H | H | H | H | H |
| 510 | H | H | H | H | H | H |
| 511 | H | H | H | H | H | H |
| 512 | H | H | H | H | H | H |
| 513 | H | H | H | L | H | H |
| 514 | H | H | H | H | H | H |
| 515 | H | H | H | H | H | H |
| 516 | H | H | H | H | H | H |
| 517 | H | H | H | H | H | H |
| 518 | H | H | H | H | H | H |
| 519 | H | H | H | H | H | H |
| 520 | H | H | H | H | H | H |
| 521 | H | H | H | H | H | H |
| 522 | H | H | H | H | H | H |
| 523 | H | H | L | L | H | L |
| 524 | H | H | H | H | H | H |
| 525 | H | H | H | H | H | H |

**[Table 8-18]**

| | | | | | | |
|---|---|---|---|---|---|---|
| 526 | H | H | H | H | H | H |
| 527 | H | H | H | H | H | H |
| 528 | H | H | H | H | H | H |
| 529 | H | H | H | H | H | H |
| 530 | H | H | H | H | H | H |
| 531 | H | H | H | H | H | H |
| 532 | H | H | H | H | H | H |
| 533 | H | H | H | H | H | H |
| 534 | H | H | H | H | H | H |
| 535 | H | H | H | H | H | H |
| 536 | H | H | H | H | H | H |
| 537 | H | H | H | L | H | H |
| 538 | H | H | H | H | H | H |
| 539 | H | H | H | H | H | H |
| 540 | H | H | H | H | H | H |
| 541 | H | H | H | H | H | H |
| 542 | H | H | H | H | H | L |
| 543 | H | H | H | H | H | H |
| 544 | H | H | H | H | H | H |
| 545 | H | H | H | H | H | H |
| 546 | H | H | H | H | H | H |
| 547 | H | H | H | H | H | H |
| 548 | H | H | H | H | H | H |
| 549 | H | H | H | H | H | H |
| 550 | H | H | H | H | H | H |
| 551 | H | H | L | L | H | L |
| 552 | H | H | L | L | L | H |
| 553 | H | H | H | H | H | H |
| 554 | H | H | H | H | H | H |
| 555 | H | H | H | H | H | H |
| 556 | H | H | H | H | H | H |

**[Table 8-19]**

| | | | | | | |
|---|---|---|---|---|---|---|
| 557 | H | H | H | H | H | H |
| 558 | H | H | L | H | H | H |
| 559 | H | H | H | H | H | H |
| 560 | H | H | H | H | H | H |
| 561 | H | H | H | L | H | H |
| 562 | H | H | L | H | H | H |
| 563 | H | H | H | H | H | H |
| 564 | H | H | H | H | H | H |
| 565 | H | H | L | H | H | H |
| 566 | H | H | H | H | H | H |
| 567 | H | H | H | H | H | H |
| 568 | H | H | H | H | H | H |
| 569 | H | H | H | H | H | H |
| 570 | H | H | H | H | H | H |
| 571 | H | H | H | H | H | H |
| 572 | H | H | H | H | H | H |
| 573 | H | H | H | H | H | H |
| 574 | H | H | H | H | H | H |
| 575 | H | H | H | H | H | H |
| 576 | H | H | H | H | H | H |
| 577 | H | H | H | H | H | L |
| 578 | H | H | H | H | H | L |
| 579 | H | H | H | H | H | L |
| 580 | H | H | H | H | L | H |
| 581 | H | H | H | H | H | L |
| 582 | H | H | H | H | H | H |
| 583 | H | H | H | H | H | H |
| 584 | H | H | H | H | H | H |
| 585 | H | H | H | H | H | H |
| 586 | H | H | H | L | H | H |
| 587 | H | H | H | L | H | H |

**[Table 8-20]**

| | | | | | | |
|---|---|---|---|---|---|---|
| 588 | H | H | L | L | L | H |
| 589 | H | H | H | H | H | H |
| 590 | H | H | H | H | H | H |
| 591 | H | H | L | L | L | L |
| 592 | H | H | L | L | L | H |
| 593 | H | H | H | H | H | H |
| 594 | H | H | H | H | H | H |
| 595 | H | H | H | L | H | L |
| 596 | H | H | H | H | H | H |
| 597 | H | H | H | H | H | H |
| 598 | H | H | H | H | H | H |
| 599 | H | H | H | H | H | H |
| 600 | H | H | H | H | H | H |
| 601 | H | H | H | H | H | H |
| 602 | H | H | H | H | H | H |
| 603 | H | H | H | H | H | H |
| 604 | H | H | H | H | H | H |
| 605 | H | H | H | H | H | H |
| 606 | H | H | H | H | H | H |
| 607 | H | H | H | H | H | H |
| 608 | H | H | H | H | H | H |
| 609 | H | H | H | H | H | H |
| 610 | H | H | H | H | H | H |
| 611 | H | H | H | H | H | H |
| 612 | H | H | H | H | H | H |
| 613 | H | H | H | H | H | H |
| 614 | H | H | H | H | H | H |
| 615 | H | H | H | H | H | L |
| 616 | H | H | H | H | H | H |
| 617 | H | H | H | H | H | L |
| 618 | H | H | H | H | H | L |

**[Table 8-21]**

| | | | | | | |
|---|---|---|---|---|---|---|
| 619 | H | H | H | H | H | H |
| 620 | H | H | H | H | H | H |
| 621 | H | H | H | H | H | H |
| 622 | H | H | H | H | H | H |
| 623 | H | H | H | H | H | H |
| 624 | H | H | H | H | H | H |
| 625 | H | H | H | H | H | H |
| 626 | H | H | H | H | H | H |
| 627 | H | H | H | H | H | H |
| 628 | H | H | H | H | H | L |
| 629 | H | H | H | H | H | H |
| 630 | H | H | H | H | H | H |
| 631 | H | H | H | H | H | H |
| 632 | H | H | H | H | H | H |
| 633 | H | H | H | H | H | H |
| 634 | H | H | H | H | H | H |
| 635 | H | H | L | H | H | L |
| 636 | H | H | H | H | H | H |
| 637 | H | H | H | H | H | H |
| 638 | H | H | H | H | H | H |
| 639 | H | H | H | H | H | H |
| 640 | H | H | H | H | H | H |
| 641 | H | H | H | H | L | L |
| 642 | H | H | H | H | L | H |
| 643 | H | H | H | H | H | H |
| 644 | H | H | H | H | H | H |
| 645 | H | H | L | H | H | H |
| 646 | H | H | H | H | H | H |
| 647 | H | H | H | H | H | H |
| 648 | H | H | H | H | H | H |
| 649 | H | H | H | H | H | H |

**[Table 8-22]**

| | | | | | | |
|---|---|---|---|---|---|---|
| 650 | H | H | L | L | H | L |
| 651 | H | H | H | H | H | H |
| 652 | H | H | H | H | H | H |
| 653 | H | H | H | H | H | H |
| 654 | H | H | H | H | H | H |
| 655 | H | H | H | H | H | H |
| 656 | H | H | H | H | H | H |
| 657 | H | H | H | H | H | H |
| 658 | H | H | H | H | H | L |
| 659 | H | H | H | H | H | H |
| 660 | H | H | H | H | H | H |
| 661 | H | H | H | H | H | H |
| 662 | H | H | H | H | H | H |
| 663 | H | H | H | H | H | H |
| 664 | H | H | H | H | H | H |
| 665 | H | H | H | H | H | H |
| 666 | H | H | H | H | H | H |
| 667 | H | H | H | H | H | H |
| 668 | H | H | H | H | H | H |
| 669 | H | H | H | L | H | H |
| 670 | H | H | H | H | H | H |
| 671 | H | H | H | H | H | H |
| 672 | H | H | H | H | H | H |
| 673 | H | H | H | H | H | H |
| 674 | H | H | H | H | H | H |
| 675 | H | H | H | H | H | H |
| 676 | H | H | H | H | H | H |
| 677 | H | H | H | H | H | H |
| 678 | H | H | H | H | H | H |
| 679 | H | H | H | H | H | H |
| 680 | H | H | H | H | H | H |

**[Table 8-23]**

| | | | | | | |
|---|---|---|---|---|---|---|
| 681 | H | H | H | H | H | H |
| 682 | H | H | H | H | H | H |
| 683 | H | H | H | H | H | H |
| 684 | H | H | H | H | H | H |
| 685 | H | H | H | H | H | H |
| 686 | H | H | H | H | H | H |
| 687 | H | H | H | H | H | H |
| 688 | H | H | H | H | H | H |
| 689 | H | H | H | H | H | H |
| 690 | H | H | H | H | H | H |
| 691 | H | H | H | H | H | H |
| 692 | H | H | H | H | H | L |
| 693 | H | H | H | H | H | H |
| 694 | H | H | H | H | H | H |
| 695 | H | H | H | H | H | H |
| 696 | H | H | H | H | H | H |
| 697 | H | H | H | H | H | H |
| 698 | H | H | H | H | H | H |
| 699 | H | H | H | H | H | H |
| 700 | H | L | L | L | H | L |
| 701 | H | H | H | H | H | H |
| 702 | H | H | L | L | H | H |
| 703 | H | H | H | H | H | H |
| 704 | H | H | H | H | H | H |
| 705 | H | H | H | H | H | H |
| 706 | H | H | H | H | H | H |
| 707 | H | H | H | H | H | H |
| 708 | H | H | H | H | H | H |
| 709 | H | H | H | H | H | H |
| 710 | H | H | H | H | H | H |
| 711 | H | H | H | H | H | H |

**[Table 8-24]**

| | | | | | | |
|---|---|---|---|---|---|---|
| 712 | H | H | H | H | H | H |
| 713 | H | H | H | H | H | H |
| 714 | H | H | H | H | H | H |
| 715 | H | H | H | H | H | H |
| 716 | H | H | H | H | H | H |
| 717 | H | H | H | H | H | H |
| 718 | H | H | H | H | L | H |
| 719 | H | H | H | H | H | H |
| 720 | H | H | H | H | H | H |
| 721 | H | H | H | H | H | H |
| 722 | H | H | H | H | H | L |
| 723 | H | H | H | H | H | H |
| 724 | H | H | H | H | H | H |
| 725 | H | H | H | H | H | H |
| 726 | H | H | H | H | H | H |
| 727 | H | H | H | H | H | H |
| 728 | H | H | H | H | H | H |
| 729 | H | H | H | H | H | H |
| 730 | H | H | H | H | H | H |
| 731 | H | H | H | H | H | H |
| 732 | H | H | H | H | H | H |
| 733 | H | H | H | H | H | H |
| 734 | H | H | H | H | H | H |
| 735 | H | H | H | H | H | H |
| 736 | H | H | H | H | H | H |
| 737 | H | H | H | H | H | H |
| 738 | H | H | H | H | H | H |
| 739 | H | H | H | H | L | H |
| 740 | H | H | H | H | H | H |
| 741 | H | H | H | H | H | H |
| 742 | H | H | H | H | L | H |

**[Table 8-25]**

| | | | | | | |
|---|---|---|---|---|---|---|
| 743 | L | L | H | H | H | H |
| 744 | H | H | H | H | H | H |
| 745 | H | H | H | H | H | H |
| 746 | H | H | H | H | H | H |
| 747 | H | H | H | H | H | H |
| 748 | H | H | H | H | H | H |
| 749 | H | H | H | H | H | H |
| 750 | H | H | H | H | H | H |
| 751 | H | H | H | H | H | H |
| 752 | H | H | H | H | H | H |
| 753 | H | H | H | H | H | H |
| 754 | H | H | H | H | H | H |
| 755 | H | H | H | H | H | H |
| 756 | H | H | H | H | H | H |
| 757 | H | H | H | H | H | H |
| 758 | H | H | H | H | H | H |
| 759 | H | H | H | H | H | H |
| 760 | H | H | H | H | H | H |
| 761 | H | H | H | H | H | H |
| 762 | H | H | H | H | H | H |
| 763 | H | H | H | H | H | H |
| 764 | H | H | H | H | H | H |
| 765 | H | H | H | H | H | H |
| 766 | H | H | H | H | H | H |
| 767 | H | H | H | H | H | H |
| 768 | H | H | H | H | H | H |
| 769 | H | H | H | H | H | H |
| 770 | H | H | H | H | H | H |
| 771 | H | H | H | H | H | H |
| 772 | H | H | H | H | H | H |
| 773 | H | H | H | H | H | L |

**[Table 8-26]**

| | | | | | | |
|---|---|---|---|---|---|---|
| 774 | H | H | H | H | H | H |
| 775 | H | H | H | H | H | H |
| 776 | H | H | H | H | H | H |
| 777 | H | H | H | H | H | H |
| 778 | L | H | H | H | H | H |
| 779 | H | H | H | H | H | H |
| 780 | H | H | H | H | H | H |
| 781 | L | H | H | H | L | H |
| 782 | H | H | H | H | H | H |
| 783 | H | H | H | H | L | H |
| 784 | L | H | H | H | H | H |
| 785 | L | H | H | L | L | H |
| 786 | L | H | H | H | L | H |
| 787 | L | H | H | H | H | H |
| 788 | H | H | H | H | H | H |
| 789 | H | H | H | H | H | H |
| 790 | H | H | H | H | H | H |
| 791 | H | H | H | H | H | H |
| 792 | H | H | H | H | H | H |
| 793 | H | H | H | H | H | H |
| 794 | H | H | H | H | H | H |
| 795 | H | H | H | H | H | H |
| 796 | H | H | H | H | H | H |
| 797 | H | H | H | H | H | H |
| 798 | H | H | H | H | H | H |
| 799 | H | H | H | H | H | H |
| 800 | H | H | H | H | H | H |
| 801 | H | L | H | L | H | L |
| 802 | H | H | H | H | H | H |
| 803 | H | H | H | L | H | H |
| 804 | H | H | H | L | H | H |

**[Table 8-27]**

| | | | | | | |
|---|---|---|---|---|---|---|
| 805 | L | H | H | H | H | H |
| 806 | H | H | H | H | H | H |
| 807 | H | H | H | H | H | H |
| 808 | H | H | H | L | H | H |
| 809 | H | H | H | H | H | H |
| 810 | H | H | H | H | H | H |
| 811 | H | H | H | H | H | H |
| 812 | H | H | H | H | H | H |
| 813 | H | H | H | H | H | H |
| 814 | H | H | H | H | H | H |
| 815 | H | H | H | H | H | H |
| 816 | H | H | H | H | H | H |
| 817 | H | H | H | H | H | H |
| 818 | H | H | H | H | H | H |
| 819 | H | H | H | H | H | H |
| 820 | H | H | H | H | H | H |
| 821 | H | H | H | H | H | H |
| 822 | L | L | L | L | L | L |
| 823 | H | L | L | L | L | L |
| 824 | L | L | L | L | L | L |
| 825 | H | H | H | H | H | H |
| 826 | H | H | H | H | H | H |
| 827 | H | H | H | H | H | H |
| 828 | H | H | H | H | H | H |
| 829 | H | H | H | H | H | H |
| 830 | H | H | H | H | H | H |
| 831 | H | H | H | H | H | H |
| 832 | H | H | H | H | H | H |
| 833 | H | H | H | H | H | H |
| 834 | H | H | H | H | H | H |
| 835 | H | H | H | H | H | H |

**[Table 8-28]**

| | | | | | | |
|---|---|---|---|---|---|---|
| 836 | H | H | H | H | H | H |
| 837 | H | H | H | H | H | H |
| 838 | H | H | H | H | H | H |
| 839 | H | H | H | H | H | H |
| 840 | H | H | H | H | H | H |
| 841 | H | H | H | H | H | H |
| 842 | H | H | H | H | H | H |
| 843 | H | H | H | H | H | H |
| 844 | H | H | H | H | H | H |
| 845 | H | H | H | H | H | H |
| 846 | H | H | H | H | H | H |
| 847 | H | H | H | H | H | H |
| 848 | H | H | H | H | H | H |
| 849 | H | H | H | H | H | H |
| 850 | H | H | H | H | H | H |
| 851 | H | H | H | H | H | H |
| 852 | H | H | H | H | H | H |
| 853 | H | H | H | H | H | H |
| 854 | H | H | H | H | H | H |
| 855 | H | H | H | H | H | H |
| 856 | H | H | H | H | H | H |
| 857 | H | H | H | H | H | H |
| 858 | H | H | H | H | H | H |
| 859 | H | H | H | H | H | H |
| 860 | H | H | H | H | H | H |
| 861 | H | H | H | H | H | H |
| 862 | H | H | H | H | H | H |
| 863 | H | H | H | H | H | H |
| 864 | H | H | H | H | H | H |
| 865 | H | H | H | H | H | H |
| 866 | H | H | H | H | H | H |

**[Table 8-29]**

| | | | | | | |
|---|---|---|---|---|---|---|
| 867 | H | H | H | H | H | L |
| 868 | H | H | H | H | H | H |
| 869 | H | H | H | H | H | H |
| 870 | H | H | H | H | H | H |
| 871 | H | H | H | H | H | H |
| 872 | H | H | H | H | H | H |
| 873 | H | H | H | H | H | H |
| 874 | H | H | H | H | H | H |
| 875 | H | H | H | H | H | H |
| 876 | H | H | H | H | H | H |
| 877 | H | H | H | H | H | H |
| 878 | H | H | H | H | H | H |
| 879 | H | H | H | H | H | H |
| 880 | H | H | H | H | H | H |
| 881 | H | H | H | H | H | H |
| 882 | H | H | H | H | H | H |
| 883 | H | H | H | H | H | H |
| 884 | H | H | H | H | H | H |
| 885 | H | H | H | H | H | H |
| 886 | H | H | H | H | H | H |
| 887 | H | H | H | H | H | H |
| 888 | H | H | H | H | H | H |
| 889 | H | H | H | H | H | H |
| 890 | H | H | H | H | H | H |
| 891 | H | H | H | H | H | H |
| 892 | H | H | H | H | H | H |
| 893 | H | H | H | L | H | H |
| 894 | H | H | H | H | H | H |
| 895 | H | H | H | H | H | H |
| 896 | H | H | H | H | H | H |
| 897 | H | H | H | H | H | H |

**[Table 8-30]**

| | | | | | | |
|---|---|---|---|---|---|---|
| 898 | H | H | H | H | H | H |
| 899 | H | H | H | H | H | H |
| 900 | H | H | H | H | H | H |
| 901 | H | H | H | H | H | H |
| 902 | H | H | H | H | H | H |
| 903 | H | H | H | H | H | H |
| 904 | H | H | H | H | H | H |
| 905 | H | H | H | H | H | L |
| 906 | H | H | H | H | H | H |
| 907 | H | H | H | H | H | H |
| 908 | H | H | H | H | H | H |
| 909 | H | H | H | H | H | H |
| 910 | H | H | H | H | H | H |
| 911 | H | H | H | H | H | H |
| 912 | H | H | H | H | H | H |
| 913 | H | H | H | H | H | H |
| 914 | H | H | H | H | H | H |
| 915 | H | H | H | H | H | H |
| 916 | H | H | H | H | H | H |
| 917 | H | H | H | H | H | H |
| 918 | H | H | H | H | H | H |
| 919 | H | H | H | H | H | H |
| 920 | H | H | H | H | H | H |
| 921 | H | H | H | H | H | H |
| 922 | H | H | H | H | H | H |
| 923 | H | H | L | H | H | L |
| 924 | H | H | H | H | H | H |
| 925 | H | H | H | H | H | H |
| 926 | H | H | H | H | H | H |
| 927 | H | H | H | H | H | L |
| 928 | H | H | H | H | H | H |

**[Table 8-31]**

| | | | | | | |
|---|---|---|---|---|---|---|
| 929 | H | H | H | L | H | H |
| 930 | H | H | H | H | H | H |
| 931 | H | H | H | H | H | H |
| 932 | H | H | H | H | H | H |
| 933 | H | H | H | H | H | H |
| 934 | H | H | H | H | H | H |
| 935 | H | H | H | H | H | H |
| 936 | H | H | H | H | H | H |
| 937 | H | H | H | H | H | H |
| 938 | H | H | H | H | H | H |
| 939 | H | H | H | H | H | H |
| 940 | H | H | H | H | H | H |
| 941 | H | H | H | H | H | H |
| 942 | H | H | H | H | H | H |
| 943 | H | H | H | H | H | H |
| 944 | H | H | H | H | H | H |
| 945 | H | H | L | H | H | L |
| 946 | H | H | H | H | H | L |
| 947 | H | H | H | H | H | H |
| 948 | L | H | L | H | H | H |
| 949 | H | H | L | H | H | H |
| 950 | H | H | L | H | H | L |
| 951 | H | H | H | H | H | H |
| 952 | H | H | H | H | H | H |
| 953 | H | H | H | H | H | H |
| 954 | H | H | H | H | H | H |
| 955 | H | H | H | H | H | H |
| 956 | H | H | H | H | H | H |
| 957 | L | H | H | H | H | H |
| 958 | H | H | H | H | H | H |
| 959 | H | H | H | H | H | H |

**[Table 8-32]**

| | | | | | | |
|---|---|---|---|---|---|---|
| 960 | H | H | H | H | H | H |
| 961 | H | H | H | H | H | H |
| 962 | H | H | H | H | H | H |
| 963 | H | H | H | H | H | H |
| 964 | H | H | H | H | H | H |
| 965 | H | H | H | H | H | H |
| 966 | H | H | H | H | H | H |
| 967 | H | H | H | H | H | H |
| 968 | H | H | H | H | H | H |
| 969 | H | L | L | L | H | L |
| 970 | H | H | L | L | L | H |
| 971 | H | H | H | H | H | H |
| 972 | H | H | H | H | H | L |
| 973 | H | H | H | H | H | L |
| 974 | H | H | H | H | H | H |
| 975 | H | H | H | H | H | H |
| 976 | H | H | H | H | H | H |
| 977 | H | H | H | H | H | H |
| 978 | H | H | H | H | H | H |
| 979 | H | H | H | H | H | H |
| 980 | H | H | H | L | H | H |
| 981 | H | H | H | H | H | H |
| 982 | H | H | H | H | H | H |
| 983 | H | H | H | H | H | H |
| 984 | H | H | H | H | H | L |
| 985 | H | H | H | H | H | H |
| 986 | H | H | H | H | H | H |
| 987 | H | H | H | H | H | H |
| 988 | H | H | H | H | H | L |
| 989 | H | H | H | H | H | H |
| 990 | H | H | H | H | H | L |

**[Table 8-33]**

| | | | | | | |
|---|---|---|---|---|---|---|
| 991 | H | H | H | H | H | H |
| 992 | H | H | H | H | H | H |
| 993 | H | H | H | H | H | H |
| 994 | H | H | H | H | H | L |
| 995 | H | H | H | H | H | H |
| 996 | H | H | H | H | H | H |
| 997 | N.T. | N.T. | H | H | H | H |
| 998 | N.T. | N.T. | H | H | H | H |
| 999 | N.T. | N.T. | L | H | H | H |
| 1000 | N.T. | N.T. | H | H | H | H |
| 1001 | N.T. | N.T. | H | H | H | H |
| 1002 | N.T. | N.T. | H | H | H | H |
| 1003 | N.T. | N.T. | H | H | H | H |
| 1004 | N.T. | N.T. | H | H | H | H |
| 1005 | N.T. | N.T. | H | H | H | H |
| 1006 | N.T. | N.T. | H | H | H | H |
| 1007 | N.T. | N.T. | H | H | H | H |
| 1008 | N.T. | N.T. | H | H | H | H |
| 1009 | N.T. | N.T. | H | H | H | H |
| 1010 | N.T. | N.T. | H | H | H | H |
| 1011 | N.T. | N.T. | H | H | H | L |
| 1012 | N.T. | N.T. | H | H | H | H |
| 1013 | N.T. | N.T. | H | H | H | H |
| 1014 | N.T. | N.T. | H | L | H | H |
| 1015 | N.T. | N.T. | H | H | H | H |
| 1016 | N.T. | N.T. | H | H | H | H |
| 1017 | N.T. | N.T. | H | H | H | H |
| 1018 | N.T. | N.T. | H | H | H | H |
| 1019 | N.T. | N.T. | H | H | H | H |
| 1020 | N.T. | N.T. | H | H | H | H |
| 1021 | N.T. | N.T. | H | H | H | H |

**[Table 8-34]**

| | | | | | | |
|---|---|---|---|---|---|---|
| 1022 | N.T. | N.T. | H | H | H | H |
| 1023 | N.T. | N.T. | L | L | H | H |
| 1024 | N.T. | N.T. | H | H | H | H |
| 1025 | N.T. | N.T. | L | L | H | H |
| 1026 | N.T. | N.T. | H | H | H | H |
| 1027 | N.T. | N.T. | H | H | H | H |
| 1028 | N.T. | N.T. | H | H | H | H |
| 1029 | N.T. | N.T. | H | H | H | H |
| 1030 | N.T. | N.T. | H | H | H | H |
| 1031 | N.T. | N.T. | H | H | H | H |
| 1032 | N.T. | N.T. | H | H | H | H |
| 1033 | N.T. | N.T. | H | H | H | H |
| 1034 | N.T. | N.T. | H | H | H | H |
| 1035 | N.T. | N.T. | H | H | H | H |
| 1036 | N.T. | N.T. | H | H | H | H |
| 1037 | N.T. | N.T. | H | H | H | H |
| 1038 | N.T. | N.T. | H | H | H | H |
| 1039 | N.T. | N.T. | H | H | H | L |
| 1040 | N.T. | N.T. | H | H | H | H |
| 1041 | N.T. | N.T. | H | H | H | H |
| 1042 | N.T. | N.T. | H | H | H | H |
| 1043 | N.T. | N.T. | H | H | H | H |
| 1044 | N.T. | N.T. | H | H | H | H |
| 1045 | N.T. | N.T. | H | H | H | H |
| 1046 | N.T. | N.T. | H | H | H | H |
| 1046 | N.T. | N.T. | H | H | H | N.T. |
| 1047 | N.T. | N.T. | H | H | H | N.T. |
| 1048 | N.T. | N.T. | H | H | H | N.T. |
| 1049 | N.T. | N.T. | H | H | H | N.T. |
| 1050 | N.T. | N.T. | H | H | H | N.T. |
| 1051 | N.T. | N.T. | H | H | H | N.T. |

**[Table 8-35]**

| | | | | | | |
|---|---|---|---|---|---|---|
| 1052 | N.T. | N.T. | H | H | H | N.T. |
| 1053 | N.T. | N.T. | H | H | H | N.T. |
| 1054 | N.T. | N.T. | H | H | H | N.T. |
| 1055 | N.T. | N.T. | N.T. | N.T. | N.T. | N.T. |
| 1056 | N.T. | N.T. | H | H | H | N.T. |
| 1057 | N.T. | N.T. | H | H | H | N.T. |
| 1058 | N.T. | N.T. | H | H | H | N.T. |
| 1059 | N.T. | N.T. | H | H | H | N.T. |
| 1060 | N.T. | N.T. | H | H | H | N.T. |
| 1061 | N.T. | N.T. | H | H | H | N.T. |
| 1062 | N.T. | N.T. | H | H | H | N.T. |
| 1063 | N.T. | N.T. | H | H | H | N.T. |
| 1064 | N.T. | N.T. | H | H | H | N.T. |
| 1065 | N.T. | N.T. | H | H | H | N.T. |
| 1066 | N.T. | N.T. | H | H | H | N.T. |
| 1067 | N.T. | N.T. | H | H | H | N.T. |
| 1068 | N.T. | N.T. | H | H | H | N.T. |
| 1069 | N.T. | N.T. | H | H | H | N.T. |
| 1070 | N.T. | N.T. | H | H | H | N.T. |
| 1071 | N.T. | N.T. | H | H | H | N.T. |
| 1072 | N.T. | N.T. | H | H | H | N.T. |
| 1073 | N.T. | N.T. | H | H | H | N.T. |
| 1074 | N.T. | N.T. | H | H | H | N.T. |
| 1075 | N.T. | N.T. | H | H | H | N.T. |
| 1076 | N.T. | N.T. | H | H | H | N.T. |
| 1077 | N.T. | N.T. | H | H | H | N.T. |
| 1078 | N.T. | N.T. | H | H | H | N.T. |
| 1079 | N.T. | N.T. | H | H | H | N.T. |
| 1080 | N.T. | N.T. | H | H | H | N.T. |
| 1081 | N.T. | N.T. | H | H | H | N.T. |
| 1082 | N.T. | N.T. | H | H | H | N.T. |

**[Table 8-36]**

| | | | | | | |
|---|---|---|---|---|---|---|
| 1083 | N.T. | N.T. | H | H | H | N.T. |
| 1084 | N.T. | N.T. | H | H | H | N.T. |
| 1085 | N.T. | N.T. | H | H | H | N.T. |
| 1086 | N.T. | N.T. | H | H | H | N.T. |
| 1087 | N.T. | N.T. | H | H | H | N.T. |
| 1088 | N.T. | N.T. | H | H | H | N.T. |
| 1089 | N.T. | N.T. | H | H | H | N.T. |
| 1090 | N.T. | N.T. | H | H | H | N.T. |
| 1091 | N.T. | N.T. | H | H | H | N.T. |
| 1092 | N.T. | N.T. | H | H | H | N.T. |
| 1093 | N.T. | N.T. | H | H | H | N.T. |
| 1094 | N.T. | N.T. | H | H | H | N.T. |
| 1095 | N.T. | N.T. | H | H | H | N.T. |
| 1096 | N.T. | N.T. | H | H | H | N.T. |
| 1097 | N.T. | N.T. | H | H | H | N.T. |
| 1098 | N.T. | N.T. | H | H | H | N.T. |
| 1099 | N.T. | N.T. | H | H | H | N.T. |
| 1100 | N.T. | N.T. | H | H | H | N.T. |
| 1101 | N.T. | N.T. | H | H | H | N.T. |
| 1102 | N.T. | N.T. | H | H | H | N.T. |
| 1103 | N.T. | N.T. | H | H | H | N.T. |
| 1104 | N.T. | N.T. | H | H | H | N.T. |
| 1105 | N.T. | N.T. | H | H | H | N.T. |
| 1106 | N.T. | N.T. | H | H | H | N.T. |
| 1107 | N.T. | N.T. | H | H | H | N.T. |
| 1108 | N.T. | N.T. | H | H | H | N.T. |
| 1109 | N.T. | N.T. | H | H | H | N.T. |
| 1110 | N.T. | N.T. | H | H | H | N.T. |
| 1111 | N.T. | N.T. | H | H | H | N.T. |
| 1112 | N.T. | N.T. | H | H | H | N.T. |
| 1113 | N.T. | N.T. | H | H | H | N.T. |

**[Table 8-37]**

| | | | | | | |
|---|---|---|---|---|---|---|
| 1114 | N.T. | N.T. | H | H | H | N.T. |
| 1115 | N.T. | N.T. | H | H | H | N.T. |
| 1116 | N.T. | N.T. | H | H | H | N.T. |
| 1117 | N.T. | N.T. | H | H | H | N.T. |
| 1118 | N.T. | N.T. | H | H | H | N.T. |
| 1119 | N.T. | N.T. | H | H | H | N.T. |
| 1120 | N.T. | N.T. | H | H | H | N.T. |
| 1121 | N.T. | N.T. | H | H | H | N.T. |
| 1122 | N.T. | N.T. | H | H | H | N.T. |
| 1123 | N.T. | N.T. | H | H | H | N.T. |
| 1124 | N.T. | N.T. | H | H | H | N.T. |
| 1125 | N.T. | N.T. | H | H | H | N.T. |
| 1126 | N.T. | N.T. | H | H | H | N.T. |
| 1127 | N.T. | N.T. | H | H | H | N.T. |
| 1128 | N.T. | N.T. | H | H | H | N.T. |
| 1129 | N.T. | N.T. | H | H | H | N.T. |
| 1130 | N.T. | N.T. | H | H | H | N.T. |
| 1131 | N.T. | N.T. | H | H | H | N.T. |
| 1132 | N.T. | N.T. | H | H | H | N.T. |
| 1133 | N.T. | N.T. | H | H | H | N.T. |
| 1134 | N.T. | N.T. | H | H | H | N.T. |
| 1135 | N.T. | N.T. | H | H | H | N.T. |
| 1136 | N.T. | N.T. | H | H | H | N.T. |
| 1137 | N.T. | N.T. | H | H | H | N.T. |
| 1138 | N.T. | N.T. | H | H | H | N.T. |
| 1139 | N.T. | N.T. | H | H | H | N.T. |
| 1140 | N.T. | N.T. | H | H | H | N.T. |
| 1141 | N.T. | N.T. | H | H | H | N.T. |
| 1142 | N.T. | N.T. | H | H | H | N.T. |
| 1143 | N.T. | N.T. | H | H | H | N.T. |
| 1144 | N.T. | N.T. | H | H | H | N.T. |

**[Table 8-38]**

| | | | | | | |
|---|---|---|---|---|---|---|
| 1145 | N.T. | N.T. | H | H | H | N.T. |
| 1146 | N.T. | N.T. | H | H | H | N.T. |
| 1147 | N.T. | N.T. | H | H | H | N.T. |
| 1148 | N.T. | N.T. | H | H | H | N.T. |
| 1149 | N.T. | N.T. | H | H | H | N.T. |
| 1150 | N.T. | N.T. | H | H | H | N.T. |
| 1151 | N.T. | N.T. | H | H | H | N.T. |
| 1152 | N.T. | N.T. | H | H | H | N.T. |
| 1153 | N.T. | N.T. | H | H | H | N.T. |
| 1154 | N.T. | N.T. | H | H | H | N.T. |
| 1155 | N.T. | N.T. | H | H | H | N.T. |

### [Industrial Applicability]

The compound of the present invention is a novel compound that can control a plant disease, and as such, is useful as an agricultural chemical, for example, an agricultural and horticultural pest control agent, particularly, an agricultural and horticultural germicide.

## Claims

1. A compound represented by the following formula (1) or a salt thereof: 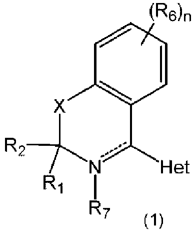 wherein
R₁ and R₂ each independently represent a group selected from the group consisting of a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C1 to C6 acyl group optionally substituted by substituent A, an aryl group optionally substituted by substituent B, a heteroaryl group optionally substituted by substituent B, -C(=O)NRaRb (wherein Ra and Rb each independently represent a group selected from the group consisting of a hydrogen atom, a C1 to C6 alkyl group optionally substituted by substituent A, a C1 to C6 haloalkyl group, a C3 to C8 cycloalkyl group, a C1 to C6 acyl group, and -C(=O)ORc (wherein Rc represents a group selected from the group consisting of a hydrogen atom, a C1 to C6 alkyl group optionally substituted by substituent A, a C1 to C6 haloalkyl group, and a C3 to C8 cycloalkyl group), or Ra and Rb form, together with the nitrogen atom bonded thereto, an aziridinyl group, an azetidinyl group, a pyrrolidinyl group, a piperidinyl group, a homopiperidinyl group, or an azocanyl group), and -C(=O)ORc (wherein Rc is as defined above), or
R₁ and R₂ together optionally form a C3 to C8 cycloalkyl ring optionally substituted by substituent A, or a C3 to C8 saturated or unsaturated heterocyclic ring optionally substituted by substituent A;
Het represents the formula (2): 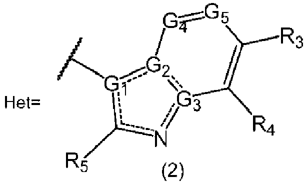
R₃, R₄, and R₅ in the formula (2) each independently represent a group selected from the group consisting of a hydrogen atom, a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, -NRaRb (wherein Ra and Rb are as defined above), - C(=O)NRaRb (wherein Ra and Rb are as defined above), -C(=O)ORc (wherein Rc is as defined above), - OC(=O)Rc (wherein Rc is as defined above), and -L-Rd (wherein Rd represents a group selected from the group consisting of a C1 to C6 alkyl group and a C1 to C6 haloalkyl group, and L represents a group selected from the group consisting of S, S(=O), and SO₂), or
R₃ and R₄ together form a C5 to C10 unsaturated ring optionally substituted by substituent A;
n represents an integer of 0 to 4;
R₆ represents a group selected from the group consisting of a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, -NRaRb (wherein Ra and Rb are as defined above), -C(=O)ORc (wherein Rc is as defined above), -C(=O)NRaRb (wherein Ra and Rb are as defined above), -OC(=O)Rc (wherein Rc is as defined above), and -L-Rd (wherein L and Rd are as defined above);
R₇ represents a group selected from the group consisting of a lone pair, a hydrogen atom, a C1 to C6 alkyl group optionally substituted by substituent A, a C1 to C6 haloalkyl group, a C2 to C6 alkenyl group optionally substituted by substituent A, a C2 to C6 haloalkenyl group, a C2 to C6 alkynyl group optionally substituted by substituent A, a C2 to C6 haloalkynyl group, and an acyl group optionally substituted by substituent A;
G₁, G₂, and G₃ each independently represent a group selected from the group consisting of a carbon atom and a nitrogen atom (on the proviso that at least one of G₁, G₂, and G₃ is a carbon atom);
G₄ and G₅ each independently represent a group selected from the group consisting of a nitrogen atom and CRx;
Rx represents a group selected from the group consisting of a hydrogen atom, a halogen atom, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, -NRaRb (wherein Ra and Rb are as defined above), - C(=O)NRaRb (wherein Ra and Rb are as defined above), -C(=O)ORc (wherein Rc is as defined above), and -L-Rd (wherein L and Rd are as defined above);
the broken line represents a single bond or a double bond;
X represents a group selected from the group consisting of O, S, S(=O), SO₂, and NRe;
Re represents a group selected from the group consisting of a hydrogen atom, a C1 to C6 alkyl group optionally substituted by substituent A, a C1 to C6 haloalkyl group, a C2 to C6 alkenyl group optionally substituted by substituent A, a C2 to C6 haloalkenyl group, a C2 to C6 alkynyl group optionally substituted by substituent A, and a C2 to C6 haloalkynyl group;
the substituent A is at least one member selected from the group consisting of a hydroxy group, a cyano group, a C3 to C8 cycloalkyl group, a C1 to C6 alkoxy group, a C1 to C6 haloalkoxy group, and a C1 to C6 cycloalkoxy group, a C1 to C6 alkenyloxy group, a C1 to C6 haloalkenyloxy group, a C1 to C6 alkynyloxy group, and a C1 to C6 haloalkynyloxy group, a C1 to C6 acyl group, an aryl group optionally substituted by substituent B, a heteroaryl group optionally substituted by substituent B, a phenoxy group optionally substituted by substituent B, -NRaRb (wherein Ra and Rb are as defined above), -C(=O)NRaRb (wherein Ra and Rb are as defined above), -C(=O)ORc (wherein Rc is as defined above), -OC(=O)Rc (wherein Rc is as defined above), and -L-Rd (wherein L and Rd are as defined above); and
the substituent B is at least one member selected from the group consisting of a halogen atom, a hydroxy group, a cyano group, a nitro group, a C1 to C6 alkyl group optionally substituted by substituent A, a C1 to C6 haloalkyl group, a C3 to C8 cycloalkyl group, a C1 to C6 alkoxy group, a C1 to C6 haloalkoxy group, and a C3 to C8 cycloalkoxy group, and Rd-L- (wherein Rd is as defined above).

2. The compound or the salt thereof according to claim 1, wherein at least one of G₄ and G₅ is a nitrogen atom.

3. The compound or the salt thereof according to claim 1, wherein
R₃, R₄, and R₅ each independently represent a group selected from the group consisting of a hydrogen atom, a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, and a C3 to C6 haloalkynyloxy group, or
R₃ and R₄ together form a C5 to C10 unsaturated ring optionally substituted by substituent A;
n represents an integer of 0 to 2;
R₆ represents a group selected from the group consisting of a halogen atom, a hydroxy group, a cyano group, a nitro group, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, a C2 to C6 haloalkynyl group, a C2 to C6 alkenyloxy group optionally substituted by substituent A, a C2 to C6 haloalkenyloxy group, a C3 to C6 alkynyloxy group optionally substituted by substituent A, a C3 to C6 haloalkynyloxy group, and -NRaRb (wherein Ra and Rb are as defined above);
R₇ represents a group selected from the group consisting of a lone pair, a hydrogen atom, a C1 to C6 alkyl group optionally substituted by substituent A, a C1 to C6 haloalkyl group, and an acyl group optionally substituted by substituent A;
G₄ and G₅ each independently represent a group selected from the group consisting of a nitrogen atom and CRx; and
Rx represents a group selected from the group consisting of a hydrogen atom, a halogen atom, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C1 to C6 haloalkoxy group, a C3 to C8 cycloalkoxy group optionally substituted by substituent A, a C2 to C6 alkenyl group optionally substituted by substituent A, a C2 to C6 haloalkenyl group, a C2 to C6 alkynyl group optionally substituted by substituent A, and a C2 to C6 haloalkynyl group.

4. The compound or the salt thereof according to claim 3, wherein at least one of G₄ and G₅ is a nitrogen atom.

5. The compound or the salt thereof according to claim 3, wherein
R₁ and R₂ each independently represent a group selected from the group consisting of a C1 to C6 alkyl group optionally substituted by substituent A, a C1 to C6 haloalkyl group, a C3 to C8 cycloalkyl group optionally substituted by substituent A, a C2 to C6 alkenyl group optionally substituted by substituent A, a C1 to C6 acyl group optionally substituted by substituent A, an aryl group optionally substituted by substituent B, a heteroaryl group optionally substituted by substituent B, and -C(=O)ORc (wherein Rc is as defined above) or
R₁ and R₂ together form a C3 to C8 cycloalkyl ring optionally substituted by substituent A, or a C3 to C8 saturated or unsaturated heterocyclic ring optionally substituted by substituent A;
R₃, R₄, and R₅ each independently represent a group selected from the group consisting of a hydrogen atom, a halogen atom, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, and a C2 to C6 alkenyl group optionally substituted by substituent A, or
R₃ and R₄ together form a C5 to C10 unsaturated ring optionally substituted by substituent A;
n represents an integer of 0 to 2;
R₆ represents a group selected from the group consisting of a halogen atom, a C1 to C6 alkyl group optionally substituted by substituent A, a C1 to C6 haloalkyl group, a C3 to C8 cycloalkyl group optionally substituted by substituent A, a C1 to C6 alkoxy group optionally substituted by substituent A, a C3 to C8 cycloalkoxy group optionally substituted by substituent A, a C2 to C6 alkenyl group optionally substituted by substituent A, and -NRaRb (wherein Ra and Rb are as defined above);
R₇ represents a lone pair;
at least one of G₁, G₂, and G₃ represents a carbon atom and at least one represents a nitrogen atom;
G₄ and G₅ each independently represent a group selected from the group consisting of a nitrogen atom and CRx;
Rx represents a group selected from the group consisting of a hydrogen atom and a C1 to C6 alkyl group optionally substituted by substituent A;
the binding containing the broken line represents a single bond or a double bond; and
X represents a group selected from the group consisting of O, S, S(=O), and SO₂.

6. The compound or the salt thereof according to claim 5, wherein the formula (2) is selected from the group consisting of following (2-1), (2-2), (2-3), (2-4), (2-5), (2-6), (2-7), (2-8), (2-9), (2-10), and (2-11), 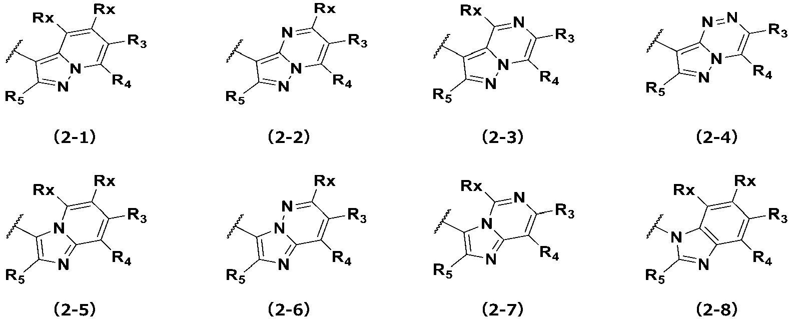 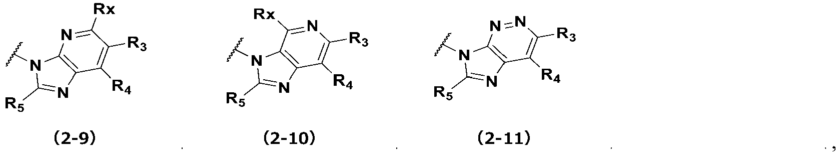 and
R₃, R₄, and Rx are as defined above.

7. The compound or the salt thereof according to claim 5, wherein the formula (2) is selected from the group consisting of following (2-1), (2-2), (2-3), (2-5), (2-6), (2-8), and (2-9), 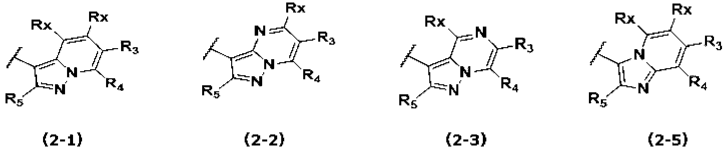 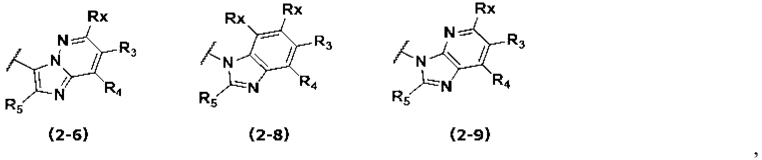 and
R₃, R₄, and Rx are as defined above.

8. The compound or the salt thereof according to claim 5, wherein at least one of G₄ and G₅ is a nitrogen atom. the formula (2) is selected from the group consisting of following (2-2), (2-3), (2-6), and (2-9), 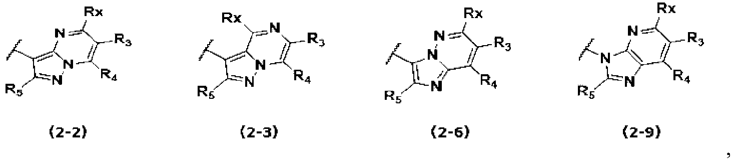 and
R₃, R₄, and Rx are as defined above.

9. An agricultural and horticultural pest control agent comprising a compound or a salt thereof according to any one of claims 1 to 8.

10. An agricultural and horticultural germicide comprising a compound or a salt thereof according to any one of claims 1 to 8.

11. A method for controlling a plant disease, comprising applying a compound or a salt thereof according to any of claim 1 to 8 or an agricultural and horticultural pest control agent according to claim 9 to a plant, a plant seed, or soil for cultivating a plant.

12. A method for controlling a plant disease, comprising applying a compound or a salt thereof according to any of claim 1 to 8 or an agricultural and horticultural germicide according to claim 10 to a plant, a plant seed, or soil for cultivating a plant.
